# EUROPÄISCHE PATENTANMELDUNG

(11) **EP 3 501 546 A2**
(43) Veröffentlichungstag der Anmeldung: **26.06.2019**
(21) Anmeldenummer: 19150066.9
(22) Anmeldetag: 20.04.2012
(51) Int. Cl.: A61K 47/54, C07K 16/30, A61P 35/00

(54) **CYSTEIN-WIRKSTOFF KONJUGATE UND IHRE VERWENDUNG**

(30) Priorität: 21.04.2011 EP 11163467; 21.04.2011 EP 11163474; 21.04.2011 EP 11163472; 21.04.2011 EP 11163470; 01.06.2011 EP 11168557; 01.06.2011 EP 11168556; 01.06.2011 EP 11168559; 01.06.2011 EP 11168558; 14.12.2011 EP 11193621; 14.12.2011 EP 11193623; 14.12.2011 EP 11193609; 14.12.2011 EP 11193618
(62) Teilanmeldung aus: 12718624.5
(71) Anmelder: Seattle Genetics, Inc., Bothell, WA 98021 (US)
(72) Erfinder: Lerchen, Hans-Georg, 51375 Leverkusen (DE); Linden, Lars, 40235 Düsseldorf (DE); El Sheikh, Sherif, 45219 Essen (DE); Willuda, Jörg, 16548 Glienicke/Nordbahn (DE); Kopitz, Charlotte Christine, 10589 Berlin (DE); Schuhmacher, Joachim, 42113 Wuppertal (DE); Greven, Simone, 41541 Dormagen (DE); Mahlert, Christoph, 42111 Wuppertal (DE); Stelte-Ludwig, Beatrix, 42489 Wülfrath (DE); Golfier, Sven, 12247 Berlin (DE); Beier, Rudolf, 13353 Berlin (DE); Heisler, Iring, 42369 Wuppertal (DE); Harrenga, Axel, 42115 Wuppertal (DE); Thierauch, Karl-Heinz, 14169 Berlin (DE); Bruder, Sandra, 53175 Leverkusen (DE); Petrul, Heike, 10249 Berlin (DE); Jörißen, Hannah, 45239 Essen (DE); Borkowski, Sandra, 16540 Hohen Neuendorf (DE)
(74) Vertreter: Hoffmann Eitle

(57) **Zusammenfassung**

Die vorliegende Anmeldung betrifft neue, gegen das Target C4.4a gerichtete Binder-Wirkstoff-Konjugate (ADCs) von N,N-Dialkylauristatinen, wirlksamer Metabolite dieser ADCs, Verfahren zur Herstellung dieser ADCs, die Verwendung dieser ADCs zur Behandlung und/oder Prävention von Krankheiten sowie die Verwendung dieser ADCs zur Herstellung von Arzneimitteln zur Behandlung und/oder Prãvention von Krankheiten, insbesondere von hyperproliferativen und/oder angiogenen Erkankungen wie beispielsweise Krebserkrankungen. Solche Behandlungen können als Monotherapie oder auch in Kombination mit anderen Arzneimitteln oder weiteren therapeutischen Maßnahmen erfolgen.

## Beschreibung

Die vorliegende Anmeldung betrifft neue, gegen das Target C4.4a gerichtete Binder-Wirkstoff-Konjugate (ADCs) von N,N-Dialkylauristatinen, wirksamer Metabolite dieser ADCs, Verfahren zur Herstellung dieser ADCs, die Verwendung dieser ADCs zur Behandlung und/oder Prävention von Krankheiten sowie die Verwendung dieser ADCs zur Herstellung von Arzneimitteln zur Behandlung und/oder Prävention von Krankheiten, insbesondere von hyperproliferativen und/oder angiogenen Erkrankungen wie beispielsweise Krebserkrankungen. Solche Behandlungen können als Monotherapie oder auch in Kombination mit anderen Arzneimitteln oder weiteren therapeutischen Maßnahmen erfolgen.

Krebserkrankungen sind die Folge unkontrollierten Zellwachstums verschiedenster Gewebe. In vielen Fällen dringen die neuen Zellen in bestehende Gewebe ein (invasives Wachstum), oder sie metastasieren in entfernte Organe. Krebserkrankungen treten in verschiedensten Organen auf und haben oft gewebespezifische Krankheitsverläufe. Daher beschreibt die Bezeichnung Krebserkrankung als Oberbegriff eine große Gruppe definierter Erkrankungen verschiedener Organe, Gewebe und Zelltypen.

Tumore früher Stadien lassen sich gegebenenfalls durch chirurgische und radiotherapeutische Maßnahmen entfernen. Metastasierte Tumore können im Regelfall durch Chemotherapeutika nur palliativ therapiert werden. Ziel hierbei ist, die optimale Kombination aus einer Verbesserung der Lebensqualität und der Verlängerung der Lebenszeit zu erreichen.

Die meisten der heutzutage parenteral applizierten Chemotherapeutika sind oft nicht zielgerichtet auf das Tumorgewebe oder die Tumorzellen, sondern durch die systemische Gabe unspezifisch im Körper verteilt, d.h. auch an Orten, an denen eine Wirkstoffexposition unerwünscht ist, wie beispielsweise in gesunden Zellen, Geweben und Organen. Dies kann zu unerwünschten Nebenwirkungen bis hin zu gravierenden allgemein-toxischen Effekten führen, die dann den therapeutisch nutzbaren Dosisbereich des Wirkstoffs oftmals stark limitieren oder ein völliges Absetzen der Medikation erfordern.

Die verbesserte und selektive Verfügbarkeit dieser Chemotherapeutika in der Tumorzelle oder dem direkt umgebenden Gewebe und die damit verbundene Wirksteigerung einerseits und Minimierung toxischer Nebeneffekte andererseits steht daher seit mehreren Jahren im Fokus bei der Entwicklung neuer Chemotherapeutika. Viele Versuche wurden bislang unternommen, effiziente Methoden für die Wirkstoffeinbringung in die Zielzelle zu entwickeln. Die Optimierung der Assoziation zwischen Wirkstoff und intrazellulärem Ziel und die Minimierung der interzellulären Wirkstoff-verteilung, beispielsweise zu Nachbarzellen, stellen jedoch nach wie vor eine schwierige Aufgabe dar.

Für die zielgerichtete Adressierung von Tumorgewebe und Tumorzellen sind beispielsweise monoklonale Antikörper geeignet. Die Bedeutung solcher Antikörper für die klinische Behandlung von Krebserkrankungen hat allgemein in den letzten Jahren erheblich zugenommen, basierend auf der Wirksamkeit solcher Agenzien wie Trastuzumab (Herceptin), Rituximab (Rituxan), Cetuximab (Erbitux) und Bevacizumab (Avastin), welche inzwischen für die Therapie einzelner, spezifischer Tumorerkrankungen zugelassen sind [siehe z.B. G. P. Adams und L. M. Weiner, Nat. Biotechnol. 23, 1147-1157 (2005)]. In Folge hiervon ist auch das Interesse an so genannten Immunokonjugaten wie bspw. den oben genannten ADCs deutlich gestiegen, in welchen ein internalisierender, gegen ein Tumor-assoziiertes Antigen gerichteter Antikörper auf kovalente Weise über eine Verknüpfungseinheit ("linker") mit einem cytotoxisch wirkenden Agens verbunden ist. Nach Einschleusung des ADCs in die Tumorzelle und anschließender Spaltung des Konjugats wird dann innerhalb der Tumorzelle entweder das cytotoxische Agens selbst oder ein anderer daraus gebildeter cytotoxisch wirksamer Metabolit freigesetzt, und kann dort seine Wirkung direkt und selektiv entfalten. Auf diesem Wege könnte die Schädigung von normalem Gewebe im Vergleich zu einer konventionellen Chemotherapie der Krebserkrankung in signifikant engeren Grenzen gehalten werden [siehe z.B. J. M. Lambert, Curr. Opin. Pharmacol. 5, 543-549 (2005); A. M. Wu und P. D. Senter, Nat. Biotechnol. 23, 1137-1146 (2005); P. D. Senter, Curr. Opin. Chem. Biol. 13, 235-244 (2009); L. Ducry und B. Stump, Bioconjugate Chem. 21, 5-13 (2010)].

Statt Antikörpern können auch Binder aus dem Wirkstoffbereich kleiner Moleküle als Binder verwendet werden, die selektiv an einen speziellen Zielort ("target"), wie beispielsweise an einen Rezeptor, binden [siehe z.B. E. Ruoslahti et al., Science 279, 377-380 (1998); D. Karkan et al., PLoS ONE 3 (6), e2469 (June 25, 2008)]. Bekannt sind auch Konjugate aus cytotoxischem Wirkstoff und adressierendem Ligand, die zwischen Ligand und Wirkstoff eine definierte Spaltstelle zur Freisetzung des Wirkstoffs aufweisen. Eine solche "Soll-Bruchstelle" kann beispielsweise in einer Peptidkette bestehen, die an einer bestimmten Stelle selektiv durch ein spezielles Enzym am Wirkort gespalten werden kann [siehe z.B. R. A. Firestone und L. A. Telan, US Patent Application US 2002/0147138].

Für die zielgerichtete Adressierung von Tumorgewebe und Tumorzellen sind insbesondere monoklonale Antikörper geeignet, die gegen das Antigen C4.4a gerichtet sind. C4.4a (Gen: LYPD3) wurde zuerst als ein Metastase-assoziiertes, Zelloberflächenprotein in Ratten-Pankreas Tumorzellen beschrieben (Rösel M. et al., Oncogene 1998,17(15):1989-2002). Humanes C4.4a wurde aus seiner plazentalen cDNA Bibliothek (Würfel, J. et. al. Gene 2001,262:35-41) isoliert. C4.4a zeigt strukturelle Homologie zum uPA Rezeptor und enthält zwei LY6 Domänen, die das typische Drei-Finger Faltungsmotiv aufweisen und über 9 Disulfidbrücken verknüpft sind (Jacobsen B. & Ploug M., Current Medicinal Chemistry 2008, 15:2559-2573). C4.4a ist über Glykophosphatidylinositol (GPI) in der Zelle verankert. Das Protein ist stark glykosyliert und enthält zahlreiche N- und O- Glykosylierungsstellen. C4.4a zeigt eine starke Expression in Tumorzellen von Lungenkrebs, Dickdarmkrebs, Brustkrebs, Gebärmutterkrebs Pankreaskrebs, Nierenkrebs, Kopf und Halstumoren und Melanomen. RNA Analysen zeigten C4.4a Expression in ∼ 50 % primärer Lungentumore und ∼75 % von Lungenkrebsmetastasen, eine Expression in gesundem Lungengewebe war hingegen nicht nachweisbar (Würfel J. et. al., Gene 2001, 262:35-41). In nicht-kleinzelligem Lungenkrebs kann C4.4a als prognostischer Marker eingesetzt werden, dabei korreliert eine hohe C4.4a Expression mit einer schlechten Prognose (Hansen L. et al., Lung Cancer 2007, 58:260-266). Gleiches gilt bei Dickdarmkrebs C4.4a wird von der Tumorzelloberfläche abgespalten und kann als prognostischer Serummarker verwendet werden (K. Konishi et al., Cancer Science 2010). Eine detaillierte Expressionsanalyse von Melanomen zeigte, dass C4.4a in ∼ 60 % der primären malignen Melanome und in 100 % von Lymphknoten- und Haut-Metastasen exprimiert wird (Seiter S. et al., J Invest Dermatol. 2001, 116(2):344-347). Eine Hochregulation der C4.4a Genexpression wird in Brustkrebsgewebe verglichen mit benachbarten Normalgeweben beobachtet (Fletcher G.C., Br. J. Cancer 2003, 88(4):579-585). C4.4a ist ein ideales Zielprotein für eine Tumortherapie, da die C4.4a Expression in gesunden Geweben auf Haut-Keratinozyten und Speiseröhrenendothelzellen, sowie Plazentazellen beschränkt ist (Würfel J. et. al., Gene 2001, 262:35-41). WO01/23553 beschreibt die Verwendung eines C4.4a Inhibitors (z.B. eines Anti-C4.4a Antikörpers), welcher bei einer Krebstherapie die C4.4a Expression oder Aktivität hemmen kann.

Die genaue Funktion von C4.4a ist unbekannt. Während der Wundheilung ist es in wanderenden Keratinozyten hochreguliert (Hansen L. et al., Biochem J. 2004, 380:845-857). Es wird vermutet, dass C4.4a eine Rolle bei der Tumorzellinvasion, vermutlich durch die Interaktion mit der extrazellulären Matrix spielt (Rösel M. et al., Oncogene 1998, 17(15):1989-2002; Paret C. et al., British Journal of Cancer 2007, 97:1146-1156). Potentielle Liganden sind Laminin 1 und 5, sowie Galectin 3 (Paret C., Int. J. Cancer 2005, 115:724-733).

Auristatin E (AE) und Monomethylauristatin E (MMAE) sind synthetische Analoga der Dolastatine, einer speziellen Gruppe von linearen Pseudopeptiden, welche ursprünglich aus marinen Quellen isoliert wurden und die zum Teil sehr potente cytotoxische Aktivität gegenüber Tumorzellen aufweisen [für eine Übersicht siehe z.B. G. R. Pettit, Prog. Chem. Org. Nat. Prod. 70, 1-79 (1997); G. R. Pettit et al., Anti-Cancer Drug Design 10. 529-544 (1995); G. R. Pettit et al., Anti-Cancer Drug Design 13, 243-277 (1998)].

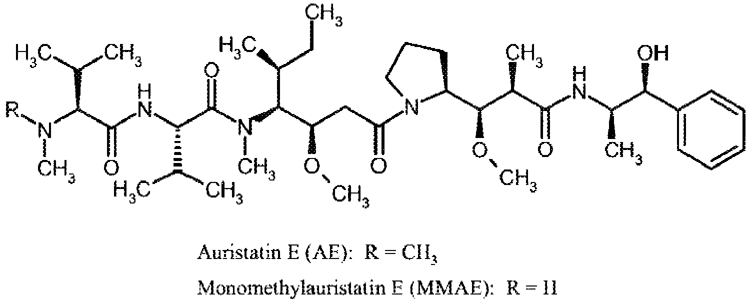

Allerdings besitzt MMAE den Nachteil einer vergleichsweise hohen systemischen Toxizität. Zur Verbesserung der Tumorselektivität wird MMAE insbesondere in Verbindung mit enzymatisch spaltbaren Valin-Citrullin-Linkern im ADC Setting zur gezielteren Tumortherapie eingesetzt [WO 2005/081711-A2; S. O. Doronina et al., Bioconjugate Chem. 17, 114-124 (2006)]. Nach proteolytischer Spaltung wird MMAE vorzugsweise intrazellulär aus entsprechenden ADCs freigesetzt.

MMAE ist jedoch bei einer Anwendung in Form von Antikörper-Wirkstoff-Konjugaten (ADCs) nicht kompatibel mit Verknüpfungseinheiten (Linkern) zwischen Antikörper und Wirkstoff, welche keine enzymatisch spaltbare Soll-Bruchstelle aufweisen [S. O. Doronina et al., Bioconjugate Chem. 17, 114-124 (2006)].

Monomethylauristatin F (MMAF) ist ein Auristatin-Derivat mit einer C-terminalen Phenylalanin-Einheit, das nur moderate anti-proliferative Wirkung im Vergleich zu MMAE aufweist. Dies ist sehr wahrscheinlich auf die freie Carboxylgruppe zurückzuführen, die aufgrund ihrer Polarität und Ladung die Zellgängigkeit dieser Verbindung negativ beeinflusst. In diesem Zusammenhang wurde der Methylester von MMAF (MMAF-OMe) als ein neutral geladenes, zellgängiges Prodrug-Derivat beschrieben, das im Vergleich zu MMAF eine um mehrere Größenordnungen erhöhte *in vitro-*Cytotoxizität gegenüber verschiedenen Karzinoma-Zelllinien zeigt [S. O. Doronina et al., Bioconjugate Chem. 17, 114-124 (2006)]. Es ist anzunehmen, dass diese Wirkung durch MMAF selbst hervorgerufen wird, das nach Aufnahme des Prodrugs in die Zellen schnell durch intrazelluläre Ester-Hydrolyse freigesetzt wird.

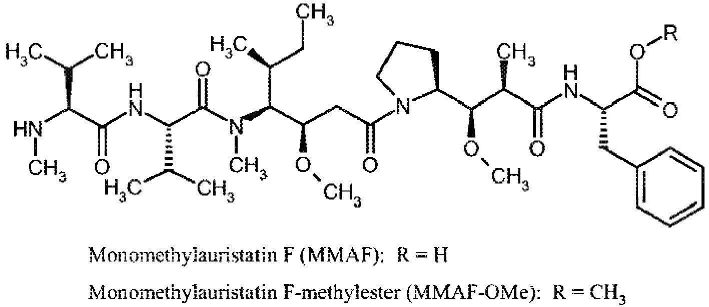

Wirkstoff-Verbindungen auf Basis von einfachen Ester-Derivaten unterliegen allgemein jedoch dem Risiko einer chemischen Instabilität infolge einer unspezifischen, vom vorgesehenen Wirkort unabhängigen Ester-Hydrolyse, beispielsweise durch im Blutplasma vorhandene Esterasen; dies kann die Anwendbarkeit solcher Verbindungen in der Therapie deutlich einschränken.

Monomethylauristatin F (MMAF) sowie verschiedene Ester- und Amid-Derivate hiervon wurden in WO 2005/081711-A2 offenbart. Weitere Auristatin-Analoga mit einer C-terminalen, amidisch substituierten Phenylalanin-Einheit sind in WO 01/18032-A2 beschrieben. In WO 02/088172-A2 und WO 2007/008603-A1 werden MMAF-Analoga beansprucht, welche Seitenketten-Modifikationen des Phenylalanins betreffen, und in WO 2007/008848-A2 solche, in denen die Carboxylgruppe des Phenylalanins modifiziert ist. Über den C-Terminus verknüpfte Auristatin-Konjugate wurden vor kurzem in WO 2009/117531-A1 beschrieben [siehe auch S. O. Doronina et al., Bioconjugate Chem. 19, 1960-1963 (2008)].

Darüber hinaus sind Auristatin-Derivate wie MMAE und MMAF auch Substrate für Transporterproteine, welche von vielen Tumorzellen exprimiert werden, was zu einer Resistenzentwicklung gegenüber diesen Wirkstoffen führen kann.

Aufgabe der vorliegenden Erfindung war die Bereitstellung neuer Binder-Wirkstoff-Konjugate (ADCs), die durch Kombination von neuen N,N-Dialkylauristatin-Derivaten mit neuartigen, geeigneten Linkern und Binder ein sehr attraktives Wirkprofil, wie beispielsweise hinsichtlich ihrer spezifischen Tumorwirkung und/oder des geringeren Potentials der intrazellulär gebildeten Metabolite als Substrat gegenüber Transporterproteinen, aufweisen, und sich daher zur Behandlung und/oder Prophylaxe von hyperproliferativen und/oder angiogenen Erkrankungen, wie beispielsweise Krebserkrankungen, eignen.

Gegenstand der vorliegenden Erfindung sind Binder-Wirkstoff Konjugate der allgemeinen Formel (Ia) 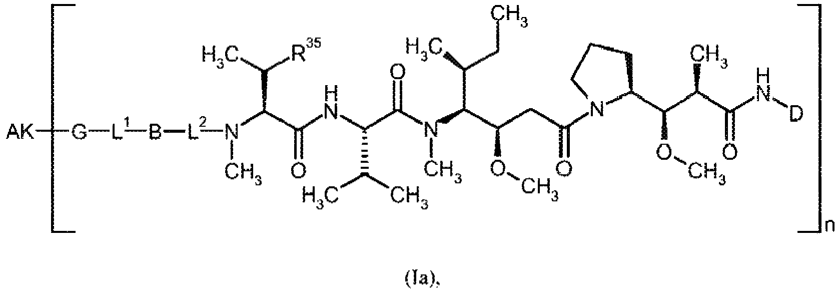 in welcher
- n: für eine Zahl von 1 bis 50 steht,
- AK: für einen Binder steht,
die Gruppe §-G-L¹-β-L²-§§ für einen Linker steht,
wobei
§ die Verknüpfungsstelle mit der Gruppe AK kennzeichnet und
§§ die Verknüpfungsstelle mit dem Stickstoffatom kennzeichnet,
- D: für eine Gruppe der Formel 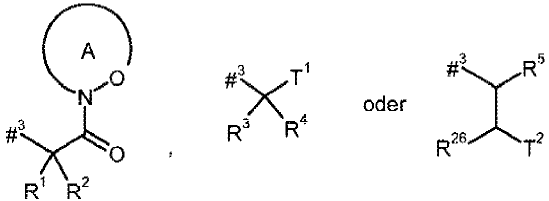 steht, wobei
#³ die Verknüpfungsstelle mit dem Stickstoffatom kennzeichnet,
R¹ für Wasserstoff oder Methyl steht,
R² für Isopropyl, Isobutyl, *sec.*-Butyl, *tert*-Butyl, Phenyl, Benzyl, 1-Hydroxyethyl, 4-Hyd-oxybenzyl, 4-Hydroxy-3-nitrobenzyl, 4-Hydroxy-3-aminobenzyl, 1-Phenylethyl, Diphenylmethyl, 1*H*-imidazol-4-ylmethyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 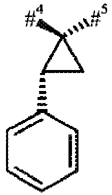 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bicyclischen, gegebenenfalls substituierten Heterocyclus der Formel 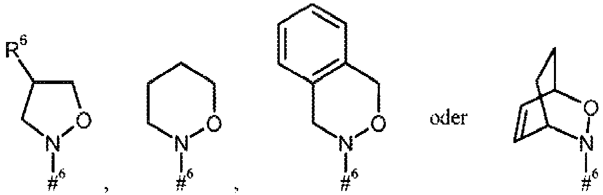 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff oder Methyl steht,
R⁴ für Isopropyl, Isobutyl, *sec*.-Butyl, *tert.*-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenyl-ethyl, Diphenylmethyl, 1*H*-Imidazol-4-ylmethyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 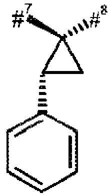 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(O)-NR⁸R⁹, -C(=O)-NH-NH-R¹⁰ oder -CH₂-O-R¹¹ steht,
worin
R⁷ für Wasserstoff, Methyl, Ethyl, *n*-Propyl, *tert*.-Butyl, Benzyl oder Adamantylmethyl sieht,
R⁸ für Wasserstoff oder Methyl steht,
R⁹ für Wasserstoff, Methyl, Ethyl, *n*-Propyl oder Benzyl steht,
oder
R⁸ und R⁹ bilden zusammen mit dem Stickstoffatom, an das sie gebunden sind, einen 4- bis 7-gliedrigen Heterocyclus,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der Formel 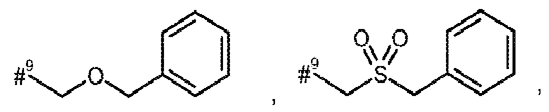 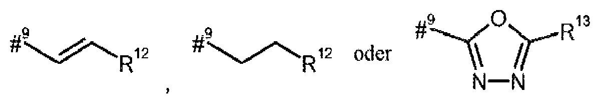 steht, worin
#⁹ die Verknüpfungsstelle mit -CHC(R²⁶)-T² kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
R²⁶ für Wasserstoff oder Hydroxy steht,
T² für Phenyl, Benzyl, 1*H-*Indol-3-yl oder 1*H*-Indol-3-ylmethyl steht,
R³⁵ für Methyl oder Hydroxy steht, sowie ihre Salze, Solvate und Solvate der Salze.

Erfindungsgemäße Verbindungen sind die Verbindungen der Formel (Ia) und (I) und deren Salze, Solvate und Solvate der Salze, die von Formel (Ia) und (I) umfassten Verbindungen der nachfolgend genannten Formeln und deren Salze, Solvate und Solvate der Salze sowie die von Formel (Ia) und (I) umfassten, nachfolgend als Ausführungsbeispiele genannten Verbindungen und deren Salze, Solvate und Solvate der Salze, soweit es sich bei den von Formel (Ia) und (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomeren und Diastereomeren und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagenzien und/oder Ausgangsverbindungen eingesetzt werden.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Benzolsulfonsäure, Toluolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methyl-piperidin, *N*-Methylmorpholin, Arginin, Lysin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₄)-Alkyl stehen im Rahmen der Erfindung für einen linearen oder verzweigten Alkylrest mit 1 bis 4 Kohlenstoffatomen. Beispielhaft und vorzugsweise seien genannt: Methyl, Ethyl, *n*-Propyl, Isopropyl, *n*-Butyl, Isobutyl, 1-Methylpropyl und *tert*.-Butyl.

Alkandiyl steht im Rahmen der Erfindung für einen linearen, α,ω-divalenten Alkylrest mit der jeweils angegebenen Zahl von Kohlenstoffatomen. Beispielhaft und vorzugsweise seien genannt: Methylen, Ethan-1,2-diyl (1,2-Ethylen), Propan-1,3-diyl (1,3-Propylen), Butan-1,4-diyl (1,4-Butylen), Pentan-1,5-diyl (1,5-Pentylen), Hexan-1,6-diyl (1,6-Hexylen), Heptan-1,7-diyl (1,7-Hexylen), Octan-1,8-diyl (1,8-Octylen), Nonan-1,9-diyl (1,9-Nonylen), Decan-1,10-diyl (1,10-Decylen).

(C₃-C₇)-Cycloalkyl bzw. 3- bis 7-gliedriger Carbocyclus steht im Rahmen der Erfindung für eine monocyclische, gesättigte Cycloalkylgruppe mit 3 bis 7 Kohlenstoffatomen. Beispielhaft und vorzugsweise seien genannt: Cyclopropyl, Cyclobutyl, Cyclopentyl, Cyclohexyl und Cycloheptyl.

Die Seitengruppe einer α-Aminosäure in der Bedeutung von R¹⁹ umfasst sowohl die Seitengruppen der natürlich vorkommenden α-Aminosäuren als auch die Seitengruppen von Homologen und Isomeren dieser α-Aminosäuren. Die α-Aminosäure kann hierbei sowohl in der L- als auch in der D-Konfiguration oder auch als Gemisch der L- und D-Form vorliegen. Als Seitengruppen seien beispielhaft genannt: Methyl (Alanin), Propan-2-yl (Valin), Propan-1-yl (Norvalin), 2-Methylpropan-1-yl (Leucin), 1-Methylpropan-1-yl (Isoleucin), Butan-1-yl (Norleucin), *tert*.-Butyl (2-*tert*.-Butyl-glycin), Phenyl (2-Phenylglycin), Benzyl (Phenylalanin), p-Hydroxybenzyl (Tyrosin), Indol-3-yl-methyl (Tryptophan), Imidazol-4-ylmethyl (Histidin), Hydroxymethyl (Serin), 2-Hydroxyethyl (Homoserin), 1-Hydroxyethyl (Threonin), Mercaptomethyl (Cystein), Methylthiomethyl (*S-*Methyleystein), 2-Mercaptoethyl (Homocystein), 2-Methylthioethyl (Methionin), Carbamoylmethyl (Asparagin), 2-Carbamoylethyl (Glutamin), Carboxymethyl (Asparaginsäure), 2-Carboxyethyl (Glutaminsäure), 4-Aminobutan-1-yl (Lysin), 4-Amino-3-hydroxybutan-1-yl (Hydroxylysin), 3-Aminopropan-1-yl (Ornithin), 2-Aminoethyl (2,4-Diaminobuttersäure), Aminomethyl (2,3-Diaminopropionsäure), 3-Guanidinopropan-1-yl (Arginin), 3-Ureidopropan-1-yl (Citrullin). Bevorzugte α-Aminosäure-Seitengruppen in der Bedeutung von R¹⁹ sind Methyl (Alanin), Propan-2-yl (Valin), 2-Methylpropan-1-yl (Leucin), Benzyl (Phenylalanin), Imidazol-4-ylmethyl (Histidin), Hydroxymethyl (Serin), 1-Hydroxyethyl (Threonin), 4-Aminobutan-1-yl (Lysin), 3-Aminopropan-1-yl (Ornithin), 2-Aminoethyl (2,4-Diaminobuttersäure), Aminomethyl (2,3-Diaminopropionsäure), 3-Guanidinopropan-1-yl (Arginin). Bevorzugt ist jeweils die L-Konfiguration.

Ein 4- bis 7-gliedriger Heterocyclus steht im Rahmen der Erfindung für einen monocyclischen, gesättigten Heterocyclus mit insgesamt 4 bis 7 Ringatomen, der ein oder zwei Ring-Heteroatome aus der Reihe N, O, S, SO und/oder SO₂ enthält und über ein Ring-Kohlenstoffatom oder gegebenenfalls ein Ring-Stickstoffatom verknüpft ist. Bevorzugt ist ein 5- bis 7-gliedriger Heterocyclus mit ein oder zwei Ring-Heteroatomen aus der Reihe N, O und/oder S, besonders bevorzugt ein 5- oder 6-gliedriger Heterocyclus mit ein oder zwei Ring-Heteroatomen aus der Reihe N und/oder O. Beispielhaft seien genannt: Azetidinyl, Oxetanyl, Pyrrolidinyl, Pyrazolidinyl, Tetrahydrofuranyl, Thiolanyl, Piperidinyl, Piperazinyl, Tetrahydropyranyl, Tetrahydrothiopyranyl, Morpholinyl, Thiomorpholinyl, Hexahydroazepinyl und Hexahydro-1,4-diazepinyl. Bevorzugt sind Pyrrolidinyl, Tetrahydrofuranyl, Piperidinyl, Piperazinyl, Tetrahydropyranyl und Morpholinyl.

In der Formel der Gruppe, für die A, B, D, G, L¹, L², L⁴, R¹, R², R³, R⁴ bzw. R⁵ stehen kann, steht der Endpunkt der Linie, an dem das Zeichen #⁶, *, **, #³, #¹, #², ##¹, ##², ##³, ##⁴, ***, ****, #⁴, #⁵, #⁶, #⁷, #⁸ bzw. #⁹ steht, nicht für ein Kohlenstoffatom beziehungsweise eine CH₂-Gruppe, sondern ist Bestandteil der Bindung zu dem jeweils bezeichneten Atom, an das A, B, D, G, L¹, L², L⁴, R¹, R², R³, R⁴ bzw. R⁵ gebunden ist.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei gleichen oder verschiedenen Substituenten ist bevorzugt. Besonders bevorzugt ist die Substitution mit einem Substituenten.

Im Rahmen der vorliegenden Erfindung haben die verwendeten Begriffe, soweit nicht anders spezifiziert, die folgende Bedeutung:
Der Begriff "Linker" wird im breitesten Sinne als eine chemische Einheit verstanden, die eine kovalente Bindung oder eine Anreihung von Atomen umfasst, die einen Binder an einen Wirkstoff kovalent knüpft. Vorzugsweise wird der Begriff "Linker" als eine Anreihung von Atomen im Sinne der vorliegenden Erfindung verstanden, die einen Binder an einen Wirkstoff kovalent knüpft. Darüber hinaus können Linker beispielsweise divalente chemische Einheiten, wie Alkyldiyle, Aryldiyle, Heteroaryldiyle, Heterocyclyldiyle, Dicarbonylsäureester, Dicarbonylsäureamide darstellen.

Der Begriff "Binder" wird im breitesten Sinne als ein Molekül verstanden, welches an ein Zielmolekül, das auf einer bestimmten, mit dem Binder-Wirkstoffkonjugat zu adressierenden Zielzellen-Population vorhanden ist, bindet. Der Begriff Binder ist in seiner breitesten Auslegung zu verstehen und umfasst z.B. auch Lektine, Proteine, die bestimmte Zuckerketten binden können, oder Phospholipid-bindende Proteine. Solche Binder umfassen beispielsweise, hochmolekulare Proteine (Bindeproteine), Polypeptide oder Peptide (Bindepeptide), nicht-peptidische (z.B. Aptamere (US5,270,163) (Übersichtsartikel von Keefe AD., et al., Nat. Rev. Drug Discov. 2010; 9:537-550), oder Vitamine) und alle anderen zellbindenden Moleküle oder Substanzen. Bindeproteine sind z.B. Antikörper und Antikörperfragmente oder Antikörpermimetika wie z.B. Affibodies, Adnectins, Anticalins, DARPins, Avimers, Nanobodies (Übersichtsartikel von Gebauer M. et al., Curr. Opinion in Chem. Biol. 2009; 13:245-255; Nuttall S.D. et al., Curr. Opinion in Pharmacology 2008; 8:608-617). Bindepeptide sind z.B. Liganden eines Liganden-Rezeptorspaares, wie z.B. VEGF des Liganden-Rezeptorpaares VEGF/KDR, wie Transferrin des Liganden-Rezeptorpaares Transferrin/Transferrin-Rezeptor oder Cytokine/Cytokin-Rezeptor, wie TNFalpha des Liganden-Rezeptorpaares TNFalpha/TNFalpha Rezeptor.

Als Binder sind erfindungsgemäß bevorzugt (insbesondere humane, monoklonale) Antikörper oder Antigen-bindende Antikörperfragmente, die an C4.4a binden. Im Falle von anti-C4.4a-Antikörpern ist n, also die Anzahl an Toxophormolekülen pro Antikörpermolekül, vorzugsweise in dem Bereich von 1 bis 10, besonders bevorzugt 2 bis 8.

Ein "Zielmolekül" wird im breitesten Sinne als ein Molekül verstanden, welches in der Zielzellenpopulation vorhanden ist, und kann ein Protein (z.B. ein Rezeptor eines Wachstumsfaktors) oder ein nicht-peptidisches Molekül sein (z.B. ein Zucker oder Phospholipid). Bevorzugt ist es ein Rezeptor oder ein Antigen.

Der Begriff "extrazelluläres" Zielmolekül beschreibt ein an die Zelle gebundenes Zielmolekül, welches sich auf der Außenseite einer Zelle befindet oder den Teil eines Zielmoleküls, welcher sich auf der Außenseite einer Zelle befindet, d.h. ein Binder kann an einer intakten Zelle an sein extrazelluläres Zielmolekül binden. Ein extrazelluläres Zielmolekül kann in der Zellmembran verankert sein oder Bestandteil der Zellmembran sein. Der Fachmann kennt Verfahren, um extrazelluläre Zielmoleküle zu identifizieren. Für Proteine kann dies über eine Bestimmung der Transmembrandomäne(n) und die Orientierung des Proteins in der Membran geschehen. Diese Angaben sind in der Regel in Proteindatenbanken (z.B. SwissProt) hinterlegt.

Der Begriff "Krebs-Zielmolekül" beschreibt ein Zielmolekül, welches auf einer oder mehreren Krebszellarten im Vergleich zu nicht-Krebszellen des gleichen Gewebetypus vermehrt vorhanden ist. Bevorzugt ist das Krebs-Zielmolekül auf einer oder mehreren Krebszellarten im Vergleich zu nicht-Krebszellen des gleichen Gewebetypus selektiv vorhanden, wobei selektiv eine mindestens zweifache Anreicherung auf Krebszellen im Vergleich zu nicht-Krebszellen des gleichen Gewebetypus beschreibt (ein "selektives Krebs-Zielmolekül"). Die Verwendung von Krebs-Zielmolekülen erlaubt die selektive Therapie von Krebszellen mit den erfindungsgemäßen Konjugaten.

Der Binder kann über eine Bindung mit dem Linker verknüpft werden. Aus der Literatur sind verschiedene Möglichkeiten der kovalenten Kopplung (Konjugation) von organischen Molekülen an Antikörper bekannt. Die Verknüpfung des Binders kann mittels eines Heteroatoms des Binders erfolgen. Erfindungsgemäße Heteroatome des Binders, die zur Verknüpfung verwendet werden können sind Schwefel (in einer Ausführungsform via einer Sulfhydrylgruppe des Binders), Sauerstoff (erfindungsgemäß mittels einer Carboxyl oder Hydroxylgruppe des Binders) und Stickstoff (in einer Ausführungsform via einer primären oder sekundären Amingruppe oder Amidgruppe des Binders). Erfindungsgemäß bevorzugt ist die Konjugation der Toxophore an den Antikörper über ein oder mehrere Schwefelatome von Cystein-Resten des Antikörpers und/oder über ein oder mehrere NH-Gruppen von Lysin-Resten des Antikörpers. Diese Heteroatome können im natürlichen Binder vorhanden sein oder durch chemische oder molekularbiologische Methoden eingeführt werden. Erfindungsgemäß hat die Verknüpfung des Binders mit dem Toxophor nur einen geringen Einfluß auf die Bindeaktivität des Binders zum Zielmolekül. In einer bevorzugten Ausführungsform hat die Verknüpfung keinen Einfluß auf die Bindeaktivität des Binders zum Zielmolekül.

Der Begriff "Antikörper" wird gemäß der vorliegenden Erfindung in seinem breitesten Sinne verstanden und umfasst Immunglobulinmoleküle, beispielsweise intakte oder modifizierte monoklonale Antikörper, polyklonale Antikörper oder multispezifische Antikörper (z.B. bispezifische Antikörper). Ein Immunglobulinmolekül umfasst bevorzugt ein Molekül mit vier Polypeptidketten, zwei schwere Ketten (H Ketten) und zwei leichte Ketten (L Ketten), welche typischerweise durch Disulfidbrücken verknüpft sind. Jede schwere Kette umfasst eine variable Domäne der schweren Kette (abgekürzt VH) und konstante Domäne der schweren Kette. Die konstante Domäne der schweren Kette kann beispielsweise drei Domänen CH1, CH2 und CH3 umfassen. Jede leichte Kette umfasst eine variable Domäne (abgekürzt VL) und eine konstante Domäne. Die konstante Domäne der leichten Kette umfasst eine Domäne (abgekürzt CL). Die VH und VL Domänen können weiter unterteilt werden in Regionen mit Hypervariabilität, auch Komplementaritäts-bestimmende Regionen genannt ("complementarity determining region", abgekürzt CDR) und Regionen mit geringerer Sequenzvariabilität ("framework region", abgekürzt FR). Jede VH und VL Region setzt sich typischerweise aus drei CDRs und bis zu vier FRs zusammen. Beispielsweise vom Aminoterminus zum Carboxyterminus in der folgenden Reihenfolge FR1, CDR1, FR2, CDR2, FR3, CDR3, FR4. Ein Antikörper kann aus jeder dafür geecigneten Spezies erhalten werden z.B. Kanninchen, Lama, Kamel, Maus, oder Ratte. In einer Ausführungsform ist der Antikörper humanen oder murinen Ursprungs. Ein Antikörper kann z.B. human, humanisiert oder chimär sein.

Der Begriff "monoklonaler" Antikörper bezeichnet Antikörper, die aus einer Population substantiell homogener Antikörper erhalten wurden, d.h. individuelle Antikörper der Population sind bis auf natürlich auftretende Mutationen, welche in geringfügiger Anzahl auftreten können, identisch. Monoklonale Antikörper erkennen mit hoher Spezifität eine einzige antigene Bindestelle. Der Begriff monoklonaler Antikörper bezieht sich nicht auf ein bestimmtes Herstellungsverfahren.

Der Begriff "intakter" Antikörper bezieht sich auf Antikörper, die sowohl eine Antigen-bindende Domäne als auch die konstante Domäne der leichten und schweren Kette umfassen. Die konstante Domäne kann eine natürlich vorkommende Domäne sein, oder eine Variante davon, bei der eine oder mehrere Aminosäurepositionen verändert wurden.

Der Begriff "modifizierter intakter" Antikörper bezieht sich auf intakte Antikörper, die mit einem weiteren Polypeptid oder Protein, welche nicht von einem Antikörper stammen, über ihren Aminoterminus oder Carboxyterminus mittels einer kovalenten Bindung (z.B. einer Peptidverknüpfung) fusioniert wurden. Des Weiteren können Antikörper dahingehend modifiziert werden, dass an definierten Stellen reaktive Cysteine eingeführt werden, um die Kopplung an ein Toxophor zu erleichtern (siehe Junutula et al. Nat Biotechnol. 2008 Aug;26(8):925-32).

Der Begriff "humaner" Antikörper bezeichnet Antikörper, die aus einem Menschen erhalten werden können oder synthetische humane Antikörper sind. Ein "synthetischer" humaner Antikörper ist ein Antikörper, der in Teilen oder als ganzes von synthetischen Sequenzen *in silico* erhältlich ist, die auf der Analyse humaner Antikörpersequenzen basieren. Ein humaner Antikörper kann z.B. durch eine Nukleinsäure kodiert sein, die aus einer Bibliothek von Antikörpersequenzen, die humanen Ursprungs sind, isoliert wurde. Ein Beispiel solcher Antikörper ist in Söderlind et al., Nature Biotech. 2000, 18:853-856 zu finden.

Der Begriff "humanisierter" oder "chimärer" Antikörper beschreibt Antikörper, die aus einem nicht-humanen und einem humanen Sequenzanteil bestehen. Bei diesen Antikörpern wird ein Teil der Sequenzen des humanen Immunoglobulins (Rezipient) durch Sequenzanteile eines nicht-humanen Immunoglobulins (Donor) ersetzt. Der Donor ist in vielen Fällen ein murines Immunoglobulin. Bei humanisierten Antikörpern werden Aminosäuren der CDR des Rezipienten durch Aminosäuren des Donors ersetzt. Manchmal werden auch noch Aminosäuren des Frameworks durch korrespondierende Aminosäuren des Donors ersetzt. In machen Fällen enthält der humanisierte Antikörper Aminosäuren, die weder im Rezipient noch im Donor enthalten waren und die während der Optimierung des Antikörpers eingefügt wurden. Bei chimären Antikörpern werden zum Beispiel die variablen Domänen des Donor-Immunoglobulins, oder auch der gesamte Fab Anteil, also VL-CL und VH + CH1 mit den konstanten Regionen eines humanen Antikörpers fusioniert.

Der Begriff Komplementaritäts-bestimmende Region (CDR) wie er hier verwendet wird, bezieht sich auf diejenigen Aminosäuren einer variablen Antikörperdomäne, die für die Bindung an das Antigen notwendig sind. Jede variable Region hat typischerweise drei CDR Regionen, welche als CDR1, CDR2 und CDR3 bezeichnet werden. Jede CDR Region kann Aminosäuren nach der Definition von Kabat und/oder Aminosäuren eines Hypervariablen Loops definiert nach Chotia umfassen. Die Definition nach Kabat umfasst zum Beispiel die Region von ungefähr Aminosäureposition 24 - 34 (CDR1), 50 - 56 (CDR2) und 89 - 97 (CDR3) der variablen leichten Kette und 31 - 35 (CDR1), 50 - 65 (CDR2) und 95 - 102 (CDR3) der variablen schweren Kette (Kabat et al., Sequences of Proteins of Immulological Interest, 5th Ed. Public Health Service, National Institutes of Health, Bethesda, MD. (1991)). Die Definition nach Chotia umfasst zum Beispiel die Region von ungefähr Aminosäureposition 26 - 32 (CDR1), 50 - 52 (CDR2) und 91 96 (CDR3) der variablen leichten Kette und 26 - 32 (CDR1), 53- 55 (CDR2) und 96 - 101 (CDR3) der variablen schweren Kette Chothia and Lesk: J Mol Biol 196: 901-917 (1987)). In manchen Fällen kann eine CDR Aminosäuren aus einer CDR Region definiert nach Kabat und Chotia umfassen.

Abhängig von der Aminosäuresequenz der konstanten Domäne der schweren Kette können Antikörper in verschiedene Klassen eingeteilt werden. Es gibt fünf Hauptklassen von intakten Antikörpern: IgA, IgD, IgE, IgG und IgM, wobei mehrere davon in weitere Unterklassen aufgegliedert werden können. (Isotypen), z.B. IgG1, IgG2, IgG3, IgG4, IgA1 und IgA2. Die konstante Domäne der schweren Kette, die zu den unterschiedlichen Klassen korrespondieren, werden als [alpha/α], [delta/δ], [epsilon/ε], [gamma/γ] und [my/µ] bezeichnet. Sowohl die dreidimensionale Struktur als auch die Untereinheitenstruktur von Antikörpern sind bekannt.

Der Begriff "funktionales Fragment" oder "antigen-bindendes Antikörperfragment" eines Antikörpers/Immunoglobulins ist definiert als ein Fragment eines Anlikörpers/Immunoglobulins (z.B. die variable Domänen eines IgG), welches die Antigen-Bindedomänen des Antikörpers/Immunoglobulins noch umfasst. Die "Antigen-Bindedomäne" eines Antikörpers umfasst typischerweise eine oder mehrere Hypervariable Regionen eines Antikörpers, z.B. die CDR1, CDR2 und/oder CDR3 Region. Allerdings kann auch die "Framework" oder die "Gerüst" Region eines Antikörpers zur Bindung des Antikörpers an das Antigen eine Rolle spielen. Die Framework Region bildet das Gerüst für die CDRs. Vorzugsweise umfasst die Antigen-Bindedomäne mindestens die Aminosäuren 4 bis 103 der variablen leichten Kette und Aminosäure 5 bis 109 der variablen schweren Kette, bevorzugter die Aminosäure 3 bis 107 der variablen leichten Kette und 4 bis 111 der variablen schweren Kette, besonders bevorzugt sind die kompletten variablen leichten und schweren Ketten , also Aminosäure 1 - 109 der VL und 1 bis 113 der VH (Nummerierung nach WO97/08320).

"Funktionale Fragmente" oder "antigen-bindende Antikörperfragmente" der Erfindung umfassen nicht abschliessend Fab, Fab', F(ab')₂ und Fv Fragmente, Diabodies, Single Domain Antibodies (DAbs), linerae Antikörper, Einzelketten Antikörper (single-chain Fv, abgekürzt scFv); und multispezifische, wie z.B. bi- und tri-spezifische, Antikörper, gebildet aus Antikörperfragmenten C. A. K Borrebaeck, editor (1995) Antibody Engineering (Breakthroughs in Molecular Biology), Oxford University Press; R. Kontermann & S. Duebel, editors (2001) Antibody Engineering (Springer Laboratory Manual), Springer Verlag). Andere Antikörper als "multi-spezifische" oder "multi-funktionale" sind solche mit identischen Bindestellen. Multispezifische Antikörper können spezifisch für unterschiedliche Epitope eines Antigens sein oder spezifisch für Epitope von mehr als einem Antigen sein (siehe z.B. WO93/17715; WO 92/08802; WO 91/00360; WO 92/05793; Tutt, et al., 1991, J. Immunol. 147:60 69; U. S. Pat. Nos. 4,474,893; 4,7 14,68 1 ; 4,925,648; 5,573,920; 5,601,8 19; oder Kostelny et al., 1992, J. Immunol. 148: 1547 1553). Ein F(ab')₂ oder Fab Molekül kann so konstruiert werden, dass die Zahl der intermolekularen Disulfid-Interaktionen, die zwischen den Ch1 und den CL Domänen stattfindet, reduziert oder oder komplett verhindert werden kann.

"Funktionale Fragmente" oder "antigen-bindende Antikörperfragmente" können mit einem weiteren Polypeptid oder Protein, welche nicht von einem Antikörper stammen, über ihren Aminoterminus oder Carboxyterminus mittels einer kovalenten Bindung (z.B. einer Peptidverknüpfung) fusioniert werden. Des Weiteren können Antikörper und antigen-bindende Fragmente dahingehend modifiziert werden, dass an definierten Stellen reaktive Cysteine eingeführt werden, um die Kopplung an ein Toxophor zu erleichtern (siehe Junutula et al. Nat Biotechnol. 2008 Aug;26(8):925-32).

Polyklonale Antikörper können durch für den Durchschnittsfachmann bekannte Methoden hergestellt werden. Monoklonale Antiköper können durch für den Durchschnittsfachmann bekannte Methoden hergestellt werden (Köhler und Milstein, Nature, 256, 495-497, 1975). Humane bzw. humanisierte monoklonale Antiköper können durch für den Durchschnittsfachmann bekannte Methoden hergestellt werden (Olsson et al., Meth Enzymol. 92, 3-16 bzw. Cabilly et al US 4,816,567 oder Boss et al US 4,816,397).

Der Durchsehnitisfachmann kennt vielfältige Methoden, um humane Antikörper und deren Fragmente herzustellen, wie beispielsweise mittels transgener Mäuse (N Lonberg und D Huszar, Int Rev Immunol. 1995;13(1):65-93) oder Phage Display Technologien (Clackson et al., Nature. 1991 Aug 15;352(6336):624-8). Antikörper der Erfindung können aus rekombinanten Antikörperbibliotheken erhalten werden, die z.B. auf den Aminosäuresequenzen einer Vielzahl von Antikörpern besteht, die aus einer großen Anzahl gesunder Freiwilliger erstellt wurden. Antikörper können auch mittels bekannter rekombinanter DNS-Technologien hergestellt werden. Die Nukleinsäuresequenz eines Antikörpers kann durch Routinesequenzierung erhalten werden, oder ist aus öffentlich zugänglichen Datenbanken erhältlich.

Ein "isolierter" Antikörper oder Binder wurde von anderen Bestandteilen der Zelle gereinigt. Kontaminierende Bestandteile einer Zelle, welche mit einer diagnostischen oder therapeutischen Verwendung interferieren können, sind z.B.Enzyme, Hormone, oder andere peptidische- oder nicht-peptidische Bestandteile einer Zelle. Bevorzugt ist ein Antikörper oder Binder, der zu mehr ais 95 Gew-% bezogen auf den Antikörper bzw. Binder aufgereinigt wurde (bestimmt durch z.B. Lowry Verfahren, UV-Vis Spektroskopie oder durch SDS-Kapillargelelektrophorese), der soweit aufgereinigt wurde, dass mindestens 15 Aminosäuren des Aminoterminus oder einer internen Aminosäuresequenz bestimmt werden können, oder zur Homogenität aufgereinigt wurde, wobei die Homogenität bestimmt wird durch SDS-PAGE unter reduzierenden oder nicht-reduzierenden Bedingungen (die Detektion kann mittels Coomassie Blau Anfärbung oder bevorzugt durch Silberfärbung bestimmt werden). Jedoch wird ein Antikörper normalerweise durch einen oder mehrere Reinigungsschritte hergestellt.

Der Begriff "spezifische Bindung" oder "bindet spezifisch" bezicht sich auf einen Antikörper oder Binder, der an ein vorbestimmtes Antigen/Zielmolekül bindet. Spezifische Bindung eines Antikörpers oder Binders beschreibt typischerweise einen Antikörper bzw. Binder mit einer Affinität von mindestens 10⁻⁷ M (als Kd-Wert; also vorzugsweise solche mit kleineren Kd-Werten als 10⁻⁷ M), wobei der Antikörper bzw. Binder eine mindestens zweifach höhere Affinität zum vorbestimmten Antigen/Zielmolekül als zu einem nicht-spezifischen Antigen/Zielmolekül hat (z.B. Rinder Serumalbumin, oder Casein), welches nicht das vorbestimmte Antigen/Zielmolekül oder ein eng verwandtes Anligen/Zielmolekül ist.

Antikörper, welche spezifisch gegen ein Krebszell-Antigen sind, können vom Durchschnittsfachmann mittels ihm bekannter Verfahren hergestellt werden (wie z.B. rekombinante Expression) oder kommerziell erworben werden (wie z.B. von Merck KGaA, Deustchland). Beispiele bekannter kommerziell erhältlicher Antikörper in der Krebstherapie sind Erbitux® (Cetuximab, Merck KGaA), Avastin® (Bevacizumab, Roche) und Herceptin®

(Trastuzumab, Genentech). Trastuzumab ist ein rekombinanter humanisierter monokloaler Antikörper vom IgG1kappa Typ, welcher mit hoher Affinität in einem Zell-basierten Assay (Kd = 5 nM) die extrazelluläre Domäne des humanen epidermalen Wachstumsrezeptors bindet. Der Antikörper wird rekombinant in CHO-Zellen hergestellt.

Die Verbindungen der Formel (I) stellen eine Untergruppe der Verbindungen der Formel (Ia) dar.

Bevorzugter Gegenstand der Erfindung sind Binder-Wirkstoff Konjugate der allgemeinen Formel (Ia), in welcher
- n: für eine Zahl von 1 bis 50 steht,
- AK: für AK₁ oder AK₂ steht
wobei
AK₁ für einen Binder (vorzugsweise einen anti-C4.4a-Antikörper), der über ein Schwefelatom des Binders an die Gruppe G gebunden ist,
AK₂ für einen Binder (vorzugsweise einen anti-C4.4a-Antikörper), der über ein Stickstoffatom des Binders an die Gruppe G gebunden ist,
- G: für den Fall, dass AK = AK₁ ist, für eine Gruppe der Formel 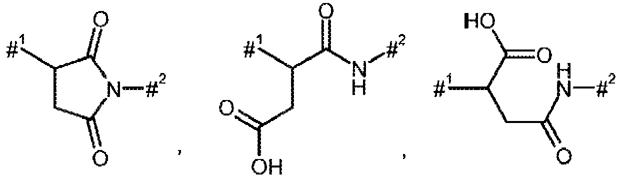 oder 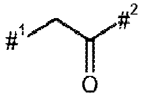 steht, wobei
#¹ die Verknüpfungsstelle mit dem Schwefelatom des Binders kennzeichnet,
#² die Verknüpfungsstelle mit der Gruppe L¹ kennzeichnet, oder
für den Fall, dass AK = AK₂ ist, für Carbonyl steht,
- L¹: für eine Bindung, lineares (C₁-C₁₀)-Alkandiyl, eine Gruppe der Formel 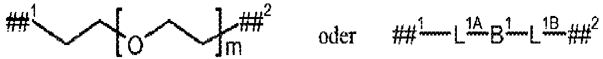 steht, wobei
m für eine Zahl von 2 bis 6 steht,
##¹ die Verknüpfungsstelle mit der Gruppe G kennzeichnet,
##² die Verknüpfungsstelle mit der Gruppe B kennzeichnet,
L^{1A} für lineares (C₂-C₁₀)-Alkandiyl steht,
B¹ für eine Gruppe der Formel 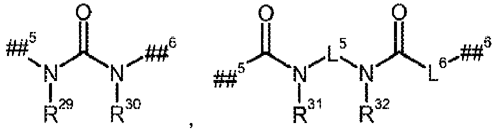 oder 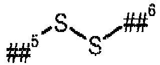 steht, worin
##⁵ die Verknüpfungsstelle mit der Gruppe L^{1A} kennzeichnet,
##⁶ die Verknüpfungsstelle mit der Gruppe L^{1B} kennzeichnet,
L⁵ für eine Bindung oder (C₂-C₄)-Alkandiyl steht,
L⁶ für eine Bindung oder eine Gruppe der Formel 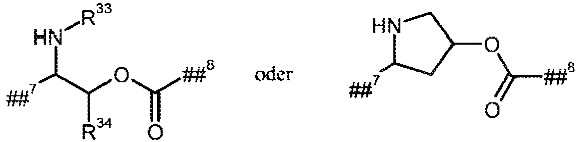 steht, worin
##⁷ die Verknüpfungsstelle mit der Carbonylgruppe kennzeichnet,
##⁸ die Verknüpfungsstelle mit L^{1B} kennzeichnet,
R³³ für Wasserstoff, (C₁-C₄)-Alkylcarbonyl, tert.-Butyloxycarbonyl oder Benzyloxycarbonyl steht,
R³⁴ für Wasserstoff oder Methyl steht,
R²⁹ für Wasserstoff oder (C₁-C₄)-Alkyl steht,
R³⁰ für Wasserstoff oder (C₁-C₄)-Alkyl steht,
oder
R²⁹ und R³⁰ zusammen mit den Atomen, an die sie gebunden sind, einen 5- oder 6-gliedrigen Heterocyclus bilden,
R³¹ für Wasserstoff oder (C₁-C₄)-Alkyl steht,
R³² für Wasserstoff oder (C₁-C₄)-Alkyl steht, oder
R³¹ und R³² zusammen mit den Atomen, an die sie gebunden sind, einen 5- oder 6-gliedrigen Heterocyclus bilden,
L^{1B} für lineares (C₂-C₁₀-Alkandiyl steht,
und
wobei (C₁-C₁₀)-Alkandiyl mit 1 bis 4 Substituenten unabhängig voneinander ausgewählt aus der Gruppe Methyl, Hydroxy und Benzyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
- B: für eine Bindung oder eine Gruppe der Formel 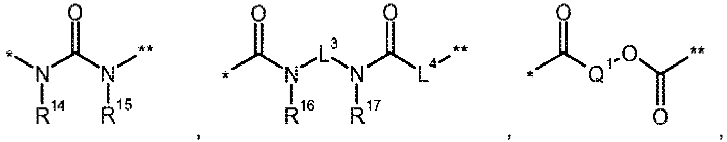 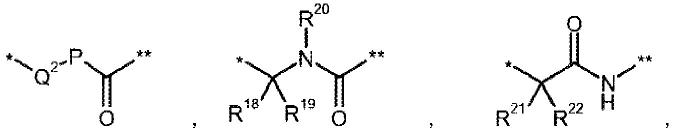 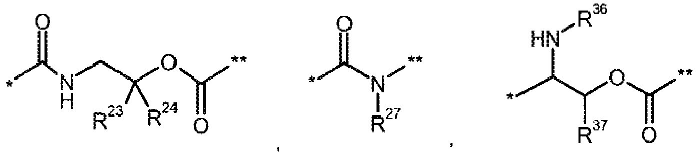 oder 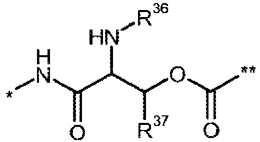 steht, wobei
* die Verknüpfungsstelle mit L¹ kennzeichnet,
** die Verknüpfungsstelle mit L² kennzeichnet,
P für O oder NH steht.
L³ für eine Bindung oder (C₂-C₄₎-Alkandiyl steht,
L⁴ für eine Bindung oder eine Gruppe der Formel 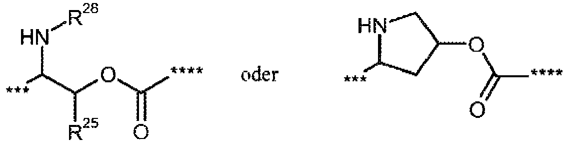 steht, worin
*** die Verknüpfungsstelle mit der Carbonylgruppe kennzeichnet,
**** die Verknüpfungsstelle mit L² kennzeichnet,
R²⁵ für Wasserstoff oder Methyl steht,
R²⁸ für Wasserstoff, (C₁-C₄)-Alkylcarbonyl, tert.-Butyloxycarbonyl oder Benzyloxycarbonyl steht,
Q¹ für einen 4- bis 7-gliedrigen Heterocyclus steht,
Q² für einen 3- bis 7-gliedrigen Carbocyclus oder einen 4- bis 7-gliedrigen Heterocyclus steht,
**R**¹⁴ für Wasserstoff oder (C₁-C₄)-Alkyl steht,
**R**¹⁵ für Wasserstoff oder (C₁-C₄)-Alky) steht,
oder
**R**¹⁴ und R¹⁵ zusammen mit den Atomen, an die sie gebunden sind, einen 5- oder 6-gliedrigen Heterocyclus bilden,
**R**¹⁶ für Wasserstoff oder (C₁-C₄)-Alkyl steht,
**R**¹⁷ für Wasserstoff oder (C₁-C₄)-Alkyl steht,
oder
**R**¹⁶ und R¹⁷ zusammen mit den Atomen, an die sie gebunden sind, einen 5- oder 6-gliedrigen Heterocyclus bilden,
**R**¹⁸ für Wasserstoff oder (C₁-C₄)-Alkyl steht,
**R**¹⁹ für Wasserstoff oder die Seitengruppe einer natürlichen α-Aminosäure oder ihrer Homologe oder Isomere steht,
**R**²⁰ für Wasserstoff oder (C₁-C₄)-Alkyl steht,
oder
**R**¹⁹ und R²⁰ zusammen mit den Atomen, an die sie gebunden sind, einen Pyrrolidinylring bilden,
**R**²¹ für Wasserstoff oder (C₁-C₄)-Alkyl steht,
**R**²² für Wasserstoff oder (C₁-C₄)-Alkyl steht,
oder
**R**²¹ und R²² zusammen mit den Atomen, an die sie gebunden sind, einen 3- bis 7-gliedrigen Carbocyclus bilden,
**R**²³ für (C₁-C₄)-Alkyl steht,
**R**²⁴ für Wasserstoff oder (C₁-C₄)-Alkyl steht,
**R**²⁷ für Wasserstoff oder (C₁-C₄)-Alkyl steht,
**R**³⁶ für Wasserstoff, (C₁-C₄)-Alkylcarbonyl, tert.-Butyloxycarbonyl oder Benzyloxy carbonyl steht,
**R**³⁷ für Wasserstoff oder Methyl steht,
oder
**R**³⁶ und R³⁷ bilden zusammen mit den Atomen, an die sie gebunden sind, einen Pyrrolidinring,
- L²: für lineares (C₂-C₁₀)-Alkandiyl oder für eine Gruppe der Formel 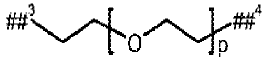 steht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
wobei (C₂-C₁₀)-Alkandiyl mit 1 bis 4 Substituenten unabhängig voneinander ausgewählt aus der Gruppe Methyl, Hydroxy und Benzyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
- D: für eine Gruppe der Formel 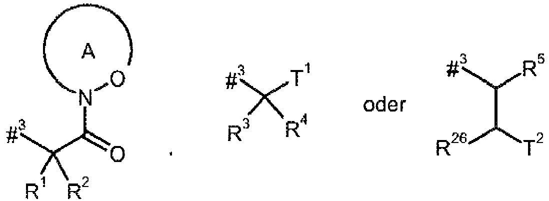 steht, wobei
#³ die Verknüpfungsstelle mit dem Stickstoffatom kennzeichnet,
**R**¹ für Wasserstoff oder Methyl steht,
**R**² für Isopropyl, Isobutyl, *sec.*-Butyl, *tert.*-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenyl-ethyl, Diphenylmethyl, 1*H*-Imidazol-4-ylmethyl oder 1*H*-Indol-3-ylmethyl steht,
oder
**R**¹ und R² zusammen mit dem Kohlenstoffatom, an das sie gebunden sind, eine (1*S*,2*R*)-2-Phenylcyclopropan-1,1-diyl-Gruppe der Formel 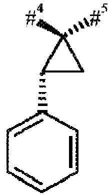 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 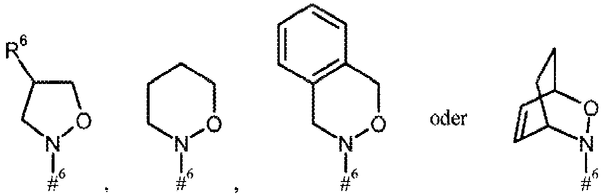 steht, worin
#⁶ die Verknüpfungsstelle mit der Carbonyl-Gruppe kennzeichnet,
R⁶ für Wasserstoff, Hydroxy oder Benzyloxy steht,
**R**³ für Wasserstoff oder Methyl steht,
**R**⁴ für Isopropyl, Isobutyl, *sec.-*Butyl, *tert.*-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenyl-ethyl, Diphenylmethyl, 1H-Imidazol-4-ylmethyl oder 1*H*-Indol-3-ylmethyl steht,
oder
**R**³ und R⁴ zusammen mit dem Kohlenstoffatom, an das sie gebunden sind, eine (1*S*,2*R*)-2-Phenylcyclopropan-1,1-diyl-Gruppe der Formel 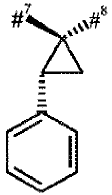 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹, -C(=O)-NH-NH-R¹⁰ oder -CH₂-O-R¹¹ steht,
worin
R⁷ für Wasserstoff, Methyl, Ethyl, *n*-Propyl, *tert*.-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
oder
R⁸ und R⁹ bilden zusammen mit dem Stickstoffatom, an das sie gebunden sind, einen 4- bis 7-gliedrigen Heterocyclus,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der Formel 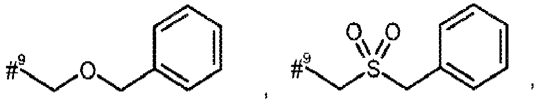 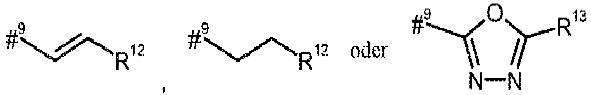 steht, worin
#⁹ die Verknüpfüngsstelle mit -CHC(R²⁶)-T² kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
R²⁶ für Wasserstoff oder Hydroxy steht,
T² für Phenyl, Benzyl, 1*H*-Indol-3-yl oder 1*H*-Indol-3-ylmethyl steht,
- R³⁵: für Methyl oder Hydroxy steht,
sowie ihre Salze, Solvate und Solvate der Salze.

Bevorzugter Gegenstand der vorliegenden Erfindung sind Binder-Wirkstoff Konjugate der allgemeinen Formel (Ia) wie oben angegeben, in welcher
- n: für eine Zahl von 1 bis 50 steht,
- AK: für AK₁ oder AK₂ steht
wobei
AK₁ für einen Binder (vorzugsweise einen anti-C4.4a-Antikörper), der über ein Schwefelatom des Binders an die Gruppe G gebunden ist,
AK₂ für einen Binder (vorzugsweise einen anti-C4.4a-Antikörper), der über ein Stickstoffatom des Binders an die Gruppe G gebunden ist,
- G: für den Fall, dass AK = AK₁ ist, für eine Gruppe der Formel 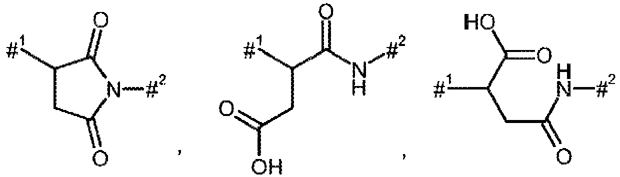 oder 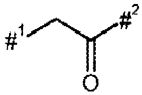 steht, wobei
#¹ die Verknüpfungsstelle mit dem Schwefelatom des Binders kennzeichnet,
#² die Verknüpfungsstelle mit der Gruppe L¹ kennzeichnet,
oder
für den Fall, dass AK = AK₂ ist, für Carbonyl steht,
- L¹: für eine Bindung, lineares (C₁-C₁₀)-Alkandiyl, eine Gruppe der Formel 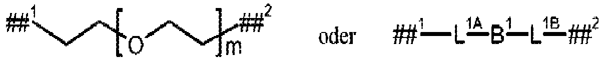 steht, wobei
m für eine Zahl von 2 bis 6 steht,
##¹ die Verknüpfungsstelle mit der Gruppe G kennzeichnet,
##² die Verknüpfungsstelle mit der Gruppe B kennzeichnet,
L^{1A} für lineares (C₂-C₁₀)-Alkandiyl steht,
B¹ für eine Gruppe der Formel 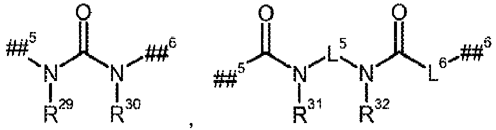 oder 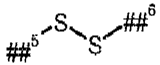 steht, worin
##⁵ die Verknüpfungsstelle mit der Gruppe L^{1A} kennzeichnet,
##⁶ die Verknüpfungsstelle mit der Gruppe L^{1B} kennzeichnet,
L⁵ für eine Bindung oder (C₂-C₄)-Alkandiyl steht,
L⁶ für eine Bindung oder eine Gruppe der Formel 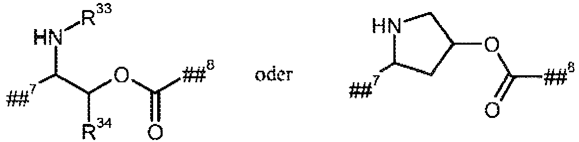 steht, worin
##⁷ die Verknüpfungsstelle mit der Carbonylgruppe kennzeichnet,
##⁸ die Verknüpfungsstelle mit L^{1B} kennzeichnet,
R³³ für Wasserstoff, (C₁-C₄)-Alkylcarbonyl, tert.-Butyloxycarbonyl oder Benzyloxycarbonyl steht,
R³⁴ für Wasserstoff oder Methyl steht,
R²⁹ für Wasserstoff oder (C₁-C₄)-Alkyl steht,
R³⁰ für Wasserstoff oder (C₁-C₄)-Alkyl steht,
oder
R²⁹ und R³⁰ zusammen mit den Atomen, an die sie gebunden sind, einen 5- oder 6-gliedrigen Heterocyclus bilden,
R³¹ für Wasserstoff oder (C₁-C₄)-Alkyl steht,
R³² für Wasserstoff oder (C₁-C₄)-Alkyl steht,
oder
R³¹ und R³² zusammen mit den Atomen, an die sie gebunden sind, einen 5- oder 6-gliedrigen Heterocyclus bilden,
**L**^{1B} für lineares (C₂-C₁₀)-Alkandiyl steht,
und
wobei (C₁-C₁₀)-Alkandiyl mit 1 bis 4 Substituenten unabhängig voneinander ausgewählt aus der Gruppe Methyl, Hydroxy und Benzyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
- B: für eine Bindung oder eine Gruppe der Formel 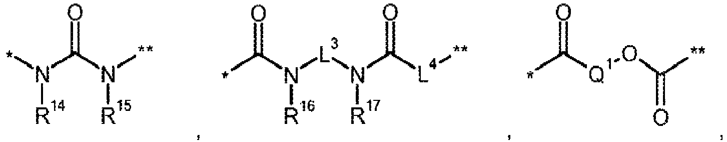 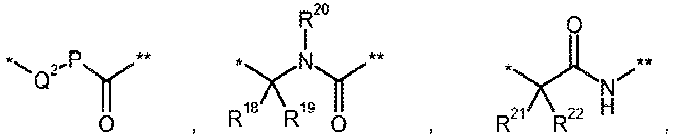 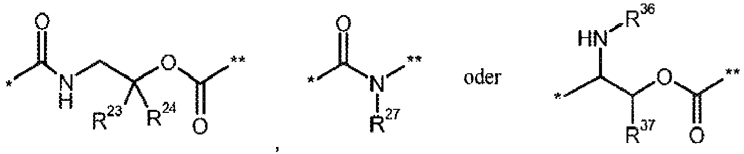 steht, wobei
* die Verknüpfungsstelle mit L¹ kennzeichnet,
** die Verknüpfungsstelle mit L² kennzeichnet,
P für O oder NH steht,
L³ für eine Bindung oder (C₂-C₄)-Alkandiyl steht,
L⁴ für eine Bindung oder eine Gruppe der Formel 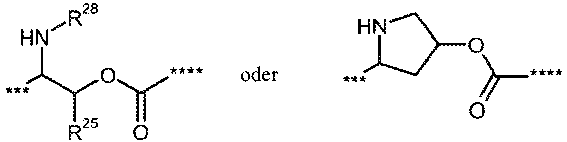 steht, worin
*** die Verknüpfungsstelle mit der Carbonylgruppe kennzeichnet,
**** die Verknüpfungsstelle mit L² kennzeichnet,
R²⁵ für Wasserstoff oder Methyl steht,
R²⁸ für Wasserstoff, (C₁-C₄)-Alkylcarbonyl, tert.-Butyloxycarbonyl oder Benzyloxycarbonyl steht,
Q¹ für einen 4- bis 7-gliedrigen Heterocyclus steht,
Q² für einen 3- bis 7-gliedrigen Carbocyclus oder einen 4- bis 7-gliedrigen Heterocyclus steht,
R¹⁴ für Wasserstoff oder (C₁-C₄)-Alkyl steht,
R¹⁵ für Wasserstoff oder (C₁-C₄)-Alkyl steht,
oder
R¹⁴ und R¹⁵ zusammen mit den Atomen, an die sie gebunden sind, einen 5- oder 6-gliedrigen Heterocyclus bilden,
R¹⁶ für Wasserstoff oder (C₁-C₄)-Alkyl steht,
R¹⁷ für Wasserstoff oder (C₁-C₄)-Alkyl steht,
oder
**R**¹⁶ und R¹⁷ zusammen mit den Atomen, an die sie gebunden sind, einen 5- oder 6-gliedrigen Heterocyclus bilden,
**R**¹⁸ für Wasserstoff oder (C₁-C₄)-Alkyl steht,
**R**¹⁹ für Wasserstoff oder die Seitengruppe einer natürlichen α-Aminosäure oder ihrer Homologe oder Isomere steht,
**R**²⁰ für Wasserstoff oder (C₁-C₄)-Alkyl steht,
oder
**R**¹⁹ und R²⁰ zusammen mit den Atomen, an die sie gebunden sind, einen Pyrrolidinylring bilden,
**R**²¹ für Wasserstoff oder (C₁-C₄)-Alkyl steht,
**R**²² für Wasserstoff oder (C₁-C₄)-Alkyl steht,
oder
**R**²¹ und R²² zusammen mit den Atomen, an die sie gebunden sind, einen 3- bis 7-gliedrigen Carbocyclus bilden,
**R**²³ für (C₁-C₄)-Alkyl steht,
**R**²⁴ für Wasserstoff oder (C₁-C₄)-Alkyl steht,
**R**²⁷ für Wasserstoff oder (C₁-C₄)-Alkyl steht,
**R**³⁶ für Wasserstoff, (C₁-C₄)-Alkylcarbonyl, tert.-Butyloxycarbonyl oder Benzyloxy carbonyl steht,
**R**³⁷ für Wasserstoff oder Methyl steht,
oder
**R**³⁶ und R³⁷ bilden zusammen mit den Atomen, an die sie gebunden sind, einen Pyrrolidinring,
- L²: für lineares (C₂-C₁₀)-Alkandiyl oder für eine Gruppe der Formel 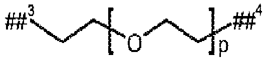 steht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
wobei (C₂-C₁₀)-Alkandiyl mit 1 bis 4 Substituenten unabhängig voneinander ausgewählt aus der Gruppe Methyl, Hydroxy und Benzyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
- D: für eine Gruppe der Formel 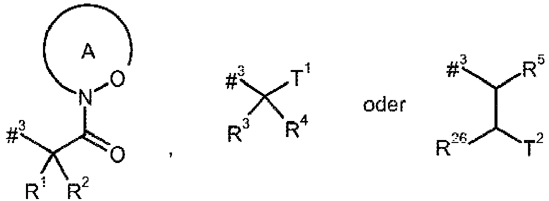 steht, wobei
#³ die Verknüpfungsstelle mit dem Stickstoffatom kennzeichnet,
R¹ für Wasserstoff oder Methyl steht,
**R**² für Isopropyl, Isobutyl, *sec*.-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenyl-ethyl, Diphenylmethyl, 1*H*-Imidazol-4-ylmethyl oder 1*H*-Indol-3-ylmethyl steht,
oder
**R**¹ und R² zusammen mit dem Kohlenstoffatom, an das sie gebunden sind, eine (1*S*,2*R*)-2-Phenylcyclopropan-1,1-diyl-Gruppe der Formel 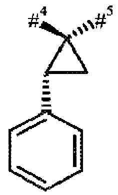 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 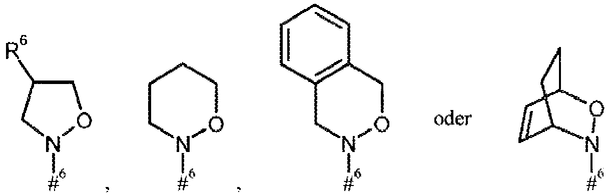 steht, worin
#⁶ die Verknüpfiingsstelle mit der Carbonyl-Gruppe kennzeichnet,
R⁶ für Wasserstoff, Hydroxy oder Benzyloxy steht,
R³ für Wasserstoff oder Methyl steht,
R⁴ für Isopropyl, Isobutyl, *sec*.-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenyl-ethyl, Diphenylmethyl, 1*H*-Imidazol-4-ylmethyl oder 1*H*-Indol-3-ylmethyl steht,
oder
R³ und R⁴ zusammen mit dem Kohlenstoffatom, an das sie gebunden sind, eine (1*S*,2*R*)-2-Phenyleyclopropan-1,1-diyl-Gruppe der Formel 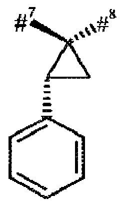 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹, -C(=O)-NH-NH-R¹⁰ oder -CH₂-O-R¹¹ steht,
worin
R⁷ für Wasserstoff, Methyl, Ethyl, *n*-Propyl, *tert*.-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
oder
R⁸ und R⁹ bilden zusammen mit dem Stickstoffatom, an das sie gebunden sind, einen 4- bis 7-gliedrigen Heterocyclus,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der Formel 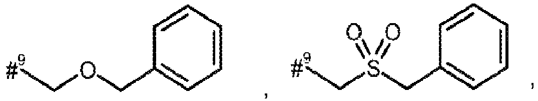 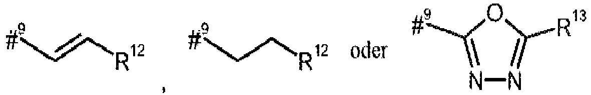 steht, worin
#⁹ die Verknüpfungsstelle mit -CHC(R²⁶)-T² kennzeichnet,
**R**¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
R²⁶ für Wasserstoff oder Hydroxy steht,
T² für Phenyl, Benzyl, 1*H-*Indol-3-yl oder 1*H*-Indol-3-ylmethyl steht,
- R³⁵: für Methyl oder Hydroxy steht,
sowie ihre Salze, Solvate und Solvate der Salze.

Bevorzugter Gegenstand der Erfindung sind Binder-Wirkstoff Konjugate der allgemeinen Formel (Ia), in welcher
- n: für eine Zahl von 1 bis 20 steht,
- AK: für AK₁ oder AK₂ steht
wobei
AK₁ für einen Antikörper oder ein Antigen-bindendes Antikörperfragment steht, der bzw. das an C4.4a bindet und über das Schwefelatom eines Cystein-Restes des Binders an die Gruppe G gebunden ist,
AK₂ für einen Antikörper oder ein Antigen-bindendes Antikörperfragment steht, der bzw. das an C4.4a bindet und über die NH-Seitengruppe eines Lysin-Restes des Binders an die Gruppe G gebunden ist,
- G: für den Fall, dass AK = AK₁ ist, für eine Gruppe der Formel 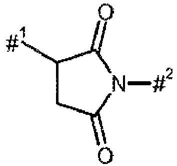 steht, wobei
#¹ die Verknüpfungsstelle mit dem Cystein-Rest des Binders kennzeichnet,
#² die Verknüpfungsstelle mit der Gruppe L¹ kennzeichnet,
oder
für den Fall, dass AK = AK₂ ist, für Carbonyl steht,
- L¹: für eine Bindung, lineares (C₂-C₆)-Alkandiyl, eine Gruppe der Formel 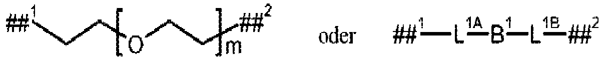 steht, wobei
in für eine Zahl von 2 bis 6 steht,
##¹ die Verknüpfungsstelle mit der Gruppe G kennzeichnet,
##² die Verknüpfungsstelle mit der Gruppe B kennzeichnet,
L^{1A} für lineares (C₂-C₆)-Alkandiyl steht,
B¹ für eine Gruppe der Formel 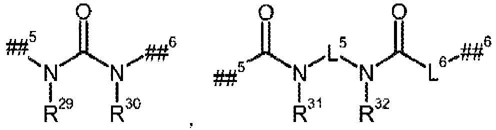 oder 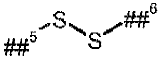 steht, worin
##⁵ die Verknüpfungsstelle mit der Gruppe L^{1A} kennzeichnet,
##⁶ die Verknüpfungsstelle mit der Gruppe L^{1B} kennzeichnet,
L⁵ für eine Bindung steht,
L⁶ für eine Bindung oder eine Gruppe der Formel 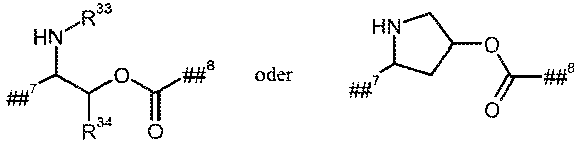 steht, worin
##⁷ die Verknüpfungsstelle mit der Carbonylgruppe kennzeichnet,
##⁸ die Verknüpfungsstelle mit L^{1B} kennzeichnet,
R³³ für Wasserstoff, Methylcarbonyl oder tert.-Butyloxycarbonyl steht,
R³⁴ für Wasserstoff oder Methyl steht,
R²⁹ für Wasserstoff steht,
R³⁰ für Wasserstoff steht,
R³¹ für Wasserstoff oder Methyl steht,
R³² für Wasserstoff oder Methyl steht,
L^{1B} für lineares (C₂-C₆)-Alkandiyl steht,
und
wobei (C₂-C₆)-Alkandiyl mit 1 oder 2 Substituenten Methyl substiuiert sein kann,
- B: für eine Bindung oder eine Gruppe der Formel 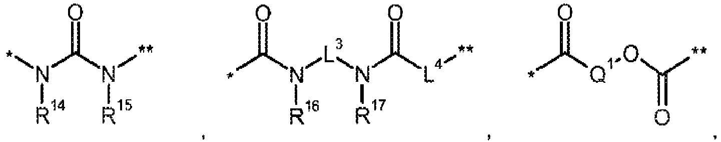 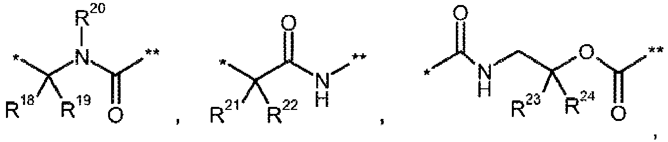 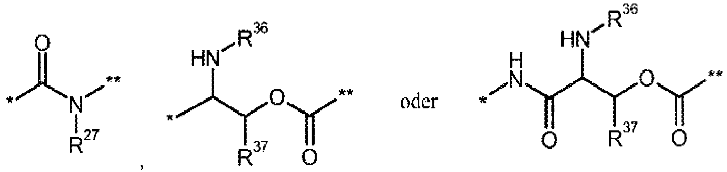 steht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Bindung oder eine Gruppe der Formel 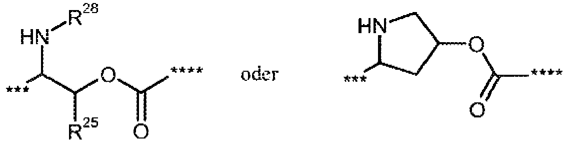 steht, worin
*** die Verknüpfungsstelle mit der Carbonylgruppe kennzeichnet.
**** die Verknüpfungsstelle mit L² kennzeichnet,
R²⁵ für Wasserstoff oder Methyl steht,
R²⁸ für Wasserstoff, Methylcarbonyl oder tert.-Butyloxycarbonyl steht,
Q¹ für einen 4- bis 7-gliedrigen Heterocyclus steht,
R¹⁴ für Wasserstoff steht,
R¹⁵ für Wasserstoff steht,
R¹⁶ für Wasserstoff oder Methyl steht,
R¹⁷ für Wasserstoff oder Methyl steht,
oder
R¹⁶ und R¹⁷ zusammen mit den Atomen, an die sie gebunden sind, einen Piperazinylring bilden,
R¹⁸ für Wasserstoff steht,
R¹⁹ für Wasserstoff, Methyl, Propan-2-yl , 2-Methylpropan-1-yl oder 1-Methylpropan-1-yl steht,
R²⁰ für Wasserstoff oder Methyl steht,
oder
R¹⁹ und R²⁰ zusammen mit den Atomen, an die sie gebunden sind, einen Pyrrolidinylring bilden,
R²¹ für Wasserstoff oder Methyl steht,
R²² für Wasserstoff oder Methyl steht,
oder
R²¹ und R²² zusammen mit den Atomen, an die sie gebunden sind, einen Cyclopropylring bilden,
R²³ für Methyl steht,
R²⁴ für Wasserstoff oder Methyl steht,
R²⁷ für Wasserstoff steht,
R³⁶ für Wasserstoff, Methylcarbonyl oder tert.-Butyloxycarbonyl steht,
R³⁷ für Wasserstoff oder Methyl steht,
oder
R³⁶ und R³⁷ bilden zusammen mit den Atomen, an die sie gebunden sind, einen Pyrrolidinring,
- L²: für lineares (C₂-C₆)-Alkandiyl oder für eine Gruppe der Formel 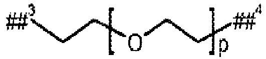 steht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
wobei (C₂-C₁₀)-Alkandiyl mit 1 oder 2 Substituenten Methyl substiuiert sein kann,
- D: für eine Gruppe der Formel 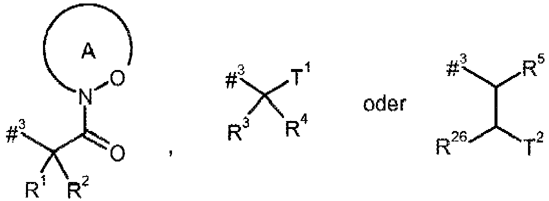 steht, wobei
#³ die Verknüpfungsstelle mit dem Stickstoffatom kennzeichnet.
R¹ für Wasserstoff steht,
R² für 1-Hydroxyethyl, Benzyl, 4-Hydroxybenzyl, 1-Phenylethyl oder 1*H*-Indol-3-yl-methyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 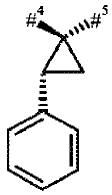 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 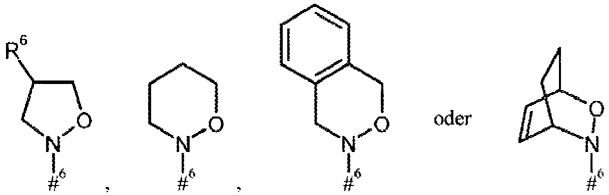 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff steht,
R⁴ für 1-Hydroxyethyl, Benzyl, 4-Hydroxybenzyl, 1-Phenylethyl oder 1*H*-Indol-3-yl-methyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 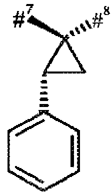 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹, -C(=O)-NH-NH-R¹⁰ oder -CH₂-O-R¹¹ steht,
worin
R⁷ für Wasserstoff, Methyl, Ethyl, *n*-Propyl, *tert*.-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
oder
R⁸ und R⁹ bilden zusammen mit dem Stickstoffatom, an das sie gebunden sind, einen 4- bis 7-gliedrigen Heterocyclus,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der Formel 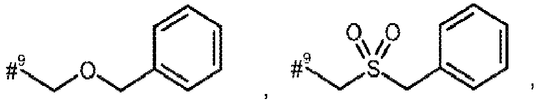 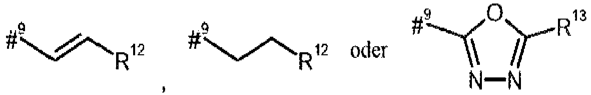 steht, worin
#⁹ die Verknüpfungsstelle mit -CHC(R²⁶)-T² kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
R²⁶ für Wasserstoff oder Hydroxy steht,
T² für Phenyl, Benzyl, 1*H-*Indol-3-yl oder 1*H*-Indol-3-ylmethyl steht,
- R³⁵: für Methyl oder Hydroxy steht,
sowie ihre Salze, Solvate und Solvate der Salze.

Bevorzugter Gegenstand der vorliegenden Erfindung sind Binder-Wirkstoff Konjugate der allgemeinen Formel (Ia) wie oben angegeben, in welcher
- n: für eine Zahl von 1 bis 20 steht,
- AK: für AK₁ oder AK₂ steht
wobei
AK₁ für einen Antikörper oder ein Antigen-bindendes Antikörperfragment steht, der bzw. das an C4.4a bindet und über das Schwefelatom eines Cystein-Restes des Binders an die Gruppe G gebunden ist,
AK₂ für einen Antikörper oder ein Antigen-bindendes Anukörperfragment steht, der bzw. das an C4.4a bindet und über die NH-Seitengruppe eines Lysin-Restes des Binders an die Gruppe G gebunden ist,
- G: für den Fall, dass AK = AK₁ ist, für eine Gruppe der 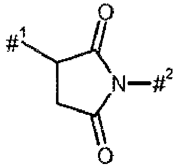 steht, wobei
#¹ die Verknüpfungsstelle mit dem Cystein-Rest des Binders kennzeichnet,
#² die Verknüpfungsstelle mit der Gruppe L¹ kennzeichnet,
oder
für den Fall, dass AK = AK₂ ist, für Carbonyl steht,
L¹ für eine Bindung, lineares (C₂-C₆)-Alkandiyl, eine Gruppe der 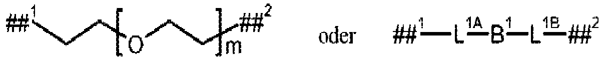 steht, wobei
m für eine Zahl von 2 bis 6 steht,
##¹ die Verknüpfungsstelle mit der Gruppe G kennzeichnet,
##² die Verknüpfungsstelle mit der Gruppe B kennzeichnet,
L^{1A} für lineares (C₂-C₆)-Alkandiyl steht,
B¹ für eine Gruppe der 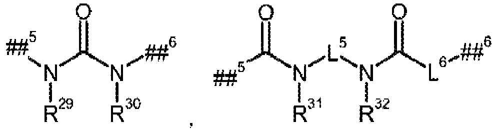 oder 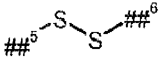 steht, worin
##⁵ die Verknüpfungsstelle mit der Gruppe L^{1A} kennzeichnet,
##⁶ die Verknüpfungsstelle mit der Gruppe L^{1B} kennzeichnet,
L⁵ für eine Bindung steht,
L⁶ für eine Bindung oder eine Gruppe der Formel 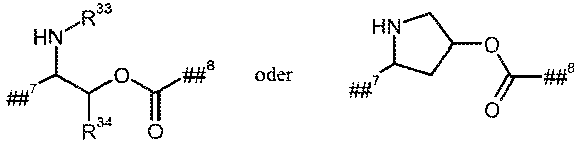 steht, worin
##⁷ die Verknüpfungsstelle mit der Carbonylgruppe kennzeichnet,
##⁸ die Verknüpfungsstelle mit L^{1B} kennzeichnet,
R³³ für Wasserstoff, Methylcarbonyl oder tert.-Butyloxycarbonyl steht,
R³⁴ für Wasserstoff oder Methyl steht,
R²⁹ für Wasserstoff steht,
R³⁰ für Wasserstoff steht,
R³¹ für Wasserstoff oder Methyl steht,
R³² für Wasserstoff oder Methyl steht,
L^{1B} für lineares (C₂-C₆)-Alkandiyl steht,
und
wobei (C₂-C₆)-Alkandiyl mit 1 oder 2 Substituenten Methyl substiuiert sein kann,
B für eine Bindung oder eine Gruppe der Formel 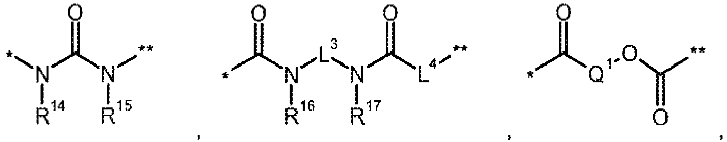 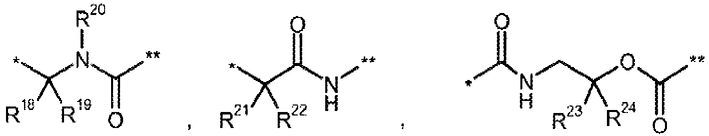 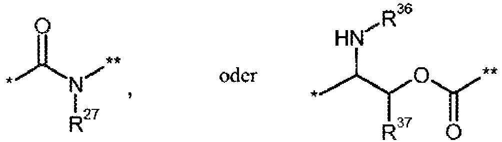 steht, wobei
* die Verknüpfungsstelle mit L¹ kennzeichnet,
** die Verknüpfüngsstelle mit L² kennzeichnet,
L³ für eine Bindung oder Ethan-1,2-diyl steht,
L⁴ für eine Bindung oder eine Gruppe der Formel 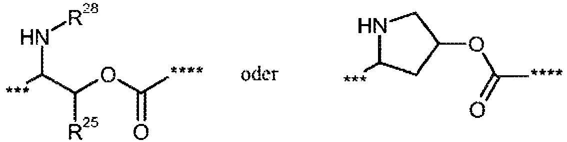 steht, worin
*** die Verknüpfungsstelle mit der Carbonylgruppe kennzeichnet,
**** die Verknüpfungsstelle mit L² kennzeichnet,
R²⁵ für Wasserstoff oder Methyl steht,
R²⁸ für Wasserstoff, Methylcarbonyl oder tert.-Butyloxycarbonyl steht,
Q¹ für einen 4- bis 7-gliedrigen Heterocyclus steht,
R¹¹ für Wasserstoff steht,
R¹⁵ für Wasserstoff steht,
R¹⁶ für Wasserstoff oder Methyl steht,
R¹⁷ für Wasserstoff oder Methyl steht,
oder
R¹⁶ und R¹⁷ zusammen mit den Atomen, an die sie gebunden sind, einen Piperazinylring bilden,
R¹⁸ für Wasserstoff sieht,
R¹⁹ für Wasserstoff, Methyl, Propan-2-yl , 2-Methylpropan-1-yl oder 1-Methylpropan-1-yl steht,
R²⁰ für Wasserstoff oder Methyl steht,
oder
R¹⁹ und R²⁰ zusammen mit den Atomen, an die sie gebunden sind, einen Pyrrolidinylring bilden,
R²¹ für Wasserstoff oder Methyl steht,
R²² für Wasserstoff oder Methyl steht,
oder
R²¹ und R²² zusammen mit den Atomen, an die sie gebunden sind, einen Cyclopropylring bilden,
R²³ für Methyl steht,
R²⁴ für Wasserstoff oder Methyl steht,
R²⁷ für Wasserstoff steht,
R³⁶ für Wasserstoff, (C₁-C₄)-Alkylcarbonyl, tert.-Butyloxycarbonyl oder Benzyloxy carbonyl steht,
R³⁷ für Wasserstoff oder Methyl steht,
L² für lineares (C₂-C₆)-Alkandiyl oder für eine Gruppe der Formel 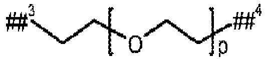 sieht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
wobei (C₂-C₁₀)-Alkandiyl mit 1 oder 2 Substituenten Methyl substiuiert sein kann,
D für eine Gruppe der 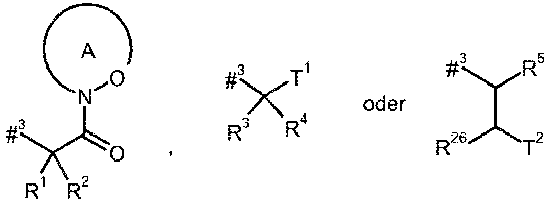 steht, wobei
#³ die Verknüpfungsstelle mit dem Stickstoffatom kennzeichnet,
R¹ für Wasserstoff steht,
R² für 1-Hydroxyethyl, Benzyl, 4-Hydroxybenzyl, 1-Phenylethyl oder 1*H*-Indol-3-yl-methyl steht, oder
R¹ und R² zusammen mit dem Kohlenstoffatom, an das sie gebunden sind, eine (1*S*,2*R*)-2-Phenylcyclopropan-1,1-diyl-Gruppe der Formel 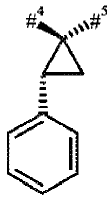 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der 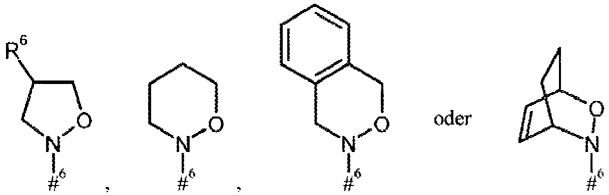 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff steht,
R⁴ für 1-Hydroxyethyl, Benzyl, 4-Hydroxybenzyl, 1-Phenylethyl oder 1*H*-Indol-3-yl-methyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 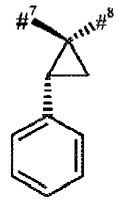 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹, -C(=O)-NH-NH-R¹⁰ oder -CH₂-O-R¹¹ steht,
worin
R⁷ für Wasserstoff, Methyl, Ethyl, *n*-Propyl, *tert*.-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
oder
R⁸ und R⁹ bilden zusammen mit dem Stickstoffatom, an das sie gebunden sind, einen 4- bis 7-gliedrigen Heterocyclus,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der 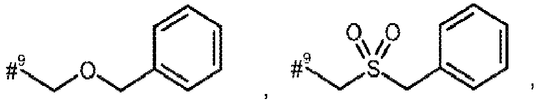 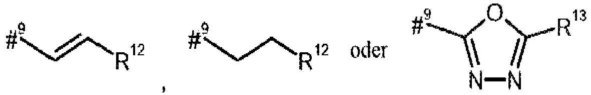 steht, worin
#⁹ die Verknüpfungsstelle mit -CHC(R²⁶)-T² kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
R²⁶ für Wasserstoff oder Hydroxy steht,
T² für Phenyl, Benzyl, 1*H-*Indol-3-yl oder 1*H*-Indol-3-ylmethyl steht,
R³⁵ für Methyl oder Hydroxy steht,
sowie ihre Salze, Solvate und Solvate der Salze.

Bevorzugter Gegenstand der Erfindung sind Binder-Wirkstoff Konjugate der allgemeinen Formel (Ia), in welcher
- n: für eine Zahl von 1 bis 10 steht,
- AK: für AK₁ oder AK₂ steht
wobei
AK₁ für einen Antikörper, der die sechs CDR-Sequenzen des Antikörpers B01-3, B01-10, M31-B01 oder D02-6, die variable leichte und variable schwere Kette des Antikörpers B01-3, B01-10, M31-B01 oder D02-6 oder die leichte und schwere Kette des Antiköpers B01-3, B01-10, M31-B01 oder D02-6 umfasst, steht, der über das Schwefelatom eines Cystein-Restes des Binders an die Gruppe G gebunden ist,
AK₂ für einen Antikörper, der die sechs CDR-Sequenzen des Antikörpers B01-3, B01-10, M31-B01 oder D02-6, die variable leichte und variable schwere Kette des Antikörpers B01-3, B01-10, M31-B01 oder D02-6 oder die leichte und schwere Kette des Antiköpers B01-3, B01-10, M31-B01 oder D02-6 umfasst, steht, der über die NH-Seitengruppe eines Lysin-Restes des Binders an die Gruppe G gebunden ist,
- G: für den Fall, dass AK = AK₁ ist, für eine Gruppe der 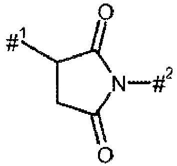 steht, wobei
#¹ die Verknüpfungsstelle mit dem Cystein-Rest des Binders kennzeichnet,
#² die Verknüpfungsstelle mit der Gruppe L¹ kennzeichnet,
oder
für den Fall, dass AK = AK₂ ist, für Carbonyl steht,
- L¹: für eine Bindung, lineares (C₂-C₆)-Alkandiyl, eine Gruppe der Formel 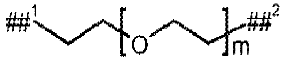 steht, wobei
m für eine Zahl von 2 oder 3 steht,
##¹ die Verknüpfungsstelle mit der Gruppe G kennzeichnet,
##² die Verknüpfungsstelle mit der Gruppe B kennzeichnet,
wobei (C₂-C₆)-Alkandiyl mit 1 oder 2 Substituenten Methyl substiuiert sein kann,
- B: für eine Bindung oder eine Gruppe der Formel 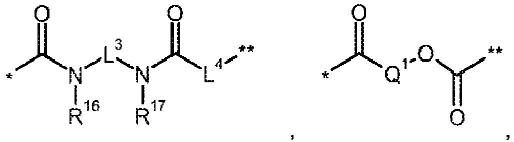 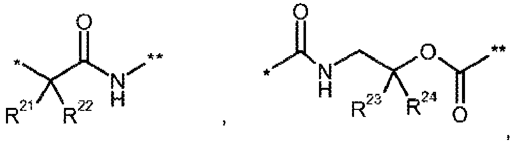 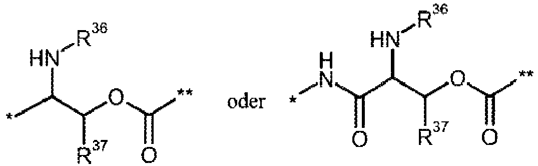 steht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Bindung oder eine Gruppe der 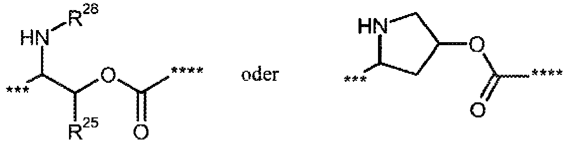 steht, worin
*** die Verknüpfungsstelle mit der Carbonylgruppe kennzeichnet,
**** die Verknüpfungsstelle mit L² kennzeichnet,
R²⁵ für Methyl steht,
R²⁸ für Wasserstoff, Methylcarbonyl oder tert.-Butyloxycarbonyl steht,
Q¹ für Piperidin-1,4-diyl steht,
**R**¹⁶ für Wasserstoff oder Methyl steht,
**R**¹⁷ für Wasserstoff oder Methyl steht,
oder
**R**¹⁶ und R¹¹ zusammen mit den Atomen, an die sie gebunden sind, einen Piperazinylring bilden,
**R**²¹ für Wasserstoff oder Methyl steht,
**R**²² für Wasserstoff oder Methyl steht,
oder
**R**²¹ und R²² zusammen mit den Atomen, an die sie gebunden sind, einen Cyclopropylring bilden,
**R**²³ für Methyl steht,
**R**²⁴ für Wasserstoff steht,
**R**³⁶ für Wasserstoff Methylcarbonyl oder tert.-Butyloxycarbonyl steht,
**R**³⁷ für Wasserstoff oder Methyl steht,
- L²: für lineares (C₂-C₆)-Alkandiyl oder für eine Gruppe der Formel 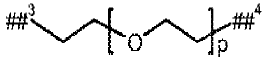 steht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet.
- D: für eine Gruppe der Formel 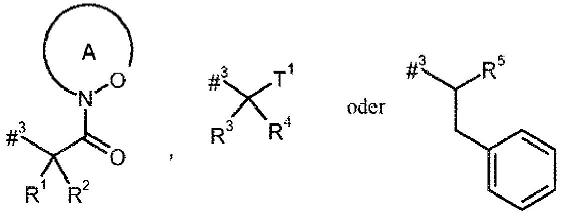 steht, wobei
#³ die Verknüpfungsstelle mit dem Stickstoffatom kennzeichnet,
R¹ für Wasserstoff steht,
R² für 1-Hydroxyethyl, Benzyl, 4-Hydroxybenzyl, 1-Phenylethyl oder 1*H*-Indol-3-yl-methyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 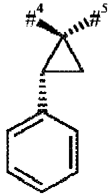 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 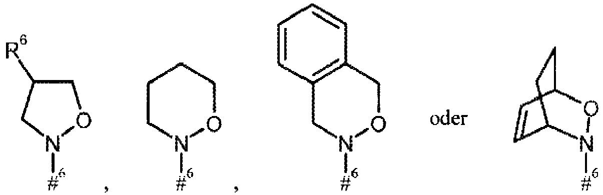 steht, worin
#⁶ die Verknüpfungsstelle mit der Carbonyl-Gruppe kennzeichnet,
R⁴ für Wasserstoff, Hydroxy oder Benzyloxy steht,
**R**³ für Wasserstoff steht,
**R**⁴ für Benzyl, 4-Hydroxybenzyl, 1-Phenylethyl oder 1*H*-Indol-3-ylmethyl steht,
oder
**R**³ und R⁴ zusammen mit dem Kohlenstoffatom, an das sie gebunden sind, eine (1*S*,2*R*)-2-Phenylcyclopropan-1,1-diyl-Gruppe der Formel 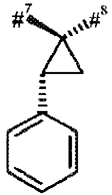 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹ oder -CH₂-O-R¹¹ steht,
worin
**R**⁷ für Wasserstoff, Methyl, Ethyl, *n*-Propyl, *tert*-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der Formel 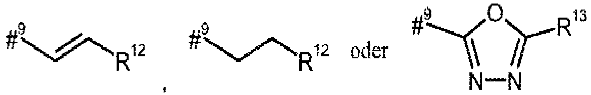 steht, worin
#⁹ die Verknüpfungsstelle mit -CHCH₂Phenyl kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
R³⁵ für Methyl oder Hydroxy steht,
sowie ihre Salze, Solvate und Solvate der Salze.

Bevorzugter Gegenstand der vorliegenden Erfindung sind Binder-Wirkstoff Konjugate der allgemeinen Formel (Ia) wie oben angegeben, in welcher
- n: für eine Zahl von 1 bis 10 steht,
- AK: für AK₁ oder AK₂ steht
wobei
AK₁ für einen Antikörper, der die sechs CDR-Sequenzen des Antikörpers B01-3, B01-10, M31-B01 oder D02-6, die variable leichte und variable schwere Kette des Antikörpers B01-3, B01-10, M31-B01 oder D02-6 oder die leichte und schwere Kette des Antiköpers B01-3, B01-10, M31-B01 oder D02-6 umfasst, steht, der über das Schwefelatom eines Cystein-Restes des Binders an die Gruppe G gebunden ist,
AK₂ für einen Antikörper, der die sechs CDR-Sequenzen des Antikörpers B01-3, B01-10, M31-B01 oder D02-6, die variable leichte und variable schwere Kette des Antikörpers B01-3, B01-10, M31-B01 oder D02-6 oder die leichte und schwere Kette des Antiköpers B01-3, B01-10, M31-B01 oder D02-6 umfasst, steht, der über die NH-Seitengruppe eines Lysin-Restes des Binders an die Gruppe G gebunden ist,
- G: für den Fall, dass AK = AK₁ ist, für eine Gruppe der Formel 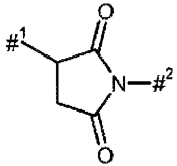 steht, wobei
#¹ die Verknüpfungsstelle mit dem Cystein-Rest des Binders kennzeichnet,
#² die Verknüpfungsstelle mit der Gruppe L¹ kennzeichnet,
oder
für den Fall, dass AK = AK₂ ist, für Carbonyl steht,
- L¹: für eine Bindung, lineares (C₂-C₆)-Alkandiyl eine Gruppe der Formel 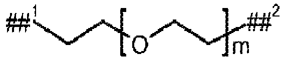 steht, wobei
m für eine Zahl von 2 oder 3 steht,
##¹ die Verknüpfungsstelle mit der Gruppe G kennzeichnet,
##² die Verknüpfungsstelle mit der Gruppe B kennzeichnet,
wobei (C₂-C₆)-Alkandiyl mit 1 oder 2 Substituenten Methyl substiuiert sein kann,
- B: für eine Bindung oder eine Gruppe der Formel 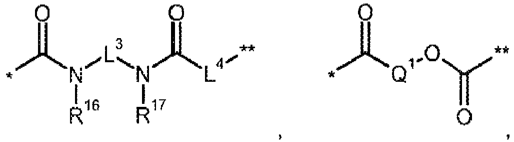 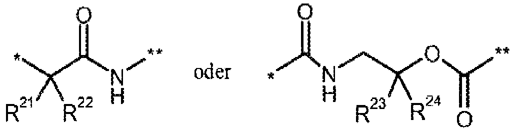 steht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Bindung oder eine Gruppe der Formel 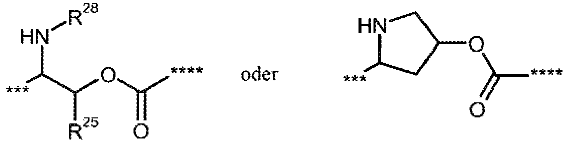 steht, worin
*** die Verknüpfungsstelle mit der Carbonylgruppe kennzeichnet,
**** die Verknüpfungsstelle mit L² kennzeichnet,
R²⁵ für Methyl steht,
R²⁸ für Wasserstoff, Methylcarbonyl oder tert.-Butyloxycarbonyl steht,
Q¹ für Piperidin-1,4-diyl steht,
R¹⁶ für Wasserstoff oder Methyl steht,
R¹⁷ für Wasserstoff oder Methyl steht,
oder
R¹⁶ und R¹⁷ zusammen mit den Atomen, an die sie gebunden sind, einen Piperazinylring bilden,
R²¹ für Wasserstoff oder Methyl steht,
R²² für Wasserstoff oder Methyl steht,
oder
R²¹ und R²² zusammen mit den Atomen, an die sie gebunden sind, einen Cyclopropylring bilden,
R²³ für Methyl steht,
R²⁴ für Wasserstoff steht,
- L²: für lineares (C₂-C₆)-Alkandiyl oder für eine Gruppe der Formel 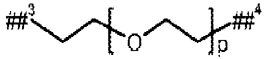 steht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
- D: für eine Gruppe der Formel 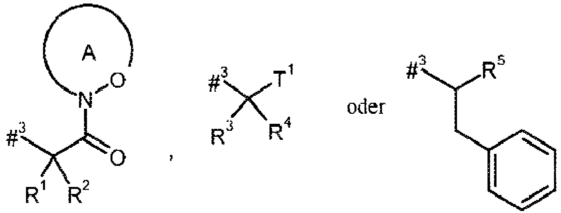 steht, wobei
#³ die Verknüpfungsstelle mit dem Stickstoffatom kennzeichnet,
R¹ für Wasserstoff steht,
R² für 1-Hydroxyethyl, Benzyl, 1-Hydroxybenzyl, 1-Phenylethyl oder 1*H*-Indol-3-yl-methyl steht, oder
R¹ und R² zusammen mit dem Kohlenstoffatom, an das sie gebunden sind, eine (1*S*,2*R*)-2-Phenylcyclopropan-1,1-diyl-Gruppe der Formel 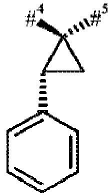 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 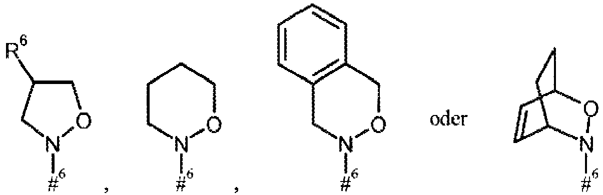 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff steht,
R⁴ für Benzyl, 1-Hydroxybenzyl, 1-Phenylethyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 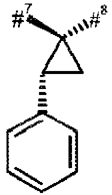 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹ oder -CH₂-O-R¹¹ steht,
worin
R⁷ für Wasserstoff, Methyl, Ethyl, *n*-Propyl, *tert*.-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der Formel 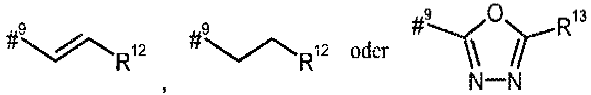 steht, worin
#⁹ die Verknüpfungsstelle mit -CHCH₂Phenyl kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
R³⁵ für Methyl oder Hydroxy steht,
sowie ihre Salze, Solvate und Solvate der Salze.

Bevorzugter Gegenstand der vorliegenden Erfindung sind Binder-Wirkstoff Konjugate der allgemeinen Formel (Ia) wie oben angegeben, in welcher
- n: für eine Zahl von 1 bis 10 steht,
- AK: für AK₂ steht.
wobei
AK₂ für einen Antikörper, der die sechs CDR-Sequenzen des Antikörpers B01-3, B01-10, M31-B01 oder D02-6, die variable leichte und variable schwere Kette des Antikörpers B01-3, B01-10, M31-B01 oder D02-6 oder die Leichte und schwere Kette des Antiköpers B01-3, B01-10, M31-B01 oder D02-6 umfasst, steht, der über die NH-Scitengruppe eines Lysin-Restes des Binders an die Gruppe G gebunden ist,
- G: für Carbonyl steht,
- L¹: für eine Bindung, steht,
- B: für eine Bindung steht,
- L²: für lineares (C₃-C₆)-Alkandiyl oder für eine Gruppe der Formel 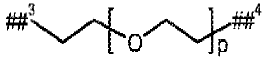 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
- D: für eine Gruppe der Formel 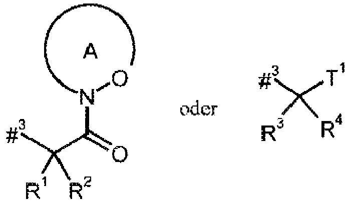 steht, wobei
#³ die Verknüpfungsstelle mit dem Stickstoffatom kennzeichnet,
R¹ für Wasserstoff steht,
R² für Benzyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 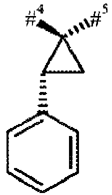 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 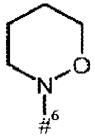 steht, worin
#⁶ die Verknüpfungsstelle mit der Carbonyl-Gruppe kennzeichnet,
R³ für Wasserstoff steht,
R⁴ für Benzyl oder 1*H*-Indol-3-ylmethyl steht,
oder
R³ und R⁴ zusammen mit dem Kohlenstoffatom, an das sie gebunden sind, eine (1*S,*2*R*)-2-Phenylcyclopan-1,1-diyl-Gruppe der Formel 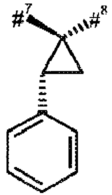 bilden, worin
#⁷ die Verknüpfungsstelle mit dem angrenzenden Stiekstoffatom kennzeichnet,
#⁸ die Verknüpfungsstelle mit der Gruppe T¹ kennzeichnet,
T¹ für eine Gruppe der Formel -C(=O)-OR⁷ oder -C(=O)-NR⁸R⁹ steht,
worin
R⁷ für Wasserstoff, Methyl, Ethyl, *n*-Propyl, *tert*.-Butyl, Benzyl oder Adamantylmethyl steht,
R⁸ für Wasserstoff steht,
R⁹ für Wasserstoff oder Benzyl steht,
R³⁵ für Methyl steht,
sowie ihre Salze, Solvate und Solvate der Saize.

Bevorzugter Gegenstand der vorliegenden Erfindung sind Binder-Wirkstoff Konjugate der allgemeinen Formel (Ia) wie oben angegeben, in welcher
- n: für eine Zahl von 1 bis 10 steht,
- AK: für AK₂ steht,
wobei
AK₂ für einen Antikörper, der die sechs CDR-Sequenzen des Antikörpers B01-3, B01-10, M31-B01 oder D02-6, die variable leichte und variable schwere Kette des Antikörpers B01-3, B01-10, M31-B01 oder D02-6 oder die leichte und schwere Kette des Antiköpers B01-3, B01-10, M31-B01 oder D02-6 umfasst, steht, der über die NH-Seitengruppe eines Lysin-Restes des Binders an die Gruppe G gebunden ist,
- G: für Carbonyl steht,
- L¹: für eine Bindung, steht,
- B: für eine Bindung steht,
- L²: für lineares (C₃-C₆)-Alkandiyl oder für eine Gruppe der Formel 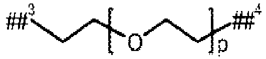 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
- D: für eine Gruppe der Formel 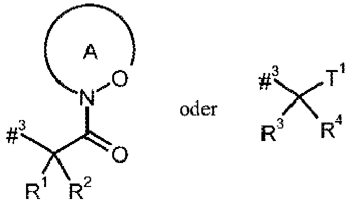 steht, wobei
#³ die Verknüpfungsstelle mit dem Stickstoffatom kennzeichnet,
R¹ für Wasserstoff steht,
R² für 4-Hydroxybenzyl oder 1*H*-lndol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 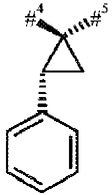 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyelus der Formel 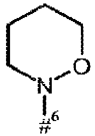 steht, worin
#⁶ die Verknüpfungsstelle mit der Carbonyl-Gruppe kennzeichnet,
R³ für Wasserstoff steht,
R⁴ für 4-Hydroxybenzyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 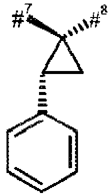 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷ oder -C(=O)-NR⁸R⁹ steht,
worin
R⁷ für Wasserstoff, Methyl, Ethyl, *n*-Propyl, *tert*.-Butyl, Benzyl oder Adamantylmethyl steht,
R⁸ für Wasserstoff steht,
R⁹ für Wasserstoff oder Benzyl steht,
R³⁵ für Methyl steht,
sowie ihre Salze, Solvate und Solvate der Salze.

Bevorzugter Gegenstand der vorliegenden Erfindung sind Binder-Wirkstoff Konjugate der allgemeinen Formel (Ia) wie oben angegeben, in welcher
- n: für eine Zahl von 1 bis 1 0 steht,
- AK: für AK; steht,
wobei
AK₁ für einen Antikörper, der die sechs CDR-Sequenzen des Antikörpers B01-3, B01-10, M31-B01 oder D02-6, die variable leichte und variable schwere Kette des Antikörpers B01-3, B01-10, M31-B01 oder D02-6 oder die leichte und schwere Kette des Antiköpers B01-3, B01-10, M31-B01 oder D02-6 umfasst, steht, der über das Schwefelatom eines Cystein-Restes des Binders an die Gruppe G gebunden ist,
- G: für eine Gruppe der Formel 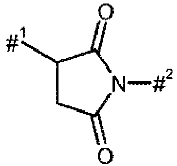 steht, wobei
#¹ die Verknüpfungsstelle mit dem Cystein-Rest des Binders kennzeichnet,
#² die Verknüpfungsstelle mit der Gruppe L¹ kennzeichnet,
- L¹: für eine Bindung, lineares (C₃-C₅)-Alkandiyl oder eine Gruppe der Formel 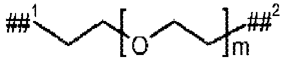 steht, wobei
m für eine Zahl von 2 oder 3 steht,
##¹ die Verknüpfungsstelle mit der Gruppe G kennzeichnet,
##² die Verknüpfungsstelle mit der Gruppe B kennzeichnet,
wobei (C₃-C₅)-Alkandiyl mit 1 oder 2 Substituenten Methyl substiuiert sein kann,
- B: für eine Bindung oder eine Gruppe der Formel 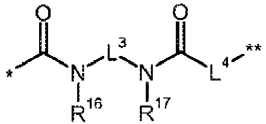 steht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Bindung oder eine Gruppe der Formel 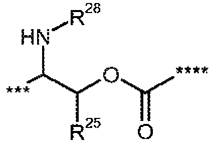 steht, worin
*** die Verknüpfungsstelle mit der Carbonylgruppe kennzeichnet,
**** die Verknüpfungsstelle mit L² kennzeichnet,
R²⁵ für Methyl steht,
R²⁸ für Wasserstoff Methylcarbonyl oder tert.-Butyloxycarbonyl steht,
R¹⁶ für Wasserstoff oder Methyl steht,
R¹⁷ für Wasserstoff oder Methyl steht,
oder
R¹⁶ und R¹⁷ zusammen mit den Atomen, an die sie gebunden sind, einen Piperazinylring bilden,
- L²: für lineares (C₃-C₅)-Alkandiyl oder für eine Gruppe der Formel 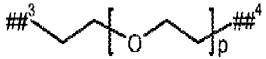 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
D für eine Gruppe der Formel 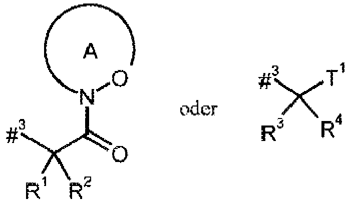 steht, wobei
#³ die Verknüpfungsstelle mit dem Stickstoffatom kennzeichnet,
R¹ für Wasserstoff steht,
R² für Benzyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 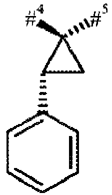 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 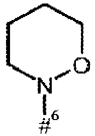 steht, worin
#⁶ die Verknüpfungsstelle mit der Carbonyl-Gruppe kennzeichnet,
R³ für Wasserstoff steht,
R⁴ für Benzyl oder 1*H*-Indol-3-ylmethyl steht,
oder
R³ und R⁴ zusammen mit dem Kohlenstoffatom, an das sie gebunden sind, eine (1*S,*2*R*)-2-Phenylcyclopropan-1,1-diyl-Gruppe der Formel 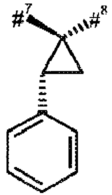 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷ oder -C(=O)-NR⁸R⁹ steht,
worin
R⁷ für Wasserstoff, Methyl, Ethyl, *n*-Propyl, *tert*.-Butyl, Benzyl oder Adamantylmethyl steht,
R⁸ für Wasserstoff steht,
R⁹ für Wasserstoff oder Benzyl steht,
R³⁵ für Methyl steht,
sowie ihre Salze, Solvate und Solvate der Salze.

Bevorzugter Gegenstand der vorliegenden Erfindung sind Binder-Wirkstoff Konjugate der allgemeinen Formel (la) wie oben angegeben, in welcher
- n: für eine Zahl von 1 bis 10 steht,
- AK: für AK₁ steht,
wobei
AK₁ für einen Antikörper, der die sechs CDR-Sequenzen des Antikörpers B01-3, B01-10, M31-B01 oder D02-6, die variable leichte und variable schwere Kette des Antikörpers B01-3, B01-10, M31-B01 oder D02-6 oder die leichte und schwere Kette des Antiköpers B01-3, B01-10, M31-B01 oder D02-6 umfasst, steht, der über das Schwefelatom eines Cystein-Restes des Binders an die Gruppe G gebunden ist.
- G: für eine Gruppe der Formel 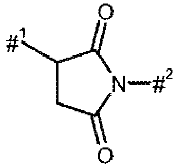 steht, wobei
#¹ die Verknüpfungsstelle mit dem Cystein-Rest des Binders kennzeichnet,
#² die Verknüpfungsstelle mit der Gruppe L¹ kennzeichnet,
- L¹: für eine Bindung, lineares (C₃-C₅)-Alkandiyl oder eine Gruppe der Formel 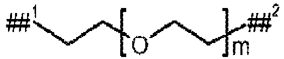 steht, wobei
m für eine Zahl von 2 oder 3 steht,
## ¹ die Verknüpfungsstelle mit der Gruppe G kennzeichnet,
##² die Verknüpfungsstelle mit der Gruppe B kennzeichnet,
wobei (C₃-C₅)-Alkandiyl mit 1 oder 2 Substituenten Methyl substituiert sein kann,
- B: für eine Bindung oder eine Gruppe der Formel 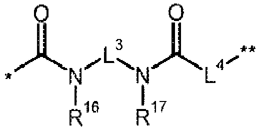 steht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Bindung oder eine Gruppe der Formel 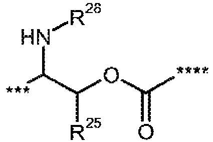 steht, worin
*** die Verknüpfungsstelle mit der Carbonylgruppe kennzeichnet,
**** die Verknüpfungsstelle mit L² kennzeichnet,
R²⁵ für Methyl steht,
R²⁸ für Wasserstoff, Methylcarbonyl oder tert.-Butyloxycarbonyl steht,
R¹⁶ für Wasserstoff oder Methyl steht,
R¹⁷ für Wasserstoff oder Methyl steht,
oder
R¹⁶ und R¹⁷ zusammen mit den Atomen, an die sie gebunden sind, einen Piperazinylring bilden,
- L²: für lineares (C₃-C₅)-Alkandiyl oder für eine Gruppe der Formel 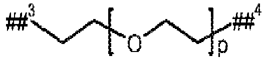 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
- D: für eine Gruppe der Formel 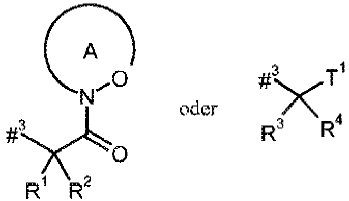 steht, wobei
#³ die Verknüpfungsstelle mit dem Stickstoffatom kennzeichnet,
R¹ für Wasserstoff steht,
R² für 4-Hydroxybenzyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 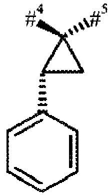 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 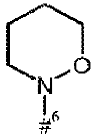 steht, worin
#⁶ die Verknüpfungsstelle mit der Carbonyl-Gruppe kennzeichnet,
**R**³ für Wasserstoff steht,
R⁴ für 4-Hydroxybenzyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 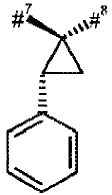 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷ oder -C(=O)-NR⁸R⁹ steht,
worin
R⁷ für Wasserstoff, Methyl, Ethyl, *n*-Propyl, *tert*.-Butyl, Benzyl oder Adamantylmethyl steht,
R⁸ für Wasserstoff steht,
R⁹ für Wasserstoff oder Benzyl steht,
R³⁵ für Methyl steht,
sowie ihre Salze, Solvate und Solvate der Salze.

Ein weiterer Gegenstand der vorliegenden Erfindung sind Verbindungen der Formel (XXXa) 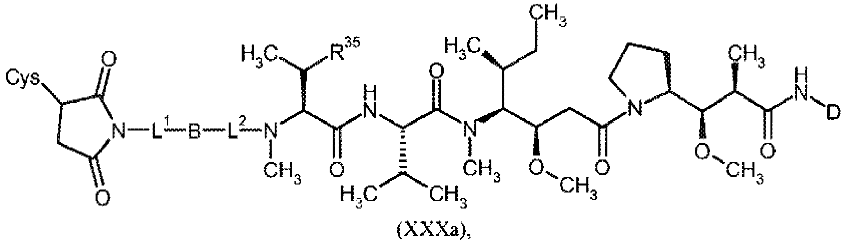 in welcher
- Cys: für einen Cystein-Rest steht, der über das Schwefelatom der Seitenkette an ein Kohlenstoffatom des Succinimids gebunden ist,
- L¹: für eine Bindung, lineares (C₁-C₁₀)-Alkandiyl, eine Gruppe der Formel 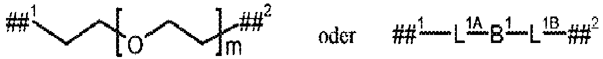 steht, wobei
m für eine Zahl von 2 bis 6 steht,
## ¹ die Verknüpfungsstelle mit der Gruppe G kennzeichnet,
##² die Verknüpfungsstelle mit der Gruppe B kennzeichnet,
L^{1A} für lineares (C₂-C₁₀)-Alkandiyl steht,
B¹ für eine Gruppe der Formel 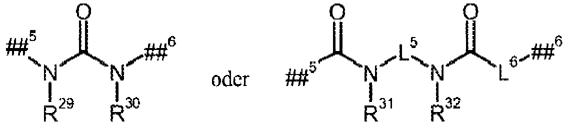 steht, worin
##⁵ die Verknüpfungsstelle mit der Gruppe L^{1A} kennzeichnet,
##⁶ die Verknüpfungsstelle mit der Gruppe L^{1B} kennzeichnet,
L⁵ für eine Bindung oder (C₂-C₄)-Alkandiyl steht,
L⁶ für eine Bindung steht,
R²⁹ für Wasserstoff oder (C₁-C₄)-Alkyl steht,
R³⁰ für Wasserstoff oder (C₁-C₄)-Alkyl steht,
oder
R²⁹ und R³⁰ zusammen mit den Atomen, an die sie gebunden sind, einen 5- oder 6-gliedrigen Heterocyclus bilden,
R³¹ für Wasserstoff oder (C₁-C₄)-Alkylsteht,
R³² für Wasserstoff oder (C₁-C₄)-Alkyl steht,
oder
R³¹ und R³² zusammen mit den Atomen, an die sie gebunden sind, einen 5- oder 6-gliedrigen Heterocyclus bilden,
L^{1B} für lineares (C₂-C₁₀)-Alkandiyl steht,
und
wobei (C₁-C₁₀)-Alkandiyl mit 1 bis 4 Substituenten unabhängig voneinander ausgewählt aus der Gruppe Methyl, Hydroxy und Benzyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
- B: für eine Bindung oder eine Gruppe der Formel 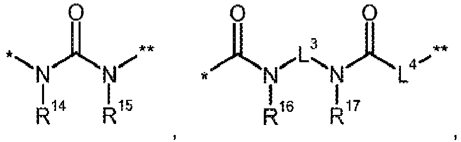 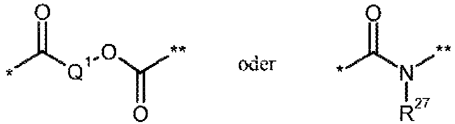 steht, wobei
* die Verknüpfungsstelle mit L¹ kennzeichnet,
** die Verknüpfungsstelle mit L² kennzeichnet,
P für O oder NH steht,
L³ für eine Bindung oder (C₂-C₄)-Alkandiyl steht,
L⁴ für eine Bindung steht,
Q¹ für einen 4- bis 7-gliedrigen Heterocyclus steht,
Q² für einen 3- bis 7-gliedrigen Carbocyclus oder einen 4- bis 7-gliedrigen Heterocyclus steht,
R¹⁴ für Wasserstoff oder (C₁-C₄)-Alkyl steht,
R¹⁵ für Wasserstoff oder (C₁-C₄)-Alkyl steht,
oder
R¹⁴ und R¹⁵ zusammen mit den Atomen, an die sie gebunden sind, einen 5- oder 6-gliedrigen Heterocyclus bilden,
R¹⁶ für Wasserstoff oder (C₁-C₄)-Alkyl steht,
R¹⁷ für Wasserstoff oder (C₁-C₄)-Alkyl steht,
oder
R¹⁶ und R¹⁷ zusammen mit den Atomen, an die sie gebunden sind, einen 5- oder 6-gliedrigen Heterocyclus bilden,
R¹⁸ für Wasserstoff oder (C₁-C₄)-Alkyl steht,
R¹⁹ für Wasserstoff oder die Seitengruppe einer natürlichen α-Aminosäure oder ihrer Homologe oder Isomere steht,
R²⁰ für Wasserstoff oder (C₁-C₄)-Alkyl steht,
oder
R¹⁹ und R²⁰ zusammen mit den Atomen, an die sie gebunden sind, einen Pyrrolidinylring bilden,
R²¹ für Wasserstoff oder (C₁₋C₄)-Alkyl steht,
R²² für Wasserstoff oder (C₁-C₄)-Alkyl sicht,
oder
R²¹ und R²² zusammen mit den Atomen, an die sie gebunden sind, einen 3- bis 7-gliedrigen Carbocyclus bilden,
R²³ für (C₁-C₄)-Alkyl steht,
R²⁴ für Wasserstoff oder (C₁-C₄)-Alkyl steht,
R²⁷ für Wasserstoff oder (C₁-C₄₎-Alkyl steht,
- L²: für lineares (C₂-C₁₀)-Alkandiyl oder für eine Gruppe der Formel 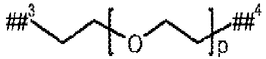 steht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
wobei (C₂-C₁₀)-Alkandiyl mit 1 bis 4 Substituenten unabhängig voneinander ausgewählt aus der Gruppe Methyl, Hydroxy und Benzyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
- D: für eine Gruppe der Formel 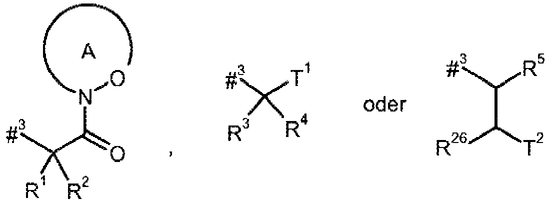 steht, wobei
#³ die Verknüpfungsstelle mit dem Stickstoffatom kennzeichnet,
R¹ für Wasserstoff oder Methyl steht,
R² für Isopropyl, Isobutyl, *sec*.-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenyl-ethyl, Diphenylmethyl, 1*H*-Imidazol-4-ylmethyl oder 1*H*-Indol-3-ylmethyl steht,
oder
R¹ und R² zusammen mit dem Kohlenstoffatom, an das sie gebunden sind, eine (1*S,2R*)-2-Phenylcyclopropan-1,1-diyl-Gruppe der Formel 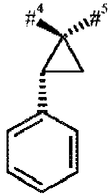 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 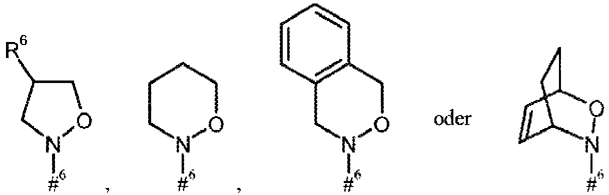 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff oder Methyl steht,
R⁴ für Isopropyl, Isobutyl, *sec*.-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenyl-ethyl, Diphenylmethyl, 1*H*-Imidazol-4-ylmethyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 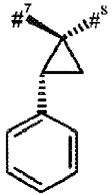 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹, -C(=O)-NH-NH-R¹⁰ oder -CH₂-O-R¹¹ steht,
worin
R⁷ für Wasserstoff, Methyl, Ethyl, *n*-Propyl, *tert*.-Butyl, Benzyl oder Adamantylmethyl steht,
**R**⁸ für Wasserstoff oder Methyl steht,
R⁹ für Wasserstoff, Methyl, Ethyl, *n*-Propyl oder Benzyl steht,
oder
R⁸ und R⁹ bilden zusammen mit dem Stickstoffatom, an das sie gebunden sind, einen 4- bis 7-gliedrigen Heterocyclus,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der Formel 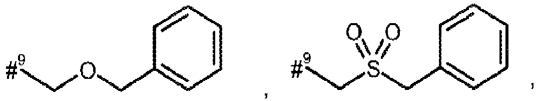 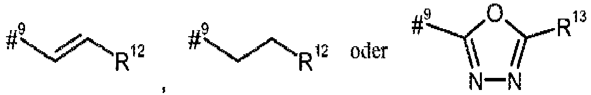 steht, worin
#⁹ die Verknüpfungsstelle mit -CHC(R²⁶)-T² kennzeichnet,
R¹² für Phenyl steht, das mit Methoxycarbonyl, Carboxyl oder einer Gruppe der Formel S(O)₂OH substituiert sein kann,
R¹³ für Phenyl steht, das mit Methoxycarbonyl oder Carboxyl substituiert sein kann,
R²⁶ für Wasserstoff oder Hydroxy steht,
T² für Phenyl, Benzyl, 1*H*-Indol-3-yl oder 1*H*-Indol-3-ylmethyl steht,
R³⁵ für Methyl oder Hydroxy steht,
sowie ihre Salze, Solvate und Solvate der Salze.

Bevorzugter Gegenstand der vorliegenden Erfindung sind Verbindungen der Formel (XXXa) wie oben angegeben, in welcher
- Cys: für einen Cystein-Rest steht, der über das Schwefelatom der Seitenkette über ein Kohlenstoffatom des Succinimids gebunden ist, steht
- L¹: für eine Bindung, lineares (C₂-C₆)-Alkandiyl, eine Gruppe der Formel 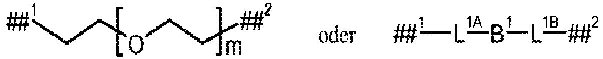 steht, wobei
m für eine Zahl von 2 oder 3 sieht,
##¹ die Verknüpfungsstelle mit der Gruppe G kennzeichnet,
##² die Verknüpfungsstelle mit der Gruppe B kennzeichnet,
L^{1A} für lineares (C₂-C₆)-Alkandiyl steht,
B¹ für eine Gruppe der Formel 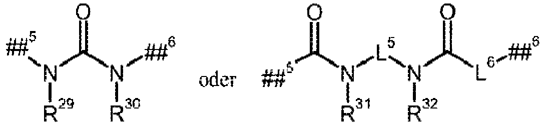 steht, worin
##⁵ die Verknüpfungsstelle mit der Gruppe L^{1A} kennzeichnet,
##⁶ die Verknüpfungsstelle mit der Gruppe L^{1B} kennzeichnet,
L⁵ für eine Bindung steht,
L⁶ für eine Bindung steht,
R²⁹ für Wasserstoff steht,
R³⁰ für Wasserstoff steht,
R³¹ für Wasserstoff oder Methyl steht,
R³² für Wasserstoff oder Methyl steht,
L^{1B} für lineares (C₂-C₆)-Alkandiyl steht,
und
wobei (C₂-C₆)-Alkandiyl mit 1 oder 2 Substituenten Methyl substiuiert sein kann,
- B: für eine Bindung oder eine Gruppe der Formel 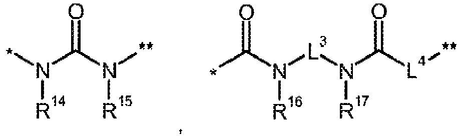 oder 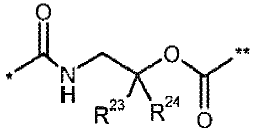 steht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Bindung steht,
R¹⁴ für Wasserstoff steht,
R¹⁵ für Wasserstoff steht,
R¹⁶ für Wasserstoff oder Methyl steht,
R¹⁷ für Wasserstoff oder Methyl steht,
oder
R¹⁶ und R¹⁷ zusammen mit den Atomen, an die sie gebunden sind, Piperazinylring bilden,
R²³ für Methyl steht,
R²⁴ für Wasserstoff oder Methyl steht,
- L²: für lineares (C₂-C₆)-Alkandiyl oder für eine Gruppe der Formel 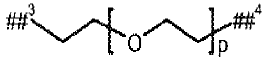 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
- D: für eine Gruppe der Formel 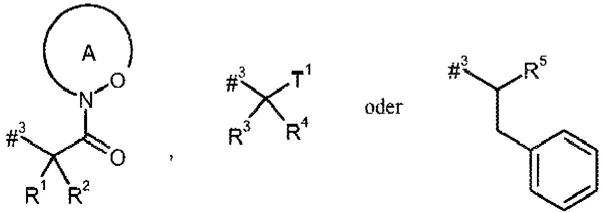 steht, wobei
#³ die Verknüpfungsstelle mit dem Stickstoffatom kennzeichnet,
R¹ für Wasserstoff steht,
R² für 1-Hydroxyethyl, Benzyl, 4-Hydroxybenzyl, 1-Phenylethyl oder 1*H*-Indol-3-yl-methyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 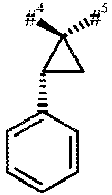 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 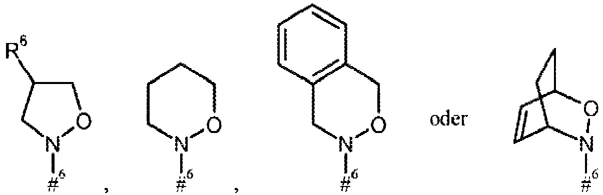 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff steht,
R⁴ für 1-Hydroxyethyl, Benzyl, 4-Hydroxybenzyl, 1-Phenylethyl oder 1*H*-Indol-3-yl-methyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 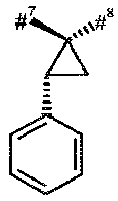 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹, -C(=O)-NH-NH-R¹⁰ oder -CH₂-O-R¹¹ steht,
worin
R⁷ für Wasserstoff, Methyl, Ethyl, *n*-Propyl, *tert*.-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
oder
R⁸ und R⁹ bilden zusammen mit dem Stickstoffatom, an das sie gebunden sind, einen 4- bis 7-gliedrigen Heterocyclus,
R¹⁰ für Benzoyl steht,
R¹¹ für Benzyl, das in der Phenylgruppe mit Methoxycarbonyl oder Carboxyl substituiert sein kann, steht,
- R⁵: für Wasserstoff, Methyl oder eine Gruppe der Formel 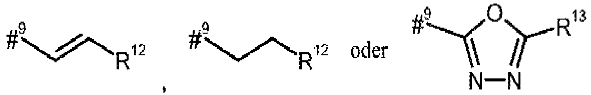 steht, worin
#⁹ die Verknüpfungsstelle mit CHCH₂Phenyl kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
- R³⁵: für Methyl oder Hydroxy steht,
sowie ihre Salze, Solvate und Solvate der Salze.

Bevorzugter Gegenstand der vorliegenden Erfindung sind Verbindungen der Formel (XXXa) wie oben angegeben, in welcher
- Cys: für einen Cystein-Rest steht, der über das Schwefelatom der Seitenkette über ein Kohlenstoffatom des Succinimids gebunden ist, steht
- L¹: für eine Bindung oder lineares (C₂-C₆)-Alkandiyl steht,
- B: für eine Bindung oder eine Gruppe der Formel 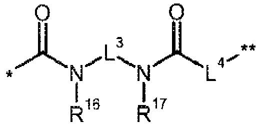 steht, wobei
* die Verknüpfungsstelle mit L¹ kennzeichnet,
** die Verknüpfungsstelle mit L² kennzeichnet,
L³ für eine Bindung steht,
L⁴ für eine Bindung steht,
R¹⁶ für Wasserstoff oder Methyl steht,
R¹⁷ für Wasserstoff oder Methyl steht,
- L²: für lineares (C₂-C₆)-Alkandiyl oder für eine Gruppe der Formel 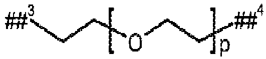 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
- D: für eine Gruppe der Formel 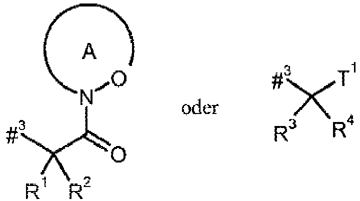 steht, wobei
#³ die Verknüpfungsstelle mit dem Stickstoffatom kennzeichnet,
R¹ für Wasserstoff steht,
R² für Benzyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 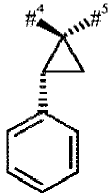 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 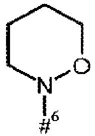 steht, worin
#⁶ die Verknüpfungsstelle mit der Carbonyl-Gruppe kennzeichnet,
R³ für Wasserstoff steht,
R⁴ für Benzyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 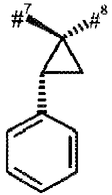 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷ oder -C(=O)-NR⁸R⁹ steht,
worin
R⁷ für Wasserstoff steht,
R⁸ für Wasserstoff steht,
R⁹ für Wasserstoff steht,
- R³⁵: für Methyl steht,
sowie ihre Salze, Solvate und Solvate der Salze.

Bevorzugter Gegenstand der vorliegenden Erfindung sind Verbindungen der Formel (XXXa) wie oben angegeben, in welcher
- Cys: für einen Cystein-Rest steht, der über das Schwefelatom der Seitenkette über ein Kohlenstoffatom des Succinimids gebunden ist, steht
- L¹: für eine Bindung oder lineares (C₂-C₆)-Alkandiyl steht,
- B: für eine Bindung oder eine Gruppe der Formel 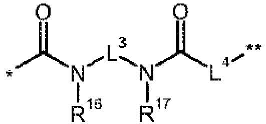 steht, wobei
* die Verknüpfungsstelle mit L¹ kennzeichnet,
** die VerknÜpfungsstelle mit L² kennzeichnet,
L³ für eine Bindung steht,
L⁴ für eine Bindung steht,
R¹⁶ für Wasserstoff oder Methyl steht,
R¹⁷ für Wasserstoff oder Methyl steht,
- L²: für lineares (C₂-C₆)-Alkandiyl oder für eine Gruppe der Formel 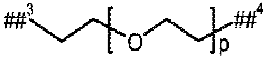 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
- D: für eine Gruppe der Formel 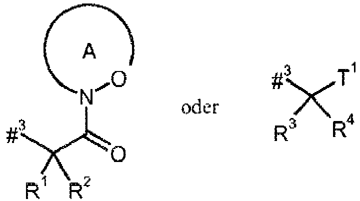 steht, wobei
#³ die Verknüpfungsstelle mit dem Stickstoffatom kennzeichnet,
R¹ für Wasserstoff steht,
R² für 4-Hydroxybenzyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 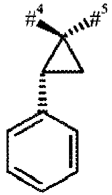 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 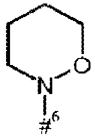 steht, worin
#⁶ die Verknüpfungsstelle mit der Carbonyl-Gruppe kennzeichnet,
R³ für Wasserstoff steht,
R⁴ für 4-Hydroxybenzyl oder 1*H*-Indol-3-ylmethyl steht,
oder
R³ und R⁴ zusammen mit dem Kohlenstoffatom, an das sie gebunden sind, eine (1*S,*2*R*)-2-Phenylcyclopropan-1,1-diyl-Gruppe der Formel 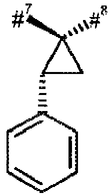 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷ oder -C(=O)-NR⁸R⁹ steht,
worin
R⁷ für Wasserstoff steht,
R⁸ für Wasserstoff steht,
R⁹ für Wasserstoff steht,
- R³⁵: für Methyl steht,
sowie ihre Salze, Solvate und Solvate der Salze.

Ein weiterer Gegenstand der vorliegenden Erfindung sind Verbindungen der Formel (XXXI) 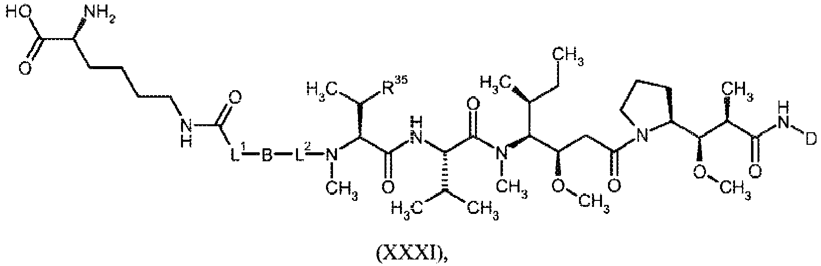 in welcher
- L¹: für eine Bindung, lineares (C₁-C₁₀)-Alkandiyl, eine Gruppe der Formel 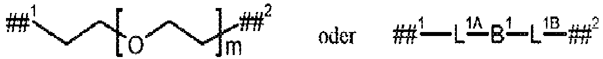 steht, wobei
m für eine Zahl von 2 bis 6 steht,
##¹ die Verknüpfungsstelle mit der Gruppe G kennzeichnet,
##² die Verknüpfungsstelle mit der Gruppe B kennzeichnet,
L^{1A} für lineares (C₂-C₁₀)-Alkandiyl steht,
B¹ für eine Gruppe der Formel 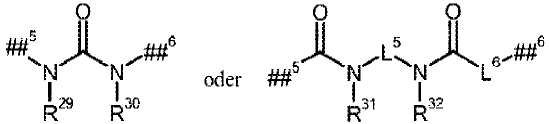 steht, worin
##⁵ die Verknüpfungsstelle mit der Gruppe L^{1A} kennzeichnet,
##⁶ die Verknüpfungsstelle mit der Gruppe L^{1B} kennzeichnet,
L⁵ für eine Bindung oder (C₂-C₄)-Alkandiyl steht,
L⁶ für eine Bindung steht,
R²⁹ für Wasserstoff oder (C₁-C₄)-Alkyl steht,
R³⁰ für Wasserstoff oder (C₁-C₄)-Alkyl steht,
oder
R²⁹ und R³⁰ zusammen mit den Atomen, an die sie gebunden sind, einen 5-oder 6-gliedrigen Heterocyclus bilden,
R³¹ für Wasserstoff oder (C₁-C₄)-Alkyl steht,
R³² für Wasserstoff oder (C₁-C₄)-Alkyl steht,
oder
R³¹ und R³² zusammen mit den Atomen, an die sie gebunden sind, einen 5-oder 6-gliedrigen Heterocyclus bilden,
L^{1B} für lineares (C₂-C₁₀)-Alkandiyl steht,
und
wobei (C₁-C₁₀)-Alkandiyl mit 1 bis 4 Substituenten unabhängig voneinander ausgewählt aus der Gruppe Methyl, Hydroxy und Benzyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
- B: für eine Bindung oder eine Gruppe der Formel 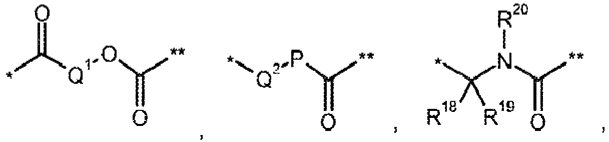 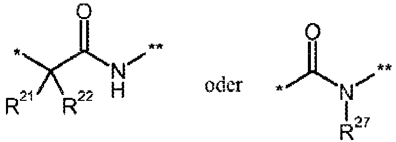 sieht, wobei
* die Verknüpfungsstelle mit L¹ kennzeichnet,
** die Verknüpfungsstelle mit L² kennzeichnet,
P für O oder NH steht,
Q¹ für einen 4- bis 7-gliedrigen Heterocyclus steht,
Q² für einen 3- bis 7-gliedrigen Carbocyclus oder einen 4- bis 7-gliedrigenHeterocyclus steht,
R¹⁸ für Wasserstoff oder (C₁-C₄)-Alkyl steht,
R¹⁹ für Wasserstoff oder die Seitengruppe einer natürlichen α-Aminosäure oder ihrer Homologe oder Isomere steht,
R²⁰ für Wasserstoff oder (C₁-C₄)-Alkyl steht,
oder
R¹⁹ und R²⁰ zusammen mit den Atomen, an die sie gebunden sind, einen Pyrrolidinylring bilden,
R²¹ für Wasserstoff oder (C₁-C₄)-Alkyl steht,
R²² für Wasserstoff oder (C₁-C₄)-Alkyl steht,
oder
R²¹ und R²² zusammen mit den Atomen, an die sie gebunden sind, einen 3- bis 7-gliedrigen Carbocyclus bilden,
R²⁷ für Wasserstoff oder (C₁-C₄)-Alkyl steht,
- L²: für lineares (C₂-C₁₀)-Alkandiyl oder für eine Gruppe der Formel 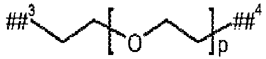 steht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
wobei (C₂-C₁₀)-Alkandiyl mit 1 bis 4 Substituenten unabhängig voneinander ausgewählt aus der Gruppe Methyl, Hydroxy und Benzyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
- D: für eine Gruppe der Formel 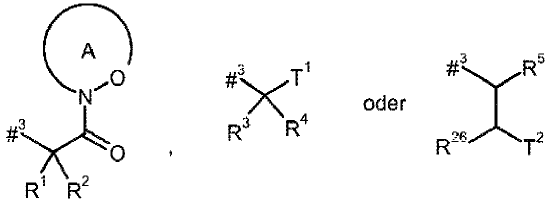 steht, wobei
#³ die Verknüpfungsstelle mit dem Stickstoffatom kennzeichnet,
R¹ für Wasserstoff oder Methyl steht,
R² für Isopropyl, Isobutyl, *sec*.-Butyl. *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenyl-ethyl, Diphenylmethyl, 1*H*-Imidazol-4-ylmethyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 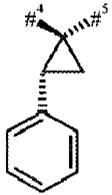 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 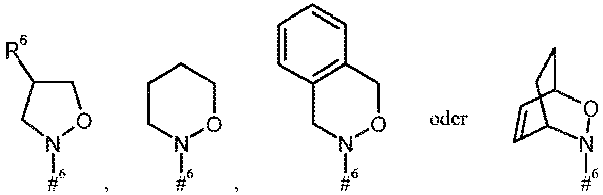 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff oder Methyl steht,
R⁴ für Isopropyl, Isobutyl, *sec*.-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenyl-ethyl, Diphenylmethyl, 1*H*-Imidazol-4-ylmethyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 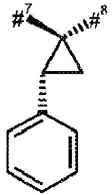 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹, -C(=O)-NH-NH-R¹⁰ oder -CH₂-O-R¹¹ steht,
worin
R⁷ für Wasserstoff, Methyl, Ethyl, *n*-Propyl, *tert*.-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
oder
R⁸ und R⁹ bilden zusammen mit dem Stickstoffatom, an das sie gebunden sind, einen 4- bis 7-gliedrigen Heterocyclus,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der Formel 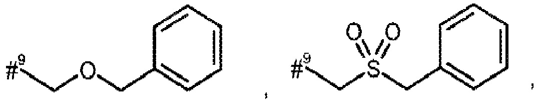 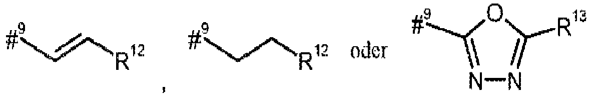 steht, worin
#⁹ die Verknüpfungsstelle mit -CHC(R²⁶)-T² kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
R²⁶ für Wasserstoff oder Hydroxy steht,
T² für Phenyl, Benzyl, 1*H*-Indol-3-yl oder 1*H*-Indol-3-ylmethyl steht,
R³⁵ für Methyl oder Hydroxy steht,
sowie ihre Salze, Solvate und Solvate der Salze.

Bevorzugter Gegenstand der vorliegenden Erfindung sind Verbindungen der Formel (XXXI) wie oben angegeben, in welcher
- L¹: für eine Bindung, lineares (C₂-C₆)-Alkandiyl oder eine Gruppe der Formel 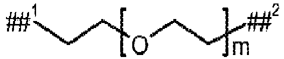 steht, wobei
m für eine Zahl von 2 oder 3 steht,
##¹ die Verknüpfungsstelle mit der Gruppe G kennzeichnet,
##² die Verknüpfungsstelle mit der Gruppe B kennzeichnet,
wobei (C₂-C₆)-Alkandiyl mit 1 oder 2 Substituenten Methyl substiuiert sein kann,
B für eine Bindung oder eine Gruppe der Formel 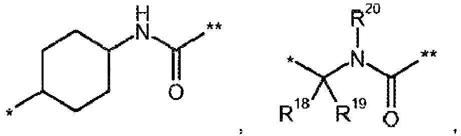 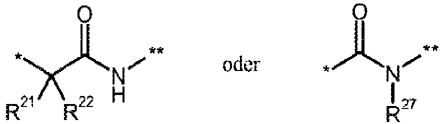 steht, wobei
* die Verknüpfungsstelle mit L¹ kennzeichnet,
** die Verknüpfungsstelle mit L² kennzeichnet,
R¹⁸ für Wasserstoff steht,
R¹⁹ für Methyl, Propan-2-yl, 2-Methylpropan-1-yl oder 1-Methylpropan-1-yl steht,
R²⁰ für Wasserstoff oder (C₁-C₄)-Alkyl steht,
oder
R¹⁹ und R²⁰ zusammen mit den Atomen, an die sie gebunden sind, einen Pyrrolidinylring bilden,
R²¹ für Wasserstoff oder Methyl steht,
R²² für Wasserstoff oder Methyl steht,
oder
R²¹ und R²² zusammen mit den Atomen, an die sie gebunden sind, einen Cyclopropyl-Ring bilden,
R²⁷ für Wasserstoff oder Methyl steht,
- L²: für lineares (C₂-C₆)-Alkandiyl oder für eine Gruppe der Formel 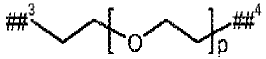 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
wobei (C₂-C₁₀)-Alkandiyl mit 1 oder 2 Substituenten Methyl substiuiert sein kann,
und
wobei zwei Kohlenstoffatome der Alkandiyl-Kette in 1,4-Relation zueinander unter Einbezug der gegebenenfalls zwischen ihnen liegenden Kohlenstoffatome zu einem Phenyl-Ring verbrückt sein können,
- D: für eine Gruppe der Formel 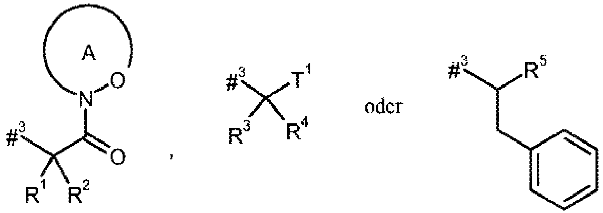 steht, wobei
#³ die Verknüpfungsstelle mit dem Stickstoffatom kennzeichnet,
R¹ für Wasserstoff steht,
R² für 1-Hydroxyethyl, Benzyl, 4-Hydroxybenzyl, 1-Phenylethyl oder 1*H*-Indol-3-yl-methyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 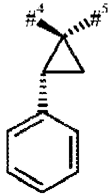 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 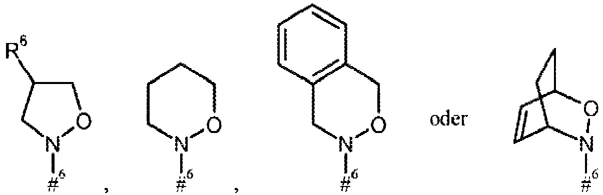 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff steht,
R⁴ für 1-Hydroxyethyl, Benzyl, 4-Hydroxybenzyl, 1-Phenylethyl oder 1*H*-Indol-3-yl-methyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 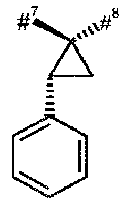 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹, -C(=O)-NH-NH-R¹⁰ oder -CH₂-O-R¹¹ steht,
worin
R⁷ für Wasserstoff, Methyl, Ethyl, *n*-Propyl, *tert*.-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
oder
R⁸ und R⁹ bilden zusammen mit dem Stickstoffatom, an das sie gebunden sind, einen 4- bis 7-gliedrigen Heterocyclus,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der Formel 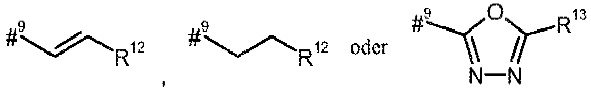 steht, worin
#⁹ die Verknüpfungsstelle mit CHCH₂Phenyl kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
R³⁵ für Methyl oder Hydroxy steht,
sowie ihre Salze, Solvate und Solvate der Salze.

Bevorzugter Gegenstand der vorliegenden Erfindung sind Verbindungen der Formel (XXXI) wie oben angegeben, in welcher
- L¹: für eine Bindung steht,
- B: für eine Bindung steht,
- L²: für lineares (C₂-C₆)-Alkandiyl oder für eine Gruppe der Formel 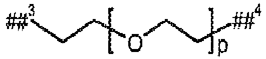 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
- D: für eine Gruppe der Formel 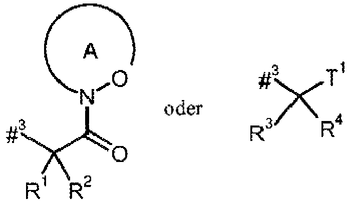 steht, wobei
#³ die Verknüpfungsstelle mit dem Stickstoffatom kennzeichnet,
R¹ für Wasserstoff steht,
R² für Benzyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 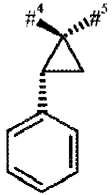 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 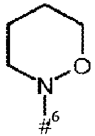 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff steht,
R⁴ für Benzyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 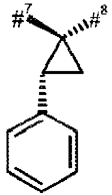 bilden, worin
#⁷ die Verknüpfungsslelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷ oder -C(=O)-NR⁸R⁹ steht,
worin
R⁷ für Wasserstoff steht,
R⁸ für Wasserstoff steht,
R⁹ für Wasserstoff steht,
- R³⁵: für Methyl steht,
sowie ihre Salze, Solvate und Solvate der Salze.

Bevorzugter Gegenstand der vorliegenden Erfindung sind Verbindungen der Fonnel (XXXI) wie oben angegeben, in welcher
- L¹: für eine Bindung steht,
- B: für eine Bindung steht,
- L²: für lineares (C₂-C₆)-Alkandiyl oder für eine Gruppe der Formel 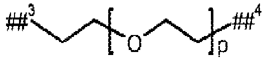 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
- D: für eine Gruppe der Formel 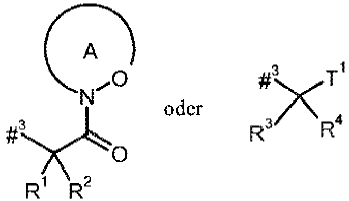 steht, wobei
#³ die Verknüpfungsstelle mit dem Stickstoffatom kennzeichnet,
R¹ für Wasserstoff steht,
R² für 4-Hydroxybenzyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 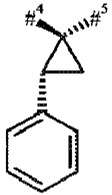 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 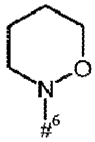 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff steht,
R⁴ für 4-Hydroxybenzyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 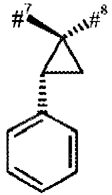 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷ oder -C(=O)-NR⁸R⁹ steht,
worin
R⁷ für Wasserstoff steht,
R⁸ für Wasserstoff steht,
R⁹ für Wasserstoff steht,
- R³⁵: für Methyl steht,
sowie ihre Salze, Solvate und Solvate der Salze.

Bevorzugter Gegenstand der vorliegenden Erfindung sind Verbindungen der Formeln (XXXa) und (XXXI) ausgewählt aus der Gruppe:
*N*-[6-(3-{[(2*R*)-2-Amino-2-carboxyethyl]sulfanyl}-2,5-dioxopyrrolidin-1-yl)hexyl]-*N*-methyl-L-valyl-*N*-[(3*R*,4*S*,5*S*)-1-{(2*S*)-2-[(1*R*,2*R*)-3-{[(1*S*)-1-carboxy-2-(1*H*-indol-3-yl)ethyl]amino}-1-methoxy-2-methyl-3-oxopropyl]pyrrolidin-1-yl}-3-methoxy-5-methyl-1-oxoheptan-4-yl]-*N-*methyl-L-valinamid,
*N*-[6-(3-{[(2*R*)-2-Amino-2-carboxyethyl]sulfanyl}-2,5-dioxopyrrolidin-1-yl)hexyl]-*N*-methyl-L-valyl-*N*-[(3*R*,4*S*,5*S*)-1-{(2*S*)-2-[(1*R*,2*R*)-3-{[(2*S*)-3-(1*H*-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid,
N-(6-{[(5S)-5-Amino-5-carboxypentyl]amino}-6-oxohexyl)-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-1-yl]amino}-1-methoxy-2-methyl-3-oxopropyl]pyrrolidin-1-yl}-3-methoxy-5-methyl-1-oxoheptan-4-yl]-N-methyl-L-valinamid-Trifluoracetat,
N-(6-{[(5S)-5-Amino-5-carboxypentyl]amino}-6-oxohexyl)-N-methyl-L-valyl-N-[(3R,4S,5S)-1-{(2S)-2-[(1R,2R)-3-{[(1S)-1-carboxy-2-(1H-indol-3-yl)ethyl]amino}-1-methoxy-2-methyl-3-oxopropyl]pyrrolidin-1-yl}-3-methoxy-5-methyl-1-oxoheptan-4-yl]-N-methyl-L-valinamid,
sowie ihre Salze, Solvate und Solvate der Salze.

Ein weiterer Gegenstand der vorliegenden Erfindung sind Binder-Wirkstoff Konjugate der allgemeinen Formel (I) 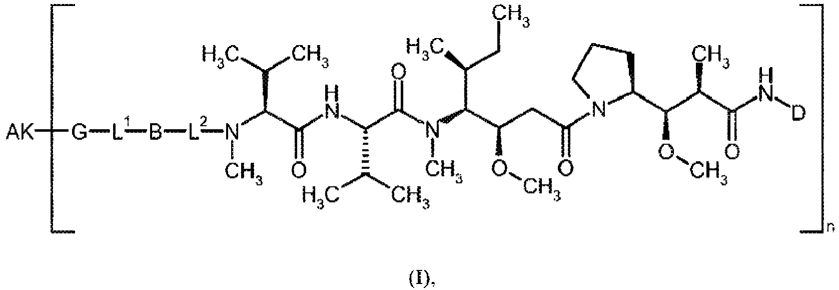 in welcher
- n: für eine Zahl von 1 bis 50 steht,
- AK: für einen Binder steht,
die Gruppe §-G-L¹-B-L²-§§ für einen Linker steht,
wobei
§ die Verknüpfungsstelle mit der Gruppe AK kennzeichnet und
§§ die Verknüpfungsstelle mit dem Stickstoffatom kennzeichnet,
- D: für eine Gruppe der Formel 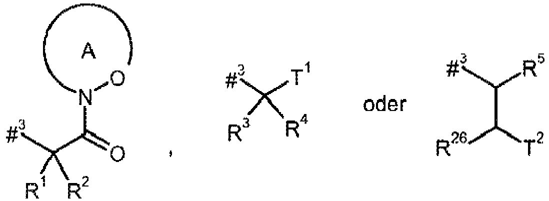 steht, wobei
#³ die Verknüpfungsstelle mit dem Stickstoffatom kennzeichnet,
R¹ für Wasserstoff steht,
R² für 1-Hydroxyethyl, Benzyl, 1-Phenylethyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 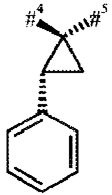 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 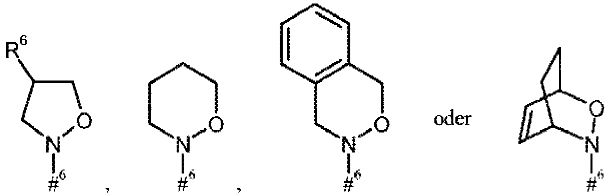 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff steht,
R⁴ für 1-Hydroxyethyl, Benzyl, 1-Phenylethyl oder 1*H*-Indol-3 -ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 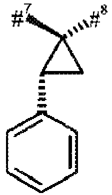 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹, -C(=O)-NH-NH-R¹⁰ oder -CH₂-O-R¹¹ steht,
worin
R⁷ für Wasserstoff, Methyl, Ethyl, *n*-Propyl, *tert*-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
oder
R⁸ und R⁹ bilden zusammen mit dem Stickstoffatom, an das sie gebunden sind, einen 4- bis 7-gliedrigen Heterocyclus,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der Formel 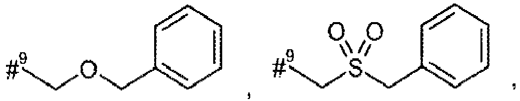 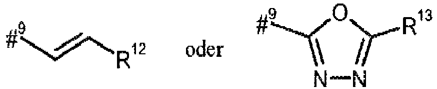 steht, worin
#⁹ die Verknüpfungsstelle mit -CHC(R²⁶)-T² kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
R²⁶ für Wasserstoff oder Hydroxy steht,
T² für Phenyl, Benzyl, 1*H*-Indol-3-yl oder 1*H*-Indol-3-ylmethyl steht,
sowie ihre Salze, Solvate und Solvate der Salze.

Bevorzugter Gegenstand der Erfindung sind Binder-Wirkstoff Konjugate der allgemeinen Formel (I), in welcher
- n: für eine Zahl von 1 bis 50 steht,
- AK: für AK₁ oder AK₂ steht
wobei
AK₁ für einen Binder, der über ein Schwefelatom des Binders an die Gruppe G gebunden ist,
AK₂ für einen Binder, der über ein Stickstoffatom des Binders an die Gruppe G gebunden ist,
- G: für den Fall, dass AK = AK₁ ist, für eine Gruppe der Formel 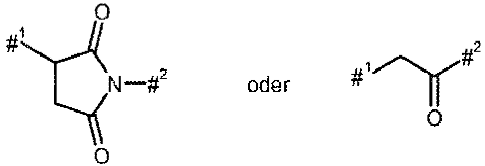 steht, wobei
#¹ die Verknüpfungsstelle mit dem Schwefelatom des Binders kennzeichnet,
#² die Verknüpfungsstelle mit der Gruppe L¹ kennzeichnet,
oder
für den Fall, dass AK = AK₂ ist, für Carbonyl steht,
- L¹: für eine Bindung, lineares (C₁-C₁₀)-Alkandiyl oder für eine Gruppe der Formel 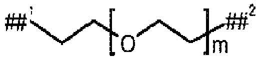 steht, wobei
m für eine Zahl von 2 bis 6 steht,
##¹ die Verknüpfungsstelle mit der Gruppe G kennzeichnet,
##² die Verknüpfungsstelle mit der Gruppe B kennzeichnet,
wobei (C₁-C₁₀)-Alkandiyl mit 1 bis 4 Substituenten Methyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
- B: für eine Bindung oder eine Gruppe der Formel 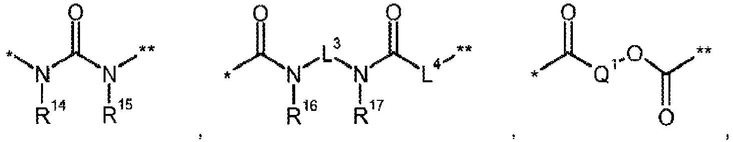 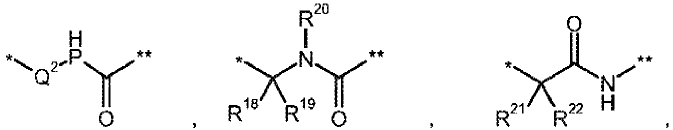 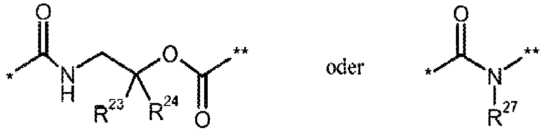 steht, wobei
* die Verknüpfungsstelle mit L¹ kennzeichnet,
** die Verknüpfungsstelle mit L² kennzeichnet,
P für O oder NH steht,
L³ für eine Bindung oder (C₂-C₄)-Alkandiyl steht,
L⁴ für eine Bindung oder eine Gruppe der Formel 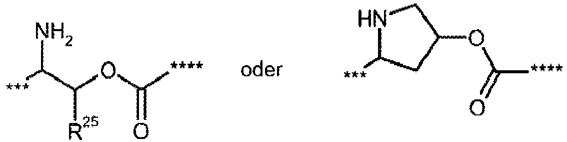 steht, worin
*** die Verknüpfungsstelle mit der Carbonylgruppe kennzeichnet,
**** die Verknüpfungsstelle mit L² kennzeichnet,
R²⁵ für Wasserstoff oder Methyl steht,
Q¹ für einen 4- bis 7-gliedrigen Heterocyclus steht,
Q² für einen 3- bis 7-gliedrigen Carbocyclus oder einen 4- bis 7-gliedrigen Heterocyclus steht,
R¹⁴ für Wasserstoff oder (C₁-C₄)-Alkyl steht,
R¹⁵ für Wasserstoff oder (C₁-C₄)-Alkyl steht,
oder
R¹⁴ und R¹⁵ zusammen mit den Atomen, an die sie gebunden sind, einen 5- oder 6-gliedrigen Heterocyclus bilden,
R¹⁶ für Wasserstoff oder (C₁-C₄)-Alkyl steht,
R¹⁷ für Wasserstoff oder (C₁-C₄)-Alkyl steht,
oder
R¹⁶ und R¹⁷ zusammen mit den Atomen, an die sie gebunden sind, einen 5- oder 6-gliedrigen Heterocyclus bilden,
R¹⁸ für Wasserstoff oder (C₁-C₄)-Alkyl steht,
R¹⁹ für Wasserstoff oder die Seitengruppe einer natürlichen α-Aminosäure oder ihrer Homologe oder Isomere steht,
R²⁰ für Wasserstoff oder (C₁-C₄)-Alkyl steht,
oder
R¹¹ und R²⁰ zusammen mit den Atomen, an die sie gebunden sind, einen Pyrrolidinylring bilden,
R²¹ für Wasserstoff oder (C₁-C₄)-Alkyl steht,
R²² für Wasserstoff oder (C₁-C₄)-Alkyl steht,
oder
R²¹ und R²² zusammen mit den Atomen, an die sie gebunden sind, einen 3- bis 7-gliedrigen Carbocyclus bilden,
R²¹ für (C₁-C₄)-Alkyl steht,
R²⁴ für Wasserstoff oder (C₁-C₄)-Alkyl steht,
R²⁷ für Wasserstoff oder (C₁-C₄)-Alkyl steht,
- L²: für lineares (C₂-C₁₀)-Alkandiyl oder für eine Gruppe der Formel 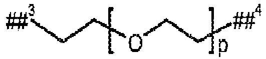 steht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
wobei (C₂-C₁₀)-Alkandiyl mit 1 bis 4 Substituenten Methyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
- D: die oben angegebenen Bedeutungen aufweist,
sowie ihre Salze, Solvate und Solvate der Salze.

Bevorzugter Gegenstand der Erfindung sind Binder-Wirkstoff Konjugate der allgemeinen Formel (I),
in welcher
- n: für eine Zahl von 1 bis 50 steht,
- AK: für AK₁ oder AK₂ steht
wobei AK₁ für einen Antikörper oder ein Antigen-bindendes Antikörperfragment steht und über ein Schwefelatom an die Gruppe G gebunden sind,
AK₂ für einen Antikörper oder ein Antigen-bindendes Antikörperfragment steht und über ein Stickstoffatom an die Gruppe G gebunden sind,
- G, L¹, B, L² und D: die oben angegebenen Bedeutungen aufweisen,
sowie ihre Salze, Solvate und Solvate der Salze.

Bevorzugter Gegenstand der vorliegenden Erfindung sind Binder-Wirkstoff Konjugate der allgemeinen Formel (I), in welcher
- n: für eine Zahl von 1 bis 20 steht,
- AK: für AK₁ oder AK₂ steht
wobei
AK₁ für einen Antikörper oder ein Antigen-bindendes Antikörperfragment steht, der bzw. das an C4.4a bindet und über das Schwefelatom eines Cystein-Restes des Binders an die Gruppe G gebunden ist,
AK₂ für einen Antikörper oder ein Antigen-bindendes Antikörperfragment steht, der bzw. das an C4.4a bindet und über die NH-Seitengruppe eines Lysin-Restes des Binders an die Gruppe G gebunden ist,
- G: für den Fall, dass AK = AK₁ ist, für eine Gruppe der Formel 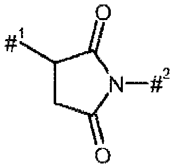 steht, wobei
#¹ die Verknüpfungsstelle mit dem Cystein-Rest des Binders kennzeichnet,
#² die Verknüpfungsstelle mit der Gruppe L¹ kennzeichnet,
oder
für den Fall, dass AK = AK₂ ist, für Carbonyl steht,
- L¹: für eine Bindung, lineares (C₂-C₆)-Alkandiyl oder für eine Gruppe der Formel 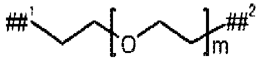 steht, wobei
m für eine Zahl von 2 bis 6 steht,
##¹ die Verknüpfungsstelle mit der Gruppe G kennzeichnet,
##² die Verknüpfungsstelle mit der Gruppe B kennzeichnet,
wobei (C₂-C₆)-Alkandiyl mit 1 oder 2 Substituenten Methyl substiuiert sein kann,
- B: für eine Bindung oder eine Gruppe der Formel 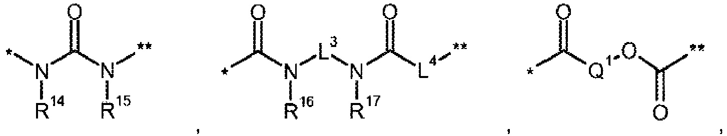 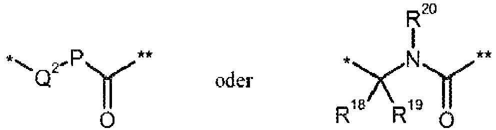 steht, wobei
* die Verknüpfungsstelle mit L¹ kennzeichnet,
** die Verknüpfungsstelle mit L² kennzeichnet,
P für O oder NH steht,
L³ für eine Bindung oder Ethan-1,2-diyl steht,
L⁴ für eine Bindung oder eine Gruppe der Formel 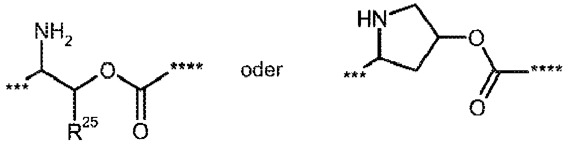 steht, worin
*** die Verknüpfungsstelle mit der Carbonylgruppe kennzeichnet,
**** die Verknüpfungsstelle mit L² kennzeichnet,
R²⁵ für Methyl steht,
Q¹ für einen 4- bis 6-gliedrigen Carbocyclus oder Piperidin-1,4-diyl steht,
Q² für Cyclopentyl oder Cyclohexyl steht,
R¹⁴ für Wasserstoff steht,
R¹⁵ für Wasserstoff steht,
R¹⁶ für Wasserstoff oder Methyl steht,
R¹⁷ für Wasserstoff oder Methyl steht,
oder
R¹⁶ und R¹⁷ zusammen mit den Atomen, an die sie gebunden sind, einen Piperazinylring bilden,
R¹⁸ für Wasserstoff steht,
R¹⁹ für Wasserstoff, Methyl, Propan-2-yl, 2-Methylpropan-1-yl oder 1-Methylpropan-1-yl steht,
R²⁰ für Wasserstoff oder Methyl steht,
oder
R¹⁹ und R²⁰ zusammen mit den Atomen, an die sie gebunden sind, einen Pyrrolidinylring bilden,
- L²: für lineares (C₂-C₆)-Alkandiyl oder für eine Gruppe der Formel 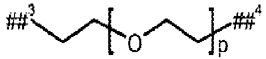 steht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
wobei (C₂-C₆)-Alkandiyl mit I oder 2 Substituenten Methyl substiuiert sein kann,
- D: für eine Gruppe der Formel 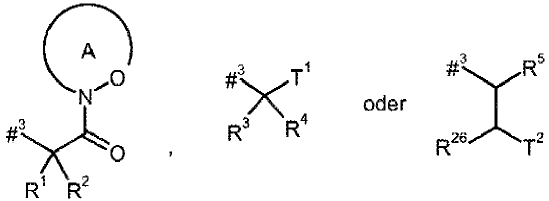 steht, wobei
#³ die Verknüpfungsstelle mit dem Stickstoffatom kennzeichnet.
R¹ für Wasserstoff steht,
R² für 1-Hydroxyethyl, Benzyl, 1-Phenylethyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 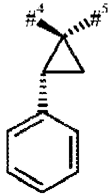 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 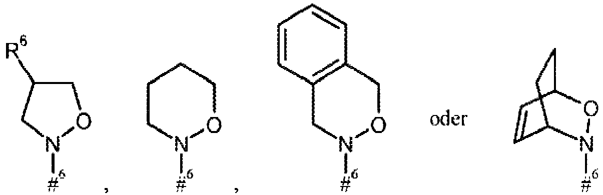 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff steht,
R⁴ für 1-Hydroxyethyl, Benzyl, 1-Phenylethyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 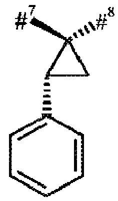 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹, -C(=O)-NH-NH-R¹⁰ oder
-CH₂-O-R¹¹ steht,
worin
R⁷ für Wasserstoff, Methyl, Ethyl, *n*-Propyl, *tert*.-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
oder
R⁸ und R⁹ bilden zusammen mit dem Stickstoffatom, an das sie gebunden sind,
einen 4- bis 7-gliedrigen Heterocyclus,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der Formel 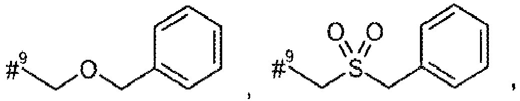 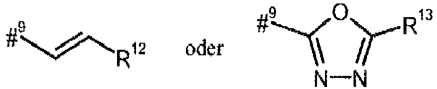 steht, worin
#⁹ die Verknüpfungsstelle mit CHC(R²⁶)-T² kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
R²⁶ für Wasserstoff oder Hydroxy steht,
T² für Phenyl, Benzyl, 1*H*-Indol-3-yl oder 1*H*-Indol-3-ylmethyl steht,
sowie ihre Salze, Solvate und Solvate der Salze.

Besonders bevorzugter Gegenstand der vorliegenden Erfindung sind Binder-Wirkstoff Konjugate der allgemeinen Formel (I), in welcher
- n: für eine Zahl von 1 bis 10 steht,
- AK: für AK₁ oder AK₂ steht
wobei
AK₁ für einen Antikörper, der die sechs CDR-Sequenzen eines in Tabelle 2 aufgeführten Antikörpers, die variable leichte und variable schwere Kette eines in Tabelle 2 aufgeführten Antikörpers oder die leichte und schwere Kette eines in Tabelle 2 aufgeführten Antikörpers umfasst, steht, der über das Schwefelalom eines Cystein-Restes des Binders an die Gruppe G gebunden ist,
AK₂ für einen Antikörper, der die sechs CDR-Sequenzen eines in Tabelle 2 aufgeführten Antikörpers, die variable leichte und variable schwere Kette eines in Tabelle 2 aufgeführten Antikörpers oder die leichte und schwere Kette eines in Tabelle 2 aufgeführten Antikörpers umfasst, steht, der über die NH-Seitengruppe eines Lysin-Restes des Binders an die Gruppe G gebunden ist,
- G: für den Fall, dass AK = AK₁ ist, für eine Gruppe der Formel 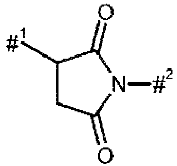 steht, wobei
#¹ die Verknüpfungsstelle mit dem Cystein-Rest des Binders kennzeichnet,
#² die Verknüpfungsstelle mit der Gruppe L¹ kennzeichnet,
oder
für den Fall, dass AK = AK₂ ist, für Carbonyl steht,
- L¹: für eine Bindung, lineares (C₂-C₆)-Alkandiyl oder für eine Gruppe der Formel 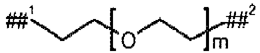 steht, wobei
m für eine Zahl von 2 oder 3 steht,
##¹ die Verknüpfungsstelle mit der Gruppe G kennzeichnet,
##² die Verknüpfungsstelle mit der Gruppe B kennzeichnet,
wobei (C₂-C₆)-Alkandiyl mit 1 oder 2 Substituenten Methyl substiuiert sein kann,
- B: für eine Bindung oder eine Gruppe der Formel 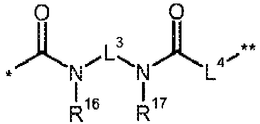 steht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Bindung oder eine Gruppe der Formel 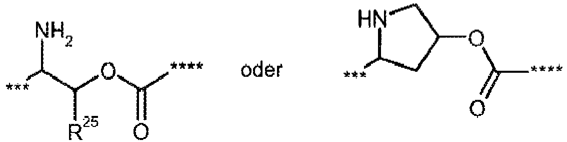 steht, worin
*** die Verknüpfungsstelle mit der Carbonylgruppe kennzeichnet,
**** die Verknüpfungsstelle mit L² kennzeichnet,
R²⁵ für Methyl steht,
R¹⁶ für Wasserstoff oder Methyl steht,
R¹⁷ für Wasserstoff oder Methyl steht,
oder
R¹⁶ und R¹⁷ zusammen mit den Atomen, an die sie gebunden sind, einen Piperazinylring bilden,
- L²: für lineares (C₂-C₆)-Alkandiyl steht,
- D: für eine Gruppe der Formel 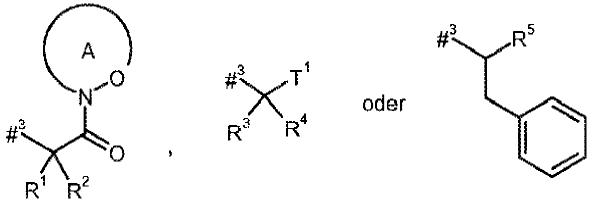 steht, wobei
#³ die Verknüpfungsstelle mit dem Stickstoffatom kennzeichnet,
R¹ für Wasserstoff steht,
R² für 1-Hydroxyethyl, Benzyl, 1-Phenylethyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 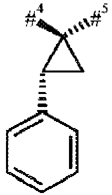 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 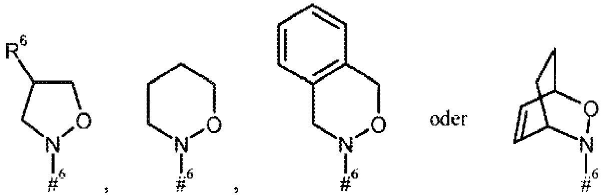 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff steht,
R⁴ für Benzyl, 1-Phenylethyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 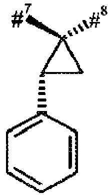 bilden, worin
#⁷ die Verknüpfungsstelle mit dem angrenzenden Stickstoffatom kennzeichnet,
#⁸ die Verknüpfungsstelle mit der Gruppe T kennzeichnet,
T¹ für eine Gruppe der Formel -C(=O)-OR⁷, -C(=O)-NR⁸R⁹ oder -CH₂-O-R¹¹ steht,
worin
R⁷ für Wasserstoff, Methyl, Ethyl, *n*-Propyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
R¹¹ für Benzyl, das in der Phenylgruppe mit Methoxycarbonyl oder Carboxyl substituiert sein kann, steht,
- R⁵: für Wasserstoff oder eine Gruppe der Formel 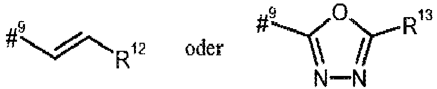 steht, worin
#⁹ die Verknüpfungsstelle mit -CHC(R²⁶)Phenyl kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
sowie ihre Salze, Solvate und Solvate der Salze.

Besonders bevorzugter Gegenstand der vorliegenden Erfindung sind Binder-Wirkstoff Konjugate der allgemeinen Formel (I), in welcher
- n: für eine Zahl von 1 bis 10 steht,
- AK: für AK₁ oder AK₂ steht
wobei
AK₁ für einen Antikörper, der die sechs CDR-Sequenzen des Antikörpers B01-3, B01-10 oder D02-6, die variable leichte und variable schwere Kette des Antikörpers B01-3, B01-10 oder D02-6 oder die leichte und schwere Kette des Antiköpers B01-3, B01-10 oder D02-6 umfasst, steht, der über das Schwefelatom eines Cystein-Restes des Binders an die Gruppe G gebunden ist,
AK₂ für einen Antikörper, der die sechs CDR-Sequenzen des Antikörpers B01-3, B01-10, R431-B01 oder D02-6, die variable leichte und variable schwere Kette des Antikörpers B01-3, B01-10, M31-B01 oder D02-6 oder die leichte und schwere Kette des Antiköpers B01-3, B01-10, M31-B01 oder D02-6 umfasst, steht, der über die NH-Seitengruppe eines Lysin-Restes des Binders an die Gruppe G gebunden ist,
- G: für den Fall, dass AK = AK₁ ist, für eine Gruppe der Formel 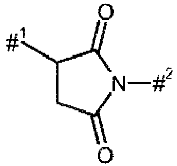 steht, wobei
#¹ die Verknüpfungsstelle mit dem Cystein-Rest des Binders kennzeichnet,
#² die Verknüpfungsstelle mit der Gruppe L¹ kennzeichnet,
oder
für den Fall, dass AK = AK₂ ist, für Carbonyl steht,
- L¹: für eine Bindung, lineares (C₂-C₆)-Alkandiyl oder für eine Gruppe der Formel 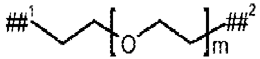 sieht, wobei
m für eine Zahl von 2 oder 3 steht,
##¹ die Verknüpfungsstelle mit der Gruppe G kennzeichnet,
##² die Verknüpfungsstelle mit der Gruppe B kennzeichnet,
wobei (C₂-C₆)-Alkandiyl mit 1 oder 2 Substituenten Methyl substiuiert sein kann,
- B: für eine Bindung oder eine Gruppe der Formel 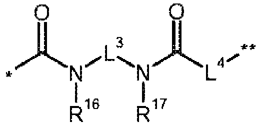 steht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Bindung oder eine Gruppe der Formel 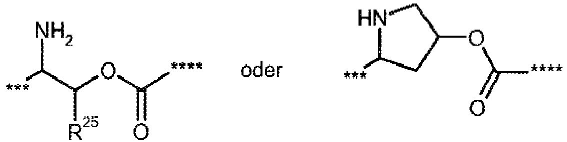 steht, worin
*** die Verknüpfungsstelle mit der Carbonylgruppe kennzeichnet,
**** die Verknüpfungsstelle mit L² kennzeichnet,
R²⁵ für Methyl steht,
R¹⁶ für Wasserstoff oder Methyl steht,
R¹⁷ für Wasserstoff oder Methyl steht,
oder
R¹⁶ und R¹⁷ zusammen mit den Atomen, an die sie gebunden sind, einen Piperazinylring bilden,
- L²: für lineares (C₂-C₆)-Alkandiyl steht,
- D: für eine Gruppe der Formel 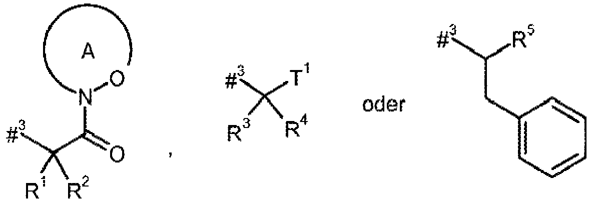 steht, wobei
#³ die Verknüpfungsstelle mit dem Stickstoffatom kennzeichnet,
R¹ für Wasserstoff steht,
R² für 1-Hydroxyethyl, Benzyl, 1-Phenylethyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 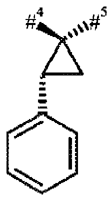 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 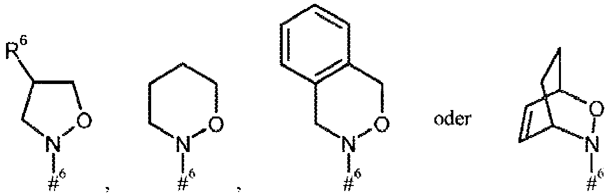 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff steht,
R⁴ für Benzyl, 1-Phenylethyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 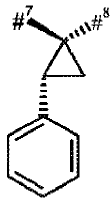 bilden, worin
#⁷ die Verknüpfungsstelle mit dem angrenzenden Stickstoffatom kennzeichnet,
#⁸ die Verknüpfungsstelle mit der Gruppe T kennzeichnet,
T¹ für eine Gruppe der Formel -C(=O)-OR⁷, -C(=O)-NR⁸R⁹ oder -CH₂-O-R¹¹ steht,
worin
R⁷ für Wasserstoff, Methyl, Ethyl, *n*-Propyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
R¹¹ für Benzyl, das in der Phenylgruppe mit Methoxycarbonyl oder Carboxyl substituiert sein kann, steht,
- R⁵: für Wasserstoff oder eine Gruppe der Formel 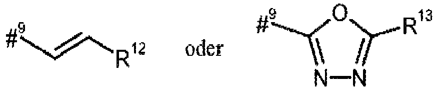 steht, worin
#⁹ die Verknüpfungsstelle mit -CHC(R²⁶)Phenyl kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
sowie ihre Salze, Solvate und Solvate der Salze.

Ein weiterer Gegenstand der vorliegenden Erfindung sind Verbindungen der Formel (XXX) 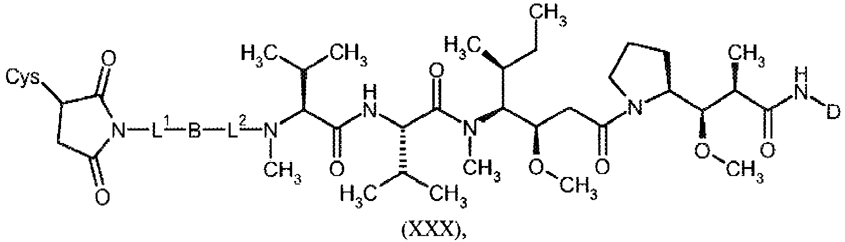 in welcher
- Cys: für einen Cystein-Rest, der über das Schwefelatom der Seitenkette an ein Kohlenstoffatom des Succinimids gebunden ist, steht
- L¹: für eine Bindung, lineares (C₁-C₁₀)-Alkandiyl oder für eine Gruppe der Formel 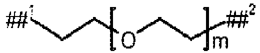 steht, wobei
m für eine Zahl von 2 bis 6 steht,
##¹ die Verknüpfungsstelle mit der Gruppe G kennzeichnet,
##² die Verknüpfungsstelle mit der Gruppe B kennzeichnet,
wobei (C₁-C₁₀)-Alkandiyl mit 1 bis 4 Substituenten Methyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
- B: für eine Bindung oder eine Gruppe der Formel 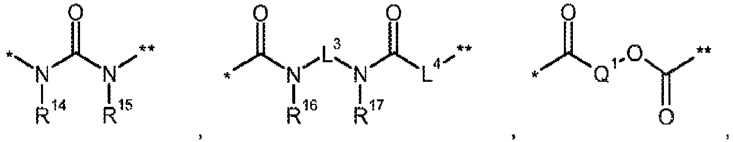 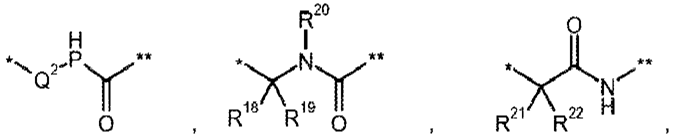 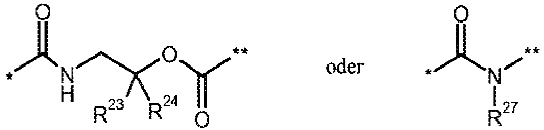 steht, wobei
* die Verknüpfungsstelle mit L¹ kennzeichnet,
** die Verknüpfungsstelle mit L² kennzeichnet,
P für O oder NH steht,
L³ für eine Bindung, oder (C₂-C₄)-Alkandiyl steht,
L⁴ für eine Bindung oder eine Gruppe der Formel 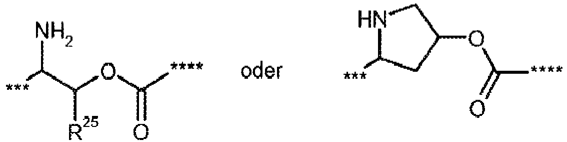 steht, worin
*** die Verknüpfungsstelle mit der Carbonylgruppe kennzeichnet,
**** die Verknüpfungsstelle mit L² kennzeichnet,
R²⁵ für Wasserstoff oder Methyl steht,
Q¹ für einen 3- bis 7-gliedrigen Carbocyclus oder einen 4- bis 7-gliedrigen Aza-Heterocyclus steht,
Q² für einen 3- bis 7-gliedrigen Carbocyclus oder einen 4- bis 7-gliedrigen Aza-Heterocyclus steht,
R¹⁴ für Wasserstoff oder (C₁-C₄)-Alkyl steht,
R¹⁵ für Wasserstoff oder (C₁-C₄)-Alkyl steht,
oder
R¹⁴ und R¹⁵ zusammen mit den Atomen, an die sie gebunden sind, einen 5- oder 6-gliedrigen Heterocyclus bilden,
R¹⁶ für Wasserstoff oder (C₁-C₄)-Alkyl steht,
R¹⁷ für Wasserstoff oder (C₁-C₄)-Alkyl steht,
oder
R¹⁶ und R¹⁷ zusammen mit den Atomen, an die sie gebunden sind, einen 5- oder 6-gliedrigen Heterocyclus bilden,
R¹⁸ für Wasserstoff oder (C₁-C₄)-Alkyl steht,
R¹⁹ für Wasserstoff oder die Seitengruppe einer natürlichen α-Aminosäure oder ihrer Homologe oder Isomere steht,
R²⁰ für Wasserstoff oder (C₁-C₄)-Alkyl steht,
oder
R¹⁹ und R²⁰ zusammen mit den Atomen, an die sie gebunden sind, einen Pyrrolidinylring bilden,
R²¹ für Wasserstoff oder (C₁-C₄)-Alkyl steht,
R²² für Wasserstoff oder (C₁-C₄)-Alkyl steht,
oder
R²¹ und R²² zusammen mit den Atomen, an die sie gebunden sind, einen 3- bis 7-gliedrigen Carbocyclus bilden,
R²³ für (C₁-C₄)-Alkyl steht,
R²⁴ für Wasserstoff oder (C₁-C₄)-Alkyl steht,
R²⁷ für Wasserstoff oder (C₁-C₄)-Alkyl steht,
- L²: für lineares (C₂-C₁₀)-Alkandiyl oder für eine Gruppe der Formel 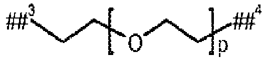 sieht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
wobei (C₂-C₁₀)-Alkandiyl mit 1 bis 4 Substituenten Methyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
- D: für eine Gruppe der Formel 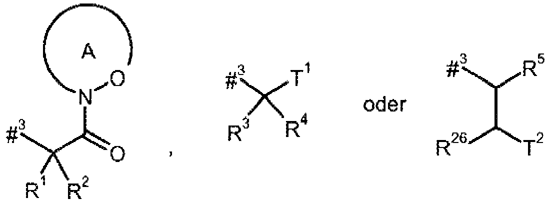 steht, wobei
#³ die Verknüpfungsstelle mit dem Stickstoffatom kennzeichnet,
R¹ für Wasserstoff steht,
R² für 1-Hydroxyethyl, Benzyl, 1-Phenylethyl oder 1*H*-Indol-3-ylmethyl steht,
oder
R¹ und R² zusammen mit dem Kohlenstoffatom, an das sie gebunden sind, eine (1*S,*2*R*)-2-Phenylcyclopropan-1,1-diyl-Gruppe der Formel 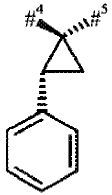 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppicrung für einen mono- oder bi-cyclischen, gegebenenfalls substituierten Heterocyclus der Formel 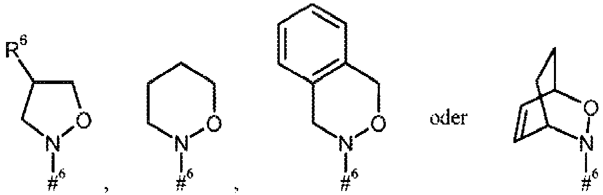 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff steht,
R⁴ für 1-Hydroxyethyl, Benzyl, 1-Phenylethyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 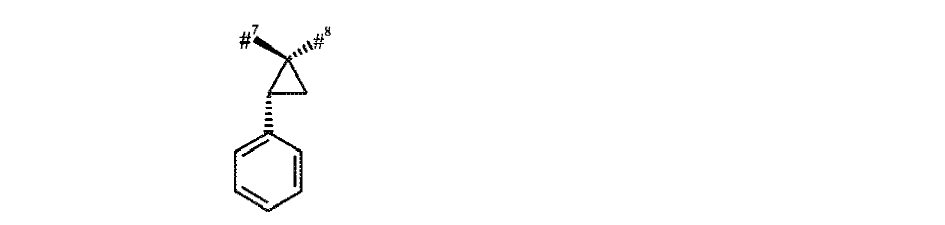 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹, -C(=O)-NH-NH-R¹⁰ oder-CH₂-O-R¹¹ steht,
worin
R⁷ für Wasserstoff, Methyl, Ethyl, *n*-Propyl, *tert*.-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
oder
R⁸ und R⁹ bilden zusammen mit dem Stickstoffatom, an das sie gebunden sind,
einen 4- bis 7-gliedrigen Heterocyclus,
R¹⁰ für Benzoyl steht,
R¹¹ für Benzyl, das in der Phenylgruppe mit Methoxycarbonyl oder Carboxyl substituiert sein kann, steht,
- R⁵: für Wasserstoff, Methyl oder eine Gruppe der Formel 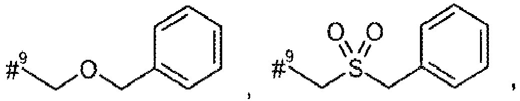 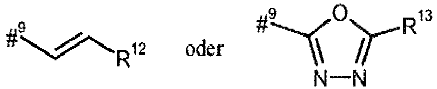 steht, worin
#⁹ die Verknüpfungsstelle mit CHC(R²⁶)-T² kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
- R²⁶: für Wasserstoff oder Hydroxy steht,
- T²: für Phenyl, Benzyl, 1*H*-Indol-3-yl oder 1*H*-Indol-3-ylmethyl steht,
sowie ihre Salze, Solvate und Solvate der Salze.

Besonders bevorzugt im Rahmen der vorliegenden Erfindung sind weiterhin auch Verbindungen der Formel (XXX), in welcher
- Cys: für einen Cystein-Rest steht, der über das Schwefelatom der Seitenkette über ein Kohlenstoffatom des Succinimids gebunden ist, steht
- L¹: für eine Bindung, lineares (C₂-C₆)-Alkandiyl oder für eine Gruppe der Formel 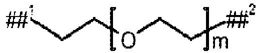 steht, wobei
m für eine Zahl von 2 bis 6 steht,
##¹ die Verknüpfungsstelle mit der Gruppe G kennzeichnet,
##² die Verknüpfungsstelle mit der Gruppe B kennzeichnet,
wobei (C₂-C₆)-Alkandiyl mit 1 oder 2 Substituenten Methyl substiuiert sein kann,
- B: für eine Bindung oder eine Gruppe der Formel 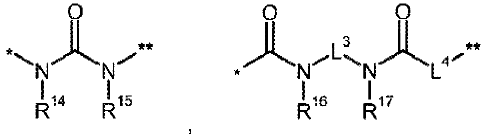 oder 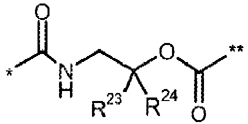 sieht, wobei
* die Verknüpfungsstelle mit L¹ kennzeichnet,
** die Verknüpfungsstelle mit L² kennzeichnet,
L³ für eine Bindung oder Ethan-1,2-diyl steht,
**L**⁴ für eine Bindung steht,
**R**¹⁴ für Wasserstoff steht,
**R**¹⁵ für Wasserstoff steht,
**R**¹⁶ für Wasserstoff oder Methyl steht,
**R**¹⁷ für Wasserstoff oder Methyl steht,
oder
**R**¹⁶ und R¹⁷ zusammen mit den Atomen, an die sie gebunden sind, einen Piperazinylring bilden,
**R**²³ für Methyl steht,
**R**²⁴ für Wasserstoff oder Methyl steht,
- L²: für lineares (C₂-C₆)-Alkandiyl oder für eine Gruppe der Formel 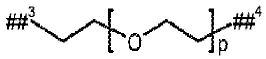 steht, wobei
p für eine Zahl 2 oder 3 steht,
##³ die Verknüpfungsstelle mit der Gruppe B kennzeichnet,
##⁴ die Verknüpfungsstelle mit dem Stickstoffatom kennzeichnet,
- D: für eine Gruppe der Formel 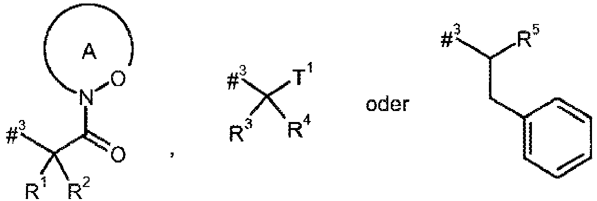 steht, wobei
#³ die Verknüpfungsstelle mit dem Stickstoffatom kennzeichnet,
R¹ für Wasserstoff steht,
R² für I-Hydroxyethyl, Benzyl, 1-Phenylethyl oder 1*H*-Indol-3-ylmethyl steht,
oder
R¹ und R¹ zusammen mit dem Kohlenstoffatom, an das sie gebunden sind, eine (1*S*,2*R*)-2-Phenylcyclopropan-1,1-diyl-Gruppe der Formel 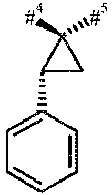 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischcn, gegebenenfalls substituierten Heterocyclus der Formel 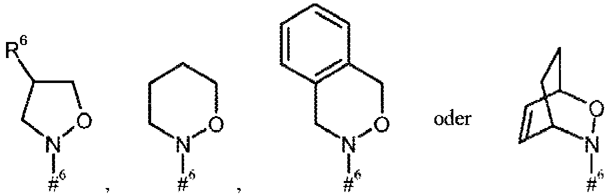 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff steht,
R⁴ für Benzyl, 1-Phenylethyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 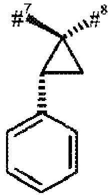 bilden, worin
#⁷ die Verknüpfungsstelle mit dem angrenzenden Stickstoffatom kennzeichnet,
#⁸ die Verknüpfungsstelle mit der Gruppe T kennzeichnet,
T¹ für eine Gruppe der Formel -C(=O)-OR⁷, -C(=O)-NR⁸R⁹, -C(=O)-NH-NH-R¹⁰ oder -CH₂-O-R¹¹ steht,
worin
**R**⁷ für Wasserstoff, Methyl, Ethyl, *n*-Propyl, Benzyl oder Adamantylmethyl steht,
**R**⁸ für Wasserstoff oder Methyl steht,
**R**⁹ für Wasserstoff, Methyl, Ethyl, *n*-Propyl oder Benzyl steht,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff oder eine Gruppe der Formel 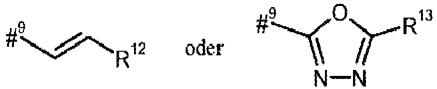 steht, worin
#⁹ die Verknüpfungsstelle mit -CHC(R²⁶)Phenyl kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
sowie ihre Salze, Solvate und Solvate der Salze.

Bevorzugt im Rahmen der vorliegenden Erfindung sind auch Verbindungen der Formel (Ia), in welcher
n = 1-20, besonders bevorzugt n = 1-10 und ganz besonders bevorzugt n = 2-8 bedeutet.

Bevorzugt sind im Rahmen der vorliegenden Erfindung sind auch Verbindungen der Formel (Ia), in welcher
- AK: für AK₁ steht
wobei
AK₁ für einen Antikörper oder ein Antigen-bindendes Antikörperfragment steht, der bzw. das an C4.4a bindet und über das Schwefelatom eines Cystein-Restes des Binders an die Gruppe G gebunden ist,
- G: für eine Gruppe der Formel 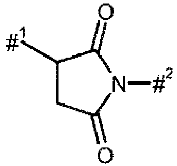 steht, wobei
#¹ die Verknüpfungsstelle mit dem Cystein-Rest des Binders kennzeichnet,
#² die Verknüpfungsstelle mit der Gruppe L¹ kennzeichnet,
und
n, L¹, B, L², D und R³⁵ die oben angegebenen Bedeutungen aufweisen,
sowie ihre Salze, Solvate und Solvate der Salze.

Bevorzugt sind im Rahmen der vorliegenden Erfindung sind auch Verbindungen der Formel (Ia), in welcher
- AK für AK₂: steht
wobei
AK₂ für einen Antikörper oder ein Antigen-bindendes Antikörperfragment steht, der bzw. das an C4.4a bindet und über die NH-Seitengruppe eines Lysin-Restes des Binders an die Gruppe G gebunden ist,
- G: für Carbonyl steht,
und
n, L¹, B, L², D und R³⁵ die oben angegebenen Bedeutungen aufweisen,
sowie ihre Salze, Solvate und Solvate der Salze.

Bevorzugt sind im Rahmen der vorliegenden Erfindung sind auch Verbindungen der Formel (la), in welcher
- AK: für AK₁ steht
wobei
AK₁ für einen Antikörper, der die sechs CDR-Sequenzen des Antikörpers B01-3, B01-10 oder D02-6, die variable leichte und variable schwere Kette des Antikörpers B01-3, B01-10 oder D02-6 oder die leichte und schwere Kette des Antiköpers B01-3, B01-10 oder D02-6 umfasst, steht, der über das Schwefelatom eines Cystein-Restes des Binders an die Gruppe G gebunden ist,
- G: für eine Gruppe der Formel 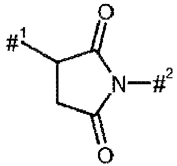 stellt, wobei
#¹ die Verknüpfungsstelle mit dem Cystein-Rest des Binders kennzeichnet,
#² die Verknüpfungsstelle mit der Gruppe L¹ kennzeichnet,
und
n, L¹, B, L², D und R³⁵ die oben angegebenen Bedeutungen aufweisen,
sowie ihre Salze, Solvate und Solvate der Salze.

Bevorzugt sind im Rahmen der vorliegenden Erfindung sind auch Verbindungen der Formel (Ia), in welcher
- AK: für AK₂ steht
wobei
AK₂ für einen Antikörper, der die sechs CDR-Sequenzen des Antikörpers B01-3, B01-10, M31-B01 oder D02-6, die variable leichte und variable schwere Kette des Antikörpers B01-3, B01-10, M31-B01 oder D02-6 oder die leichte und schwere Kette des Antiköpers B01-3, B01-10, M31-B01 oder D02-6 umfasst, steht, der über die NH-Seitengruppe eines Lysin-Restes des Binders an die Gruppe G gebunden ist,
- G: für Carbonyl steht,
und
n, L¹, B, L², D und R³⁵ die oben angegebenen Bedeutungen aufweisen,
sowie ihre Salze, Solvate und Solvate der Salze.

Bevorzugt sind im Rahmen der vorliegenden Erfindung auch Verbindungen der allgemeinen Formel (la), in welcher
- AK: für AK₂ steht
wobei
AK₂ für einen Antikörper, der die sechs CDR-Sequenzen des Antikörpers B01-3, B01-10, M31-B01 oder D02-6, die variable leichte und variable schwere Kette des Antikörpers B01-3, B01-10, M31-B01 oder D02-6 oder die leichte und schwere Kette des Antiköpers B01-3, B01-10, M31-B01 oder D02-6 umfasst, steht, der über die NH-Seitengruppe eines Lysin-Restes des Binders an die Gruppe G gebunden ist,
- G: für Carbonyl steht,
- L¹: für eine Bindung, steht,
- B: für eine Bindung sieht,
- L²: für lineares (C₃-C₆)-Alkandiyl oder für eine Gruppe der Formel 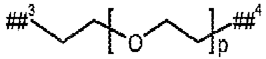 sieht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
n, D und R³⁵ die oben angegebenen Bedeutungen aufweisen,
sowie ihre Salze, Solvate und Solvate der Salze.

Bevorzugt sind im Rahmen der vorliegenden Erfindung auch Verbindungen der allgemeinen Formel (Ia), in welcher
- AK: für AK₁ steht
wobei
AK₁ für einen Antikörper, der die sechs CDR-Sequenzen des Antikörpers B01-3, B01-10 oder D02-6, die variable leichte und variable schwere Kette des Antikörpers B01-3, B01-10 oder D02-6 oder die leichte und schwere Kette des Antiköpers B01-3, B01-10 oder D02-6 umfasst, steht, der über das Schwefelatom eines Cystein-Restes des Binders an die Gruppe G gebunden ist,
- G: für eine Gruppe der Formel 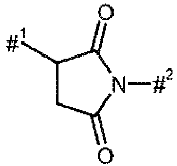 steht, wobei
#¹ die Verknüpfungsstelle mit dem Cystein-Rcst des Binders kennzeichnet,
#² die Verknüpfungsstelle mit der Gruppe L¹ kennzeichnet,
- L¹: für eine Bindung, lineares (C₃-C₅)-Alkandiyl oder eine Gruppe der Formel 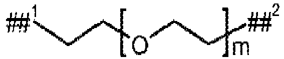 steht, wobei
m für eine Zahl von 2 oder 3 steht,
##¹ die Verknüpfungsstelle mit der Gruppe G kennzeichnet,
##² die Verknüpfungsstelle mit der Gruppe B kennzeichnet,
wobei (C₃-C₅)-Alkandiyl mit 1 oder 2 Substituenten Methyl substiuiert sein kann,
- B: für eine Bindung oder eine Gruppe der Formel 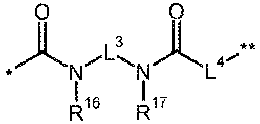 sieht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Bindung oder eine Gruppe der Formel 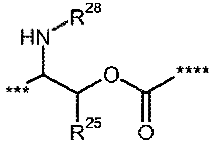 steht, worin
*** die Verknüpfungsstelle mit der Carbonylgruppe kennzeichnet,
**** die Verknüpfungsstelle mit L² kennzeichnet,
R²⁵ für Methyl sieht,
R²⁸ für Wasserstoff, Methylcarbonyl oder tert.-Butyloxycarbonyl steht,
R¹⁶ für Wasserstoff oder Methyl steht,
R¹⁷ für Wasserstoff oder Methyl steht,
oder
R¹⁶ und R¹⁷ zusammen mit den Atomen, an die sie gebunden sind, einen Piperazinylring bilden,
- L²: für lineares (C₃-C-₅)-Alkandiyl oder für eine Gruppe der Formel 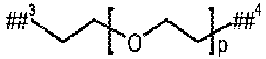 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
und
n, D und R³⁵ die oben angegebenen Bedeutungen aufweisen,
sowie ihre Salze, Solvate und Solvate der Salze.

Bevorzugt sind im Rahmen der vorliegenden Erfindung auch Verbindungen der Formel (Ia), (XXXa) und (XXXI), in welcher
- L¹: für eine Bindung steht,
- B: für eine Bindung steht,
- L²: für lineares (C₃-C₆)-Alkandiyl oder für eine Gruppe der Formel 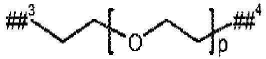 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
und
n, AK. Cys, G, D und R³⁵ die oben angegebenen Bedeutungen aufweisen,
sowie ihre Salze, Solvate und Solvate der Salze.

Bevorzugt sind im Rahmen der vorliegenden Erfindung auch Verbindungen der Formel (Ia), in welcher
- L¹: für lineares (C₁-C₁₀)-Alkandiyl oder eine Gruppe der Formel 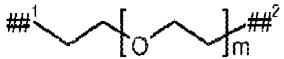 steht, wobei
m für eine Zahl von 2 bis 6 steht,
##¹ die Verknüpfungsstelle mit der Gruppe G kennzeichnet,
##² die Verknüpfungsstelle mit der Gruppe B kennzeichnet,
wobei (C₁-C₁₀)-Alkandiyl mit 1 bis 4 Substituenten unabhängig voneinander ausgewählt aus der Gruppe Methyl, Hydroxy und Benzyl substiuiert sein kann,
B für eine Bindung oder eine Gruppe der Formel 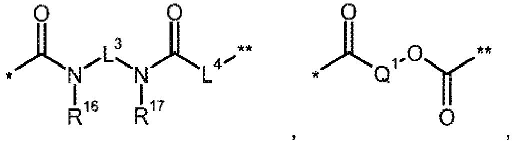 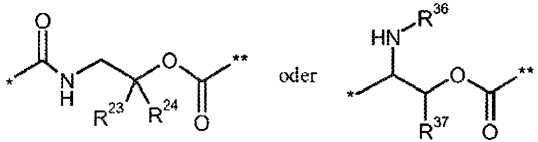 steht, wobei
* die Verknüpfungsstelle mit L¹ kennzeichnet,
** die Verknüpfungsstelle mit L² kennzeichnet,
L³ für eine Bindung oder (C₂-C₄)-Alkandiyl steht,
L⁴ für eine Gruppe der Formel 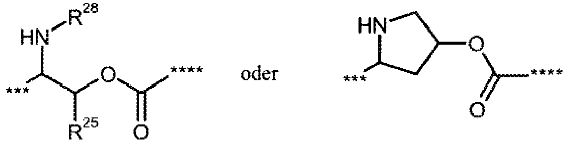 steht, worin
*** die Verknüpfungsstelle mit der Carbonylgruppe kennzeichnet,
**** die Verknüpfungsstelle mit L² kennzeichnet,
R²⁵ für Wasserstoff oder Methyl steht,
R²⁸ für Wasserstoff, (C₁-C₄)-Alkylcarbonyl, tert.-Butyloxycarbonyl oder Benzyloxycarbonyl steht,
Q¹ für einen 4- bis 7-gliedrigen Heterocyclus steht,
R¹⁶ für Wasserstoff oder (C₁-C₄)-Alkyl steht,
R¹⁷ für Wasserstoff oder (C₁-C₄)-Alkyl steht,
oder
R¹⁶ und R¹⁷ zusammen mit den Atomen, an die sie gebunden sind, einen 5- oder 6-gliedrigen Heterocyclus bilden,
R²³ für (C₁-C₄)-Alkyl steht,
R²⁴ für Wasserstoff oder (C₁-C₄)-Alkyl steht,
R³⁶ für Wasserstoff, (C₁-C₄)-Alkylcarbonyl, tert.-Butyloxycarbonyl oder Benzyloxy carbonyl steht,
R³⁷ für Wasserstoff oder Methyl steht,
oder
R³⁶ und R³⁷ bilden zusammen mit den Atomen, an die sie gebunden sind, einen Pyrrolidinring,
L² für lineares (C₂-C₁₀)-Alkandiyl oder für eine Gruppe der Formel 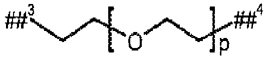 steht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
wobei (C₂-C₁₀)-Alkandiyl mit 1 bis 4 Substituenten unabhängig voneinander ausgewählt aus der Gruppe Methyl, Hydroxy und Benzyl substiuiert sein kann,
und
n, AK, G, D und R³⁵ die oben angegebenen Bedeutungen aufweisen,
sowie ihre Salze, Solvate und Solvate der Salze.

Bevorzugt sind im Rahmen der vorliegenden Erfindung auch Verbindungen der Formel (Ia), in welcher
- L¹: für lineares (C₂-C₆)-Alkandiyl oder eine Gruppe der Formel 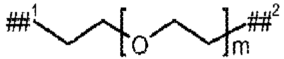 sieht, wobei
m für eine Zahl von 2 oder 3 steht,
##¹ die Verknüpfungsstelle mit der Gruppe G kennzeichnet,
##² die Verknüpfungsstelle mit der Gruppe B kennzeichnet,
- B: für eine Bindung oder eine Gruppe der Formel 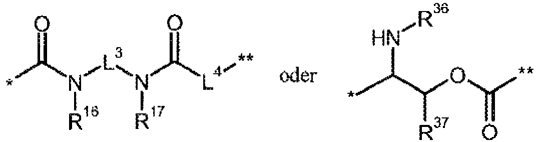 steht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Gruppe der Formel 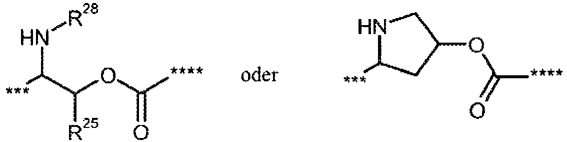 steht, worin
*** die Verknüpfungsstelle mit der Carbonylgruppe kennzeichnet,
**** die Verknüpfungsstelle mit L² kennzeichnet,
R²⁵ für Wasserstoff oder Methyl steht,
R²⁸ für Wasserstoff, Methylcarbonyl oder tert.-Butyloxycarbonyl steht,
R¹⁶ für Wasserstoff oder Methyl steht,
R¹⁷ für Wasserstoff oder Methyl steht,
oder
R¹⁶ und R¹⁷ zusammen mit den Atomen, an die sie gebunden sind, einen Piperazinylring bilden,
R³⁶ für Wasserstoff, Methylcarbonyl oder tert.-Butyloxycarbonyl steht,
R³⁷ für Wasserstoff oder Methyl steht,
oder
R³⁶ und R³⁷ bilden zusammen mit den Atomen, an die sie gebunden sind, einen Pyrrolidinring,
- L²: für lineares (C₂-C₆)-Alkandiyl oder für eine Gruppe der Formel 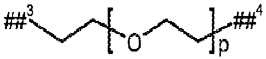 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
und
n, AK, G, D und R³⁵ die oben angegebenen Bedeutungen aufweisen,
sowie ihre Salze, Solvate und Solvate der Salze.

Bevorzugt sind im Rahmen der vorliegenden Erfindung sind auch Verbindungen der Formel (Ia) und (XXXa), in welcher
- G: für eine Gruppe der Formel 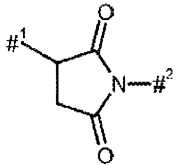 steht, wobei
#¹ die Verknüpfungsstelle mit dem Cystein-Rest des Binders kennzeichnet,
#² die Verknüpfungsstelle mit der Gruppe L¹ kennzeichnet,
- L¹: für lineares (C₃-C₅)-Alkandiyl oder eine Gruppe der Formel 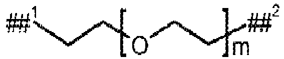 steht, wobei
m für eine Zahl von 2 oder 3 steht,
##¹ die Verknüpfungsstelle mit der Gruppe G kennzeichnet,
##² die Verknüpfungsstelle mit der Gruppe B kennzeichnet.
wobei (C₃-C₅)-Alkandiyl mit 1 oder 2 Substituenten Methyl substiuiert sein kann,
- B: für eine Bindung oder eine Gruppe der Formel 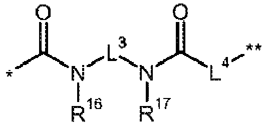 steht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Bindung steht,
- L²: für lineares (C₃-C₅)-Alkandiyl oder für eine Gruppe der Formel 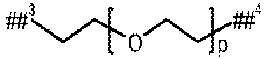 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
und
n, AK₁, Cys, D, R¹⁶ und R¹⁷ die oben angegebenen Bedeutungen aufweisen,
sowie ihre Salze, Solvate und Solvate der Salze.

Bevorzugt sind im Rahmen der vorliegenden Erfindung sind auch Verbindungen der Formel (Ia) und (XXXa), in welcher
- B: für eine Bindung oder eine Gruppe der Formel 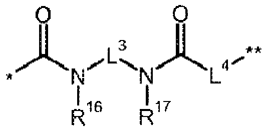 steht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Bindung steht,
n, AK, Cys, G, L¹, L², D, R¹⁶, R¹⁷ und R³⁵ die oben angegebenen Bedeutungen aufweisen,
sowie ihre Salze, Solvate und Solvate der Salze.

Bevorzugt sind im Rahmen der vorliegenden Erfindung sind auch Verbindungen der Formel (Ia), (XXXa) und (XXXI), in welcher
- L¹: für eine Bindung, lineares (C₃-C₅)-Alkandiyl oder eine Gruppe der Formel 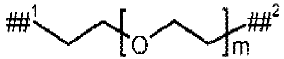 steht, wobei
m für eine Zahl von 2 oder 3 steht,
## die Verknüpfungsstelle mit der Gruppe G kennzeichnet,
##² die Verknüpfungsstelle mit der Gruppe B kennzeichnet,
wobei (C₃-C₅)-Alkandiyl mit 1 oder 2 Substituenten Methyl substiuiert sein kann,
- B: für eine Bindung oder eine Gruppe der Formel 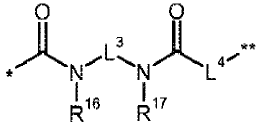 steht, wobei
* die Verknüpfungsstelle mit L¹ kennzeichnet,
** die Verknüpfungsstelle mit L² kennzeichnet,
L³ für eine Bindung steht,
L⁴ für eine Bindung
R¹⁶ für Wasserstoff steht,
R¹⁷ für Wasserstoff steht,
- L²: für lineares (C₃-C₆)-Alkandiyl oder für eine Gruppe der Formel 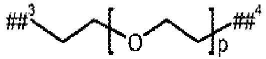 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
n, AK, Cys, G, D und R³⁵ die oben angegebenen Bedeutungen aufweisen,
sowie ihre Salze, Solvate und Solvate der Salze.

Bevorzugt sind im Rahmen der vorliegenden Erfindung sind auch Verbindungen der Formel (Ia), (XXXa) und (XXXI), in welcher
- L¹: für eine Bindung steht,
- B: für eine Bindung steht,
- L²: für lineares (C₃-C₆)-Alkandiyl oder für eine Gruppe der Formel 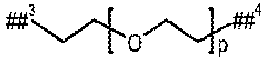 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
n, AK, Cys, G, D und R³⁵ die oben angegebenen Bedeutungen aufweisen,
sowie ihre Salze, Solvate und Solvate der Salze.

Bevorzugt sind im Rahmen der vorliegenden Erfindung sind auch Verbindungen der Formel (Ia), (XXXa) und (XXXI), in welcher
- L¹: für lineares (C₃-C₅)-Alkandiyl oder eine Gruppe der Formel 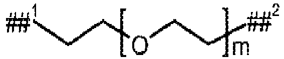 steht, wobei
m für eine Zahl von 2 oder 3 steht,
##¹ die Verknüpfungsstelle mit der Gruppe G kennzeichnet,
##² die Verknüpfungsstelle mit der Gruppe B kennzeichnet,
wobei (C₃-C₅)-Alkandiyl mit 1 oder 2 Substituenten Methyl substiuiert sein kann,
- B: für eine Gruppe der Formel 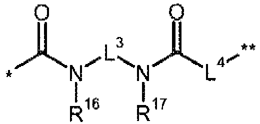 steht, wobei
* die Verknüpfungsstelle mit L¹ kennzeichnet,
** die Verknüpfungsstelle mit L² kennzeichnet,
**L**³ für eine Bindung steht,
**L**⁴ für eine Bindung
**R**¹⁶ für Wasserstoff steht,
- **R**¹⁷: für Wasserstoff steht,
- L²: für lineares (C₃-C₆)-Alkandiyl oder für eine Gruppe der Formel 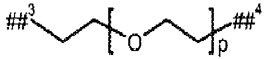 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet.
n, AK, Cys, G, D und R³⁵ die oben angegebenen Bedeutungen aufweisen,
sowie ihre Salze, Solvate und Solvate der Salze.

Bevorzugt sind im Rahmen der vorliegenden Erfindung sind auch Verbindungen der Formel (Ia), in welcher
- n: für eine Zahl von 2 bis 8, vorzugsweise 2 bis 5, steht,
- AK: für AK₁ oder AK₂ steht
wobei
AK₁ für einen Antikörper oder ein Antigen-bindendes Antikörperfragment steht, der bzw. das an C4.4a bindet und über das Schwefelatom eines Cystein-Restes des Binders an die Gruppe G gebunden ist,
AK₂ für einen Antikörper oder ein Antigen-bindendes Antikörperfragment steht, der bzw. das an C4.4a bindet und über die NH-Seitengruppe eines Lysin-Restes des Binders an die Gruppe G gebunden ist,
- G: für den Fall, dass AK = AK₁ ist, für eine Gruppe der Formel 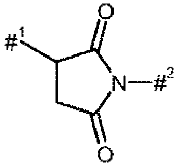 steht, wobei
#¹ die Verknüpfungsstelle mit dem Cystein-Rest des Binders kennzeichnet,
#² die Verknüpfungsstelle mit der Gruppe L¹ kennzeichnet,
oder
für den Fall, dass AK = AK₂ ist, für Carbonyl steht,
- L¹: für eine Bindung, lineares (C₃-C₅)-Alkandiyl oder eine Gruppe der Formel 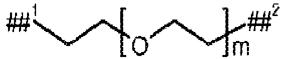 steht, wobei
m für eine Zahl von 2 oder 3 steht,
##¹ die Verknüpfungsstelle mit der Gruppe G kennzeichnet,
##² die Verknüpfungsstelle mit der Gruppe B kennzeichnet,
wobei (C₃-C₅)-Alkandiyl mit 1 oder 2 Substituenten Methyl substiuiert sein kann,
- B: für eine Bindung oder eine Gruppe der Formel 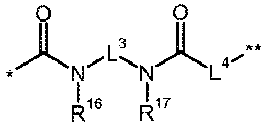 steht, wobei
* die Verknüpfungsstelle mit L¹ kennzeichnet,
** die Verknüpfungsstelle mit L² kennzeichnet,
**L**³ für eine Bindung steht,
**L**⁴ für eine Bindung
**R**¹⁶ für Wasserstoff steht,
**R**¹⁷ für Wasserstoff steht,
- L²: für lineares (C₃-C₆)-Alkandiyl oder für eine Gruppe der Formel 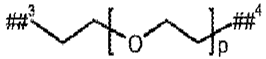 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
und
D und R³⁵ die oben angegebenen Bedeutungen aufweisen,
sowie ihre Salze, Solvate und Solvate der Salze.

Bevorzugt sind im Rahmen der vorliegenden Erfindung sind auch Verbindungen der Formel (Ia), in welcher
- n: für eine Zahl von 2 bis 8, vorzugsweise 2 bis 5, steht,
- AK: für AK₁ oder AK₂ steht
wobei
AK₁ für einen Antikörper oder ein Antigen-bindendes Antikörperfragment steht, der bzw. das an C4.4a bindet und über das Schwefelatom eines Cystein-Restes des Binders an die Gruppe G gebunden ist,
AK₂ für einen Antikörper oder ein Antigen-bindendes Antikörperfragment steht, der bzw. das an C4.4a bindet und über die NH-Seitengruppe eines Lysin-Restes des Binders an die Gruppe G gebunden ist,
- G: für den Fall, dass AK = AK₁ ist, für eine Gruppe der Formel 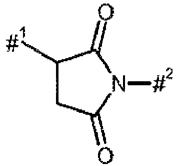 steht, wobei
#¹ die Verknüpfungsstelle mit dem Cystein-Rest des Binders kennzeichnet,
#² die Verknüpfungsstelle mit der Gruppe L¹ kennzeichnet,
oder
für den Fall, dass AK = AK₂ ist, für Carbonyl steht,
- L¹: für eine Bindung steht,
- B: für eine Bindung steht,
- L²: für lineares (C₃-C₆)-Alkandiyl oder für eine Gruppe der Formel 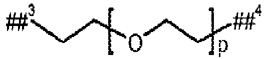 sieht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
D und R³⁵ die oben angegebenen Bedeutungen aufweisen,
sowie ihre Salze, Solvate und Solvate der Salze.

Bevorzugt sind im Rahmen der vorliegenden Erfindung sind auch Verbindungen der Formel (la), in welcher
- n: für eine Zahl von 2 bis 8, vorzugsweise 2 bis 5, sieht,
- AK: für AK₁ steht,
wobei
AK₁ für einen Antikörper oder ein Antigen-bindendes Antikörperfragment steht, der bzw. das an C4.4a bindet und über das Schwefelatom eines Cystein-Restes des Binders an die Gruppe G gebunden ist,
- G: für eine Gruppe der Formel 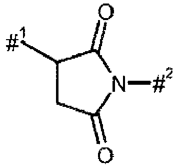 steht, wobei
#¹ die Verknüpfungsstelle mit dem Cystein-Rest des Binders kennzeichnet,
#² die Verknüpfungsstelle mit der Gruppe L¹ kennzeichnet,
- L¹: für lineares (C₃-C₅)-Alkandiyl oder eine Gruppe der Formel 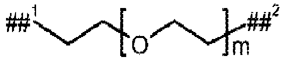 steht, wobei
m für eine Zahl von 2 oder 3 steht,
##¹ die Verknüpfungsstelle mit der Gruppe G kennzeichnet,
##² die Verknüpfungsstelle mit der Gruppe B kennzeichnet,
wobei (C₃-C₅)-Alkandiyl mit 1 oder 2 Substituenten Methyl substiuiert sein kann,
- B: für eine Gruppe der Formel 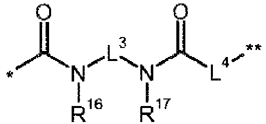 steht, wobei
* die Verknüpfungsstelle mit L¹ kennzeichnet,
** die Verknüpfungsstelle mit L² kennzeichnet,
L³ für eine Bindung steht,
L⁴ für eine Bindung
R¹⁶ für Wasserstoff steht,
R¹⁷ für Wasserstoff steht,
- L²: für lineares (C₃-C₆)-Alkandiyl oder für eine Gruppe der Formel 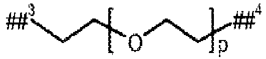 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
D und R³⁵ die oben angegebenen Bedeutungen aufweisen,
sowie ihre Salze, Solvate und Solvate der Salze.

Bevorzugt sind im Rahmen der vorliegenden Erfindung sind auch Verbindungen der Formel (Ia), in welcher
- L¹: für eine Bindung, lineares (C₃-C₅)-Alkandiyl, eine Gruppe der Formel 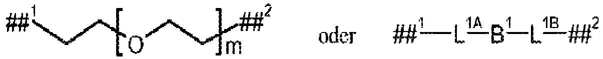 steht, wobei
m für eine Zahl von 2 oder 3 steht,
##¹ die Verknüpfungsstelle mit der Gruppe G kennzeichnet,
##² die Verknüpfungsstelle mit der Gruppe B kennzeichnet,
L^{1A} für lineares (C₃-C₆)-Alkandiyl steht,
B¹ für eine Gruppe der Formel 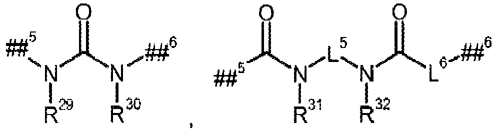 oder 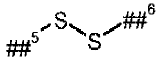 steht, worin
##⁵ die Verknüpfungsstelle mit der Gruppe L^{1A} kennzeichnet,
##⁶ die Verknüpfungsstelle mit der Gruppe L^{1B} kennzeichnet,
L⁵ für eine Bindung oder Ethan-1,2-diyl steht,
L⁶ für eine Bindung oder eine Gruppe der Formel 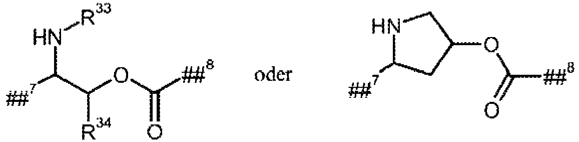 steht, worin
##⁷ die Verknüpfungsstelle mit der Carbonylgruppe kennzeichnet,
##⁸ die Verknüpfungsstelle mit L^{1B} kennzeichnet,
R³³ für Wasserstoff, (C₁-C₄)-Alkylcarbonyl oder tert.-Butyloxy-carbonyl steht,
R³⁴ für Wasserstoff oder Methyl steht,
R²⁹ für Wasserstoff oder Methyl steht,
R³⁰ für Wasserstoff oder Methyl steht,
R³¹ für Wasserstoff oder Methyl steht,
R³² für Wasserstoff oder Methyl steht,
L^{1B} für lineares (C₃-C₆)-Alkandiyl steht,
B für eine Bindung oder eine Gruppe der Formel 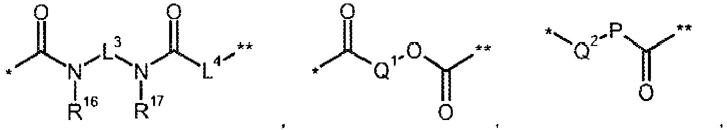 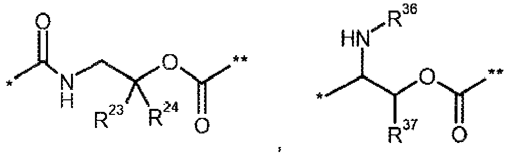 oder 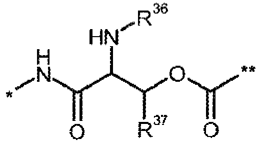 steht, wobei
* die Verknüpfungsstelle mit L¹ kennzeichnet,
** die Verknüpfungsstelle mit L² kennzeichnet,
P für O steht,
L³ für eine Bindung oder Ethan-1,2-diyl steht,
L⁴ für eine Gruppe der Formel 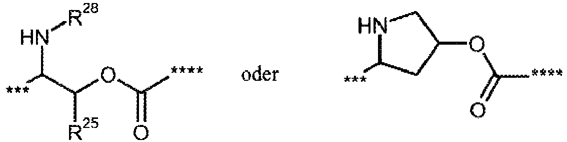 steht, worin
*** die Verknüpfungsstelle mit der Carbonylgruppe kennzeichnet,
**** die Verknüpfungsstelle mit L² kennzeichnet,
R²⁵ für Wasserstoff oder Methyl steht,
R²⁸ für Wasserstoff, (C₁-C₄)-Alkylcarbonyl oder tert.-Butyloxycarbonyl steht,
Q¹ für einen 4- bis 7-gliedrigen Heterocyclus steht,
Q² für einen 3- bis 7-gliedrigen Carbocyclus oder einen 4- bis 7-gliedrigen Heterocyclus steht,
R¹⁶ für Wasserstoff oder Methyl steht,
R¹⁷ für Wasserstoff oder Methyl steht,
R²³ für (C₁-C₄)-Alkyl steht,
R²⁴ für Wasserstoff oder (C₁-C₄)-Alkyl steht,
R³⁶ für Wasserstoff, (C₁-C₄)-Alkylcarbonyl oder tert.-Butyloxycarbonyl steht,
R³⁷ für Wasserstoff oder Methyl steht,
oder
R³⁶ und R³⁷ bilden zusammen mit den Atomen, an die sie gebunden sind, einen Pyrrolidinring,
L² für lineares (C₂-C₆)Alkandiyl oder für eine Gruppe der Formel 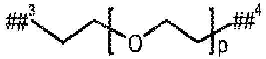 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
sowie ihre Salze, Solvate und Solvate der Salze.

Bevorzugt sind im Rahmen der vorliegenden Erfindung sind auch Verbindungen der Formel (la), in welcher
- L¹: für lineares (C₃-C₅)-Alkandiyl oder eine Gruppe der Formel 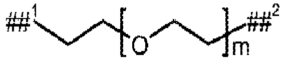 steht, wobei
m für eine Zahl von 2 oder 3 steht,
##¹ die Verknüpfungsstelle mit der Gruppe G kennzeichnet,
##² die Verknüpfungsstelle mit der Gruppe B kennzeichnet,
- B: für eine Gruppe der Formel 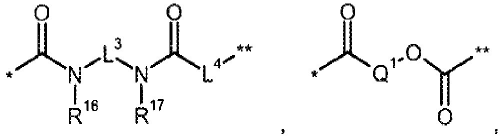 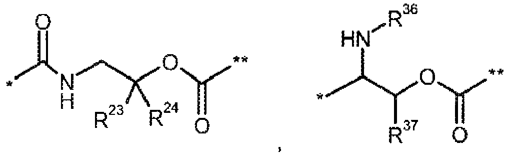 oder 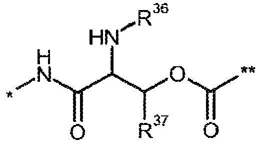 steht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Gruppe der Formel 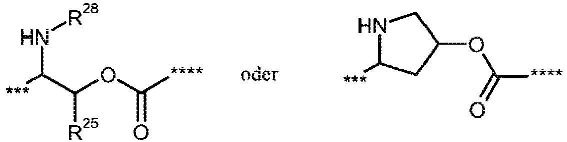 steht, worin
*** die Verknüpfungsstelle mit der Carbonylgruppe kennzeichnet,
**** die Verknüpfungsstelle mit L² kennzeichnet,
R²⁵ für Methyl steht,
R²⁸ für Wasserstoff, Methylcarbonyl oder tert.-Butyloxycarbonyl steht,
Q¹ für Piperidin-1,4-diyl steht,
R¹⁶ für Wasserstoff oder Methyl steht,
R¹⁷ für Wasserstoff oder Methyl steht,
R²³ für Methyl steht,
R²⁴ für Wasserstoff steht,
R³⁶ für Wasserstoff, Methylcarbonyl oder tert.-Butyloxycarbonyl steht,
R³⁷ für Wasserstoff oder Methyl steht,
- L²: für lineares (C₂-C₆)-Alkandiyl oder für eine Gruppe der Formel 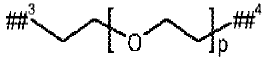 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
sowie ihre Salze, Solvate und Solvate der Salze.

Bevorzugt sind im Rahmen der vorliegenden Erfindung auch Verbindungen der Formel (Ia), (XXXa) und (XXXI), in welcher
- D: für eine Gruppe der Formel 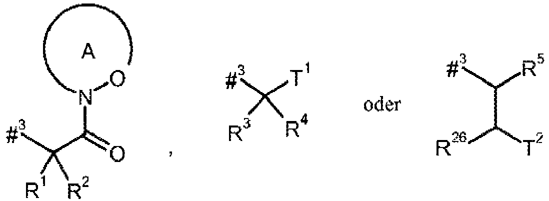 steht, wobei
#³ die Verknüpfungsstelle mit dem Stickstoffatom kennzeichnet,
R¹ für Wasserstoff oder Methyl steht,
R² für Isopropyl, Isobutyl, *sec*.-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenyl-ethyl, Diphenylmethyl, 1*H*-Imidazol-4-ylmethyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 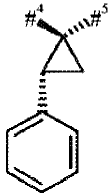 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 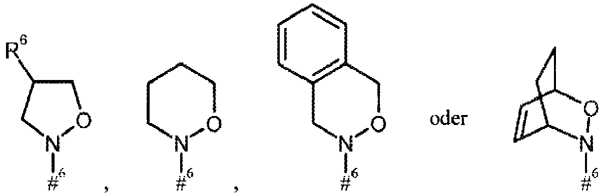 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff oder Methyl steht,
R⁴ für Isopropyl, Isobutyl, *sec*.-Butyl, *tert.*-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenyl-ethyl, Diphenylmethyl, 1*H*-Imidazol-4-ylmethyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 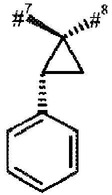 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹, -C(=O)-NH-NH-R¹⁰ oder -CH₂-O-R¹¹ steht,
worin
R⁷ für Wasserstoff, Methyl, Ethyl, *n*-Propyl, *tert*.-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
oder
R⁸ und R⁹ bilden zusammen mit dem Stickstoffatom, an das sie gebunden sind, einen 4- bis 7-gliedrigen Heterocyclus,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der Formel 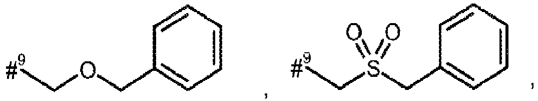 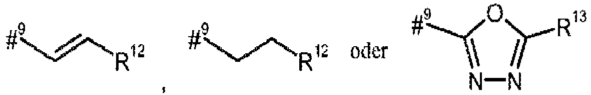 steht, worin
#⁹ die Verknüpfungsstelle mit -CHC(R²⁶)-T² kennzeichnet,
R¹² für Phenyl steht, das mit Methoxycarbonyl, Carboxyl oder einer Gruppe der Formel S(O)₂OH substituiert sein kann,
R¹³ für Phenyl steht, das mit Methoxycarbonyl oder Carboxyl substituiert sein kann,
R²⁶ für Wasserstoff steht,
T² für Phenyl, Benzyl, 1*H*-Indol-3-yl oder 1*H*-Indol-3-ylmethyl sieht,
und
n, AK, Cys, G, L¹, B, L², D und R³⁵ die oben angegebenen Bedeutungen aufweisen,
sowie ihre Salze, Solvate und Solvate der Salze.

Bevorzugt sind im Rahmen der vorliegenden Erfindung auch Verbindungen der Formel (Ia), (XXXa) und (XXXI), in welcher
- D: für eine Gruppe der Formel 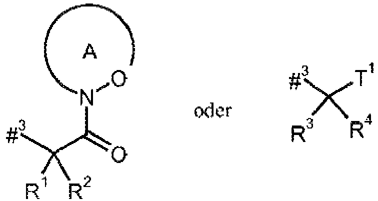 steht, wobei
#³ die Verknüpfungsstelle mit dem Stickstoffatom kennzeichnet,
R¹ für Wasserstoff steht,
R² für Benzyl, 4-Hydroxybenzyl, 1-Phenylethyl oder 1*H*-Indol-3-ylmethyl steht,
der Ring A mit der darin enthaltenen N-O-Gruppierung für einen Heterocyclus der Formel 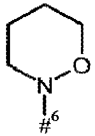 steht, worin
#⁶ die Verknüpfungsstelle mit der Carbonyl-Gruppe kennzeichnet,
R³ für Wasserstoff steht,
R⁴ für Benzyl, 4-Hydroxybenzyl, 1-Phenylethyl oder 1*H*-Indol-3-ylmethyl steht,
T¹ für eine Gruppe der Formel -C(=O)-OR⁷ oder -C(=O)-NR⁸R⁹ steht,
worin
R⁷ für Wasserstoff steht,
R⁸ für Wasserstoff steht,
R⁹ für Wasserstoff steht,
n, AK, Cys, G, L¹, B, L², D und R³⁵ die oben angegebenen Bedeutungen aufweisen,
sowie ihre Salze, Solvate und Solvate der Salze.

Bevorzugt sind im Rahmen der vorliegenden Erfindung auch Verbindungen der Formel (Ia), (XXXa) und (XXXI), in welcher
- D: für eine Gruppe der Formel 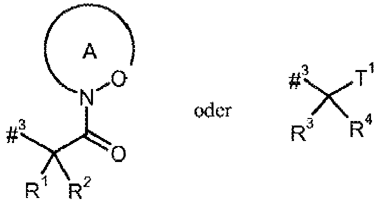 steht, wobei
#³ die Verknüpfungsstelle mit dem Stickstoffatom kennzeichnet,
R¹ für Wasserstoff steht,
R² für Benzyl, 4-Hydroxybenzyl, 1-Phenylethyl oder 1*H*-Indol-3-ylmethyl steht,
der Ring A mit der darin enthaltenen N-O-Gruppierung für einen Heterocyclus der Formel 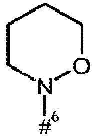 steht, worin
#⁶ die Verknüpfungsstelle mit der Carbonyl-Gruppe kennzeichnet,
R³ für Wasserstoff steht,
R⁴ für Benzyl, 4-Hydroxybenzyl, 1-Phenylethyl oder 1*H*-Indol-3-ylmethyl steht,
T¹ für eine Gruppe der Formel -C(=O)-NR⁸R⁹ steht,
worin
R⁸ für Wasserstoff steht,
R⁹ für Wasserstoff steht,
n, AK, Cys, G, L¹, B, L², D und R³⁵ die oben angegebenen Bedeutungen aufweisen,
sowie ihre Salze, Solvate und Solvate der Salze.

Bevorzugt sind im Rahmen der vorliegenden Erfindung auch Verbindungen der Formel (Ia), (XXXa) und (XXXI), in welcher
- D: für eine Gruppe der Formel 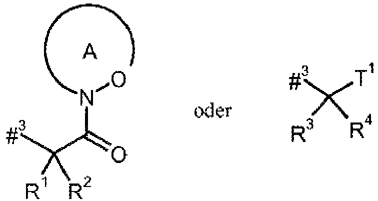 steht, wobei
#³ die Verknüpfungsstelle mit dem Stickstoffatom kennzeichnet,
R¹ für Wasserstoff steht,
R² für 4-Hydroxybenzyl oder 1*H*-Indol-3-ylmethyl steht,
der Ring A mit der darin enthaltenen N-O-Gruppierung für einen Heterocyclus der Formel 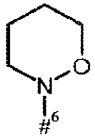 steht, worin
#⁶ die Verknüpfungsstelle mit der Carbonyl-Gruppe kennzeichnet,
R³ für Wasserstoff steht,
R⁴ für 4-Hydroxybenzyl oder 1*H*-Indol-3-ylmethyl steht,
T¹ für eine Gruppe der Formel -C(=O)-NR⁸R⁹ steht,
worin
R⁸ für Wasserstoff steht,
R⁹ für Wasserstoff steht,
n, AK, Cys, G, L¹, B, L², D und R³⁵ die oben angegebenen Bedeutungen aufweisen,
sowie ihre Salze, Solvate und Solvate der Salze.

Bevorzugt sind im Rahmen der vorliegenden Erfindung auch Verbindungen der Formel (Ia), (XXXa) und (XXXI), in welcher
- D: für eine Gruppe der Formel 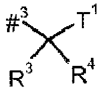 steht, wobei
#³ die Verknüpfungsstelle mit dem Stickstoffatom kennzeichnet,
R³ für Wasserstoff steht,
R⁴ für 4-Hydroxybenzyl oder 1*H*-Indol-3-ylmethyl steht,
T¹ für eine Gruppe der Formel -C(=O)-OR⁷ oder -C(=O)-NR⁸R⁹ steht,
worin
R⁷ für Wasserstoff steht,
R⁸ für Wasserstoff steht,
R⁹ für Wasserstoff steht,
n, AK, Cys, G, L¹, B, L², D und R³⁵ die oben angegebenen Bedeutungen aufweisen,
sowie ihre Salze, Solvate und Solvate der Salze.

Bevorzugt sind im Rahmen der vorliegenden Erfindung auch Verbindungen der Formel (Ia), (XXXa) und (XXXI), in welcher
- D: für eine Gruppe der Formel 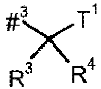 steht, wobei
#³ die Verknüpfungsstelle mit dem Stickstoffatom kennzeichnet,
R³ für Wasserstoff steht,
R⁴ für 4-Hydroxybenzyl oder 1*H*-Indol-3-ylmethyl steht,
T¹ für eine Gruppe der Formel -C(=O)-NR⁸R⁹ steht,
worin
R⁸ für Wasserstoff steht,
R⁹ für Wasserstoff steht,
n, AK, Cys, G, L¹, B, L², D und R³⁵ die oben angegebenen Bedeutungen aufweisen,
sowie ihre Salze, Solvate und Solvate der Salze.

Bevorzugt sind im Rahmen der vorliegenden Erfindung auch Verbindungen der Formel (Ia), (XXXa) und (XXXI), in welcher
- D: für eine Gruppe der Formel 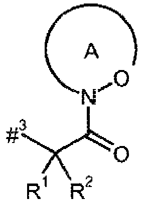 steht, wobei
#³ die Verknüpfungsstelle mit dem Stickstoffatom kennzeichnet,
R¹ für Wasserstoff oder Methyl steht,
R² für Isopropyl, Isobutyl, *sec*.-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenyl-ethyl, Diphenylmethyl, 1*H*-Imidazol-4-ylmethyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 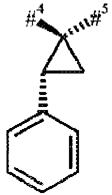 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischen, gegebenenfalls substituierten Heterocyclus der Formel 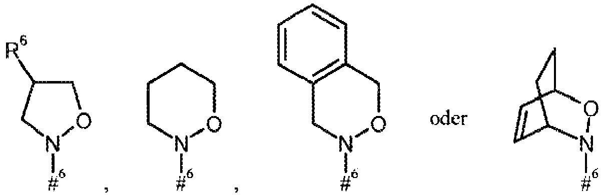 steht, worin
#⁶ die Verknüpfungsstelle mit der Carbonyl-Gruppe kennzeichnet,
R⁶ für Wasserstoff, Hydroxy oder Benzyloxy steht,
und
n, AK, Cys, G, L¹, B, L² und R³⁵ die oben angegebenen Bedeutungen aufweisen,
sowie ihre Salze, Solvate und Solvate der Salze.

Bevorzugt sind im Rahmen der vorliegenden Erfindung auch Verbindungen der Formel (Ia), (XXXa) und (XXXI), in welcher
- D: für eine Gruppe der Formel 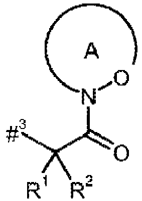 steht, wobei
#³ die Verknüpfungsstelle mit dem Stickstoffatom kennzeichnet,
R¹ für Wasserstoff steht,
R² für Benzyl, 4-Hydroxybenzyl, 1-Phenylethyl oder 1*H*-Indol-3-ylmethyl steht,
oder
R¹ und R² zusammen mit dem Kohlenstoffatom, an das sie gebunden sind, eine (1*S,*2*R*)-2-Phenylcyclopropan-1,1-diyl-Gruppe der Formel 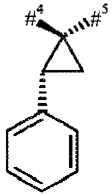 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischcn, gegebenenfalls substituierten Heterocyclus der Formel 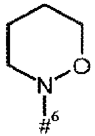 steht, worin
#⁶ die Verknüpfungsstelle mit der Carbonyl-Gruppe kennzeichnet,
und
n, AK, Cys, G, L¹, B, L² und R³⁵ die oben angegebenen Bedeutungen aufweisen,
sowie ihre Salze, Solvate und Solvate der Salze.

Bevorzugt sind im Rahmen der vorliegenden Erfindung auch Verbindungen der Formel (Ia), (XXXa) und (XXXI), in welcher
- R³⁵: für Hydroxy steht,
und
n, AK, Cys, G, L¹, B, L², D und R³⁵ die oben angegebenen Bedeutungen aufweisen,
sowie ihre Salze, Solvate und Solvate der Salze.

Bevorzugt sind im Rahmen der vorliegenden Erfindung auch Verbindungen der Formel (Ia), (XXXa) und (XXXI), in welcher
- R³⁵: für Methyl steht,
und
n, AK, Cys, G, L¹, B, L², D und R³⁵ die oben angegebenen Bedeutungen aufweisen,
sowie ihre Salze, Solvate und Solvate der Salze.

Besonders bevorzugt im Rahmen der vorliegenden Erfindung sind weiterhin auch Verbindungen der Formel (XXXa), in welcher
- Cys: für einen L-Cystein-Rest steht, der über das Schwefelatom der Seitenkette über ein Kohlenstoffatom des Succinimids gebunden ist, steht
sowie ihre Salze, Solvate und Solvate der Salze.

Bevorzugt sind im Rahmen der vorliegenden Erfindung auch Verbindungen der Formel (I) und (XXX), in welcher
- D: für eine Gruppe der Formel 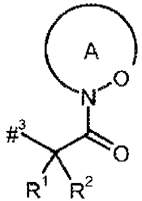 steht, wobei
#³ die Verknüpfungsstelle mit dem Stickstoffatom kennzeichnet,
R¹ für Wasserstoff steht,
R² für Benzyl, 1-Phenylethyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 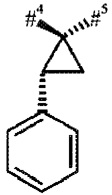 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bi-cyclischcn, gegebenenfalls substituierten Heterocyclus der Formel 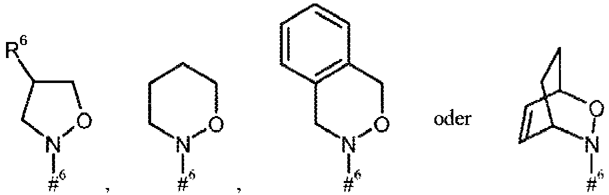 steht, worin
#⁶ die Verknüpfungsstelle mit der Carbonyl-Gruppe kennzeichnet,
R⁶ für Wasserstoff, Hydroxy oder Benzyloxy steht,
n, AK, Cys, G, L¹, L² und B die oben angegebenen Bedeutungen aufweisen,
sowie ihre Salze, Solvate und Solvate der Salze.

Besonders bevorzugt sind im Rahmen der vorliegenden Erfindung auch Verbindungen der Formel (I) und (XXX), in welcher
- D: für eine Gruppe der Formel 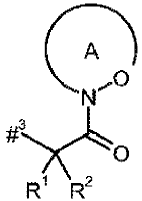 steht, wobei
#³ die Verknüpfungsstelle mit dem Stickstoffatom kennzeichnet,
R¹ für Wasserstoff steht,
R² für Benzyl oder 1*H*-Indol-3-ylmethyl steht,
oder
R¹ und R² zusammen mit dem Kohlenstoffatom, an das sie gebunden sind, eine (1*S,*2*R*)-2-Phenylcyclopropan-1,1-diyl-Gruppe der Formel 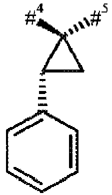 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen Heterocyclus der Formel 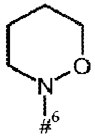 steht, worin
#⁶ die Verknüpfungsstelle mit der Carbonyl-Gruppe kennzeichnet,
n, AK, Cys, G. L¹, L² und B die oben angegebenen Bedeutungen aufweisen,
sowie ihre Salze, Solvate und Solvate der Salze.

Ein weiterer besonders bevorzugter Gegenstand der vorliegenden Erfindung sind Verbindungen der Formel (I), in welcher
- D: für eine Gruppe der Formel 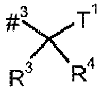 steht, wobei
T¹ für -C(=O)-OH oder -C(=O)-NH₂ steht und
n, AK, G, L¹, B, L², #³, R³ und R⁴ die oben angegebenen Bedeutungen aufweisen.

Bevorzugt im Rahmen der vorliegenden Erfindung sind auch Verbindungen der Formel (I), in welcher
n = 1-20, besonders bevorzugt n = 1-10 und ganz besonders bevorzugt n = 2-8 bedeutet.

Bevorzugt sind im Rahmen der vorliegenden Erfindung sind auch Verbindungen der Formel (I) und (XXX), in welcher
- B: für eine Bindung oder eine Gruppe der Formel 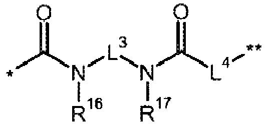 steht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Bindung steht,
n, AK, Cys, G, L¹, L², D, R¹⁶ und R¹⁷ die oben angegebenen Bedeutungen aufweisen,
sowie ihre Salze, Solvate und Solvate der Salze.

Besonders bevorzugt sind im Rahmen der vorliegenden Erfindung sind auch Verbindungen der Formel (I) und (XXX), in welcher
- B: für eine Bindung oder eine Gruppe der Formel 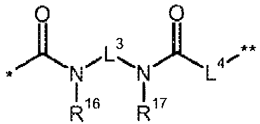 steht, wobei
* die Verknüpfungsstelle mit L¹ kennzeichnet,
** die Verknüpfungsstelle mit L² kennzeichnet,
L³ und L⁴ für eine Bindung steht,
n, AK, Cys, G, L¹, L², D, R¹⁶ und R¹⁷ die oben angegebenen Bedeutungen aufweisen,
sowie ihre Salze, Solvate und Solvate der Salze.

Bevorzugt sind im Rahmen der vorliegenden Erfindung auch Binder-Wirkstoff Konjugate der allgemeinen Formel (I), in welcher
- AK: für AK₁ steht
wobei
AK₁ für einen Binder, der über das Schwefelatom eines Cystein-Restes des Binders an die Gruppe G gebunden ist,
- G: für eine Gruppe der Formel 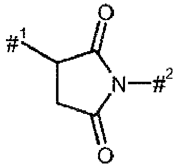 steht, wobei
#¹ die Verknüpfungsstelle mit dem Cystein-Rest des Binders kennzeichnet,
#² die Verknüpfungsstelle mit der Gruppe L¹ kennzeichnet,
L¹ für eine Bindung, lineares (C₁-C₁₀)-Alkandiyl oder für eine Gruppe der Formel 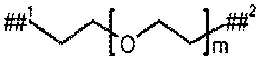 steht, wobei
m für eine Zahl von 2 bis 6 steht,
##¹ die Verknüpfungsstelle mit der Gruppe G kennzeichnet,
##² die Verknüpfungsstelle mit der Gruppe B kennzeichnet,
wobei (C₁-C₁₀)-Alkandiyl mit 1 bis 4 Substituenten Methyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
B für eine Bindung oder eine Gruppe der Formel 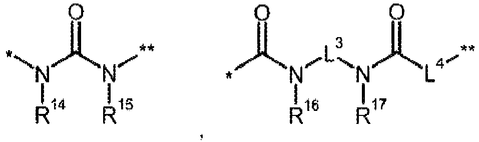 oder 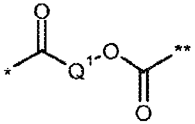 steht, wobei
* die Verknüpfungsstelle mit L¹ kennzeichnet,
** die Verknüpfungsstelle mit L² kennzeichnet,
L³ für eine Bindung oder (C₂-C₄)-Alkandiyl steht,
L⁴ für eine Bindung oder eine Gruppe der Formel 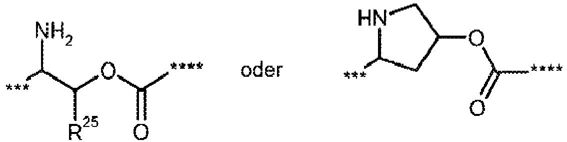 steht, worin
*** die Verknüpfungsstelle mit der Carbonylgruppe kennzeichnet,
**** die Verknüpfungsstelle mit L² kennzeichnet,
R²⁵ für Wasserstoff oder Methyl steht,
Q¹ für einen 4- bis 7-gliedrigen Heterocyclus steht,
R¹⁴ für Wasserstoff oder (C₁-C₄)-Alkyl steht,
R¹⁵ für Wasserstoff oder (C₁-C₄)-Alkyl steht,
oder
R¹¹ und R¹⁵ zusammen mit den Atomen, an die sie gebunden sind, einen 5- oder 6-gliedrigen Heterocyclus bilden,
R¹⁶ für Wasserstoff oder (C₁-C₄)-Alkyl steht,
R¹⁷ für Wasserstoff oder (C₁-C₄)-Alkyl steht,
oder
R¹⁶ und R¹⁷ zusammen mit den Atomen, an die sie gebunden sind, einen 5- oder 6-gliedrigen Heterocyclus bilden,
L² für lineares (C₂-C₁₀)-Alkandiyl oder für eine Gruppe der Formel 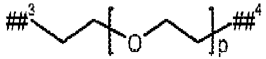 steht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
wobei (C₂-C₁₀)-Alkandiyl mit 1 bis 4 Substituenten Methyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
sowie ihre Salze, Solvate und Solvate der Salze.

Bevorzugt sind im Rahmen der vorliegenden Erfindung auch Binder-Wirkstoff Konjugate der allgemeinen Formel (I), in welcher
- AK: für AK₂ steht
wobei
AK₂ für einen Binder, der über die NH-Seitengruppe eines Lysin-Restes des Binders an die Gruppe G gebunden ist,
- G: für Carbonyl steht,
- L⁵: für eine Bindung, lineares (C₁-C₁₀)-Alkandiyl oder für eine Gruppe der Formel 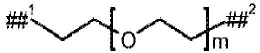 steht, wobei
m für eine Zahl von 2 bis 6 steht,
##¹ die Verknüpfungsstelle mit der Gruppe G kennzeichnet,
##² die Verknüpfungsstelle mit der Gruppe B kennzeichnet,
wobei (C₁-C₁₀)-Alkandiyl mit 1 bis 4 Substituenten Methyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring, oder einem Phenyl-Ring verbrückt sein können,
- B: für eine Bindung oder eine Gruppe der Formel 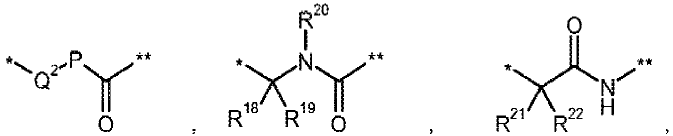 oder 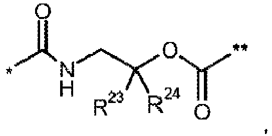 steht, wobei
* die Verknüpfungsstelle mit kennzeichnet,
** die Verknüpfungsstelle mit L² kennzeichnet,
P für O oder NH steht,
Q² für einen 3- bis 7-gliedrigen Carbocyclus oder einen 4- bis 7-gliedrigen Heterocyclus steht,
R¹⁸ für Wasserstoff oder (C₁-C₄)-Alkyl steht,
R¹⁹ für Wasserstoff oder die Seitengruppe einer natürlichen α-Aminosäure oder ihrer Homologe oder Isomere steht,
R²⁰ für Wasserstoff oder (C₁-C₄)-Alkyl steht,
oder
R¹⁹ und R²⁰ zusammen mit den Atomen, an die sie gebunden sind, einen Pyrrolidinylring bilden,
R²¹ für Wasserstoff oder (C₁-C₄)-Alkyl steht,
R²² für Wasserstoff oder (C₁-C₄)-Alkyl steht,
oder
R²¹ und R²² zusammen mit den Atomen, an die sie gebunden sind, einen 3- bis 7-gliedrigen Carbocyclus bilden,
R²³ für (C₁-C₄)-Alkyl steht,
R²⁴ für Wasserstoff oder (C₁-C₄)-Alkyl steht,
- L²: für lineares (C₂-C₁₀)-Alkandiyl oder für eine Gruppe der Formel 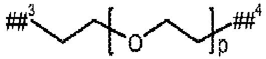 steht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
wobei (C₂-C₁₀)-Alkandiyl mit 1 bis 4 Substituenten Methyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
sowie ihre Salze, Solvate und Solvate der Salze.

Besonders bevorzugt sind im Rahmen der vorliegenden Erfindung Binder-WirkstotT-Konjugate der Formel (Ia), in welcher
- n: für eine Zahl von 2 bis 8 steht,
- AK: für AK₁ oder AK₂ steht,
wobei
AK₁ für einen humaner oder humanisierter Antikörper oder ein Antigen-bindendes Antikörperfragment steht, der bzw. das an C4.4a bindet und über das Schwefelatom eines Cystein-Restes des Binders an die Gruppe G gebunden ist,
AK₂ für einen humaner oder humanisierter Antikörper oder ein Antigen-bindendes Antikörperfragment steht, der bzw. das an C4.4a bindet und über die NH-Seitengruppe eines Lysin-Restes des Binders an die Gruppe G gebunden ist,
- G: für den Fall, dass AK = AK₁ ist, für eine Gruppe der Formel 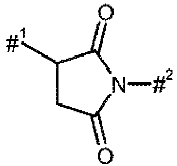 steht, wobei
#¹ die Verknüpfungsstelle mit dem Cystein-Rest des Binders kennzeichnet,
#² die Verknüpfungsstelle mit der Gruppe L¹ kennzeichnet,
oder
für den Fall, dass AK = AK₂ ist, für Carbonyl steht,
- L¹: für eine Bindung, lineares (C₂-C₆)-Alkandiyl, eine Gruppe der Formel 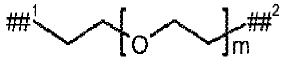 steht, wobei
m für eine Zahl von 2 oder 3 steht,
##¹ die Verknüpfungsstelle mit der Gruppe G kennzeichnet,
##² die Verknüpfungsstelle mit der Gruppe B kennzeichnet,
wobei (C₂-C₆)-Alkandiyl mit 1 oder 2 Substituenten Methyl substiuiert sein kann,
- B: für eine Bindung oder eine Gruppe der Formel 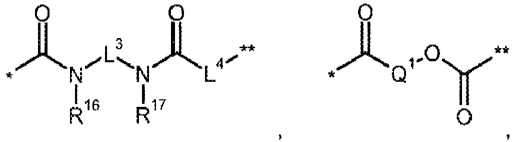 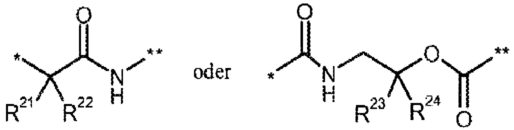 steht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Bindung oder eine Gruppe der Formel 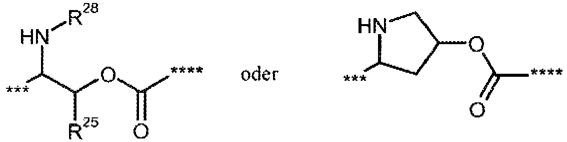 steht, worin
*** die Verknüpfungsstelle mit der Carbonylgruppe kennzeichnet,
**** die Verknüpfungsstelle mit L² kennzeichnet,
R²⁵ für Methyl steht,
R²⁸ für Wasserstoff, Methylcarbonyl oder tert.-Butyloxycarbonyl steht,
Q¹ für Piperidin-1,4-diyl steht,
R¹⁶ für Wasserstoff oder Methyl steht,
R¹⁷ für Wasserstoff oder Methyl steht,
oder
R¹⁶ und R¹⁷ zusammen mit den Atomen, an die sie gebunden sind, einen Piperazinylring bilden,
R²¹ für Wasserstoff oder Methyl steht,
R²² für Wasserstoff oder Methyl steht,
oder
R²¹ und R²² zusammen mit den Atomen, an die sie gebunden sind, einen Cyclopropylring bilden,
R²³ für Methyl steht,
R²⁴ für Wasserstoff steht,
- L²: für lineares (C₂-C₆)-Alkandiyl oder für eine Gruppe der Formel 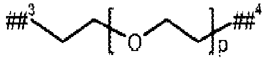 steht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
D für eine Gruppe der Formel 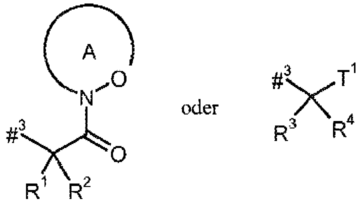 steht, wobei
#³ die Verknüpfungsstelle mit dem Stickstoffatom kennzeichnet,
R¹ für Wasserstoff steht,
R² für 4-Hydroxybenzyl oder 1*H*-Indol-3-ylmethyl steht,
der Ring A mit der darin enthaltenen N-O-Gruppierung für 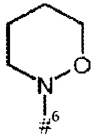 steht, worin
#⁶ die Verknüpfungsstelle mit der Carbonyl-Gruppe kennzeichnet,
R³ für Wasserstoff steht,
R⁴ für 4-Hydroxybenzyl oder 1*H*-Indol-3-ylmethyl steht,
T¹ für eine Gruppe der Formel -C(=O)-NR⁸R⁹ steht,
R⁸ für Wasserstoff oder Methyl steht,
R⁹ für Wasserstoff, Methyl, oder Ethyl steht,
R³⁵ für Methyl steht,
sowie ihre Salze, Solvate und Solvate der Salze.

Besonders bevorzugt sind im Rahmen der vorliegenden Erfindung Binder-Wirkstoff-Konjugate der Formel (Ia), in welcher
- n: für eine Zahl von 2 bis 8, vorzugsweise 2 bis 5 steht,
- AK: für AK₁ steht,
wobei
AK₁ für einen humaner oder humanisierter Antikörper oder ein Antigen-bindendes Antikörperfragment steht, der bzw. das an C4.4a bindet und über das Schwefelatom eines Cystein-Restes des Binders an die Gruppe G gebunden ist,
- G: für eine Gruppe der Formel 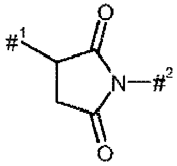 steht, wobei
#¹ die Verknüpfungsstelle mit dem Cystein-Rest des Binders kennzeichnet,
#² die Verknüpfungsstelle mit der Gruppe L¹ kennzeichnet,
- L⁵: für Pentan-1,5-diyl steht,
- B: für eine Gruppe der Formel 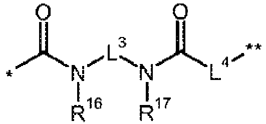 steht, wobei
* die Verknüpfungsstelle mit L¹ kennzeichnet,
** die Verknüpfungsstelle mit L² kennzeichnet,
L³ für eine Bindung steht,
L⁴ für eine Bindung steht,
R¹⁶ für Wasserstoff steht,
R¹⁷ für Wasserstoff steht,
- L²: für Propan-1,3-diyl steht,
- D: für eine Gruppe der Formel 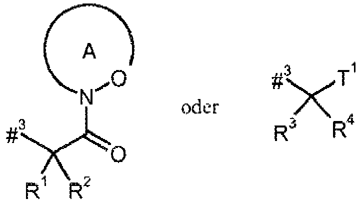 steht, wobei
#³ die Verknüpfungsstelle mit dem Stickstoffatom kennzeichnet,
R¹ für Wasserstoff steht,
R² für 4-Hydroxybenzyl oder 1*H*-Indol-3-ylmethyl steht,
der Ring A mit der darin enthaltenen N-O-Gruppierung für 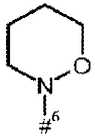 steht, worin
#⁶ die Verknüpfungsstelle mit der Carbonyl-Gruppe kennzeichnet,
R³ für Wasserstoff steht,
R⁴ für 4-Hydroxybenzyl oder 1*H*-Indol-3-ylmethyl steht,
T¹ für eine Gruppe der Formel -C(=O)-NR⁸R⁹ steht,
R⁸ für Wasserstoff steht,
R⁹ für Wasserstoff steht,
R³⁵ für Methyl steht,
sowie ihre Salze, Solvate und Solvate der Salze.

Besonders bevorzugt sind im Rahmen der vorliegenden Erfindung Binder-Wirkstoff-Konjuaate der Formel (Ia), in welcher
- n: für eine Zahl von 2 bis 8, vorzugsweise 2 bis 5 steht,
- AK: für AK₁ steht,
wobei
AK₁ für einen humaner oder humanisierter Antikörper oder ein Antigen-bindendes Antikörperfragment steht, der bzw. das an C4.4a bindet und über das Schwefelatom eines Cystein-Restes des Binders an die Gruppe G gebunden ist,
- G: für eine Gruppe der Formel 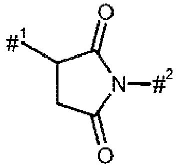 steht, wobei
#¹ die Verknüpfungsstelle mit dem Cystein-Rest des Binders kennzeichnet,
#² die Verknüpfungsstelle mit der Gruppe L¹ kennzeichnet,
- L¹: für eine Bindung steht,
- B: für eine Bindung steht,
- L²: für Hexan-1,6-diyl steht,
und D die zuvor angegebene Bedeutung hat,
sowie ihre Salze, Solvate und Solvate der Salze.

Besonders bevorzugt sind im Rahmen der vorliegenden Erfindung Binder-Wirkstoff-Konjugate der Formel (Ia), in welcher
- n: für eine Zahl von 2 bis 8, vorzugsweise von 2 bis 5, steht,
- AK: für AK₂ steht,
wobei
AK₂ für einen humaner oder humanisierter Antikörper oder ein Antigen-bindendes Antikörperfragment steht, der bzw. das an C4.4a bindet und über die NH-Seitengruppe eines Lysin-Restes des Binders an die Gruppe G gebunden ist,
- G: für Carbonyl steht,
- L¹: für eine Bindung steht,
- B: für eine Bindung steht,
- L¹: für Pentan-1,5-diyl steht,
- D: für eine Gruppe der Formel 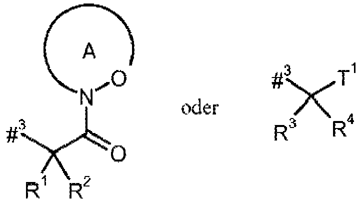 steht, wobei
#³ die Verknüpfungsstelle mit dem Stickstoffatom kennzeichnet,
**R**¹ für Wasserstoff steht,
**R**² für 4-Hydroxybenzyl oder 1*H*-Indol-3-ylmethyl steht,
der Ring A mit der darin enthaltenen N-O-Grupppierung für 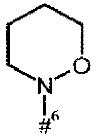 steht, worin
#⁶ die Verknüpfungsstelle mit der Carbonyl-Gruppe kennzeichnet,
R³ für Wasserstoff steht,
R⁴ für 4-Hydroxybenzyl oder 1*H*-Indol-3-ylmethyl steht,
T¹ für eine Gruppe der Formel -C(=O)-NR⁸R⁹ steht,
R⁸ für Wasserstoff steht,
R⁹ für Wasserstoff steht,
- R³⁵: für Methyl steht,
sowie ihre Salze, Solvate und Solvate der Salze.

Besonders bevorzugt sind im Rahmen der vorliegenden Erfindung Binder-Wirkstoff-Konjugate der Formel (la), in welcher
- n: für eine Zahl von 2 bis 8, vorzugsweise 2 bis 5 steht,
- AK: für AK₂ steht,
wobei
AK₂ für einen humaner oder humanisierter Antikörper oder ein Antigen-bindendes Antikörperfragment steht, der bzw. das an C4.4a bindet und über die NH-Seitengruppe eines Lysin-Restes des Binders an die Gruppe G gebunden ist,
- G: für Carbonyl steht,
- L¹: für eine Bindung sieht,
- B: für eine Bindung steht,
- L²: für eine Gruppe der Formel 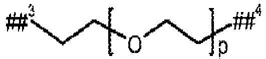 steht, wobei
p für die Zahl 3 steht,
##³ die Verknüpfungsstelle mit der Gruppe B kennzeichnet,
##⁴ die Verknüpfungsstelle mit dem Stickstoffatom kennzeichnet,
und D die zuvor angegebene Bedeutung hat,
sowie ihre Salze, Solvate und Solvate der Salze.

Besonders bevorzugt sind im Rahmen der vorliegenden Erfindung auch Wirkstoff-Binder-Konjugate ausgewählt aus den folgenden Verbindungen: 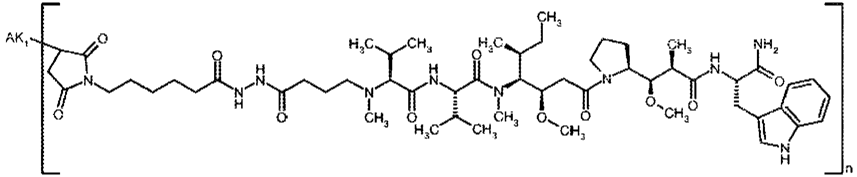 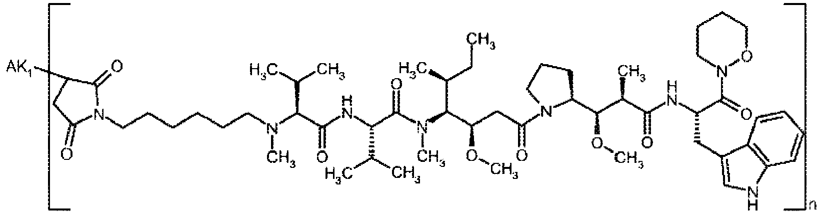 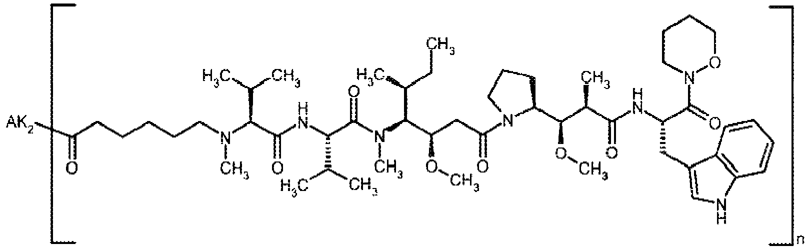 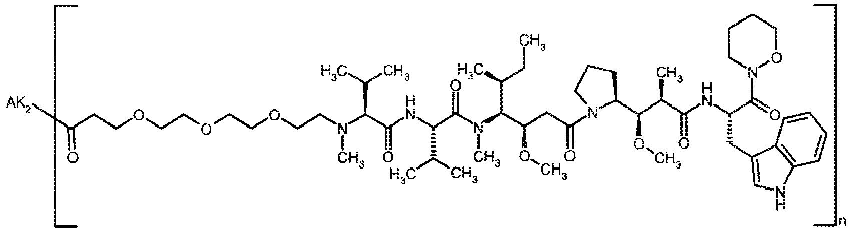 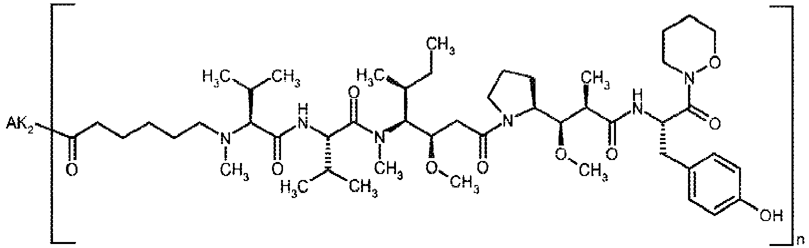 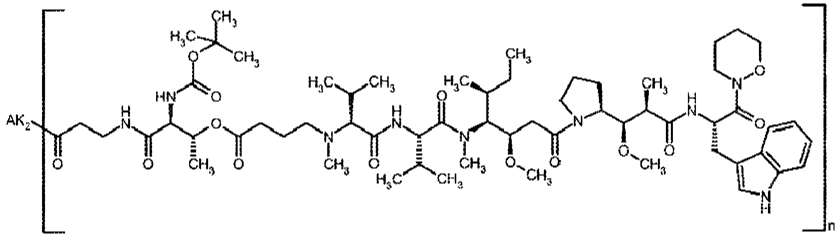 wobei jeweils
- n: für eine Zahl von 2 bis 8, vorzugsweise 2 bis 5 steht,
- AK₁: für einen humaner oder humanisierter Antikörper oder ein Antigen-bindendes Antikörperfragment steht, der bzw. das an C4.4a bindet und über das Schwefelatom eines Cystein-Restes des Binders an die Gruppe G gebunden ist,
und
- AK₂: für einen humaner oder humanisierter Antikörper oder ein Antigen-bindendes Antikörperfragment steht, der bzw. das an C4.4a bindet und über die NH-Seitengruppe eines Lysin-Restes des Binders an die Gruppe G gebunden ist,

Insbesondere bevorzugt sind im Rahmen der vorliegenden Erfindung Wirkstoff-Binder-Konjugate ausgewählt aus den folgenden Verbindungen: 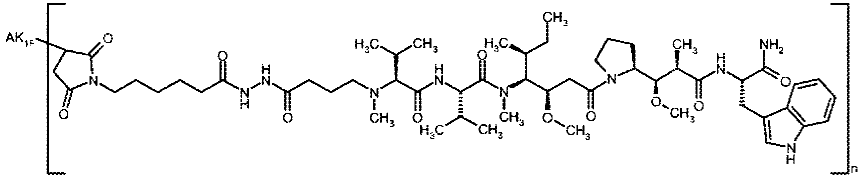 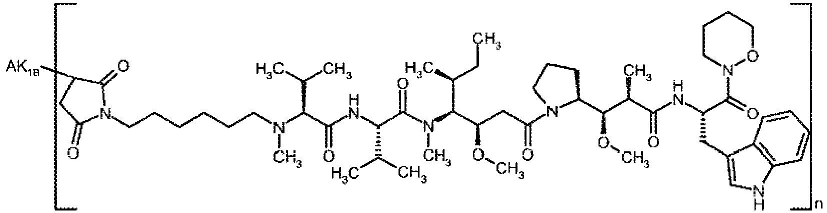 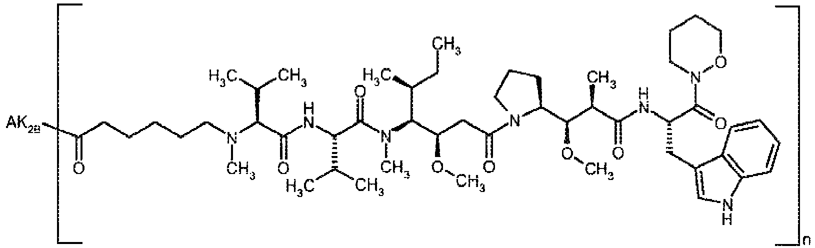 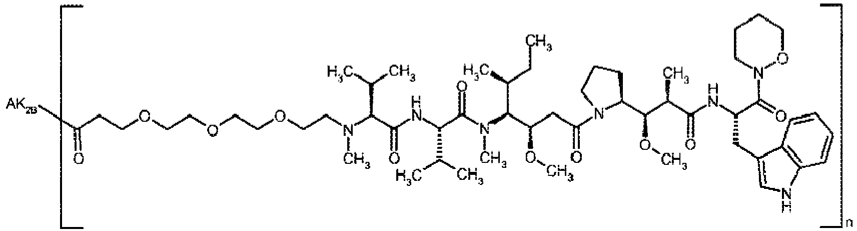 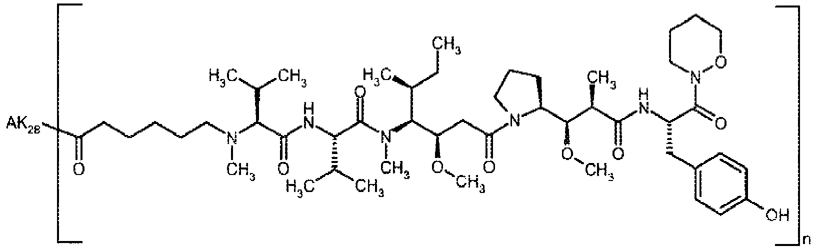 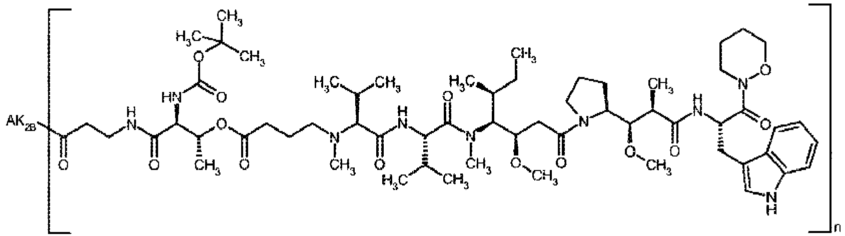 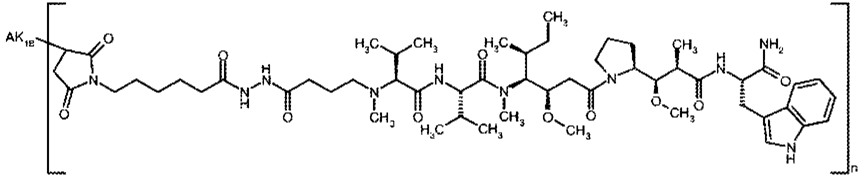 wobei jeweils
- n: für eine Zahl von 2 bis 8, vorzugsweise 2 bis 5 steht,
und
- AK: für einen humanen oder humanisierten Antikörper oder ein Antigen-bindendes Antikörperfragment steht, der bzw. das an C4.4a bindet.

In diesen Formeln können AK_{1F}, AK_{1B} und AK_{2B} durch andere humane oder humanisierte anti-C4.4a-Antikörper ersetzt werden.

Besonders bevorzugt sind Wirkstoff-Binder-Konjugate ausgewählt aus den folgenden Verbindungen: 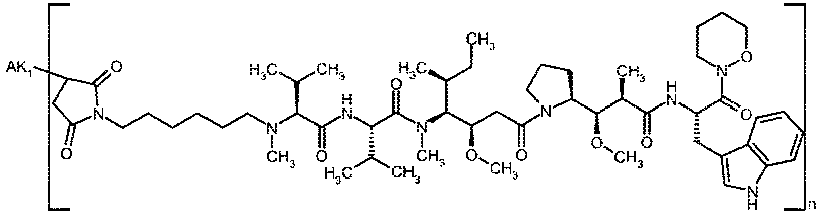 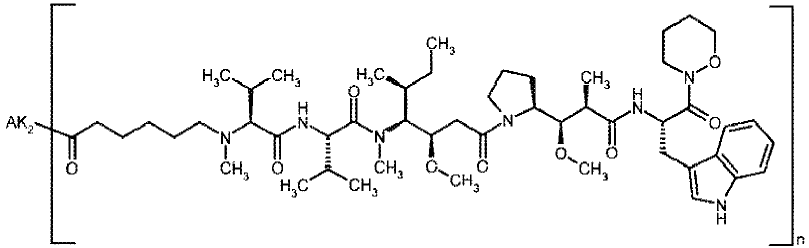 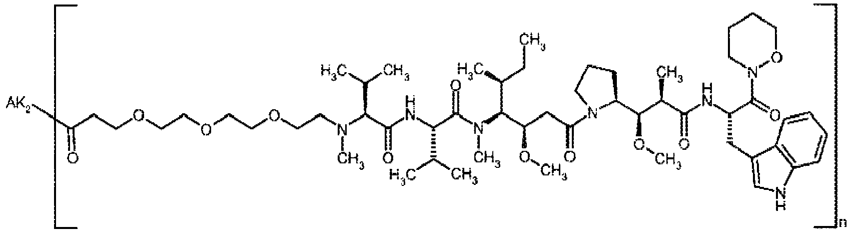 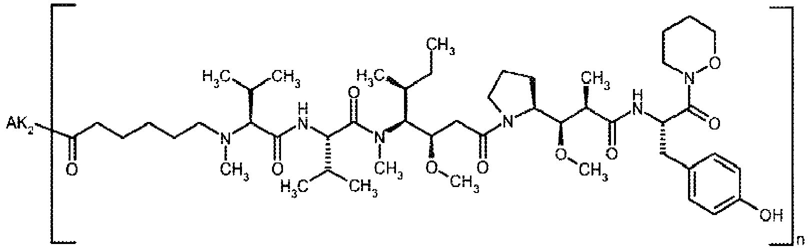 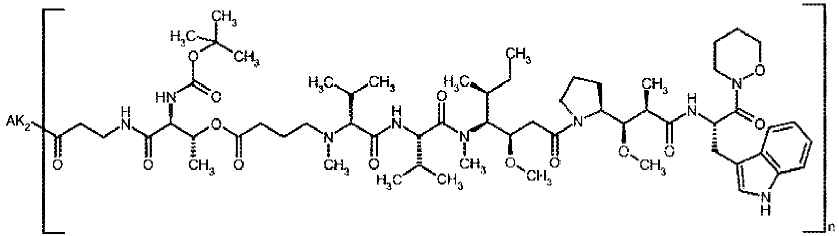 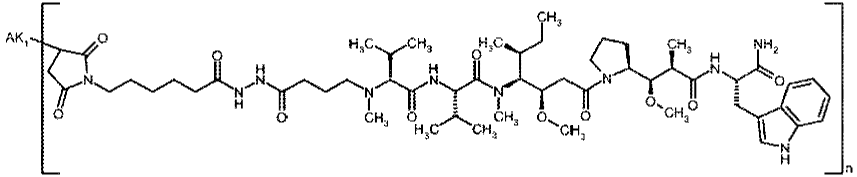 wobei jeweils
- n: für eine Zahl von 2 bis 8, vorzugsweise 2 bis 5 steht,
und
- AK₁: für einen humanen oder humanisierten Antikörper oder ein Antigen-bindendes Antikörperfragment steht, der bzw. das an C4.4a bindet und über eine Cysteingruppe an die Toxophor-Linker-Einheit gebunden ist und
- AK₂: für einen humanen oder humanisierten Antikörper oder ein Antigen-bindendes Antikörperfragment steht, der bzw. das an C4.4a bindet und über eine Lysingruppe an die Toxophor-Linker-Einheit gebunden ist, steht.

Besonders bevorzugt sind Wirkstoff-Binder-Konjugate ausgewählt aus den folgenden Verbindungen: 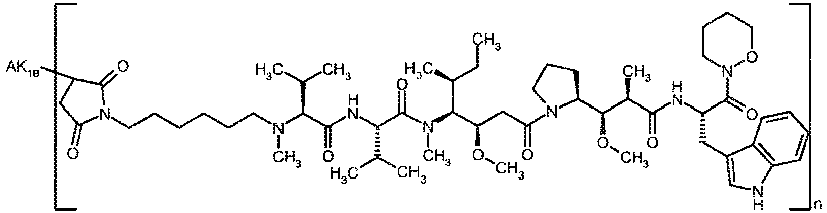 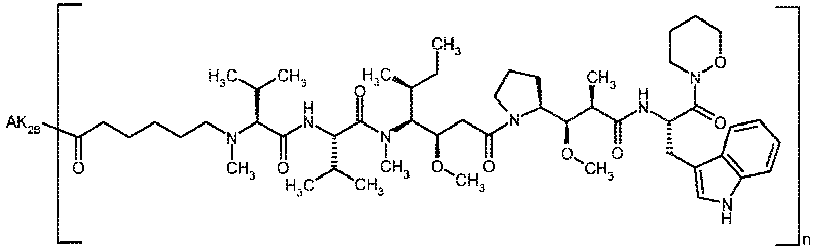 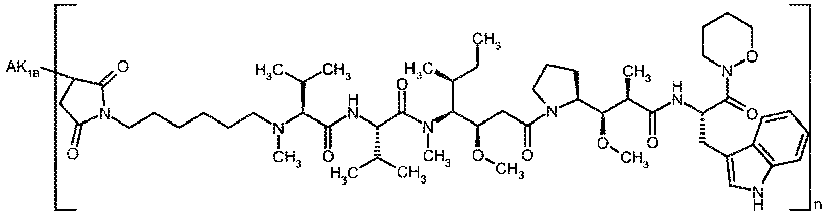 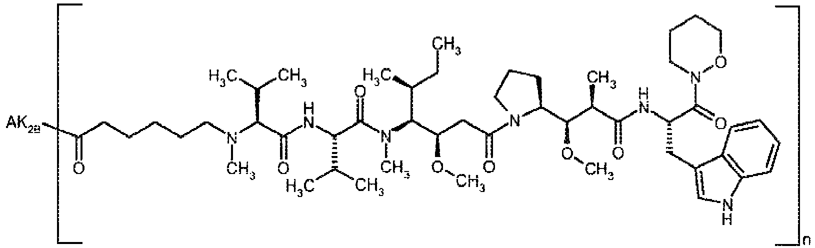 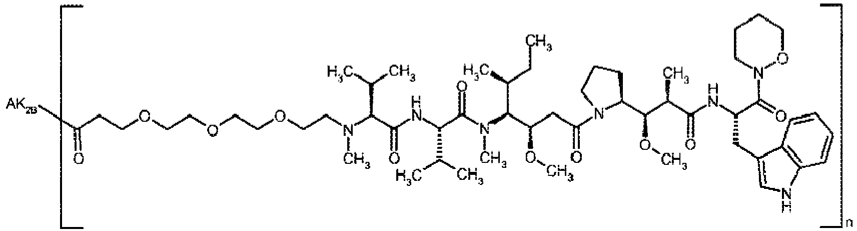 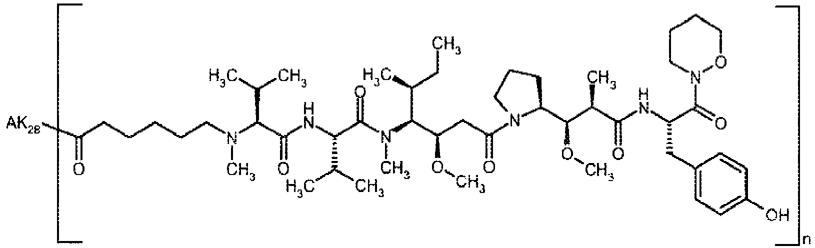 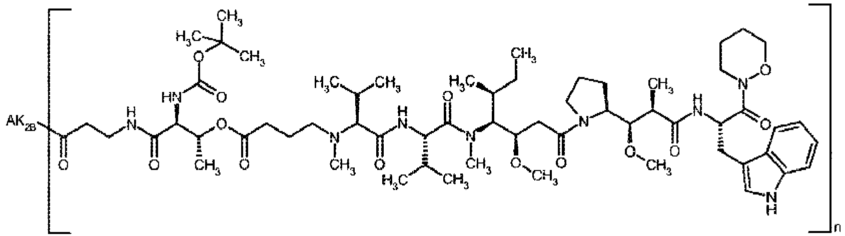 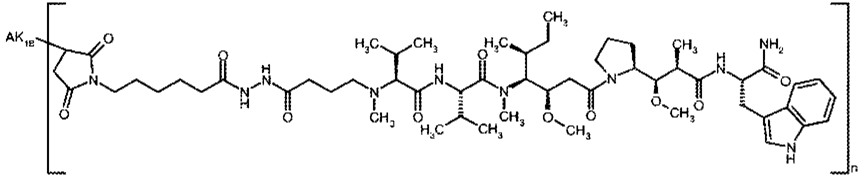 wobei jeweils
- n: für eine Zahl von 2 bis 8, vorzugsweise 2 bis 5 steht,
und
- AK_{1B} und AK_{2B}: für B01-3 steht.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (Ia), dadurch gekennzeichnet, dass man eine Lösung des Binders (vorzugsweise in Puffer wie z.B. PBS-Puffer)
[A] mit einem geeigneten Reduktionsmittel, wie beispielsweise Dithiothreitol oder Tris(2-carboxyethyl)phosphin-Hydrochlorid, versetzt, und anschließend mit einer Verbindung der Formel (IIa) 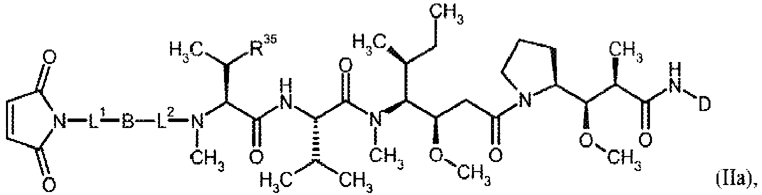 in welcher D, L¹, B, L² und R³⁵ jeweils die oben angegebenen Bedeutungen haben, zu einer Verbindung der Formel (I-A) 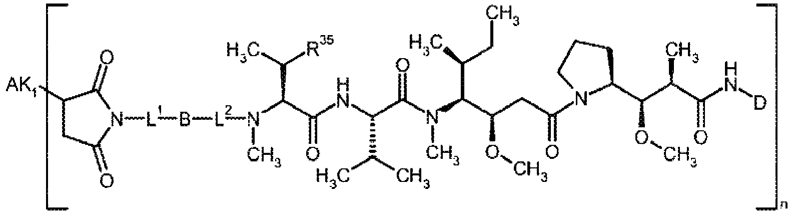 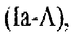 in welcher n, AK₁, D, L¹, B, L² und R³⁵ jeweils die oben angegebenen Bedeutungen haben, umsetzt,
   oder
[B] mit einer Verbindung der Formel (IIIa) 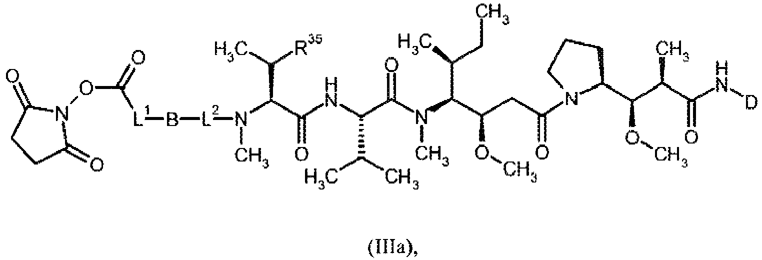 in welcher D, L¹, B, L² und R³⁵ jeweils die oben angegebenen Bedeutungen haben, zu einer Verbindung der Formel (Ia-B) 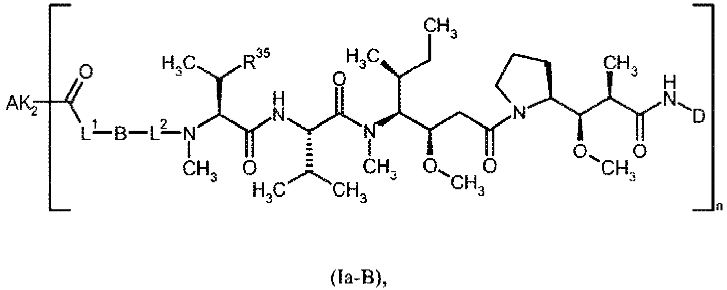 in welcher n, AK₂, D, L¹, B, L² und R³⁵ jeweils die oben angegebenen Bedeutungen haben, umsetzt.

Weiterer Gegenstand der Erfindung ist ein Verfahren zur Herstellung der erfindungsgemäßen Verbindungen der Formel (I), dadurch gekennzeichnet, dass man eine Lösung des Binders in PBS-Puffer
[A] mit einem geeigneten Reduktionsmittel, wie beispielsweise Dithiothreitol oder Tris(2-carboxyethyl)phosphin-Hydrochlorid, versetzt, und anschließend mit einer Verbindung der Formel (II) 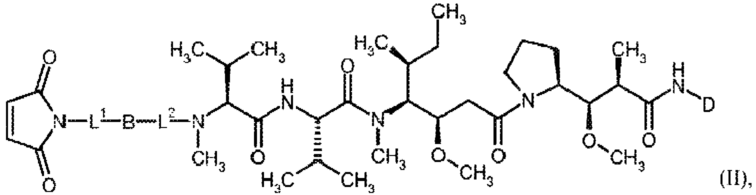 in welcher D, L¹, B und L² jeweils die oben angegebenen Bedeutungen haben, zu einer Verbindung der Formel (I-A) 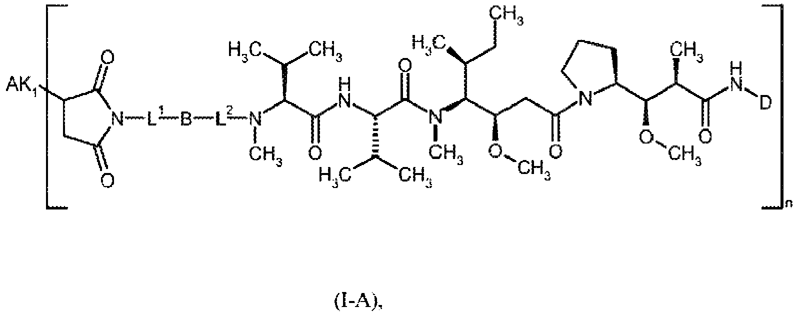 in welcher n, AK₁, D, L¹, B und L² jeweils die oben angegebenen Bedeutungen haben, umsetzt,
   oder
[B] mit einer Verbindung der Formel (III) 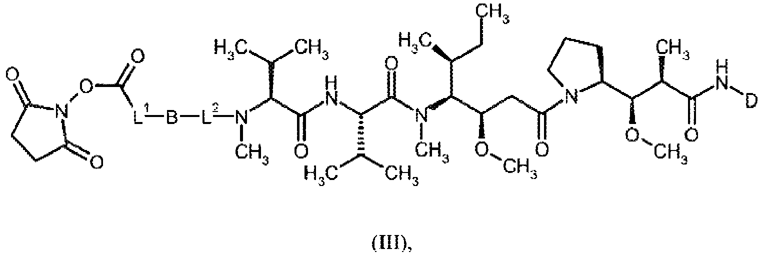 in welcher D, L¹, B und L² jeweils die oben angegebenen Bedeutungen haben, zu einer Verbindung der Formel (I-B) 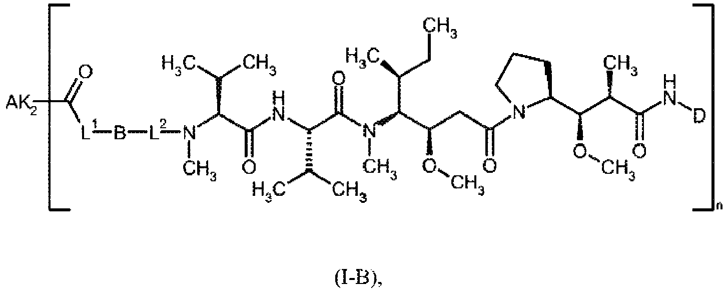 in welcher n, AK₂, D, L¹, B und L² jeweils die oben angegebenen Bedeutungen haben, umsetzt.

### Cystein-Kupplung:

Die partielle Reduktion des Antikörpers sowie die anschließende Konjugation des (partiell) reduzierten Antikörpers mit einer Verbindung der Formel (II) bzw. (IIa) erfolgt nach den dem Fachmann bekannten Methoden, siehe z.B. Ducry et. al., Bioconj. Chem. 2010, 21, 5 und Referenzen hierin, Klussman et.al., Bioconj. Chem. 2004, 15(4), 765-773. Bevorzugt erfolgt die milde Reduktion des Antikörpers durch Zugabe von 2-6 Equivalenten TCEP zu dem in geeigneter Pufferlösung, bevorzugt Phospatpuffer, vorliegenden Antikörpers und 30-180-minütigem Rühren bei Temperaturen zwischen 15 und 40°C, bevorzugt bei RT. Anschließend erfolgt die Konjugation durch Zugabe einer Lösung einer Verbindung der Formel (II) bzw. (IIa) in DMSO, Acetonitril oder DMF zur Lösung des (partiell) reduzierten Antikörpers in PBS-Puffer, und anschliessender Umsetzung bei einer Temperatur von 0°C bis +40°C, insbesondere von + 10°C bis +30°C, für einen Zeitraum von 30 min bis 6 Stunden, insbesondere 1 bis 2 Stunden.

### Lysin-Kupplung:

Zunächst werden die Verbindungen der Formel (III) bzw. (IIa) oder vergleichbare aktivierte Carboxylkomponenten nach klassischen Methoden der Peptidchemie hergestellt. Diese werden dann in inerten Lösungsmitteln wie z.B. DMSO oder DMF aufgenommen und zu dem bevorzugt in Phosphatpuffer bei neutralem pH-Wert vorliegenden Antikörper zugegeben. Die Lösung wird 1-16h bei einer Temperatur zwischen 15 und 40°C, bevorzugt RT gerührt.

Die zuvor beschriebenen Herstellungsverfahren werden anhand der nachfolgenden Schemata (Schema 1 und 2) beispielhaft erläutert: 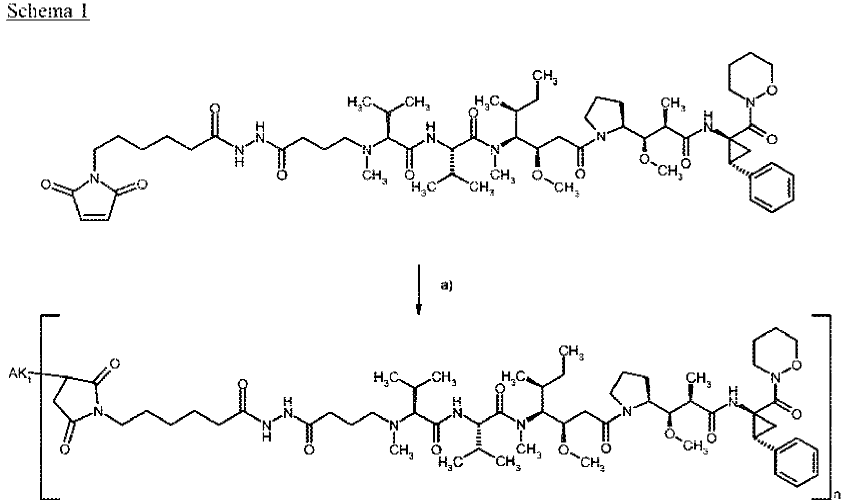 [a): 1. AK, TCEP, PBS-Puffer, RT; 2. Zugabe vom Maleinimid-Derivat in DMSO, RTJ. 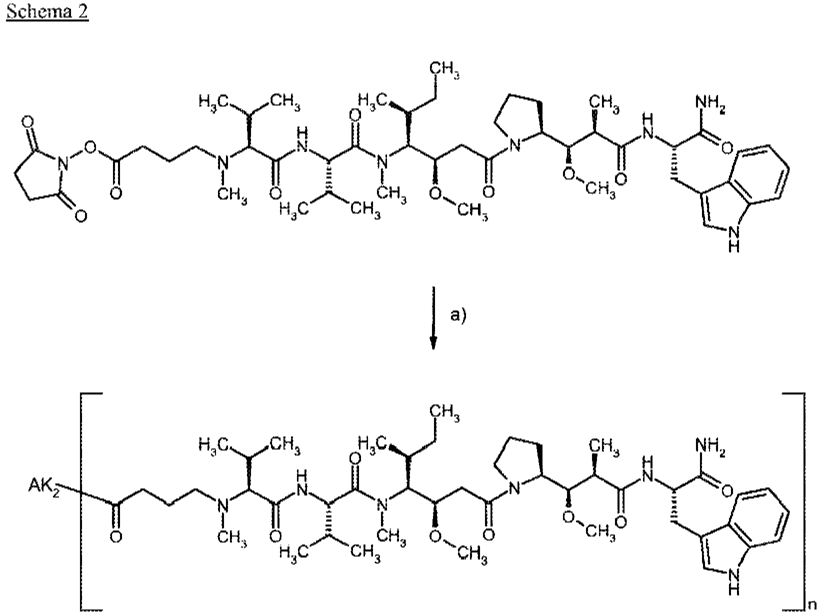 [a): AK, PBS-Puffer, RT mit aktiviertem Carboxylderivat der Linker-Wirkstoff-Komponenten versetzen].

Die Verbindungen der Formel (II), in welcher L¹ und B für eine Bindung stehen, können hergestellt werden, indem man eine Verbindung der Formel (IV) 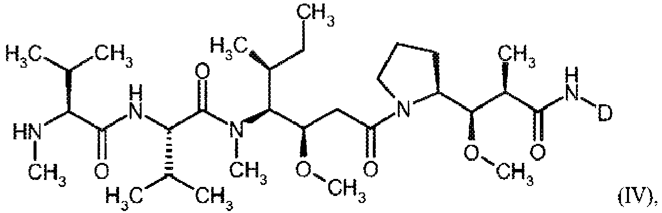 in welcher D die oben angegebene Bedeutung hat,
in einem inerten Lösungsmittel mit einer Verbindung der Formel (V) 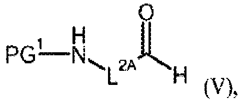 in welcher
- L^{2A}: die oben definierte Bedeutung von L² hat, jedoch in der Alkyl-Kettenlänge um ein Kohlenstoffatom verkürzt ist,
- PG¹: für eine Amino-Schutzgruppe wie beispielsweise (9*H*-Fluoren-9-ylmethoxy)carbonyl, *tert.-*Butoxycarbonyl oder Benzyloxycarbonyl steht,
zu einer Verbindung der Formel (VI) 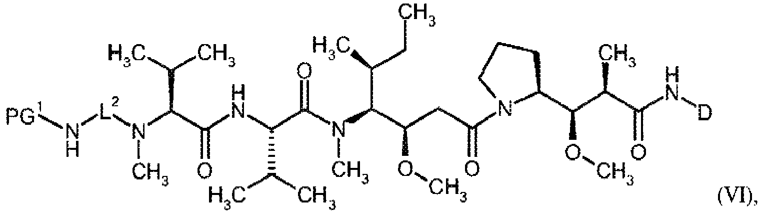 in welcher D, L³ und PG¹ die oben angegebene Bedeutung hat,
reduktiv aminiert, von dieser Verbindung nach dem Fachmann bekannten Methoden die Schutzgruppe PG¹ abspaltet, und die entschützte Verbindung in einem inerten Lösungsmittel in Gegenwart einer geeigneten Base mit Methyl-2,5-dioxo-2,5-dihydro-1H-pyrrol-1-carboxylat zu einer Verbindung der Formel (II-A) 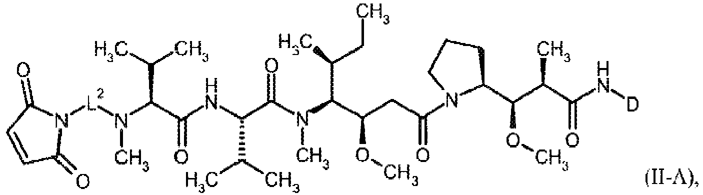 in welcher D und L² jeweils die oben angegebenen Bedeutungen haben,
umsetzt.

Die Verbindungen der Formel (II), in welcher B für eine Gruppe der Formel (B¹) 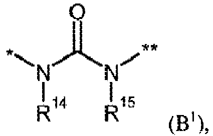 in weicher *, **, R¹⁴ und R¹⁵ jeweils die oben angegebenen Bedingungen haben,
steht, können hergestellt werden, indem man von einer Verbindung der Formel (VI) nach dem Fachmann bekannten Methoden die Schutzgruppe PG¹ abspaltet, und die entschützte Verbindung in einem inerten Lösungsmittel in Gegenwart einer geeigneten Base mit einer Verbindung der Formel (VII) 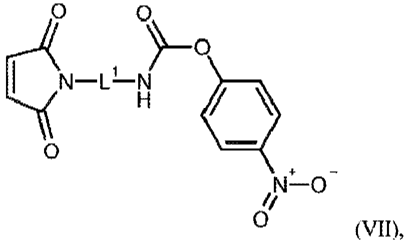 in weicher L¹ die oben angegebene Bedeutung hat,
zu einer Verbindung der Formel (II-B) 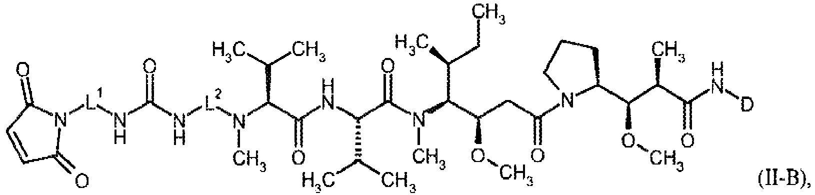 in welcher D, L¹ und L² jeweils die oben angegebenen Bedeutungen haben,
umsetzt.

Die Verbindungen der Formel (II), in welcher B für eine Gruppe der Formel (B²) 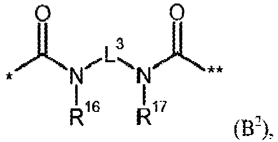 in welcher *, **, L³, R¹⁶ und R¹⁷ jeweils die oben angegebenen Bedingungen haben,
steht, können hergestellt werden, indem man eine Verbindung der Formel (IV)

in einem inerten Lösungsmittel mit einer Verbindung der Formel (VIII) 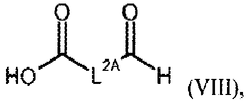 in welcher
- L^{2A}: die oben definierte Bedeutung von L² hat, jedoch in der Alkyl-Kettenlänge um ein Kohlenstoffatom verkürzt ist,
zu einer Verbindung der Formel (IX) 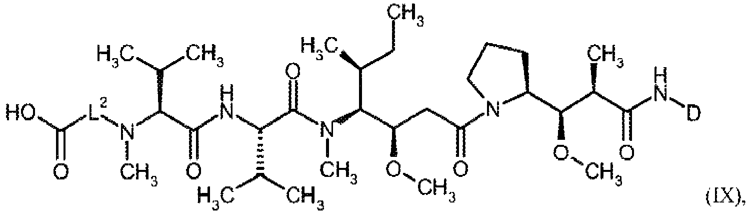 in welcher D und L² die oben angegebenen Bedeutungen haben,
reduktiv aminiert, und diese Verbindung in einem inerten Lösungsmittel in Gegenwart eines geeigneten Kupplungsreagenzes und einer geeigneten Base mit einer Verbindung der Formel (X) 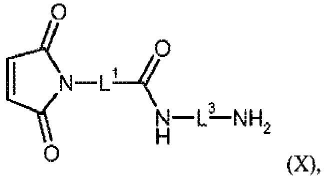 in welcher L¹ und L³ jeweils die oben angegebenen Bedeutungen haben,
zu einer Verbindung der Formel (II-C) 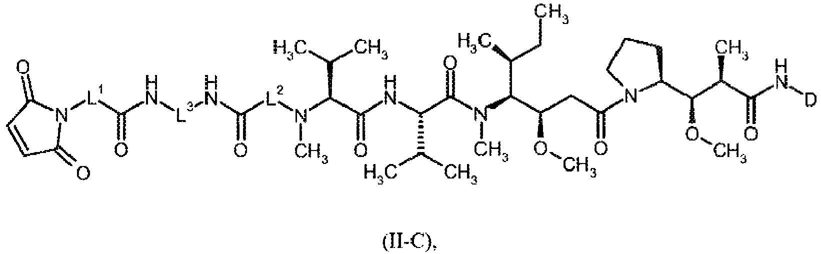 in welcher D, L¹, L² und L³ jeweils die oben angegebenen Bedeutungen haben,
umsetzt.

Verbindung der Formel (II), in welcher B für eine Gruppe der Formel (B³) 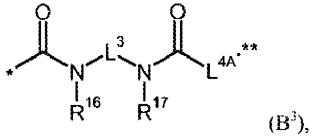 in welcher *, **, L³, R¹⁶ und R¹⁷ jeweils die oben angegebenen Bedingungen haben und
- L^{4A}: für eine Gruppe der Formel 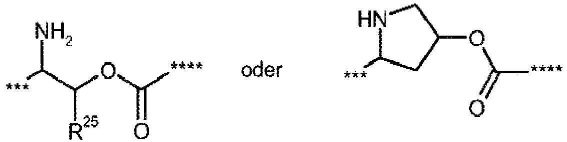
steht, worin
- ***: die Verknüpfungsstelle mit der Carbonylgruppe kennzeichnet,
- ****: die Verknüpfungsstelle mit L² kennzeichnet,
- R²⁵: für Wasserstoff oder Methyl steht,
steht,
können hergestellt werden, indem man eine Verbindung der Formel (IX) in einem inerten Lösungsmittel in Gegenwart einer geeigneten Base und eines geeigneten Kupplungsreagenzes mit einer Verbindung der Formel (XI-A) oder (XI-B) 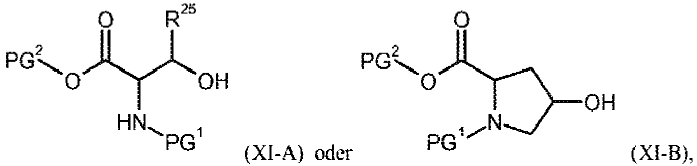 in welchen R²⁵ und PG¹ jeweils die zuvor angegebenen Bedeutungen haben und
- PG²: für eine geeignete Carboxyl-Schutzgruppe, insbesondere Benzyl, steht,
zu einer Verbindung (XII-A) bzw. (XII-B) 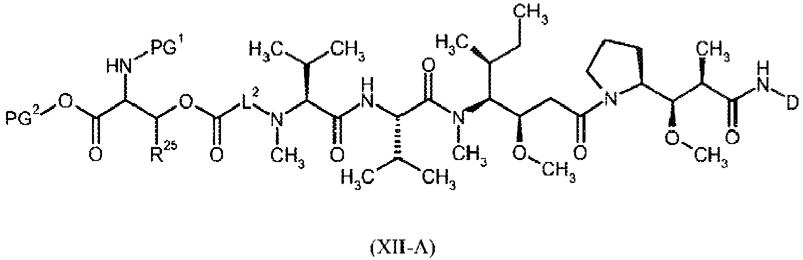 bzw. 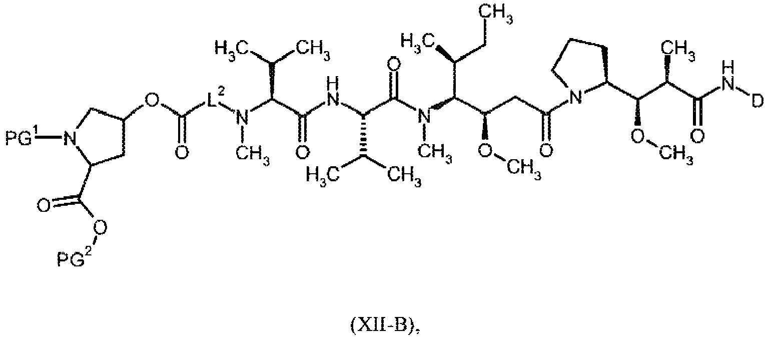 in welchen D, PG¹, PG² und L² die oben angegebenen Bedeutungen haben,
umsetzt, von dieser anschliessend nach dem Fachmann bekannten Methoden die Schutzgruppe PG² abspaltet und die entschützte Verbindung in einem inerten Lösungsmittel in Gegenwart eines geeigneten Kupplungsreagenzes und einer geeigneten Base mit einer Verbindung der Formel (X) umsetzt, und von dieser schließlich nach dem Fachmann bekannten Methoden die Schutzgruppe PG1 zu einer Verbindung der Formel (II-D-A) bzw. (II-D-B) 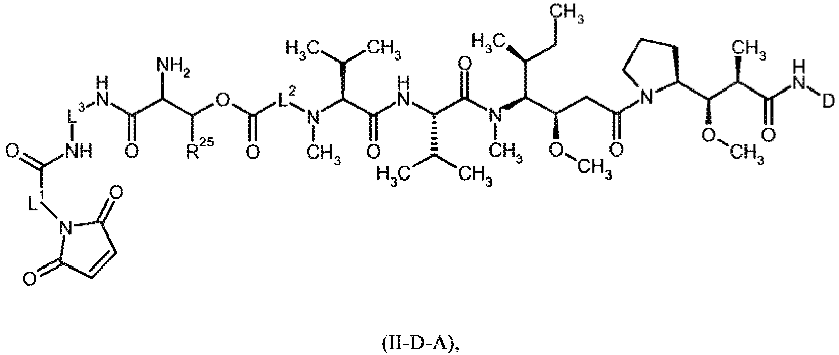 bzw. 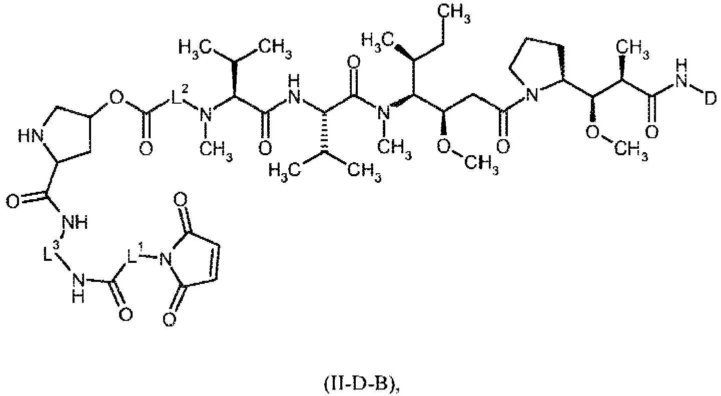 in welchen D, L¹, L² und L³ die oben angegebenen Bedeutungen haben,
abspaltet.

Verbindung der Formel (II), in welcher B für eine Gruppe der Formel (B⁴) 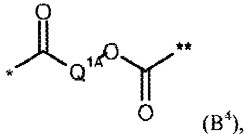 in welcher *, ** jeweils die oben angegebenen Bedingungen haben und
- Q^{1A}: für einen N-verknüpften 4- bis 7-gliedrigen Heterocyclus steht,
können hergestellt werden, indem man eine Verbindung der Formel (IX) in einem inerten Lösungsmittel in Gegenwart einer geeigneten Base und eines geeigneten Kupplungsreagenzes mit einer Verbindung der Formel (XXI) 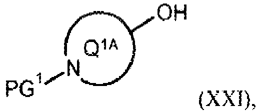 in welcher PG¹ und Q^{1A} jeweils die oben angegebenen Bedeutungen haben,
zu einer Verbindung der Formel (XXII) 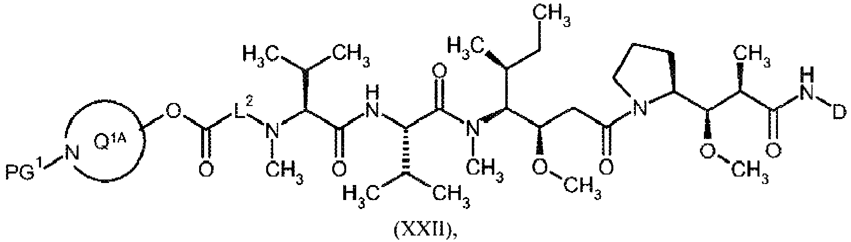 in welcher PG¹, Q^{1A}, D und L² die oben angegebenen Bedeutungen haben,
umsetzt, von dieser nach dem Fachmann bekannten Methoden die Schutzgruppe PG¹ abspaltet und die entschützte Verbindung anschliessend in einem inerten Lösungsmittel in Gegenwart eines geeigneten Kupplungsreagenzes und einer geeigneten Base mit einer Verbindung der Formel (XXIII) 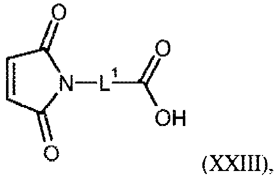 in welcher L¹ die oben angegebene Bedeutung hat,
zu einer Verbindung der Formel (II-D) 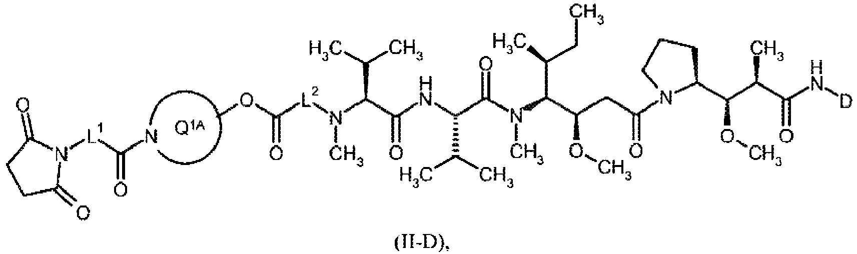 in welcher Q^{1A}, D, L¹ und L² die oben angegebenen Bedeutungen haben,
umsetzt.

Die Verbindungen der Formel (III), in welcher L¹ und B für eine Bindung stehen, können hergestellt werden, indem man eine Verbindung der Formel (IX) in einem inerten Lösungsmittel in Gegenwart eines geeigneten Kupplungsreagenzes und einer geeigneten Base mit N-Hydroxysuccinimid zu einer Verbindung der Formel (III-A) 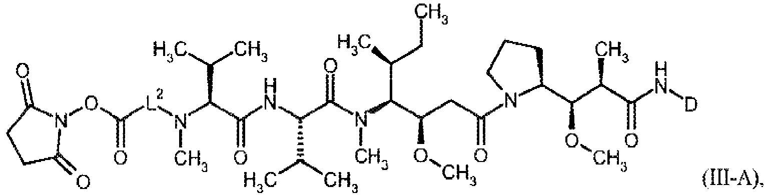 in welcher D und L² jeweils die oben angegebenen Bedeutungen haben,
umsetzt.

Die Verbindungen der Formel (III), in welcher L¹ für eine Bindung und B für eine Gruppe der Formel (B^{5A}) 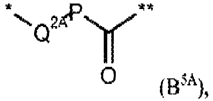 in welcher *, ** und P jeweils die oben angegebenen Bedeutungen haben und
- Q^{2A}: für einen 3- bis 7-gliedrigen Carbocyclus steht,
stehen, können hergestellt werden, indem man eine Verbindung der Formel (IX) in einem inerten Lösungsmittel in Gegenwart eines geeigneten Kupplungsreagenzes und einer geeigneten Base mit einer Verbindung der Formel (XIII) 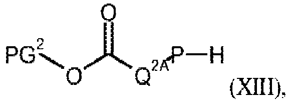 in welcher P, Q^{2A} und PG² jeweils die oben angegebenen Bedeutungen haben,
zu einer Verbindung der Formel (XIV) 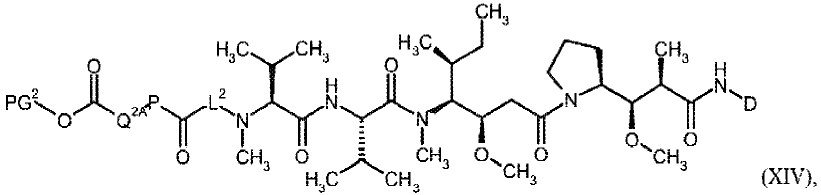 in welcher D, P, Q^{2A}, L² und PG² jeweils die oben angegebenen Bedeutungen haben,
umsetzt, von dieser nach dem Fachmann bekannten Methoden die Schutzgruppe PG² abspaltet und die entschützte Verbindung anschliessend in einem inerten Lösungsmittel in Gegenwart einer geeigneten Base mit N-Hydroxysuccinimid zu einer Verbindung der Formel (III-B) 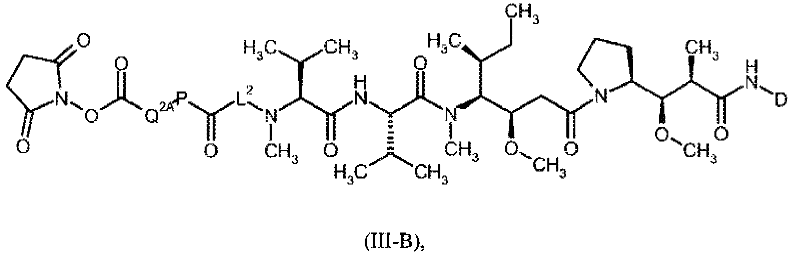 in welcher D, P, Q^{2A} und L² jeweils die oben angegebenen Bedeutungen haben,
umsetzt.

Die Verbindungen der Formel (III), in welcher L¹ für eine Bindung und B für eine Gruppe der Formel (B⁶) 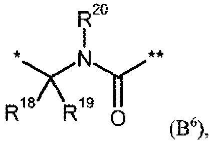 in welcher *, **, R¹⁸, R¹⁹ und R²⁰ jeweils die oben angegebenen Bedeutungen haben,
stehen, können hergestellt werden, indem man eine Verbindung der Formel (IX) in einem inerten Lösungsmittel in Gegenwart eines geeigneten Kupplungsreagenzes und einer geeigneten Base mit einer Verbindung der Formel (XV) 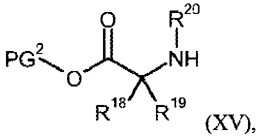 in welcher R¹⁸, R¹⁹, R²⁰ und PG² jeweils die oben angegebenen Bedeutungen haben,
zu einer Verbindung der Formel (XVI) 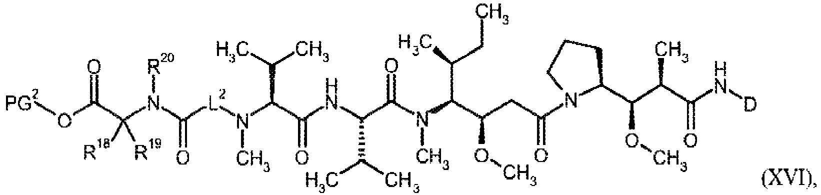 in welcher D, R¹⁸, R¹⁹, R²⁰, L² und PG² jeweils die oben angegebenen Bedeutungen haben,
umsetzt, von dieser nach dem Fachmann bekannten Methoden die Schutzgruppe PG² abspaltet und die entschützte Verbindung anschliessend in einem inerten Lösungsmittel in Gegenwart eines geeigneten Kupplungsreagenzes und einer geeigneten Base mit N-Hydroxysuccinimid zu einer Verbindung der Formel (III-C) 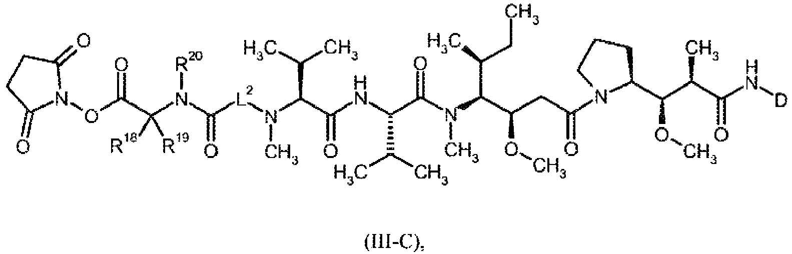 in weicher D, R¹⁸, R¹⁹, R²⁰ und L² jeweils die oben angegebenen Bedeutungen haben,
umsetzt.

Die Verbindungen der Formel (III), in welcher L¹ für eine Bindung und B für eine Gruppe der Formel (B⁷) 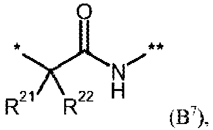 in welcher *, **, R²¹ und R²² jeweils die oben angegebenen Bedeutungen haben,
stehen, können hergestellt werden, indem man von einer Verbindung der Formel (VI) nach dem Fachmann bekannten Methoden die Schutzgruppe PG¹ abspaltet, und die resultierende entschützte Verbindung in einem inerten Lösungsmittel in Gegenwart einer geeigneten Base mit einer Verbindung der Formel (XVII) 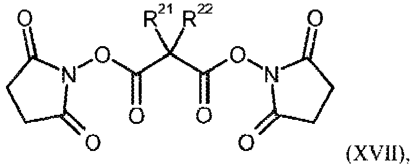 in welcher R²¹ und R²² jeweils die oben angegebenen Bedeutungen haben,
zu einer Verbindung der Formel (III-D) 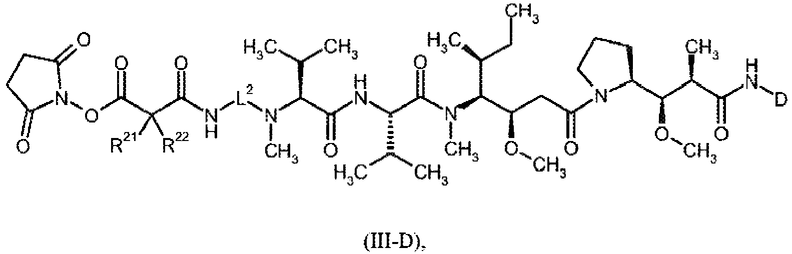 in welcher D, R²¹, R²² und L² jeweils die oben angegebenen Bedeutungen haben,
umsetzt.

Die Verbindungen der Formel (III), in welcher B für eine Gruppe der Formel (B⁸) 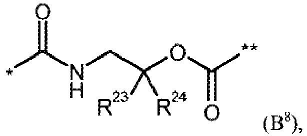 in welcher *, **, R²³ und R²⁴ jeweils die oben angegebenen Bedeutungen haben,
steht, können hergestellt werden, indem man eine Verbindung der Formel (IX) in einem inerten Lösungsmittel in Gegenwart eines geeigneten Kupplungsreagenzes und einer geeigneten Base mit einer Verbindung der Formel (XVIII) 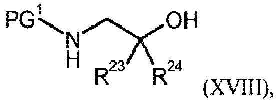 in welcher R²³, R²⁴ und PG¹ jeweils die oben angegebenen Bedeutungen haben,
zu einer Verbindung der Formel (XIX) 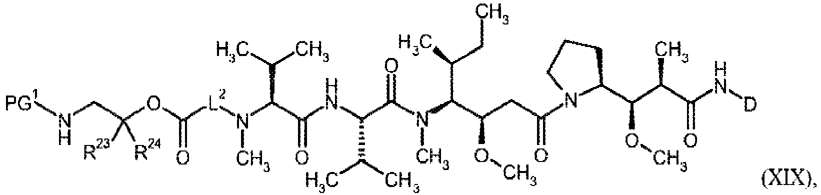 in welcher D, R²³, R²⁴, L² und PG¹ jeweils die oben angegebenen Bedeutungen haben,
umsetzt, von dieser nach dem Fachmann bekannten Methoden die Schutzgruppe PG¹ abspaltet und die entschützte Verbindung anschliessend in einem inerten Lösungsmittel in Gegenwart eines geeigneten Kupplungsreagenzes und einer geeigneten Base mit einer Verbindung der Formel (XX) 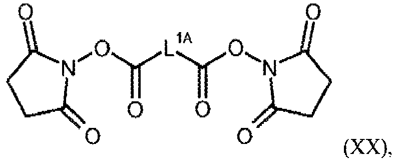 in welcher
- L^{1A}: für lineares (C₁-C₁₀)-Alkandiyl oder für eine Gruppe der Formel 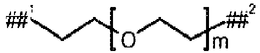 steht, wobei
m für eine Zahl von 2 bis 6 steht,
##¹ die Verknüpfungsstelle mit der Gruppe G kennzeichnet,
##² die Verknüpfungsstelle mit der Gruppe B kennzeichnet,
wobei (C₁-C₁₀)-Alkandiyl mit 1 bis 4 Substituenten Methyl substiuiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
zu einer Verbindung der Formel (III-E) 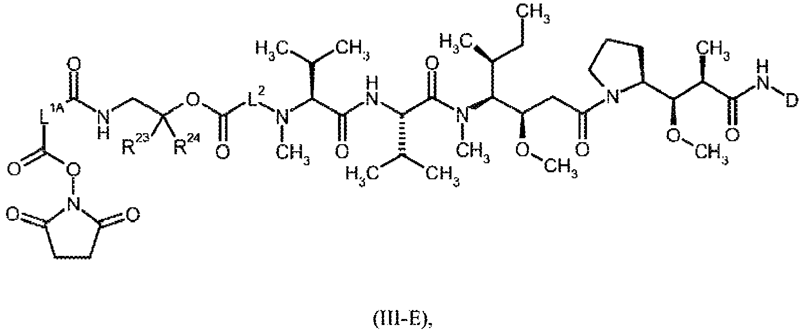 in welcher D, R²³, R²⁴, L^{1A} und L² jeweils die oben angegebenen Bedeutungen haben,
umsetzt.

Die Verbindungen der Formel (III), in welcher B für eine Gruppe der Formel (B^{5B}) 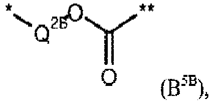 in welcher * und ** jeweils die oben angegebenen Bedeutungen haben und
- Q^{2B}: für einen N-verknüpften 4- bis 7-gliedrigen Heterocyclus steht,
können hergestellt werden, indem man eine Verbindung der Formel (IX) in einem inerten Lösungsmittel in Gegenwart einer geeigneten Base und eines geeigneten Kupplungsreagenzes mit einer Verbindung der Formel (XXIV) 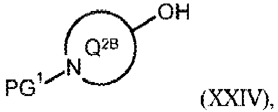 in welcher PG¹ und Q^{2B} jeweils die oben angegebenen Bedeutungen haben,
zu einer Verbindung der Formel (XXV) 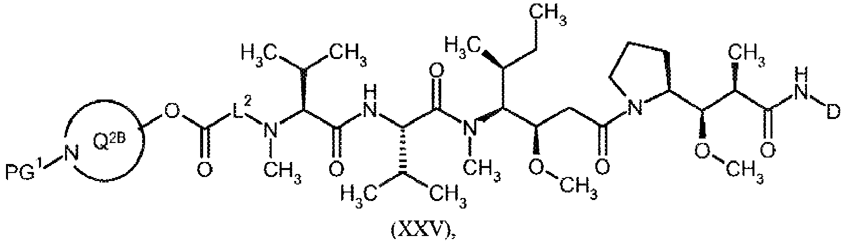 in welcher PG¹, Q^{2B}, D und L² die oben angegebenen Bedeutungen haben,
umsetzt, von dieser nach dem Fachmann bekannten Methoden die Schutzgruppe PG¹ abspaltet,
und die entschützte Verbindung anschliessend in einem inerten Lösungsmittel in Gegenwart einer geeigneten Base mit einer Verbindung der Formel (XX) in eine Verbindung der Formel (III-F) 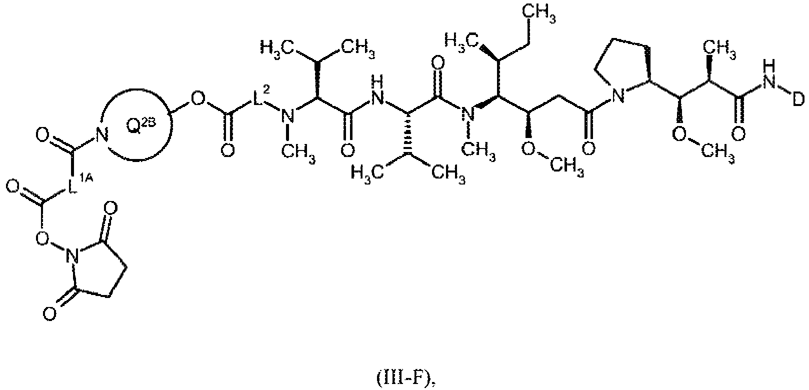 in welcher Q^{2B}, D, L^{1A} und L² die oben angegebenen Bedeutungen haben,
überführt.

Die Umsetzungen (IV) + (V) → (VI) und (IV) (VIII) → (IX) erfolgen in den für eine reduktive Aminierung üblichen, unter den Reaktionsbedingungen inerten Lösungsmitteln, gegebenenfalls in Gegenwart einer Säure und/ oder eines wasserentziehenden Mittels als Katalysator. Zu solchen Lösungsmitteln gehören beispielsweise Alkohole wie Methanol, Ethanol, *n*-Propanol, Isopropanol, *n*-Butanol oder *tert.*-Butanol, Ether wie Tetrahydrofuran, 1,4-Dioxan, 1,2-Dimethoxyethan oder Bis-(2-methoxyethyl)-ether, oder andere Solventien wie Dichlormethan, 1,2-Dichlorethan, *N,N*-Dimethylformamid oder auch Wasser. Ebenso ist es möglich, Gemische dieser Lösungsmittel einzusetzen. Bevorzugt wird als Lösungsmittel ein 1,4-Dioxan/Wasser-Gemisch verwendet unter Zusatz von Essigsäure oder verdünnter Salzsäure als Katalysator.

Als Reduktionsmittel eignen sich für diese Reaktion insbesondere komplexe Borhydride, wie beispielsweise Natriumborhydrid, Natriumcyanoborhydrid, Natriumtriacetoxyborhydrid, Tetra*n*-butylammoniumborhydrid oder Boran-Pyridin-Komplex. Bevorzugt wird Natriumcyanoborhydrid oder Boran-Pyridin-Komplex eingesetzt.

Die Umsetzungen (IV) (V) → (VI) und (IV) + (VIII) → (IX) erfolgen im Allgemeinen in einem Temperaturbereich von 0°C bis +120°C, bevorzugt bei +50°C bis + 100°C. Die Reaktionen können bei normalem, bei erhöhtem oder bei erniedrigtem Druck durchgeführt werden (z.B. von 0.5 bis 5 bar); in der Regel arbeitet man bei Normaldruck.

Die oben beschriebenen Kupplungsreaktionen (IX) + (X) → (II-C), (XII-A) bzw. (XII-B) + (X) → (II-D-A) bzw. (II-D-B), (IX) (XIII) → (XIV), (IX) + (XV) → (XVI) und (XXII) + (XXIII) → (II-D) (Amid-Bildung aus jeweiliger Amin- und Carbonsäure-Komponente) werden nach allgemein üblichen Methoden der Peptidchemie durchgeführt [siehe z.B. M. Bodanszky, Principles of Peptide Synthesis, Springer-Verlag, Berlin, 1993; M. Bodanszky und A. Bodanszky, The Practice of Peptide Synthesis, Springer-Verlag, Berlin, 1984; H.-D. Jakubke und H. Jeschkeit, Aminosäuren, Peptide, Proteine, Verlag Chemie, Weinheim, 1982].

Inerte Lösungsmittel für diese Kupplungsreaktionen sind beispielsweise Ether wie Diethylether, Diisopropylether, *tert*.-Butylmethylether, Tetrahydrofuran, 1,4-Dioxan, 1,2-Dimethoxyethan oder Bis-(2-methoxyethyl)-ether, Kohlenwasserstoffe wie Benzol, Toluol, Xylol, Pentan, Hexan, Heptan, Cyclohexan oder Erdölfraktionen, Halogenkohlenwasserstoffe wie Diehlormethan, Trichlormethan, Tetrachlormethan, 1,2-Dichlorethan, Trichlorethylen oder Chlorbenzol, oder dipolaraprotische Lösungsmittel wie Aceton, Methylethylketon, Acetonitril, Ethylacetat, Pyridin, Dimethylsulfoxid (DMSO), *N,N*-Dimethylformamid (DMF), *N,N*-Dimethylacetamid (DMA), *N,N'-*Dimethylpropylenharnstoff (DMPU) oder *N*-Methylpyrrolidinon (NMP). Ebenso ist es möglich, Gemische solcher Lösungsmittel zu verwenden. Bevorzugt wird *N*,*N*-Dimethylformamid eingesetzt.

Als Aktivicrungs-/Kondensationsmittel für diese Kupplungen eignen sich beispielsweise Carbodiimide wie *N,N'*-Diethyl-, *N*,*N'*-Dipropyl-, *N,N*'-Diisopropyl-, *N,N*'-Dicyclohexylcarbodiimid (DCC) oder *N*-(3-Dimethylaminoisopropyl)-*N*'-ethylcarbodiimid-Hydrochlorid (EDC), Phosgen-Derivate wie *N*,*N*'-Carbonyldiimidazol (CDI) oder Isobutylchlorformiat, 1,2-Oxazolium-Verbindungen wie 2-Ethyl-5-phenyl-1,2-oxazolium-3-sulfat oder 2-*tert*.-Butyl-5-methylisoxazolium-perchlorat, Acylamino-Verbindungen wie 2-Ethoxy-1-ethoxycarbonyl-1,2-dihydrochinolin, Phosphor-Verbindungen wie Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat oder Benzotriazol-1-yloxy-tris(pyrrolidino)phosphonium-hexafluorophosphat (PyBOP), oder Uronium-Verbindungen wie *O*-(Benzotriazol-1-yl)-*N*,*N,N*',*N*'-tetramethyluronium-tetrafluoroborat (TBTU), *O*-(Benzotriazol-1-yl)-*N*,*N*,*N'*,*N'*-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N*,*N*,*N',N'*-tetramethyluronium-hexafluorophosphat (HATU) oder *O*-(1*H*-6-Chlorbenzotriazol-1-yl)-1,1,3,3-tetramethyluronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder tertiäre Aminbasen wie Triethylamin, *N*-Methylmorpholin, *N*-Methylpiperidin, *N*,*N*-Diisopropylethylamin, Pyridin oder 4-*N*,*N*-Dimethylaminopyridin.

Im Rahmen der vorliegenden Erfindung wird als Aktivierungs-/Kondensationsmittel für solche Kupplungsreaktionen bevorzugt *N*-(3-Dimethylaminoisopropyl)-*N'*-ethylcarbodiimid-Hydrochlorid (EDC) in Kombination mit 1-Hydroxybenzotriazol (HOBt) und *N,N*-Diisopropylethylamin, oder *O*-(7-Azabenzotriazol-1-yl)-*N*,*N*,*N'*,*N'*-tetramethyluronium-hexafluorophosphat (HATU) gleichfalls in Verbindung mit *N,N*-Diisopropylethylamin verwendet.

Die Kupplungsreaktionen (IX) + (X) → (II-C), (XII-A) bzw. (XII-B) + (X) → (II-D-A) bzw. (II-D-B), (IX) + (XIII) → (XIV), (IX) + (XV) → (XVI) und (XXII) + (XXIII) → (II-D) werden in der Regel in einem Temperaturbereich von -20°C bis +60°C, bevorzugt bei 0°C bis +40°C durchgeführt. Die Umsetzungen können bei normalem, bei erhöhtem oder bei erniedrigtem Druck erfolgen (z.B. von 0.5 bis 5 bar); im Allgemeinen arbeitet man bei Normaldruck.

Die Esterbildungen (IX) + (XVIII) → (XII) und (IX) + (XI-A) bzw. (XI-B) → (XII-A) bzw. (XII-B), (IX) + (XXIV) → (XXV) sowie (IX) + (XXI) → (XXII) erfolgen in Analogie zu den oben beschriebenen Amid-Kupplungsreaktionen. Bevorzugt erfolgen diese Reaktionen in Dichlormethan unter Verwendung von *N*-(3-Dimethylaminoisopropyl)-*N'*-ethylarbodiimid-Hydrochlorid (EDC) und 4-Dimethylaminopyridin bei einer Temperatur von +50°C bis 100°C bei Normaldruck.

Die in den Verbindungen gegebenenfalls vorhandenen funktionellen Gruppen - wie insbesondere Amino-, Hydroxy- und Carboxylgruppen - können bei den zuvor beschriebenen Verfahrenssehritten, falls zweckmäßig oder erforderlich, auch in temporär geschützter Form vorliegen. Die Einführung und Entfernung solcher Schutzgruppen erfolgt hierbei nach üblichen, aus der Peptidchemie bekannten Methoden [siehe z.B. T.W. Greene und P.G.M. Wuts, Protective Groups in Organic Synthesis, Wiley, New York, 1999; M. Bodanszky und A. Bodanszky. The Practice of Peptide Synthesis, Springer-Verlag, Berlin, 1984]. Bei Vorhandensein mehrerer geschützter Gruppen kann deren Wiederfreiseizung gegebenenfalls simultan in einer Eintopf-Reaktion oder auch in separaten Reaktionsschritten vorgenommen werden.

Als Amino-Schutzgruppe PG¹ wird bevorzugt *tert.*-Butoxycarbonyl (Boc), Benzyloxycarbonyl (Z) oder (9*H*-Fluoren-9-ylmethoxy)carbonyl (Fmoc) verwendet; für eine Hydroxy- oder CarboxylFunktion wird vorzugsweise *tert*.-Butyl oder Benzyl als Schutzgruppe PG² eingesetzt. Die Abspaltung einer *tert*.-Butyl- oder *tert*.-Butoxycarbonyl-Gruppe geschieht üblicherweise durch Behandlung mit einer starken Säure, wie Chlorwasserstoff, Bromwasserstoff oder Trifluoressigsäure, in einem inerten Lösungsmittel wie Diethylether, 1,4-Dioxan, Dichlormethan oder Essigsäure; gegebenenfalls kann diese Reaktion auch ohne Zusatz eines inerten Lösungsmittels durchgeführt werden. Im Falle von Benzyl oder Benzyloxycarbonyl als Schutzgruppe werden diese bevorzugt durch Hydrogenolyse in Gegenwart eines geeigneten Palladium-Katalysators, wie beispielsweise Palladium auf Aktivkohle, entfernt. Die (9*H*-Fluoren-9-ylmethoxy)carbonyl-Gruppe wird im Allgemeinen mit Hilfe einer sekundären Aminbase wie Diethylamin oder Piperidin abgespalten.

Die Umsetzung (VI) → (II-A) erfolgt in einem unter den Reaktionsbedingungen inerten Lösungsmittel wie beispielsweise Ether wie Tetrahydrofuran, 1,4-Dioxan, 1,2-Dimethoxyethan oder Bis-(2-methoxyethyl)-ether, Alkohole wie Methanol, Ethanol, Isopropanol, n-Butanol oder *tert*.-Butanol, oder dipolar-aprotische Lösungsmittel wie Aceton, Methylethylketon, Acetonitril, Ethylacetat, Pyridin, Dimethylsulfoxid (DMSO), *N*,*N-*Dimethylfonnamid (DMF), *N*,*N*-Dimethylacetamid (DMA), *N,N'*-Dimethylpropylenharnstoff (DMPU) oder *N*-Methylpyrrolidinon (NMP) oder Wasser. Ebenso ist es möglich, Gemische solcher Lösungsmittel zu verwenden. Bevorzugt wird ein Gemisch von 1,4-Dioxan und Wasser eingesetzt.

Geeignete Basen für die Umsetzung (VI) → (II-A) sind beispielsweise Alkalicarbonate wie Kaliumcarbonat, Natriumcarbonat oder Lithiumcarbonat, Alkalihydrogencarbonate wie Natrium- oder Kaliumhydrogencarbonat oder Alkalialkoholate wie Natriummethanolat, Natriumethanolat oder Kalium-tert.-butylat. Bevorzugt wird Natriumhydrogencarbonat verwendet.

Die Reaktion (VI) → (II-A) erfolgt in einem Temperaturbereich von 0°C bis +50°C, bevorzugt bei +10°C bis +30°C. Die Umsetzung kann bei normalem, bei erhöhtem oder bei erniedrigtem Druck erfolgen (z.B. von 0.5 bis 5 bar); im Allgemeinen arbeitet man bei Normaldruck.

Die Umsetzung (VI) + (VII) → (II-B) erfolgt in einem unter den Reaktionsbedingungen inerten Lösungsmittel wie beispielsweise Ether wie Tetrahydrofuran, 1,4-Dioxan, 1,2-Dimethoxyethan oder Bis-(2-methoxyethyl)-ether, Alkohole wie Methanol, Ethanol, Isopropanol, n-Butanol oder *tert*.-Butanol, oder dipolar-aprotische Lösungsmittel wie Aceton, Methylethylketon, Acetonitril, Ethylacetat, Pyridin, Dimethylsulfoxid (DMSO), *N,N*-Dimethylfornamid (DMF), *N,N*-Dimethylacetamid (DMA), *N,N'*-Dimethylpropylenharnstoff (DMPU) oder *N*-Methylpyrrolidinon (NMP) oder Wasser. Ebenso ist es möglich, Gemische solcher Lösungsmittel zu verwenden. Bevorzugt wird DMF eingesetzt.

Geeignete Basen für die Umsetzung (VI) + (VII) → (II-B) sind beispielsweise tertiäre Aminbasen wie Triethylamin, *N*-Methylmorpholin, *N*-Methylpiperidin, *N,N*-Diisopropylethylamin, Pyridin oder 4-*N*,*N*-Dimethylaminopyridin. Bevorzugt wird *N,N*-Diisopropylethylamin verwendet.

Die Reaktion (VI) +(VII) → (II-B) erfolgt in einem Temperaturbereich von 0°C bis +50°C, bevorzugt bei +10°C bis +30°C. Die Umsetzung kann bei normalem, bei erhöhtem oder bei erniedrigtem Druck erfolgen (z.B. von 0.5 bis 5 bar); im Allgemeinen arbeitet man bei Normaldruck.

Die Umsetzungen (IX) → (III-A), (XIV) → (III-B) und (XVI) → (III-C) sowie (VI) + (XVII) → (III-D), (XIX) + (XX) → (III-E) und (XXV) + (XX) → (III-F) erfolgen in einem Lösungsmittel, weiches unter den Reaktionsbedingungen inert ist. Geeignete Lösungsmittel sind beispielsweise Ether wie Diethylether, Diisopropylether, *tert*.-Butylmethylether, Tetrahydrofuran, 1,4-Dioxan, 1,2-Dimethoxyethan oder Bis-(2-methoxyethyl)-ether, Kohlenwasserstoffe wie Benzol, Toluol, Xylol, Pentan, Hexan, Heptan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder dipolar-aprotische Lösungsmittel wie Aceton, Methylethylketon, Acetonitril, Ethylacetat, Pyridin, Dimethylsulfoxid (DMSO), *N*,*N*-Dimethylformamid (DMF), *N,N-*Dimethylacetamid (DMA), *N,N'*-Dimethylpropylenharnstoff (DMPU) oder *N*-Methylpyrrolidinon (NMP). Ebenso ist es möglich, Gemische solcher Lösungsmittel zu verwenden. Bevorzugt wird *N,N*-Dimethylformamid eingesetzt.

Geeignete Basen für diese Umsetzungen sind beispielsweise tertiäre Amine wie Triethylamin, *N*-Methylmorpholin, *N*-Methylpiperidin, *N,N*-Diisopropylethylamin, Pyridin oder 4-*N,N*-Dimethylaminopyridin. Bevorzugt wird *N*,*N*-Diisopropylethylamin, verwendet, gegebenenfalls unter Zusatz von 4-*N,N*-Dimethylaminopyridin.

Die Umsetzungen (IX) → (III-A), (XIV) → (III-B) und (XVI) → (III-C) sowie (VI) + (XVII) → (III-D) und (XIX) + (XX) → (III-E) erfolgen in einem Temperaturbereich von 0°C bis +50°C, bevorzugt bei +10°C bis +30°C. Die Umsetzung kann bei normalem, bei erhöhtem oder bei erniedrigten Druck erfolgen (z.B. von 0.5 bis 5 bar); im Allgemeinen arbeitet man bei Normaldruck.

Die Verbindungen der Formeln (II), (III), (I-A) bzw. (I-B) sind Untermengen der Verbindungen der Formeln (IIa), (IIIa), (Ia-A) bzw. (Ia-B), wobei R³⁵ für Methyl steht. Die Herstellung der Verbindungen (IIa) und (IIIa) erfolgt in Analogie zur Herstellung der Verbindung der Formeln (II) und (III) wie zuvor beschrieben.

Die zuvor beschriebenen Verfahren werden durch die nachfolgenden Syntheseschemata (Schema 3 bis 13, 18) beispielhaft verdeutlicht:

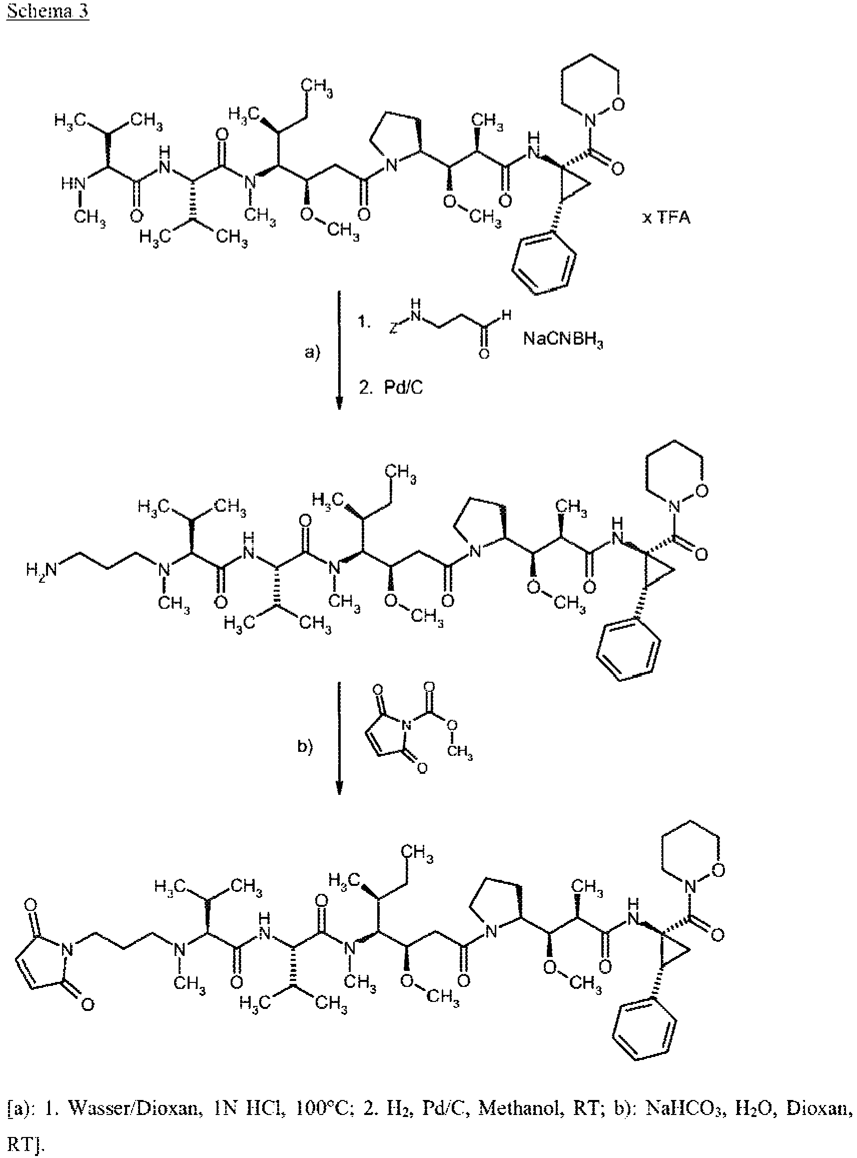

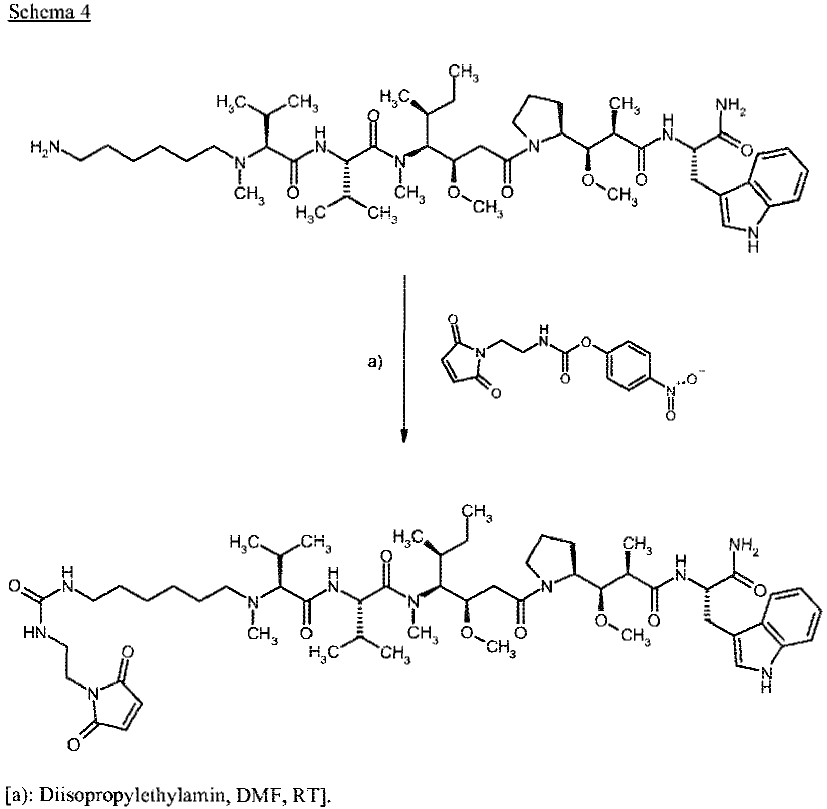

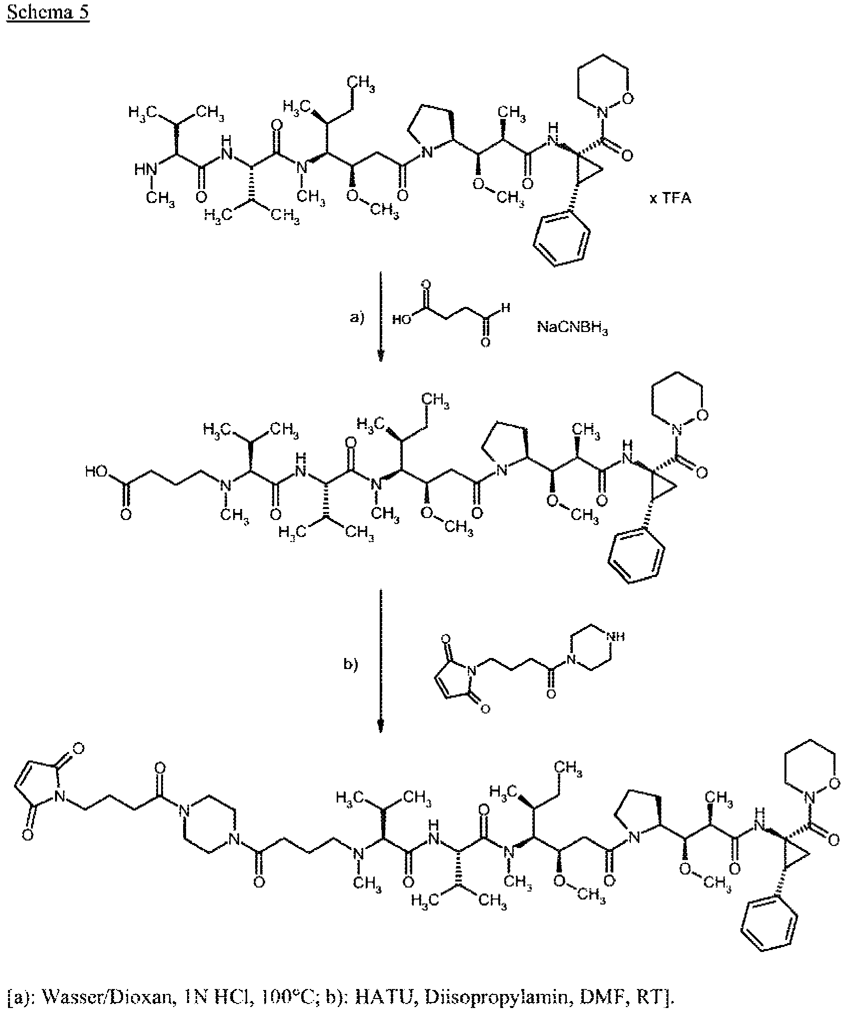

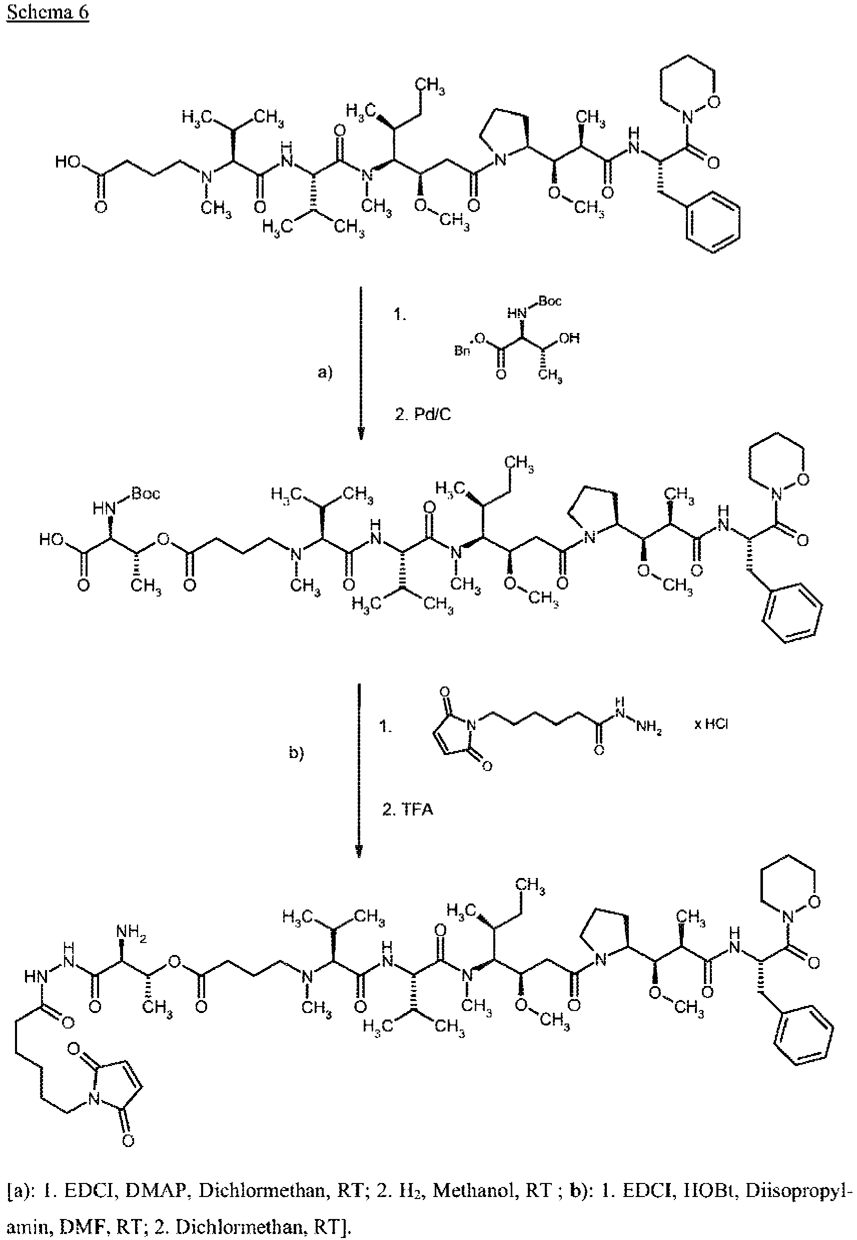

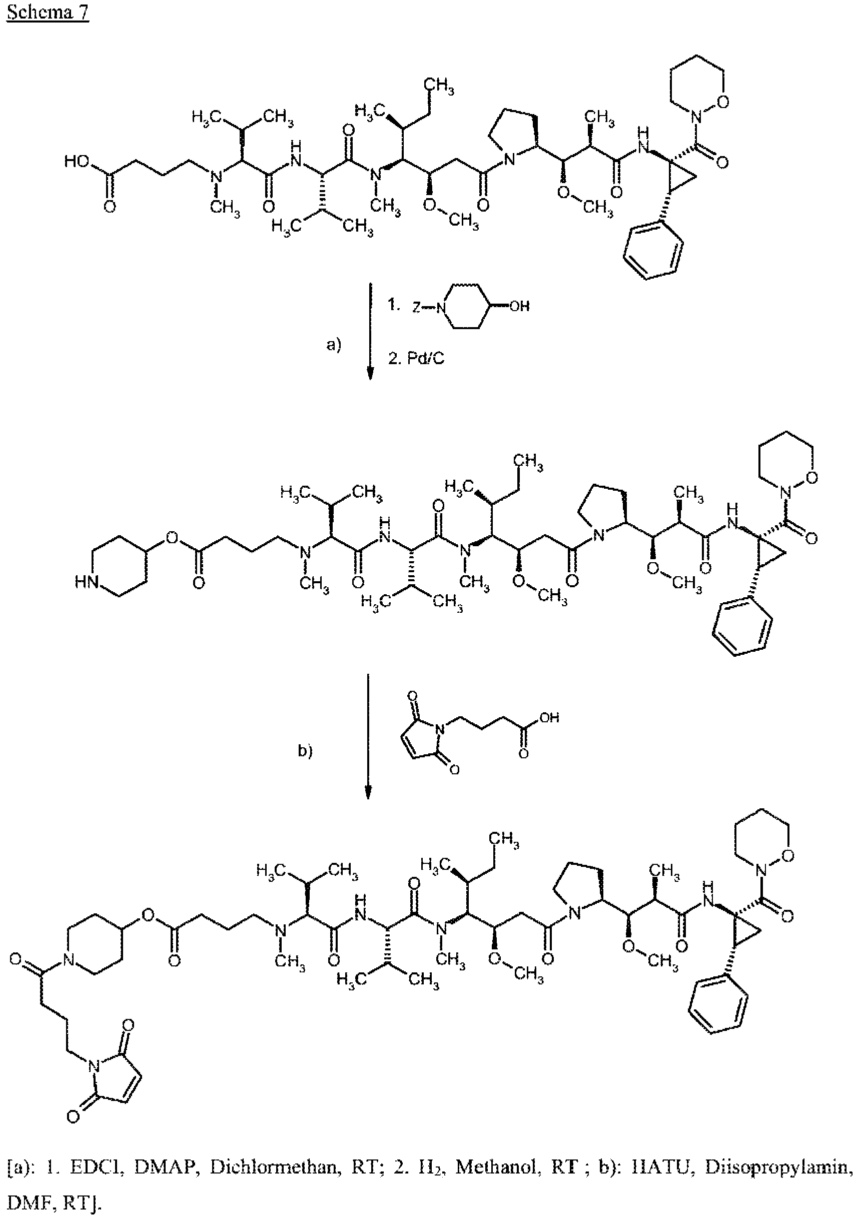

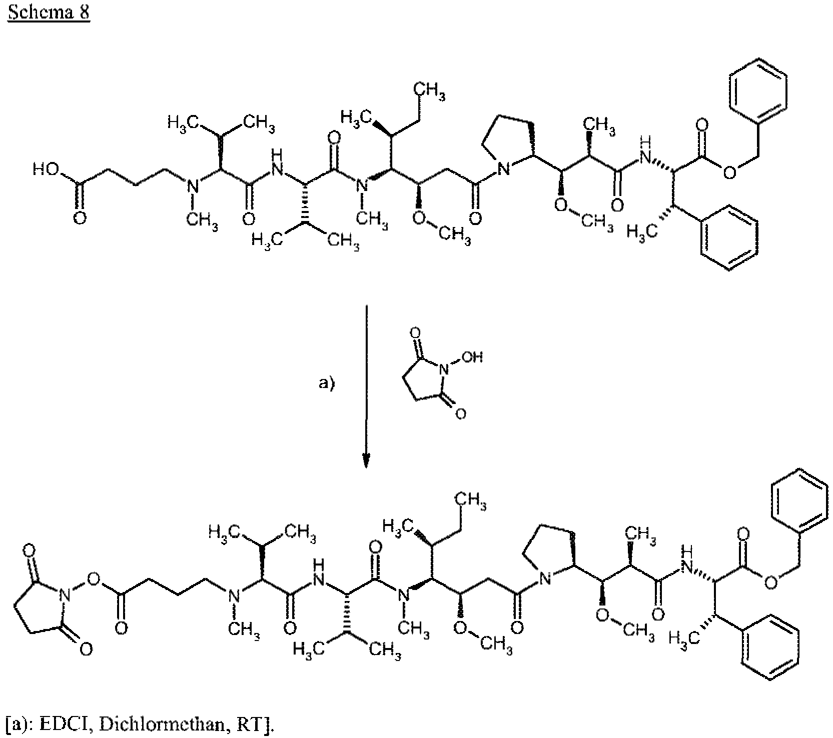

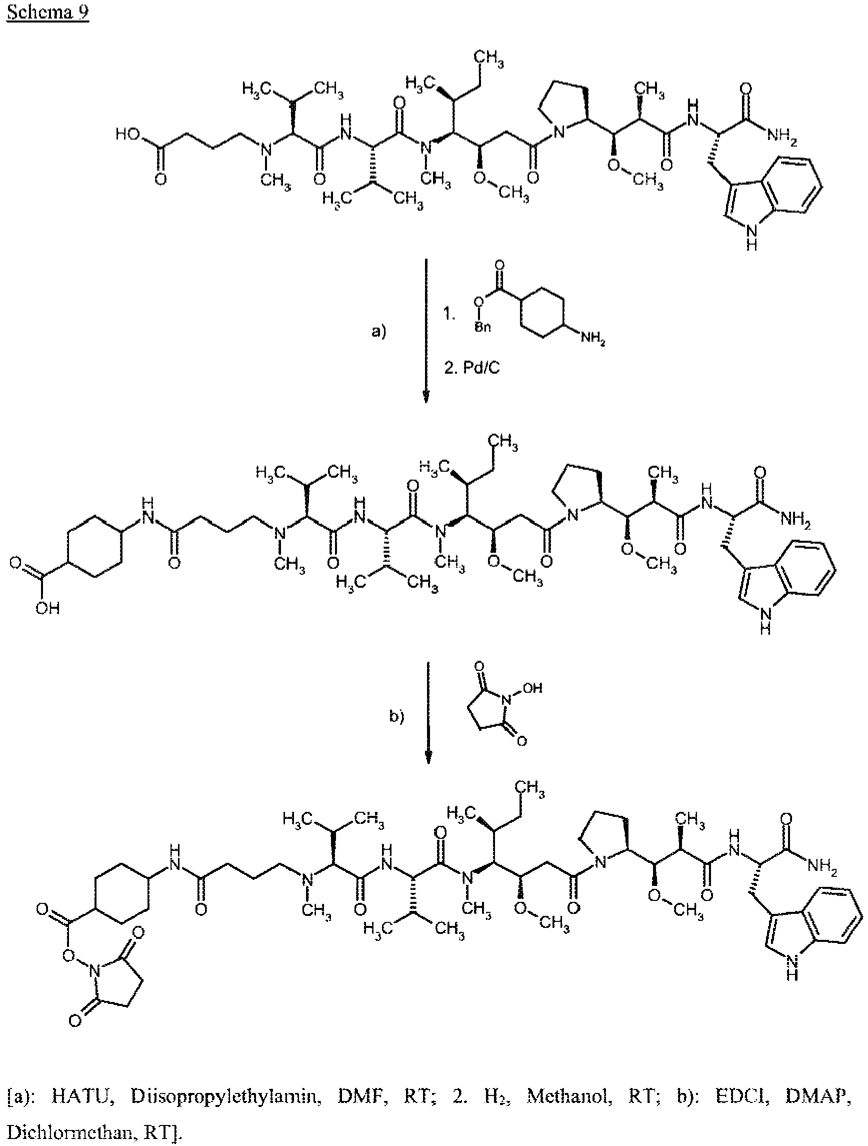

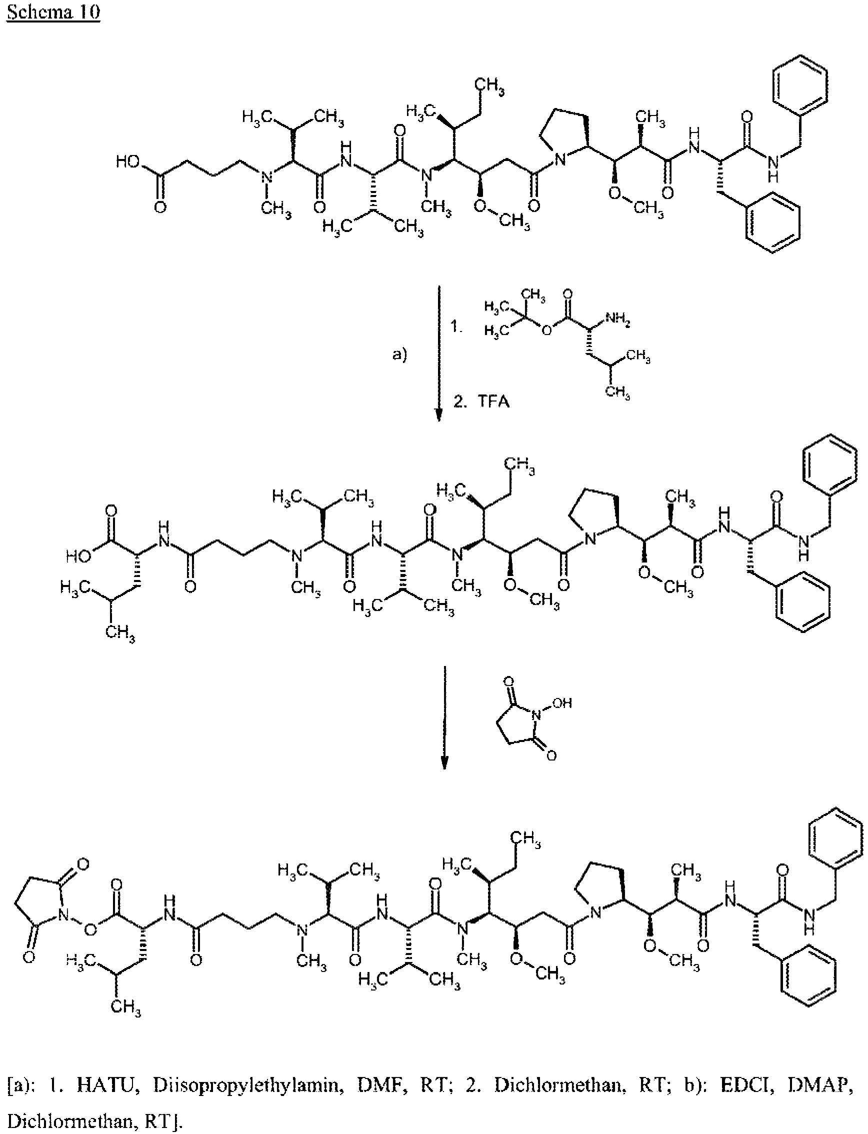

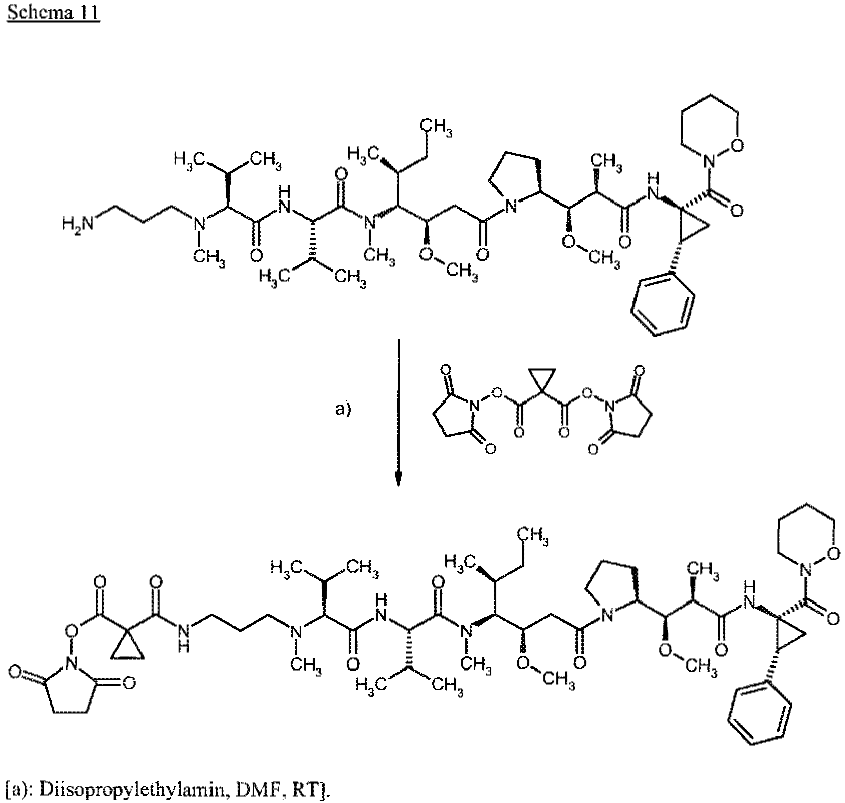

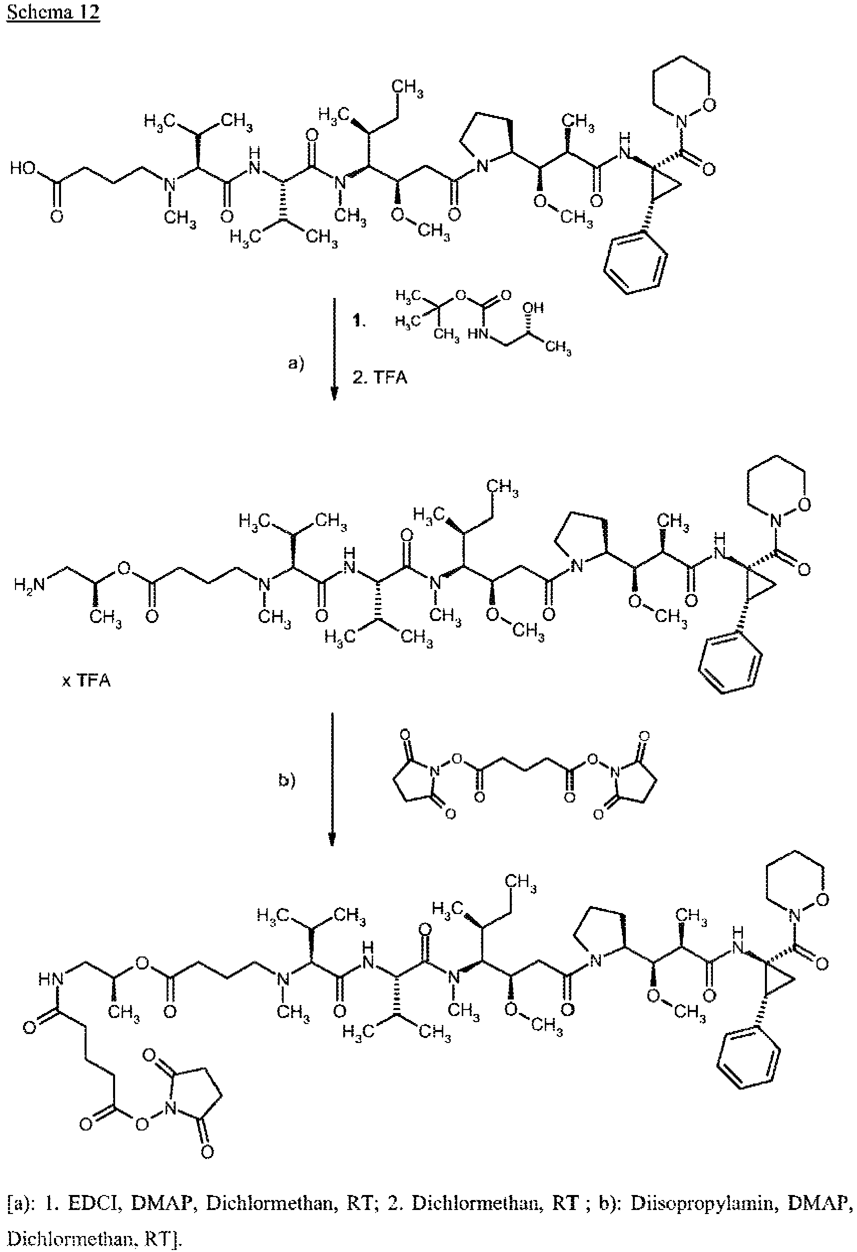

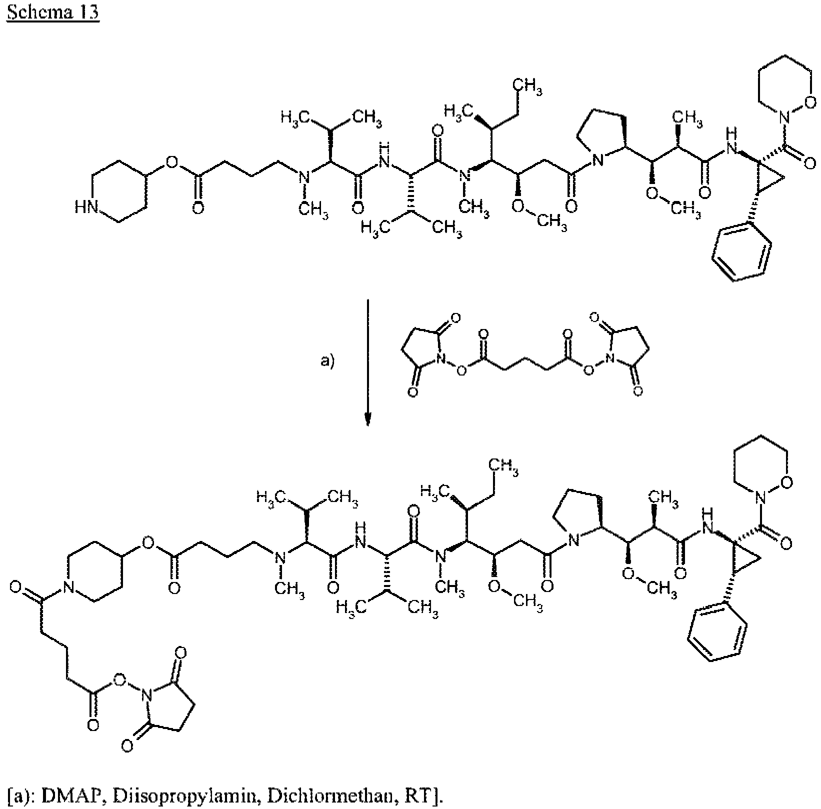

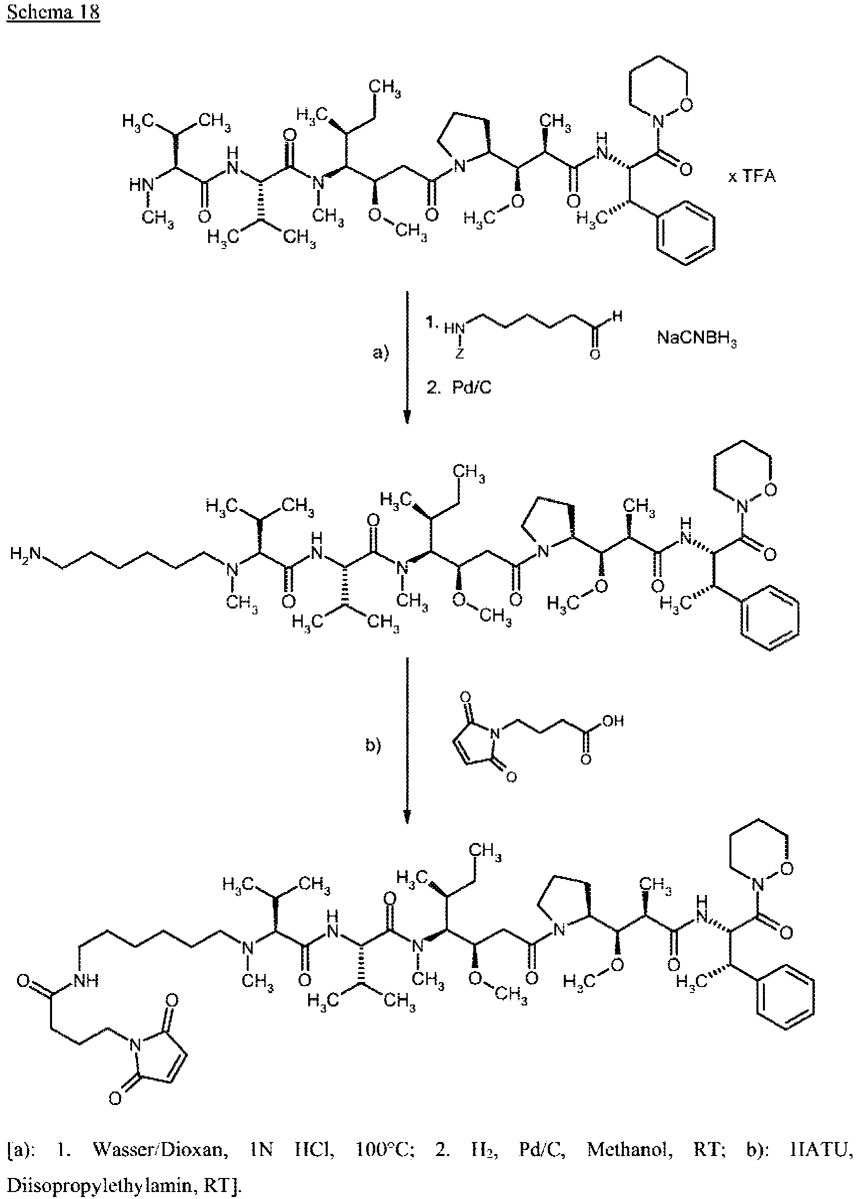

Die Verbindungen der Formel (IV) können aus kommerziell erhältlichen oder literaturbekannten Aminosäure-Bausteinen (siehe z. B. Pettit et al., Synthesis 1996, 719; Shioiri et al., Tetrahedron Lett. 1991, 32, 931; Shioiri et al., Tetrahedron 1993, 49, 1913; Koga et al., Tetrahedron Lett. 1991, 32, 2395: Vidal et al., Tetrahedron 2004, 60, 9715; Poncet et al., Tetrahedron 1994, 50, 5345. Pettit et a/., J. Org. Chem. 1994, 59, 1796) in Analogie zu literaturbekannten Verfahren nach üblichen Methoden der Peptidchemie, und wie im vorliegenden experimentellen Teil beschrieben, hergestellt werden. Die nachfolgenden Syntheseschemata (Schema 14 bis 16) verdeutlichen die Herstellung beispielhaft.

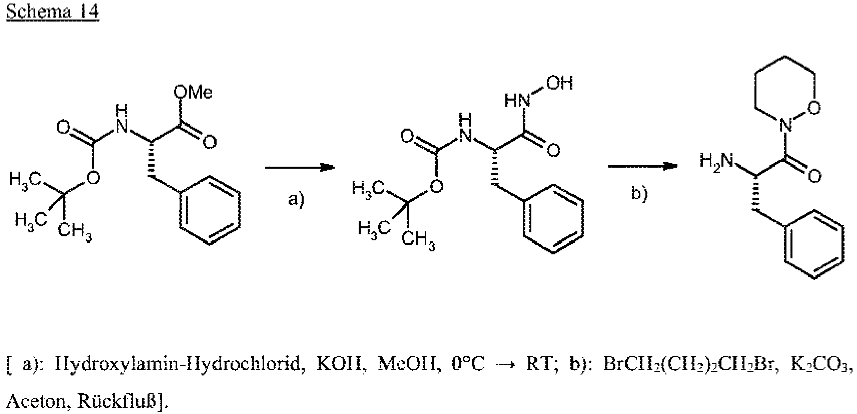

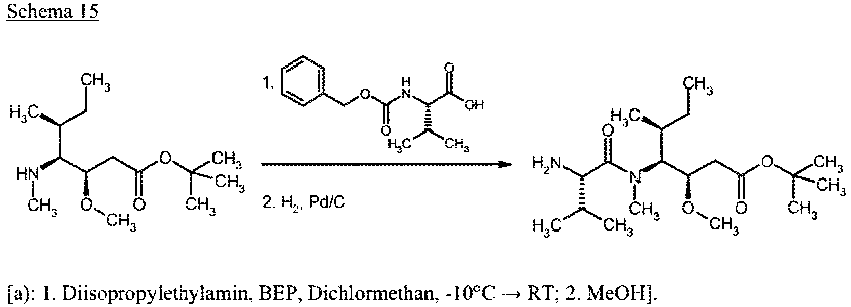

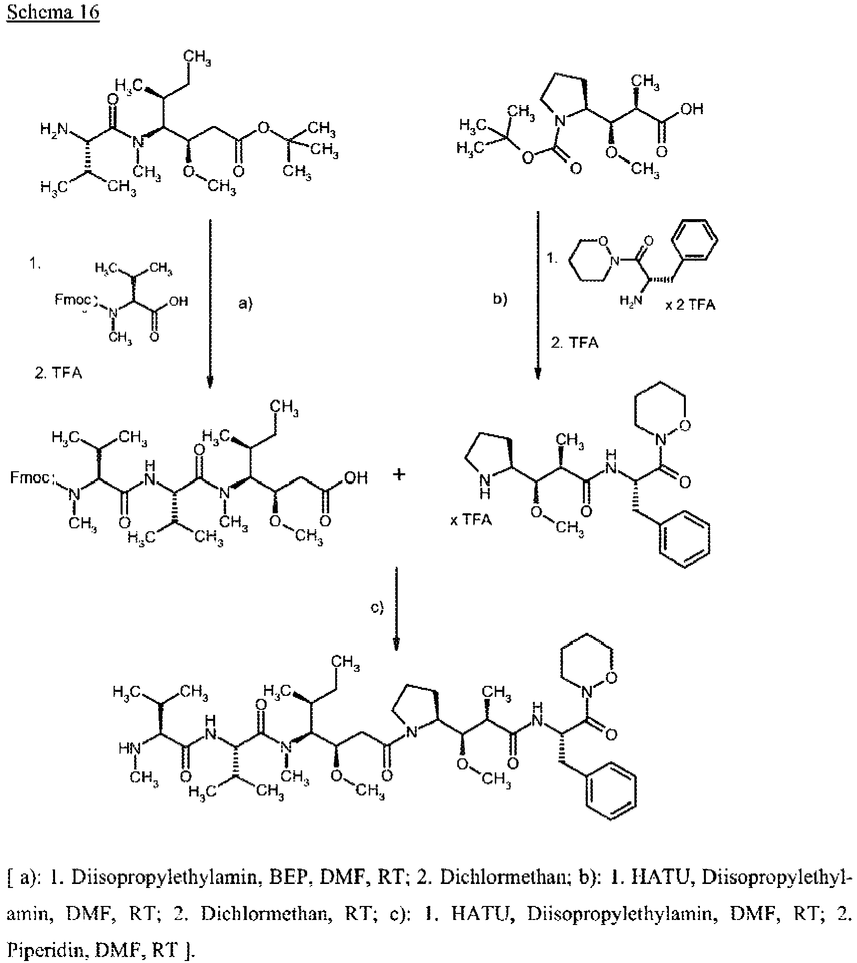

Die Verbindungen der Formeln (XI), (XIII), (XV), (XVII) und (XXI) einschließlich, wo zutreffend, chiraler oder diastereomerer Formen hiervon sind kommerziell erhältlich oder als solche in der Literatur beschrieben, oder sie können auf für den Fachmann offenkundigem Wege in Analogie zu in der Literatur publizierten Methoden hergestellt werden. Zahlreiche ausführliche Vorschriften sowie Literaturangaben zur Herstellung der Ausgangsmaterialien befinden sich auch im Experimentellen Teil im Abschnitt zur Herstellung der Ausgangsverbindungen und Intermediate.

Die Verbindungen der Formeln (V), (VII), (VIII). (X), (XVIII), (XX) und (XXIII) einschließlich, wo zutreffend, chiraler oder diastereomerer Formen hiervon sind literaturbekannt, oder sie können auf für den Fachmann offenkundigem Wege in Analogie zu in der Literatur publizierten Methoden hergestellt werden. Zahlreiche ausführliche Vorschriften sowie Literaturangaben zur Herstellung der Ausgangsmaterialien befinden sich auch im Experimentellen Teil im Abschnitt zur Herstellung der Ausgangsverbindungen und Intermediate.

Alternativ können einzelne Schritte der Herstellungssequenz in anderer Reihenfolge durchgeführt werden. Dieses Vorgehen wird in den nachfolgenden Syntheseschemata (Schema 17, 19 und 20) beispielhaft verdeutlicht.

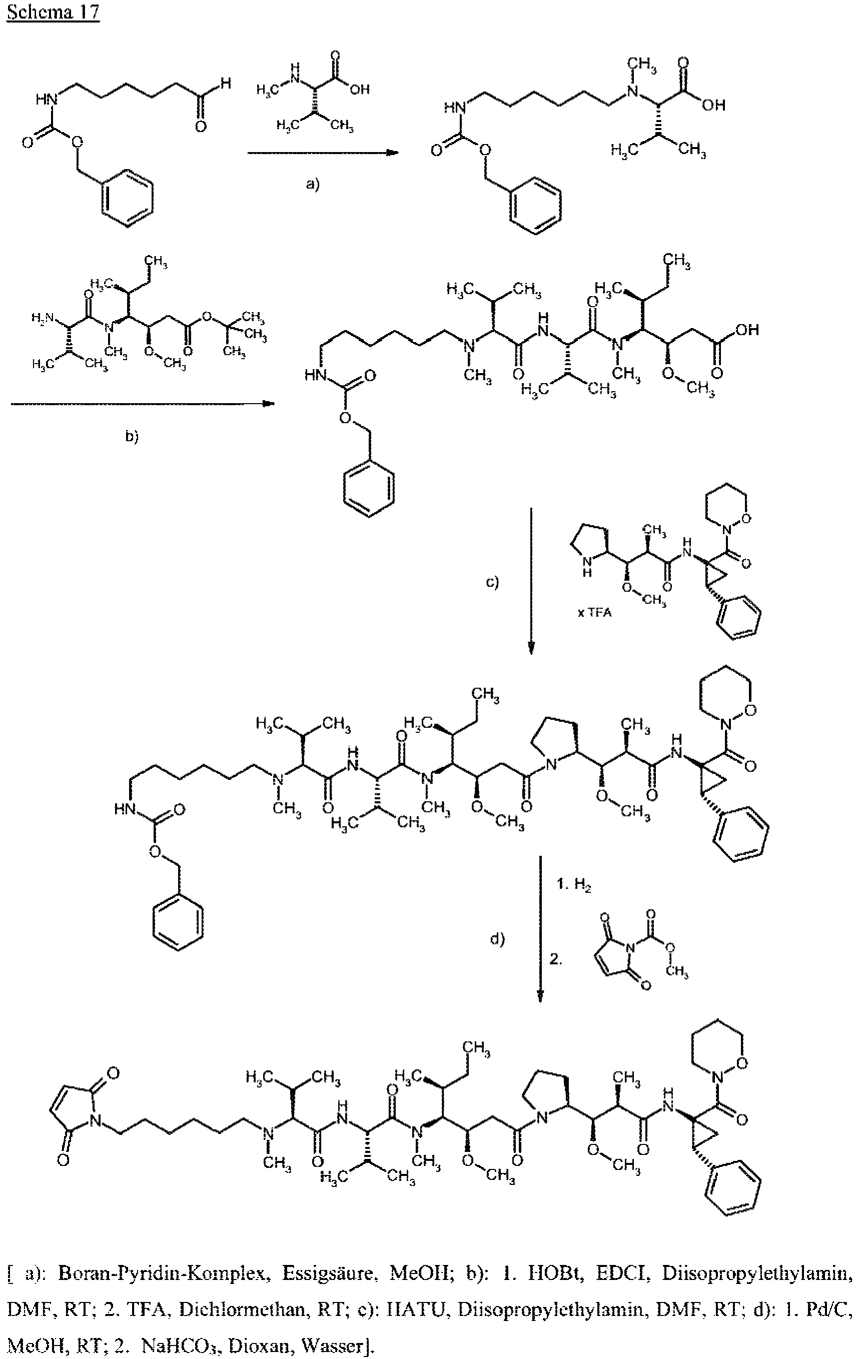

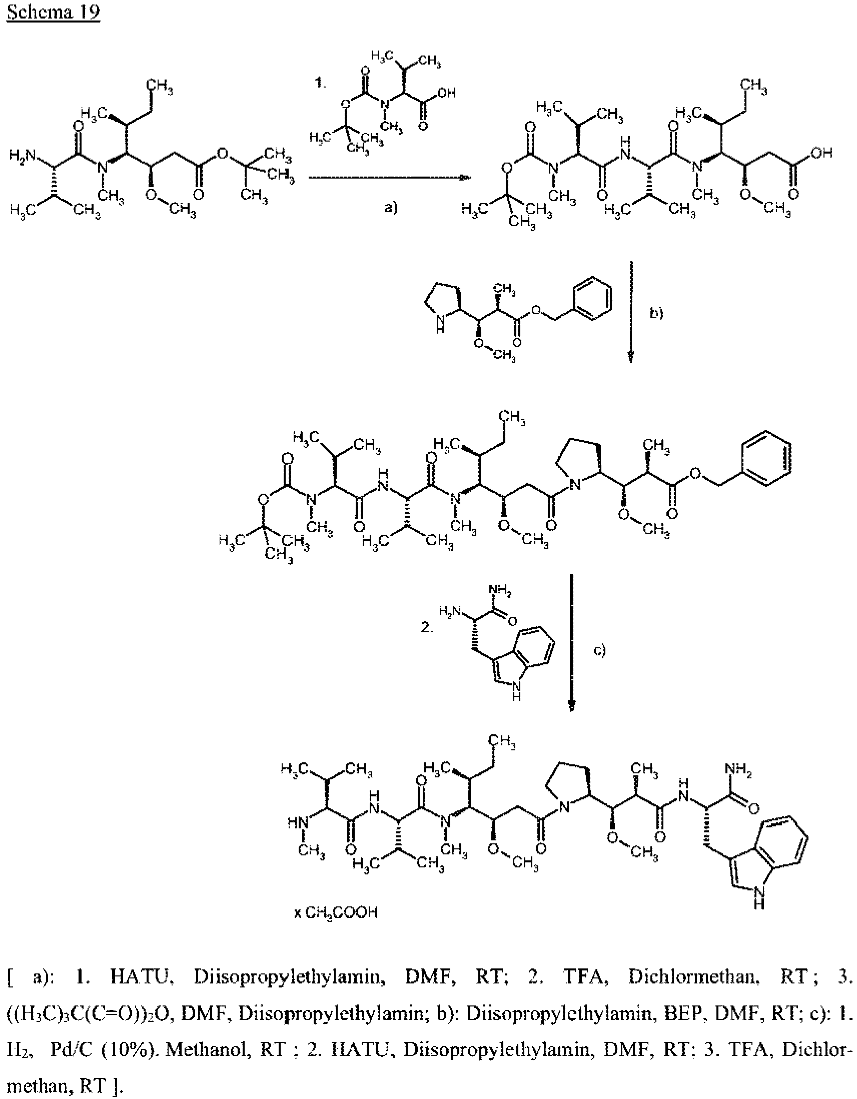

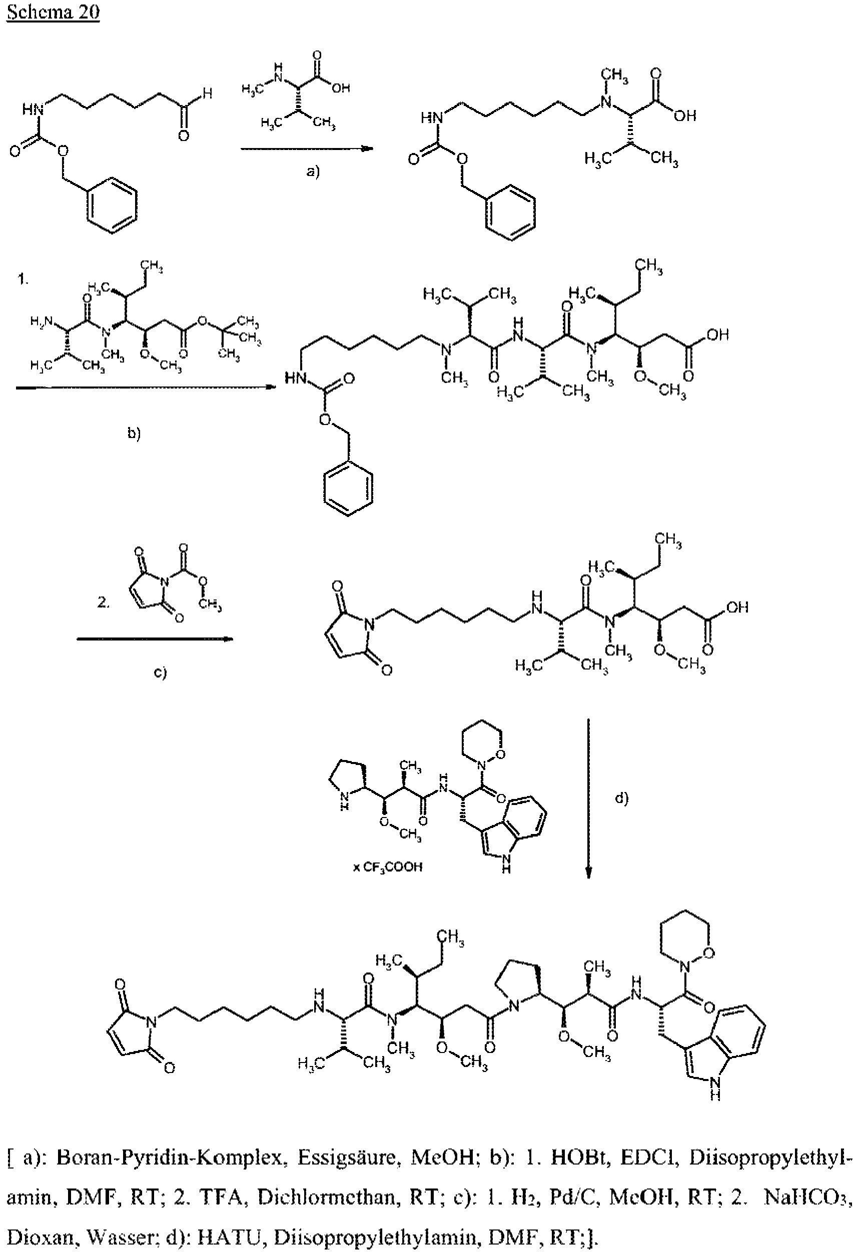

In einer Ausführungsform bindet der Binder an ein Zielmolekül, welches auf einer Krebszelle vorhanden ist. In einer bevorzugten Ausführungsform bindet der Binder an ein Krebs-Zielmolekül. In einer weiteren bevorzugten Ausführungsform ist das Zielmolekül ein selektives Krebs-Zielmolekül.

In einer besonders bevorzugten Ausführungsform ist das Zielmolekül ein Protein.

In einer Ausführungsform ist das Zielmolekül ein extrazelluläres Zielmolekül. In einer bevorzugten Ausfuhrungsform ist das extrazelluläre Zielmolekül ein Protein.

Krebs-Zielmoleküle sind dem Fachmann bekannt. Beispiele hierfür sind im Folgenden aufgeführt.

### Beispiele für Krebs-Zielmoleküle sind:

(1) EGF-Rezeptor (NCBI reference sequence NP_005219-2)
   Sequenz (1210 Aminosäuren):
   >gi|29725609|ref|NP_005219.2| epidermal growth factor receptor isoform a precursor [Homo sapiens] Die extrazelluläre Domäne ist durch Unterstreichung gekennzeichnet.
(2) Mesothelin (SwissProt reference Q13421-3)
   Sequenz (622 Aminosäuren):
   >sp|Q13421-3|MSIN_HUMAN Isoform 2 of Mesothelin OS=Homo sapiens GN=MSLN 
MALPTARPLLGSCGTPALGSLLFLLFSLGWVQPSRTLAGETGQEAAPLDGVLANPPNISS
LSPRQLLGFPCAEVSGLSTERVRELAVALAQKNVKLSTEQLRCLAHRLSEPPEDLDALPL
DLLLFLNPDAFSGPQACTRFFSRITKANVDLLPRGAPERQRLLPAALACWGVRGSLLSEA
DVRALGGLACDLPGRFVAESAEVLLPRLVSCPGPLDQDQQEAARAALQGGGPPYGPPSTW
SVSTMDALRGLLPVLGQPIIRSIPQGIVAAWRQRSSRDPSWRQPERTILRPRFRREVEKT
ACPSGKKAREIDESLIFYKKWELEACVDAALLATQMDRVNAIPFTYEQLDVLKHKLDELY
PQGYPESVIQHLGYLFLKMSPEDIRKWNVTSLETLKALLEVNKGHEMSPQVATLIDRFVK
 
GRGQLDKDTLDTLTAFYPGYLCSLSPEELSSVPPSSIWAVRPQDLDTCDPRQLDVLYPKA
RLAFQNMNGSEYFVKIQSFLGGAPTEDLKALSQQNVSMDLATFMKLRTDAVLPLTVAEVQ
KLLGPHVEGLKAEERHRPVRDWILRQRQDDLDTLGLGLQGGIPNGYLVLDLSMQEALSGT
PCLLGPGPVLTVLALLLASTLA
   Wobei Mesothelin von den Aminosäuren 296-598 kodiert wird. Aminosäuren 37-286 kodieren für "megakaryocyte-polemiating factor". Mesothelin ist durch einen GPI-Anker in der Zellmembran verankert und ist extrazellulär lokalisiert.
(3) Carboanhydrase IX (SwissProt reference Q16790)
   Sequenz (459 Aminosäuren):
   >sp|Q16790|CAH9_HUMAN Carbonic anhydrase 9 CS=Homo sapiens GN=CA9 FE=1 SV=2 
MAPLCPSPWLPLLIPAPAPGLTVQLLLSLLLLVPVHPQRLPRMQEDSPLGGGSSGEDDPL
GEEDLPSEEDSPREEDPPGEEDLPGEEDLPGEEDLPEVKPKSEEEGSLKLEDLPTVEAPG
DPQEPQNNAHRDKEGDDQSHWRYGGDPPWPRVSPACAGRFQSPVDIRPQLAAFCPALRPL
ELLGFQLPPLPELRLRNNGHSVQLTLPPGLEMALGPGREYRALQLHLHWGAAGRPGSEHT
VEGHRFPAEIHVVHLSTAFARVDEALGRPGGLAVLAAFLEEGPEENSAYEQLLSRLEEIA
EEGSETQVPGLDISALLPSDFSRYFQYEGSLTTPPCAQGVIWTVFNQTVMLSAKQLHTLS
DTLWGPGDSRLQLNFRATQPLNGRVIEASFPAGVDSSPRAAEPVQLNSCLAAGDILALVF
GLLFAVTSVAFLVQMRRQHRRGTKGGVSYRPAEVAETGA
 Die extrazelluläre Domäne ist durch Unterstreichung gekennzeichnet.
(4) C4.4a (NCBI reference sequence NP_055215.2; Synonym LYPD3)
   Sequenz (346 Aminosäuren):
   >gi|93004088|ref|NP_055215.2| ly6/PLAUR domain-containing protein 3 precursor [Homo sapiens] Die maturierte, extrazelluläre Domäne ist durch Unterstreichung gekennzeichnet (SEQ ID NO: 1).
(5) CD52 (NCBI reference sequence NP_001794.2)
   >gi|682342030|ref|NP_001794.2| CAMPATH-1 antigen precursor [Homo sapiens]
      MKRFLFLLLTISLLVMVQIQTGLSGQNDTSQTSSPSASSNISGGIFLFFVANAIIHLFCFS
(6) Her2 (NCBI reference sequence NP_004439.2)
   >gi|54792096|ref|NF_004439.2| receptor tyrosine-protein kinase erbB-2 isoform a [Homo sapiens]
(7) CD20 (NCBI reference sequence NP_068769-2)
   >gi|23110987|ref|NP_068769.2| B-lymphocyte antigen CD20 [Homo sapiens] 
MTTPRNSVNGTFPAEPMKGPIAMQSGPKPLFRRMSSLVGPTQSFFMRESKTLGAVQIMNGLFHIAL
GGLLMIPAGIYAPICVTVWYPLWGGIMYIISGSLLAATEKNSRKCLVKGKMIMNSLSLFAAISGMI
LSIMDILNIKISHFLKMESLNFIRAHTPYINIYNCEPANPSEKNSPSTQYCYSIQSLFLGILSVML
IFAFFQELVIAGIVENEWKRTCSRPKSNIVLLSAEEKKEQTIEIKEEVVGLTETSSQPKNEEDIEI
IPIQEEEEEETETNFPEPPQDQESSPIENDSSP
(8) das Lymphozyten aktivierungs Antigen CD30 (SwissProt ID P28908)
   >gi|68348711|ref|NP_001234.2| tumor necrosis factor receptor superfamily member 8 isoform 1 precursor [Homo sapiens] 
MRVLLAALGLLFLGALRAFPQDRPFEDTCHGNPSHYYDKAVRRCCYRCPMGLFPTQQCPQRPTDCR
KQCEPDYYLDEADRCTACVTCSRDDLVEKTPCAWNSSRVCECRPGMFCSTSAVNSCARCFFHSVCP
AGMIVKFPGTAQKNTVCEPASPGVSPACASPENCKEPSSGTIPQAKPTPVSPATSSASTMPVRGGT
RLAQEAASKLTRAPDSPSSVGRPSSDPGLSPTQPCPEGSGDCRKQCEPDYYLDEAGRCTACVSCSR
DDLVEKTPCAWNSSRTCECRPGMICATSATNSRARCVPYPICAAETVTKPQDMAEKDTTFEAPPLG
TQPDCNPTPENGEAPASTSPTQSLLVDSQASKTLPIPTSAPVALSSTGKPVLDAGPVLFWVILVLV
VVVGSSAFLLCHRRACRKRIRQKLHLCYPVQTSQPKLELVDSRPRRSSTQLRSGASVTEPVAEERG
LMSQPLMETCHSVGAAYLESLPLQDASPAGGPSSPRDLPEPRVSTEHTNNKIEKIYIMKADTVIVG
TVKAELPEGRGLAGPAEPELEEELEADHTPHYPEQETEPPLGSCSDVMLSVEEEGKEDPLPTAASG
K
(9) das Lymphozyten Adhesionsmolekül CD22 (SwissProt ID **P20273**)
   >gi|157168355|ref|NP_001762.2| B-cell receptor CD22 isoform 1 precursor [Homo sapiens] 
MHLLGPWLLLLVLEYLAFSDSSKWVFEHPETLYAWEGACVWIPCTYRALDGDLESFILFHNPEYNK
NTSKFDGTRLYESTKDGKVPSEQKRVQFLGDKNKNCTLSIHPVHLNDSGQLGLRMESKTEKWMERI
HLNVSERPFPPHIQLPPEIQESQEVTLTCLLNFSCYGYPIQLQWLLEGVPMRQAAVTSTSLTIKSV
FTRSELKFSPQWSHHGKIVTCQLQDADGKFLSNDTVQLNVKHTPKLEIKVTPSDAIVREGDSVTMT
CEVSSSNPEYTTVSWLKDGTSLKKQNTFTLNLREVTKDQSGKYCCQVSNDVGPGRSEEVFLQVQYA
PEPSTVQILHSPAVEGSQVEFLCMSLANPLPTNYTWYHNGKEMQGRTEEKVHIPKILPWHAGTYSC
VAENILGTGQRGPGAELDVQYPPKKVTTVIQNPMPIREGDTVTLSCNYNSSNPSVTRYEWKPHGAW
EEPSLGVLKIQNVGWDNTTIACAACNSWCSWASPVALNVQYAPRDVRVRKIKPLSEIHSGNSVSLQ
CDFSSSHPKEVQFFWEKNGRLLGKESQLNFDSISPEDAGSYSCWVNNSIGQTASKAWTLEVLYAPR
RLRVSMSPGDQVMEGKSATLTCESDANPPVSHYTWFDWNNQSLPYHSQKLRLEPVKVQHSGAYWCQ
 
GTNSVGKGRSPLSTLTVYYSPETIGRRVAVGLGSCLAILILAICGLKLQRRWKRTQSQQGLQENSS
GQSFFVRNKKVRRAPLSEGPHSLGCYNPMMEDGISYTTLRFPEMNIPRTGDAESSEMQRPPPDCDD
TVTYSALHKRQVGDYENVIPDFPEDEGIHYSELIQFGVGERPQAQENVDYVILKH
(10) das Myloidzellen Oberflächenantigen CD33 (SwissProt ID **P20138**)
   >gi|130979981|ref|NP_001763.3| myeloid cell surface antigen CD33 isoform 1 precursor [Homo sapiens] 
MPLLLLLPLLWAGALAMDPNFWLQVQESVTVQEGLCVLVPCTFFHPIPYYDKNSPVHGYWFREGAI
ISRDSPVATNKLDQEVQEETQGRFRLLGDPSRNNCSLSIVDARRRDNGSYFFRMERGSTKYSYKSP
QLSVHVTDLTHRPKILIPGTLEPGHSKNLTCSVSWACEQGTPPIFSWLSAAPTSLGPRTTHSSVLI
ITPRPQDHGTNLTCQVKFAGAGVTTERTIQLNVTYVPQNPTTGIFPGDGSGKQETRAGVVHGAIGG
AGVTALLALCLCLIFFIVKTHRRKAARTAVGRNDTHPTTGSASPKHQKKSKLHGPTETSSCSGAAP
TVEMDEELHYASLNFHGMNPSKDTSTEYSEVRTQ
(11) das Transmembran Glykoprotein NMB (SwissProt ID Q14956)
   >gi|52694752|ref|NP_001005340.1| transmembrane glycoprotein NMB isoform a precursor [Homo sapiens] 
MECLYYFLGFLLLAARLPLDAAKRFHDVLGNERPSAYMREHNQLNGWSSDENDWNEKLYPVWKRGD
MRWKNSWKGGRVQAVLTSDSPALVGSNITFAVNLIFPRCQKEDANGNIVYEKNCRNEAGLSADPYV
YNWTAWSEDSDGENGTGQSHHNVFPDGKPFPHHPGWRRWNFIYVFHTLGQYFQKLGRCSVRVSVNT
ANVTLGPQLMEVTVYRRHGRAYVPIAQVKDVYVVTDQIPVFVTMFQKNDRNSSDETFLKDLPIMFD
VLIHDPSHFLNYSTINYKWSFGDNTGLFVSTNHTVNHTYVLNGTFSLNLTVKAAAPGPCPPPPPPP
RPSKPTPSLATTLKSYDSNTPGPAGDNPLELSRIPDENCQINRYGHFQATITIVEGILEVNIIQMT
DVLMPVPWPESSLIDFVVTCQGSIPTEVCTIISDPTCEITQNTVCSPVDVDEMCLLTVRRTFNGSG
TYCVNLTLGDDTSLALTSTLISVPDRDPASPLRMANSALISVGCLAIFVTVISLLVYKKHKEYNPI
ENSPGNVVRSKGLSVFLNRAKAVFFPGNQEKDPLLKNQEFKGVS
(12) das Adhesionsmolekül CD56 (SwissProt ID P13591)
   >gi|94420689|ref|NP_000606.3| neural cell adhesion molecule 1 isoform 1 [Homo sapiens] 
MLQTKDLIWTLFFLGTAVSLQVDIVPSQGEISVGESKFFLCQVAGDAKDKDISWFSPNGEKLTPNQ
QRISVVWNDDSSSTLTIYNANIDDAGIYKCVVTGEDGSESEATVNVKIFQKLMFKNAPTPQEFREG
EDAVIVCDVVSSLPPTIIWKHKGRDVILKKDVRFIVLSNNYLQIRGIKKTDEGTYRCEGRILARGE
INFKDIQVIVNVPPTIQARQNIVNATANLGQSVTLVCDAEGFPEPTMSWTKDGEQIEQEEDDEKYI
FSDDSSQLTIKKVDKNDEAEYICIAENKAGEQDATIHLKVFAKPKITYVENQTAMELEEQVTLTCE
ASGDPIPSITWRTSTRNISSEEKTLDGHMVVRSHARVSSLTLKSIQYTDAGEYICTASNTIGQDSQ
SMYLEVQYAPKLQGPVAVYTWEGN
QVNITCEVFAYPSATISWFRDGQLLPSSNYSNIKIYNTPSASYLEVTPDSENDFGNYNCTAVNRIG
QESLEFILVQADTPSSPSIDQVEPYSSTAQVQFDEPEATGGVPILKYKAEWRAVGEEVWHSKWYDA
KEASMEGIVTIVGLKPETTYAVRLAALNGKGLGEISAASEFKTQPVQGEPSAPKLEGQMGEDGNSI
KVNLIKQDDGGSPIRHYLVRYRALSSEWKPEIRLPSGSDHVMLKSLDWNAEYEVYVVAENQQGKSK
AAHFVFRTSAQPTAIPANGSPTSGLSTGAIVGILIVIFVLLLVVVDITCYFLNKCGLFMCIAVNLC
GKAGPGAKGKDMEEGKAAFSKDESKEPIVEVRTEEERTPNHDGGKHTEPNETTPLTEPEKGPVEAK
PECQETETKPAPAEVKTVPNDATQTKENESKA
(13) das Oberflächenmolekül CD70 (SwissProt ID P32970)
   >gi|4507605|ref|NP_001243.1|CD70 antigen [Homo sapiens] 
MPEEGSGCSVRRRPYGCVLRAALVPLVAGLVICLVVCIQRFAQAQQQLPLESLGWDVAELQLNHTG
PQQDPRLYWQGGPALGRSFLHGPELDKGQLRIHRDGIYMVHIQVTLAICSSTTASRHHPTTLAVGI
CSPASRSISLLRLSFHQGCTIASQRLTPLARGDTLCTNLTGTLLPSRNTDETFFGVQWVRP
(14) das Oberflächenmolekül CD74 (SwissProt ID P04233)
   >gi|10835071|ref|NP_004346.1| HLA class II histocompatibility antigen gamma chain isoform b [Homo sapiens] 
MHRRRSRSCREDQKPVMDDQRDLISNNEQLPMLGRRPGAPESKCSRGALYTGFSILVTLLLAGQAT
TAYFLYQQQGRLDKLTVTSQNLQLENLRMKLPKPPKPVSKMRMATPLLMQALPMGALPQGPMQNAT
KYGNMTEDHVMHLLQNADPLKVYPPLKGSFPENLRHLKNTMETIDWKVFESWMHHWLLFEMSRHSL
EQKPTDAPPKESLELEDPSSGLGVTKQDLGPVPM
(15) das B-Lymphozyten Antigen CD19 (SwissProt ID P15391)
   >gi|296010921|ref|NP_001171569.1| B-lymphocyte antigen CD19 isoform 1 precursor [Homo sapiens] 
MPPPRLLFFLLFLTPMEVRPEEPLVVKVEEGDNAVLQCLKGTSDGPTQQLTWSRESPLKPFLKLSL
GLPGLGIHMRPLAIWLFIFNVSQQMGGFYLCQPGPPSEKAWQPGWTVNVEGSGELFRWNVSDLGGL
GCGLKNRSSEGPSSPSGKLMSPKLYVWAKDRPEIWEGEPPCLPPRDSLNQSLSQDLTMAPGSTLWL
SCGVPPDSVSRGPLSWTHVHPKGPKSLLSLELKDDRPARDMWVMETGLLLPRATAQDAGKYYCHRG
NLTMSFHLEITARPVLWHWLLRTGGWKVSAVTLAYLIFCLCSLVGILHLQRALVLRRKRKRMTDPT
RRFFKVTPPPGSGPQNQYGNVLSLPTPTSGLGRAQRWAAGLGGTAPSYGNPSSDVQADGALGSRSP
PGVGPEEEEGEGYEEPDSEEDSEFYENDSNLGQDQLSQDGSGYENPEDEPLGPEDEDSFSNAESYE
NEDEELTQPVARTMDFLSPHGSAWDPSREATSLAGSQSYEDMRGILYAAPQLRSIRGQPGPNHEED
ADSYENMDNPDGPDPAWGGGGRMGTWSTR
(16) das Oberflächenprotein Mucin-1 (SwissProt ID P15941)
   >gi|65301117|ref|NP_002447.4| mucin-1 isoform 1 precursor [Homo sapiens] 
MTPGTQSPFFLLLLLTVLTVVTGSGHASSTPGGEKETSATQRSSVPSSTEKNALSTGVSFFFLSFH
ISNLQFNSSLEDPSTDYYQELQRDISEMFLQIYKQGGFLGLSNIKFRPGSVVVQLTLAFREGTINV
HDVETQFNQYKTEAASRYNLTISDVSVSDVPFPFSAQSGAGVPGWGIALLVLVCVLVALAIVYLIA
LAVCQCRRKNYGQLDIFPARDTYHPMSEYPTYHTHGRYVPPSSTDRSPYEKVSAGNGGSSLSYTNP
AVAATSANL
(17) das Oberflächenprotein CD138 (SwissProt ID P18827)
   >gi|29569086|ref|NP_002988.3| syndecan-1 precursor [Homo sapiens] 
MRRAALWLWLCALALSLQPALPQIVATNLPPEDQDGSGDDSDNFSGSGAGALQDITLSQQTPSTWK
DTQLLTAIPTSPEPTGLEATAASTSTLPAGEGPKEGEAVVLPEVEPGLTAREQEATPRPRETTQLP
TTHQASTTTATTAQEPATSHPHRDMQPGHHETSTPAGPSQADLHTPHTEDGGPSATERAAEDGASS
QLPAAEGSGEQDFTFETSGENTAVVAVEPDRRNQSPVDQGATGASQGLLDRKEVLGGVIAGGLVGL
IFAVCLVGFMLYRMKKKDEGSYSLEEPKQANGGAYQKPTKQEEFYA
(18) das Integrin alphaV (Genbank Accession No.: NP_002201.1)
   >gi|4504763|ref|NP_002201.1| integrin alpha-V isoform 1 precursor [Homo sapiens] 
MAFPPRRRLRLGPRGLPLLLSGLLLPLCRAFNLDVDSPAEYSGPEGSYFGFAVDFFVPSASSRMFL
LVGAPKANTTQPGIVEGGQVLKCDWSSTRRCQPIEFDATGNRDYAKDDPLEFKSHQWFGASVRSKQ
DKILACAPLYHWRTEMKQEREPVGTCFLQDGTKTVEYAPCRSQDIDADGQGFCQGGFSIDFTKADR
VLLGGPGSFYWQGQLISDQVAEIVSKYDPNVYSIKYNNQLATRTAQAIFDDSYLGYSVAVGDFNGD
GIDDFVSGVPRAARTLGMVYIYDGKNMSSLYNFTGEQMAAYFGFSVAATDINGDDYADVFIGAPLF
MDRGSDGKLQEVGQVSVSLQRASGDFQTTKLNGFEVFARFGSAIAPLGDLDQDGFNDIAIAAPYGG
EDKKGIVYIFNGRSTGLNAVPSQILEGQWAARSMPPSFGYSMKGATDIDKNGYPDLIVGAFGVDRA
ILYRARPVITVNAGLEVYPSILNQDNKTCSLPGTALKVSCFNVRFCLKADGKGVLPRKLNFQVELL
LDKLKQKGAIRRALFLYSRSPSHSKNMTISRGGLMQCEELIAYLRDESEFRDKLTPITIFMEYRLD
 
YRTAADTTGLQPILNQFTPANISRQAHILLDCGEDNVCKPKLEVSVDSDQKKIYIGDDNPLTLIVK
AQNQGEGAYEAELIVSIPLQADFIGVVRNNEALARLSCAFKTENQTRQVVCDLGNPMKAGTQLLAG
LRFSVHQQSEMDTSVKFDLQIQSSNLFDKVSPVVSHKVDLAVLAAVEIRGVSSPDHIFLPIPNWEH
KENPETEEDVGPVVQHIYELRNNGPSSFSKAMLHLQWPYKYNNNTLLYILHYDIDGPMNCTSDMEI
NPLRIKISSLQTTEKNDTVAGQGERDHLITKRDLALSEGDIHTLGCGVAQCLKIVCQVGRLDRGKS
AILYVKSLLWTETFMNKENQNHSYSLKSSASFNVIEFPYKNLPIEDITNSTLVTTNVTWGIQPAPM
PVPVWVIILAVLAGLLLLAVLVFVMYRMGFFKRVRPPQEEQEREQLQPHENGEGNSET
(19) das teratocarcinoma-derived growth factor 1 Protein TDGF1 (Genbank Accession No.: KP_003203.1)
   >gi|4507425|ref|NP_003203.1| teratocarcinoma-derived growth factor 1 isoform 1 precursor [Homo sapiens] 
MDCRKMARFSYSVIWIMAISKVFELGLVAGLGHQEFARPSRGYLAFRDDSIWPQEEPAIRPRSSQR
VPPMGIQHSKELNRTCCLNGGTCMLGSFCACPPSFYGRNCEHDVRKENCGSVPHDTWLPKKCSLCK
CWHGQLRCFPQAFLPGCDGLVMDEHLVASRTPELPPSARTTTFMLVGICLSIQSYY
(20) das Prostata spezifiche Membranantigen PSMA (Swiss Prot ID: Q04609)
   >gi|4758398|ref|NP_004467.1| glutamate carboxypeptidase 2 isoform 1 [Homo sapiens] 
MWNLLHETDSAVATARRPRWLCAGALVLAGGFFLLGFLFGWFIKSSNEATNITPKHNMKAFLDELK
AENIKKFLYNFTQIPHLAGTEQNFQLAKQIQSQWKEFGLDSVELAHYDVLLSYPNKTHPNYISIIN
EDGNEIFNTSLFEPPPPGYENVSDIVPPFSAFSPQGMPEGDLVYVNYARTEDFFKLERDMKINCSG
KIVIARYGKVFRGNKVKNAQLAGAKGVILYSDPADYFAPGVKSYPDGWNLPGGGVQRGNILNLNGA
GDPLTPGYPANEYAYRRGIAEAVGLPSIPVHPIGYYDAQKLLEKMGGSAPPDSSWRGSLKVPYNVG
PGFTGNFSTQKVKMHIHSTNEVTRIYNVIGTLRGAVEPDRYVILGGHRDSWVFGGIDPQSGAAVVH
EIVRSFGTLKKEGWRPRRTILFASWDAEEFGLLGSTEWAEENSRLLQERGVAYINADSSIEGNYTL
RVDCTPLMYSLVHNLTKELKSPDEGFEGKSLYESWTKKSPSPEFSGMPRISKLGSGNDFEVFFQRL
GIASGRARYTKNWETNKFSGYPLYHSVYETYELVEKFYDPMFKYHLTVAQVRGGMVFELANSIVLP
FDCRDYAVVLRKYADKIYSISMKHPQEMKTYSVSFDSLFSAVKNFTEIASKFSERLQDFDKSNPIV
LRMMNDQLMFLERAFIDPLGLPDRPFYRHVIYAPSSHNKYAGESFPGIYDALFDIESKVDPSKAWG
EVKRQIYVAAFTVQAAAETLSEVA
(21) die Tyrosin-Proteinkinase EPHA2 (Swiss Prot ID: P29317)
   >gi|32967311|ref|NP_004422.2| ephrin type-A receptor 2 precursor [Homo sapiens]
(22) das Oberflächenprotein SLC44A4 (Genbank Accession No: NP_001171515)
   >gi|95849282|ref|NP_001171515.1| choline transporter-like protein 4 isoform 2 [Homo sapiens] 
MGGKQRDEDDEAYGKPVKYDPSFRGPIKNRSCTDVICCVLFLLFILGYIVVGIVAWLYGDPRQVLY
PRNSTGAYCGMGENKDKPYLLYFNIFSCILSSNIISVAENGLQCPTPQTVITSLQQELCPSFLLPS
APALGRCFPWTNVTPPALPGITNDTTIQQGISGLIDSLNARDISVKIFEDFAQSWYWILVALGVAL
VLSLIFILLLRLVAGPLVLVLILGVLGVLAYGIYYCWEEYRVLRDKGASISQLGFTTNLSAYQSVQ
ETWLAALIVLAVLEAILLLMLIFLRQRIRIAIALLKEASKAVGQMMSTMFYPLVTFVLLLICIAYW
AMTALYLATSGQPQYVLWASNISSPGCEKVPINTSCNPTAHLVNSSCPGLMCVFQGYSSKGLIQRS
VFNLQIYGVLGLFWTLNWVLALGQCVLAGAFASFYWAFHKPQDIPTFPLISAFIRTLRYHTGSLAF
GALILTLVQIARVILEYIDHKLRGVQNPVARCIMCCFKCCLWCLEKFIKFLNRNAYIMIAIYGKNF
CVSAKNAFMLLMRNIVRVVVLDKVTDLLLFFGKLLVVGGVGVLSFFFFSGRIPGLGKDFKSPHLNY
YWLPIMTSILGAYVIASGFFSVFGMCVDTLFLCFLEDLERNNGSLDRPYYMSKSLLKILGKKNEAP
PDNKKRKK
(23) das Oberflächenprotein BMPR1B (SwissProt: O00238)
(24) das Transportprotein SLC7A5 (SwissProt: Q01650)
(25) das epitheliale Psostataantigen STEAP1 (SwissProt: Q9UHE8)
(26) das Ovarkarzinomantigen MUC16 (SwissProt: Q8WXI7)
(27) das Transportprotein SLC34A2 (SwissProt: 095436)
(28) das Oberflächenprotein SEMA5b (SwissProt: Q9P283)
(29) das Oberflächenprotein LYPD1 (SwissProt: Q8N2G4)
(30) der Endothelin Rezeptor Typ B EDNRB (SwissProt: P24530)
(31) das Ringfingerprotein RNF43 (SwissProt: Q68DV7)
(32) das Prostatakarzinom-assozierte Protein STEAP2 (SwissProt: Q8NFT2)
(33) der Kationenkanal TRPM4 (SwissProt: Q8TD43)
(34) der Komplementrezepior CD21 (SwissProt: P20023)
(35) das B-Zell Antigen Rezeptorkomplex-assozierte Protein CD79b (SwissProt: P40259)
(36) das Zelladhäsionsantigen CEACAM6 (SwissProt: P40199)
(37) die Dipeptidase DPEP1 (SwissProt: P16444)
(38) der Interleukinrezeptor IL20Ralpha (SwissProt: Q9UIIF4)
(39) das Proteoglykan BCAN (SwissProt: Q96GW7)
(40) der Ephrin Rezeptor EPHB2 (SwissProt: P29323)
(41) das Prostatastammzellenl-assozierte Protein PSCA (Genbank Accession No: NP_005663.2)
(42) das Oberflächenprotein LHFPL3 (SwissProt: Q86UP9)
(43) das Rezeptorprotein TNFRSF13C (SwissProt: Q96RJ3)
(44) das B-Zell Antigen Rezeptorkomplex-assozierte Protein CD79a (SwissProt: P11912)
(45) das Rezeptorprotein CXCR5 (SwissProt: P32302)
(46) der Ionenkanal P2X5 (SwissProt: Q93086)
(47) das Lymphozytenantigen CD180 (SwissProt: Q99467)
(48) das Rezeptorprotein FCRL1 (SwissProt: Q96LA6)
(49) das Rezeptorprotein FCRL5 (SwissProt: Q96RD9)
(50) das MHC Klasse II Molekül Ia Antigen HLA-DOB (Genbank Accession No: NP_002111.1)
(51) das T-Zell Protein VTCN1 (SwissProt: Q7Z7D3).

In einem bevorzugten Gegenstand der Erfindung ist das Krebs-Zielmolekül ausgewählt aus der Gruppe bestehend aus den Krebs-Zielmolckülen (1) - (51).

In einem weiteren besonders bevorzugten Gegenstand der Erfindung bindet der Binder an ein extrazelluläres Krebs-Zielmolekül, welches ausgewählt wird aus der Gruppe bestehend aus aus den Krebs-Zielmolekülen (1) - (51).

In einem weiteren besonders bevorzugten Gegenstand der Erfindung bindet der Binder spezifisch an ein extrazelluläres Krebs-Zielmolekül, welches ausgewählt wird aus der Gruppe bestehend aus aus den Krebs-Zielmolekülen (1) - (51).

In einem besonders bevorzugten Gegenstand der Erfindung ist das Krebs-Zielmoleküi ausgewählt aus der Gruppe bestehend aus EGF-Rezeptor (NP_005219.2), Mesothelin (Q13421-3), C4.4a (NP_055215.2) und Carboanhydrase IX (CA IX; NP_001207.2), insbesondere C4.4a (NP_055215.2).

In einem weiteren besonders bevorzugten Gegenstand der Erfindung bindet der Binder an ein extrazelluläres Krebs-Zielmolekül, welches ausgewählt wird aus der Gruppe bestehend aus EGF-Rezeptor (NP_005219.2), Mesothelin (Q13421-3), C4.4a (NP_055215.2) und Carboanhydrase IX (CA IX; Q16790)), insbesondere C4.4a (NP_055215.2).

In einem weiteren besonders bevorzugten Gegenstand der Erfindung bindet der Binder spezifisch an ein extrazelluläres Krebs-Zielmolekül, welches ausgewählt wird aus der Gruppe bestehend aus EGF-Rezeptor (NP_005219.2), Mesothelin (Q13421-3), C4.4a (NP_055215.2) und Carboanhydrase IX (CA IX: Q16790)), insbesondere C4.4a (NP_055215.2).

In einer bevorzugten Ausfuhrungsform wird der Binder nach Bindung an sein extrazelluläres Zielmolekül auf der Zielzelle durch die Bindung von der Zielzelle internalisiert. Dies bewirkt, dass das Binder-Wirkstoftkonjugat, welches ein Immunokonjugat oder ein ADC sein kann, von der Zielzelle aufgenommen wird.

In einer Ausführungsform ist der Binder ein Bindeprotein. In einer bevorzugten Ausführungsform ist der Binder ein Antikörper, ein antigen-bindendes Antikörperfragment, ein multispezifischer Antikörper oder ein Antikörpennimetikum.

Bevorzugte Antikörpermimetika sind Affibodies, Adnectins, Anticalins, DARPins, Avimers, oder Nanobodies, Bevorzugte multispezifischer Antikörper sind bispezifische und trispezifische Antikörper.

In einer bevorzugten Ausführungsform ist der Binder ein Antikörper oder ein antigen-bindendes Antikörperfragment, weiter bevorzugt ist ein isolierter Antikörper oder ein isoliertes antigen-bindendes Antikörperfragment.

Bevorzugte antigen-bindende Antikörperfragmente sind Fab, Fab', F(ab')₂ und Fv Fragmente, Diabodies, DAbs, lineare Antikörper und scFv. Besonders bevorzugt sind Fab, Diabodies und scFv.

In einer besonders bevorzugten Ausführungsform ist der Binder ein Antikörper. Besonders bevorzugt sind monoklonale Antikörper oder antigen-bindende Antikörperfragmente davon. Weiter besonders bevorzugt sind humane, humanisierte oder chimäre Antikörper oder antigen-bindende Antikörperfragmente davon.

Antikörper oder antigen-bindende Antikörperfragmente, die Krebs-Zielmoleküle binden, können vom Durchschnittsfachmann mit bekannten Verfahren hergestellt werden, wie z.B. chemische Synthese oder rekombinante Expression. Binder für Krebs-Zielmoleküle können kommerziell erworben werden oder können durch den Durchschnittsfachmann mit bekannten Verfahren hergestellt werden, wie z.B. chemische Synthese oder rekombinante Expression. Weitere Verfahren zur Herstellung von Antikörpern oder antigen-bindenden Antikörperfragmenten sind in WO 2007/070538 beschrieben (siehe Seite 22 "Antibodies"). Der Fachmann kennt Verfahren wie sogenannte Phage-Display Bibliotheken (z.B. Morphosys HuCAL Gold) erstellt und zur Auffindung von Antikörpern oder antigen-bindenden Antikörperfragmenten verwendet werden können (siehe WO 2007/070538, Seite 24 ff und Beispiel 1 auf Seite 70, Beispiel 2 auf Seite 72). Weitere Verfahren zur Herstellung von Antikörper, die DNA Bibliotheken aus B-Zellen verwenden, sind zum Beispiel auf Seite 26 (WO 2007/070538) beschrieben. Verfahren zur Humanisierung von Antikörpern sind auf Seite 30-32 von WO2007070538 und im Detail in Queen, et al.,.Pros. Natl. Acad. Sci. USA 86: 10029-10033,1989 oder in WO 90/0786 beschrieben. Des Weiteren sind dem Fachmann Verfahren zur rekombinanten Expression von Proteinen im allgemeinen und im speziellen von Antikörpern bekannt (siehe z.B. in Berger and Kimmel (Guide to Molecular Cloning Techniques, Methods in Enzymology, Vol. 152, Academic Press, Inc.); Sambrook, et al., (Molecular Cloning: A Laboratory Manual, (Second Edition, Cold Spring Harbor Laboratory Press; Cold Spring Harbor, N.Y.; 1989) Vol. 1-3); Current Protocols in Molecular Biolony, (F. M. Ausabel et al. [Eds.], Current Protocols, Green Publishing Associates, Inc. / John Wiley & Sons, Inc.); Harlow et al., (Monoclonal Antibodies: A Laboratory Manual, Cold Spring Harbor Laboratory Press (19881, Paul [Ed.]); Fundamental Immunology, (Lippincott Williams & Wilkins (1998)): and Harlow, et al., (Using Antibodies: A Laboratory Manual, Cold Spring Harbor Laboratory Press (1998)). Der Fachmann kennt die entsprechenden Vektoren, Promotoren und Signalpeptide die zur Expression eines Proteins/Antikörpers notwendig sind. Gebräuchliche Verfahren sind auch in WO 2007/070538 auf den Seiten 41 - 45 beschrieben. Verfahren zur Herstellung eines IgG1-Antikörpers sind z.B. in WO 2007/070538 in Beispiel 6 auf Seite 74 ff beschrieben. Verfahren, mit denen die Internalisierung eines Antikörpers nach Bindung an sein Antigen bestimmt werden kann, sind dem Fachmann bekannt und sind z.B. in WO 2007/070538 auf Seite 80 beschrieben. Der Fachmann kann die in WO 2007/070538 beschriebenen Verfahren, die zur Herstellung von Carboanhydrase IX (Mn)-Antikörpcrn verwendet wurden, anlog zur Herstellung für Antikörper mit anderer Zielmolekülspezifität verwenden.

### EGFR-Antikörper

Beispiele für Antikörper, die das Krebs-Zielmoleküle EGFR binden, sind Cetuximab (INN Nummer 7906), Panitumumab (INN Nummer 8499) und Nimotuzumab (INN Nummer 8545). Cetuximab (Drug Bank Accession Number DB00002) ist ein chimärer anti-EGFR1-Antikörper, der in SP2/0 Maus-Myelom-Zellen produziert wird und von ImClone Systems Inc/Merck KgaA/Bristol-Myers Squibb Co vertrieben wird. Cetuximab ist indiziert zur Behandlung des metastasierenden, EGFR exprimierenden Kolorektalkarzinoms mit Wildtyp-K-Ras Gen. Er hat eine Affinität von 10⁻¹⁰M.

### Sequenz:

Cetuximab Leichte Kette (kappa): 
DILLTQSPVILSVSPGERVSFSCRASQSIGTNIHWYQQRTNGSPRLLIKYASESISGIPSRFSGSG
SGTDFTLSINSVESEDIADYYCQQNNNWPTTFGAGTKLELKRTVAAPSVFIFPPSDEQLKSGTASV
 
VCLLNNFYPREAKVQWKVDNALQSGNSQESVTEQDSKDSTYSLSSTLTLSKADYEKHKVYACEVTH
QGLSSPVTKSFNRGEC
Cetuximab Schwere Kette:

QVQLKQSGPGLVQPSQSLSITCTVSGFSLTNYGVHWVRQSPGKGLEWLGVIWSGGNTDYNTPFTSR
LSINKDNSKSQVFFKMNSLQSNDTAIYYCARALTYYDYEFAYWGQGTLVTVSAASTKGPSVFPLAP
SSKSTSGGTAALGCLVKDYFPEPVTVSWNSGALTSGVHTFPAVLQSSGLYSLSSVVTVPSSSLGTQ
TYICNVNHKPSNTKVDKRVEPKSCDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNGKEYKCKVSNKAL
PAPIEKTISKAKGQPREPQVYTLPPSREEMTKNQVSLTCLVKGFYPSDIAVEWESNGQPENNYKTT
PPVLDSDGSFFLYSKLTVDKSRWQQGNVFSCSVMHEALHNHYTQKSLSLSPGK

Panitumumab (INN Nummer 8499) (Drug Bank Accession Number DB01269) ist ein rekombinanter monoklonaler humaner IgG2 Antikörper, der spezifisch an den humanen EGF-Rezeptor I bindet und von Abgenix / Amgen vertrieben wird. Panitumumab stammt aus der Immunisierung von transgenen Mäusen (XenoMouse). Diese Mäuse sind in der Lage humane Immunglobuline (leichte und schwere Ketten) zu produzieren. Es wurde ein spezieller B-Zell-Klon ausgewählt, der Antikörper gegen EGFR produziert, und dieser mit CHO-Zellen (Chinese hamster ovary cells) immortalisiert. Diese Zellen werden jetzt für die Produktion eines zu 100 % humanen Antikörpers verwendet. Panitumumab ist indiziert zur Behandlung des EGFR-exprimierenden, metastasierenden Kolorektalkarzinoms, das refraktär gegenüber einer chemotherapeutischen Behandlung mit Fluropyrimidin, Oxaliplatin und Irinotecan ist. Er hat eine Affinität von 10-11M.

### Sequenz:

Panitumumab Leichte Kette (kappa): 
DIQMTQSPSSLSASVGDRVTITCQASQDISNYLNWYQQKPGKAPKLLIYDASNLETGVPSRFSGSG
SGTDFTFTISSLQPEDIATYFCQHFDHLPLAFGGGTKVEIKRTVAAPSVFIFPPSDEQLKSGTASV
VCLLNNFYPREAKVQWKVDNALQSGNSQESVTEQDSKDSTYSLSSTLTLSKADYEKHKVYACEVTH
QGLSSPVTKSFNRGEC
Panitumumab Schwere Kette:

QVQLQESGPGLVKPSETLSLTCTVSGGSVSSGDYYWTWIRQSPGKGLEWIGHIYYSGNTNYNPSLK
SRLTISIDTSKTQFSLKLSSVTAADTAIYYCVRDRVTGAFDIWGQGTMVTVSSASTKGPSVFPLAP
CSRSTSESTAALGCLVKDYFPEPVTVSWNSGALTSGVHTFPAVLQSSGLYSLSSVVTVPSSNFGTQ
TYTCNVDHKPSNTKVDKTVERKCCVECPPCPAPPVAGPSVFLFPPKPKDTLMISRTPEVTCVVVDV
SHEDPEVQFNWYVDGVEVHNAKTKPREEQFNSTFRVVSVLTVVHQDWLNGKEYKCKVSNKGLPAPI
EKTISKTKGQPREPQVYTLPPSREEMTKNQVSLTCLVKGFYPSDIAVEWESNGQPENNYKTTPPML
DSDGSFFLYSKLTVDKSRWQQGNVFSCSVMHEALHNHYTQKSLSLSPG

Bei Nimotuzumab (INN Nummer 8545) (EP 00586002, EP 00712863) handelt es sich um einen humanisierten monoklonalen IgG1 Antikörper, der spezifisch an den humanen EGF-Rezeptor I bindet und von YM BioSciences Inc. (Mississauga Canada) vertrieben wird. Er wird in nichtsekretierenden NSO-Zellen (Säugerzelllinie) produziert. Nimotuzumab ist zugelassen zur Behandlung von Kopf- und Halstumoren, hoch-malignem Astrocytoma und Glioblastoma multiforme (nicht in EU und US) und Pankrcaskarzinom (Orphan drug, EMA). Er hat eine Affinität von 10⁻⁸ M.

Weitere Ausführungsformen für EGFR-Antikörper sind:
- Zalutumumab / 2F8 / HuMax-EGFr, Firma Genmab A/S (WO 02/100348, WO 2004/056847,INN-Nummer 8605)
- Necitumumab / 11F8, ImClone / IMC-11F8, Firma ImClone Systems Inc [Eli Lilly & Co] (WO 2005/090407 (EP 01735348-A1, US 2007/0264253-A1, US 7,598,350, WO 2005/090407-A1), INN- Nummer 9083)
- Matuzumab / anti-EGFR MAb, Merck KGaA / anti-EGFR MAb, Takeda / EMD 72000 / EMD-6200 / EMD-72000 und EMD-55900 / MAb 425 / monoclonal antibody 425, Firma Merck KGaA / Takeda (WO 92/15683, INN-Nummer 8103 (Matuzumab))
- RG-7160 / GA-201 / GA-101 / R-7160 / R7160 / RG7160/ RO-4858696 / RO-5083945 / RO4858696 / RO5083945, Firma Glycart Biotechnology AG (Roche Holding AG) (WO 2010/112413-A1, WO 2010/115554)
- GT-MAB 5.2-GEX / CetuGEX, Firma Glycotope GmbII (WO 2008/028686-A2 (EP 01900750-A1, EP 0191 1766-A1, EP 02073842-A2, US 2010/0028947-A1)
- ISU-101, Firma Isu Abxis Inc (ISU Chemical Co Ltd) / Scancell (WO 2008/004834-A1)
- ABT-806 / mAb-806 / ch-806 /anti-EGFR monoc. antibody 806, Firma Ludwig Institute for Cancer Research / Abbott / Life Science Pharmaceuticals (WO 02/092771, WO 2005/081854 und WO 2009/023265)
- SYM-004 (consists of two chimeric IgG1 antibodies (992 and 1024)), Firma Symphogen A/S (WO 2010/022736-A2)
- MR1-1 /MR1-1KDEL, Firma IVAX Corp (Teva Pharmaceutical Industries Ltd) (Duke University), (Patent: WO2001/062931-A2)
- Antikörper gegen die Deletionsmutante, EGFRvIII, Firma Amgen/Abgenix (WO 2005/010151, US 7,628,986)
- SC-100, Firma Scancell Ltd (WO 01/088138-A1)
- MDX-447 / EMD 82633 / BAB-447 / H 447 / MAb, EGFR, Medarex/Merck KgaA, Firma Bristol-Myers Squibb (US) / Merck KGaA (DE) / Takeda (JP), (WO 91/05871, WO 92/15683)
- anti-EGFR-Mab, Firma Xencor (WO 2005/056606)
- DXL-1218 / anti-EGFR monoclonal antibody (cancer), InNexus, Firma InNexus Biotechnology Inc, Pharmaprojects PH048638

In einer bevorzugten Ausführungsform werden die anti-EGFR Antikörper ausgewählt aus der Gruppe bestehend aus Cetuximab, Panitumumab, Nimotuzumab, Zalutumumab, Necitumumab, Matuzumab, RG-716, GT-MAB 5.2-GEX, ISU-101, ABT-806, SYM-004, MR1-1, SC-100, MDX-447, und DXL-1218.

In einer besonders bevorzugten Ausführungsform werden die anti-EGFR Antikörper ausgewählt aus der Gruppe bestehend aus Cetuximab, Panitumumab, Nimotuzumab, Zalutumumab, Necitumumab und Matuzumab.

Der Fachmann kennt Verfahren, mit denen aus den CDR Regionen der obengenannten Antikörper durch Sequenzvariationen weitere Antikörper hergestellt werden können, die eine ähnliche oder besser Affinität und/oder Spezifität zum Zielmolekül haben.

In einer weietern Ausführungsform werden die anti-EGFR Antikörper oder antigen-bindenden Antikörperfragmente ausgewählt aus der Gruppe bestehend aus

Antikörper oder antigenbindende Antikörperfragmente, die die drei CDR Regionen der leichten Kette und die drei CDR Regionen der schweren Kette eines der folgenden Antikörper umfasst :Cetuximab, Panitumumab, Nimotuzumab, Zalutumumab, Necitumumab, Matuzumab, RG-716, GT-MAB 5.2-GEX, ISU-101, ABT-806, SYM-004, MR1-1, SC-100, MDX-447, und DXL-1218.

In einer weiteren Ausführungsform werden die anti-EGFR Antikörper oder antigen-bindenden Antikörperfragmente ausgewählt aus der Gruppe bestehend aus

Antikörper oder antigenbindende Antikörperfragmente, die die drei CDR Regionen der leichten Kette und die drei CDR Regionen der schweren Kette eines der folgenden Antikörper umfasst: Cetuximab, Panitumumab, Nimotuzumab, Zalutumumab, Necitumumab, Matuzumab.

### Carboanhydrase IX Antikörper

Beispiele für Antikörper, die das Krebs-Zielmoleküle Carboanhydrase IX binden, sind in WO 2007/070538-A2 (z.B. Anspüche 1 - 16) beschrieben.

In einer bevorzugten Ausführungsform werden die anti-Carboanhydrase IX Antikörper oder antigen-bindenden Antikörperfragmente ausgewählt aus der Gruppe bestehend aus anti-Carboanhydrase IX Antikörper oder antigen-bindenden Antikörperfragmente 3ee9 (Anspruch 4 (a) in WO 2007/070538-A2), 3ef2 (Anspruch 4 (b) in WO 2007/070538-A2), 1e4 (Anspruch 4 (c) in WO 2007/070538-A2), 3a4 (Anspruch 4 (d) in WO 2007/070538-A2), 3ab4 (Anspruch 4 (e) in WO 2007/070538-A2), 3ah10 (Anspruch 4 (f) in WO 2007/070538-A2), 3bb2 (Anspruch 4 (g) in WO 2007/070538-A2), 1aa1 (Anspruch 4 (h) in WO 2007/070538-A2), 5a6 (Anspruch 4 (i) in WO 2007/070538-A2) und 5aa3 (Anspruch 4 (j) in WO 2007/070538-A2).

In einer bevorzugten Ausführungsform werden die anti-Carboanhydrase IX Antikörper oder antigen-bindenden Antikörperfragmente ausgewählt aus der Gruppe bestehend aus:
anti-Carboanhydrase IX Antikörper oder antigen-bindenden Antikörperfragmenten davon, die die Sequenzen der drei CDR Regionen der leichten Kette und die Sequenzen der drei CDR Regionen der schweren Kette des Antikörpers 3ee9 (aus WO 2007/070538-A2) umfassen,
anti-Carboanhydrase IX Antikörper oder antigen-bindenden Antikörperfragmenten davon, die die Sequenzen der drei CDR Regionen der leichten Kette und die Sequenzen der drei CDR Regionen der schweren Kette des Antikörpers 3cf2 (aus WO 2007/070538-A2) umfassen,
anti-Carboanhydrase IX Antikörper oder antigen-bindenden Antikörperfragmenten davon, die die Sequenzen der drei CDR Regionen der leichten Kette und die Sequenzen der drei CDR Regionen der schweren Kette des Antikörpers le4 (aus WO 2007/070538-A2) umfassen,
anti-Carboanhydrase IX Antikörper oder antigen-bindenden Antikörperfragmenten davon, die die Sequenzen der drei CDR Regionen der leichten Kette und die Sequenzen der drei CDR Regionen der schweren Kette des Antikörpers 3a4 (aus WO 2007/070538-A2) umfassen,
anti-Carboanhydrase IX Antikörper oder antigen-bindenden Antikörperfragmenten davon, die die Sequenzen der drei CDR Regionen der leichten Kette und die Sequenzen der drei CDR Regionen der schweren Kette des Antikörpers 3ab4 (aus WO 2007/070538-A2) umfassen,
anti-Carboanhydrase IX Antikörper oder antigen-bindenden Antikörperfragmenten davon, die die Sequenzen der drei CDR Regionen der leichten Kette und die Sequenzen der drei CDR Regionen der schweren Kette des Antikörpers 3ah10 (aus WO 2007/070538-A2) umfassen,
anti-Carboanhydrase IX Antikörper oder antigen-bindenden Antikörperfragmenten davon, die die Sequenzen der drei CDR Regionen der leichten Kette und die Sequenzen der drei CDR Regionen der schweren Kette des Antikörpers 3bb2 (aus WO 2007/070538-A2) umfassen, anti-Carboanhydrase IX Antikörper oder antigen-bindenden Antikörperfragmenten davon, die die Sequenzen der drei CDR Regionen der leichten Kette und die Sequenzen der drei CDR Regionen der schweren Kette des Antikörpers 1aa1 (aus WO 2007/070538-A2) umfassen,
anti-Carboanhydrase IX Antikörper oder antigen-bindenden Antikörperfragmenten davon, die die Sequenzen der drei CDR Regionen der leichten Kette und die Sequenzen der drei CDR Regionen der schweren Kette des Antikörpers 5a6 (aus WO 2007/070538-A2) umfassen und
anti-Carboanhydrase IX Antikörper oder antigen-bindenden Antikörperfragmenten davon, die die Sequenzen der drei CDR Regionen der leichten Kette und die Sequenzen der drei CDR Regionen der schweren Kette des Antikörpers 5aa3 (aus WO 2007/070538-A2) umfassen.

Die her angegebenen Sequenzen der CDR Regionen sind in Abbildungen 2a-2c, Seite 128-130 in WO 2007/070538-A2 offenbart.

In einer bevorzugten Ausführungsform werden die anti-Carboanhydrase IX Antikörper oder antigen-bindenden Antikörperfragmente ausgewählt aus der Gruppe bestehend aus:
einem Antikörper oder antigen-bindendem Fragment, der die Aminosäuresequenz der variablen leichten und variablen schweren Ketten des Antikörpers 3ee9, wie in WO 2007/070538-A2 in Abbildung 4b auf Seite 137 angegeben, umfasst,
einem Antikörper oder antigen-bindendem Fragment, der die Aminosäuresequenz der variablen leichten und variablen schweren Ketten des Antikörpers 3ef2, wie in WO 2007/070538-A2 in Abbildung 4c auf Seite 138, bzw. in Abbildung 4b auf Seite 137 angegeben, umfasst,
einem Antikörper oder antigen-bindendem Fragment, der die Aminosäuresequenz der variablen leichten und variablen schweren Ketten des Antikörpers le4, wie in WO 2007/070538-A2 in Abbildung 4a auf Seite 136 angegeben, umfasst,
einem Antikörper oder antigen-bindendem Fragment, der die Aminosäuresequenz der variablen leichten und variablen schweren Ketten des Antikörpers 3a4, wie in WO 2007/070538-A2 in Abbildung 4a auf Seite 136 angegeben, umfasst,
einem Antikörper oder antigen-bindendem Fragment, der die Aminosäuresequenz der variablen leichten und variablen schweren Ketten des Antikörpers 3ab4, wie in WO 2007/070538-A2 in Abbildung 4a auf Seite 136 angegeben, umfasst,
einem Antikörper oder antigen-bindendem Fragment, der die Aminosäuresequenz der variablen leichten und variablen schweren Ketten des Antikörpers 3ah10, wie in WO 2007/070538-A2 in Abbildung 4a auf Seite 136 angegeben, umfasst,
einem Antikörper oder antigen-bindendem Fragment, der die Aminosäuresequenz der variablen leichten und variablen schweren Ketten des Antikörpers 3bb2, wie in WO 2007/070538-A2 in Abbildung 4b auf Seite 137 angegeben, umfasst,
einem Antikörper oder antigen-bindendem Fragment, der die Aminosäuresequenz der variablen leichten und variablen schweren Ketten des Antikörpers 1aa1, wie in WO 2007/070538-A2 in Abbildung 4a auf Seite 136 angegeben, umfasst,
einem Antikörper oder antigen-bindendem Fragment, der die Aminosäuresequenz der variablen leichten und variablen schweren Ketten des Antikörpers 5a6, wie in WO 2007/070538-A2 in Abbildung 4b auf Seite 137 angegeben, umfasst, und
einem Antikörper oder antigen-bindendem Fragment, der die Aminosäuresequenz der variablen leichten und variablen schweren Ketten des Antikörpers 5aa3, wie in WO 2007/070538-A2 in Abbildung 4b auf Seite 137 angegeben, umfasst.

In einer besonders bevorzugten Ausführungsform ist der anti-Carboanhydrase IX Antikörper Antikörper 3ee9 aus WO 2007/070538-A2.

In einer besonders bevorzugten Ausführungsform umfasst der anti-Carboanhydrase IX Antikörper oder das antigen-bindenden Antikörperfragment die Aminosäuresequenzen der CDR Regionen der variablen schweren Kette des Antikörpers 3ee9 (VH3-CDR1: GFTFSSYGMS; VH3-CDR2: GlSSLOSTTYYADSVKG; VH3-CDR3: TGSPGTFMHGDH, siehe Abbildung 2a, Seite 128 in WO2007070538-A2) und die Aminosäuresequenzen der CDR Regionen der variablen leichten Kette des Antikörpers 3ee9 (VLk1-CDR1: RASQDINNYLS; VLk1-CDR2: YGASNLQS; VLk1-CDR3: QQYYGRPT, siehe Abbildung 2b, Seite 129 in WO 2007/070538-A2).

In einer besonders bevorzugten Ausführungsform umfasst der anti-Carboanhydrase IX Antikörper oder das antigen-bindenden Antikörperfragment die Aminosäuresequenzen der variablen schweren Kette des Antikörpers 3ee9

(VH3:ELVESGGGLVQPGGSLRLSCAASGFTFSSYGMSWVRQAPGKGLEWVSGlSSLGSTTY YADSVKGRFTISRDNSKNTLYLQMNSLRAEDTAVYYCARTGSPGTFMHGDHWGQGTLVT VSS, siehe Abbildung 4b, Seite 137 in WO2007070538-A2) und die Aminosäuresequenzen der variablen leichten Kette des Antikörpers 3ee9

(VLk1:DIQMTQSPSSLSASVGDRVTITCRaSQDINNYLSWYQQKPGKAPKLLIYGASNLQSG VPSRFSGSGSGTDFTLTISSLQPEDFAVYYCQQYYGRPTTFGQGTKVEIKRT, siehe Abbildung 4b, Seite 137 in WO2007070538-A2).

In einer bevorzugten Ausführungsform ist der anti-Carboanhydrase IX Antikörper 3cc9 ein IgG Antikörper.

In einer besonders bevorzugten Ausführungsform ist der anti-Carboanhydrase IX Antikörper 3ee9 ein IgG1 Antikörper (3ee9-IgG1),
wobei die Aminosäuresequenz der schweren Kette die folgende Sequenz umfasst: 
QVELVESGGGLVQPGGSLRLSCAASGFTFSSYGMSWVRQAPGKGLEWVSGISSLGSTTYYADSVKG
RFTISRDNSKNTLYLQMNSLRAEDTAVYYCARTGSPGTFMHGDHWGQGTLVTVSSASTKGPSVFPL
APSSKSTSGGTAALGCLVKDYFPEPVTVSWNSGALTSGVHTFPAVLQSSGLYSLSSVVTVPSSSLG
TQTYICNVNHKPSNTKVDKKVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLSPGK
und die Aminosäuresequenzen der leichten Kette die folgende Sequenz umfasst: 
DIQMTQSPSSLSASVGDRVTITCRASQDINNYLSWYQQKPGKAPKLLIYGASNLQSGVPSRFSGSG
SGTDFTLTISSLQPEDFAVYYCQQYYGRPTTFGQGTKVEIKRTVAAPSVFIFPPSDEQLKSGTASV
VCLLNNFYPREAKVQWKVDNALQSGNSQESVTEQDSKDSTYSLSSTLTLSKADYEKHKVYACEVTH
QGLSSPVTKSFNRGEC
 anti-Carboanhydrase IX Antikörper 3ee9-IgG1:
   Ein weiterer Aspekt der vorliegenden Erfindung ist die Bereitstellung des anti-Carboanhydrase IX Antikörpers 3ee9-IgG1,

### C4.4a Antikörper:

Erfindungsgemäß besonders bevorzugte Binder sind anti-C4.4a-Antikörper, insbesondere humane oder humanisierte anti-C4.4a-Antikörper. Die Antikörper weisen vorzugsweise eine Affinität von mindestens 10⁻⁷ M (als Kd-Wert; also vorzugsweise solche mit kleineren Kd-Werten als 10⁻⁷ M), vorzugsweise von mindestens 10⁻⁸ M, besonders bevorzugt in dem Bereich von 10⁻⁹ M bis 10⁻¹¹ M auf. Die Kd-Werte können z.B. durch Oberflächenplasmonenresonanzspektroskopie bestimmt werden.

Die erfindungsgemäßen Antikörper-Wirkstoff-Konjugate weisen ebenfalls Affinitäten in diesen Bereichen auf. Durch die Konjugation der Wirkstoffe wird die Affinität vorzugsweise nicht wesentlich beeinflusst (in der Regel wird die Affinität weniger als eine Größenordnung verringert, also z.B. maximal von 10⁻⁸ M auf 10⁻⁷ M).

Die erfindungsgemäßen verwendeten Antikörper zeichnen sich weiterhin vorzugsweise durch eine hohe Selektivität aus. Eine hohe Selektivität liegt vor, wenn der erfindungsgemäße Antikörper eine mindestens um den Faktor 2, bevorzugt Faktor 5 oder insbesondere bevorzugt Faktor 10 bessere Affinität am Zielprotein aufweist als an einem unabhängigen anderen Antigen, z.B. humanem Serumalbumin (die Affinität kann z.B. durch Oberflächenplasmonenresonanzspektroskopie bestimmt werden).

Zudem sind die erfindungsgemäßen verwendeten Antikörper vorzugsweise kreuzreaktiv. Um präklinische Studien, z.B. toxikologische oder Wirksamkeitsstudien (z.B. in Xenograft-Mäusen), zu erleichtern und besser interpretieren zu können, ist es von Vorteil, wenn der erfindungsgemäß verwendete Antikörper nicht nur das humane Zielprotein bindet, sondern auch in der für die Studien verwendeten Spezies das Spezies-Zielprotein bindet. In einer Ausführungsform ist der erfindungsgemäß verwendete Antikörper zusätzlich zum humanen Zielprotein kreuzreaktiv zum Zielprotein mindestens einer weiteren Spezies. Für toxikologische und Wirksamkeitsstudien werden bevorzugt Spezien der Familien Nager, Hunde und nicht-humane Primaten, verwendet. Bevorzugte Nager Spezien sind Maus und Ratte. Bevorzugte nicht-humane Primaten sind Rhesusaffen, Schimpansen und Langschwanzmakaken.

In einer Ausführungsform ist der erfindungsgemäß verwendete Antikörper zusätzlich zum humanen Zielprotein kreuzreaktiv zum Zielprotein mindestens einer weiteren Spezies ausgewählt aus der Gruppe von Spezien bestehend aus Maus, Ratte und Langschwanzmakak (Macaca fascicularis). Insbesondere bevorzugt sind erfindungsgemäß verwendete Antikörper, die zusätzlich zum humanen Zielprotein mindestens kreuzreaktiv zum Maus-Zielprotein sind. Bevorzugt sind kreuzreaktive Antikörper, deren Affinität zum Zielprotein der weiteren nicht-humanen Spezies sich nicht um mehr als den Faktor 50, insbesondere nicht mehr als den Faktor zehn von der Affinität zum humanen Zielprotein unterscheidet.

Anti-C4.4a-Antikörper sind z.B. in WO01/23553 oder WO2011070088 beschrieben. Diese Antikörper können erfindungsgemäß verwendet werden.

Beispiele für C4.4a Antikörper und antigen-bindende Fragmente sind nachfolgend beschrieben. Die Sequenzen der Antikörper sind in Tabelle 1 angegeben, wobei jede Zeile die jeweiligen CDR Aminosäuresequenzen der variablen leichten Kette bzw. der variablen Schweren Kette des in Spalte 1 aufgeführten Antikörpers wiedergibt. Die Aminosäuresequenzen der variablen leichten Kette und der variablen schweren Kette und die Nukleinsäuresequenz des jeweils in Spalte 1 angegeben Antikörpers ist ebenso angegeben.

In einer Ausführungsform binden die anti-C4.4a Antikörper oder antigen-hindenden Antikörperfragmente an die S1 Domäne S1 (Aminosäureposition 1-85 von SEQ ID NO: 1) von C4.4a.

In einer Ausführungsform sind die anti-C4.4a Antikörper oder antigen-bindenden Antikörperfragmente kreuzreaktiv mit humanem C4.4a (SEQ ID NO:1) und mit murinem C4.4a (SEQ ID NO:2).

In einer Ausführungsform werden die anti-C4.4a Antikörper oder antigen-bindenden Antikörperfragmente davon nach Bindung an eine C4.4a exprimierende Zelle von der Zelle internalisiert.

In einer weiteren Ausführungsform kompetieren die anti-C4.4a Antikörper oder antigen-bindenden Antikörperfragmente mit dem Antikörper M31-B01 und/oder mit dem Antikörper M20-D02-S-A um Bindung an C4.4a. Antikörper M31-B01 und M20-D02-S-A kompetieren um die Bindung an C4.4a. Die Antikörper B01-1 bis B01-12 wurden mittels Affinitätsmaturierung aus M31-B01 hergestellt und kompetieren mit M31-B01 um Bindung an C4.4a. Die Antikörper D02-1 bis D02-13 wurden mittels Affinitätsmaturierung aus M20-D02-S-A hergestellt und kompetieren mit M20-D02-S-A um Bindung an C4.4a.

In einer weiteren Ausführungsform umfassen die anti-C4.4a Antikörper oder antigen-bindenden Antikörperfragmente mindestens eine, zwei oder drei der in Tabelle 1 oder Tabelle 2 angeführten CDR Aminosäuresequenzen.

In einer weiteren Ausführungsform umfassen die anti-C4.4a Antikörper oder antigen-bindenden Antikörperfragmente mindestens eine, zwei oder drei CDR Aminosäuresequenzen eines in Tabelle 1 oder Tabelle 2 angeführten Antikörpers.

In einer weiteren Ausführungsform umfassen die anti-C4.4a Antikörper oder antigen-bindenden Antikörperfragmente mindestens eine, zwei oder drei CDR Aminosäuresequenzen der variablen leichten Kette und mindestens eine, zwei oder drei CDR Aminosäuresequenzen der variablen schweren Kette eines in Tabelle 1 oder Tabelle 2 angeführten Antikörpers.

In einer weiteren Ausführungsform umfassen die anti-C4.4a Antikörper oder antigen-bindenden Antikörperfragmente, die zu mindestens 50%, 60%, 70%, 80%, 90% oder 95% identisch sind mit den CDR Aminosäuresequenzen der variablen leichten Kette und mit den CDR Aminosäuresequenzen der variablen schweren Kette, eines in Tabelle 1 oder Tabelle 2 angeführten Antikörpers.

In einer weiteren Ausführungsform umfassen die CDR Sequenzen der anti-C4.4a Antikörper oder antigen-bindenden Antikörperfragmente

CDR Sequenzen der schweren Kette, die zu den CDR Sequenzen SEQ ID NO: 297 (CDR H1), SEQ ID NO: 298 (CDR H2) und SEQ ID NO: 299 (CDR H3) konform sind, und CDR Sequenzen der leichten Kette , die zu den CDR Sequenzen SEQ ID NO: 300 (CDR L1), SEQ ID NO: 22 (CDR L2) and SEQ ID NO: 301 (CDR L3) konform sind, oder

CDR Sequenzen der schweren Kette, die zu den CDR Sequenzen SEQ ID NO: 302 (CDR III), SEQ ID NO: 303 (CDR H2) and SEQ ID NO: 304 (CDR H3) konform sind, und CDR Sequenzen der leichten Kette , die zu den CDR Sequenzen SEQ ID NO: 305 (CDR LI), SEQ ID NO: 306 (CDR L2) and SEQ ID NO: 307 (CDR L3) konform sind.

In einer weiteren Ausführungsform umfassen die anti-C4.4a Antikörper oder antigen-bindenden Antikörperfragmente, die zu mindestens 50%, 60%, 70%, 80%, 90% oder 95% identisch sind mit der variablen leichten Kette und mit der variablen schweren Kette, eines in Tabelle 1 oder Tabelle 2 angeführten Antikörpers.

In einer weiteren Ausführungsform umfassen die anti-C4.4a Antikörper oder antigen-bindenden Antikörperfragmente die drei CDR Aminosäuresequenzen der variablen leichten Kette und die drei CDR Aminosäuresequenzen der variablen schweren Kette eines in Tabelle 1 oder Tabelle 2 angeführten Antikörpers.

In einer weiteren Ausführungsform umfassen die anti-C4.4a Antikörper oder antigen-bindenden Antikörperfragmente eine variable leichte Kette und/oder eine variable schwere Kette eines in Tabelle 1 oder Tabelle 2 angeführten Antikörpers.

In einer weiteren Ausführungsform umfassen die anti-C4.4a Antikörper oder antigen-bindenden Antikörperfragmente die variable leichte Kette und die variable schwere Kette eines in Tabelle 1 oder Tabelle 2 angeführten Antikörpers.

In einer bevorzugten Ausführungsform werden die C4.4a Antikörper und die antigen-bindenden Antikörperfragmente ausgewählt aus der Gruppe bestehend aus Antikörper, der die CDR Sequenzen der variablen schweren Kette wiedergegeben durch die Sequenzen SEQ ID NO: 75-77 und der die CDR Sequenzen der variablen leichten Kette wiedergegeben durch die Sequenzen SEQ ID NO: 78-80 umfasst (B01-10),
Antikörper, der die CDR Sequenzen der variablen schweren Kette wiedergegeben durch die Sequenzen SEQ ID NO: 5, 9 und 13 und der die CDR Sequenzen der variablen leichten Kette wiedergegeben durch die Sequenzen SEQ ID NO: 17, 21 und 25 umfasst (M31-B01),
Antikörper, der die CDR Sequenzen der variablen schweren Kette wiedergegeben durch die Sequenzen SEQ ID NO: 6, 10 und 14 und der die CDR Sequenzen der variablen leichten Kette wiedergegeben durch die Sequenzen SEQ ID NO: 18, 22 und 26 umfasst (M20-D02-S-A),
Antikörper, der die CDR Sequenzen der variablen schweren Kette wiedergegeben durch die Sequenzen SEQ ID NO: 7, 11 und 15 und der die CDR Sequenzen der variablen leichten Kette wiedergegeben durch die Sequenzen SEQ ID NO: 19, 23 und 27 umfasst (M60-G03),
Antikörper, der die CDR Sequenzen der variablen schweren Kette wiedergegeben durch die Sequenzen SEQ ID NO: 8, 12 und 16 und der die CDR Sequenzen der variablen leichten Kette wiedergegeben durch die Sequenzen SEQ ID NO: 20, 24 und 28 umfasst (36-H02),
Antikörper, der die CDR Sequenzen der variablen schweren Kette wiedergegeben durch die Sequenzen SEQ ID NO: 45-47 und der die CDR Sequenzen der variablen leichten Kette wiedergegeben durch die Sequenzen SEQ ID NO: 48-50 umfasst (B01-3), Antikörper, der die CDR Sequenzen der variablen schweren Kette wiedergegeben durch die Sequenzen SEQ ID NO: 55-57 und der die CDR Sequenzen der variablen leichten Kette wiedergegeben durch die Sequenzen SEQ ID NO: 58-60 umfasst (B01-5), Antikörper, der die CDR Sequenzen der variablen schweren Kette wiedergegeben durch die Sequenzen SEQ ID NO: 65-67 und der die CDR Sequenzen der variablen leichten Kette wiedergegeben durch die Sequenzen SEQ ID NO: 68-70 umfasst (B01-7), Antikörper, der die CDR Sequenzen der variablen schweren Kette wiedergegeben durch die Sequenzen SEQ ID NO: 85-87 und der die CDR Sequenzen der variablen leichten Kette wiedergegeben durch die Sequenzen SEQ ID NO: 88-90 umfasst (B01-12), Antikörper, der die CDR Sequenzen der variablen schweren Kette wiedergegeben durch die Sequenzen SEQ ID NO: 95-97 und der die CDR Sequenzen der variablen leichten Kette wiedergegeben durch die Sequenzen SEQ ID NO: 98-100 umfasst (D02-4), Antikörper, der die CDR Sequenzen der variablen schweren Kette wiedergegeben durch die Sequenzen SEQ ID NO: 105-107 und der die CDR Sequenzen der variablen leichten Kette wiedergegeben durch die Sequenzen SEQ ID NO: 108-110 umfasst (D02-6),
Antikörper, der die CDR Sequenzen der variablen schweren Kette wiedergegeben durch die Sequenzen SEQ ID NO: 115-117 und der die CDR Sequenzen der variablen leichten Kette wiedergegeben durch die Sequenzen SEQ ID NO: 118-120 umfasst (D02-7), Antikörper, der die CDR Sequenzen der variablen schweren Kette wiedergegeben durch die Sequenzen SEQ ID NO: 125-127 und der die CDR Sequenzen der variablen leichten Kette wiedergegeben durch die Sequenzen SEQ ID NO: 128-130 umfasst (D02-1 1), und Antikörper, der die CDR Sequenzen der variablen schweren Kette wiedergegeben durch die Sequenzen SEQ ID NO: 135-137 und der die CDR Sequenzen der variablen leichten Kette wiedergegeben durch die Sequenzen SEQ ID NO: 138-140 umfasst (D02-13).

In einer bevorzugten Ausführungsform werden die C4.4a Antikörper und die antigen-bindenden Antikörperfragmente ausgewählt aus der Gruppe bestehend aus Antikörper, die die Aminosäuresequenz der variablen schweren Kette wiedergegeben durch die Sequenz SEQ ID NO: 81 und die die Aminosäuresequenz der variablen leichten Kette wiedergegeben durch die Sequenz SEQ ID NO: 82 umfassen (B01-7). Antikörper, die die Aminosäuresequenz der variablen schweren Kette wiedergegeben durch die Sequenz SEQ ID NO: 33 und die die Aminosäuresequenz der variablen leichten Kette wiedergegeben durch die Sequenz SEQ ID NO: 29 umfassen (M31-B01), Antikörper, die die Aminosäuresequenz der variablen schweren Kette wiedergegeben durch die Sequenz SEQ ID NO: 34 und die die Aminosäuresequenz der variablen leichten Kette wiedergegeben durch die Sequenz SEQ ID NO: 30 umfassen (M20-D02 S-A), Antikörper, die die Aminosäuresequenz der variablen schweren Kette wiedergegeben durch die Sequenz SEQ ID NO: 35 und die die Aminosäuresequenz der variablen leichten Kette wiedergegeben durch die Sequenz SEQ ID NO: 31 umfassen (M60-G03), Antikörper, die die Aminosäuresequenz der variablen schweren Kette wiedergegeben durch die Sequenz SEQ ID NO: 36 und die die Aminosäuresequenz der variablen leichten Kette wiedergegeben durch die Sequenz SEQ ID NO: 32 umfassen (M36-H02), Antikörper, die die Aminosäuresequenz der variablen schweren Kette wiedergegeben durch die Sequenz SEQ ID NO: 51 und die die Aminosäuresequenz der variablen leichten Kette wiedergegeben durch die Sequenz SEQ ID NO: 52 umfassen (B01-3), Antikörper, die die Aminosäuresequenz der variablen schweren Kette wiedergegeben durch die Sequenz SEQ ID NO: 61 und die die Aminosäuresequenz der variablen leichten Kette wiedergegeben durch die Sequenz SEQ ID NO: 62 umfassen (B01-5), Antikörper, die die Aminosäuresequenz der variablen schweren Kette wiedergegeben durch die Sequenz SEQ ID NO: 71 und die die Aminosäuresequenz der variablen leichten Kette wiedergegeben durch die Sequenz SEQ ID NO: 72 umfassen (B01-7), Antikörper, die die Aminosäuresequenz der variablen schweren Kette wiedergegeben durch die Sequenz SEQ ID NO: 91 und die die Aminosäuresequenz der variablen leichten Kette wiedergegeben durch die Sequenz SEQ ID NO: 92 umfassen (B01-12), Antikörper, die die Aminosäuresequenz der variablen schweren Kette wiedergegeben durch die Sequenz SEQ ID NO: 101 und die die Aminosäuresequenz der variablen leichten Kette wiedergegeben durch die Sequenz SEQ ID NO: 102 umfassen (D02-4), Antikörper, die die Aminosäuresequenz der variablen schweren Kette wiedergegeben durch die Sequenz SEQ ID NO: 111 und die die Aminosäuresequenz der variablen leichten Kette wiedergegeben durch die Sequenz SEQ ID NO: 112 umfassen (D02-6), Antikörper, die die Aminosäuresequenz der variablen schweren Kette wiedergegeben durch die Sequenz SEQ ID NO: 121 und die die Aminosäuresequenz der variablen leichten Kette wiedergegeben durch die Sequenz SEQ ID NO: 122 umfassen (D02-7), Antikörper, die die Aminosäuresequenz der variablen schweren Kette wiedergegeben durch die Sequenz SEQ ID NO: 131 und die die Aminosäuresequenz der variablen leichten Kette wiedergegeben durch die Sequenz SEQ ID NO: 132 umfassen (D02-11), und Antikörper, die die Aminosäuresequenz der variablen schweren Kette wiedergegeben durch die Sequenz SEQ ID NO: 141 und die die Aminosäuresequenz der variablen leichten Kette wiedergegeben durch die Sequenz SEQ ID NO: 142 umfassen (D02-13).

In einer weiteren Ausführungsform umfassen die anti-C4.4a Antikörper die leichte Kette und die schwere Kette eines in Tabelle 2 angeführten Antikörpers.

In einer bevorzugten Ausführungsform umfassen die anti-C4.4a Antikörper die leichte Kette und die schwere Kette eines in Tabelle 2 angeführten Antikörpers.

In einer besonders bevorzugten Ausführungsform wird der C4.4a Antikörper ausgewählt aus der Gruppe bestehend aus

Antikörper, der die Aminosäuresequenz der leichten Kette repräsentiert durch SEQ ID NO: 346 und der die Aminosäuresequenz der schweren Kette repräsentiert durch SEQ ID NO: 347 umfasst (M31-B01) umfasst,

Antikörper, der die Aminosäuresequenz der leichten Kette repräsentiert durch SEQ ID NO: 352 und der die Aminosäuresequenz der schweren Kette repräsentiert durch SEQ ID NO: 353 umfasst (B01-3) umfasst,

Antikörper, der die Aminosäuresequenz der leichten Kette repräsentiert durch SEQ ID NO: 364 und der die Aminosäuresequenz der schweren Kette repräsentiert durch SEQ ID NO: 365 umfasst (B01-10) umfasst, und

Antikörper, der die Aminosäuresequenz der leichten Kette repräsentiert durch SEQ ID NO: 382 und der die Aminosäuresequenz der schweren Kette repräsentiert durch SEQ ID NO: 383 umfasst (D02-6) umfasst.

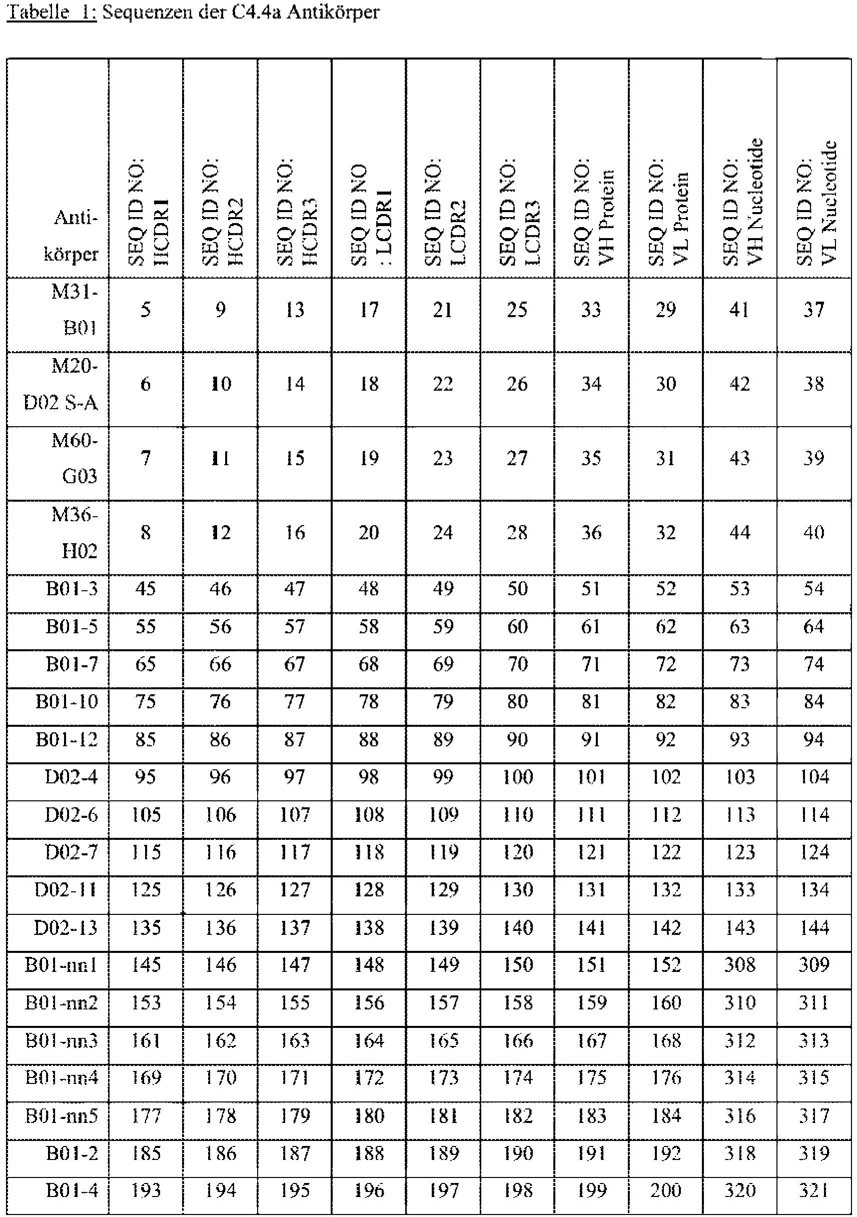

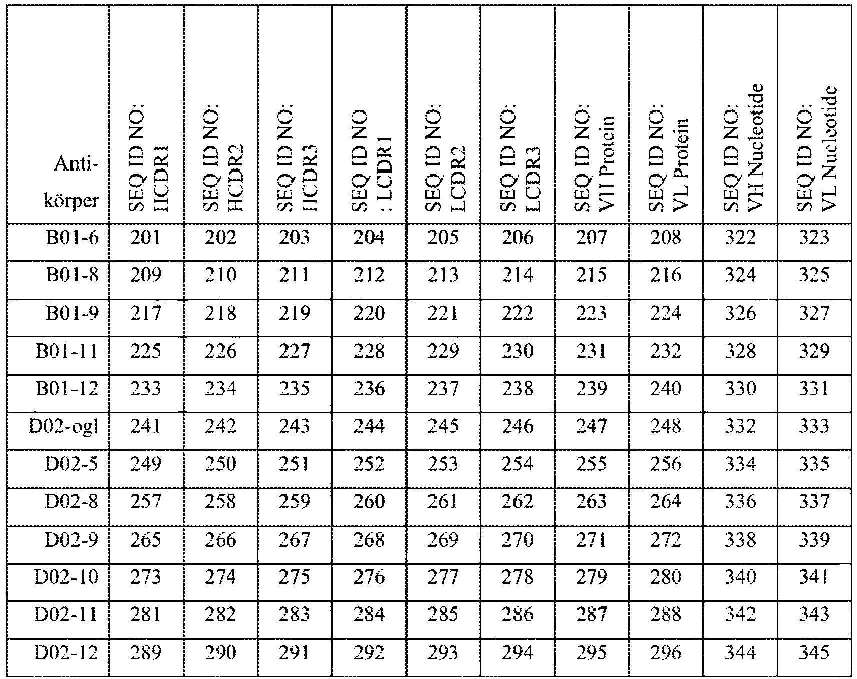

**Tabelle 2: Sequenzen der leichten und schweren Kette der C4.4a Antikörper**

| Antikörper | Leichte Kette SEQ ID NO: | Schwere Kette SEQ ID NO: |
|---|---|---|
| M31-B01 | 346 | 347 |
| B01-1 | 348 | 349 |
| B01-2 | 350 | 351 |
| B01-3 | 352 | 353 |
| B01-4 | 354 | 355 |
| B01-5 | 356 | 357 |
| B01-6 | 358 | 359 |
| B01-7 | 360 | 361 |
| B01-8 | 362 | 363 |
| B01-10 | 364 | 365 |
| B01-11 | 366 | 367 |
| B01-12 | 368 | 369 |
| M20-D02 S-A | 370 | 371 |
| D02-1 | 372 | 373 |
| D02-2 | 374 | 375 |
| D02-3 | 376 | 377 |
| D02-4 | 378 | 379 |
| D02-5 | 380 | 381 |
| D02-6 | 382 | 383 |
| D02-7 | 384 | 385 |
| D02-8 | 386 | 387 |
| D02-9 | 388 | 389 |
| D02-10 | 390 | 391 |
| D02-11 | 392 | 393 |
| D02-12 | 394 | 395 |
| D02-13 | 396 | 397 |

### anti-C4.4a Antikörper IgG:

Ein weiterer Aspekt der vorliegenden Erfindung ist die Bereitstellung eines anti-C4.4a IgG1 Antikörpers, der die Aminosäuresequenz der leichten Kette und der schweren Kette eines in Tabelle 2 angeführten Antikörpers umfaßt.

### Mesothelin Antikörper

Ein weiterer Aspekt der vorliegenden Erfindung ist die Bereitstellung eines neuen anti-Mesothelin Antikörpers (MF-Ta), dessen Aminosäuresequenz die CDR Sequenzen der variablen schweren Kette wiedergegeben durch die Sequenzen SEQ ID NO:398 (HCDR1), SEQ ID NO:399 (HCDR2) und SEQ ID NO:400 (HCDR3) und die CDR Sequenzen der variablen leichten Kette wiedergegeben durch die Sequenzen SEQ ID NO:401 (LCDR1). SEQ ID NO:402 (LCDR2) und SEQ ID NO:403 (LCDR3) umfasst.

In einer bevorzugten Ausführungsform umfasst die Aminosäuresequenz des anti-Mesothelin Antikörpers MF-Ta oder antigen-bindender Antikörperfragmente die Sequenz der variablen schweren Kette wiedergegeben durch die Sequenzen SEQ ID NO:404 und die Sequenz der variablen leichten Kette wiedergegeben durch die Sequenz SEQ ID NO:405. In einer bevorzugten Ausführungsform umfasst die Aminosäuresequenz des anti-Mesothelin Antikörpers MF-Ta oder antigen-bindender Antikörperfragmente die Sequenz der variablen schweren Kette, welche durch die Nukleinsäuresequenz SEQ ID NO:406 kodiert wird, und die Sequenz der variablen leichten Kette, welche durch die Nukleinsäuresequenz SEQ ID NO:407 kodiert wird.

In einer besonders bevorzugten Ausführungsform umfasst die Aminosäuresequenz des anti-Mesothelin Antikörpers MF-Ta die Sequenz der schweren Kette wiedergegeben durch die Sequenzen SEQ ID NO:408 und die Sequenz der leichten Kette wiedergegeben durch die Sequenz SEQ ID NO:409.

In einer besonders bevorzugten Ausführungsform umfasst die Aminosäuresequenz des anti-Mesothelin Antikörpers MF-Ta die Sequenz schweren Kette, welche durch die Nukleinsäuresequenz SEQ ID NO:410 kodiert wird, und die Sequenz der leichten Kette, welche durch die Nukleinsäuresequenz SEQ ID NO: 411 kodiert wird.

Weitere Beispiele für Antikörper, die das Krebs-Zielmoleküle Mesothelin binden, sind dem Fachmann bekannt und sind z.B. in WO 2009/068204 beschrieben und können für die erfindungsgemässen Binder-Wirkstoff-Konjugate verwendet werden.

In einer Ausführungsform der Binder-Wirkstoff-Konjugate ist der Binder ein anti-Mesothelin Antikörper oder antigen-bindendes Antikörperfragment, wobei der Antikörper an Mesothelin bindet und invariante Bindung zeigt.

In einer Ausführungsform der Binder-Wirkstoff-Konjugate umfasst ein anti-Mesothelin Antikörper oder antigen-bindendes Antikörperfragment die Aminosäuresequenzen der drei CDR Regionen der leichten Kette und die Aminosäuresequenzen der drei CDR Regionen der schweren Kette eines in WOv2009/068204-A1 (Tabelle 7; Seite 61 - 63) beschriebenen Antikörpers.

In einer bevorzugten Ausführungsform werden die Mesothelin Antikörper oder antigen-bindenden Antikörperfragmente ausgewählt aus der Gruppe bestehend aus
anti- Mesothelin Antikörper oder antigen-bindenden Antikörperfragmenten davon, die die Sequenzen der drei CDR Regionen der leichten Kette und die Sequenzen der drei CDR Regionen der schweren Kette des Antikörpers MF-Ta umfassen,
anti- Mesothelin Antikörper oder antigen-bindenden Antikörperfragmenten davon, die die Sequenzen der drei CDR Regionen der leichten Kette und die Sequenzen der drei CDR Regionen der schweren Kette des Antikörpers MF-J (WO2009068204-A1; Tabelle 7; Seite 61) umfassen,
anti- Mesothelin Antikörper oder antigen-bindenden Antikörperfragmenten davon, die die Sequenzen der drei CDR Regionen der leichten Kette und die Sequenzen der drei CDR Regionen der schweren Kette des Antikörpers MOR06640 (WO 2009/068204-A1; Tabelle 7; Seite 61) umfassen,
anti- Mesothelin Antikörper oder antigen-bindenden Antikörperfragmenten davon, die die Sequenzen der drei CDR Regionen der leichten Kette und die Sequenzen der drei CDR Regionen der schweren Kette des Antikörpers MF-226 (WO 2009/068204-A1, Tabelle 7; Seite 61) umfassen, und
anti-Mesothelin Antikörper oder antigen-bindenden Antikörperfragmenten davon, die die Sequenzen der drei CDR Regionen der leichten Kette und die Sequenzen der drei CDR Regionen der schweren Kette des Antikörpers MOR06626 (WO 2009/068204-A1; Tabelle 7; Seite 61) umfassen.

In einer besonders bevorzugten Ausführungsform werden die Mesothelin Antikörper oder antigen-bindenden Antikörperfragmente ausgewählt aus der Gruppe bestehend aus
anti-Mesothelin Antikörper oder antigen-bindenden Antikörperfragmenten davon, die die Sequenz der variablen leichten Kette und die Sequenz der variablen schweren Kette des Antikörpers MF-Ta umfassen,
anti-Mesothelin Antikörper oder antigen-bindenden Antikörperfragmenten davon, die die Sequenz der variablen leichten Kette und die Sequenz der variablen schweren Kette des Antikörpers MF-J (WO 2009/068204-A1; Tabelle 7; Seite 61) umfassen,
anti-Mesothelin Antikörper oder antigen-bindenden Antikörperfragmenten davon, die die Sequenz der variablen leichten Kette und die Sequenz der variablen schweren Kette des Antikörpers MOR06640 (WO 2009/068204-A1; Tabelle 7; Seite 61) umfassen,
anti-Mesothelin Antikörper oder antigen-bindenden Antikörperfragmenten davon, die die Sequenz der variablen leichten Kette und die Sequenz der variablen schweren Kette des Antikörpers MF-226 (WO 2009/068204-A1; Tabelle 7; Seite 61) umfassen, und
anti-Mesothelin Antikörper oder antigen-bindenden Antikörperfragmenten davon, die die Sequenz der variablen leichten Kette und die Sequenz der variablen schweren Kette des Antikörpers MOR06626 (WO 2009/068204-A1; Tabelle 7; Seite 61) umfassen.

### Weitere Antikörper:

Ein Beispiel für einen Antikörper, der das Krebs-Zielmoleküle Her2 bindet, ist Trastuzumab (Genentech). Trastuzumab ist ein humanisierter Antikörper, der zur unter anderem Behandlung von Brustkrebs eingesetzt wird. Ein Beispiel für einen Antikörper, der das Krebs-Zielmoleküle CD20 bindet, ist Rituximab (Genentech). Rituximab (CAS-Nummer: 174722-31-7) ist ein chimärer Antikörper, der zur Behandlung von Non-Hodgkin-Lymphom verwendet wird. Ein Beispiel für einen Antikörper, der das Krebs-Zielmoleküle CD52 bindet, ist Alemtuzumab (Genzyme). Alemtuzumab (CAS-Nummer: 216503-57-0) ist ein humanisierter Antikörper, der zur Behandlung von chronischer lymphatischer Leukämie eingesetzt wird.

Weitere Beispiele für Antikörper, die an HER2 binden, sind neben Trastuzumab (INN 7637, CAS NR: RN: 180288-69-1) und Pertuzumab (Cas NR: 380610-27-5), auch Antikörper, wie offenbart in WO 2009/123894-A2, WO 200/8140603-A2, oder in WO 2011/044368-A2. Beispiel für ein anti-HER2 Konjugat ist Trastuzumab-Emtansine (INN-Nr. 9295).

Beispiele für Antikörper, die das Krebs-Zielmolekül CD30 binden und zur Behandlung von Krebs z.B. Hodgkin-Lymphoma verwendet werden können, sind Brentuximab, Iratumumab und Antikörper, wie in WO 2008/092117, WO 2008/036688 oder WO 2006/089232 offenbart. Beispiele für ein anti- CD30 Konjugat ist Brentuximab Vedotin (INN-Nr. 9144).

Beispiele für Antikörper, die das Krebs-Zielmolekül CD22 binden und zur Behandlung von Krebs z.B. Lymphoma verwendet werden können, sind Inotuzumab oder Epratuzumab. Beispiele für anti-CD22 Konjugate sind Inotuzumab Ozagamycin (INN-Nr. 8574), oder anti-CD22-MMAE und anti-CD22-MC-MMAE (CAS RN: 139504-50-0 bzw. 474645-27-7).

Beispiele für Antikörper, die das Krebs-Zielmolekül CD33 binden und zur Behandlung von Krebs z.B. Leukämie verwendet werden können, sind Gemtuzumab oder Lintuzumab (INN 7580). Ein Beispiel für ein anti-CD33 Konjugat ist Gemtuzumab-Ozagamycin.

Ein Beispiel für einen Antikörper, der das Krebs-Zielmolekül NMB bindet und zur Behandlung von Krebs z.B. Melanom oder Brustkrebs verwendet werden kann, ist Glembatumumab (INN 9199). Ein Beispiel für ein anti-NMB Konjugat ist Glembatumumab Vedotin (CAS RN: 474645-27-7).

Ein Beispiel für einen Antikörper, der das Krebs-Zielmolekül CD56 bindet und zur Behandlung von Krebs z.B. Multiples Myelom, Kleinzelliges Lungenkarzinom, MCC oder Ovarialkarzinom verwendet werden kann, ist Lorvotuzumab. Ein Beispiel für ein anti-CD56 Konjugat ist Lorvotuzumab Mertansine (CAS RN: 139504-50-0).

Beispiele für Antikörper, die das Krebs-Zielmolekül CD70 binden und zur Behandlung: von Krebs z.B. Non-Hodgkin-Lymphom oder Nierenzellkrebs verwendet werden können, sind in WO 2007/038637-A2 oder WO 2008/070593-A2 offenbart. Ein Beispiel für ein anti-CD70 Konjugat ist SGN-75 (CD70 MMAF)

Ein Beispiel für einen Antikörper, der das Krebs-Zielmolekül CD74 bindet und zur Behandlung von Krebs z.B. Multiples Myelom verwendet werden kann, ist Milatuzumab. Ein Beispiel für ein anti-CD74 Konjugat ist Milatuzumab-Doxorubicin (CAS RN: 23214-92-8).

Ein Beispiel für einen Antikörper, der das Krebs-Zielmolekül CD19 bindet und zur Behandlung von Krebs z.B. Non-Hodgkin-Lymphom verwendet werden kann, ist in WO 2008/031056-A2 offenbart. Weitere Antikörper und Beispiele für ein anti-CD19 Konjugal (SAR3419) sind in WO 2008/047242-A2 offenbart.

Beispiele für Antikörper, die das Krebs-Zielmolekül Mucin-1 binden und zur Behandlung von Krebs z.B. Non-Hodkin-Lymphom verwendet werden können, sind Clivatuzumab oder die in WO 2003/106495-A2, WO 2008/028686-A2 offenbarten Antikörper. Beispiele für anti-Mucin Konjugate sind in WO 2005/009369-A2 offenbart.

Beispiele für Antikörper, die das Krebs-Zielmolekül CD138 binden und Konjugate davon, die zur Behandlung von Krebs z.B. Multiples Myelom verwendet werden können, sind WO 2009/080829-A1, WO 2009/080830-A1 offenbart.

Beispiele für Antikörper, die das Krebs-Zielmolekül Integrin alphaV binden und zur Behandlung von Krebs z.B. Melanoma, Sarcoma oder Carcinoma verwendet werden können, sind Intetumumab (Cas RN: 725735-28-4), Abciximab (Cas-RN: 143653-53-6), Etaracizumab (Cas-RN: 892553-42-3) oder die in US 7,465,449, EP 719859-A1, WO 2002/012501-A1 oder WO2006/062779-A2 offenbarten Antikörper. Beispiele für anti-Integrin AlphaV Konjugate sind Intetumumab-DM4 und weitere in WO 2007/024536-A2 offenbarte ADCs.

Beispiele für Antikörper, die das Krebs-Zielmolekül TDGF1 binden und zur Behandlung von Krebs verwendet werden können, sind die in WO 02/077033-A1, US 7,318,924, WO 2003/083041-A2 und WO 2002/088170-A2 offenbarten Antikörper. Beispiele für anti-TDGF1 Konjugate sind in WO 2002/088170-A2 offenbart.

Beispiele für Antikörper, die das Krebs-Zielmolekül PSMA binden und zur Behandlung von Krebs z.B. Prostatakarzinom verwendet werden können, sind die in WO 97/35616-A1, WO 99/47554-A1, und WO 01/009192-A1 offenbarten Antikörper. Beispiele für anti-PSMA Konjugate sind in WO 2009/026274-A1 offenbart.

Beispiele für Antikörper, die das Krebs-Zielmolekül EPHA2 binden, zur Herstellung eines Konjugats und zur Behandlung von Krebs verwendet werden können, sind in WO 2004/091375-A2 offenbart.

Beispiele für Antikörper, die das Krebs-Zielmolekül SLC44A4 binden, zur Herstellung eines Konjugats und zur Behandlung von Krebs, z.B. Pankreas- oder Prostatakarzinom verwendet werden können, sind in WO2009/033094-A2 und US2009/0175796-A1 offenbart.

Ein Beispiel für einen Antikörper, der das Krebs-Zielmolekül HLA-DOB bindet, ist der Antikörper Lym-1 (Cas-RN: 301344-99-0), der zur Behandlung von Krebs, z.B. Non-Hodgkin-Lymphom verwendet werden kann. Beispiele für anti-HLA-DOB Konjugate sind z.B. in WO 2005/081711-A2 offenbart.

Beispiele für Antikörper, die das Krebs-Zielmolekül VTCN1 binden, zur Herstellung eines Konjugats und zur Behandlung von Krebs, z.B. Ovarialkarzinom, Pankreas-, Lungen-, oder Brustkrebs verwendet werden können, sind in WO 2006/074418-A2 offenbart.

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Binder-Wirkstoff-Konjugate (ADCs) der Formel (Ia) weisen eine hohe und spezifische cytotoxische Aktivität gegenüber Tumorzellen auf, welche anhand der im vorliegenden experimentellen Teil (C-1. bis C-6.) aufgeführten Assays gezeigt werden kann. Diese hohe und spezifische cytotoxische Aktivität der erfindungsgemäßen Binder-Wirkstoff-Konjugate (ADCs) der Formel (la) wird durch die geeignete Kombination vom neuen N,N-Dialkylauristatin-Derivaten und Binder mit Linkem, welche sowohl eine enzymatisch, hydrolytisch oder reduktiv spaltbare Soll-Bruchstelle zur Freisetzung der Toxophore als auch keine solche Soll-Bruchstelle aufweisen, erreicht. Insbesondere durch Verwendung von stabilen Linkern, welche keine enzymatisch, hydrolytisch oder reduktiv spaltbare Soll-Bruchstelle zur Freisetzung der Toxophore aufweisen, und die nach Aufnahme des ADCs in die Tumorzelle und nach komplettem intrazellulären, enzymatischen Abbau des Antikörpers noch ganz oder teilweise intakt bleiben, wird die Wirkung sehr spezifisch auf die Tumorzelle eingegrenzt. Die Kompatibilität von ADCs mit stabilen Linkern setzt unter anderem voraus, dass die intrazellulär gebildeten Metabolite ausreichend effizient entstehen, ihr Target erreichen und dort ihre anti-proliferative Wirkung am Target in ausreichender Potenz entfalten können, ohne dass sie zuvor von Transporterproteinen wieder aus der Tumorzelle ausgeschleust werden. Die nach Aufnahme der erfindungsgemäßen Verbindungen der Formel (la) intrazellulär gebildeten Metabolite weisen ein vemindertes Potential als Substrat gegenüber Transporterproteinen auf, wodurch eine Rückverteilung in die systemische Zirkulation und damit die Auslösung von potentiellen Nebenwirkungen durch das Toxophor selbst unterdrückt wird.

Die Kompatibilität der ADCs mit einer stabilen Linkerchemie und dem betreffenden Target in Verbindung mit Metaboliten, die in geringerem Maße ein Substrat für Transporterproteine darstellen, bietet ein vergrößertes therapeutisches Fenster.

Insbesondere weisen die erfindungsgemäßen Binder-Wirkstoff-Konjugate der Formel (Ia) eine hohe und spezifische cytotoxische Aktivität gegenüber C4.4a-exprimierenden Tumorzellen auf. Die Aktivität gegenüber nicht-C4.4a-exprimierenden Tumorzellen ist dabei deutlich schwächer ausgeprägt.

Die erfindungsgemäßen Verbindungen sind aufgrund dieses Eigenschaftsprofils daher in besonderem Maße zur Behandlung von hyperproliferativen Erkrankungen beim Menschen und bei Säugetieren allgemein geeignet. Die Verbindungen können einerseits die Zellproliferation und Zellteilung hemmen, blockieren, verringern oder senken und andererseits die Apoptose verstärken.

Zu den hyperproliferativen Erkrankungen, zu deren Behandlung die erfindungsgemäßen Verbindungen eingesetzt werden können, zählt insbesondere die Gruppe der Krebs- und Tumorerkrankungen. Hierunter werden im Rahmen der vorliegenden Erfindung insbesondere die folgenden Erkrankungen verstanden, ohne jedoch auf sie beschränkt zu sein: Brustkarzinome und Brusttumore (ductale und lobuläre Formen, auch *in situ*), Atemwegstumore (kleinzelliges und nicht-kleinzelliges Karzinom, Bronchialkarzinom), Hirntumore (z.B. des Hirnstamms und des Hypothalamus, Astrocytoma, Medulloblastoma, Ependymoma sowie neuro-ectodermale und pineale Tumore), Tumore der Verdauungsorgane (Speiseröhre, Magen, Gallenblase, Dünndarm, Dickdarm, Rektum), Lebertumore (u.a. hepatozelluläres Karzinom, Cholangiokarzinom und gemischt-hepatozelluläres Cholangiokarzinom), Tumore des Kopf- und Halsbereiches (Larynx, Hypopharynx, Nasopharynx, Oropharynx, Lippen und Mundhöhle), Hauttumore (Plattenepithelkarzinom, Kaposi-Sarkom, malignes Melanom, Merkelzell-Hautkrebs und nicht-melanomartiger Hautkrebs), Tumore der Weichteile (u.a. Weichteilsarkome, Osteosarkome, maligne fibröse Histiozytome, Lymphosarkome und Rhabdomyosarkome), Tumore der Augen (u.a. intraokuläres Melanom und Retinoblastom), Tumore der endokrinen und exokrinen Drüsen (z.B. thyroide und parathyroide Drüsen, Bauchspeicheldrüse und Speicheldrüse), Tumore des Harntrakts (Blasen-, Penis-, Nieren-, Nierenbecken- und Harnleitertumore) sowie Tumore der reproduktiven Organe (Endometrium-, Zervix-, Ovarial-, Vaginal-, Vulva- und Uteruskarzinome der Frau sowie Prostata- und Hodenkarzinome des Mannes). Dazu gehören auch proliferative Bluterkrankungen in solider Form und als zirkulierende Blutzellen, wie Lymphome, Leukämien und myeloproliferative Erkrankungen, z.B. akute myeloide, akute lymphoblastische, chronisch-lymphozytische, chronischmyelogene und Haarzell-Leukämie, sowie AIDS-korrelierte Lymphome, Hodgkin-Lymphome, Non-Hodgkin-Lymphome, kutane T-Zell-Lymphome, Burkitt-Lymphome und Lymphome im zentralen Nervensystem.

### Bevorzugte hyperproliferative Erkrankungen für anti-CA9 Binder-Wirkstoff-Konjugate

Hyperproliferative Erkrankungen, zu deren Behandlung die erfindungsgemäßen Verbindungen bevorzugt eingesetzt werden können, sind CA9 überexprimierende Tumore, Brustkarzinome und Brusttumore (z.B. ductale und lobuläre Formen, auch *in situ*); Atemwegstumore (z.B. kleinzelliges und nicht-kleinzelliges Karzinom, Bronchialkarzinom), davon bevorzugt nichtkleinzelliges Lungenkarzinom; Hirntumore (z.B. des Hirnstamms und des Hypothalamus, Astrocytoma, Medulloblastoma, Ependymoma und/oder neuro-ectodermale und pineale Tumore); Tumore der Verdauungsorgane (z.B. Speiseröhre, Magen, Gallenblase, Dünndarm, Dickdarm, Rektum), davon besonders bevorzugt sind Magen- und Darmtumore; Lebertumore (u.a. hepatozelluläres Karzinom, Cholangiokarzinom und gemischt-hepatozelluläres Cholangiokarzinom); Tumore des Kopf- und Halsbereiches (z.B. Larynx, Hypopharynx, Nasopharynx, Oropharynx, Lippen, Mundhöhle, Zunge und Speiseröhre); Tumore des Harntrakts (Blasen-, Penis-, Nieren-, Nierenbecken- und Harnleitertumore), davon besonders bevorzugt Tumore der Nieren und der Blase; und/oder Tumore der reproduktiven Organe (Endometrium-, Zervix-, Ovarial-, Vaginal-, Vulva- und Uteruskarzinome der Frau und/oder Prostata- und Hodenkarzinome des Mannes), davon besonders bevorzugt Zervix- und Uteruskarzinome.

### Bevorzugte hyperproliferative Erkrankungen für anti-EGFR Binder-Wirkstoff-Konjugate

Hyperproliferative Erkrankungen, zu deren Behandlung die erfindungsgemäßen Verbindungen bevorzugt eingesetzt werden können, sind EGFR überexprimierende Tumore, Atemwegstumore (z.B. kleinzelliges und nicht-kleinzelliges Karzinom, Bronchialkarzinom), davon bevorzugt nichtkleinzelliges Lungenkarzinom; Tumore der Verdauungsorgane (z.B. Speiseröhre, Magen, Gallenblase, Dünndarm, Dickdarm, Rektum), davon besonders sind Darmtumore; Tumore der endokrinen und exokrinen Drüsen (z.B. thyroide und parathyroide Drüsen, Bauchspeicheldrüse und Speicheldrüse), davon bevorzugt Pankreas; Tumore des Kopf- und Halsbereiches (z.B. Larynx, Hypopharynx, Nasopharynx, Oropharynx, Lippen, Mundhöhle, Zunge und Speiseröhre); und/oder Gliome.

### Bevorzugte hyperproliferative Erkrankungen für anti-Mesothelin Binder-Wirkstoff-Konjugate

Hyperproliferative Erkrankungen, zu deren Behandlung die erfindungsgemäßen Verbindungen bevorzugt eingesetzt werden können, sind Mesothelin überexprimierende Tumore, Tumore der reproduktiven Organe (Endometrium-, Zervix-, Ovarial-, Vaginal-, Vulva- und Uteruskarzinome der Frau und/oder Prostata- und Hodenkarzinome des Mannes), davon bevorzugt Ovarialkarzinome; Tumore der endokrinen und exokrinen Drüsen (z.B. thyroide und parathyroide Drüsen, Bauchspeicheldrüse und Speicheldrüse), davon bevorzugt Pankreas; Atemwegstumore (z.B. kleinzelliges und nicht-kleinzelliges Karzinom, Bronchialkarzinom), davon bevorzugt nichtkleinzelliges Lungenkarzinom; und/oder Mesotheliome.

### Bevorzugte hyperproliferative Erkrankungen für anti-C4.4a Binder-Wirkstoff-Konjugate

Hyperproliferative Erkrankungen, zu deren Behandlung die erfindungsgemäßen Verbindungen bevorzugt eingesetzt werden können, sind C4.4a überexprimierende Tumore, Plattenepithelkarzinome (z.B. der Cervix, Vulva, Vagina, des Analkanals, Endometriums, Fallopian Rohrs, Penis, Scrotum, der Spesieröhre, Brust, der Blase, des Gallenganges, Endometriums, Uterus, und Ovars); Brustkarzinome und Brusttumore (z.B. ductale und lobuläre Formen, auch *in situ*); Atemwegstumore (z.B. kleinzelliges und nicht-kleinzelliges Karzinom, Bronchialkarzinom), davon bevorzugt nichtkleinzelliges Lungenkarzinom, Plattenepithel- und Adenokarzinom der Lunge; Tumore des Kopf- und Halsbereiches (z.B. Larynx, Hypopharynx, Nasopharynx, Oropharynx, Lippen, Mundhöhle, Zunge und Speiseröhre, Plattenepithelkarzinome des Kopf- und Halsbereiches); Tumore des Harntrakts (Blasen-, Penis-, Nieren-, Nierenbecken- und Harnleitertumore, Plattenepithelkarzinome der Blase), davon besonders bevorzugt Tumore der Nieren und der Blase; Hauttumore (Plattenepithelkarzinom, Kaposi-Sarkom, malignes Melanom, Merkelzell-Hautkrebs und nicht-melanomartiger Hautkrebs), davon besonders bevorzugt Melanome; Tumore der endokrinen und exokrinen Drüsen (z.B. thyroide und parathyroide Drüsen, Bauchspeicheldrüse und Speicheldrüse), davon bevorzugt Pankreas; Tumore der Verdauungsorgane (z.B. Speiseröhre, Magen, Gallenblase, Dünndarm, Dickdarm, Rektum), davon besonders kolorectale Karzinome; und/oder Tumore der reproduktiven Organe (Endometrium-, Zervix-, Ovarial-, Vaginal-, Vulva- und Uteruskarzinome der Frau und/oder Prostata- und Hodenkarzinome des Mannes), davon besonders bevorzugt Uteruskarzinome.

Diese gut beschriebenen Krankheiten des Menschen können mit vergleichbarer Ätiologie auch in anderen Säugetieren vorkommen und dort mit den Verbindungen der vorliegenden Erfindung behandelt werden.

Der Begriff "Behandlung" oder "behandeln" wird im Rahmen dieser Erfindung konventionell verwendet und bedeutet die Versorgung, Pflege und Betreuung eines Patienten mit dem Ziel, eine Krankheit oder gesundheitliche Abweichung zu bekämpfen, zu verringern, abzuschwächen oder zu erleichtern und die Lebensbedingungen zu verbessern, die durch diese Krankheit beeinträchtigt werden, wie beispielsweise bei einer Krebserkrankung.

Weiterer Gegenstand der vorliegenden Erfindung ist somit die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Das erfindungsgemäße anti-C4.4a Binder-Wirkstoff-Konjugat wird vorzugsweise zur Behandlung von Krebs in einem Patienten eingesetzt, wobei die zu behandelnden Krebszellen des Patienten C4.4a Expression haben. Bevorzugterweise werden Patienten behandelt, deren C4.4a Expression in Krebszellen höher ist als in gesunden Zellen.

Ein Verfahren zur Identifizierung von Patienten, die vorteilhaft auf ein anti-C4.4a Binder-Wirkstoff-Konjugat zur Behandlung von Krebs ansprechen, umfasst die Bestimmung der C4.4a Expression in Krebszellen des Patienten. In einer Ausführungsform wird die C4.4a Expression durch C4.4a Genexpressionsanalyse ermittelt. Der Fachmann kennt Verfahren zur Genexpressionsanalyse wie z.B. RNA Detektion, quantitative oder qualitative Polymerasekettenreaktion oder Fluoreszenz in-situ Hybridisierung (FISH). In einer weiteren bevorzugten Ausführungsform wird die C4.4a Expression mittels Immunohistochemie mit einem anti-C4.4a Antikörper ermittelt. Bevorzugt wird die Immunohistochemie an Fomaldehyd-fixiertem Gewebe durchgeführt. Der Antikörper zur Verwendung in der Immunohistochemie ist der gleiche Antikörper sein, der auch im Konjugat Verwendung findet. Der Antikörper zur Verwendung in der Immunohistochemie ist ein zweiter Antikörper, der, bevorzugt spezifisch, das Zielprotein C4.4a erkennt.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen.

Beispielsweise können die Verbindungen der vorliegenden Erfindung mit bekannten anti-hyperproliferativen, zytostatischen oder zytotoxischen Substanzen zur Behandlung von Krebserkrankungen kombiniert werden. Als geeignete Kombinationswirkstoffe seien beispielhaft genannt:
Aldesleukin, Alendronsäure, Alfaferon, Alitretinoin, Allopurinol, Aloprim, Aloxi, Altretamin, Aminoglutethimid, Amifostin, Amrubicin, Amsacrin, Anastrozol, Anzmet, Aranesp, Arglabin, Arsentrioxid, Aromasin, 5-Azacytidin, Azathioprin, BCG oder tice-BCG, Bestatin, Betamethason-Acetat, Betamethason-Natriumphosphat, Bexaroten, Bleomycin-Sulfat, Broxuridin, Bortezomib, Busulfan, Calcitonin, Campath, Capecitabin, Carboplatin, Casodex, Cefeson, Celmoleukin, Cerubidin, Chlorambucil, Cisplatin, Cladribin, Clodronsäure, Cyclophosphamid, Cytarabin, Dacarbazin, Dactinomycin, DaunoXome, Decadron, Decadron-Phosphat, Delestrogen, Denileukin Diftitox, Depomedrol, Deslorelin, Dexrazoxan, Diethylstilbestrol, Diflucan, Docetaxel, Doxifluridin, Doxorubicin, Dronabinol, DW-166HC, Eligard, Elitek, Ellence, Emend, Epirubicin, Epoetin-alfa, Epogen, Eptaplatin, Ergamisol, Estrace, Estradiol, Estramustin-Natriumphosphat, Ethinylestradiol, Ethyol, Etidronsäure, Etopophos, Etoposid, Fadrozol, Farston, Filgrastim, Finasterid, Fligrastim, Floxuridin, Fluconazol, Fludarabin, 5-Fluordeoxyuridin-Monophosphat, 5-Fluoruracil (5-FU), Fluoxymesteron, Flutamid, Formestan, Fosteabin, Fotemustin, Fulvestrant, Gammagard, Gemcitabin, Gemtuzumab, Gleevec, Gliadel, Goserelin, Granisetron-Hydrochlorid, Histrelin, Hycamtin, Hydrocorton, erythro-Hydroxynonyladenin, Hydroxyharnstoff, Ibritumomab Tiuxetan, Idarubicin, Ifosfamid, Interferon-alpha, Interferon-alpha-2, Interferon-alpha-2α, Interferon-alpha-2β, Interferon-alpha-n1, Interferon-alpha-n3, Interferon-beta, Interferon-gamma-1α, Interlcukin-2. Intron A, Iressa, Irinotecan, Kytril, Lentinan-Sulfat, Letrozol, Leucovorin, Leuprolid, Leuprolid-Acetat, Levamisol, Levofolinsäure-Calciumsalz, Levothroid, Levoxyl, Lomustin, Lonidamin, Marinol, Mechlorethamin, Mecobalamin, Medroxyprogesteron-Acetat, Megestrol-Acetat, Melphalan, Menest, 6-Mercaptopurin, Mesna, Methotrexat, Metvix, Miltefosin, Minocyclin, Mitomycin C, Mitotan, Mitoxantron, Modrenal, Myocet, Nedaplatin, Neulasta, Neumega, Neupogen, Nilutamid, Nolvadex, NSC-631570, OCT-43, Octreotid, Ondansetron-Hydrochlorid, Orapred, Oxaliplatin, Paclitaxel, Pediapred, Pegaspargase, Pegasys, Pentostatin, Picibanil, Pilocarpin-Hydrochlorid, Pirarubicin, Plicamycin, Porfimer-Natrium, Prednimustin, Prednisolon, Prednison, Premarin, Procarbazin, Procrit, Raltitrexed, Rebif, Rhenium-186-Etidronat, Rituximab, Roferon-A, Romurtid, Salagen, Sandostatin, Sargramostim, Semustin, Sizofiran, Sobuzoxan, Solu-Medrol, Streptozocin, Strontium-89-chlorid, Synthroid, Tamoxifen, Tamsulosin, Tasonermin, Tastolacton, Taxoter, Teceleukin, Temozolomid, Teniposid, Testosteron-Propionat, Testred, Thioguanin, Thiotepa, Thyrotropin, Tiludronsäure, Topotecan, Toremifen, Tositumomab, Tastuzumab, Teosulfan, Tretinoin, Trexall, Trimethylmelamin, Trimetrexat, Triptorelin-Acetat, Triptorelin-Pamoat, UFT, Uridin, Valrubicin, Vesnarinon, Vinblastin, Vincristin, Vindesin, Vinorelbin, Virulizin, Zinecard, Zinostatin-Stimalamer, Zofran; ABI-007, Acolbifen, Actimmun, Affinitak, Aminopterin, Arzoxifen, Asoprisnil, Atamestan, Atrasentan, Avastin, BAY 43-9006 (Sorafenib), CCI-779, CDC-501, Celebrex, Cetuximab, Crisnatol, Cyproteron-Acetat, Decitabin, DN-101, Doxorubicin-MTC, dSLIM, Dutasterid, Edotecarin, Eflornithin, Exatecan, Fenretinid, Histamin-Dihydrochlorid, Histrelin-Hydrogel-Implant, Holmium-166-DOTMP, Ibandronsäure, Interferon-gamma, Intron-PEG, Ixabepilon, Keyhole Limpet-IIemocyanin, L-651582, Lanreotid, Lasofoxifen, Libra, Lonafarnib, Miproxifen, Minodronat, MS-209, liposomales MTP-PE, MX-6, Nafarelin, Nemorubicin, Neovastat, Nolatrexed, Oblimersen, Onko-TCS, Osidem, Paclitaxel-Polyglutamat, Pamidronat-Dinatrium, PN-401, QS-21, Quazepam, R-1549, Raloxifen, Ranpimas, 13-*cis*-Retinsäure, Satraplatin, Seocalcitol, T-138067, Tarceva, Taxoprexin, Thymosin-alpha-1, Tiazofurin, Tipifarnib, Tirapazamin, TLK-286, Toremifen, TransMID-107R, Valspodar, Vapreotid, Vatalanib, Verteporfin, Vinflunin, Z-100, Zoledronsäure, sowie Kombinationen hiervon.

In einer bevorzugten Ausführungsform können die Verbindungen der vorliegenden Erfindung mit anti-hyperproliferativen Agenzien kombiniert werden, welche beispielhaft - ohne dass diese Aufzählung abschließend wäre - sein können:
Aminoglutethimid, L-Asparaginase, Azathioprin, 5-Azacytidin, Bleomycin, Busulfan, Carboplatin, Carmustin, Chlorambucil, Cisplatin, Colaspase, Cyclophosphamid, Cytarabin, Dacarbazin, Dactinomycin, Daunorubicin, Diethylstilbestrol, 2',2'-Difluordeoxycytidin, Docetaxel, Doxorubicin (Adriamycin), Epirubicin, Epothilon und seine Derivate, erythro-Hydroxynonyladenin, Ethinylestradiol, Etoposid, Fludarabin-Phosphat, 5-Fluordeoxyuridin, 5-Fluordeoxyuridin-Monophosphat, 5-Fluoruracil, Fluoxymesteron, Flutamid, Hexamethylmelamin, Hydroxyharnstoff, Hydroxyprogesteron-Caproat, Idarubicin, Ifosfamid, Interferon, Irinotecan, Leucovorin, Lomustin, Mechlorethamin, Medroxyprogesteron-Acetat, Megestrol-Acetat, Melphalan, 6-Mercaptopurin, Mesna, Methotrexat, Mitomycin C, Mitotan, Mitoxantron, Paclitaxel, Pentostatin, *N*-Phosphonoacetyl-L-aspartat (PALA), Plicamycin, Prednisolon, Prednison, Procarbazin, Raloxifen, Semustin, Streptozocin, Tamoxifen, Teniposid, Testosteron-Propionat, Thioguanin, Thiotepa, Topotecan, Trimethylmelamin, Uridin, Vinblastin, Vincristin, Vindesin und Vinorelbin.

In viel versprechender Weise lassen sich die erfindungsgemäßen Verbindungen auch mit biologischen Therapeutika wie Antikörpern (z.B. Avastin, Rituxan, Erbitux, Herceptin) kombinieren. Die erfindungsgemäßen Verbindungen können auch in Kombination mit gegen die Angiogenese gerichteten Therapien positive Effekte erzielen, wie zum Beispiel mit Avastin, Axitinib, Recentin, Regorafenib, Sorafenib oder Sunitinib. Kombinationen mit Inhibitoren des Proteasoms und von mTOR sowie Kombinationen mit Antihormonen und steroidalen metabolischen Enzyminhibitoren sind wegen ihres günstigen Nebenwirkungsprofils ebenfalls besonders geeignet.

Generell können mit der Kombination von Verbindungen der vorliegenden Erfindung mit anderen, zytostatisch oder zytotoxisch wirksamen Agenzien folgende Ziele verfolgt werden:
- eine verbesserte Wirksamkeit bei der Verlangsamung des Wachstums eines Tumors, bei der Reduktion seiner Größe oder sogar bei seiner völligen Eliminierung im Vergleich zu einer Behandlung mit einem einzelnen Wirkstoff;
- die Möglichkeit, die verwendeten Chemotherapeutika in geringerer Dosierung als bei der Monotherapie einzusetzen:
- die Möglichkeit einer verträglicheren Therapie mit weniger Nebeneffekten im Vergleich zur Einzelgabe;
- die Möglichkeit zur Behandlung eines breiteren Spektrums von Tumorerkrankungen;
- das Erreichen einer höheren Ansprechrate auf die Therapie;
- eine längere Überlebenszeit der Patienten im Vergleich zur heutigen Standardtherapie.

Darüber hinaus können die erfindungsgemäßen Verbindungen auch in Kombination mit einer Strahlentherapie und/oder einer chirurgischen Intervention eingesetzt werden.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie beispielsweise oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapsein), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subkutan, intrakutan, perkutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pilaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanolcat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- A431NS: humane Tumorzelllinie
- A549: humane Tumorzelllinie
- ABCB1: ATP-binding cassette sub-family B member 1 (Synonym für P-gp und MDR1)
- abs.: Absolut
- ADC: Antibody-drug-conjugate = Antikörper-Wirkstoff-Konjugat
- Ac: Acetyl
- aq.: wässrig, wässrige Lösung
- ATP: Adenosintriphosphat
- BCRP: Brustkrebs-Resistenz-Protein, ein Efflux-Transporter
- Boc: *tert*.-Butoxycarbonyl
- br.: breit (bei NMR)
- Bsp.: Beispiel
- *ca.*: *circa,* ungefähr
- CAIX: Carboanhydrase IX
- CI: chemische Ionisation (bei MS)
- d: Dublett (bei NMR)
- d: Tag(e)
- DC: Dünnschichtchromatographie
- DCI: direkte chemische Ionisation (bei MS)
- dd: Dublett von Dublett (bei NMR)
- DMAP: 4-*N,N*-Dimethylaminopyridin
- DME: 1,2-Dimethoxyethan
- DMEM: Dulbecco's Modified Eagle Medium (standardisiertes Nährmedium für die Zellkultur)
- DMF: *N,N*-Dimethylformamid
- DMSO: Dimethylsulfoxid
- DPBS, D-PBS, PBS: Dulbecco's Phosphat-gepufferte Salz-Lösung
PBS = DPBS = D-PBS, pH7,4, Fa. Sigma, No D8537
Zusammensetzung:
0,2 g KCl
0,2 g KH₂PO₄ (anhyd)
8,0 g NaCl
1,15 g Na₂HPO₄ (anhyd)
ad 1 l mit H₂O auffüllen
- dt: Dublett von Triplett (bei NMR)
- DTT: DL-Dithiothreitol
- d. Th.: der Theorie (bei chemischer Ausbeute)
- EDC: *N'*-(3-Dimethylaminopropyl)-*N*-ethylcarbodiimid-Hydrochlorid
- EGFR: Epidermal growth factor receptor = Epidermaler Wachstumsfaktor Rezeptor
- EI: Elektronenstoß-Ionisation (bei MS)
- ELISA: Enzyme-linked Immunosorbent Assay
- eq.: Äquivalent(e)
- ESI: Elektrospray-Ionisation (bei MS)
- ESI-MicroTofq: ESI-MicroTofq (Name des Massenspektrometer mit Tof = Time Of Flight und q = Quadrupol)
- FCS: fötales Kälberserum
- Fmoc: (9*H*-Fluoren-9-ylmethoxy)carbonyl
- ges.: gesättigt
- GTP: Guanosin-5'-triphosphat
- h: Stunde(n)
- HATU: *O*-(7-Azabenzotriazol-1-yl)-*N*,*N,N'*,*N*'-tetramethyluronium-hexafluorophosphat
- HCT-116: HumaneTumorzelllinie
- HEPES: 4-(2-Hydroxyethyl)piperazin-1-ethansulfonsäure
- HOAc: Essigsäure
- HOBt: 1-Hydroxy-1*H*-benzotriazol-Hydrat
- HOSu: *N*-Hydroxysuccinimid
- HPLC: Hochdruck-, Hochleistungsflüssigchromatographie
- HT29: humane Tumorzelllinie
- IC₅₀: halbmaximale Inhibitionskonzentration
- i.m.: intramuskulär, Applikation in den Muskel
- i.v.: intravenös, Applikation in die Vene
- konz.: konzentriert
- LC-MS: Flüssigchromatographie-gekoppelte Massenspektrometrie
- LLC-PK1-Zellen: Lewis lung carcinoma pork kidney cell line
- L-MDR: Human MDR1 transfizierte LLC-PK1 Zellen
- m: Multiplett (bei NMR)
- MDR1: Multidrug resistence protein 1
- min: Minute(n)
- MS: Massenspektrometrie
- MTT: 3-(4,5-Dimethylthiazol-2-yl)-2,5-diphenyl-2*H*-tetrazoliumbromid
- NCI-H292: humane Tumorzelllinie
- NCI-H520: humane Tumorzelllinie
- NMM: *N*-Methylmorpholin
- NMP: *N*-Methyl-2-pyrrolidinon
- NMR: Kernresonanzspektrometrie
- NMRI: Mausstamm, Herkunft aus dem Naval Medical Research Institute (NMRI)
- Nude Mäuse: Nacktmäuse (Versuchstiere)
- NSCLC: Non small cell lung cancer (Nicht-kleinzelliges Bronchialkarzinom)
- PBS: Phosphat-gepufferte Salz-Lösung
- Pd/C: Palladium auf Aktivkohle
- P-gp: P-Glycoprotein, ein Transporterprotein
- PNGaseF: Enzym zur Zuckerabspaltung
- quant.: quantitativ (bei Ausbeute)
- quart: Quartett (bei NMR)
- quint: Quintett (bei NMR)
- R_{f}: Retentionsindex (bei DC)
- RT: Raumtemperatur
- Rₜ: Retentionszeit (bei HPLC)
- s: Singulett (bei NMR)
- s.c.: subcutan, Applikation unter die Haut
- SCC-4: Humane Tumorzelllinie
- SCC-9: Humane Tumorzelllinie
- SCID Mäuse: Versuchsmäuse mit einem schweren kombinierten Immundefekt (severe combined immunodeficiency)
- t: Triplett (bei NMR)
- *tert.*: tertiär
- TFA: Trifluoressigsäure
- THF: Tetrahydrofuran
- UV: Ultraviolett-Spektrometrie
- v/v: Volumen zu Volumen-Verhältnis (einer Lösung)
- Z: Benzyloxycarbonyl

### HPLC- und LC-MS-Methoden:

### Methode 1 (LC-MS):

Instrument: Waters Acquity SQD UPLC System; Säule: Waters Acquity UPLC HSS T3 1.8µ 50 mm x 1 mm; Eluent A: 1 l Wasser + 0.25 ml 99%-ige Ameisensäure, Eluent B: 1 l Acetonitril + 0.25 ml 99%-ige Ameisensäure; Gradient: 0.0 min 90% A → 1,2 min 5% A → 2.0 min 5% A; Fluss: 0.40 ml/min; Ofen: 50°C; UV-Detektion: 210-400 nm.

### Methode 2 (LC-MS):

Instrument: Micromass QuattroPremier mit Waters UPLC Acquity; Säule: Thermo Hypersil GOLD 1.9µ 50 mm x 1 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min: Ofen: 50°C; UV-Detektion: 210 nm.

### Methode 3 (LC-MS):

Instrument: Micromass Quattro Micro MS mit HPLC Agilent Serie 1100; Säule: Thermo Hypersil GOLD 3µ 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 4 (LC-MS):

Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C: UV-Detektion: 210 nm.

### Methode 5 (HPLC):

Gerät: HP 1090 Serie II; Säule: Merck Chromolith SpeedROD RP-18e, 50 mm x 4.6 mm; Vorsäule: Merck Chromolith Guard Cartridge Kit RP-18e, 5 mm x 4.6 mm; Injektionsvolumen: 5 µl; Eluent A: 70% HClO₄ in Wasser (4 ml/Liter), Eluent B: Acetonitril, Gradient: 0.00 min 20% B → 0.50 min 20% B → 3.00 min 90% B → 3.50 min 90% B → 3.51 min 20% B → 4.00 min 20% B; Fluss: 5 ml/min; Säulentemperatur: 40°C.

### Methode 6 (HPLC):

Gerät: Waters 2695 mit DAD 996; Säule: Merck Chromolith SpeedROD RP-18e, 50 mm x 4.6 mm; BestNr: 1.51450.0001. Vorsäule: Merck Chromolith Guard Cartridge Kit RP-18e, 5 mm x 4,6 mm; BestNr: 1.51470.0001. Eluent A: 70% HClO₄ in Wasser (4 ml/Liter), Eluent B: Acetonitril; Gradient: 0.00 min 5% B → 0.50 min 5% B → 3.00 min 95% B → 4.00 min 95% B; Fluss: 5 ml/min.

### Methode 7(LC-MS):

Gerätetyp MS: Waters ZQ; Gerätetyp HPLC: Agilent 1100 Series; UV DAD: Säule: Thermo Hypersil GOLD 3µ 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 3.0 min 10% A → 4.0 min 10% A → 4.1 min 100% A (Fluss 2.5 ml/min); Ofen: 55°C; Fluss: 2 ml/min; UV-Detektion: 210 nm.

### Methode 8 (LC-MS):

Gerätetyp MS: Waters ZQ; Gerätetyp HPLC: Agilent 1100 Series; UV DAD; Säule: Thermo Hypersil GOLD 3µ 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 2.0 min 60% A → 2.3 min 40% A → 3.0 min 20% A → 4.0 min 10% A → 4.2 min 100% A (Fluss 2.5 ml/min); Ofen: 55°C; Fluss: 2 ml/min; UV-Detektion: 210 nm.

### Methode 9 (LC-MS):

Instrument: Waters Acquity SQD UPLC System; Säule: Waters Acquity UPLC HSS T3 1.8µ 50 mm x 1 mm; Eluent A: 1 l Wasser + 0.25 ml 99%-ige Ameisensäure, Eluent B: 1 l Acetonitril + 0.25 ml 99%-ige Ameisensäure; Gradient: 0.0 min 95% A → 6.0 min 5% A → 7.5 min 5% A; Ofen: 50°C; Fluss: 0.35 ml/min; UV-Detektion: 210-400 nm.

### Methode 10 (HPLC):

Gerät: Agilent 1200 Series; Säule: Agilent Eclipse XDB-C18 5µ 4.6 mm x 150 mm: Vorsäule: Phenomenex KrudKatcher Disposable Pre-Column; Injektionsvolumen: 5 µl; Eluent A: 1 l Wasser + 0.01% Trifluoressigsäure; Eluent B: 1 l Acetonitril + 0.01% Trifluoressigsäure; Gradient: 0.00 min 10% B → 1.00 min 10% B → 1.50 min 90% B → 5.5 min 10% B; Fluss: 2 ml/min; Säulentemperatur: 30°C.

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

### Methode 11 (LC-MS):

Instrument: Waters ACQUITY SQD UPLC System; Säule: Waters Acquity UPLC HSS T3 1.8 µ 30 x 2 mm; Eluent A: 1 1 Wasser + 0.25 ml 99%ige Ameisensäure, Eluent B: 1 l Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Methode 12 (HPLC):

Gerät: Agilent 1200 Series mit Säulenofen und DAD; Säule: Merck Chromolith SpeedROD RP-18e, 50 mm x 4.6 mm; BestNr: 1.51450,0001, Vorsäule: Merck Chromolith Guard Cartridge Kit RP-18e, 5 mm x 4.6 mm; BcstNr: 1.51470.0001. Eluent A: 70% HClO₄ in Wasser (4 ml/Liter), Eluent B: Aectonitril; Gradient: 0.00 min 5% B → 0.50 min 5% B → 3.00 min 95% B → 4.00 min 95% B; Fluss: 5 ml/min; Säulentemperatur: 30°C.

### Methode 13 (LC-MS):

Instrument MS: Waters (Micromass) Quattro Micro; Instrument HPLC: Agilent 1100 Serie; Säule : YMC-Triart C18 3 µ 50 x 3 mm; Eluent A: 1 l Wasser + 0.01 mol Ammoniumcarbonat, Eluent B: 1 l Acetonitril; Gradient: 0.0 min 100% A → 2.75 min 5% A → 4.5 min 5% A; Ofen: 40°C; Fluss: 1.25 ml/min; UV-Detektion: 210 nm

### Ausganasverbindungen und Intermediate:

### Ausgangsverbindung 1

### (2R,3R)-3-[(2S)-1-(tert.-Butoxycarbonyl)pyrrolidin-2-yl]-3-methoxy-2-methylpropansäure (Boc-Dolaproin)

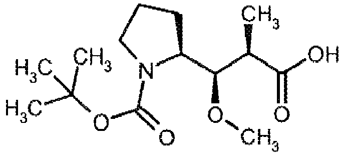

Die Titelverbindung kann auf verschiedenen Wegen nach Literaturvorschriften hergestellt werden, siehe z.B. Pettit et al., Synthesis 1996, 719; Shioiri et al., Tetrahedron Lett. 1991, 32, 931; Shioiri et al., Tetrahedron 1993, 49, 1913; Koga et al., Tetrahedron Lett 1991, 32, 2395; Vidal et al., Tetrahedron 2004, 60, 9715; Poncet et al., Tetrahedron 1994, 50, 5345. Sie wurde entweder als freie Säure oder als 1:1 Salz mit Dicycclohexylamin hergestellt.

### Ausgangsverbindung 2a

### tert.-Butyl-(3R,4S,5S)-3-methoxy-5-methyl-4-(methylamino)heptanoat-Hydrochlorid (Dolaisoleucin-OtBu x HCl)

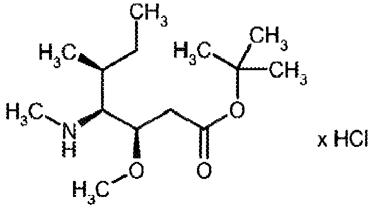

Die Titelverbindung kann auf verschiedenen Wegen nach Literaturvorschriften hergestellt werden, siehe z.B. Pettit et al., J. Org. Chem. 1994, 59, 1796; Koga et al., Tetrahedron Lett. 1991, 32, 2395; Shioiri et al., Tetrahedron Lett. 1991, 32, 931; Shioiri et al., Tetrahedron 1993, 49, 1913.

### Ausgangsverbindung 2b

### tert.-Butyl-(3R,4S,5S)-3-methoxy-5-methyl-4-(methylamino)heptanoat (Dolaisoleucin-O'Bu)

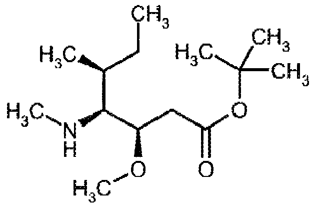

Die Verbindung wurde in Analogie zur Ausgangsverbindung 2a hergestellt, jedoch wurde die Hydrierung ohne Zusatz von IN Salzsäure durchgeführt.

### Ausgangsverbindung 3

### N^{α}-(tert.-Butoxycarbonyl)-N-hydroxy-L-phenylalaninamid

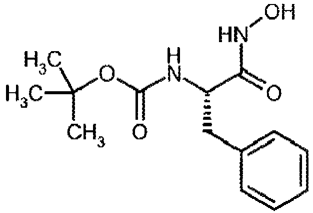

Die Titelverbindung wurde nach Literaturvorschrift hergestellt (A. Ritter et al., J. Org. Chem. 1994, 59,4602).
Ausbeute: 750 mg (75% d. Th.)
LC-MS (Methode 3): Rₜ = 1.67 min; MS (ESIpos): m/z = 281 (M+H)⁺.

### Ausgangsverbindung 4

### 1,2-Oxazolidin-Hydrochlorid

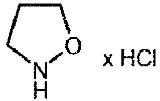

Die Titelverbindung kann nach Literaturvorschriften hergestellt werden, siehe z.B. H. King, J. Chem. Soc. 1942,432; sie ist auch kommerziell erhältlich.

### Ausgangsverbindung 5

### 1,2-Oxazinan-Hydrochlorid

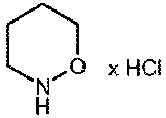

Die Titelverbindung kann nach Literaturvorschriften hergestellt werden, siehe z.B. H. King, J. Chem. Soc. 1942, 432.

### Ausgangsverbindung 6

### 2-Oxa-3-azabicyclo[2.2.2]oct-5-en

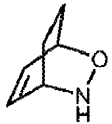

Die Titelverbindung kann in Boc-geschützter Form nach Literaturvorschrift hergestellt werden (siehe z.B. C. Johnson et al., Tetrahedron Lett. 1998, 39, 2059); die Entschützung erfolgte auf übliche Weise durch Behandlung mit Trifluoressigsäure und anschließende Neutralisation.
Ausbeute: 149 mg (89% d. Th.).

### Ausgangsverbindung 7

### tert.-Butyl-[(1S,2R)-1-(hydroxycarbamoyl)-2-phenylcyclopropyl]carbamat

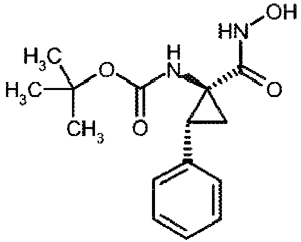

Die Titelverbindung wurde gemäß einer Literaturvorschrift (A. Ritter et al., J. Org. Chem. 1994, 59, 4602) hergestellt ausgehend von kommerziell erhältlicher (1*S*,2*R*)-1-[(*tert*.-Butoxycarbonyl)-amino]-2-phenylcyclopropancarbonsäure (C. Cativiela et al., Chirality 1999, 11, 583).
Ausbeute: 339 mg (59% d. Th.)
LC-MS (Methode 1): Rₜ = 0.82 min; MS (ESIpos): m/z = 293 (M+H)⁺.

### Intermediat 1

### tert.-Butyl-(3R,4S,5S)-4-[{N-[(benzyloxy)carbonyl]-L-valyl}(meythyl)amino]-3-methoxy-5-methyl-heptanoat

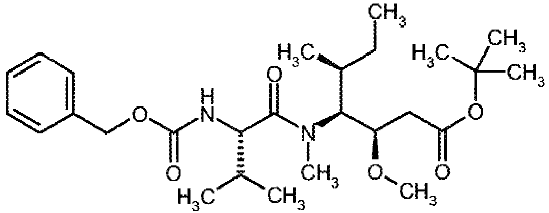

10.65 g (41.058 mmol) *tert*.-Butyl-(3*R*,4*S*,5*S*)-3-methoxy-5-methyl-4-(methylamino)-heptanoat (Ausgangsverbindung 2b) wurden in 250 ml Dichlormethan aufgenommen und die Lösung auf - 10°C abgekühlt. Dann wurden unter Rühren 10.317 g (41.058 mmol) N-[(Benzyloxy)carbonyl]-L-valin, 16.866 g (61.586 mmol) 2-Brom-1-ethylpyridinium-Tetrafluoroborat (BEP) sowie 28.6 ml *N,N*-Diisopropylethylamin zugegeben und die Mischung anschließend 20 h bei RT gerührt. Das Reaktionsgemisch wurde danach mit Dichlormethan verdünnt und zweimal mit gesättigter Natriumchloridlösung ausgeschüttelt, über Natriumsulfat getrocknet, filtriert und eingeengt. Der Rückstand wurde durch Flash-Chromatographie an Kieselgel mit Petrolether/Ethylacetat 4:1 als Eluent gereinigt. Die entsprechenden Fraktionen wurden eingeengt und der Rückstand über Nacht im Hochvakum getrocknet. Es wurden 10.22 g (51% d. Th.) der Titelverbindung als gelbliches Öl erhalten.
HPLC (Methode 5): Rₜ = 2.3 min;
LC-MS (Methode 2): Rₜ = 1.59 min; MS (ESIpos): m/z = 493 (M+H)⁺.

### Intermediat 2

### tert.-Butyl-(3R,4S,5S)-3-methoxy-5-methyl-4-[methyl(L-valyl)amino]heptanoat

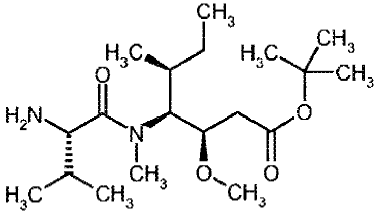

500 mg (1 mmol) *tert*.-Butyl-(3*R*,4*S*,5*S*)-4-[{*N*-[(benzyloxy)carbonyl]-L-valyl}(methyl)amino]-3-methoxy-5-methylheptanoat (Intermediat 1) wurden in 50 ml Methanol gelöst und nach Zugabe von 100 mg 10%-igem Palladium auf Aktivkohle 1 h lang bei RT unter Wasserstoff-Normaldruck hydriert. Der Katalysator wurde danach abfiltriert und das Lösungsmittel im Vakuum entfernt. Man erhielt 370 mg (quant.) der Titelverbindung als nahezu farbloses Öl.
HPLC (Methode 5): Rₜ = 1.59 min:
LC-MS (Methode 1): Rₜ = 0.74 min; MS (ESIpos): m/z = 359 (M+H)⁺.

### Intermediat 3

### N-[(9H-Fluoren-9-ylmethoxy)carbonyl]-N-methyl-L-valyl-N-[(3R,4S,5S)-1-tert.-butoxy-3-methoxy-5-methyl-1-oxoheptan-4-yl]-N-methyl-L-valinamid

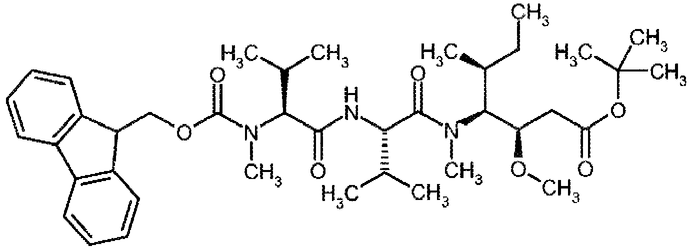

4.64 g (13.13 mmol) *N*-[(9*H*-Fluoren-9-ylmethoxy)carbony]-*N*-methyl-L-valin wurden in 20 ml DMF gelöst und nacheinander mit 4.28 g (11.94 mmol) *tert*.-Butyl-(3*R*,4*S*,5*S*)-3-methoxy-5-methyl-4-[methyl(L-valyl)amino]heptanoat (Intermediat 2), 2.75 g (14.33 mmol) 1-(3-Dimethylaminopropyl)-3-ethylcarbodiimid-Hydrochlorid sowie 2.2 g (14.33 mmol) 1-Hydroxy-1*H*-benzotriazol-Hydrat versetzt. Die Mischung wurde über Nacht bei RT gerührt. Das Reaktionsgemisch wurde danach in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter NatriumchloridLösung gewaschen, über Magnesiumsulfat getrocknet, filtriert und eingeengt. Der Rückstand wurde direkt, ohne weitere Reinigung, in der nächsten Stufe eingesetzt.
Ausbeute: 9.1 g (quant., 60%-ige Reinheit)
HPLC (Methode 5): Rₜ = 2.7 min;
LC-MS (Methode 2): Rₜ = 1.99 min; MS (ESIpos): m/z = 694 (M+H)⁺.

### Intermediat 4

### N-[(9H-Fluoren-9-ylmethoxy)carbonyl]-N-methyl-L-valyl-N-[(2R,3S,4S)-1-carboxy-2-methoxy-4-methylhexan-3-yl]-N-methyl-L-valinamid

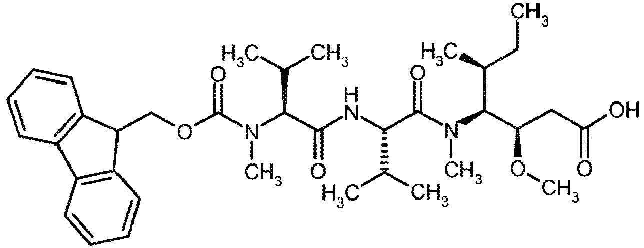

9.1 g des Rohproduktes *N*-[(9*H*-Fluoren-9-ylmethoxy)carbonyl]-*N*-methyl-L-valyl-*N*-[(3*R*,4*S*,5*S*)-1-*tert*.-butoxy-3-methoxy-5-methyl-1-oxoheptan-4-yl]-*N*-methyl-L-valinamid (Intermediat 3) wurden in 56.6 ml Dichlormethan aufgenommen, mit 56.6 ml Trifluoressigsäure versetzt und 2h bei RT gerührt. Anschließend wurde das Reaktionsgemisch im Vakuum eingeengt und der verbliebene Rückstand durch Flash-Chromatographie gereinigt, wobei als Eluent Dichlormethan, Dichlormethan/Ethylacetat 3:1 und Dichlormethan/Ethylacetat/Methanol 15:5:0.5 verwendet wurden. Nach Vereinigung der entsprechenden Fraktionen und Einengen wurden 5.8 g (86% d. Th.) der Titelverbindung als farbloser Schaum erhalten.
HPLC (Methode 5): Rₜ = 2.2 min;
LC-MS (Methode 1): Rₜ = 1.3 min; MS (ESIpos): m/z = 638 (M+H)⁺.

### Intermediat 5

### tert,-Butyl-[(2S)-1-(1,2-oxazinan-2-yl)-1-oxo-3-phenylpropan-2-yl]carbamat

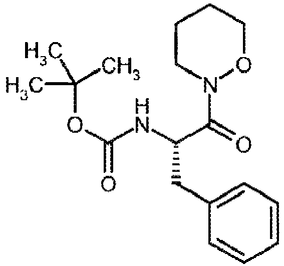

500 mg (1.9 mmol) *N*-(*tert*.-Butoxycarbonyl)-L-phenylalanin wurden in 10 ml DMF gelöst und nacheinander mit 466 mg (3.8 mmol) 1,2-Oxazinan-Hydrochlorid (Ausgangsverbindung 5), 433 mg (2.3 mmol) 1-(3-Dimethylaminopropyl)-3-ethylcarbodiimid-Hydrochlorid, 382 mg (2.8 mmol) 1-Hydroxy-1*H*-benzotriazol-Hydrat sowie 731 mg (5.7 mmol) *N,N*-Diisopropylethylamin versetzt. Die Mischung wurde über Nacht bei RT gerührt. Das Reaktionsgemisch wurde danach in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Magnesiumsulfat getrocknet, filtriert und eingeengt. Man erhielt 620 mg (98% d. Th.) der Titelverbindung.
HPLC (Methode 5): Rₜ = 1.8 min;
LC-MS (Methode 2): Rₜ = 1.62 min; MS (ESIpos): m/z = 235 (M-C₄H₈-CO₂+H)⁺.

### Intermediat 6

### (2S)-2-Amino-1-(1,2-oxazinan-2-yl)-3-phenylpropan-1-on-Trifluoracetat

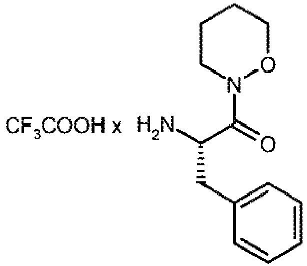

620 mg (1.85 mmol) *tert*.-Butyl-[(2*S*)-1-(1,2-oxazinan-2-yl)-1-oxo-3-phenylpropan-2-yl]carbamat (Intermediat 5) wurden in 5 ml Dichlormethan aufgenommen, mit 10 ml Trifluoressigsäure versetzt und 30 min bei RT gerührt. Anschließend wurde das Reaktionsgemisch im Vakuum eingeengt und der verbliebene Rückstand aus Wasser/Acetonitril lyophilisiert. Auf diese Weise wurden 779 mg (91% d.Th.) der Titelverbindung als farbloser Schaum erhalten.
HPLC (Methode 5): Rₜ = 0.45 min;
LC-MS (Methode 3): Rₜ = 1.09 min; MS (ESIpos): m/z = 235 (M+H)⁺.

### Intermediat 7

### (2R,3R)-3-Methoxy-2-methyl-N-[(2S)-1-(1,2-oxazinan-2-yl)-1-oxo-3-phenylpropan-2-yl]-3-[(2S)-pyrrolidin-2-yl]propanamid-Trifluoracetat

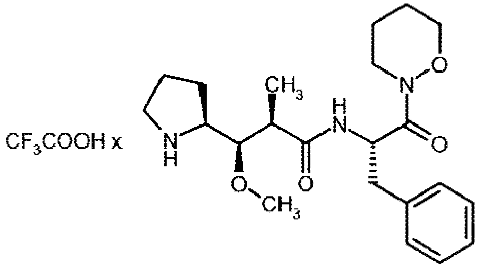

360 mg (1.25 mmol) (2*R*,3*R*)-3-[(2*S*)-1-(*tert*.-Butoxycarbonyl)pyrrolidin-2-yl]-3-methoxy-2-methylpropansäure (Ausgangsverbindung 1) wurden in 10 ml DMF aufgenommen und nacheinander mit 579.2 mg (1.25 mmol) (2*S*)-2-Amino-1-(1,2-oxazinan-2-yl)-3-phenylpropan-1-on-Trifluoracetat (Intermediat 6), 714.5 mg (1.88 mmol) *O*-(7-Azabenzotriazol-1-yl)-*N,N,N',N'-*tetramethyluronium-hexafluorophosphat (HATU) sowie 655 µl *N,N*-Diisopropylethylamin versetzt. Die Mischung wurde 16 h bei RT gerührt. Das Reaktionsgemisch wurde danach eingeengt, der Rückstand in Ethylacetat aufgenommen und zuerst mit 5%-iger wässriger Zitronensäure-Lösung, dann mit 5%-iger wässriger Natriumhydrogencarbonat-Lösung und anschließend mit gesättigter Natriumchloridlösung ausgeschüttelt. Die organische Phase wurde eingeengt und der Rückstand durch Flash-Chromatographie an Kieselgel mit Dichlormethan/Methanol 16:4 als Eluent gereinigt. Die entsprechenden Fraktionen wurden vereinigt und das Lösungsmittel im Vakuum entfernt. Nach Trocknen des Rückstands im Hochvakuum wurden 503.5 mg (74% d. Th.) des Boc-geschützten Intermediats *tert.*-Butyl-(2*S*)-2-[(1*R*,2*R*)-1-methoxy-2-methyl-3-{[(2*S*)-1-(1,2-oxazinan-2-yl)-1-oxo-3-phenylpropan-2-yl]amino}-3-oxopropyl)pyrrolidin-1-carboxylat erhalten.
HPLC (Methode 12): Rₜ = 2.0 min;
LC-MS (Methode 1): Rₜ = 1.12 min; MS (ESIpos): m/z = 504 (M+H)⁺.

503 mg (1 mmol) dieses Intermediats wurden in 20 ml Dichlormethan aufgenommen, mit 10 ml Trifluoressigsäure versetzt und 30 min bei RT gerührt. Anschließend wurde das Reaktionsgemisch im Vakuum eingeengt und mit Dichlormethan rückdestilliert. Der verbliebene Rückstand wurde aus Ethylacetat mit n-Pentan gefällt, das Lösungsmittel abdekantiert. Der so erhaltene Rückstand wurde in Wasser gelöst und mit Ethylacetat ausgeschüttelt und die wässrige Phase anschließend lyophilisiert. Auf diese Weise wurden 462 mg (89% d. Th.) der Titelverbindung als farbloser Schaum erhalten.
HPLC (Methode 12): Rₜ = 1.53 min;
LC-MS (Methode 11): Rₜ = 0.57 min; MS (ESIpos): m/z = 404 (M+H)⁺.

### Intermediat 8

### N-(tert.-Butoxycarbonyl)-N-methyl-L-valyl-N-[(2R,3S,4S)-1-carboxy-2-methoxy-4-methylhexan-3-yl]-N-methyl-L-valinamid

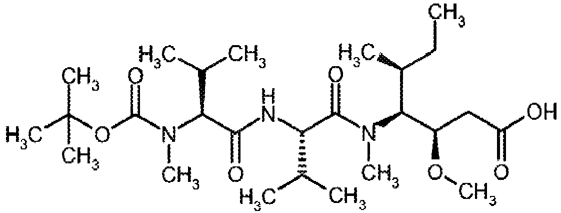

51 mg (0.08 mmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) wurden in 10 ml DMF gelöst und mit 0.5 ml Piperidin versetzt. Nach 10 min Rühren bei RT wurde das Reaktionsgemisch im Vakuum eingeengt und der Rückstand mit Diethylether verrührt. Die unlöslichen Bestandteile wurden abfiltriert und mehrfach mit Diethylether gewaschen. Dann wurde der Filter-Rückstand in 5 ml Dioxan/Wasser aufgenommen und die Lösung mit 1 N Natronlauge auf pH 11 eingestellt. Unter Ultraschallbehandlung wurden in mehreren Portionen insgesamt 349 mg (1.6 mmol) Di-*tert*.-butyldicarbonat zugegeben, wobei der pH-Wert der Lösung bei 11 gehalten wurde. Nach Beendigung der Reaktion wurde das Dioxan abgedampft und die wässrige Lösung mit Zitronensäure auf einen pH-Wert von 2-3 eingestellt. Man extrahierte zweimal mit je 50 ml Ethylacetat. Die organischen Phasen wurden vereinigt, über Magnesiumsulfat getrocknet und im Vakuum eingeengt. Der Rückstand wurde in Diethylether aufgenommen und das der Titelverbindung mit Pentan ausgefällt. Durch Dekantieren wurde das Lösungsmittel abgetrennt. Der Rückstand wurde noch mehrmals mit Pentan digeriert und schließlich im Hochvakuum getrocknet. Es wurden so 40 mg (97% d. Th.) der Titelverbindung erhalten.
HPLC (Methode 6): Rₜ = 2.2 min;
LC-MS (Methode 2): Rₜ = 1.32 min; MS (ESIpos): m/z = 516 (M+H)⁺.

### Intermediat 9

### tert.-Butyl-(2S)-2-[1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-carboxylat

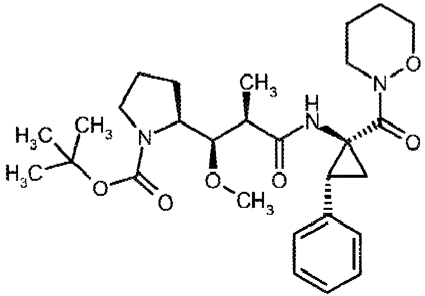

Die Titelverbindung wurde in Analogie zur Synthese der Intermediate 5, 6 und 7 über drei Stufen durch Kupplung von kommerziell erhältlicher (1*S*,2*R*)-1-[(*tert*.-Butoxycarbonyl)amino]-2-phenylcyclopropancarbonsäure mit 1,2-Oxazinan-Hydrochlorid (Ausgangsverbindung 5), anschließende Entschützung mit Trifluoressigsäure und Kupplung mit Ausgangsverbindung 1 hergestellt. Das Endprodukt wurde durch präparative HPLC gereinigt.
HPLC (Methode 5): Rₜ = 2.12 min;
LC-MS (Methode 2): Rₜ = 1.25 min; MS (ESIpos): m/z = 516 (M+H)⁺.

### Intermediat 10

### N-[(9H-Fluoren-9-ylmethoxy)carbonyl]-N-methyl-L-valyl-N-[(3R,4S,5S)-1-{(2S)-2-[(1R,2R)-2-carboxy-1-methoxypropyl]pyrrolidin-1-yl}-3-methoxy-5-methyl-1-oxoheptan-4-yl]-N-methyl-L-valinamid

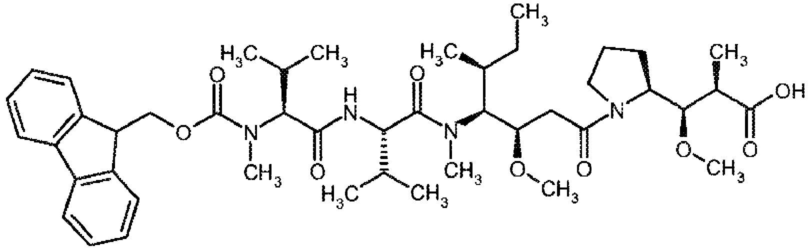

315 mg (0.494 mmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) wurden in 12 ml DMF gelöst, mit 104 mg (0.543 mmol) 1-(3-Dimethylaminopropyl)-3-ethylcarbodiimid-Hydrochlorid sowie 83 mg (0.543 mmol) 1-Hydroxy-1*H*-benzotriazol-Hydrat versetzt und 90 min bei RT gerührt. Anschließend wurden 112 µl *N,N*-Diisopropylethylamin sowie 149 mg (0.494 mmol) (2*R*,3*R*)-3-Methoxy-2-methyl-3-[(2*S*)-pyrrolidin-2-yl]propansäure-Trifluoracetat, welches zuvor aus der Ausgangsverbindung 1 durch Abspaltung der Boc-Schutzgruppe mittels Trifluoressigsäure hergestellt worden war, hinzugegeben. Die Mischung wurde 2 h bei RT gerührt und anschließend im Hochvakuum eingeengt. Der verbliebene Rückstand wurde durch zweimalige präparative IIPLC aufgereinigt. Es wurden 140 mg (35% d. Th.) der Titelverbindung in Form eines farblosen Schaumes erhalten.
HPLC (Methode 5): Rₜ = 2.40 min:
LC-MS (Methode 1): Rₜ = 1.38 min; MS (ESIpos): m/z = 807 (M+H)⁺.

### Intermediat 11

### N-[(Benzyloxy)carbonyl]-N-methyl-L-threonyl-N-[(2R,3S,4S)-1-carboxy-2-methoxy-4-methyl-hexan-3-yl]-N-methyl-L-valinamid

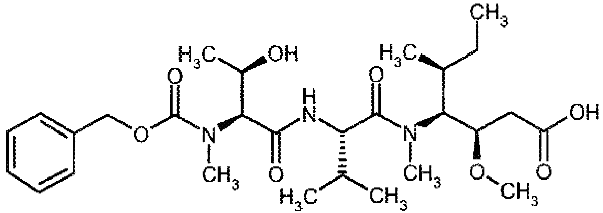

Zunächst wurde *N*-[(Benzyloxy)carbonyl]-*N*-methyl-L-threonin aus 237 mg (0.887 mmol) seines Dicyclohexylamin-Salzes durch Aufnehmen in Ethylacetat und Ausschütteln mit 5%-iger wässriger Schwefelsäure freigesetzt. Die organische Phase wurde über Magnesiumsulfat getrocknet, filtriert und eingeengt. Der Rückstand wurde in 16 ml DMF aufgenommen und nacheinander mit 365 mg (1 mmol) *tert*.-Butyl-(3*R*,4*S*,5*S*)-3-methoxy-5-methyl-4-[methyl(L-valyl)amino]heptanoat (Intermediat 2), 185 mg (0.967 mmol) 1-(3-Dimethylaminopropyl)-3-ethylcarbodiimid-Hydrochlorid sowie 148 mg (0.967 mmol) 1-Hydroxy-1*H*-benzotriazol-Hydrat versetzt. Die Mischung wurde 2 h bei RT gerührt. Das Reaktionsgemisch wurde danach in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Magnesiumsulfat getrocknet, filtriert und eingeengt. Der Rückstand wurde durch präparative IIPLC aufgereinigt. Es wurden so 283 mg (53% d. Th.) des *tert,-*Butylester-Intermediats *N*-[(Benzyloxy)carbonyl]-*N-*methyl-L-threonyl-*N*-[(3*R*,4*S*,5*S*)-1-*tert*.-butoxy-3-methoxy-5-methyl-1-oxoheptan-4-yl]-*N*-methyl-L-valinamid erhalten.
HPLC (Methode 5): Rₜ = 2.17 min.

283 mg (0.466 mmol) dieses Intermediats wurden in 5 ml Dichlormethan aufgenommen, mit 5 ml wasserfreier Trifluoressigsäure versetzt und 2 h bei RT gerührt. Anschließend wurde das Reaktionsgemisch im Hochvakuum eingeengt und der verbliebene Rückstand mittels präparativer HPLC gereinigt. Man erhielt 156 mg (61% d. Th.) der Titelverbindung als farblosen Schaum.
HPLC (Methode 5): Rₜ = 1.50 min;
LC-MS (Methode 2): Rₜ = 1.09 min; MS (ESIpos): m/z = 552 (M+H)⁺.

### Intermediat 12

### Benzyl-N-{(2R,3R)-3-methoxy-2-methyl-3-[(2S)-pyrrolidin-2-yl]propanoyl}-L-phenylalaninat-Trifluoracetat

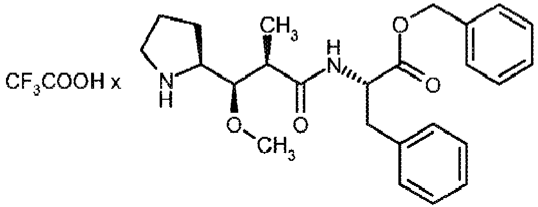

Im ersten Schritt wurde die Ausgangsverbindung 1 aus 600 mg (1.28 mmol) des entsprechenden Dicyclohexylammoniumsalz freigesetzt durch Auflösen des Salzes in 100 ml Ethylacetat und Ausschütteln zunächst mit 50 ml 0.5%-iger Schwefelsäure und dann mit gesättigter Natriumchloridlösung. Daraufhin wurde die organische Phase über Magnesiumsulfat getrocknet, filtriert, eingeengt und sofort mit Benzyl-L-phenylalaninat in Analogie zur Synthese von **Intermediat** 7 umgesetzt und anschließend entschützt.
Ausbeute: 650 mg (94% über 2 Stufen)
HPLC (Methode 6): Rₜ = 1.76 min;
LC-MS (Methode 2): Rₜ = 1.68 min; MS (ESIpos): m/z = 425 (M+H)⁺.

### Intermediat 13

### Benzyl-(βS)-N-{(2R,3R)-3-methoxy-2-methyl-3-[(2S)-pyrrolidin-2-yl]propanoyl}-β-methyl-L-phenylalaninat-Trifluoracetat

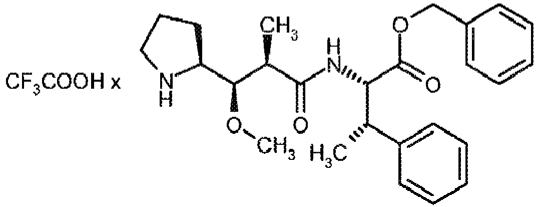

Zunächst wurde (2*R*,3*R*)-3-[(2*S*)-1-(*tert*.-Butoxycarbonyl)pyrrolidin-2-yl]-3-methoxy-2-methylpropansäure aus 351 mg (0.75 mmol) des Dicyclohexylamin-Salzes (Ausgangsverbindung 1) durch Aufnehmen in Ethylacetat und Ausschütteln mit wässriger 5%iger Kaliumhydrogensulfat-Lösung freigesetzt. Die organische Phase wurde über Magnesiumsulfat getrocknet, filtriert und eingeengt. Der Rückstand wurde in 10 ml DMF aufgenommen und nacheinander mit 373 mg (0.75 mmol) Benzyl-(β*S*)-β-methyl-L-phenylalaninat-Trifluoracetat [hergestellt aus kommerziell erhältlichem (β*S*)-*N*-(*tert*.-Butoxycarbonyl)-β-methyl-L-phenylalanin durch EDC/DMAP-vermitielte Veresterung mit Benzylalkohol und nachfolgende Abspaltung der Boc-Schutzgruppe mit Trifluoressigsäure], 428 mg (1.125 mmol) *O*-(7-Azabenzotriazol-1-yl)-*N,N,N*',*N*'-tetramethyluroniumhexafluorophosphat (HATU) sowie 392 µl *N*,*N*-Diisopropylethylamin versetzt. Die Mischung wurde 20 h bei RT gerührt. Das Reaktionsgemisch wurde dann auf eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen und anschließend eingeengt. Der Rückstand wurde mittels präparativer HPLC gereinigt. Man erhielt so 230 mg (57% d. Th.) des Boc-geschützten Intermediats Benzyl-(β*S*)-*N*-{(2*R*,3*R*)-3-[(2*S*)-1-(*tert.*-butoxycarbonyl)pyrrolidin-2-yl]-3-methoxy-2-methylpropanoyl}-β-methyl-L-phenylalaninat.
HPLC (Methode 6): Rₜ = 2.3 min;
LC-MS (Methode 1): Rₜ = 1.36 min; MS (ESIpos): m/z = 539 (M+H)⁺.

230 mg (0.42 mmol) dieses Intermediats wurden in 5 ml Dichlormethan aufgenommen, mit 5 ml Trifluoressigsäure versetzt und 30 min bei RT gerührt. Anschließend wurde das Reaktionsgemisch im Vakuum eingeengt. Der verbliebene Rückstand wurde das Reaktionsgemisch im Vakuum weiter getrocknet und dann aus Acetonitril/Wasser lyophilisiert. Auf diese Weise wurden 230 mg (quant.) der Titelverbindung erhalten.
HPLC (Methode 6): Rₜ = 1.6 min.

### Intermediat 14

### N-Methyl-L-valyl-N-[(3R,4S,5S)-3-methoxy-1-{(2S)-2-[(1R,2R)-1-methoxy-2-methyl-3-{(2S)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid-Trifluoracetat

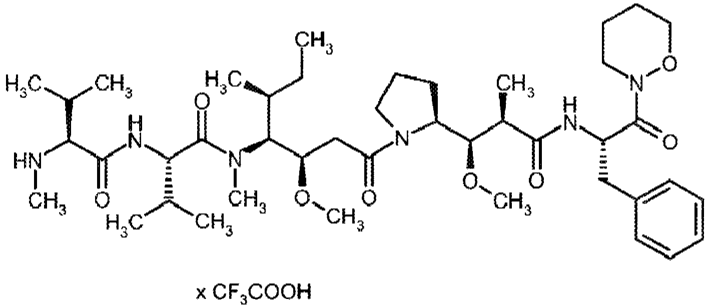

143 mg (0.223 mmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intemiediat 4) wurden in 15 ml DMF aufgenommen und nacheinander mit 141 mg (0.22 mmol) (2*R*,3*R*)-3-Methoxy-2-methyl-*N-*[(2*S*)-1-(1,2-oxazinan-2-yl)-1-oxo-3-phenylpropan-2-yl]-3-[(2*S*)-pyrrolidin-2-yl]propanamid-Trifluoracetat (Intermediat 7), 102 mg (0.27 mmol) *O*-(7-Azabenzotriazol-1-yl)-*N,N,N*',*N'*-tetramethyluronium-hexafluorophosphat (HATU) sowie 128 µl (0.74 mmol) *N,N*-Diisopropylethylamin versetzt. Die Mischung wurde 3 h bei RT gerührt. Das Reaktionsgemisch wurde danach in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Magnesiumsulfat getrocknet, filtriert und eingeengt. Man erhielt so 275 mg (quant.) des Fmoc-geschützten intermediats *N*-[(9*H-*Fluoren-9-ylmethoxy)carbonyl]-*N*-methyl-L-valyl-*N*-[(3*R*,4*S*,5*S*)-3-methoxy-1-{(2*S*)-2-[(1*R,*2*R*)-1-methoxy-2-methyl-3-{[(2*S*)-1-(1,2-oxazinan-2-yl)-1-oxo-3-phenylpropan-2-yl]amino}-3-oxopropyl]pyrrolidin-1-yl}-5-methyl-1-oxoheptan-4-yl]-*N*-methyl-L-valinamid.

### HPLC (Methode 5): Rₜ = 2.73 min;

LC-MS (Methode 4): Rₜ = 3.19 min; MS (ESIpos): m/z = 1023 (M+H)⁺.
46 mg (0.045 mmol) dieses Intermediats wurden in 4 ml DMF gelöst. Nach Zugabe von 1 ml Piperidin wurde das Reaktionsgemisch 1 h bei RT gerührt. Anschließend wurde das Reaktionsgemisch im Vakuum eingeengt und der Rückstand mittels präparativer HPLC gereinigt (Eluent: Acetonitril + 0.01% TFA / Wasser + 0.01% TFA). Es wurden 22 mg (54% d. Th.) der Titelverbindung als farbloser Schaum erhalten.
HPLC (Methode 5): Rₜ = 1.68 min;
LC-MS (Methode 2): Rₜ = 1.03 min; MS (ESIpos): m/z = 801 (M+H)⁺
¹H-NMR (600 MHz, DMSO-d₆): δ = 8.8 (m, 2H), 8.7 (m, 1H), 8.42 und 8.15 (2d, 1H), 7.3-7.1 (m, 5H), 5.12 und 4.95 (2m, 1H), 4.70 und 4.62 (2m, 1H), 4.62 und 4.50 (2t, 1H), 4.1-3.9 (m, 3H), 3.85 (m, 1H), 3.75-3.6 (m, 2H), 3.23, 3.18, 3.17, 3.14, 3.02 und 2.96 (6s, 9H), 3.1-2.9 und 2.75 (2m, 2H), 2.46 (m, 3H), 2.4-2.1 (m, 2H), 2.05 (br. m, 2H), 1.85-1.55 (br. m, 611), 1.5-1.2 (br. m, 3H), 1.1-0.8 (m, 18H), 0.75 (t, 3H) [weitere Signale unter H₂O-Peak verborgen].

### Intermediat 15

### N-Methyl-L-valyl-N-[(3R,4S,5S)-3-methoxy-1-{(2S)-2-[(1R,2R)-1-methoxy-2-methyl-3-{[(2S,3S)-1-(1,2-oxazinan-2-yl)-1-oxo-3-phenylbutan-2-yl]amino}-3-oxopropyl]pyrrolidin-1-yl}-5-methyl-1-oxoheptan-4-yl]-N-methyl-L-valinamid-Trifluoracetat

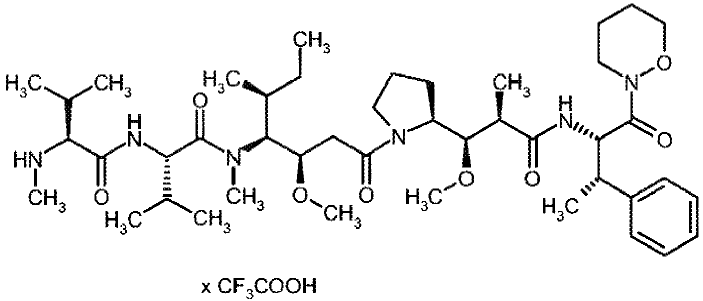

126 mg (0.198 mmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (intermediat 4) wurden in 10 ml DMF aufgenommen und nacheinander mit 105 mg (0.198 mmol) (2*R*,3*R*)-3-Methoxy-2-methyl-*N-*[(2*S*,3*S*)-1-(1,2-oxazinan-2-yl)-1-oxo-3-phenylbutan-2-yl]-3-[(2*S*)-pyrrolidin-2-yl]propanamid-Trifluoracetat (Intermediat 17), 41.6 mg (0.217 mmol) 1-(3-Dimethylaminopropyl)-3-ethylcarbodiimid-Hydrochlorid, 33 mg (0.217 mmol) 1-Hydroxy-1*H*-benzotriazol-Hydrat sowie 79 µl (0.454 mmol) *N,N*-Diisopropylethylamin versetzt. Die Mischung wurde über Nacht bei RT gerührt. Das Reaktionsgemisch wurde danach in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Magnesiumsulfat getrocknet, filtriert und eingeengt. Es wurden so 220 mg (quant.) des Fmoc-geschützten Intermediats *N*-[(9*H*-Fluoren-9-ylmethoxy)carbonyl]-*N*-methyl-L-valyl-*N-*[(3*R*,4*S*,5*S*)-3-methoxy-1-{(2*S*)-2-[(1*R,*2*R*)-1-methoxy-2-methyl-3-{[(2*S*,3*S*)-1-(1,2-oxazinan-2-yl)-1-oxo-3-phenylbutan-2-yl]amino}-3-oxopropyl]pyrrolidin-1-yl}-5-methyl-1-oxoheptan-4-yl]-*N*-methyl-L-valinamid erhalten.
HPLC (Methode 5): Rₜ = 2.77 min:
LC-MS (Methode 1): Rₜ = 1.5 min; MS (ESIpos): m/z = 1037 (M+H)⁺.

220 mg (0.212 mmol) dieses Intermediats wurden in 5 ml DMF gelöst. Nach Zugabe von 1 ml Piperidin wurde das Reaktionsgemisch 1 h bei RT gerührt. Anschließend wurde das Reaktionsgemisch im Vakuum eingeengt und der Rückstand mittels präparativer HPLC gereinigt (Eluent: Acetonitril + 0.01% TFA / Wasser + 0.01% TFA). Es wurden 91 mg (46% d. Th.) der Titelverbindung als farbloser Schaum erhalten.
HPLC (Methode 5): Rₜ = 1.71 min:
LC-MS (Methode 1): Rₜ = 0.9 min; MS (ESIpos): m/z = 815 (M+H)⁺

¹H-NMR (600 MHz, DMSO-d₆): δ = 8.87 und 8.80 (2d, 2H), 8.75 (m, 1H), 8.40 und 7.98 (2d, 1H), 7.3-7.1 (m, 5H), 5.45 und 5.2 (2t, 1H), 4.78 und 4.62 (2m, 1H), 4.73 und 4.58 (2t, 1H), 4.2-4.0 (m, 3H), 3.7-3.6 (m, 1H), 3.35, 3.20, 3.18, 3.14, 3.12 und 3.00 (6s, 9H), 3.1 und 2.95 (2m, 2H), 2.46 (m, 3H), 2.4-2.0 (m, 4H), 1.9-1.6 (m, 4H), 1.6-1.2 (m, 5H), 1.1-0.75 (m, 21H), 0.80 (t, 3H) [weitere Signale unter H₂O-Peak verborgen].

### Intermediat 16

### N-Methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid-Trifluoracetat

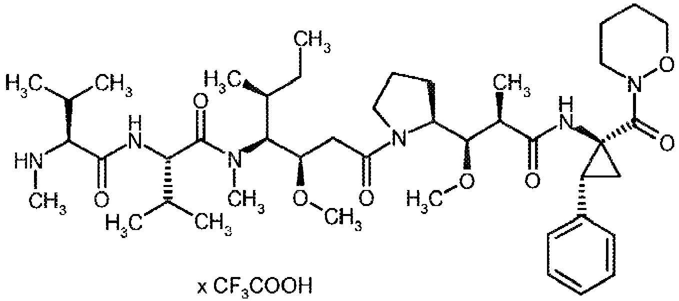

617 mg (1.2 mmol) *tert*.-Butyl-(2*S*)-2-[(1*R*,2*R*)-1-methoxy-2-methyl-3-{[(1*S*,2*R*)-1-(1,2-oxazinan-2-ylcarbonyl)-2-phenylcyclopropyl]amino}-3-oxopropyl]pyrrolidin-1-carboxylat (Intermediat 24) wurden in 44 ml Dichlormethan aufgenommen, mit 4.4 ml Trifluoressigsäure versetzt und 30 min bei RT gerührt. Anschließend wurde das Reaktionsgemisch im Vakuum eingeengt und der verbliebene Rückstand aus Dioxan/Wasser lyophilisiert. Es wurden 702 mg (quant.) der entschützten Verbindung (2*R*,3*R*)-3-Methoxy-2-methyl-*N*-[(1*S*,2*R*)-1-(1,2-oxazinan-2-ylcarbonyl)-2-phenylcyclopropyl]-3-[(2*S*)-pyrrolidin-2-yl]propanamid-Trifluoracetat als Rohprodukt erhalten, das ohne weitere Reinigung in der folgenden Stufe eingesetzt wurde.

470 mg (0.74 mmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) wurden in 57 ml DMF aufgenommen und nacheinander mit 390 mg (ca. 0.74 mmol) des oben erhaltenen (2*R*,3*R*)-3-Methoxy-2-methyl-*N*-[(1*S*,2*R*)-1-(1,2-oxazinan-2-ylcarbonyl)-2-phenylcyclopropyl]-3-[(2*S*)-pyrrolidin-2-yl]propanamid-Trifluoracetates, 336 mg (0.88 mmol) *O*-(7-Azabenzotriazol-1-yl)-*N*,*N*,*N*',*N*'-tetramethyluronium-hexafluorophosphat (HATU) sowie 423 µl (2.4 mmol) *N,N*-Diisopropylethylamin versetzt. Die Mischung wurde 2 h bei RT gerührt. Das Reaktionsgemisch wurde dann in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Natriumsulfat getrocknet, filtriert und eingeengt. Der Rückstand wurde durch präparative HPLC gereinigt. Man erhielt so 453 mg (59% d. Th.) des Fmoc-geschützten Intermediats *N*-[(9*H*-Fluoren-9-yl-methoxy)carbonyl]-*N*-methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl]amino}-3-oxopropyl]pyrrolidin-1-yl}-5-methyl-1-oxoheptan-4-yl]-*N*-methyl-L-valinamid.
HPLC (Methode 5): Rₜ = 2.58 min;
LC-MS (Methode 1): Rₜ = 3.10 min; MS (ESIpos): m/z = 1035 (M+H)⁺.

453 mg (0.438 mmol) dieses Intermediats wurden in 24 ml DMF gelöst. Nach Zugabe von 2.4 ml Piperidin wurde das Reaktionsgemisch 30 min bei RT gerührt. Anschließend wurde das Reaktionsgemisch im Vakuum eingeengt und der Rückstand durch präparative HPLC gereinigt (Eluent: Acetonitril / 0.1% TFA in Wasser). Es wurden 260 mg (64% d. Th.) der Titelverbindung als farbloser Schaum erhalten.
HPLC (Methode 5): Rₜ = 1.64 min;
LC-MS (Methode 1): Rₜ = 0.86 min; MS (ESIpos): m/z = 813 (M+H)⁺
¹H-NMR (400 MHz, DMSO-d₆): δ = 8.8 (m, 2H), 8.65 (m, 2H), 7.3-7.1 (m, 5H), 4.8-4.05 (m, 2H), 4.0 und 3.82 (2m, 2H), 3.8-3.5 (m, 8H), 3.32, 3.29, 3.20, 3.19, 3.12 und 3.00 (6s, 9H), 2.65 (t, 1H), 2.5-2.45 (m, 3H), 2.4-1.3 (m, 15H), 1.15-0.85 (m, 18H), 0.8 und 0.75 (2d, 3H) [weitere Signale unter H₂O-Peak verborgen].

### Intermediat 17

### N-Benzyl-N-methyl-L-phenylalaninamid-Trifluoracetat

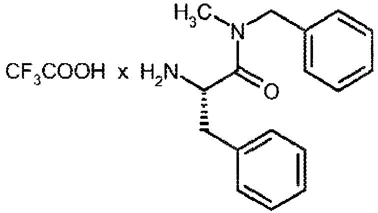

1000 mg (3.77 mmol) *N*-(*tert*.-Butoxycarbonyl)-L-phenylalanin wurden in 10 ml DMF gelöst und mit 457 mg (3.77 mmol) *N*-Methylbenzylamin, 2150 mg (5.65 mmol) O-(7-Azabenzotriazol-1-yl)-*N*,*N*,*N'*,*N'*-tetramethyluronium-hexafluorophosphat und 657 µl *N,N*-Diisopropylethylamin versetzt. Das Reaktionsgemisch wurde 30 min bei RT gerührt und anschließend im Vakuum eingeengt. Der Rückstand wurde in Dichlormethan aufgenommen und dreimal mit Wasser ausgeschüttelt. Die organische Phase wurde über Magnesiumsulfat getrocknet und eingeengt. Der Rückstand wurde durch Flash-Chromatographie an Kieselgel mit Petrolether/Ethylacetat 3:1 als Laufmittel gereinigt. Die Produktfraktionen wurden eingeengt und der Rückstand im Hochvakuum getrocknet. Man erhielt so 1110 mg (75% d. Th.) des Boc-geschützten Intermediats *N*-Benzyl-*N^{α}*-(*tert*.-butoxycarbonyl)-*N*-methyl-L-phenylalaninamid.
HPLC (Methode 6): Rₜ = 2.1 min;
LC-MS (Methode 1): Rₜ = 1.14 min; MS (ESIpos): m/z = 369 (M+H)⁺.

1108 mg (3.007 mmol) dieses Intermediats wurden in 30 ml Dichlormethan aufgenommen, mit 10 ml Trifluoressigsäure versetzt und 30 min bei RT gerührt. Anschließend wurde das Reaktionsgemisch im Vakuum eingeengt, der verbliebene Rückstand mit Dichlormethan verrührt und das Lösungsmittel abdestilliert. Der Rückstand wurde noch zweimal mit Pentan verrührt, das Lösungsmittel jeweils wieder abdekantiert und das der Titelverbindung schließlich im Hochvakuum getrocknet. Es wurden so 1075 mg (93% d. Th.) der Titelverbindung als ein Harz erhalten.
HPLC (Methode 6): Rₜ = 1.6 min;
LC-MS (Methode 1): Rₜ = 0.6 min; MS (ESIpos): m/z = 269 (M+H)⁺.

### Intermediat 18

### N-Benzyl-N^{α}-{(2R,3R)-3-methoxy-2-methyl-3-[(2S)-pyrrolidin-2-yl]propanoyl}-N-methyl-L-phenylalaninamid-Trifluoracetat

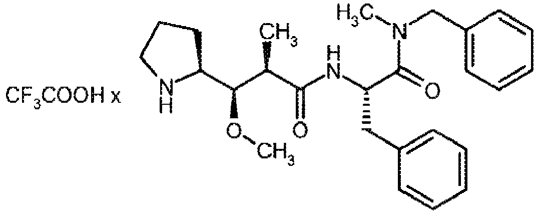

Zunächst wurde (2*R*,3*R*)-3-[(2*S*)-1-(*tert*.-Butoxycarbonyl)pyrrolidin-2-yl]-3-methoxy-2-methylpropansäure (Ausgangsverbindung 1) aus 141 mg (0.491 mmol) ihres Dicyclohexylamin-Salzes durch Aufnehmen in Ethylacetat und Ausschütteln mit 5%-iger wässriger Schwefelsäure freigesetzt. Die organische Phase wurde über Magnesiumsulfat getrocknet, filtriert und eingeengt. Der Rückstand wurde in 10 ml DMF aufgenommen und mit 187.6 mg (0.49 mmol) *N*-Benzyl-*N-*methyl-L-phenyl-alaninamid-Trifluoracetat (Intermediat 9), 190.3 mg (1.47 mmol) *O*-(7-Azabenzotriazol-1-yl)-*N*,*N*,*N*'*,*N'-tetramethyluronium-hexaflurophosphat (HATU) sowie 256 µl *N*,*N*-Diisopropylethyl-amin versetzt. Die Mischung wurde 1 h bei RT gerührt. Das Reaktionsgemisch wurde danach eingeengt, der Rückstand in Ethylacetat aufgenommen und die Lösung nacheinander mit gesättigter Ammoniumchlorid-Lösung, gesättigter Natriumhydrogencarbonat-Lösung und Wasser ausgeschüttelt. Die organische Phase wurde über Magnesiumsulfat getrocknet und eingeengt. Der Rückstand wurde per Flash-Chromatographie an Kieselgel mit Acetonitril/Wasser 30:1 als Eluent gereinigt. Die Produktfraktionen wurden eingeengt und der Rückstand im Hochvakuum getrocknet. Man erhielt so 168 mg (64% d. Th.) des Boc-geschützten Intermediats *tert*.-Butyl-(2*S*)-2-[(1*R*-2*R*)-3-{(2*S*)-1-[benzyl(methyl)amino]-1-oxo-3-phenylpropan-2-yl}amino)-1-methoxy-2-methyl-3-oxopropyl]pyrrolidin-1-carboxy lat.
HPLC (Methode 6): Rₜ = 2.2 min;
LC-MS (Methode 2): Rₜ = 1.22 min; MS (ESIpos): m/z - 538 (M+H)⁺.

168 mg (0.312 mmol) dieses Intermediats wurden in 15 ml Dichlormethan aufgenommen, mit 3 ml Trifluoressigsäure versetzt und 30 min bei RT gerührt. Anschließend wurde das Reaktionsgemisch im Vakuum eingeengt. Der verbliebene Rückstand wurde zunächst mit Dichlormethan, dann mit Diethylether verrührt und das Lösungsmittel jeweils wieder abdestilliert. Nach Trocknen im Hochvakuum wurden 170 mg (99% d. Th.) der Titelverbindung als ein Harz erhalten.
HPLC (Methode 6): Rₜ = 1.7 min;
LC-MS (Methode 1): Rₜ = 0.73 min; MS (ESIpos): m/z = 438 (M+H)⁺.

### Intermediat 19

### Methyl-N-{(2R-3R)-3-methoxy-2-methyl-3-[(2S)-pyrrolidin-2-yl]propanoyl}-L-phenylalaninat-Trifluoracetat

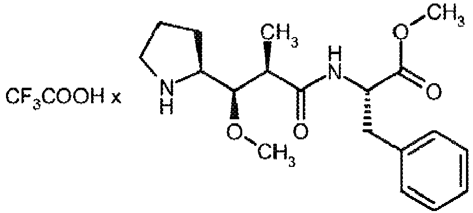

Die Titelverbindung wurde in Analogie zur Synthese von Intermediat 18 ausgehend von (2*R*,3*R*)-3-[(2*S*)-1-(*tert*.-Butoxycarbonyl)pyrrolidin-2-yl]-3-methoxy-2-methylpropansäure (Ausgangsverbindung 1), die aus dem Dicyclohexylamin-Salz freigesetzt wurde, und Methyl-L-phenylalaninat-Hydrochlorid hergestellt.
HPLC (Methode 5): Rₜ = 0.6 min;
LC-MS (Methode 3): Rₜ = 1.17 min; MS (ESIpos): m/z = 349 (M+H)⁺.

### Intermediat 20

### Benzyl-N-{(2R,3R)-3-methoxy-2-methyl-3-[(2S)-pyrrolidin-2-yl]propanoyl}-L-tryptophanat-Trifluoracetat

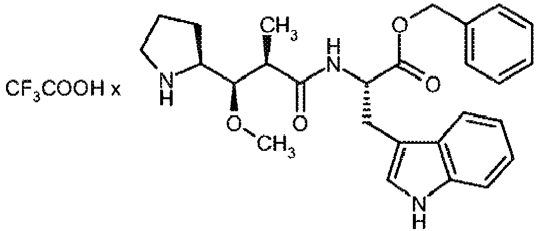

Die Titelverbindung wurde in Analogie zur Synthese von Intermediat 18 ausgehend von (2*R,*3*R*)-3-[(2*S*)-1-(*tert*.-Butoxycarbonyl)pyrrolidin-2-yl]-3-methoxy-2-methylpropansäure (Ausgangsverbindung 1), die aus dem Dicyclohexylamin-Salz freigesetzt wurde, und Benzyl-L-ttyptophanat hergestellt
HPLC (Methode 6): Rₜ = 2.0 min;
LC-MS (Methode 1): Rₜ = 0.8 min; MS (ESIpos): *m*/*z* = 464 (M+H)⁺.

### Intermediat 21

### Benzyl-(1S,2R)-1-({(2R,3R)-3-methoxy-2-methyl-3-[(2S)-pyrrolidin-2-yl]propanoyl}amino)-2-phenylcyclopropancarboxylat-Trifluoracetat

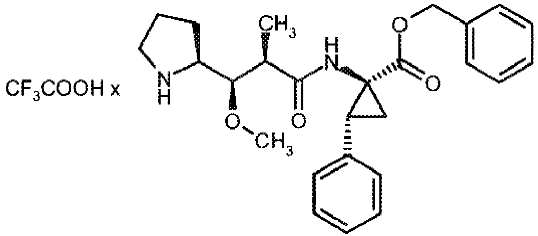

Die Titelverbindung wurde in Analogie zur Synthese von Intermediat 18 ausgehend von (2*R*,3*R*)-3-[(2*S*)-1-(*tert*.-Butoxycarbonyl)pyrrolidin-2-yl]-3-methoxy-2-methylpropansäure (Ausgangsverbindung 1), die aus dem Dicyclohexylamin-Salz freigesetzt wurde, und Benzyl-(1*S*,2*R*)-1-amino-2-phenylcyclopropancarboxylat hergestellt. Benzyl-(1*S*,2*R*)-1-amino-2-phenylcyclopropancarboxylat wurde zuvor nach Standardmethoden durch Veresterung von kommerziell erhältlicher (1*S,*2*R*)-1-[(*tert*.-Butoxycarbonyl)amino]-2-phenylcyclopropancarbonsäure mit Benzylalkohol und anschließende Boc-Abspaltung mit Trifluoressigsäure hergestellt
HPLC (Methode 5): Rₜ = 1.5 min;
LC-MS (Methode 2): Rₜ = 0.93 min; MS (ESIpos): m/z = 437 (M+H)⁺.

### Intermediat 22

### 6-(2,5-Dioxo-2,5-dihydro-1H-pyrrol-1-yl)-N'-methylhexanhydrazid-Trifluoracetat

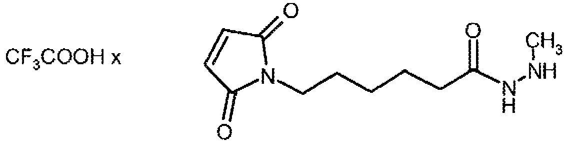

100 mg (473 µmol) 6-(2,5-Dioxo-2,5-dihydro-1*H*-pyrrol-1-yl)hexansäure wurden in 71 µl DMF gelöst und anschließend mit 139 mg (947 µmol) tert.-Butyl-1-methylhydrazincarboxylat, 182 mg (947 µmol) 1-(3-Dimethylaminopropyl)-3-ethylcarbodiimid-Hydrochlorid und 145 mg (947 µmol) 1-Hydroxy-1*H*-benzotriazol-Hydrat versetzt. Die Mischung wurde über Nacht bei RT gerührt und anschließend im Vakuum eingeengt. Der verbliebene Rückstand wurde mittels präparativer HPLC aufgereinigt. Nach Lyophilisation aus Dioxan/Wasser wurden 129 mg (80% d. Th.) des geschützten Intermediats als farbloser Schaum erhalten.

Anschließend wurden die 129 mg (380 µmol) mit 2 ml Trifluoressigsäure in 8 ml Dichlormethan deblockiert. Nach 1 h Rühren bei RT wurde das Reaktionsgemisch im Vakuum eingeengt. Der Rückstand wurde aus Acetonitril/Wasser lyophilisiert und es verblieben 125 mg (83% d.Th.) der Titelverbindung als farbloser Schaum.
LC-MS (Methode 1): Rₜ = 0.38 min; MS (ESIpos): m/z = 240 (M+H)⁺

### Intermediat 23

### N-(2-Aminoethyl)-4-(2,5-dioxo-2,5-dihydro-1H-pyrrol-1-yl)-N-methylbutanamid-Trifluoracetat

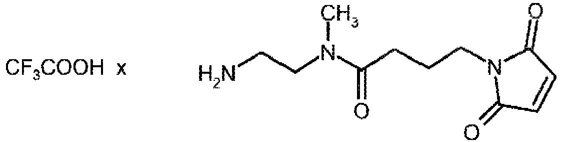

Zunächst wurden 35 mg (164 µmol) tert.-Butyl-[2-(methylamino)ethyl]carbamat-Hydrochlorid-Trifluoracetat, 30 mg (164 µmol) 4-(2,5-Dioxo-2,5-dihydro-1*H*-pyrrol-1-yl)butansäure. 75 mg (197 µmol) O-(7-Azabenzotriazol-1-yl)-*N*,*N*,*N*',*N*'-tetramethyluronium-hexafluorophosphat und 57 µl *N*,*N*-Diisopropylethylamin in 5 ml DMF zusammengegeben und über Nacht bei RT gerührt. Anschließend wurde das Lösungsmittel im Vakuum entfernt und der verbliebene Rückstand mittels präparativer HPLC aufgereinigt. Die entsprechenden Fraktionen wurden eingeengt und durch Lyophilisation aus Dioxan/Wasser wurden 35 mg (63% d.Th.) des geschützten Intermediats erhalten.
HPLC (Methode 12): Rₜ = 1.6 min;
LC-MS (Methode 1): Rₜ = 0.71 min; MS (ESIpos): m/z = 340 (M+H)⁺.

Anschließend wurde die gesamte Menge des geschützten Intermediats mit 1ml Trifluoressigsäre in 5 ml Dichlormethan deblockiert, wobei 28 mg (77% d.Th.) der Titelverbindung erhalten wurden.

LC-MS (Methode 3): Rₜ = 0.75 min; MS (ESIpos): m/z = 240 (M+H)⁺.

### Intermediat 24

### 4-(2,5Dioxo-2,5-dihydro-1H-pyrrol-1-yl)-N-[2-(methylamino)ethyl]butanamid-Trifluoracetat

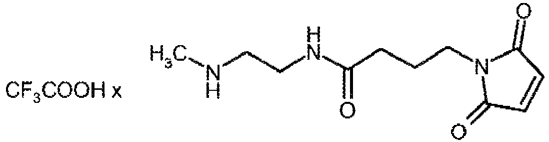

Zunächst wurden 35 mg (164 umol) tert.-Butyl-(2-aminoethyl)methylcarbamathydrochlorid-Trifluoracetat, 30 mg (164 µmol) 4-(2-,5-Dioxo-2,5-dihydro-1*H*-pyrrol-1-yl)butansäure 75 mg (197 µmol) O-(7-Azabenzotriazol-1-yl)-*N*,*N*,*N*',*N'*-tetramethyluronium-hexafluorophosphat und 57 µl *N*,*N*-Diisopropylethylamin in 5 ml DMF zusammengegeben und 30 min bei RT gerührt. Anschließend wurde das Lösungsmittel im Vakuum entfernt und der verbliebene Rückstand mittels präparativer HPLC aufgereinigt. Die entsprechenden Fraktionen wurden eingeengt und durch Lyophilisation aus Dioxan/Wasser wurden 51 mg (91% d.Th.) des geschützten Intermediats erhalten.
HPLC (Methode 12): Rₜ = 1.6 min:
LC-MS (Methode 1): Rₜ = 0.77 min; MS (ESIpos): m/z = 340 (M+H)⁺.

Anschließend wurde die gesamte Menge mit 1 ml Trifluoressigsäre in 5 ml Dichlormethan entschützt, wobei 45 mg (69% d.Th.) der Titelverbindung erhalten wurden.

LC-MS (Methode 1): Rₜ = 0.19 min; MS (ESIpos): m/z = 240 (M+H)⁺.

### Intermediat 25

### Benzyl-(2R-3R)-3-methoxy-2-methyl-3-[(2S)-pyrrolidin-2-yl]propanoat-Trifluoracetat

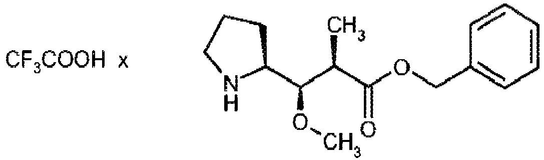

Zunächst wurde (*2*R,3*R*)-3-[(2*S*)-1-(*tert*.-Butoxycarbonyl)pyrrolidin-2-yl]-3-methoxy-2-methylpropansäure aus 1.82 g (3.88 mmol) ihres Dicyclohexylamin-Salzes durch Aufnehmen in 150 ml Ethylacetat und Ausschütteln mit 100 ml 0.5 %-iger Schwefelsäure freigesetzt. Die organische Phase wurde über Magnesiumsulfat getrocknet, filtriert und eingeengt. Der Rückstand wurde in 10 ml Dioxan und 10 ml Wasser aufgenommen und mit 1517 mg (4.66 mmol) Cäsiumcarbonat versetzt, 5 min im Ultraschallbad behandelt und anschließend im Vakuum eingeengt und einmal mit DMF nachdestilliert. Der Rückstand wurde dann in 15 ml DMF aufgenommen und mit 1990 mg (11.64 mmol) Benzylbromid versetzt. Die Mischung wurde 15 min im Ultraschallbad behandelt und anschließend im Vakuum eingeengt. Der Rückstand wurde zwischen Ethylacetat und Wasser verteilt, die organische Phase abgetrennt und mit gesättigter Natriumchloridlösung ausgeschüttelt und anschließend eingeengt. Rer Rückstand wurde dann durch präparative HPLC gereinigt. Man erhielt so 1170 mg (80% d. Th.) des Boc-geschützten Intermediats.

Anschließend wurden diese 1170 mg sofort mit 5 ml Trifluoressigsäure in 15 ml Dichlormethan entschützt. Nach 15 min Rühren bei RT wurde das Reaktionsgemisch im Vakuum eingeengt. Der Rückstand wurde aus Dioxan lyophilisiert. Nach Trocknung im Hochvakuum verblieben 1333 mg (84% d.Th.) der Titelverbindung als gelbes Öl.
HPLC (Methode 6): Rₜ = 1.5 min;
LC-MS (Methode 1): Rₜ = 0.59 min; MS (ESIpos): m/z = 278 (M+H)⁺.

### Intermediat 26

### N-(tert.-Butoxycarbonyl)-N-methyl-L-valyl-N-[(3R,4S,5S)-1-{(2S)-2-[(1R,2R)-2-carboxy-1-methoxypropyl]pyrrolidin-1-yl}-3-methoxy-5-methyl-1-oxoheptan-4-yl]-N-methyl-L-valinamid

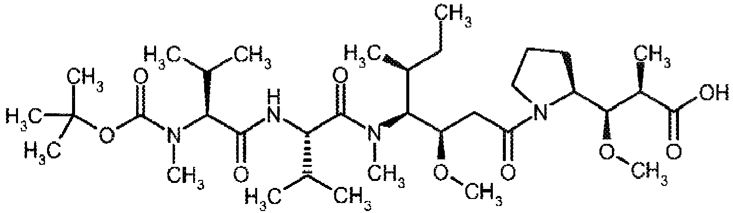

1200 mg (2.33 mmol) N-(*tert*.-Butoxycarbonyl)-*N*-methyl-L-valyl-*N*-[(2*R*,3*S*,4*S*)-1-carboxy-2-methoxy-4-methylhexan-3-yl]-*N*-methyl-L-valinamid (Intermediat 5) wurden mit 910.8 mg (2.33 mmol) Benzyl-(2*R*,3*R*)-3-methoxy-2-methyl-3-[(2*S*)-pyrrolidin-2-yl]propanoat-Trifluoracetat (Intermediat 14), 1327 mg (3.49 mmol) *O*-(7-Azabenzotriazol-1-yl)-*N*,*N*,*N'*,*N*'-tetramethyluronium-hexafluorophosphat und 2027 µl *N*,*N*-Diisopropylethylamin in 50 ml DMF zusammengegeben und 5 min bei RT gerührt. Danach wurde das Lösungsmittel im Vakuum entfernt. Der verbliebene Rückstand wurde in Ethylacetat aufgenommen und nacheinander mit 5%-iger wässriger Zitronensäure-Lösung und gesättigter Natriumhydrogencarbonat-Lösung ausgeschüttelt. Die organische Phase wurde abgetrennt und eingeengt. Der Rückstand wurde mittels präparativer HPLC gereinigt. Die Produktfraktionen wurden vereinigt, eingeengt und der Rückstand im Hochvakuum getrocknet. Es wurden so 1000 mg (55% d. Th.) des Benzylester-Intermediats *N*-*tert*.-Butoxy-carbonyl)-*N*-methyl-L-valyl-*N*-[(3*R*,4*S*,5*S*)-1-{(2*S*)-2-[(1*R*,2*R*)-3-(benzyloxy)-1-methoxy-2-methyl-3-oxopropyl]pyrrolidin-1-yl}-3-methoxy-5-methyl-1-oxoheptan-4-yl]-*N*-methyl-L-valin-amid als ein Harz erhalten.
LC-MS (Methode 1): Rₜ = 1.56 min; MS (ESIpos): m/z = 775 (M+H)⁺.

Die gesamte erhaltene Menge dieses Intermediats wurde in 25 ml einer Mischung aus Methanol und Dichlormethan (20:1) aufgenommen und die Benzylester-Gruppe durch Hydrierung unter Wasserstoff-Normaldruck mit 10% Palladium auf Aktivkohle als Katalysator entfernt. Nach 30 min Rühren bei RT wurde der Katalysator abfiltriert und das Filtrat im Vakuum eingeengt. Man erhielt 803 mg (91% d. Th.) der Titelverbindung als weißen Feststoff.
HPLC (Methode 6): Rₜ = 2.1 min;
LC-MS (Methode 1): Rₜ = 1.24 min; MS (ESIpos): m/z = 685 (M+H)⁺.

### Intermediat 27

### (1S,2R)-1-Amino-2-phenyl-N-propylcyclopropancarboxamid-Trifluoracetat

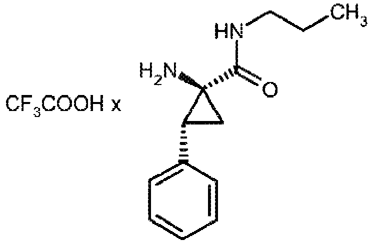

Die Titelverbindung wurde durch Kupplung von kommerziell erhältlicher (1*S*,2*R*)-1-[(*tert*.-Butoxycarbonyl)amino-2-phenylcyclopropancarbonsäure mit n-Propylamin in Gegenwart von *O*-(7-Azabenzotriazol-1-yl)-*N*,*N*,*N*',*N*'-tetramethyluronium-hexafluorophosphat (HATU) und nachfolgende Boc-Abspaltung mit Trifluoressigsäure hergestellt (Ausbeute 85% d. Th. über beide Stuten).
HPLC (Methode 6): Rₜ = 1.2 min;
LC-MS (Methode 1): Rₜ = 0.52 min; MS (ESIpos): m/z = 219 (M+H)⁺.

### Intermediat 28

### Ethyl-(1S,2R)-1-amino-2-phenylcyclopropancarboxylat-Trifluoracetat

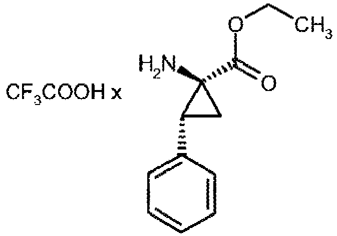

Die Titelverbindung wurde nach Standardmethoden durch Veresterung von kommerziell erhältlicher (1*S*,2*R*)-1-[(*tert*.-Butoxycarbonyl)amino]-2-phenylcyclopropancarbonsäure mit Ethanol und anschließende Boc-Abspaltung mit Trifluoressigsäure hergestellt.
LC-MS (Methode 1): Rₜ = 0.50 min; MS (ESIpos): m/z = 206 (M+H)⁺.

### Intermediat 29

### 4-(2,5-Dioxo-2,5-dihydro-1H-pyrrol-1-yl)-2,2-dimethylbutansäure

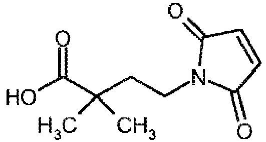

Zu einer Lösung von 1.39 g (8.95 mmol) N-Methoxycarbonylmaleinimid in 44 ml gesättigter Natriumhydrogencarbonat-Lösung wurden bei 0°C 1.5 g (8.95 mmol) 4-Amino-2,2-dimethylbuttersäure gegeben und 40 min nachgerührt. Anschließend wurde das Kühlbad entfernt und das Reaktionsgemisch 1 h weitergerührt. Unter Eiskühlung wurde das Reaktionsgemisch dann durch Zugabe von Schwefelsäure auf pH 3 eingestellt und mit Ethylacetat extrahiert. Die vereinigten organischen Phasen wurden über Magnesiumsulfat getrocknet und eingeengt. Es wurden 1.17 g (79%ig, 49% d.Th.) der Titelverbindung erhalten.
LC-MS (Methode 1): Rₜ = 0.64 min; m/z = 212 (M+H)'.

### Intermediat 30

### tert.-Butyl-2-[4-(2,5-dioxo-2,5-dihydro-1H-pyrrol-1-yl)-2,2-dimethylbutanoyl]hydrazincarboxylat

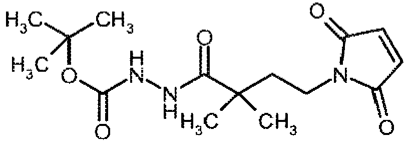

Zu einer Lösung von 50 mg (237 µmol) 4-(2,5-Dioxo-2,5-dihydro-1*H*-pyrrol-1-yl)-2,2-dimethylbutansäure in 2 ml THF wurden bei 0°C zunächst 26 µl (237 µmol) 4-Methylmorpholin und anschließend 31 µl (237 µmol) Isobutylchloroformiat gegeben. Nach Entfernen des Kühlbades und 15 min. Nachrühren bei RT wurden 31.3 mg (237 µmol) *tert*.-Butyloxycarbonylhydrazid zugegeben. Das Reaktionsgemisch wurde über Nacht gerührt und anschließend eingeengt. Der Rückstand wurde per präparativer HPLC gereinigt. Es wurden 50.8 mg (66% d.Th.) der Titelverbindung erhalten.

LC-MS (Methode 1): Rₜ = 0.71 min; m/z = 324 (M-H)⁻.

### Intermediat 31

### 4-(2,5-Dioxo-2,5-dihydro-1H-pyrrol-1-yl)-2,2-dimethylbutanhydrazid-Trifluoracetat

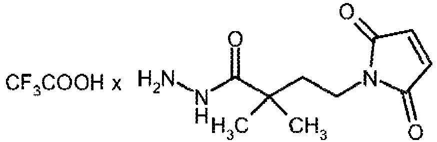

50 mg (154 mmol) *tert.-*Butyl-2-[4-(2,5-dioxo-2,5-dihydro-1*H*-pyrrol-1-yl)-2-2-dimethyl-butanoyl]hydrazincarboxylat wurden in 2 ml Dichiormethan gelöst und mit 0.4 ml Trifluoressigsäure versetzt. Das Reaktionsgemisch wurde 30 min bei RT gerührt und anschließend eingeengt. Es wurden 55.2 mg (93%ige Reinheit, 99% d.Th.) der Titetverbindung erhalten.
LC-MS (Methode 1): Rₜ - 0.36 min; m/z = 226 (M+H)⁺.

### Intermediat 32

### Adamantan-1-ylmethyl-N-(tert.-butoxycarbonyl)-L-phenylalaninat

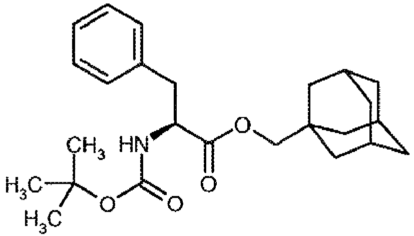

Zu einer Lösung von 500 mg (1.89 mmol) N-Boc-L-Phenylalanin in 25 ml Dichlormethan wurden bei RT 1192 mg (6.2 mmol) EDC, 578 µl (4.1 mmol) Triethylamin, 345 mg (2.8 mmol) DMAP und 345 mg (2.1 mmol) 1-Adamantylmethanol gegeben. Das Reaktionsgemisch wurde über Nacht gerührt, dann mit 50 ml Dichlormethan verdünnt und nacheinander mit 10%-iger wässriger Zitronensäure-Lösung, Wasser und gesättigter Natriumchlorid-Lösung gewaschen. Die organische Phase wurde über Magnesiumsulfat getrocknet, anschließend eingeengt und der Rückstand durch präparative HPLC gereinigt. Es wurden 769 mg (90% d. Th.) der Titelverbindung erhalten.
LC-MS (Methode 2): Rₜ = 1.84 min; m/z = 414 (M+H)⁺.

### Intermediat 33

### Adamantan-1-ylmethyl-L-phenylalaninat-Hydrochlorid

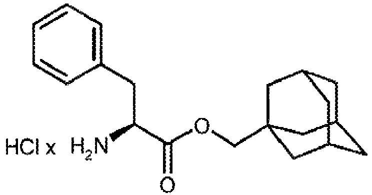

769 mg (1.86 mmol) Adamantan-1-ylmethyl-N-(*tert*.-butoxycarbonyl)-*L*-phenylalaninat (Intermediat 13) wurden in 25 ml einer 4 N Lösung von Chlorwasserstoff in Dioxan gelöst und 1 h bei RT gerührt. Anschließend wurde das Reaktionsgemisch eingeengt und der Rückstand im Vakuum getrocknet. Es wurden 619 mg (95% d. Th.) der Titelverbindung erhalten.
LC-MS (Methode 1): Rₜ = 0.82 min; m/z = 314 (M+H)⁺.

### Intermediat 34

*N-*(*tert.-*Butoxycarbonyl*)-N-*methyl*-L-*valyl*-*N*-*[(3*R,*4*S,*5*S*)-1-{(2*S*)-2-[(1*R,*2*R*)-3-{[(2*,S)-*1-(adamantan-1-ylmethoxy)-1-oxo-3-phenylpropan-2-yl]amino}-1-methoxy-2-methyl-3-oxopropyl]-pyrrolidin-1-yl}-3-methoxy-5-methyl-1-oxoheptan-4-yl]-N*-*methyl-*L-v*alinamid

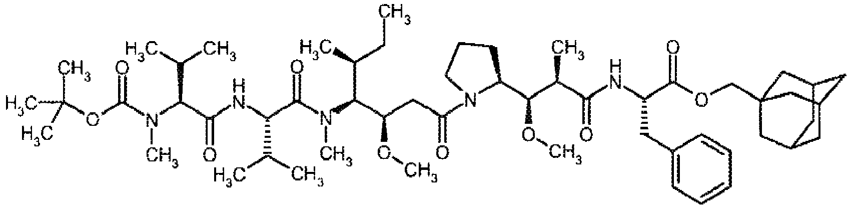

Zu einer Lösung von 20 mg (29 µmol) *N*-(*tert*.-Butoxycarbonyl)-*N*-methyl-L-valyl-*N*-[(3*R*,4*S*,5*S*) 1-{(25)-2-[(1*R*,2*R*)-2-carboxy-1-methoxypropyl]pyrrolidin-1-yl}-3-methoxy-5-methyl-1-oxo-heptan-4-yl]-*N*-methyl-L-valinamid in 1 ml DMF wurden bei RT 15.3 µl (88 µmol) *N,N-*Diisopropylethylamin, 6.7 mg (44 µmol) HOBt und 6.7 mg (35 µmol) EDC gegeben und die Mischung 30 min lang gerührt. Anschließend wurden 10.1 mg (32 µmol) Adamantan-1-yl-*L-*phenylalaninat-Hydrochlorid hinzugefügt. Nach Rühren über Nacht wurde das Reaktionsgemisch direkt über präparative HPLC in seine Komponenten aufgetrennt. Es wurden 27.5 mg (93% d. Th.) der Titelverbindung erhalten.
LC-MS (Methode 1): Rₜ = 1.70 min; m/z - 980 (M+H)⁺.

### Intermediat 35

### N-Methyl-L-valyl-N-[(3R,4S,5S)-1-{(2S)-2-[(1R,2R)-3-{[(2S)-1-(adamantan-1-ylmethoxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid-Trifluoracetat

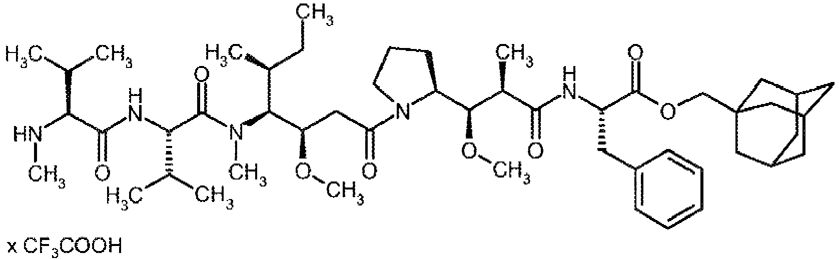

27.5 mg (28 µmol) *N*-(*tert*.-Butoxycarbonyl)-*N*-methyl-L-valyl-*N*-[(3*R*,4*S*,5*S*)-1-{(2*S*)-2-[(1*R,*2*R*)-3-{[(2*S*)-1-(adamantan-1-ylmethoxy)-1-oxo-3-phenylpropan-2-yl]amino}-1-methoxy-2-methyl-3-oxopropyl]pyrrolidin-1-yl}-3-methoxy-5-methy-1-oxoheptan-4-yl]-*N*-methyl-L-valinamid wurden in 1.8 ml Dichlormethan gelöst und mit 361 µl TFA versetzt. Das Reaktionsgemisch wurde 30 min gerührt und dann eingeengt. Der Rückstand wurde in Wasser aufgenommen und lyophilisiert. Es wurden 22.7 mg (81 % d. Th.) der Titelverbindung erhalten.
LC-MS (Methode 1): Rₜ = 1.14 min; m/z = 880 (M+H)⁺.

### Intermediat 36

### tert.-Butyl-[(2S)-1-(benzyloxy)-3-phenylpropan-2-yl]carbamat

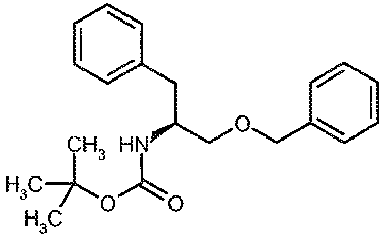

Unter Argonatmosphäre wurden 500 mg (1.99 mmol) *N*-Boc-*L*-Phenylalaninol in 5 ml DMF gelöst und auf 0°C gekühlt. Anschließend wurden 159 mg (3.98 mmol) einer 60%-igen Suspension von Natriumhydrid in Paraffinöl zugegeben. Das Reaktionsgemisch wurde bis zur Beendigung der Gasentwicklung gerührt und anschließend mit 260 µl (2.19 mmol) Benzylhromid versetzt. Das Kühlbad wurde entfernt und die Reaktionsmischung 2 h bei RT gerührt. Danach wurde das Reaktionsgemisch eingeengt, der Rückstand in Eiswasser aufgenommen und das Gemisch mit Dichlormethan extrahiert. Die organische Phase wurde mit gesättigter Natriumchlorid-Lösung gewaschen, über Magnesiumsulfat getrocknet und eingeengt. Der Rückstand wurde mittels präparativer HPLC gereinigt. Es wurden 226 mg (33% d. Th.) der Titelverbindung erhalten.
LC-MS (Methode 1): Rₜ = 1.28 min; m/z = 342 (M+H)⁺.

### Intermediat 37

### (2S)-1-(Benzyloxy)-3-phenylpropan-2-amin-Hydrochlorid

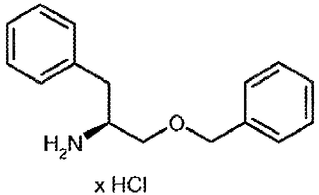

220 mg (644 µmol) *tert*.-Butyl-[(2*S*)-1-(benzyloxy)-3-phenylpropan-2-yl]carbamat wurden in 11 ml einer 4 N Lösung von Chlorwasserstoff in Dioxan gelöst und 1 h bei RT gerührt. Dann wurde das Reaktionsgemisch eingeengt und der Rückstand im Vakuum getrocknet. Es wurden 138 mg (77% d. Th.) der Titelverbindung erhalten.
LC-MS (Methode 1): Rₜ = 0.65 min; m/z = 242 (M+H)⁺.

### Intermediat 38

### N-(tert.-Butoxycarbonyl)-N-methyl-L-valyl-N-[(3R,4S,5S)-1-{(2S)-2-[(1R,2R)-3-{[(2S}-1-(benzyl-oxy)-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

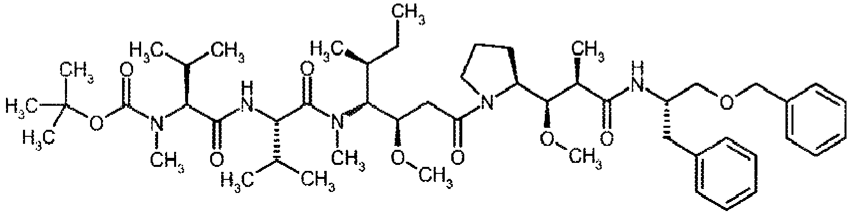

Zu einer Lösung von 20 mg (29 µmol) *N*-(*tert*.-Butoxycarhonyl)-*N*-methyl-L-valyl-*N*-[(3*R*,4*S*,5*S*)-1-{(2*S*)-2-[(1*R*,*R*)-2-carboxy-1-methoxypropyl]pyrrolidin-1-yl}-3-methoxy-5-methyl-1-oxo-heptan-4-yl]-*N*-methyl-L-valinamid in 1 ml DMF wurden bei RT 15.3 µl (88 µmol) *N*,*N-*Diisopropylethylamin, 6.7 mg (44 µmol) HOBt und 6.7 mg (35 µmol) EDC gegeben und die Mischung 30 min lang gerührt. Anschließend wurden 7.8 mg (32 µmol) (2*S*)-1-(Benzyloxy)-3-phenylpropan-2-amin-Hydrochlorid hinzugefügt Nach Rühren über Nacht wurde das Reaktionsgemisch direkt über präparative HPLC in seine Komponenten aufgetrennt. Es wurden 26 mg (98% d. Th.) der Titelverbindung erhalten.
LC-MS (Methode 1): Rt = 1.51 min; m/z = 909 (M+H)+.

### Intermediat 39

### N-Methyl-L-valyl-N-[(3R,4S,5S)-1-{(2S)-2-[(1R,2R)-3-{[(2S)-1-(benzyloxy)-3-phenylpropan-2-yl]ammo}-1-methoxy-2-methyl-3-oxopropyl]pyrrolidin-1-yl}-3-methoxy-5-methyl-1-oxoheptan-4-yl]-N-methyl-L-valinamid-Trifluoracetat

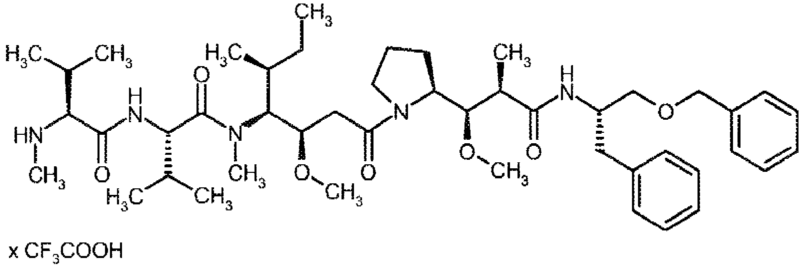

26 mg (29 µmol) *N*-(*tert*.-Butoxycarbonyl)-*N*-methyl-L-valyl-*N*-[(3*R*,4*S*,5*S*)-1-{(2*S*)-2-[(1*R*,2*R*)-3-{[(2*S*)-1-(benzyloxy)-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid wurden in 1.8 ml Dichlormethan gelöst und mit 370 µl TFA versetzt. Das Reaktionsgemisch wurde 30 min bei RT gerührt und anschließend eingeengt. Der Rückstand wurde in Wasser aufgenommen und lyophilisiert. Es wurden 26.4 mg (quant.) der Titelverbindung erhalten.
LC-MS (Methode 1): Rₜ = 0.97 min; m/z = 809 (M+H)⁺.

### Intermediat 40

### N-Methyl-L-valyl-N-[(3R,4S,5S)-1-{(2S)-2-[(1R,2R)-3-{[(1S,2R)-hydroxy-1-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

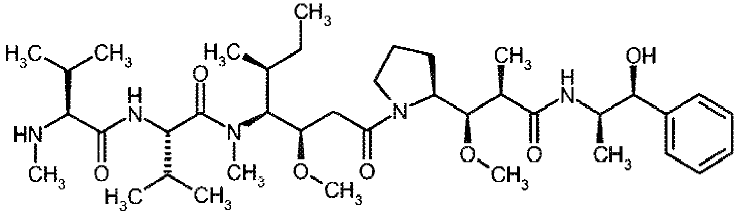

50 mg (70µmol) von Intermediat 26 und 11 mg (70 µmol) (1S,2R)-2-Amino-1-phenylpropan-1-ol in 10 ml DMF wurden mit 42 mg (0.11 µmol) O-(7-Azabenzotriazol-1-yl)-N,N,N',N'-tetramethyluronium-hexafluorophosphat und 25 µL *N,N*-Diisopropylethylamin versetzt und das Reaktionsgemisch 5 min bei RT gerührt. Anschließend wurde eingeengt und der Rückstand mittels präparativer HPLC gereinigt. Nach Vereinigung der entsprechenden Fraktionen, Einengen und Trocknung im Hochvakuum wurden 49 mg (81%) des geschützten Intermediats erhalten. Anschließend wurde die Boc-Gruppe nach bekannten Bedingungen mit Trifluoressigsäure in Dichlormethan abgespalten. Nach Einengen erfolgte die Reinigung der Titelverbindung durch präparative HPLC und es wurden 26 mg (52%) der Titelverbindung erhalten.
HPLC (Methode 12): Rₜ = 1.65 min;
LC-MS (Methode 1): Rₜ = 0.77 min; MS (ESIpos): m/z = 718 (M+H)⁺.

### Intermediat 41

### 3-{2-[2-(2-Aminoethoxy)ethoxy]ethoxy}propansäure-Trifluoracetat

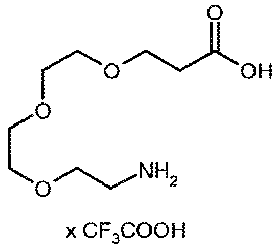

150 mg (541 µmol) *tert*.-Butyl-3-{2-[2-(2-aminoethoxy)ethoxy]ethoxy}propanoat wurden in 3 ml Dichlormethan gelöst, mit 1.5 ml Trifluoressigsäure versetzt und 1 h bei RT gerührt, anschließend wurde das Reaktionsgemisch eingeengt.
Es wurden 181 mg (100% d.Th.) Produkt erhalten.
MS (EI): m/z 222 (M+H)⁺

### Intermediat 42

### 3-(2-{2-[2-(2,5-Dioxo-2,5-dihydro-1H-pyrrol-1-yl)ethoxy]ethoxy}elhoxy)propansäure

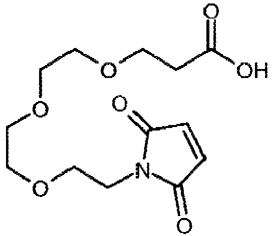

186 mg (555 µmol) 3-{2-[2-(2-Aminoethoxy)ethoxy]ethoxy}propansäure-Trifluoracetat wurden in 2.6 ml gesättigter Natriumhydrogencarbonat-Lösung gelöst und bei 0°C mit 86 mg (555 µmol) N-Methoxycarbonylmaleinimid versetzt. Das Reaktionsgemisch wurde 40 min. bei 0 °C und 1 h bei RT gerührt, dann wieder auf 0°C abgekühlt, mit Schwefelsäure auf pII 3 eingestellt und mit 3x 25 ml Ethylacetat extrahiert. Die vereinigten organischen Phasen wurden über Magnesiumsulfat getrocknet und eingeengt.
Es wurden 126 mg (75% d.Th.) der Titelverbindung erhalten.
LC-MS (Methode 1): Rₜ = 0.53 min: m/z = 302 (M+H)⁺.

### Intermediat 43

### tert.-Butyl-15-(2,5-dioxo-2,5-dihydro-1H-pyrrol-1-yl)-4-oxo-7,10,13-trioxa-2,3-diazapentadecan-1-oat

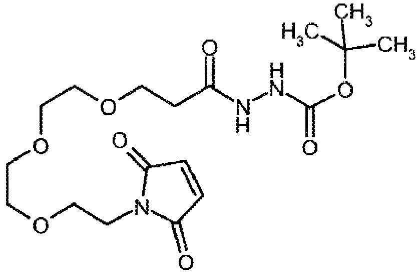

125 mg (417 µmol) 3-(2-{2-[2-(2,5-Dioxo-2,5-dihydro-1*H-*pyrrol-1-yl)ethoxy]ethoxy}ethoxy) propansäure wurden bei 0°C in 2.1 ml THF gelöst und mit 46 µl (417 mmol) 4-Methylmorpholin und 54.5 µl (417 µmol) Isobutylchloroformiat versetzt. Das Eisbad wurde entfernt und das Reaktionsgemisch 30 min. bei RT gerührt. Anschließend wurden bei 0°C 55 mg (417 µmol) tert.-Butyloxycarbonylhydrazid zugegeben. Das Reaktionsgemisch wurde über Nacht auf RT erwärmt, eingeengt und per präparativer IIPLC gereinigt.

Es wurden 60 mg (33% d.Th.) der Titelverbindung erhalten.

LC-MS (Methode 1): Rₜ = 0.66 min; m/z = 416 (M+H)⁺.

### Intermediat 44

### 3-(2-{2-[2-(2,5-dioxo-2,5-dihydro-1H-pyrrol-1-yl)ethoxy]ethoxy}ethoxy)propanhydrazid-Trifluoracetat

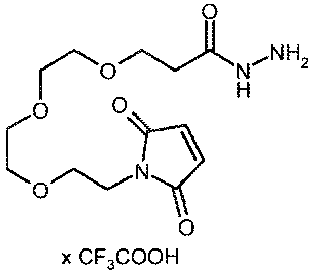

60 mg (145 µmol) *tert*.-Butyl-15-(2,5-dioxo-2,5-dihydro-1*H*-pyrrol-1-yl)-4-oxo-7,10,13-trioxa-2,3-diazapentadecan-1-oat wurden in 1 ml Dichlormethan gelöst und mit 0.2 ml Trifluoressigsäure versetzt. Das Reaktionsgemisch wurde 30 min. bei RT gerührt und anschließend eingeengt.
Es wurden 62 mg (100% d.Th.) Produkt erhalten.
LC-MS (Methode 1): Rₜ = 0.35 min; m/z = 316 (M+H)⁺.

### Intermediat 45

### Benzyl-(1S,2R)-1-amino-2-phenylcyclopropancarboxylat-Trifluoracetat

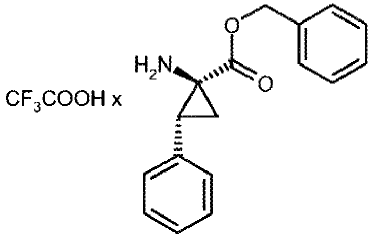

Die Titelverbindung wurde nach Standardmethoden durch Veresterung von kommerziell erhältlicher (1*S*,2*R*)-1-[(*tert*.-Butoxycarbonyl)amino]-2-phenylcyclopropancarbonsäure mit Benzylalkohol und anschließende Boc-Abspaltung mit Trifluoressigsäure hergestellt.
LC-MS (Methode 1): Rₜ = 0.72 min; MS (ESIpos): m/z = 268 (M+H)⁺.

### Intermediat 46

### N-(tert.-Butoxycarbonyl)-N-methyl-L-valyl-N-[(3R,4S,5S)-1-{(2S)-2-[(1R,2R)-3-{[(1S)-1-carboxy-2-phenylethyl]amino}-1-methoxy-2-methyl-3-oxopropyl]pyrrolidin-1-yl}-3-methoxy-5-methyl-1-oxoheptan-4-yl]-N-methyl-L-valinamid

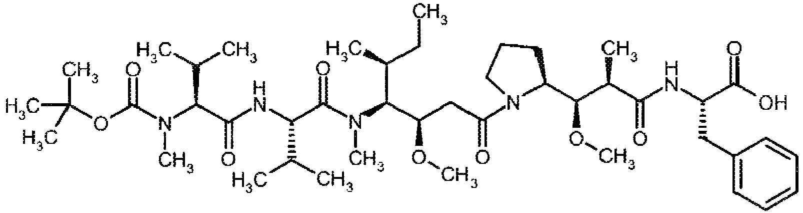

383 mg (0.743 mmol) *N*-(*tert*.-Butoxycarbonyl)-*N*-methyl-L-valyl-*N*-(2*R*,3*S*,4*S*)-*N*-carboxy-2-methoxy-4-methylhexan-3-yl]-*N*-methyl-L-valinamid (Intermediat 8) wurden mit 485 mg (0.743 mmol) Benzyl-*N*-{(2*R*,3*R*)-3-methoxy-2-methyl-3-[(2*S*)-pyrrolidin-2-yl]propanoyl}-L-phenylalaninat-Tritluoracetat (Intermediat 12), 424 mg (1.114 mmol) *O*-(7-Azabenzotriazol-1-yl)-*N*,*N*,*N'*,*N'-*tetramethyluronium-hexafluorophosphat und 388 µl *N,N*-Diisopropylethylamin in 15 ml DMF zusammengegeben und 10 min bei RT gerührt. Anschließend wurde das Lösungsmittel im Vakuum entfernt. Der verbliebene Rückstand wurde in Ethylacetat aufgenommen und nacheinander mit 5%-iger wässriger Zitronensäure-Lösung und gesättigter Natriumhydrogencarbonat-Lösung ausgeschüttelt. Die organische Phase wurde abgetrennt, eingeengt und der Rückstand mittels präparativer HPLC gereinigt. Die Produktfraktionen wurden vereinigt, eingeengt und der Rückstand im Hochvakuum getrocknet. Es wurden 335 mg (48% d. Th.) des Benzylester-Intermediats als ein Schaum erhalten.
LC-MS (Methode 1): Rₜ = 1.49 min; MS (ESIpos): m/z = 922 (M+H)⁺.

100 mg (0.11 mmol) dieses Intermediats wurden in 15 ml Methanol aufgenommen und die Benzylester-Gruppe durch Hydrierung unter Wasserstoff-KonnaMnick mit 10% Palladium auf Aktivkohle als Katalysator entfernt. Nach 1 h Rühren bei RT wurde der Katalysator abfiltriert und das Filtrat im Vakuum eingeengt. Nach Lyophilisation aus Dioxan wurden 85 mg (94% d. Th.) der Titelverbindung als Feststoff erhalten.
HPLC (Methode 12): Rₜ = 2.4 min;
LC-MS (Methode 1): Rₜ = 1.24 min; MS (ESIpos): m/z = 832 (M+H)⁺.

### Intermediat 47

### N-Benzyl-L-tryptophanamid-Trifluoracetat

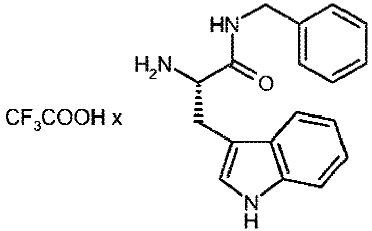

202 mg (0.5 mmol) 2,5-Dioxopyrrolidin-1-yl-*N*-(tert.-butoxycarbonyl)-L-tryptophanat und 45 mg (0.42 mmol) Benzylamin wurden in 10 ml DMF gelöst und mit 110 µl (630 µmol) *N,N*-Diiso-propylethylamin versetzt. Das Reaktionsgemisch wurde 3 h bei RT gerührt. Anschließend wurde das Reaktionsgemisch im Vakuum eingeengt und der Rückstand durch Flash-Chromatographie an Kieselgel gereinigt (Laufmittel: Dichlormethan/Methanol/17% aq. Ammoniak 20:0.5:0.05). Die entsprechenden Fraktionen wurden vereinigt und eingeengt. Der resultierende Rückstand wurde mit Diethylether digeriert und dann im Hochvakuum getrocknet. Anschließend wurde dieser Rückstand in 10 ml Dichlormethan aufgenommen und mit 3 ml wasserfreier Trifluoressigsäure versetzt. Nach 45 min Rühren bei RT wurde eingeengt und der Rückstand durch präparative HPLC gereinigt. Nach Trocknen im Hochvakuum wurden 117 mg (57% d. Th. über beide Stufen) der Titelverbindung erhalten.
HPLC (Methode 12): Rₜ = 1.6 min;
LC-MS (Methode 1): Rₜ = 0.66 min; MS (ESIpos): m/z = 294 (M+H)⁺.

### Intermediat 48

### (1S,2R)-1-Amino-2-phenylcyclopropancarboxamid-Trifluoracetat

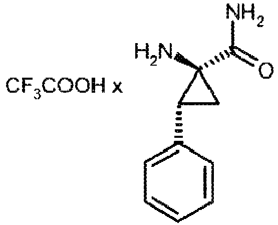

50 mg (180 µmol) kommerziell erhältlicher (1*S*,2*R*)-1-[(*tert*.-Butoxycarbonyl)amino]-2-phenyl-cyclopropancarbonsäure wurden in 5 ml DMF gelöst, mit 94 µl (541 µmol) *N,N*-Diisopropylethyl-amin, 31 mg (270 µmol) *N*-Hydroxysuccinimid sowie 41.5 mg (216 µmol) EDC versetzt und anschließend über Nacht bei RT gerührt. Das Reaktionsgemisch wurde danach eingeengt, der Rückstand in Dioxan aufgenommen, mit 71 mg (901 µmol) Ammoniumhydrogencarbonat versetzt und das Reaktionsgemisch dann 3 Tage bei RT stehen gelassen. Das Reaktionsgemisch wurde danach mit einer 1:1-Mischung aus Ethylacetat und Wasser verdünnt. Die organische Phase wurde abgetrennt, über Magnesiumsulfat getrocknet und eingeengt. Der resultierende Rückstand wurde anschließend in 3 ml Dichlormethan aufgenommen und mit 3 ml wasserfreier Trifluoressigsäure versetzt. Nach 1 h Rühren bei RT wurde eingeengt. Der Rückstand wurde mit Pentan verrührt, abgesaugt und aus Dioxan lyophilisiert. Auf diese Weise wurden 32 mg (62% d. Th. über beide Stufen) der Titelverbindung erhalten.
HPLC (Methode 6): Rₜ = 0.38 min;
LC-MS (Methode 1): Rₜ = 0.20 min; MS (ESIpos): m/z = 177 (M+H)⁺.

### Intermediat 49

### N^{α}-{(2R,3R)-3-Methoxy-2-methyl-3-[(2S)-pyrrolidin-2-yl]propanoyl}-L-tryptophanamid-Trifluoracetat

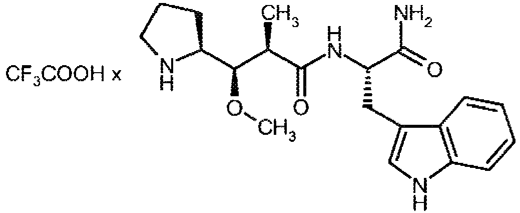

Die Titelverbindung wurde in Analogie zur Synthese von Intermediat 13 aus der Ausgangsverbindung 1 und L-Tryptophanamid-Hydrochlorid hergestellt.
HPLC (Methode 5): Rₜ = 1 .4 min;
LC-MS (Methode 1): Rₜ = 0.92 min; MS (ESIpos): m/z = 473 (M+H)⁺.

### Intermediat 50

### 4-Nitrophenyl-[(2-(2,5-dioxo-2,5-dihydro-1H-pyrrol-1-yl)ethyl]carbamat

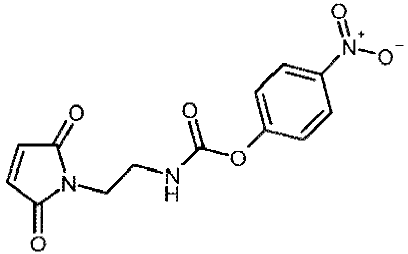

813 mg (3.1 mmol) Triphenylphosphin wurden in 25 ml THF gelöst und unter Argon auf -70°C abgekühlt. Nach tropfenweiser Zugabe von 627 mg (3.1 mmol) Diisopropylazodicarboxylat wurde die Mischung 5 min gerührt. Anschließend wurden 500 mg (3.1 mmol) tert.-Butyl-(2-aminoethyl)carbamat gelöst in 5 ml THF zugetropft und das Reaktionsgemisch weitere 5 min bei -70°C gerührt. Dann wurden 136.6 mg (1.55 mmol) 2,2 Dimethyl-l-propanol gelöst in 1 ml TIIF sowie 301 mg (3.1 mmol) Maleinimid zugesetzt, das Reaktionsgemisch weitere 10 min bei -70°C gerührt und anschließend auf RT erwärmt. Nach weiteren 16h Rühren bei RT wurde das Lösungsmittel im Vakuum entfernt und der Rückstand mittels präparativer HPLC gereinigt. Man erhielt 463 mg (62%) des geschützten Intermediats.

Nach Entfernen der Boc-Schutzgruppe unter Standardbedingungen wurden 652 mg von 1-(2-aminoethyl)-1*H*-pyrrol-2,5-dion als Trifluoracetat erhalten.

112.9 mg (543 µmol) Chlorameisensäurenitrophenylester wurden in 30 ml THF gelöst und nach Zugabe von 100 mg (271.6 µmol) 1-(2-Aminoethyl)-1*H*-pyrrol-2,5-dion-Trifluoracetat 30 min bei RT gerührt. Die Mischung wurde filtriert und das Filtrat wurde bis zur Trockne eingeengt und anschließend mit Diethylether aufgeschlämmt. Nach Absaugen und Trocknen wurden 60 mg (95% d.Th.) der Titelverbindung erhalten.
HPLC (Methode 5): Rt = 0.65 min;
LC-MS (Methode 1): Rt = 0.74 min; MS (ESIpos): m/z = 306 (M+H)+.

### Intermediat 51

### (1S)-2-Phenyl-1-(5-phenyl-1,3,4-oxadiazol-2-yl)ethanamin-Trifluoracetat

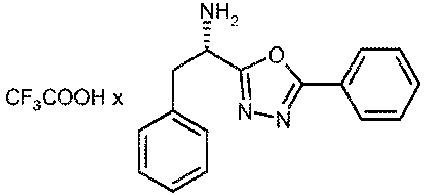

200 mg (0.75 mmol) *N*-(*tert*.-Butoxycarbonyl)-L-phenylalanin wurden bei 0°C in 5.5 ml Dichlormethan vorgelegt und mit 128 mg (0.79 mmol) 1,1'-Carbonyldiimidazol versetzt. Nach 30 min wurden 103 mg (0.75 mmol) Benzoylhydrazid zugegeben. Nach weiteren 45 min bei 0°C wurden schließlich 500 mg (1.5 mmol) Tetrabromkohlenstoff und 395 mg (1.5 mmol) Triphenylphosphin hinzugefügt. Das Reaktionsgemisch wurde zunächst 2 h bei 0°C und dann über Nacht bei R.T nachgerührt. Das Gemisch wurde anschließend am Rotationsverdampfer eingeengt und der Rückstand im Hochvakuum getrocknet. Das so erhaltene Rohprodukt wurde mittels präparativer HPLC gereinigt. Es wurden 217 mg (78% d. Th.) des Boc-geschützten Intermediats *tert*.-Butyl-[(1*S*)-2-phenyl-1-(5-phenyl-1,3,4-oxadiazol-2-yl)ethyl]carbamat erhalten.
LC-MS (Methode 12): Rₜ = 1.15 min. MS (ESIpos): m/z = 366 (M+H)⁺

217 mg (0.59 mmol) dieses Intermediats wurden in 3 ml Dichlormethan aufgenommen, mit 0.6 ml Trifluoressigsäure versetzt und 30 min bei RT gerührt. Anschließend wurde das Reaktionsgemisch im Vakuum eingeengt. Der verbliebene Rückstand wurde das Reaktionsgemisch im Vakuum weiter getrocknet und dann aus Dioxan lyophilisiert. Auf diese Weise wurden 214 mg (90% d. Th.) der Titelverbindung erhalten.
LC-MS (Methode 11): Rₜ = 0.62 min; MS (ESIpos): m/z = 266 (M+H)⁺

### Intermediat 52

### (1R)-2-Phenyl-1-(5-phenyl-1,3,4-oxadiazol-2-yl)ethanamin-Trifluoracetat

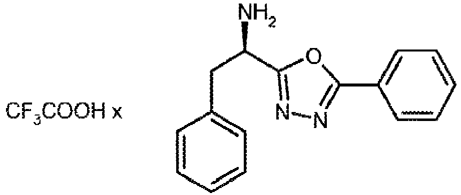

200 mg (0.75 mmol) *N*-(*tert.*-Butoxycarbonyl)-D-phenylalanin wurden bei 0°C in 5.5 ml Dichlormethan vorgelegt und mit 128.3 mg (0.79 mmol) 1,1'-Carbonyldiimidazol versetzt. Nach 30 min wurden 103 mg (0.75 mmol) Benzoylhydrazid zugegeben. Nach weiteren 45 min bei 0°C wurden schließlich 500 mg (1.5 mmol) Tetrabromkohlenstoff und 395 mg (1.5 mmol) Triphenylphosphin hinzugefügt. Das Reaktionsgemisch wurde zunächst 2 h bei 0°C und dann über Nacht bei RT nachgerührt. Das Gemisch wurde anschließend am Rotationsverdampfer eingeengt und der Rückstand im Hochvakuum getrocknet. Das so erhaltene Rohprodukt wurde mittels präparativer HPLC gereinigt. Es wurden 219 mg (80% d. Th.) des Boc-geschützten Intermediats *tert.*-Butyl-[(1*R*)-2-phenyl-1-(5-phenyl-1,3,4-oxadiazol-2-yl)ethyl]carbamat erhalten.
LC-MS (Methode 2): Rₜ = 1.36 min; MS (ESIpos): m/z = 366 (M+H)⁺

219 mg (0.6 mmol) dieses Intermediats wurden in 3 ml Dichlormethan aufgenommen, mit 0.6 ml Trifluoressigsäure versetzt und 30 min bei RT gerührt. Anschließend wurde das Reaktionsgemisch im Vakuum eingeengt. Der verbliebene Rückstand wurde das Reaktionsgemisch im Vakuum weiter getrocknet und dann aus Dioxan lyophilisiert. Auf diese Weise wurden 196 mg (86% d. Th.) der Titelverbindung als Feststoff erhalten.
HPLC (Methode 10): Rₜ = 2.41 min

### Intermediat 53

### (2S)-1-(Benzylsulfonyl)-3-phenylpropan-2-amin

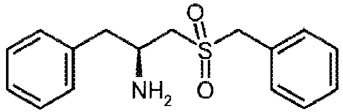

200 mg (1.13 mmol) (4*S*)-4-Benzyl-1,3-oxazolidin-2-on wurden in 3 ml *tert*.-Butanol vorgelegt und mit 280 mg (2.26 mmol) Benzylmercaptan versetzt. Das Gemisch wurde anschließend 2 Tage unter Rückfluss erhitzt. Danach wurde das Reaktionsgemisch am Rotationsverdampfer eingeengt und das erhaltene Intermediat (2*S*)-1-(Benzylsulfanyl)-3-phenylpropan-2-amin ohne Aufarbeitung direkt weiter umgesetzt.
HPLC (Methode 10): Rₜ = 2.63 min
LC-MS (Methode 1): Rₜ = 0.67 min; MS (ESIpos): m/z = 258 (M+H)⁺

Das oben erhaltene rohe Intermediat wurde in einer Lösung von 2 ml 30%-igem Wasserstoffperoxid und 5 ml Ameisensäure gelöst und 12 h bei RT gerührt. Dann wurde das Reaktionsgemisch auf gesättigte Natriumsulfat-Lösung gegeben und dreimal mit Ethylacetat extrahiert. Die organische Phase wurde über Magnesiumsulfat getrocknet und im Vakuum eingeengt. Das erhaltene Rohprodukt wurde mittels präparativer HPLC gereinigt. Es wurden so 343 mg (61% d. Th.) der Titelverbindung erhalten.
HPLC (Methode 10): Rₜ = 2.40 min;
LC-MS (Methode 12): Rₜ = 0.65 min; MS (ESIpos): m/z = 290 (M+H)⁺

### Intermediat 54

### (2S,3E)-1,4-Diphenylbut-3-en-2-amin

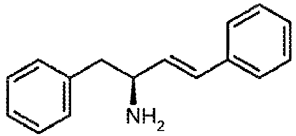

552.7 mg (9.85 mmol) Kaliumhydroxid wurden in Methanol gelöst, auf 1.1 g neutrales Aluminiumoxid aufgezogen und dann im Hochvakuum getrocknet. Zu einer Lösung von 240 mg (0.82 mmol) (*2S*)-1-(Benzylsulfonyl)-3-phenylpropan-2-amin und 1.56 g des so präparierten Kaliumhydroxid auf Aluminiumoxid in 6.2 ml *n-*Butanol wurden bei 5-10°C 307 µl (3.3 mmol) Dibromdifluormethan getropft. Die Reaktionsmischung wurde 2 h bei RT gerührt, dann über Celite filtriert und der Rückstand gut mit Dichlormethan nachgewaschen. Das Filtrat wurde eingeengt und der resultierende Rückstand im Vakuum getrocknet. Das so erhaltene Rohprodukt wurde mittels präparativer HPLC gereinigt. Es wurden 98 mg (35% d. Th.) der Titelverbindung mit einem E/Z-Diastereomerenverhältnis von 4:1 erhalten.
HPLC (Methode 10): Rₜ = 2.46 min;
LC-MS (Methode 12): Rₜ = 0.75 min; MS (ESIpos): m/z = 224 (M+H)⁺

Das oben erhaltene *E*/*Z*-Diastereomerengemisch wurde in 2 ml Ethanol und 0.2 ml *N*,*N*-Diisopropylethylamin gelöst und über IIPLC an chiraler Phase aufgetrennt [Säule: Daicel Chiralpak AD-H, 5 µm, 250 mm x 20 mm; Eluent: Hexan/(Ethanol + 0.2% Diethylamin) 50:50 v/v; UV-Detektion: 220 nm; Temperatur: 30°C]. Die entsprechenden Fraktionen wurden am Rotationsverdampfer eingeengt und der Rückstand im Vakuum getrocknet. Es wurden 45 mg der Titelverbindung erhalten.

¹H NMR (400 MHz, DMSO-d₆) δ └ppm┘ = 2.62 - 2.83 (m, 2 H) 3.52 - 3.71 (m, 1 H) 6.18 - 6.30 (m, 1 H) 6.34 - 6.46 (m, 1 H) 6.98 - 7.57 (m, 10 H) [weitere Signale unter Lösungsmittel-Peaks verborgen].

### Intermediat 55

### N-Methyl-L-valyl-N-[(3R,4S,5S)-3-methoxy-1-{(2S)-2-[(1R,2R)-1-methoxy-2-methyl-3-oxo-3-{[(1S)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-L-valinamid-Trifluoracetat

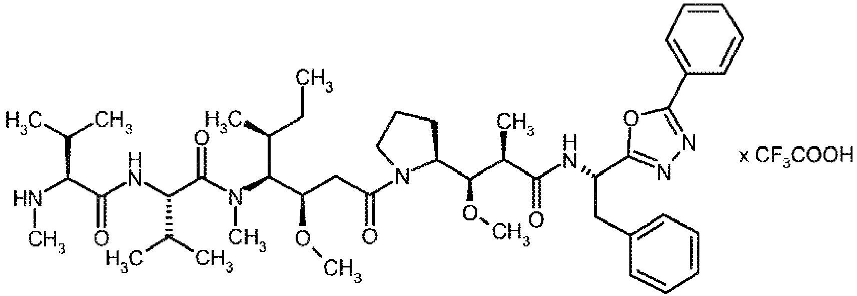

20 mg (29 µmol) *N*-(*tert*.-Butoxycarbonyl)-N-methyl-L-valyl-*N*-[(3*R*,4*S,*5*S*)-1-{(2*S*)-2-[(1*R*,2*R*)-2-carboxy-1-methoxypropyl]pyrrolidin-1-yl}-3-methoxy-5-methyl-1-oxohetan-4-yl]-*N*-methyl-L-valinamid wurden in 1 mL DMF gelöst, mit 13.3 mg (35 µmol) HATU und 15.3 µl (88 µmol) *N,N-*Diisopropylethylamin versetzt und 30 min. bei RT gerührt. Anschließend wurden 12.2 mg (32 µmol) (1*S*)-2-Phenyl-1-(5-phenyl-1,3,4-oxadiazol-2-yl)ethanamin-Trifluoracetat zugegeben. Das Reaktionsgemisch wurde über Nacht bei RT gerührt und anschließend per präparativer HPLC aufgetrennt. Es wurden so 22 mg (81% d.Th) *N*-(*tert*.-Butoxycarbonyl)-*N*-methyl-L-valyl-*N-*[(3*R*,4*S*,5*S*)-3-methoxy-1-{(2*S*)-2-[(1*R*,2*R*)-1-methoxy-2-methyl-3-oxo-3-{[(1*S*)-2-phenyl-1-(5-phenyl-1,3,4-oxadiazol-2-yl)ethyl]amino}propyl]pyrrolidin-1-yl}1-5-methyl-1-oxoheptan-4-yl]-*N-*methyl-L-valinamid erhalten.
LC-MS (Methode 12): Rₜ = 1.45 min; MS (ESIpos): m/z = 933 (M+H)⁺
Durch nachfolgende Abspaltung der BOC-Schutzgruppe mit Trifluoressigsäure wurden dann 22.4 mg (98% d. Th.) der Titel verbindung erhalten.
LC-MS (Methode 11): Rₜ = 0.85 min; MS (ESIpos): m/z = 833 (M+H)⁺

### Intermediat 56

### N-Methyl-L-valyl-N-[(3R,4S,5S)-3-methoxy-1-{(2S)-2-[(1R,2R)-1-methoxy-2-methyl-3-oxo-3-{[(1R)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-L-valinamid-Trifluoracetat

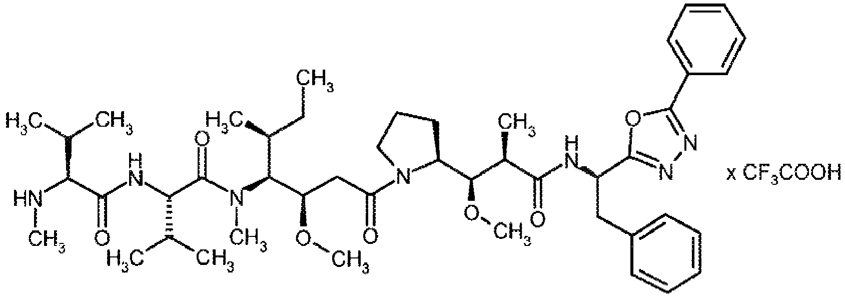

*N*-(*tert*.-Butoxycarbonyl)-*N*-methyl-L-valyl-*N*-[(3*R*,4*S*,5*S*)-3-methoxy-1-{(2*S*)-2-[(1*R,2R*)-1-methoxy-2-methyl-3-oxo-3-{[(1*R*)-2-phenyl-1-(5-phenyl-1,3,4-oxadiazol-2-yl)ethyl]amino}propyl]pyrrolidin-1-yl}-5-methyl-1-oxoheptan-4-yl]-*N*-methyl-L-valinamid wurde in Analogie zur Synthese von Intermediat 55 durch Umsetzung von 20 mg (29 µmol) *N*-(*tert.-*Butoxycarbonyl)-*N-*methyl-L-valyl-*N*-[(3*R*,4*S*,5*S*)-1-{(2*S*)-[(1*R*,2*R*)-carboxy-1-methoxy-propyl]pyrrolidin-1-yl}-3-methoxy-5-methyl-1-oxoheptan-4-yl]-*N*-methyl-L-valinamid mit 12.2 mg (32 µmol) (1*R*)-2-Phenyl-1-(5-phenyl-1,3,4-oxadiazol-2-yl)ethanamin-Trifluoracetat hergestellt.
Ausbeute: 17 mg (64 % d. Th.)
HPLC (Methode 10): Rₜ = 3.74 min;
LC-MS (Methode 1): Rₜ = 1.45 min; MS (ESIpos): m/z = 933 (M+H)⁺
Durch nachfolgende Abspaltung der BOC-Schutzgruppe mit Trifluoressigsäure erhielt man dann 17.1 mg (99 % d. Th.) der Titelverbindung.
HPLC (Methode 10): Rₜ = 2.55 min;
LC-MS (Methode 11): Rₜ = 0.85 min; MS (ESIpos): m/z = 833 (M+H)⁺

### Intermediat 57

### N-Methyl-L-valyl-N-[(3R,4S,5S)-1-{(2S)-2-[(1R,2R)-3-{[(2S)-1-(benzylsulfonyl)-3-pherylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid-Trifluoracetat

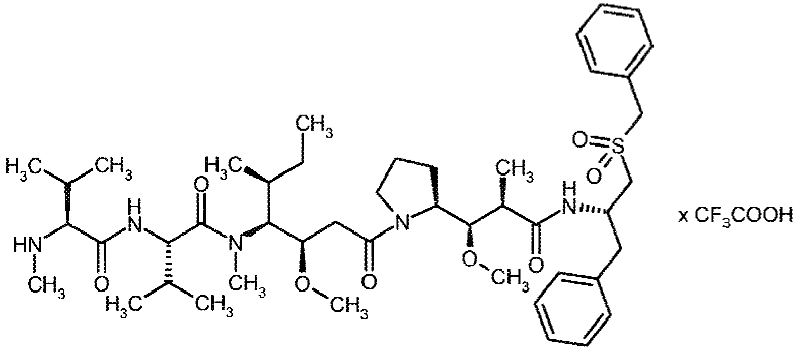

*N*-(*tert.*-Butoxycarbonyl)-*N*-methyl-L-valyl-*N-*[(3*R*,4*S*,5*S*)-1-{(2*S*)-2-[(1*R,*2*R*)-3-{[(2*S*)-1-(benzyl-sulfonyl)-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid wurde in Analogie zur Synthese von Intermediat 55 durch Umsetzung von 20 mg (29 µmol) *N*-(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid mit 9.3 mg (20 µmol) (2*S*)-1-(Benzylsulfonyl)-3-phenylpropan-2-amin hergestellt.
Ausbeute: 19.2 mg (68 % d. Th.)
HPLC (Methode 10): Rₜ = 3.5 min;
LC-MS (Methode 12): Rₜ = 1.41 min; MS (ESIpos): m/z = 957 (M+H)⁺
Durch nachfolgende Abspaltung der BOC-Schutzgruppe mit Trifluoressigsäure erhielt man dann 19.3 mg (99 % d. Th.) der Titel Verbindung.
HPLC (Methode 10): Rₜ = 2.52 min;
LC-MS (Methode 1): Rₜ = 0.86 min; MS (ESIpos): m/z = 857 (M+H)⁺

### Intermediat 58

### N-Methyl-L-valyl-N-[(3R,4S,5S)-1-{(2S)-2-[(1R,2R)-3-{[(2S,3E)-1,4-diphenylbut-3-en-2-yl]amino}-1-methoxy-2-methyl-3-oxopropyl]pyrrolidin-1-yl}-3-methoxy-5-methyl-1-oxoheptan-4-yl]-N-methyl-L-valinamid-Trifluoracetat

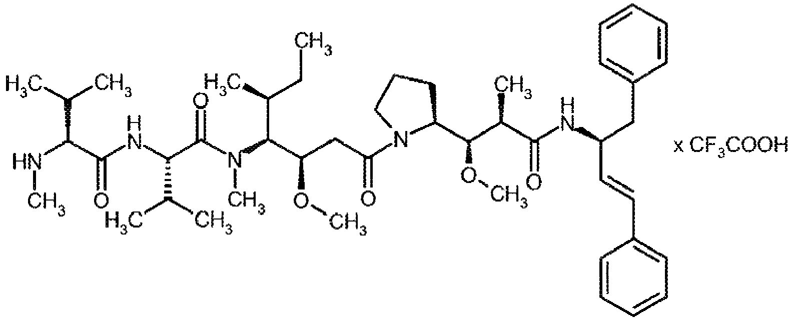

*N*-(*tert*.-Butoxycarbonyl)-*N*-methyl-L-valyl-*N*-[(3*R*,4*S,*5*S*)-1-{(2*S*)-2-[(1*R*,2*R*)-3-{[(2*S*,3*E*)-1,4-diphenylbut-3-en-2-yl]amino}-1-methoxy-2-methyl-3-oxopropyl]pyrrolidin-1-yl}-3-methoxy-5-methyl-1-oxoheptan-4-yl]-*N*-methyl-L-valinamid wurde in Analogie zur Synthese von Intermediat 55 durch Umsetzung von 20 mg (29 µmol) *N*-(*tert*.-Butoxycarbonyl)-*N*-methyl-L-valyl-*N-*[(3*R*,4*S,*5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid mit 7.1 mg (32 µmol) (2*S*,3*E*)-1,4-Diphenylbut-3-en-2-amin hergestellt.
Ausbeute: 15.1 mg (58 % d. Th.)
HPLC (Methode 10): Rₜ = 4.2 min;
LC-MS (Methode 12): Rₜ = 1.51 min; MS (ESIpos): m/z = 891 (M+H)⁺
Durch nachfolgende Abspaltung der BOC-Schutzgruppe mit Trifluoressigsäure erhielt man dann 15.7 mg (99 % d. Th.) der Titelverbindung.
HPLC (Methode 10): Rₜ = 2.62 min;
LC-MS (Methode 12): Rₜ = 0.97 min; MS (ESIpos): m/z = 791 (M+H)⁺

### Intermediat 61

### N-(3-Carboxypropyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]-pyrrolidin-1-yl}-5-methyl-1-oxoheptan-4-yl]-N-methyl-L-valinamid

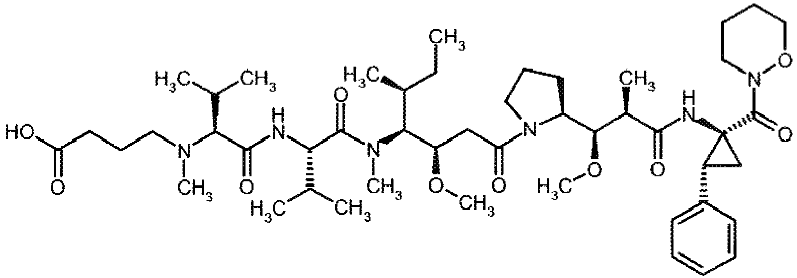

50 mg (0.054 mmol) *N*-Methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl]amino}-3-oxopropyl]-pyrrolidin-1-yl}-5-methyl-1-oxoheptan-4-yl]-*N*-methyl-L-valinamid-Trifluoracetat (Intermediat 16) wurden in 8 ml Dioxan/Wasser gelöst und mit 70 ml (0.108 mmol) einer 15%-igen Lösung von 4-Oxobutansäure in Wasser versetzt. Das Reaktionsgemisch wurde anschließend 1 h bei 100°C gerührt. Nach Abkühlen auf RT wurden 3.7 mg (0.059 mmol) Natriumcyanoborhydrid zugegeben und die Mischung durch Zugabe von etwa 300 µl 0.1 N Salzsäure auf einen pH-Wert von 3 eingestellt. Das Reaktionsgemisch wurde danach weitere 2 h bei 100°C gerührt. Nach Abkühlen wurden erneut 70 ml (0.108 mmol) der 15%-igen 4-Oxobutansäure-Lösung zugesetzt und das Reaktionsgemisch wiederum 1 h bei 100°C gerührt. Dann wurden weitere 3.7 mg (0.059 mmol) Natriumcyanoborhydrid zugegeben und anschließend mit etwa 300 µl 0.1 N Salzsäure der pH-Wert wieder auf 3 eingestellt. Das Reaktionsgemisch wurde danach erneut 2 h bei 100°C gerührt. Bei immer noch nicht vollständiger Umsetzung wurde diese Prozedur noch ein drittes Mal wiederholt. Das Reaktionsgemisch wurde schließlich eingeengt und der Rückstand mittels präparativer HPLC gereinigt. Es wurden auf diese Weise 32 mg (65% d. Th.) der Titelverbindung in Form eines farblosen Schaums erhalten.
HPLC (Methode 5): Rₜ = 1.64 min;
LC-MS (Methode 9): Rₜ = 4.76 min; MS (ESIpos): m/z = 899 (M+H.)⁺
¹H-NMR (500 MHz, DMSO-d₆): δ = 8.95 und 8.8 (2m, 1H), 8.88 und 8.65 (2s, 1H), 7.4-7.1 (m, 5H), 5.0, 4.78, 4.65 und 4.55 (4m, 2H), 4.1-3.7 (m, 5H), 3.32, 3.29, 3.20, 3.12, 3.1 und 3.0 (6s, 9H), 2.75 (m, 2H), 2.63 (t, 1H), 2.4-2.2 (m, 4H), 2.1-1.2 (m, 12H), 1.2-0.8 (m, 16H), 0.75 (m, 3H) [weitere Signale unter H₂O- und DMSO-Peaks verborgen].

### Intermediat 62

### N-(3-Carboxypropyl)-N-methyl-L-valyl-N-[(3R,4S,5S)-3-methoxy-1-{(2S)-2-[(1R,2R)-1-methoxy-2-methyl-3-{[(2S)-1-(1,2-oxazinan-2-yl)-1-oxo-3-phenylpropan-2-yl]amino}-3-oxopropyl]-pyrrolidin-1-yl}-5-methyl-1-oxoheptan-4-yl]-N-methyl-L-valinamid

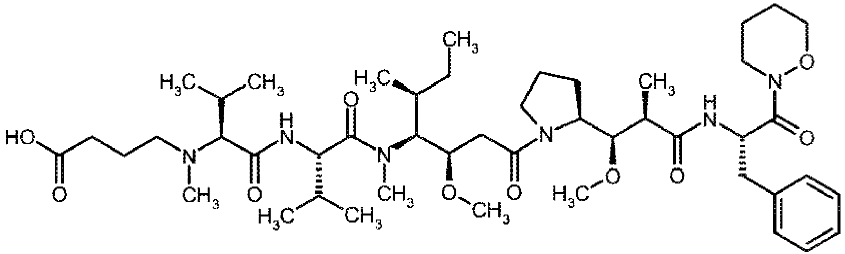

Die Titelverbindung wurde in Analogie zur Synthese von Intermediat 61 durch Umsetzung von 50 mg *N*-Methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(1,2-oxazinan-2-yl)-1-oxo-3-phenylpropan-2-yl]amino}-3-oxopropyl]pyrrolidin-1-yl}-5-methyl-1-oxoheptan-4-yl]-*N*-methyl-L-valinamid-Trifluoracetat (Intermediat 14) mit 4-Oxobutansäure hergestellt
Ausbeute: 34 mg (70% d. Th.)
HPLC (Methode 5): Rₜ = 1.64 min;
LC-MS (Methode 9): Rₜ = 4.77 min; MS (ESIpos): m/z = 887 (M+H)⁺.

### Intermediat 63

### N-(4-Carboxybenzyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]-pyrrolidin-1-yl}-5-methyl-1-oxoheptan-4-yl]-N-methyl-L-valinamid

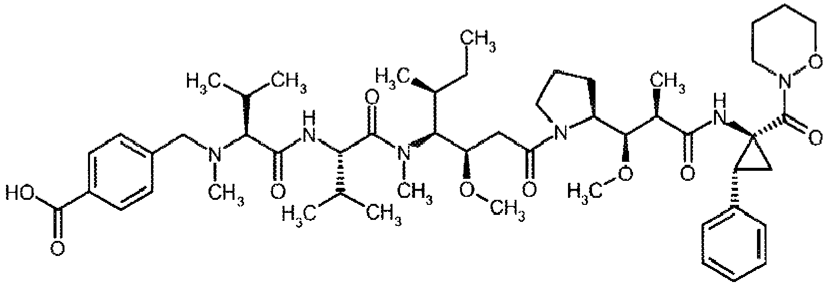

Die Titelverbindung wurde in Analogie zur Synthese von Intermediat 61 durch Umsetzung von 15 mg *N*-Methyl-L-valyl-*N*-[(3*R*,4*S*,5*S*)-3-methoxy-1-{(2*S*)-2-[(*R*,2*R*)-1-methoxy-2-methyl-3-{[(1*S*,2*R*)-1-(1,2-oxazinan-2-ylcarbonyl)-2-phenylcyclopropyl]amino}-3-oxopropyl]pyrrolidin-1-yl}-5-methyl-1-oxoheptan-4-yl]-*N*-methyl-L-valinamid-Trifluoracetat (Intermediat 16) mit 4-Formylbenzoesäure hergestellt.
Ausbeute: 7.5 mg (48% d. Th.)
HPLC (Methode 5): Rₜ = 1.75 min;
LC-MS (Methode 1): Rₜ = 0.97 min; MS (ESIpos): m/z = 947 (M+H)⁺.

### Intermediat 64

### N-(5-Carboxypentyl)-N-methyl-L-valyl-N-[(3R,4S,5S)-3-methoxy-1-{(2S)-2-[(1R,2R)-1-methoxy-2-methyl-3-{[(S,2R)-1-(1,2-oxazinan-2-ylcarbonyl)-2-phenylcyclopropyl]amino}-3-oxopropyl]-pyrrolidin-1-yl}-5-methyl-1-oxoheptan-4-yl]-N-methyl-L-valinamid

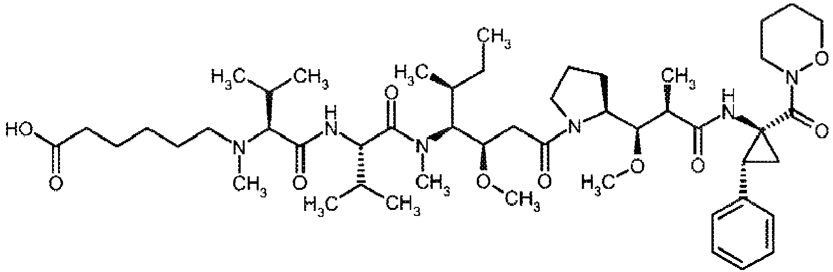

10 mg (0.011 mmol) *N*-Methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl]amino}-3-oxopropyl]-pyrrolidin-l-yl}-5-methyl-1-oxoheptan-4-yl]-*N*-methyl-L-valinamid-Trifluoracetat (Intermediat 16) wurden in 2 ml Dioxan/Wasser gelöst und mit 2.8 mg (0.022 mmol) 6-Oxohexansäure versetzt. Das Reaktionsgemisch wurde anschließend 1 h bei 100°C gerührt. Nach Abkühlen auf RT wurden 0.75 mg (0.012 mmol) Natriumcyanoborhydrid zugegeben und die Mischung durch Zugabe von 0.1 N Salzsäure auf einen pH-Wert von 3 eingestellt. Das Reaktionsgemisch wurde danach eine weitere Stunde bei 100°C gerührt. Nach Abkühlen wurden emeut 2.8 mg (0.022 mmol) 6-Oxohexansäure zugesetzt und das Reaktionsgemisch wiederum 1 h bei 100°C gerührt. Es wurden weitere 0.75 mg (0.012 mmol) Natriumcyanoborhydrid zugegeben und anschließend mit 0.1 N Salzsäure der pH-Wert wieder auf 3 eingestellt. Das Reaktionsgemisch wurde danach erneut 1 h bei 100°C gerührt. Diese Prozedur wurde dann noch ein drittes Mal wiederholt. Das Reaktionsgemisch wurde schließlich eingeengt und das Rohprodukt mittels präparativer HPLC gereinigt. Es wurden so 6.4 mg (64% d. Th.) der Titelverbindung in Form eines farblosen Schaums erhalten.
HPLC (Methode 5): Rₜ = 1.68 min;
LC-MS (Methode 9): Rₜ = 4.86 min; MS (ESIpos): m/z = 927 (M+H)⁺.

### Intermediat 65

### N-(2-Aminoethyl)-N-methyl-L-valyl-N-[(3R,4S,5S)-3-methoxy-1-{(2S)-2-[(R,2R)-1-methoxy-2-methyl-3-{[(2S)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid-Bistrifluoracetat

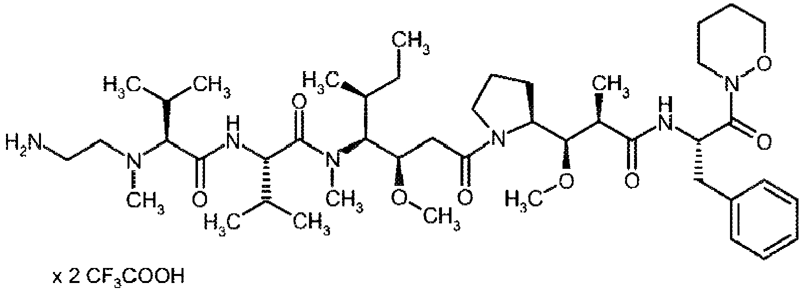

Die Titelverbindung wurde durch Umsetzung von 68 mg *N*-Methyl-L-valyl-*N*-[(3*R*,4*S*,5*S*)-3-methoxy-1-{(2*S*)-2-[(*R,*2*R*)-1-methoxy-2-methyl-3-{[(2*S*)-1-(1,2-oxazinan-2-yl)-1-oxo-3-phenylpropan-2-yl]amino}-3-oxopropyl]pyrrolidin-1-yl}-5-methyl-1-oxoheptan-4-yl]-*N*-methyl-L-valinamid-Trifluoracetat (Intermediat 14) mit *tert*.-Butyl-(2-oxoethyl)carbamat und nachfolgende Abspaltung der Boc-Schutzgruppe mit Trifluoressigsäure hergestellt.
Ausbeute: 49 mg (62% d. Th. über zwei Stufen)
IIPLC (Methode 5): Rₜ = 1.58 min;
LC-MS (Methode 2): Rₜ = 1.05 min; MS (ESIpos): m/z = 844 (M+H)⁺
¹H-NMR (600 MHz, DMSO-d₆): δ = 8.25 (m, 1H), 8.45 und 8.15 (2d, 1H), 7.65-7.55 (m, 3H), 7.23-7.1 (m, 5H), 5.12 und 4.95 (2m, 1H), 4.72 und 4.62 (2m, 1H), 4.6 und 4.52 (2t, 1H), 4.2-3.8 (m, 4H), 3.7 (d, 1H), 3.23, 3.20, 3.19, 3.18, 3.03 und 2.98 (6s, 9H), 3.0-2.7 (m, 6H), 2.4-1.2 (m, 15H), 1.05, 1.0, 0.88 und 0.82 (4d, 6H), 0.92 (m, 6H), 0.73 (m, 6H) [weitere Signale unter H₂O-Peak verborgen].

### Intermediat 66

### N-(3-Aminopropyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]-pyrrolidin-1-yl}-5-methyl-1-oxoheptan-4-yl]-N-methyl-L-valinamid

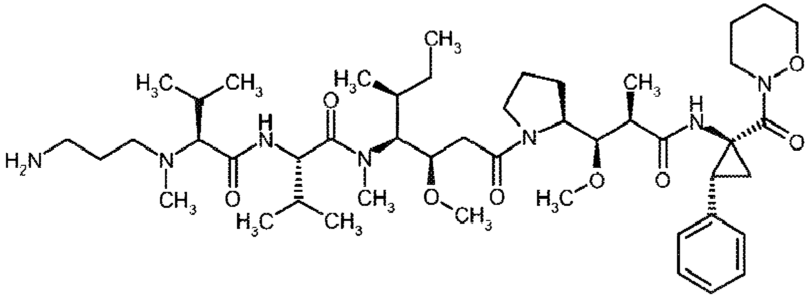

Die Titelverbindung wurde in Analogie zur Synthese von Intermediat 65 durch Umsetzung von 25 mg (0.027 mmol) *N*-Methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl]amino}-3-oxopropyl]-pyrrolidin-1-yl}-5-methyl-1-oxoheptan-4-yl]-*N*-methyl-L-valinamid-Trinuoracetat (Intermediat 16) mit Benzyl-(3-oxopropyl)carbamat und nachfolgende hydrogenolytische Abspaltung der Z-Schutzgruppe (mit 10% Palladium auf Kohle als Katalysator, in Ethanol als Lösungsmittel) hergestellt.
Ausbeute: 11 mg (41% d. Th. über zwei Stufen)
IIPLC (Methode 5): Rₜ = 1.53 min;
LC-MS (Methode 1): Rₜ = 0.72 min; MS (ESIpos): m/z = 870 (M+H)⁺.

### Intermediat 67

### N-(3-Carboxypropyl)-N-methyl-L-valyl-N-[(3R,4S,5S)-1-{(2S)-2-[(1R,2R)-3-{[(2S)-1-(adamantan-1-ylmethoxy)-1-oxo-3-phenylpropan-2-yl]amiro}-1-methoxy-2-methyl-3-oxopropyl]pyrrolidin-1-yl}-3-methoxy-5-methyl-1-oxoheptan-4-yl]-N-methyl-L-valinamid

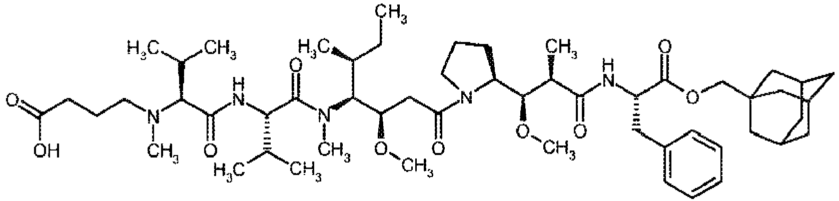

26 mg (26 µmol) *N*-Methyl-L-valyl-*N*-[(3*R,*4*S*,5*S*)-1-{(2*S*)-2-[(1*R*,2*R*)-3-{[(2*S*)-1-(adamantan-1-yl-methoxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid-Trifluoracetat und 33.9 µl einer 15%-igen wässrigen Bernsteinaldehydsäure-Lösung (53 µmol) wurden in 957 µl eines 1:1-Dioxan/Wasser-Gemisches gelöst und für 1 h auf 100 °C erhitzt. Nach kurzem Abkühlen wurden 1.81 mg (29 µmol) Natriumcyanoborhydrid zugegeben. Das Reaktionsgemisch wurde durch Zugabe von 0.1 N Salzsäure auf pH 3 eingestellt und für weitere 2 h auf 100°C erhitzt. Nach erneuter Zugabe gleicher Mengen an Bernsteinaldehydsäure-Lösung, Natriumcyanoborhydrid und Salzsäure wurde nochmals für 2 h auf 100 °C erhitzt. Das Reaktionsgemisch wurde danach direkt mittels präparativer HPLC in seine Komponenten aufgetrennt. Es wurden so 18.5 mg (73% d. Th.) der Titelverbindung erhalten.
LC-MS (Methode 1): Rₜ = 1.17 min; m/z = 967 (M+H)⁺.

### Intermediat 68

### N-(3-Carboxypropyl)-N-methyl-L-valyl-N-[(3R,4S,5S)-1-{(2S)-2-[(1R,2R)-3-{[(2S)-1-(benzyloxy)-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

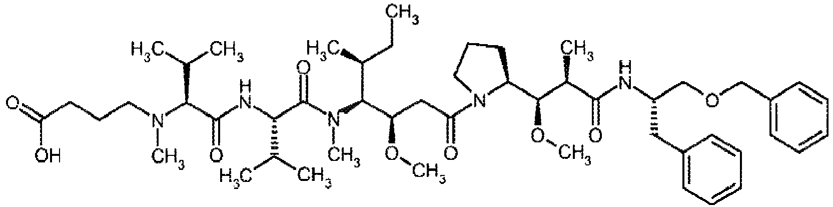

24 mg (26 µmol) *N*-Methyl-L-valyl-*N*-[(3*R*,4*S*,5*S*)-1-{(2*S*)-2-[(1*R*,2*R*)-3-{[(2*S*)-1-(benzyloxy)-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid-Trifluoracetat und 33.7 µl einer 15%-igen wässrigen Bernsteinaldehydsäure-Lösung (52 µmol) wurden in 953 µl eines 1:1-Dioxan/Wasser-Gemisches gelöst und für 1 h auf 100°C erhitzt. Nach kurzem Abkühlen wurden 1.80 mg (29 µmol) Natriumcyanoborhydrid zugegeben. Das Reaktionsgemisch wurde durch Zugabe von 0.1 N Salzsäure auf pH 3 eingestellt und für weitere 2 h auf 100°C erhitzt. Nach erneuter Zugabe gleicher Mengen an Bernsteinaldehydsäure-Lösung, Natriumcyanoborhydrid und Salzsäure wurde nochmals für 2 h auf 100°C erhitzt. Das Reaktionsgemisch wurde danach direkt mittels präparativer HPLC in seine Komponenten aufgetrennt. Es wurden so 15.2 mg (65% d. Th.) der Titelverbindung erhalten.
LC-MS (Methode 1): Rₜ = 1.01 min; m/z = 895 (M+H)⁺

### Intermediat 69

### N-(3-Carboxypropyl)-N-methyl-L-valyl-N-[(3R,4S,5S)-1-{(2S)-2-[(1R,2R)-3-{[(2S)-1-(benzyloxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

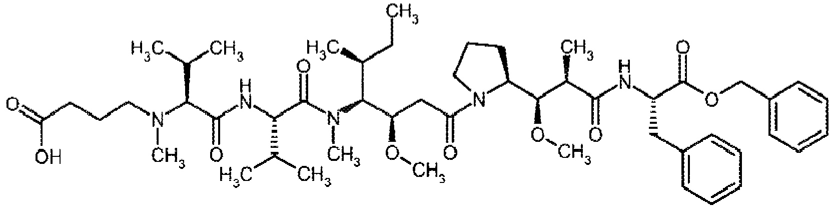

53 mg (84 µmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) und 45 mg (84 µmol) Benzyl-*N*-{(2*R*,3*R*)-3-methoxy-2-methyl-3-[(2*S*)-pyrrolidin-2-yl]propanoyl}-L-phenylalaninat-Trifluoracetat (Intermediat 12) wurden in 2 ml DMF aufgenommen, mit 19 µl *N,N*-Diisopropylethylamin, 14 mg (92 µmol) HOBt sowie 17.6 mg (92 µmol) EDC versetzt und dann über Nacht bei RT gerührt. Anschließend wurde das Reaktionsgemisch eingeengt und der Rückstand mittels präparativer HPLC gereinigt. Es wurden so 59 mg (68% d. Th.) des Fmoc-geschützten Intermediats *N*-[(9*H*-Fluoren-9-ylmethoxy)carbonyl]-*N*-methyl-L-valyl-*N*-[(3*R*,4*S*,5*S*)-1-{(2*S*)-2-[(1*R*,2*R*)-3-{[(2*S*)-1-(benzyloxy)-1-oxo-3-phenylpropan-2-yl]amino}-1-methoxy-2-methyl-3-oxopropyl]-pyrrolidin-1-yl}-3-methoxy-5-methyl-1-oxoheptan-4-yl]-*N*-methyl-L-valinamid erhalten.
LC-MS (Methode 1): Rₜ = 1.55 min; m/z = 1044 (M+H)⁺.

57 mg (0.055 mmol) dieses Intermediats wurden zur Abspaltung der Fmoc-Schutzgruppc mit 1.2 ml Piperidin in 5 ml DMF behandelt. Nach Einengen und Reinigung mittels präparativer HPLC wurden 39 mg (76% d. Th.) des freien Amin-Intermediats *N-*Methyl-L-valyl-*N*-[(3*R*,4*S*,5*S*)-1-{(2*S*)-2-[(1*R*,2*R*)-3-{[(2*S*)-1-(benzyloxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid als Trifluoracetat erhalten.
HPLC (Methode 5): Rₜ = 1.9 min;
LC-MS (Methode 1): Rₜ = 1.01 min; m/z = 822 (M+H)⁺.

37 mg (0.045 mmol) dieses Intermediats wurden in 5 ml Dioxan/Wasser gelöst und analog zur Herstellung der Verbindung in Intermediat 66 mit einer 15%-igen wässrigen Lösung von 4-Oxobutansäure in Gegenwart von Natriumcyanoborhydrid umgesetzt. Es wurden 16 mg (39% d. Th.) der Titelverbindung in Form eines farblosen Schaums erhalten.
HPLC (Methode 6): Rₜ = 2.1 min;
LC-MS (Methode 1): Rₜ = 1.01 min; MS (ESIpos): m/z = 908 (M+H)⁺.

### Intermediat 70

### N-(3-Carboxypropyl)-N-methyl-L-valyl-N-[(3R,4S,5S)-1-{(2S)-2-[(1R,2R)-3-{[(2S,3S)-1-(benzyloxy)-1-oxo-3-phenylbutan-2-yl]amino}-1-methoxy-2-methyl-3-oxopropyl]pyrrolidin-1-yl}-3-methoxy-5-methyl-1-oxoheptan-4-yl]-N-methyl-L-valinamid

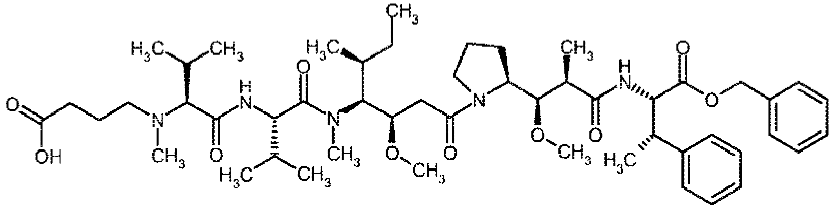

Zunächst wurde in Analogie zu der in Intermediat 14 beschriebenen Synthese ausgehend von den Intermediaten 4 und 26 die Amin-Verbindung *N*-Methyl-L-valyl-*N*-[(3*R*,4*S*,5*S*)-1-{(2*S*)-2-[(1*R*,2*R*)-3-{[(2*S*,3*S*)-1-(benzyloxy)-1-oxo-3-phenylbutan-2-yl]amino}-1-methoxy-2-methyl-3-oxopropyl]pyrrolidin-1-yl}-3-methoxy-5-methyl-1-oxoheptan-4-yl]-*N*-methyl-L-valinamid hergestellt.

30 mg (0.032 mmol) dieser Verbindung wurden in 6 ml Dioxan/Wasser gelöst und mit 41 µl (0.063 mmol) einer 15%-igen wässrigen Lösung von 4-Oxobutansäure versetzt. Das Reaktionsgemisch wurde anschließend 1 h bei 100°C gerührt. Nach Abkühlen auf RT wurden 2.2 mg (0.035 mmol) Natriumcyanoborhydrid zugegeben und die Mischung durch Zugabe von etwa 300 µl 0.1 N Salzsäure auf einen pII-Wert von 3 eingestellt. Das Reaktionsgemisch wurde danach weitere 2 h bei 100°C gerührt. Nach Abkühlen wurden erneut 41 µl (0.063 mmol) der 15%-igen 4-Oxobutansäure-Lösung zugesetzt und das Reaktionsgemisch wiederum 1 h bei 100°C gerührt. Dann wurden weitere 2.2 mg (0.035 mmol) Natriumcyanoborhydrid zugegeben und anschließend mit etwa 300 µl 0.1 N Salzsäure der pH-Wert wieder auf 3 eingestellt. Das Reaktionsgemisch wurde danach erneut 2 h bei 100°C gerührt. Bei immer noch nicht vollständiger Umsetzung wurde diese Prozedur noch ein drittes Mal wiederholt. Das Reaktionsgemisch wurde schließlich eingeengt und das Rohprodukt mittels präparativer HPLC gereinigt. Es wurden so 24 mg (82% d. Th.) der Titelverbindung in Form eines farblosen Schaums erhalten.
IIPLC (Methode 5): Rₜ = 1.9 min;
LC-MS (Methode 9): Rₜ = 5.15 min; MS (ESIpos): m/z = 922 (M+H)⁺.

### Intermedia 71

### N-(3-Carboxypropyl)-N-methyl-L-valyl-N-[(3R,4S,5S)-3-methoxy-1-{(2S)-2-[(1R,2R)-1-methoxy-3-{[(2S)-1-methoxy-1-oxo-3-phenylpropan-2-yl]amino}-2-methyl-3-oxopropyl]pyrrolidin-1-yl}-5-methyl-1-oxoheptan-4-yl]-N-methyl-L-valinamid

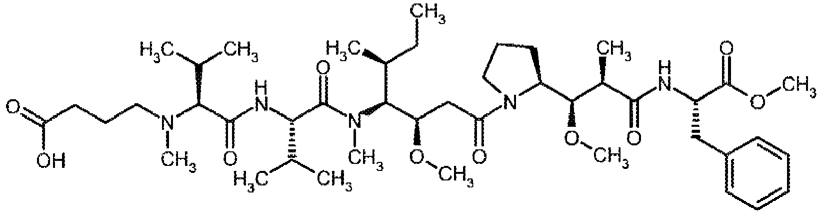

Zunächst wurde in Analogie zu der in Intermediat 14 beschriebenen Synthese ausgehend von den Intermediaten 4 und 39 die Amin-Verbindung *N*-Methyl-L-valyl-*N*-[(3*R*,4*S*,5*S*)-3-methoxy-1-{(2*S*)-2-[(1*R*,2*R*)-1-methoxy-3-{[(2*S*)-1-methoxy-1-oxo-3-phenylpropan-2-yl]amino}-2-methyl-3-oxopropyl]pyrrolidin-1-yl}-5-methyl-1-oxoheptan-4-yl]-*N*-methyl-L-valinamid hergestellt. Aus 7 mg (0.009 mmol) dieser Verbindung wurden dann in Analogie zur Herstellung von Intermediat 61 durch Umsetzung mit 4-Oxobutansäure in Gegenwart von Natriumcyanoborhydrid 2 mg (22% d. Th.) der Titelverbindung in Form eines farblosen Schaums erhalten.
HPLC (Methode 6): Rₜ = 1.9 min;
LC-MS (Methode 2): Rₜ = 1.06 min; MS (ESIpos): m/z = 832 (M+H)⁺.

### Intermediat 72

### N-(3-Carboxypropyl)-N-methyl-L-valyl-N-[(3R,4S,5S)-1-{(2S)-2-[(1R,2R)-3-{[(2S)-1-(benzyloxy)-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

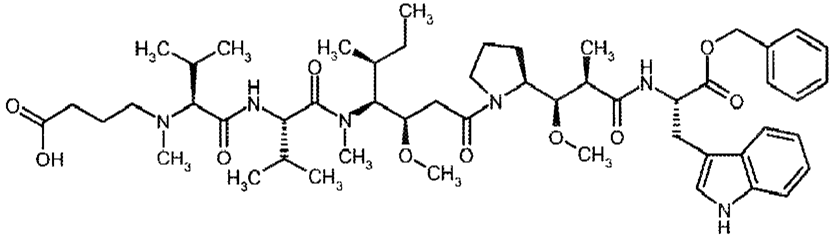

212 mg (411 µmol) *N*-(*tert*.-Butoxycarbonyl)-*N*-methyl-L-valyl-*N*-[(2*R*,3*S,*4*S*)-1-carboxy-2-methoxy-4-methylhexan-3-yl]-*N*-methyl-L-valinamid (Intermediat 8) und 237 mg (411 µmol) Benzyl-*N*-{(2*R*,3*R*)-3-methoxy-2-methyl-3-[(2*S*)-pyrrolidin-2-yl]propanoyl}-L-tryptophanat-Trifluoracetat (Intermediat 20) wurden in 30 ml DMF aufgenommen und mit 188 mg (493 µmol) *O*-(7-Azabenzotriazol-1-yl)-*N,N,N',N*'-tetramethyluronium-hexafluorophosphat sowie 215 µl *N,N*-Diisopropylethylamin versetzt. Das Reaktionsgemisch wurde 20 h bei RT gerührt, anschließend im Vakuum eingeengt und der Rückstand mittels präparativer HPLC aufgereinigt. Die Produktfraktionen wurden vereinigt, eingeengt und der Rückstand im Hochvakuum getrocknet. Es wurden so 315 mg (80% d. Th.) des Boc-geschützten Intermediats *N*-(*tert*.-Butoxycarbonyl)-*N-*methyl-L-valyl-*N*-[(3*R*,4*S*,5*S*)-1-{(2*S*)-2-[(1*R*,2*R*)-3-{[(2*S*)-1-(benzyloxy)-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid als farbloser Schaum erhalten.
LC-MS (Methode 1): Rₜ = 1.45 min; m/z = 961 (M+H)⁺.

50 mg (52 µmοl) dieses Intermediats wurden zur Abspaltung der Boc-Schutzgruppe mit 1 ml Trifluoressigsäure in 9 ml Dichlormethan behandelt. Nach Einengen und Reinigung mittels präparativer HPLC wurden 29 mg (57% d. Th.) des freien Amin-Intermediats *N*-Methyl-L-valyl-*N-*[(3*R*,4*S*,5*S*)-1-{(2*S*)-2-[(1*R*,2*R*)-3-{[(2*S*)-1-(benzyloxy)-3-(1*H*-indol-3-yl)-1-oxopropan-2-yl]-amino}-1-methoxy-2-methyl-3-oxopropyl]pyrrolidin-1-yl}-3-methoxy-5-methyl-1-oxoheptan-4-yl]-*N*-methyl-L-valinamid als Trifluoracetat erhalten.
LC-MS (Methode 1): Rₜ = 0.99 min; m/z = 861 (M+H)⁺.

29 mg (0.03 mmol) dieses Intermediats wurden in 6 ml Dioxan/Wasser gelöst und mit 39 µl (0.059 mmol) einer 15%-igen wässrigen Lösung von 4-Oxobutansäure versetzt. Das Reaktionsgemisch wurde anschließend 1 h bei 100°C gerührt. Nach Abkühlen auf RT wurden 2 mg (0.033 mmol) Natriumcyanoborhydrid zugegeben und die Mischung durch Zugabe von etwa 300 µl 0.1 N Salzsäure auf einen pH-Wert von 3 eingestellt. Das Reaktionsgemisch wurde danach weitere 2 h bei 100°C gerührt. Nach Abkühlen wurden erneut 39 µl (0.059 mmol) der 15%-igen 4-Oxobutansäure-Lösung zugesetzt und das Reaktionsgemisch wiederum 1 h bei 100°C gerührt. Dann wurden weitere 2 mg (0.033 mmol) Natriumcyanoborhydrid zugegeben und anschließend mit etwa 300 µl 0.1 N Salzsäure der pH-Wert wieder auf 3 eingestellt. Das Gemisch wurde erneut 2 h bei 100°C gerührt. Danach wurde das Reaktionsgemisch auf ein 1:1-Gemisch aus halbgesättigter wässriger Ammoniumchlorid-Lösung und Ethylacetat gegossen. Die organische Phase wurde abgetrennt, mit gesättigter Natriumchlorid-Lösung gewaschen, über Natriumsulfat getrocknet und eingeengt Der Rückstand wurde aus Wasser/ Acetonitril gefriergetrocknet. Es wurden so 27 mg (94% d. Th.) der Titelverbindung in Form eines farblosen Schaums erhalten.
HPLC (Methode 5): Rₜ = 2.2 min;
LC-MS (Methode 9): Rₜ = 5.04 min; MS (ESlpos): m/z = 947 (M+H)⁺.

### Intermediat 73

### N-(3-Carboxypropyl)-N-methyl-L-valyl-N-[(3R,4S,5S)-1-{(2S)-2-[(1R,2R)-3-({(2S)-1-[benzyl-(methyl)amino]-1-oxo-3-phenylpropan-2-yl}amino)-1-methoxy-2-methyl-3-oxopropyl]pyrrolidin-1-yl}-3-methoxy-5-methyl-1-oxoheptan-4-yl]-N-methyl-L-valinamid

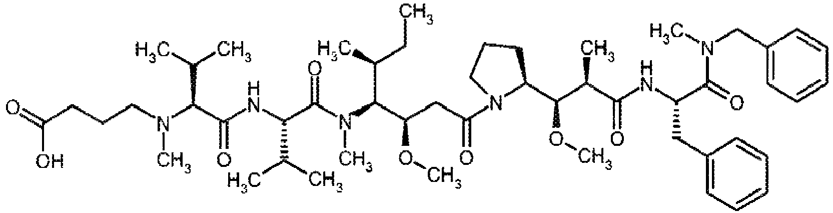

Zunächst wurde in Analogie zu der für Intermediat 14 beschriebenen Synthese ausgehend von den Intermediaten 4 und 38 die Amin-Verbindung *N*-Methyl-L-valyl-*N*-[(3*R*,4*S*,5*S*)-1-{(2*S*)-2-[(1*R*,2*R*)-3-({(2*S*)-1-[benzyl(methyl)amino]-1-oxo-3-phenylpropan-2-yl}amino)-1-methoxy-2-methyl-3-oxopropyl]pyrrolidin-1-yl}-3-methoxy-5-methyl-1-oxoheptan-4-yl]-*N*-methyl-L-valinamid hergestellt. Aus 25 mg (0.026 mmol) dieser Verbindung wurden dann in Analogie zur Herstellung von Intermediat 61 durch Umsetzung mit 4-Oxobutansäure in Gegenwart von Natriumcyanoborhydrid 13 mg (54% d. Th.) der Titelverbindung in Form eines farblosen Schaums erhalten.
HPLC (Methode 12): Rₜ = 2.2 min;
LC-MS (Methode 9): Rₜ = 5.01 min; MS (ESIpos): m/z = 921 (M+H)⁺.

### Intermediat 74

### N-(3-Carboxypropyl)-N-methyl-L-valyl-N-[(3R,4S,5S)-1-{(2S)-2-[(1R,2R)-3-({(1S,2R)-1-[(benzyl-oxy)carbonyl]-2-phenylcyclopropyl}amino)-1-methoxy-2-methyl-3-oxopropyl]pyrrolidin-1-yl}-3-methoxy-5-methyl-1-oxoheptan-4-yl]-N-methyl-L-valinamid

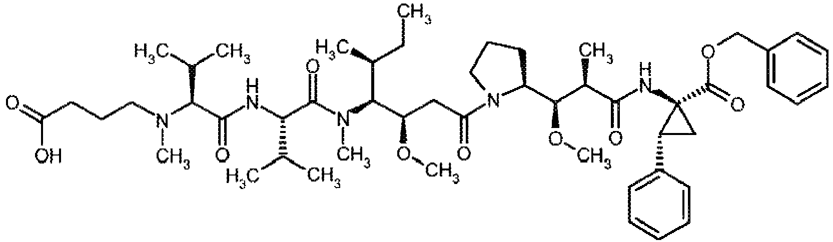

50 mg (73 µmol) *N*-(*tert*.-Buloxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 26) und 28 mg (73 µmol) Benzyl-(1*S*,2*R*)-1-amino-2-phenylcyclopropancarboxylat-Trifluoracetat (Intermediat 45) wurden in 5 ml DMF aufgenommen und mit 42 mg (110 µmol) *O*-(7-Azabenzotriazol-1-yl)-*N*,*N*,*N*',*N*'-tetramethyluronium-hexafluorophosphat und 38 µl *N,N*-Diisopropylethylamin versetzt. Das Reaktionsgemisch wurde 5 h bei RT gerührt, anschließend im Vakuum eingeengt und der Rückstand mittels präparativer HPLC aufgereinigt. Die Produktfraktionen wurden vereinigt und eingeengt. Nach Lyophilisation aus Dioxan/Wasser wurden 35 mg (51% d. Th.) des Boc-geschützten Intermediats *N*-(*tert*.-Butoxycarbonyl)-*N*-methyl-L-valyl-*N-*[(3*R,*4*S,*5*S*)-1-{(2*S*)-2-[(1*R*,2*R*)-3-({(1*S*,2*R*)-1-[(benzyloxy)carbonyl]-2-phenylcyclopropyl}-amino)-1-methoxy-2-methyl-3-oxopropyl]pyrrolidin-1-yl}-3-methoxy-5-methyl-1-oxoheptan-4-yl]-*N*-methyl-L-valinamid als farbloser Schaum erhalten.
LC-MS (Methode 1): Rₜ = 1.52 min; m/z = 934 (M+H)⁺.

35 mg dieses Intermediats wurden zur Abspaltung der Boc-Schutzgruppe mit 1 ml Trifluoressigsäure in 5 ml Dichlormethan behandelt. Nach Einengen und Lyophilisation aus Dioxan/Wasser wurden 34 mg (97% d. Th.) des freien Amin-Intermediats *N*-Methyl-L-valyl-*N*-[(3*R*,4*S*,5*S*)-1-{(2*S*)-2-[(1*R*,2*R*)-3-({(1*S*,2*R*)-1-[(benzyloxy)carbonyl]-2-phenylcyclopropyl}amino)-1-methoxy-2-methyl-3-oxopropyl]pyrrolidin-1-yl}-3-methoxy-5-methyl-1-oxoheptan-4-yl]-*N*-methyl-L-valinamid als Trifluoracetat erhalten.
LC-MS (Methode 1): Rₜ = 0.91 min; m/z = 834 (M+H)⁺.

Aus 11 mg (0.011 mmol) dieses Intermediats wurden dann in Analogie zur Herstellung von Intermediat 66 durch Umsetzung mit 4-Oxobutansäure in Gegenwart von Natriumcyanoborhydrid 2.5 mg (24% d. Th.) der Titelverbindung in Form eines farblosen Schaums erhalten.
HPLC (Methode 12): Rₜ = 2.2 min;
LC-MS (Methode 9): Rₜ = 5.1 min; MS (ESIpos): m/z = 920 (M+H)⁺.

### Intermediat 75

### N-(3-Carboxypropyl)-N-methyl-L-valyl-N-[(3R,4S,5S)-3-methoxy-1-{(2S)-2-[(1R,2R)-1-methoxy-2-methyl-3-oxo-3-{[(1S,2R)-2-phenyl-1-(propylcarbamoyl)cyclopropyl]amino}propyl]pyrrolidin-1-yl}-5-methyl-1-oxoheptan-4-yl]-N-methyl-L-valinamid

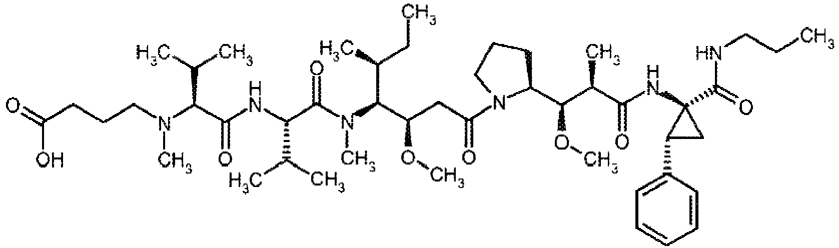

Zunächst wurde in Analogie zu der in Intermediat 74 beschriebenen Synthese durch Kupplung von *N*-(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 26) und (1*S*,2*R*)-1-Amino-2-phenyl-*N*-propylcyclopropancarboxamid-Trifluoracetat (Intermediat 27) in Gegenwart von *O*-(7-Azabenzotriazol-1-yl)-*N,N,N',N*'-tetramethyluronium-hexafluorophosphat und anschließende Abspaltung der Boc-Schutzgruppe mittels Trifluoressigsäure die Amin-Verbindung *N*-Methyl-L-valyl-*N*-[(3*R*,4*S*,5*S*)-3-methoxy-1-{(2*S*)-2-[(1*R*,2*R*)-1-methoxy-2-methyl-3-oxo-3-{[(1*S*,2*R*)-2-phenyl-1-(propylcarbamoyl)cyclopropyl]amino}propyl]pyrrolidin-1-yl}-5-methyl-1-oxoheptan-4-yl]*-N*-methyl-L-valinamid als Trifluoracetat hergestellt. Aus 14 mg (0.016 mmol) dieser Verbindung wurden dann in Analogie zur Herstellung von Intermediat 61 durch Umsetzung mit 4-Oxobutansäure in Gegenwart von Natriumcyanoborhydrid 11.3 mg (83% d. Th.) der Titelverbindung erhalten.
HPLC (Methode 6): Rₜ = 1.9 min;
LC-MS (Methode 2): Rₜ = 1.27 min; MS (ESIpos): m/z = 871 (M+H)⁺.

### Intermediat 76

### N-(3-Carboxypropyl)-N-methyl-L-valyl-N-[(3R,4S,5S)-1-{(2S)-2-[(1R,2R)-3-{[(1S,2R)-1-(ethoxycarbonyl)-2-phenylcyclopropyl]amino}-1-methoxy-2-methyl-3-oxopropyl]pyrrolidin-1-yl}-3-methoxy-5-methyl-1-oxoheptan-4-yl]-N-methyl-L-valinamid

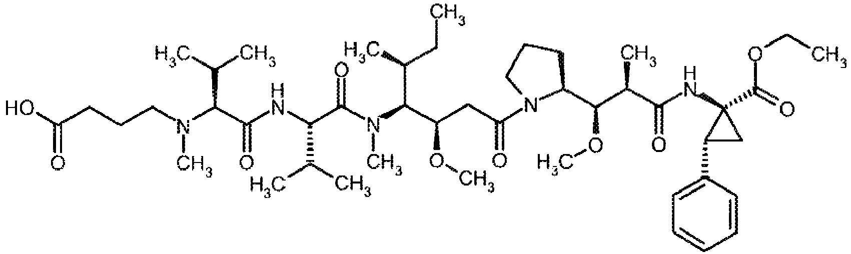

Zunächst wurde durch Kupplung von Intermediat 46 (*N-*(*tert*.-Butoxycarbonyl)-*N*-methyl-L-valyl-*N*-[(3*R*,4*S*,5*S*)-1-{(25)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid) mit Intermediat 48 (Ethyl-(1*S*,2*R*)-1-amino-2-phenylcyclopropancarboxylat-Trifiuoracetat) in Gegenwart von O-(7-Azabenzotriazol-1-yl)-*N*,*N,N',N'*-tetramethyluronium-hexafluorophosphat und anschließende Boc-Abspaltung die Ausgangsverbindung *N*-Methyl-L-valyl-*N*-[(3*R*,4*S*,5*S*)-1-{(2*S*)-2-[(1*R*,2*R*)-3-{[(1*S*,2*R*)-1-(ethoxycarbonyl)-2-phenylcyclopropyl]amino}-1-methoxy-2-methyl-3-oxopropyl]pyrrolidin-1-yl}-3-methoxy-5-methyl-1-oxoheptan-4-yl]-*N*-methyl-L-valinamid-Trifluoracetat hergestellt. Aus 70 mg (0.079 mmol) dieses Ausgangsmaterials wurden dann durch Umsetzung mit 4-Oxobutansäure in Analogie zu Intermediat 61 46 mg (68% d. Th.) der Titelverbindung erhalten.
HPLC (Methode 6): Rₜ = 1.9 min;
LC-MS (Methode 2): Rₜ = 1.28 min; MS (ESIpos): m/z = 858 (M+H)⁺

### Intermediat 77

### N-(3-Carboxypropyl)-N-methyl-L-valyl-N-[(3R,4S,5S)-1-{(2S)-2-[(1R,2R)-3-{[(2S)-1-amino-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

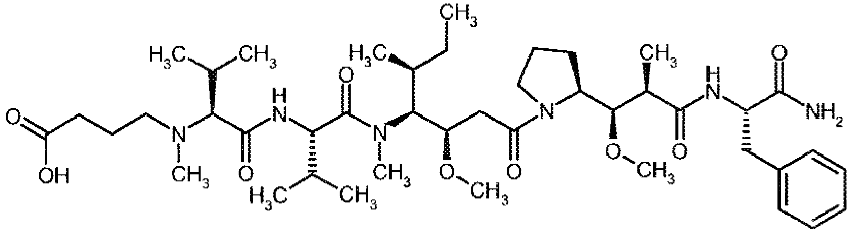

Zunächst wurde in Analogie zu der in Intermediat 75 beschriebenen Synthese durch Kupplung von *N*-(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 26) und L-Phenylalaninamid-Hydrochlorid in Gegenwart von O-(7-Azabenzotriazol-1-yl)-*N,N*,*N*',*N'-*tetramethyluronium-hexafluorophosphat und anschließende Abspaltung der Boc-Schutzgruppe mittels Trifluoressigsäure die Amin-Verbindung *N*-Methyl-L-valyl-*N*-[(3*R*,4*S*,5*S*)-1-{(2*S*)-2-[(1*R*,2*R*)-3-{[(2*S*)-1-amino-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid als Trifluoracetat hergestellt. Aus 47 mg (0.049 mmol) dieser Verbindung wurden dann in Analogie zur Herstellung von Intermediat 61 durch Umsetzung mit 4-Oxobutansäure in Gegenwart von Natriumcyanoborhydrid 39 mg (96% d. Th.) der Titelverbindung erhalten.
HPLC (Methode 6): Rₜ = 1.7 min;
LC-MS (Methode 9): Rₜ = 4.44 min; MS (ESIpos): m/z = 817 (M+H)⁺
¹H-NMR (500 MHz, DMSO-d₆): δ = 8.95 und 8.8 (2m, 1H), 8.25 und 8.0 (2d, 1H), 7.45, 7.35 und 7.0 (3s, breit, 2H), 7.3-7.1 (m, 5H), 4.8-4.4 (2m, 3H), 3.95 (m, 1H), 3.82 (m, 1H), 3.72 (d, 1H), 3.22, 3.18, 3.15, 3.05 und 3.00 (5s, 9H), 2.85-2.7 (m, 4H), 2.45-1.6 (m, 12H), 1.5-1.2 (m, 3H), 1.1-0.7 (m, 21H) [weitere Signale unter Lösungsmittel-Peaks verborgen].

### Intermediat 78

### N-(6-Aminohexyl)-N-methyl-L-valyl-N-[(3R,4S,5S)-3-methoxy-1-{(2S)-2-[(1R,2R)-1-methoxy-2-methyl-3-{[(2S)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid

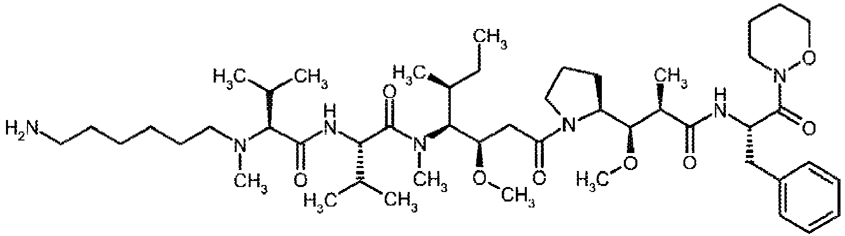

Diese Verbindung wurde in Analogie zu Intermediat 66 über 2 Stufen ausgehend von 20 mg (16 µmol) der Verbindung aus Intermediat 14 und Benzyl-(6-oxohexyl)carbamat hergestellt, wobei die Hydrierung in Methanol als Lösungsmittel durchgeführt wurde.
Ausbeute: 7.6 mg (55% d.Th. über 2 Stufen)
HPLC (Methode 6): Rₜ = 1.8 min;
LC-MS (Methode 1): Rₜ = 0.7 min; MS (ESIpos): m/z = 901 (M+H)⁺.

### Intermediat 79

### N-(3-Carboxypropyl)-N-methyl-L-valyl-N-[(3R,4S,5S)-1-{(2S)-2-[(1R,2R)-3-{[(2S)-1-(benzylamino)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

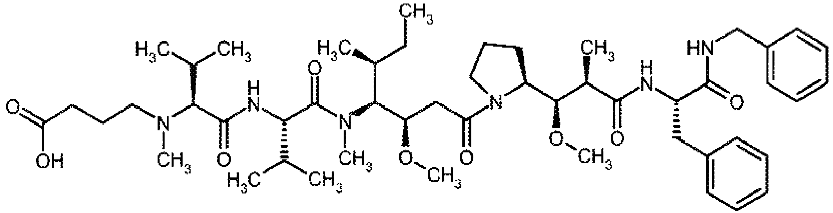

36 mg (43 µmοl) *N*-(*tert*.-Butoxycarbonyl)-*N*-methyl-L-valyl-*N*-[(3*R*,4*S*,5*S*)-1-{(2*S*)-2-[(1*R*,2*R*)-3-{[(1*S*)-1-carboxy-2-phenylethyl]amino}-1-methoxy-2-methyl-3-oxopropyl]pyrrolidin-1-yl}-3-methoxy-5-methyl-1-oxoheptan-4-yl]-*N*-methyl-L-valinamid (Intermediat 46) und 4.6 mg (43 µmol) Benzylamin wurden in 5 ml DMF aufgenommen, mit 7.5 µl (88 µmοl) *N,N*-Diisopropylethylamin, 10 mg (65 µmol) HOBt sowie 10 mg (52 µmol) EDC versetzt und dann über Nacht bei RT gerührt. Anschließend wurde das Reaktionsgemisch eingeengt und der Rückstand mittels präparativer HPLC gereinigt. Es wurden 29 mg (73% d. Th.) des Boc-geschützten Intermediats *N*-(*tert*.-Butoxycarbonyl)-*N*-methyl-L-valyl-*N*-[(3*R,*4*S*,5*S*)-1-{(2*S*)-2-[(1*R*,2*R*)-3-{[(2*S*)-1-(benzylamino)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid erhalten.
LC-MS (Methode 1): Rₜ = 1.43 min; m/z = 921 (M+H)⁺.

29 mg dieses Intermediats wurden zur Abspaltung der Boc-Schutzgruppe mit 1 ml Trifluoressigsäure in 6 ml Dichlormethan behandelt. Nach Einengen und Lyophilisation aus Dioxan/Wasser wurden 30 mg (quant.) des freien Amin-Intermediats *N*-Methyl-L-valyl-*N*-[(3*R*,4*S*,5*S*)-1-{(2*S*)-2-[(1*R*,2*R*)-3-{[(2*S*)-1-(benzylamino)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid als Trifluoracetat erhalten.
LC-MS (Methode 1): Rₜ = 0.95 min; m/z = 821 (M+H)⁺.

Aus 17 mg (0.018 mmol) dieses Intermediats wurden dann in Analogie zur Herstellung von Intermediat 61 durch Umsetzung mit 4-Oxobutansäure in Gegenwart von Natriumcyanoborhydrid 13 mg (80% d. Th.) der Titelverbindung in Form eines farblosen Schaums erhalten.
HPLC (Methode 5): Rₜ = 1.7 min;
LC-MS (Methode 9): Rₜ = 4.97 min; MS (ESIpos): m/z = 907 (M+H)⁺.

### Intermediat 80

### N-(3-Carboxypropyl)-N-methyl-L-valyl-N-[(3R,4S,5S)-1-{(2S)-2-[(1R,2R)-3-{[(2S)-1-(benzylamino)-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

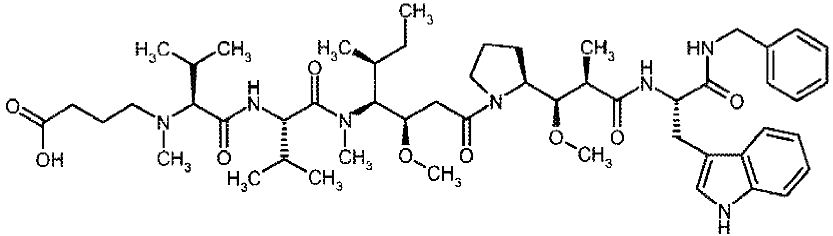

Zunächst wurde in Analogie zu der in Intermediat 74 beschriebenen Synthese durch Kupplung von *N*-(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 26) und *N*-Benzyl-L-tryptophanamid-Trifluoracetat (Intermediat 47) in Gegenwart von *O-*(7-Azabenzotriazol-1-yl)-*N,N*,*N',N'*-tetramethyluronium-hexafluorophosphat und anschließende Abspaltung der Boc-Schutzgruppe mittels Trifluoressigsäure die Amin-Verbindung *N*-Methyl-L-valyl-*N*-[(3*R*,4*S*,5*S*)-1-{(2*S*)-2-[(1*R*,2*R*)-3-{[(2*S*)-1-(benzylamino)-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid als Trifluoracetat hergestellt. Aus 10 mg (0.01 mmol) dieser Verbindung wurden dann in Analogie zur Herstellung von Intermediat 61 durch Umsetzung mit 4-Oxobutatisäure in Gegenwart von Natriumcyanoborhydrid 2.5 mg (26% d. Th.) der Titelverbindung erhalten.
HPLC (Methode 5): Rₜ = 1.7 min;
LC-MS (Methode 2): Rₜ = 1.13 min; MS (ESIpos): m/z = 946 (M+H)⁺.

### Intermediat 81

### N-(3-Carboxypropyl)-N-methyl-L-valyl-N-[(3R,4S,5S)-1-{(2S)-2-[(1R,2R)-3-{[(1S,2R)-1-carbamoyl-2-phenylcyclopropyl]amino}-1-methoxy-2-methyl-3-oxopropyl]pyrrolidin-1-yl}-3-methoxy-5-methyl-1-oxoheptan-4-yl]-N-methyl-L-valinamid

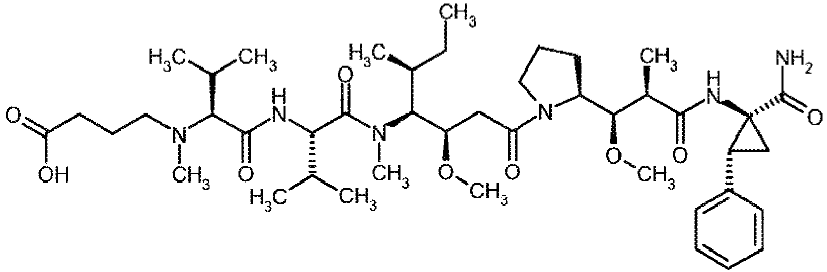

Zunächst wurde in Analogie zu der in Intermediat 74 beschriebenen Synthese durch Kupplung von *N*-(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 26) und (1*S*,2*R*)-1-Amino-2-phenylcyclopropancarboxamid-Trifluoracetat (Intermediat 48) in Gegenwart von *O*-(7-Azabenzotriazol-1-yl)-*N,N,N',N'*-tetramethyluronium-hexafluorophosphat und anschließende Abspaltung der Boc-Schutzgruppe mittels Trifluoressigsäure die Amin-Verbindung *N*-Methyl-L-valyl-*N*-[(3*R,*4*S*,5*S*)-1-{(2*S*)-2-[(1*R*,2*R*)-3-{[(1*S*,2*R*)-1-carbamoyl-2-phenylcyclopropyl]amino}-1-methoxy-2-methyl-3-oxopropyl]pyrrolidin-1-yl}-3-methoxy-5-methyl-1-oxoheptan-4-yl]-*N*-methyl-L-valinamid als Trifluoracetat hergestellt. Aus 14 mg (0.0163 mmol) dieser Verbindung wurden dann in Analogie zur Herstellung von Intermediat 61 durch Umsetzung mit 4-Oxobutansäure in Gegenwart von Natriumcyanoborhydrid 8 mg (57% d. Th.) der Titelverbindung erhalten.
HPLC (Methode 5): Rₜ = 1.6 min;
LC-MS (Methode 9): Rₜ = 4.64 min; MS (ESIpos): m/z = 829 (M+H)⁺.

### Intermediat 82

### N-(3-Carboxypropyl)-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

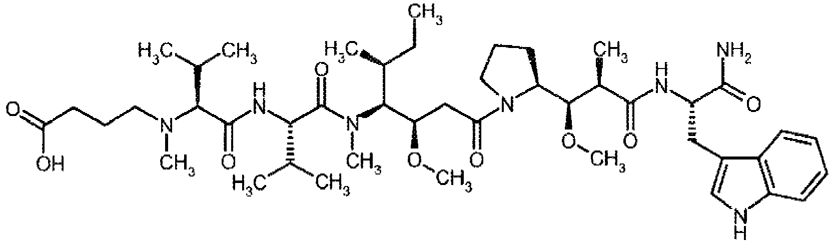

Zunächst wurde in Analogie zu der in den Intermediat 69 beschriebenen Synthese durch Kupplung von *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) und *N^{α}*-{(2*R*,3*R*)-3-Methoxy-2-methyl-3-[(2*S*)-pyrrolidin-2-yl]propanoyl}-L-tryptophanamid-Trifluoracetat (Intermediat 49) in Gegenwart von *O*-(7-Azabenzotriazol-1-yl)-*N,N,N',N'*-tetramethyluronium-hexafluorophosphat und anschließende Abspaltung der Fmoc-Schutzgruppe mittels Piperidin die Amin-Verbindung *N-*Methyl-L-valyl-*N-*[(3*R*,4*S*,5*S*)-1-{(2*S*)-2-[(1*R*,2*R*)-3-{[(2*S*)-1-amino-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid als Trifluoracetat hergestellt. Aus 78 mg (0.088 mmol) dieser Verbindung wurden dann in Analogie zur Herstellung von Intermediat 61 durch Umsetzung mit 4-Oxobutansäure in Gegenwart von Natriumcyanoborhydrid 68 mg (90% d. Th.) der Titelverbindung erhalten.
HPLC (Methode 5): Rₜ = 1.8 min;
LC-MS (Methode 9): Rₜ = 4.49 min; MS (ESIpos): m/z = 856 (M+H)⁺.

### Intermediat 83

### N-(5-Carboxypentyl)-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

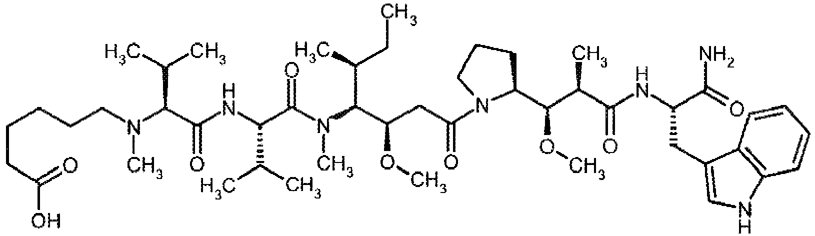

Diese Verbindung wurde in Analogie zu der Verbindung in Intermediat 82 hergestellt ausgehend von 20 mg (26 µmol) *N*-Methyl-L-valyl-*N*-[(3*R,*4*S*,5*S*)-1-{(2*S*)-2-[(1*R*,2*R*)-3-{[(2*S*)-1-amino-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid-Trifluoracetat durch Umsetzung mit 4-Oxobutansäure in Gegenwart von Natriumcyanoborhydrid hergestellt.
Ausbeute: 5 mg (25% d. Th.)
HPLC (Methode 5): Rₜ = 1.6 min,
LC-MS (Methode 11): Rₜ = 0.72 min; MS (ESIpos): m/z = 884 (M+H)⁺.

### Intermediat 84

### N-(3-Carboxypropyl)-N-methyl-L-valyl-N-[(3R,4S,5S)-3-methoxy-1-{(2S)-2-[(1R,2R)-1-methoxy-2-methyl-3-{[(2S)-1-(morpholin-4-yl)-1-oxo-3-phenylpropan-2-yl]amino}-3-oxopropyl]pyrrolidin-1-yl}-5-methyl-1-oxoheptan-4-yl]-N-methyl-L-vatinamid

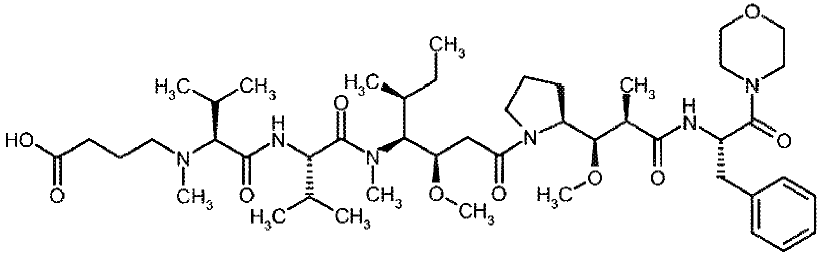

Zunächst wurde in Analogie zu der in intermediat 79 beschriebenen Synthese durch Kupplung von *N*-(*tert*.-Butoxycarbonyl)-*N*-methyl-L-valyl-*N*-[(3*R*,4*R*,5*S*)-1-{(2*S*)-2-[(1*R*,2*R*)-3-{[(1*S*)-1-carboxy-2-phenylethyl]amino}-1-methoxy-2-methyl-3-oxopropyl]pyrrolidin-1-yl}-3-methoxy-5-methyl-1-oxoheptan-4-yl]-*N*-methyl-L-valinamid (Intermediat 46) und Morpholin in Gegenwart von EDC und HOBT und anschließender Abspaltung der Boc-Schutzgruppe mittels Trifluoressigsäure die Amin-Verbindung *N*-Methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(morpholin-4-yl)-1-oxo-3-phenylpropan-2-yl]amino}-3-oxopropyl]pyrrolidin-1-yl}-5-methyl-1-oxoheptan-4-yl]-*N-*methyl-L-valinamid als Trifluoracetat hergestellt. Aus 30 mg (0.033 mmol) dieser Verbindung wurden dann in Analogie zur Herstellung von Intermediat 61 durch Umsetzung mit 4-Oxobutansäure in Gegenwart von Natriumcyanoborhydrid 22 mg (76% d. Th.) der Titelverbindung erhalten.
HPLC (Methode 5): Rₜ = 1.6 min;
LC-MS (Methode 9): Rₜ = 4.58 min; MS (ESIpos): m/z = 887 (M+H)⁺.

### Intermediat 85

### N-(3-Carboxypropyl)-N-methyl-L-valyl-N-[(3R,4S,5S)-1-{(2S)-2-[(1R,2R)-3-{[(2S,3R)-1-(benzylamino)-3-hydroxy-1-oxobutan-2-yl]amino}-1-methoxy-2-methyl-3-oxopropyl]pyrrolidin-1-yl}-3-methoxy-5-methyl-1-oxoheptan-4-yl]-N-methyl-L-valinamid

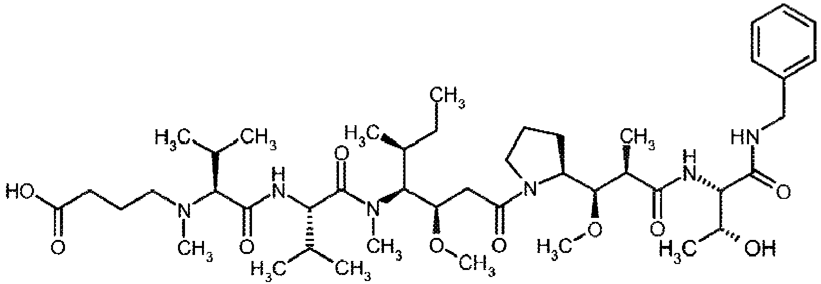

Zunächst wurde in Analogie zu der in Intermediat 79 beschriebenen Synthese durch Kupplung von *N*-(*tert*.-Butoxycarbonyl)-*N*-methyl-L-valyl-*N*-[(3*R*,4*S*,5*S*)-1-{(2*S*)-2-[(1*R*,2*R*)-3-{[(1*S*)-1-carboxy-2-phenylethyl]amino}-1-methoxy-2-methyl-3-oxopropyl]pyrrolidin-1-yl}-3-methoxy-5-methyl-1-oxoheptan-4-yl]-*N*-methyl-L-valinamid (Intermediat 46) und *N*-Benzyl-L-threoninamid-Trifluoracetat in Gegenwart von HATU und anschließender Abspaltung der Boc-Schutzgruppe mittels Trifluoressigsäure die Amin-Verbindung *N*-Methyl-L-valyl-*N*-[(3*R*,4*S*,5*S*)-1-{(2*S*)-2-[(1*R*,2*R*)-3-1[(2*S*,3*R*)-1-(benzylamino)-3-hydroxy-1-oxobutan-2-yl]amino}-1-methoxy-2-methyl-3-oxopropyl]pyrrolidin-1-yl}-3-methoxy-5-methyl-1-oxoheptan-4-yl]-*N*-methyl-L-valinamid als Trifluoracetat hergestellt. Aus 21 mg (0.024 mmol) dieser Verbindung wurden dann in Analogie zur Herstellung von Intermediat 61 durch Umsetzung mit 4-Oxobutansäure in Gegenwart von Natriumcyanoborhydrid 20 mg (97% d. Th.) der Titelverbindung erhalten.
HPLC (Methode 5): Rₜ = 1.54 min;
LC-MS (Methode 9): Rₜ = 4.49 min; MS (ESIpos): m/z = 861 (M+H)⁺.

### Intermediat 86

### N-(3-Carboxypropyl)-N-methyl-L-valyl-N-[(3R,4S,5S)-1-{(2S)-2-[(1R,2R)-3-{[(2S)-1-tert-butoxy-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

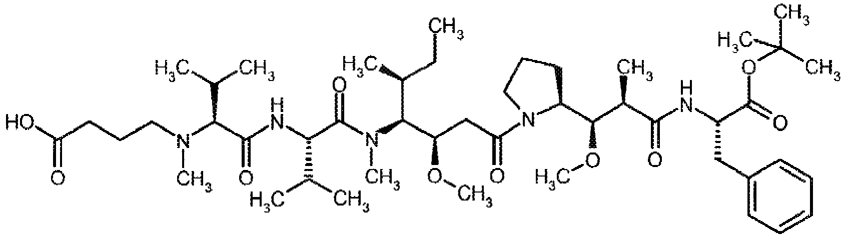

Zunächst wurde in Analogie zu der in Inteemediat 74 beschriebenen Synthese durch Kupplung von *N*-(*tert.*-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 26) und *tert*.-Butyl-L-phenylalaninat-hydrochlorid in Gegenwart von *O*-(7-Azabenzotriazol-1-yl)-*N,N,N',N'*-tetramethyluronium-hexafluorophosphat und anschließende Abspaltung der Boc-Schutzgruppe mittels Trifluoressigsäure unter Erhalt des *tert*.-Butylesters (40 min Rühren mit Trifluoressigsäure in Dichlormethan) die Amin-Verbindung *N*-Methyl-L-valyl-*N-*[(3*R,*4*S,*5*S*)*-*1*-*{(2*S*)-2-[(1*R*,2*R*)-3-{[(2*S*)-1-*tert*.-butoxy-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N-*methyl-L-valinamid als Trifluoracetat hergestellt. Aus 22 mg (0.02 mmol) dieser Verbindung wurden dann in Analogie zur Herstellung von Intermediat 61 durch Umsetzung mit 4-Oxobutansäure in Gegenwart von Natriumcyanoborhydrid 16 mg (94% d. Th.) der Titelverbindung erhalten.
HPLC (Methode 5): Rₜ = 2.0 min;
LC-MS (Methode 9): Rₜ = 5.05 min; MS (ESIpos): m/z = 874 (M+H)⁺.

### Intermediat 87

### N-(3-Carboxypropyl)-N-methyl-L-valyl-N-[(3R,4S,5S)-1-{(2S)-2-[(1R,2R)-3-{[(2S)-1-tert-butoxy-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

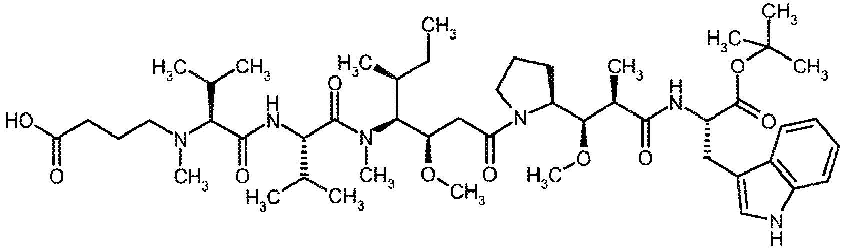

Die Herstellung dieser Verbindung erfolgte in Analogie zur in Intermediat 86 beschriebenen Synthese ausgehend von 230 mg (336 µmol) *N*-(*tert*.-Butoxycarbonyl)-*N*-methyl-L-valyl-N-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 26) und *tert*.-Butyl-L-tryptophanat-Hydrochlorid über 3 Stufen.
Ausbeute: 95 mg (31% d.Th. über 3 Stufen)
IIPLC (Methode 5): Rₜ = 2.0 min;
LC-MS (Methode 9): Rₜ = 5.05 min; MS (ESIpos): m/z = 913 (M+H)⁺.

### Intermediat 88

### N-(6-Aminohexyl)-N-methyl-L-valyl-N-[(3R,45,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

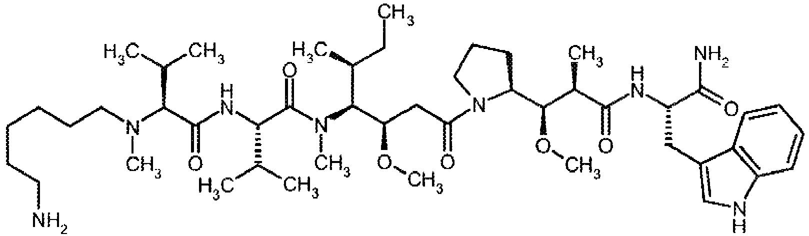

Zunächst wurde in Analogie zu der in den Intermediat 69 beschriebenen Synthesen durch Kupplung von *N*-[(9H-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) und *N^{α}*-{(2*R*,3*R*)-3-Methoxy-2-methyl-3-[(2*S*)-pyrrolidin-2-yl]propanoyl}-L-tryptophanamid-Trifluoracetat (Intermediat 49) in Gegenwart von *O*-(7-Azabenzotriazol-1-yl)-*N,N,N',N'*-tetramethyluronium-hexafluorophosphat und anschließende Abspaltung der Fmoc-Schutzgruppe mittels Piperidin die Amin-Verbindung *N*-Methyl-L-valyl-*N*-[(3*R*,4*S*,5*S*)-1-{(2*S*)-2-[(1*R*,2*R*)-3-{[(2*S*)-1-amino-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid als Trifluoracetat hergestellt. Aus 30 mg (0.03 mmol) dieser Verbindung wurden dann in Analogie zur Herstellung der Verbindung von Intermediat 61 durch Umsetzung mit Benzyl-(6-oxohexyl)carbamat, das vorher durch Oxidation von Benzyl-(6-hydroxyhexyl)carbamat gewonnen wurde, in Gegenwart von Natriumcyanoborhydrid 17 mg (45% d. Th.) der Z-geschützten Verbindung erhalten. Anschließend wurde durch Hydrogenolyse in Methanol über 10% Palladium/Aktivkohle die Titelverbindung erhalten.
Ausbeute: 14 mg (95% d. Th.)
HPLC (Methode 5): Rₜ = 1.5 min;
LC-MS (Methode 1): Rₜ = 0.73 min; MS (ESIpos): m/z = 869 (M+H)⁺.

### Intermediat 89

### N-(6-Aminohexyl)-N-methyl-L-valyl-N-[(3R,4S,5S)-1-{(2S)-2-[(1R,2R)-3-{[(2S)-1-tert.-butoxy-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

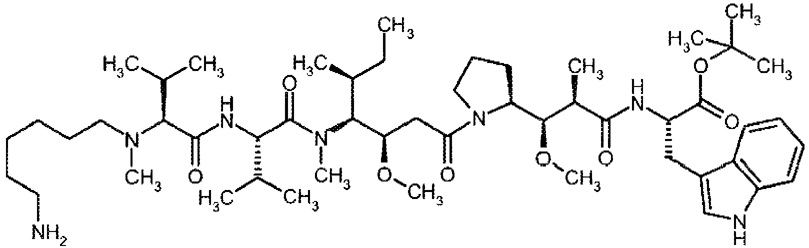

Zunächst wurde in Analogie zu der in Intermediat 86 beschriebenen Synthese durch Kupplung von *N*-(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 26) und *tert*.-Butyl-L-tryptophanat-hydrochlorid in Gegenwart von *O*-(7-Azabenzotriaxol-1-yl)-*N,N,N',N'*-tetramethyluronium-hexafluorophosphat und anschließende Abspaltung der Boc-Schulzgruppe mittels Trifluoressigsäure unter Erhalt des *tert*.-Butylesters (30 min Rühren mit Trifluoressigsäure/Dichlormethan. 1:10) die Amin-Verbindung *N*-Methyl-L-valyl-*N*-[(3*R*,4*S*,5*S*)-1-{(2*S*)-2-[(1*R*,2*R*)-3-{[(2*S*)-1-*tert*.-butoxy-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid als Trifluoracetat hergestellt. Aus 71 mg (0.075 mmol) dieser Verbindung wurden dann in Analogie zur Herstellung der Verbindung von Intermediat 61 durch Umsetzung mit Benzyl-(6-oxohexyl)carbamat, das vorher durch Oxidation von Benzyl-(6-hydroxyhexyl)carbamat gewonnen wurde, in Gegenwart von Natriumcyanoborhydrid 35 mg (44% d. Th.) der Z-geschützten Verbindung erhalten. Anschließend wurde durch Hydrogenolyse in Methanol über 10% Palladium/Aktivkohle die Titelverbindung erhalten.
Ausbeute: 30 mg (98% d. Th.)
HPLC (Methode 5): Rt = 1.9 min;
LC-MS (Methode 1): Rt = 0.77 min; MS (ESIpos): m/z = 926 (M+H)⁺.

### Intermediat 90

### N-(3-Carboxypropyl)-N-methyl-L-valyl-N-[(3R,4S,5S)-1-{(2S)-2-[(1R,2R)-3-{[2-(1H-indol-3-yl)ethyl]amino}-1-methoxy-2-methyl-3-oxopropyl]pyrrolidin-1-yl}-3-methoxy-5-methyl-1-oxoheptan-4-yl]-N-methyl-L-valinamid

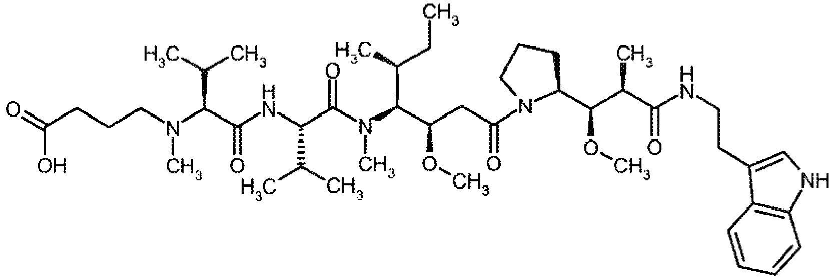

Zunächst wurde in Analogie zu der in Intermediat 74 beschriebenen Synthese durch Kupplung von *N*-(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 26) und 2-(1*H*-Indol-3-yl)ethanamin in Gegenwart von *O*-(7-Azabenzotriazol-1-yl)-*N,N,N',N'*-tetramethyluronium-hexafluorophosphat und anschließende Abspaltung der Boc-Schutzgruppe mittels Trifluoressigsäure die Amin-Verbindung *N*-Methyl-L-valyl-*N*-[(3*R*,4*S*,5*S*)-1-{(2*S*)-2-[(1*R*,2*R*)-3-{[2-(1*H*-indol-3-yl)ethyl]amino}-1-methoxy-2-methyl-3-oxopropyl]pyrrolidin-1-yl}-3-methoxy-5-methyl-1-oxoheptan-4-yl]-*N*-methyl-L-valinamid als Trifluoracetat hergestellt. Aus 100 mg (0.119 mmol) dieser Verbindung wurden dann in Analogie zur Herstellung von Intermediat 61 durch Umsetzung mit 4-Oxobutansäure in Gegenwart von Natriumcyanoborhydrid 50 mg (49% d. Th.) der Titelverbindung erhalten. Die Reinigung der Titelverbindung erfolgte hier durch Flash-Chromatographie an Kieselgel mit Dichlormethan/Methanol/Ammoniak 17% als Eluent, wobei das Mischungsverhältnis von zunächst 15/2/02 auf 15/4/0.5 umgestellt wurde.
HPLC (Methode 6): Rₜ = 1.8 min;
LC-MS (Methode 1): Rₜ = 0.87 min; MS (ESIpos): m/z = 813 (M⁺H)⁺.

### Intermediat 91

### N-(3-Carboxypropyl)-N-methyl-L-valyl-N-{(3R,4S,5S)-3-methoxy-1-[(2S)-2-{(1R,2R)-1-methoxy-2-methyl-3-oxo-3-[(2-phenylethyl)amino]propyl}pyrrolidin-1-yl]-5-methyl-1-oxoheptan-4-yl}-N-methyl-L-valinamid

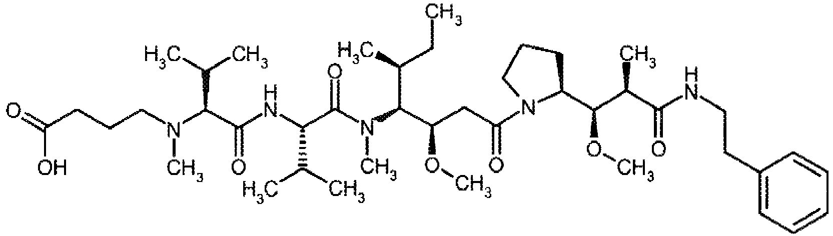

Zunächst wurde in Analogie zu der in Intermediat 74 beschriebenen Synthese durch Kupplung von *N*-(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 26) und Phenylethylamin in Gegenwart von *O*-(7-Azabenzotriazol-1-yl)-*N,N,N',N'-*tetramethyluronium-hexafluorophosphat und anschließende Abspaltung der Boc-Schutzgruppe mittels Trifluoressigsäure die Amin-Verbindung *N*-Methyl-L-valyl-*N*-{(3*R*,4*S*,5*S*)-3-methoxy-1-[(2*S*)-2-{(1*R*,2*R*)-1-methoxy-2-methyl-3-oxo-3-[(2-phenylethyl)amino]propyl}pyrrolidin-1-yl]-5-methyl-1-oxoheptan-4-yl}-*N*'-methyl-L-valinamid als Trifluoracetat hergestellt. Aus 57 mg (0.071 mmol) dieser Verbindung wurden dann in Analogie zur Herstellung von Intermediat 61 durch Umsetzung mit 4-Oxobutansäure in Gegenwart von Natriumcyanoborhydrid 44 mg (80% d. Th.) der Titelverbindung erhalten. Die Reinigung der Titelverbindung kann auch hier durch Flash-Chromatographie an Kieselgel mit Dichlormethan/Methanol/Ammoniak 17% als Eluent erfolgen (15/2/02 -> 15/4/0.5).
HPLC (Methode 5): Rₜ = 1.7 min;
LC-MS (Methode 9): Rₜ = 4.64 min; MS (ESIpos): m/z = 774 (M+H)⁺.

### Intermediat 92

### N-(3-Carboxypropyl)-N-methyl-L-valyl-N-[(3R,4S,5S)-1-{(2S)-2-[(1R,2R)-3-{[(1S,2R)-1-hydroxy-1-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

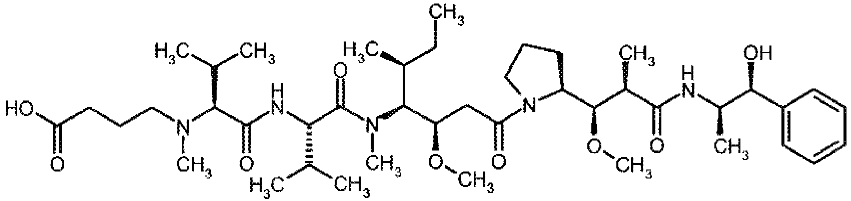

Aus 100 mg (0.139 mmol) *N*-Methyl-L-valyl-*N*-[(3*R*,4*S*,5*S*)-1-{(2*S*)-2-[(*1R*,2*R*)-3-{(1*S*,2*R*)-1-hydroxy-1-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid (Intermediat 40) wurden in Analogie zur Herstellung von Intermediat 61 durch Umsetzung mit 4-Oxobutansäure in Gegenwart von Natriumcyanoborhydrid 94 mg (84% d. Th.) der Titelverbindung erhalten. Die Reinigung der Titelverbindung erfolgte durch präparative HPLC.
HPLC (Methode 5): Rₜ = 1.5 min;
LC-MS (Methode 9): Rₜ = 4.46 min; MS (ESIpos): m/z = 804 (M+H)⁺.

### Intermediat 93

### N-(3-Carboxypropyl)-N-methyl-L-valyl-N-[(3R,4S,5S)-3-methoxy-1-{(2S)-2-[(1R,2R)-1-methoxy-2-methyl-3-oxo-3-{[(1S)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-L-valinamid

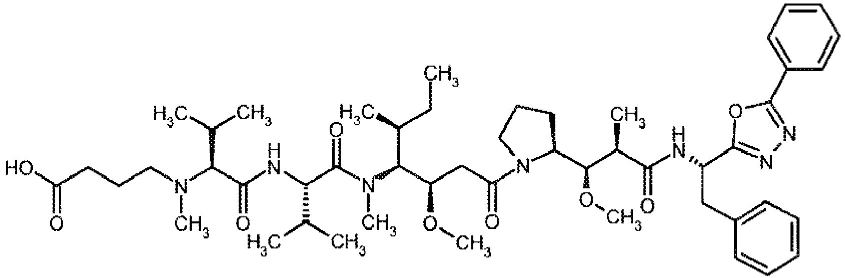

22.4 mg (24 µmol) *N*-Methyl-L-valyl-*N*-[(3*R*,4*S*,5*S*)-3-methoxy-1-{(2*S*)-2-[(1*R*,2*R*)-1-methoxy-2-methyl-3-oxo-3-{[(1*S*)-2-phenyl-1-(5-phenyl-1,3,4-oxadiazol-2-yl)ethyl]amino}propyl]pyrrolidin-l-yl}-5-methyl-1-oxoheptan-4-yl]-*N*-methyl-L-valinamid-Trifluoracetat wurden in 1.4 ml Dioxan/Wasser gelöst und analog zur Herstellung von Intermediat 61 mit 15%-iger wässriger Lösung von 4-Oxobutansäure in Gegenwart von Natriumcyanoborhydrid umgesetzt. Nach Lyophilisaton aus Dioxan wurden 8.2 mg (38% d. Th.) der Titelverbindung in Form eines weißen Feststoffs erhalten.
HPLC (Methode 10): Rₜ = 2.54 min
LC-MS (Methode 12): Rₜ = 0.94 min; MS (ESIpos): m/z = 919 (M+H)⁺

### Intermediat 94

### N-(3-Carboxypropyl)-N-methyl-L-valyl-N-[(3R,4S,5S)-3-methoxy-1-{(2S)-2-[(1R,2R)-1-methoxy-2-methyl-3-oxo-3-{[(1R)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-L-valinamid

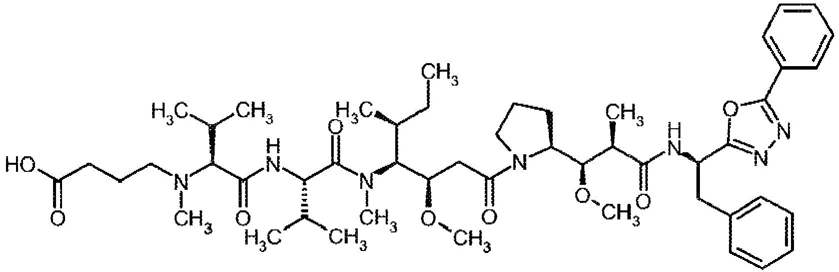

17.1 mg (18 µmol) *N*-Methyl-L-valyl-*N*-[(3*R*,4*S*,5*S*)-3-methoxy-1-{(2*S*)-2-[(1*R*,2*R*)-1-methoxy-2-methyl-3-oxo-3-{[(1*R*)-2-phenyl-1-(5-phenyl-1,3,4-oxadiazol-2-yl)ethyl]amino}propyl]pyrrolidin-1-yl}-5-methyl-1-oxoheptan-4-yl]-*N*-methyl-L-valinamid-Trifluoracetat wurden in 1.1 ml Dioxan/Wasser gelöst und analog zur Herstellung von Intermediat 61 mit 15%-iger wässriger Lösung von 4-Oxobutansäure in Gegenwart von Natriumcyanoborhydrid umgesetzt. Nach Lyophilisation aus Dioxan wurden 14.8 mg (89% d. Th.) der Titelverbindung in Form eines weißen Feststoffs erhalten.
HPLC (Methode 10): Rₜ = 2.54 min;
LC-MS (Methode 12): Rₜ = 0.92 min; MS (ESIpos): m/z = 919 (M+H)⁺

### Intermediat 95

### N-(3-Carboxypropyl)-N-methyl-L-valyl-N-[(3R,4S,5S)-1-{(2S)-2-[(1R,2R)-3-{[(2S)-1-(benzylsulfonyl)-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

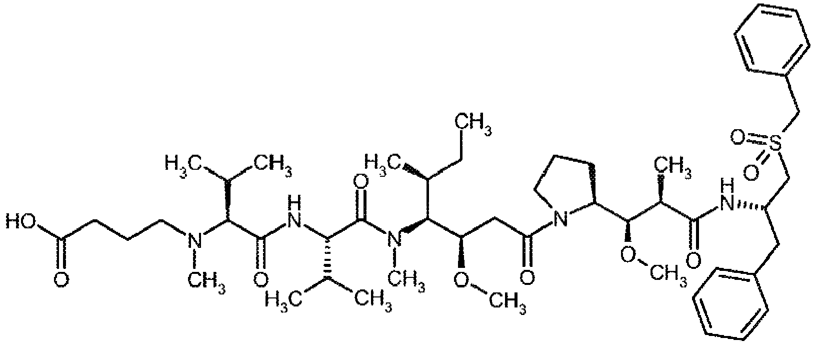

19.3 mg (20 µmol) *N*-Methyl-L-valyl-*N*-[(3*R,*4*S*,5*S*)-1-{(2*S*)-2-[1*R*,2*R*}-3-{[(2*S*)-1-(benzylsulfonyl)-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid-Trifluoracetat wurden in 1.2 ml Dioxan/Wasser gelöst und analog zur Herstellung von Intermediat 61 mit 15%-iger wässriger Lösung von 4-Oxobutansäure in Gegenwart von Natriumcyanoborhydrid umgesetzt. Nach Lyophilisation aus Dioxan wurden 8.6 mg (45% d. Th.) der Titelverbindung in Form eines Feststoffs erhalten.
LC-MS (Methode 11): Rₜ = 0.85 min; MS (ESIpos): m/z = 943 (M+H)⁺

### Intermediat 96

### N-(3-Carboxypropyl)-N-methyl-L-valyl-N-[(3R,4S,5S)-1-{(2S)-2-[(1R,2R)-3-{[(2S,3E)-1,4-diphenylbut-3-en-2-yl]amino}-1-metboxy-2-methyl-3-oxopropyl]pyrrolidin-1-yl}-3-methoxy-5-methyl-1-oxoheptan-4-yl]-N-methyl-L-valinamid

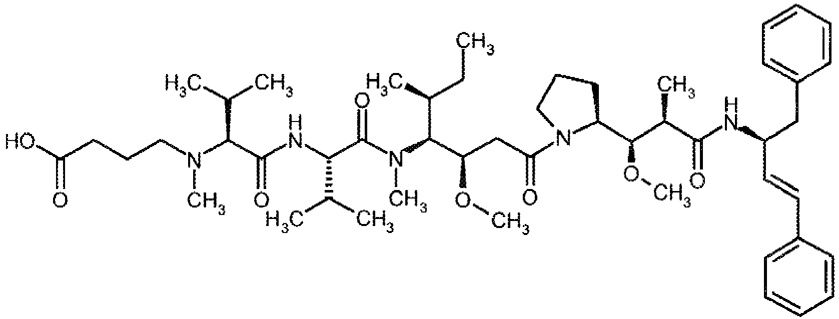

15.5 mg (10 µmol) *N*-Methyl-L-valyl-*N*-[(3*R*,4*S*,5*S*)-1-{(2*S*)-2-[(1*R*,2*R*)-3-{[(2*S*,3*E*)-1,4-diphenyl-but-3-en-2-yl]amino}-1-methoxy-2-methyl-3-oxopropyl]pyrrolidin-1-yl}-3-methoxy-5-methyl-1-oxoheptan-4-yl]-*N*-methyl-L-valinamid-Trifluoracetat wurden in 1.0 ml Dioxan/Wasser gelöst und analog zur Herstellung von Intermediat 61 mit 15%-iger wässriger Lösung von 4-Oxobutansäure in Gegenwart von Natriumcyanoborhydrid umgesetzt. Nach Lyophilisaton aus Dioxan wurden 10.3 mg (68% d. Th.) der Titelverbindung in Form eines weißen Feststoffs erhalten.
HPLC (Methode 10): Rₜ = 2.59 min;
LC-MS (Methode 11): Rₜ = 0.94 min; MS (ESIpos): m/z = 877 (M+H)⁺

### Intermediat 97

### N-(6-Aminohexyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

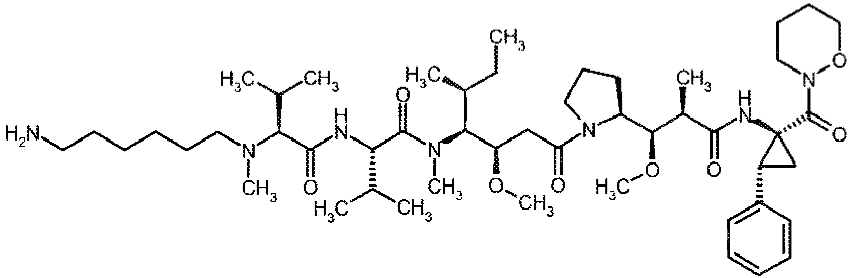

Die Titelverbindung wurde in Analogie zur Synthese von Intermediat 66 durch Umsetzung von 200 mg (0.108 mmol) *N*-Methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl]amino}-3-oxopropyl]-pyrrolidin-1-yl}-5-methyl-1-oxoheptan-4-yl]-*N*-methyl-L-valinamid-Trifluoracetat (Intermediat 16) mit Benzyl-(6-oxohexyl)carbamat und nachfolgende hydrogenolytische Abspaltung der Z-Schutzgruppe (mit 5% Palladium auf Kohle als Katalysator, in Methanol als Lösungsmittel) hergestellt.
Ausbeute: 69 mg (65% d. Th. über zwei Stufen)
HPLC (Methode 5): Rₜ = 1.7 min;
LC-MS (Methode I): Rₜ = 0.76 min; MS (ESIpos): m/z = 912 (Mh-H)⁺.

### Intermediat 98

### N-(5-Carboxypentyl)-N-methyl-L-valyl-N-[(3R,4S,5S)-1-{(2S)-2-[(1R,2R)-3-{[(2S)-1-(benzylamino)-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

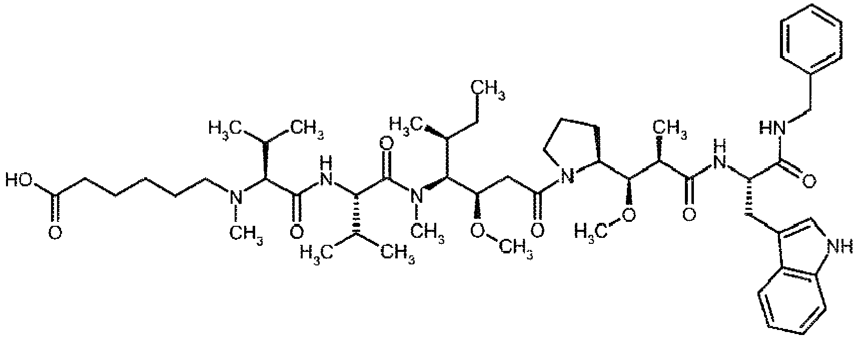

Die Herstellung dieser Verbindung erfolgte in Analogie zur in Intermediat 80 beschriebenen Synthese. Die Reinigung erfolgte durch präparative HPLC.
Ausbeute: 40 mg (29% d.Th. über 3 Stufen)
HPLC (Methode 5): Rₜ = 1.9 min;
LC-MS (Methode 1): Rₜ = 0.92 min; MS (ESIpos): m/z = 974 (M+H)⁺.

### Intermediat 99

### (2S)-2-Amino-3-(1H-indol-3-yl)-1-(1,2-oxazinan-2-yl)propan-1-on-Trifluoracetat

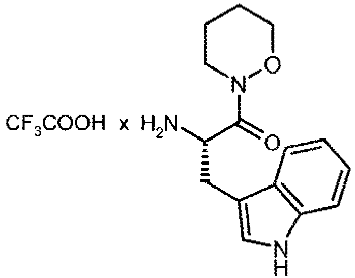

324 mg (0.81 mmol) 2,5-Dioxopyrrolidin-1-yl-*N*-(*tert*.-butoxycarbonyl)-L-tryptophanat wurden in 20 ml DMF gelöst und mit 200 mg (1.62 mmol) 1,2-Oxazinan-Hydrochlorid (Ausgangsverbindung 5) und 850 µl *N,N*-Diisopropylethylamin versetzt. Das Reaktionsgemisch wurde über Nacht bei 50°C gerührt und anschließend im Vakuum eingeengt. Der Rückstand wurde in Dichlormethan aufgenommen und mit Wasser extrahiert. Die organische Phase wurde über Magnesiumsulfat getrocknet und eingeengt. Der Rückstand wurde durch Flash-Chromatographie an Kieselgel mit Dichlormethan/Ethylacetat 4:1 als Laufmittel gereinigt. Die Produktfraktionen wurden eingeengt und der Rückstand im Hochvakuum getrocknet. Man erhielt so 147.5 mg (48% d. Th.) des Boc-geschützten Intermediats.
HPLC (Methode 12): Rₜ = 1.9 min;
LC-MS (Methode 1): Rₜ = 1.03 min; MS (ESIpos): m/z = 374 (M+H)⁺.

Aus 166 mg (444.5 µmol) dieses Intermediats wurde nach Standardbedingungen mit 3 ml Trifluoressigsäure in 20 ml Dichlormethan die Boc-Schutzgruppe abgespalten und nach HPLC Reinigung 155 mg (86% d.Th.) der Titelverbindung erhalten.
HPLC (Methode 12): Rₜ = 1.43 min;
LC-MS (Methode 11): Rₜ = 0.56 min; MS (ESIpos): m/z = 274 (M+H)⁺.

### Intermediat 100

### N-(6-{[(Benzyloxy)carbonyl]amino}hexyl)-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

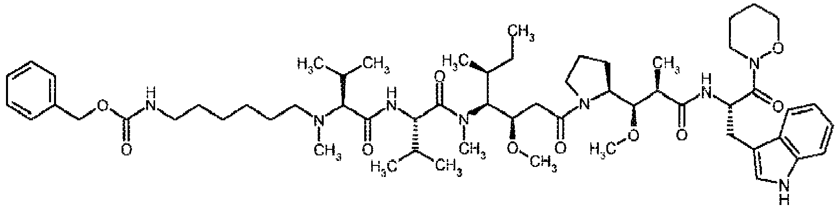

177 mg (260 µmol) *N*-(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 26) und 100 mg (260 µmol) (2*S*)-2-Amino-3-(1*H*-indol-3-yl)-1-(1,2-oxazinan-2-yl)propan-1-on-Trifluoracetat (Intermediat 99) wurden in 15 ml DMF aufgenommen und mit 118 mg (310 µmol) *O*-(7-Azabenzotriazol-1-yl)-*N*,*N,N'*,*N*'-tetramethyluronium-hexafluorophosphat und 140 µl *N,N*-Diisopropylethylamin versetzt. Das Reaktionsgemisch wurde 30 min bei RT gerührt, anschließend im Vakuum eingeengt und der Rückstand mittels präparativer HPLC aufgereinigi. Die Produktfraktionen wurden vereinigt und eingeengt. Nach Lyophilisaton aus Dioxan wurden 170 mg (68% d. Th.) des Boc-geschützten Intermediats erhalten.
LC-MS (Methode 1): Rₜ = 1.36 min; m/z = 940 (M+H)⁺.

170 mg dieses Intermediats wurden zur Abspaltung der Boc-Schutzgruppe 30 min mit 3 ml Trifluoressigsäure in 30 ml Dichlormethan behandelt. Dann wurde das Reaktionsgemisch im Vakuum eingeengt und der Rückstand mittels präparativer HPLC gereinigt, wobei 155 mg (86% d.Th.) des entschützten Intermediats N-Methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid erhalten wurden.
HPLC (Methode 12): Rₜ = 1.85 min;
LC-MS (Methode 1): Rₜ = 0.86 min; MS (ESIpos): m/z = 840 (M+H)⁺.

Aus 50 mg (0.052 mmol) dieses Intermediats wurden dann in Analogie zur Herstellung von Intermediat 97 mit Benzyl-(6-oxohexyl)carbamat in Gegenwart von Natriumcyanoborhydrid und nachfolgender hydrogenolytischer Abspaltung der Z-Schutzgruppe (mit 5% Palladium auf Kohle als Katalysator, in Methanol als Lösungsmittel) hergestellt die Titelverbindung hergestellt.
Ausbeute: 21 mg (37% d. Th.)
HPLC (Methode 12): Rₜ - 2.1 min;
LC-MS (Methode 1): Rₜ - 1.02 min; MS (ESIpos): m/z = 1073 (M+H)⁺.

### Intermediat 101

### N-(6-Aminohexyl)-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

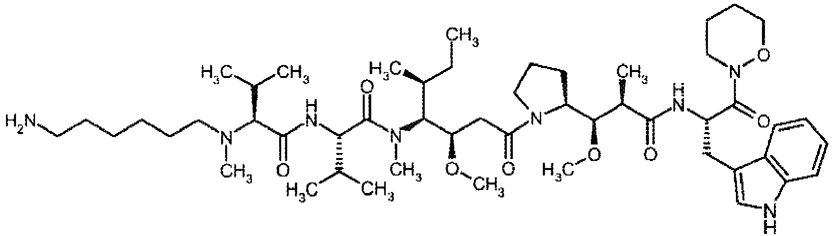

26.7 mg (24.87 µmol) von Intermediat 100 wurden in 10 ml Methanol gelöst und über Palladium/Aktivkohle (5%) 30 min bei Wasserstoff-Normaldruck hydriert. Der Katalysator wurde abfiltriert und das Lösungsmittel im Vakuum abgedampft. Nach Trocknung des Rückstands im Hochvakuum wurden 22.5 mg (96% d. Th.) der Titelverbindung erhalten.
HPLC (Methode 5): Rₜ = 1.7 min;
LC-MS (Methode 1): Rₜ = 0.76 min; MS (ESIpos): m/z = 939 (M+H)⁺.

### Intermediat 102

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-3-methoxy-1-{(2S)-2-[(1R,2R)-1-methoxy-2-methyl-3-{[(2S)-1-(morpholin-4-yl)-1-oxo-3-phenypropan-2-yl]amino}-3-oxopropyl]pyrrolidin-1-yl}-5-methyl-1-oxoheptan-4-yl]-N-methyl-L-valinamid

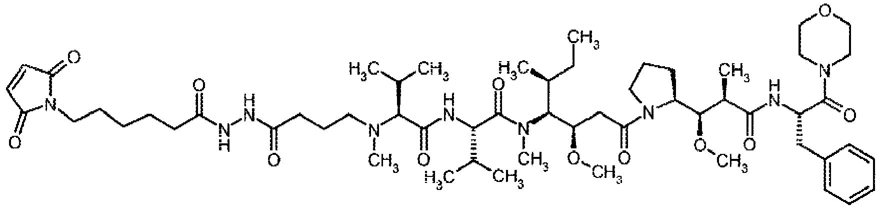

Diese Verbindung wurde in Analogie zur in Intermediat 157 beschriebenen Synthese aus *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(morpholin-4-yl)-1-oxo-3-phenylpropan-2-yl]amino}-3-oxopropyl]pyrrolidin-1-yl}-5-methyl-1-oxoheptan-4-yl]-*N*-methyl-L-valinamid und kommerziell erhältlichem 6-(2,5-Dioxo-2,5-dihydro-1*H*-pyrrol-1-yl)hexanhydrazid hergestellt.
Ausbeute: 8 mg (71% d.Th.)
HPLC (Methode 12): Rₜ = 1.9 min;
LC-MS (Methode 1): Rₜ = 0.87 min; MS (ESIpos): m/z = 1094 (M+H)⁺.

### Intermediat 103

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(2S,3R)-1-(benzylamino)-3-hydroxy-1-oxobutan-2-yl]amino}-1-methoxy-2-methyl-3-oxopropyl]pyrrolidin-1-yl}-3-methoxy-5-methyl-1-oxoheptan-4-yl]-N-methyl-L-valinamid

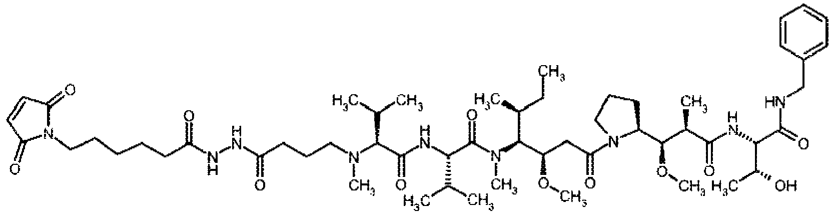

Diese Verbindung wurde in Analogie zur in Intermediat 157 beschriebenen Synthese aus *N*-(3-Carboxypropyl)-*N*-methyl-L-valyl-*N*-[(3*R*,4*S*,5*S*)-1-{(2*S*)-2-[(1*R*,2*R*)-3-{[(2*S*,3*R*)-1-(benzyl-amino)-3-hydroxy-1-oxobutan-2-yl]amino}-1-methoxy-2-methyl-3-oxopropyl]pyrrolidin-1-yl}-3-methoxy-5-methyl-1-oxoheptan-4-yl]-*N*-methyl-L-valinamid und kommerziell erhältlichem 6-(2,5-Dioxo-2,5-dihydro-1*H*-pyrrol-1-yl)hexanhydrazid hergestellt.
Ausbeute: 3 mg (22% d.Th.)
IIPLC (Methode 5): Rₜ = 1.6 min;
LC-MS (Methode 1): Rₜ = 0.78 min; MS (ESIpos): m/z = 1069 (M+H)⁺.

### Intermediat 104

### N-{4-[(trans-4-{[(2,5-Dioxopyrrolidin-1-yl)oxy]carbonyl}cyclohexyl)amino]-4-oxobutyl}-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

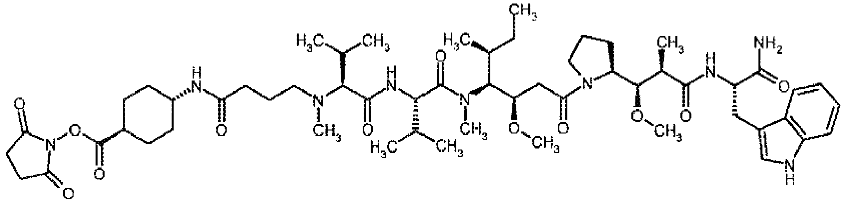

Zunächst wurde Benzyl-trans-4-aminocyclohexancarboxylat-Trifluoracetat aus *trans*-4-Aminocyclohexancarbonsäure durch Einführung der Boc-Schutzgruppe, anschließende Einführung der Benzylesterschutzgruppe und nachfolgende Abspaltung der Boc-Schutzgruppe nach klassischen Methoden der Peptidchemie hergestellt.

15 mg (18 µmol) *N*-(3-Carboxypropyl)-*N*-methyl-L-valyl-*N*-[(3*R*,4*S*,5*S*)-1-{(2*S*)-2-[(1*R*,2*R*)-3-{[(2*S*)-1-amino-3-(1*H*-indol-3-yl)-1-oxopropan-2-yl]amino}-1-methoxy-2-methyl-3-oxopropyl] pyrrolidin-1-y}-3-methoxy-5-methyl-1-oxoheptan-4-yl]-*N*-methyl-L-valinamid wurden dann in 5 ml Dimethylformamid gelöst und anschließend mit 13 mg (35 µmol) *O*-(7-Azabenzotriazol-1-yl)-*N,N,N',N'*-tetramethyluronium-hexafluorophosphat, 9 µL *N,N*-Diisopropylethylamin sowie mit 15 mg (44 µmol) Benzyl-*trans*-4-aminocyclohexancarboxylat-Trifluoracetat versetzt. Die Mischung wurde 1 h bei RT gerührt und anschließend im Vakuum eingeengt. Der verbliebene Rückstand wurde mittels präparativer HPLC aufgereinigt. Die entsprechenden Fraktionen wurden vereinigt und das Lösungsmittels im Vakuum abgedampft. Nach Trocknung des Rückstands im Hochvakuum wurden 14.7 mg (78% d. Th.) des geschützten Intermediats als farbloser Schaum erhalten.
HPLC (Methode 6): Rₜ = 2.0 min;
LC-MS (Methode 1): Rₜ = 0.95 min; MS (ESIpos): m/z = 1072 (M+H)⁺.

Von diesem geschützten Intermediat wurde zunächst hydrogenolytisch der Benzylester entfernt und die freie Carboxylkomponente in quantitativer Ausbeute erhalten. 14 mg (14 µmol; 1 Äquiv.) der entschützten Verbindung wurden in 5 ml DMF aufgenommen und mit 3.3 mg (29 µmol; 2.1 Äquiv.) *N*-Hydroxysuccinimid in Gegenwart von 4.1 mg (21 µmol; 1.5 Äquiv.) 1-(3-Dimethylaminopropyl)-3-ethylcarbodiimid-Hydrochlorid, 7.5 µL (44 µmol; 3.1 Äquiv.) *N,N-*Diisopropylethylamin und 0.9 mg (7 µmol; 0.5 Äquiv.) 4-Dimethylaminopyridin umgesetzt und über Nacht bei RT gerührt. Dann wurden nochmals 10 Äquiv. *N*-Hydroxysuccinimid, 5 Äquiv. 1-(3-Dimethylaminopropyl)-3-ethylcarbodiimid-Hydrochlorid, 5 Äquiv. *N*,*N*-Diisopropylethylamin und 0.5 Äquiv. 4-Dimethylaminopyridin nachgesetzt und das Reaktionsgemisch 5 h im Ultraschallbad behandelt. Anschließend wurde das Lösungsmittel abgedampft, der Rückstand mittels präparativer HPLC gereinigt und die entsprechenden Fraktionen vereinigt und eingeengt. Nach Lyophilisaton des Rückstandes aus Dioxan wurden 9.7 mg (62% d. Th.) der Titelverbindung als farbloser Schaum erhalten.
HPLC (Methode 6): Rₜ = 1.8 min;
LC-MS (Methode 11): Rₜ = 0.77 min; MS (ESIpos): m/z = 1078 (M+H)⁺.

### Intermediat 105

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(1S)-1-carboxy-2-phenylethyl]amino}-1-methoxy-2-methyl-3-oxopropyl]pyrrolidin-1-yl}-3-methoxy-5-methyl-1-oxoheptan-4-yl]-N-methyl-L-valinamid

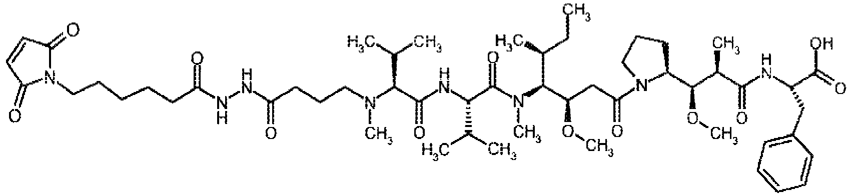

Diese Verbindung wurde in Analogie zur in Intermediat 157 beschriebenen Synthese ausgehend von 4-{[(2*S*)-1-{[(2*S*)-1-{[(3*R*,4*S*,5*S*)-1-{(2*S*)-2-[(1*R*,2*R*)-3-{[(2*S*)-1-*tert*.-Butoxy-1-oxo-3-phenyl-propan-2-yl]amino}-1-methoxy-2-methyl-3-oxopropyl]pyrrolidin-1-yl}-3-methoxy-5-methyl-1-oxoheptan-4-yl](methyl)amino}-3-methylbutan-2-yl]amino}-3-methyl-1-oxobutan-2-yl](methyl)amino}butansäure und kommerziell erhältlichem 6-(2,5-Dioxo-2,5-dihydro-1*H*-pyrrol-1-yl)hexanhydrazid hergestellt. Das Ester-Intermediat wurde in 42%-iger Ausbeute erhalten. In einem zweiten Schritt wurde aus 6 mg (6 µmol) dieses Intermediats mit Trifluoressigsäure der tert.-Butylester gespalten. Nach HPLC Reinigung wurden 3.4 mg (48% d.Th.) der Titelverbindung erhalten.
HPLC (Methode 5): Rₜ = 1.66 min;
LC-MS (Methode 2): Rₜ = 1.04 min; MS (ESIpos): m/z = 1025 (M+H)⁺.

### Intermediat 106

### N-[6-(2,5-Dioxo-2,5-dihydro-1H-pyrrol-1-yl)hexyl]-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

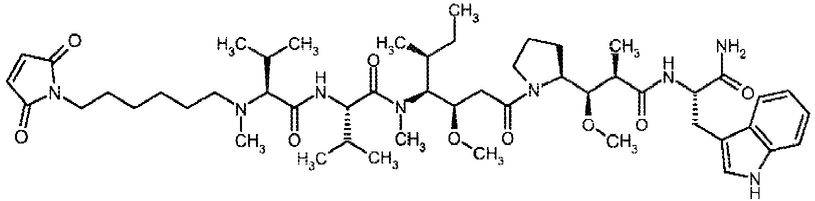

14 mg (16 µmol) *N*-(6-Aminohexyl)-*N*-methyl-L-valyl-*N*-[(3*R*,4*S*,5*S*)-1-{(2*S*)-2-[(1*R*,2*R*)-3-{[(2*S*)-1-amino-3-(1*H*-indol-3-yl)-1-oxopropan-2-yl]amino}-1-methoxy-2-methyl-3-oxopropyl] pyrrolidin-1-yl}-3-methoxy-5-methyl-1-oxoheptan-4-yl]-*N*-methyl-L-valinamid (Intermediat 88) wurden in 750 µl Dioxan aufgenommen und mit 1.5 ml gesättigter Natriumhydrogencarbonat-Lösung und anschließend mit 3.2 mg (21 µmol) Methyl-2,5-dioxo-2,5-dihydro-1*H*-pyrrol-1-carboxylat versetzt. Das Reaktionsgemisch wurde 1h bei RT gerührt und danach im Vakuum eingeengt. Der verbliebene Rückstand wurde mittels präparativer HPLC gereinigt. Nach Lyophilisation wurden 5.5 mg (36% d.Th.) der Titelverbindung erhalten.
HPLC (Methode 5): Rₜ = 1.7 min;
LC-MS (Methode 1): Rₜ = 0.84 min; MS (ESIpos): m/z = 949 (M+H)⁺.

### Intermediat 107

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[2-(1H-indol-3-yl)ethyl]amino}-1-methoxy-2-methyl-3-oxopropyl]pyrrolidin-1-yl}-3-methoxy-5-methyl-1-oxoheptan-4-yl]-N-methyl-L-valinamid

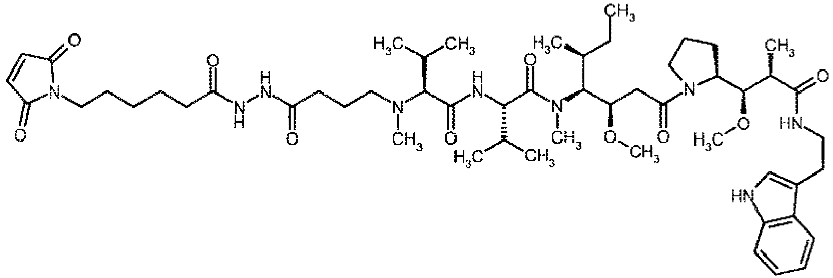

38 mg (47 µmol) *N*-(3-Carboxypropyl)-*N*-methyl-L-valyl-*N*-[(3*R*,4*S*,5*S*)-1-{(2*S*)-2-[(1*R*,2*R*)-3-{[2-(1*H*-indol-3-yl}ethyl]amino}-1-methoxy-2-methyl-3-oxopropyl]pyrrolidin-1-yl}-3-methoxy-5-methyl-1-oxoheptan-4-yl]-*N*-methyl-L-valinamid wurden in 37 ml DMF gelöst und anschließend mit 71 mg (187 µmol) *O*-(7-Azabenzotriazol-1-yl)-*N,N,N'*,*N'*-tetramethyluronium-hexafluorophosphat, 33 µL *N,N*-Diisopropylethylamin sowie mit 37 mg (140 µmοl) von kommerziell erhältlichem 6-(2,5-Dioxo-2,5-dihydro-1*H*-pyrrol-1-yl)hexanhydrazid versetzt. Die Mischung wurde 1 h bei RT gerührt. Anschließend wurde im Hochvakuum eingeengt und der verbliebene Rückstand mittels präparativer HPLC aufgereinigt. So wurden 12.2 mg (26 % d.Th.) der Titelverbindung als farbloser Schaum erhalten.
HPLC (Methode 5): Rₜ = 1.6 min;
LC-MS (Methode 1): Rₜ = 0.85 min; MS (ESIpos): m/z = 1020 (M+H)⁺.

### Intermediat 108

### N-(4-{2-[6-(2,5-Dioxo-2,5-dihydro-1H-pyrrol-1-yl)hexanoyl]hydrazino}-4-oxobutyl)-N-methyl-L-valyl-N-{(3R,4S,5S)-3-methoxy-1-[(2S)-2-{(1R,2R)-1-methoxy-2-methyl-3-oxo-3-[(2-phenylethyl) amino]propyl}pyrrolidin-1-yl]-5-methyl-1-oxoheptan-4-yl}-N-methyl-L-valinamid

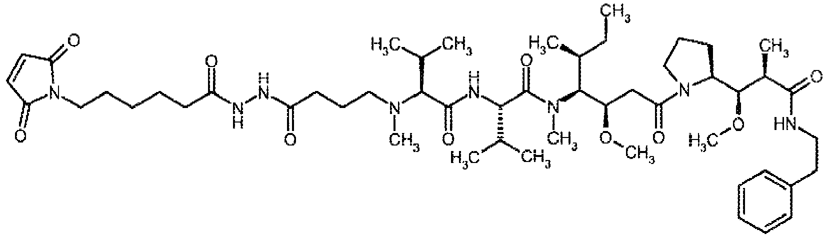

Die Verbindung wurde in Analogie zu Intermediat 107 hergestellt.
Ausbeute: 2.5 mg (30 % d.Th.)
HPLC (Methode 12): Rₜ = 1.9 min;
LC-MS (Methode 1): Rₜ = 0.9 min; MS (ESIpos): m/z = 981 (M+H)⁺.

### Intermediat 109

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(1S,2R)-1-hydroxy-1-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

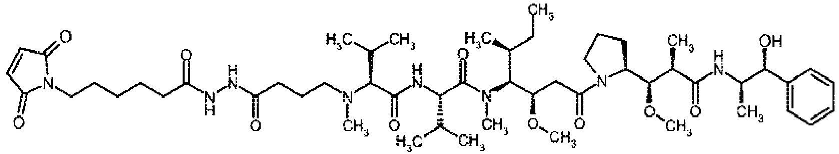

Die Verbindung wurde in Analogie zu Intermediat 107 aus der Verbindung in Intermediat 92 hergestellt.
Ausbeute: 35 mg (65 % d.Th.)
HPLC (Methode 5): Rₜ = 1.9 min;
LC-MS (Methode 11): Rₜ = 0.76 min; MS (ESIpos): m/z = 1011 (M+H)⁺.

### Intermediat 110

### N-{6-[(2,5-Dioxopyrrolidin-1-yl)oxy]-6-oxohexyl}-N-methyl-L-valyl-N-[(3R,4S,5S)-1-{(2S}-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

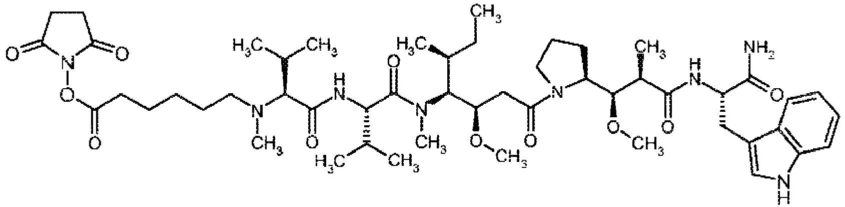

Diese Verbindung wurde in Analogie zu Intermediat 147 aus der Verbindung in Intermediat 83 hergestellt.
Ausbeute: 2.4 mg (24 % d.Th.)
HPLC (Methode 6): Rₜ = 1.8 min;
LC-MS (Methode 1): Rₜ = 0.87 min; MS (ESIpos): m/z = 981 (M+H)⁺.

### Intermediat 111

### N-(4-{2-[6-(2,5-Dioxo-2,5-dihydro-1H-pyrrol-1-yl)hexanoyl]-1-methylhydrazino}-4-oxobutyl)-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

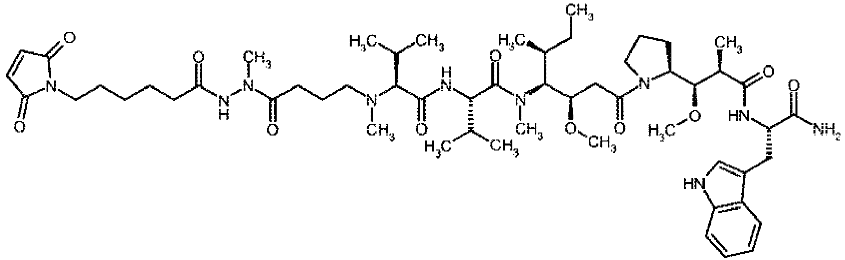

Diese Verbindung wurde in Analogie zu Intermediat 140 aus Intermediat 82 und Intermediat 22 hergestellt.
Ausbeute: 6.5 mg (51 % d.Th.)
HPLC (Methode 6): Rₜ = 1.8 min;
LC-MS (Methode 1): Rₜ = 4.71 min; MS (ESIpos): m/z = 1077 (M+H)⁺.

### Intermediat 112

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(1S,2R)-1-carbamoyl-2-phenylcyclopropyl]amino}-1-methoxy-2-methyl-3-oxopropyl]pyrrolidin-1-yl}-3-methoxy-5-methyl-1-oxoheptan-4-yl]-N-methyl-L-valinamid

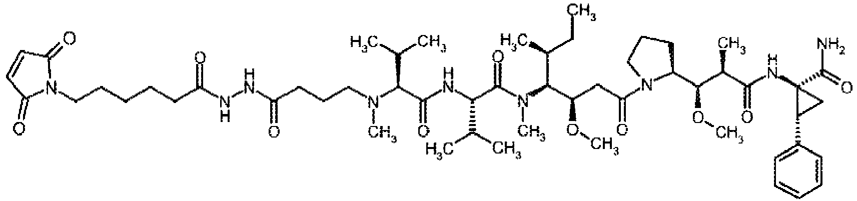

Diese Verbindung wurde in Analogie zu Intermediat 157 aus der Verbindung in Intermediat 81 hergestellt.
Ausbeute: 5.7 mg (57 % d.Th.)
HPLC (Methode 5): Rₜ = 1.6 min;
LC-MS (Methode 1): Rₜ = 0.87 min; MS (ESIpos): m/z = 1036 (M+H)⁺.

### Intermediat 113

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(1S)-1-carboxy-2-(1H-indol-3-yl)ethyl]amino}-1-methoxy-2-methyl-3-oxopropyl]pyrrolidin-1-yl}-3-methoxy-5-methyl-1-oxoheptan-4-yl]-N-methyl-L-valinamid

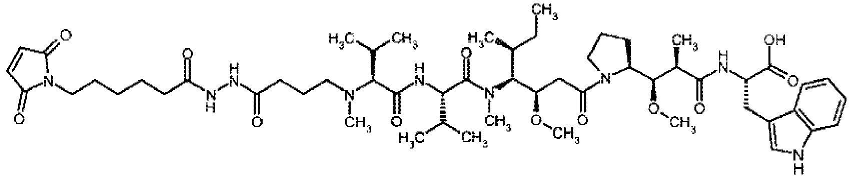

95 mg (104 µmol) von 4-{[(2*S*)-1-{[(2*S*)-1-{[(3*R*,4*S*,5*S*)-1-{(2*S*)-2-[(1*R*,2*R*)-3-{[(2*S*)-1-*tert-*Butoxy-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl](methyl)amino}-3-methylbutan-2-yl]amino}-3-methyl-1-oxobutan-1-yl](methyl)amino}butansäure wurden in DMF gelöst und anschließend mit 79.5 mg (209 µmol) *O*-(7-Azabenzotriazol-1-yl)-*N*,*N*,*N*',*N*'-tetramethyluronium-hexafluorophosphat, 73 µL *N*,*N*-Diisopropylethylamin sowie mit 68 mg (261 µmol) von kommerziell erhältlichem 6-(2,5-Dioxo-2,5-dihydro-1*H*-pyrrol-1-yl)hexanhydrazid versetzt. Die Mischung wurde 2 h bei RT gerührt. Anschließend wurde im Hochvakuum eingeengt und der verbliebene Rückstand mittels präparativer HPLC aufgereinigt. So wurden 104 mg (89 % d.Th.) des tert.-Butylesters der Titelverbindung als farbloser Schaum erhalten.
HPLC (Methode 5): Rₜ = 2.0 min;
LC-MS (Methode 1): Rₜ = 0.93 min; MS (ESIpos): m/z = 1121 (M+H)⁺.

Das Intermediat wurde in 33.4 ml Dichlormethan aufgenommen, mit 17 ml Trifluoressigsäure versetzt und 1 h bei RT gerührt. Anschließend wurde das Reaktionsgemisch im Vakuum eingeengt und der Rückstand durch präparative HPLC gereinigt.
So wurden 61 mg (62 % d.Th.) der Titelverbindung als farbloser Schaum erhalten.
HPLC (Methode 5): Rₜ = 1.7 min;
LC-MS (Methode 1): Rₜ = 0.86 min; MS (ESIpos): m/z = 1064 (M+H)⁺.

### Intermediat 114

### N-[6-({[2-(2,5-Dioxo-2,5-dihydro-1H-pyrrol-1-yl)ethyl]carbamoyl}amino)hexyl]-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

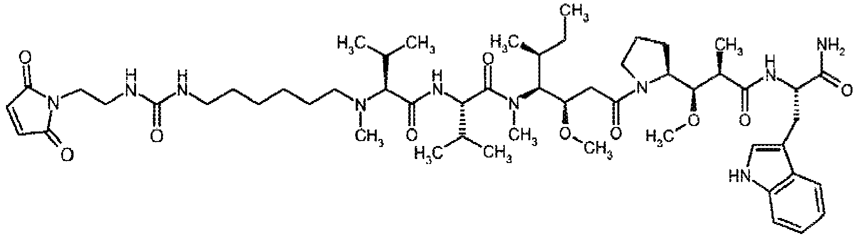

5 mg (5µmol) von *N*-(6-Aminohexyl)-*N*-methyl-L-valyl-*N*-[(3*R*,4*S*,5*S*)-1-{(2*S*)-2-[(1*R*,2*R*)-3-{[(2*S*)-1-amino-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid wurden in 885 µl DMF aufgenommen und mit 5.3 mg (8 µmol) 4-Nitrophenyl-[2-(2,5-dioxo-2,5-dihydro-1*H*-pyrrol-1-yl)ethyl]carbamat sowie 2.8 µl *N*,*N*-Diisopropylethylamin versetzt. Das Reaktionsgemisch wurde 2h bei RT gerührt und anschließend bis zur Trockene eingeengt. Der Rückstand wurde mittels präparativer HPLC gereinigt.
Ausbeute: 0.58 mg (11 % d.Th.) eines farblosen Schaums
HPLC (Methode 5): Rₜ = 1.6 min;
LC-MS (Methode 1): Rₜ = 0.83 min; MS (ESIpos): m/z = 1035 (M+H)⁺.

### Intermediat 115

### N-{4-[(2,5-Dioxopyrrolidin-1-yl)oxy]-4-oxobutyl}-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

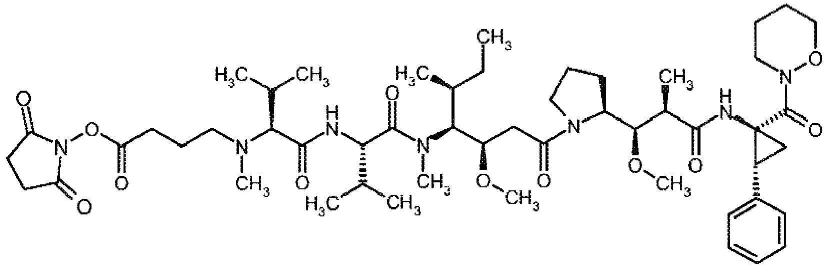

Diese Verbindung wurde in Analogie zu der Verbindung in Intermediat 147 ausgehend von 8 mg (9 µmol) *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl]amino}-3-oxo-propyl]pyrrolidin-1-yl}-5-methyl-1-oxoheptan-4-yl]-*N*-methyl-L-valinamid hergestellt. Nach dem Einengen wurde der aktivierte Ester mittels präparativer HPLC gereinigt und nach Entfernung des Lösungsmittels im Vakuum sofort mit dem Antikörper umgesetzt.
Ausbeute: 3 mg (27% d.Th.) (hydrolyseempfindlich)
HPLC (Methode 5): Rₜ = 1.7 min;
LC-MS (Methode 1): Rₜ = 0.87 min; MS (ESIpos): m/z = 996 (M+H)⁺.

### Intermediat 116

### N-{4-[(2,5-Dioxopyrrolidin-1-yl)oxy]-4-oxobutyl}-N-methyl-L-valyl-N-[(3R,4S,5S)-3-methoxy-1-{(2S)-2-[(1R,2R)-1-methoxy-2-methyl-3-{[(2S)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid

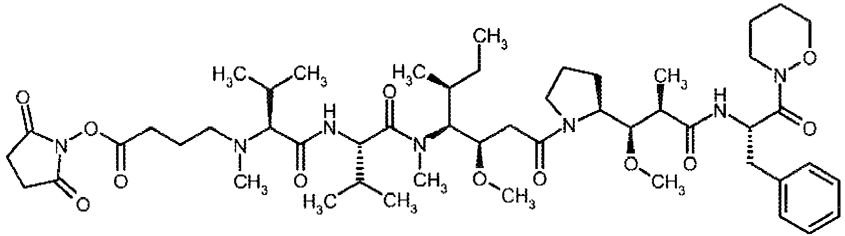

Diese Verbindung wurde in Analogie zu der Verbindung in Intermediat 147 ausgehend von 5 mg (6 µmol) *N*-(3-Carboxypropyl)-*N*-methyl-L-valyl-*N*-(3*R*,4*S*,5*S*)-3-methoxy-1-{(2*S*)-2-[(1*R*,2*R*)-1-methoxy-2-methyl-3-{[(2*S*)-1-(1,2-oxazinan-2-yl)-1-oxo-3-phenylpropan-2-yl]amino}-3-oxopropyl]pyrrolidin-1-yl}-5-methyl-1-oxoheptan-4-yl]-*N*-methyl-L-valinamid hergestellt. Nach dem Einengen wurde der aktivierte Ester mittels präparativer HPLC gereinigt und nach Entfernung des Lösungsmittels im Vakuum sofort mit dem Antikörper umgesetzt.
Ausbeute: 3.2 mg (43% d.Th.) (hydrolyseempfindlich)
HPLC (Methode 5): Rₜ = 1.7 min;
LC-MS (Methode 1): Rₜ = 0.92 min; MS (ESIpos): m/z = 984 (M+H)⁺.

### Intermediat 117

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(2S)-1-tert-butoxy-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

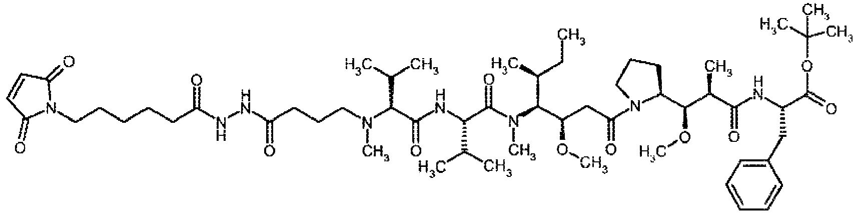

Diese Verbindung wurde in Analogie zu Intermediat 157 aus der Verbindung in Intermediat 86 hergestellt.
Ausbeute: 7 mg (42 % d.Th.)
HPLC (Methode 5): Rₜ = 1.6 min;
LC-MS (Methode 1): Rₜ = 0.94 min; MS (ESIpos): m/z = 1081 (M+H)⁺.

### Intermediat 118

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(2R)-1-(benzyloxy)-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

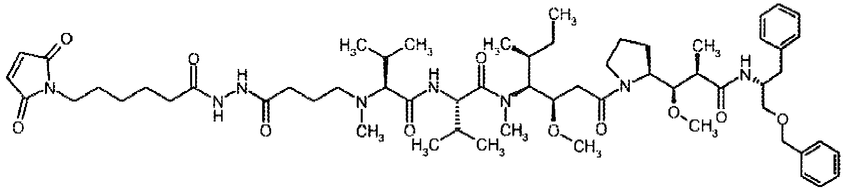

Die Zielverbindung wurde analog zu Intermediat 157 aus 7 mg (7.8 µmol) der Verbindung in Intermediat 68 hergestellt. Ausbeute: 6.3 mg (53% d.Th.)
LC-MS (Methode 1): Rₜ = 1.00 min; MS (ESIpos): m/z = 1102 (M+H)⁺.

### Intermediat 119

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-3-methoxy-1-{(2S)-2-[(1R,2R)-1-methoxy-2-methyl-3-oxo-3-{[(1S)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-L-valinamid

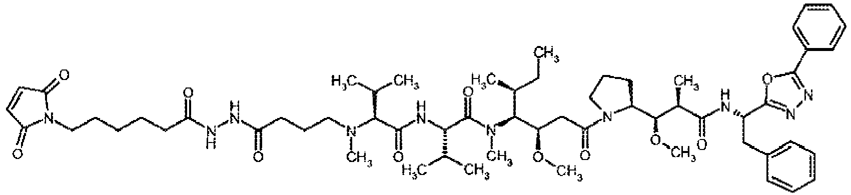

7.4 mg (8.1 mmol) *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-2-methyl-3-oxo-3-{[(1*S*)-2-phenyl-1-(5-phenyl-1,3,4-oxadiazol-2-yl)ethyl]amino}propyl]pyrrolidin-1-yl}-5-methyl-1-oxoheptan-4-yl]-*N*-methyl-L-valinamid und 6.3 mg (24.2 mmol) 6-(2,5-Dioxo-2,5-dihydro-1*H*-pyrrol-1-yl)hexanhydrazid-hydrochlorid wurden in Analogie zu Intermediat 157 gekuppelt und aufgearbeitet. Es wurden 1.6 mg (13%. d. Th.) der Titelverbindung als Feststoff erhalten.
LC-MS (Methode 11): Rₜ = 0.89 min; MS (ESIpos): m/z = 1126 (M+H)⁺

### Intermediat 120

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-3-methoxy-1-{(2S)-2-[(1R,2R)-1-methoxy-2-methyl-3-oxo-3-{[(1R)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-L-valinamid

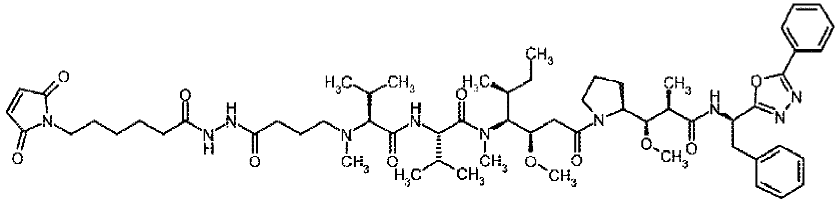

12.8 mg (13.9 mmol) *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-2-methyl-3-oxo-3-{[(1*R*)-2-phenyl-1-(5-phenyl-1,3,4-oxadiazol-2-yl)ethyl]amino}propyl]pyrrolidin-1-yl}-5-methyl-1-oxoheptan-4-yl]-*N*-methyl-L-valinamid und 10.9 mg (41.8 mmol) 6-(2,5-Dioxo-2,5-dihydro-1*H*-pyrrol-1-yl)hexanhydrazid-Hydrochlorid wurden in Analogie zu Intermediat 157 gekuppelt und aufgearbeitet. Es wurden 10.8 mg (59%. d. Th.) der Titelverbindung als Feststoff erhalten.
LC-MS (Methode 11): Rₜ = 0.90 min; MS (ESIpos): m/z = 1126 (M+H)⁺

### Intermediat 121

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(2S)-1-(benzylsulfonyl)-3-phenylpropan-1-yl]amino}-1-methoxy-2-methyl-3-oxopropyl]pyrrolidin-1-yl}-3-methoxy-5-methyl-1-oxoheptan-4-yl]-N-methyl-L-valinamid

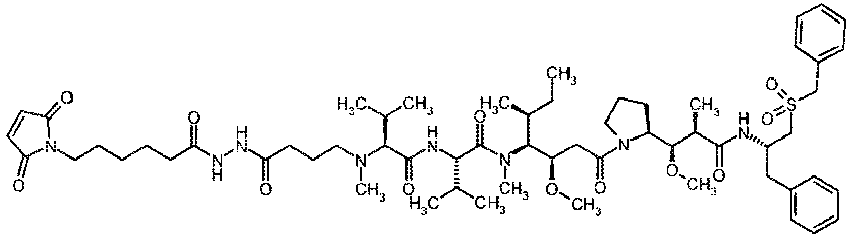

7.4 mg (7.9 mmol) *N*-(3-Carboxypropyl)-*N*-methyl-L-valyl-*N*-[(3*R*,4*S*,5*S*)-1-{(2*S*)-2-[(1*R*,2*R*)-3-{[(2*S*)-1-(benzylsulfonyl)-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid und 6.2 mg (23.5 mmol) 6-(2,5-Dioxo-2,5-dihydro-1*H*-pyrrol-1-yl)hexanhydrazid-1-Hydrochlorid wurden in Analogie zu Intermediat 157 gekuppelt und aufgearbeitet. Es wurden 6.9 mg (7 4% d. Th.) der Titelverbindung als Feststoff erhalten.
LC-MS (Methode 11): Rₜ = 0.87 min; MS (ESIpos): m/z = 1150 (M+H)⁺

### Intermediat 122

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(2S,3E)-1,4-diphenylbut-3-en-2-yl]amino}-1-methoxy-2-methyl-3-oxopropyl]pyrrolidin-1-yl}-3-methoxy-5-methyl-1-oxoheptan-4-yl]-N-methyl-L-valinamid

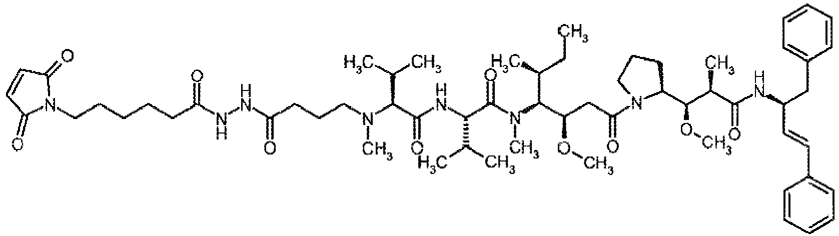

8 mg (9.1 mmol) *N*-(3-Carboxypropyl)-*N*-methyl-L-valyl-*N*-[(3*R*,4*S*,5*S*)-1-{(2*S*)-2-[(1*R*,2*R*)-3-{[(2*S*,3*E*)-1,4-diphenylbut-3-en-2-yl]amino}-1-methoxy-2-methyl-3-oxopropyl]pyrrolidin-1-yl}-3-methoxy-5-methyl-1-oxoheptan-4-yl]-*N*-methyl-valinamid und 7.2 mg (27.4 mmol) 6-(2,5-Dioxo-2,5-dihydro-1*H*-pyrrol-1-yl)hexanhydrazid-Hydrochlorid wurden in Analogie zu Intermediat 157 gekuppelt und aufgearbeitet. Es wurden 8.2 mg (82 % d. Th.) der Titelverbindung als weißer Feststoff erhalten.
LC-MS (Methode 11): Rₜ = 0.95 min; MS (ESIpos): m/z = 1083 (M+H)⁺

### Intermediat 123

### N-[6-(2,5-Dioxo-2,5-dihydro-1H-pyrrol-1-yl)hexyl]-N-methyl-L-valyl-N-[(3R,4S,5S)-1-{(2S)-2-[(1R,2R)-3-{[(2S)-1-tert-butoxy-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

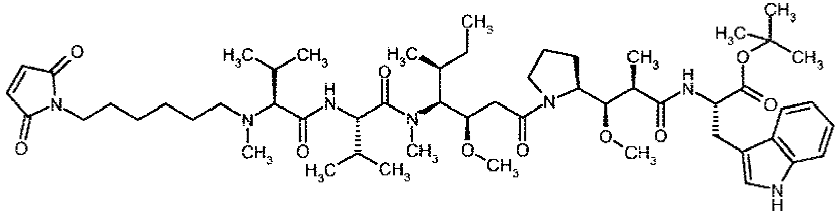

30 mg (30 µmol) von Intermediat 89 wurden in 2 ml 1,4-Dioxan aufgenommen und mit 4 ml gesättigter Natriumhydrogencarbonat-Lösung und anschließend mit 7.5 mg (50 µmol) Methyl-2,5-dioxo-2,5-dihydro-1*H*-pyrrol-1-carboxylat versetzt. Das Reaktionsgemisch wurde 1h bei RT gerührt und danach im Vakuum eingeengt. Der verbliebene Rückstand wurde mittels präparativer HPLC gereinigt. Nach Lyophilisation wurden 24 mg (74% d.Th.) der Titelverbindung erhalten.
HPLC (Methode 5): Rₜ = 2.2 min;
LC-MS (Methode 1): Rₜ = 1.01 min; MS (ESIpos): m/z = 1006 (M+H)⁺.

### Intermediat 124

### N-[6-(2,5-Dioxo-2,5-dihydro-1H-pyrrol-1-yl)hexyl]-N-methyl-L-valyl-N-[(3R,4S,5S)-1-{(2S)-2-[(1R,2R)-3-{[(1S)-1-carboxy-2-(1H-indol-3-yl)ethyl]amino}-1-methoxy-2-methyl-3-oxopropyl]pyrrolidin-1-yl}-3-methoxy-5-methyl-1-oxoheptan-4-yl]-N-methyl-L-valinamid

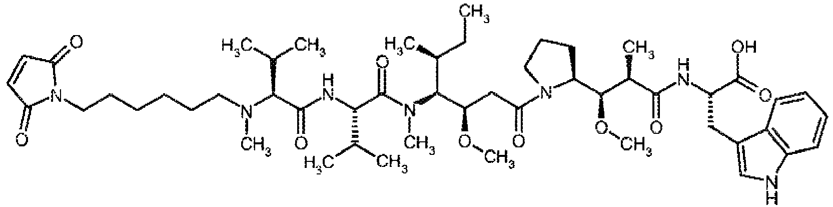

22 mg (20 µmοl) von Intermediat 123 wurden mit 4 ml Trifluoressigsäure in 8 ml Dichlormethan 1 h bei RT umgesetzt. Danach wurde das Reaktionsgemisch im Vakuum eingeengt. Der verbliebene Rückstand wurde mittels präparativer HPLC gereinigt. Nach Lyophilisation wurden 11 mg (54% d.Th.) der Titelverbindung erhalten.
HPLC (Methode 5): Rₜ = 1.8 min;
LC-MS (Methode 11): Rₜ = 0.85 min; MS (ESIpos): m/z = 950 (M+H)⁺.

### Intermediat 125

### N-[6-(2,5-Dioxo-2,5-dihydro-1H-pyrrol-1-yl)hexyl]-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

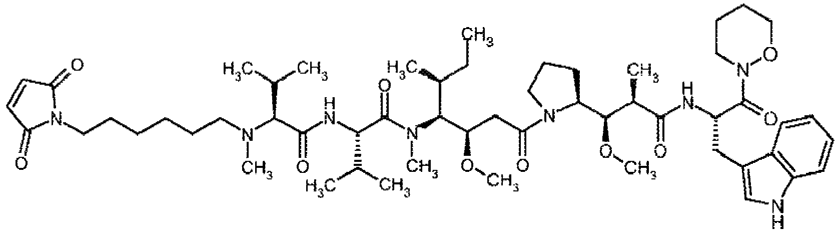

22.5 mg (20 µmol) von Intermediat 101 wurden in 2 ml Dioxan/Wasser 1:1 aufgenommen und anschließend mit 5.6 mg (40 µmol) Methyl-2,5-dioxo-2,5-dihydro-1*H*-pyrrol-1-carboxylat sowie mit 0.25 ml gesättigter Natriumhydrogencarbonat-Lösung versetzt. Das Reaktionsgemisch wurde 30 min bei RT gerührt. Dann wurden nochmals 0.25 ml der gesättigten Natriumhydrogencarbonatlösung zugegeben und das Reaktionsgemisch weitere 15 min bei RT gerührt und anschließend im Vakuum eingeengt. Der verbliebene Rückstand wurde mittels präparativer HPLC gereinigt. Nach Lyophilisation wurden 12.8 mg (50% d.Th.) der Titelverbindung als farbloser Schaum erhalten.
HPLC (Methode 5): Rₜ = 1.9 min;
LC-MS (Methode 1): Rₜ = 0.95 min; MS (ESIpos): m/z = 1019 (M+H)⁺.

### Intermediat 126

### N-[6-(2,5-Dioxo-2,5-dihydro-1H-pyrrol-1-yl)hexyl]-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

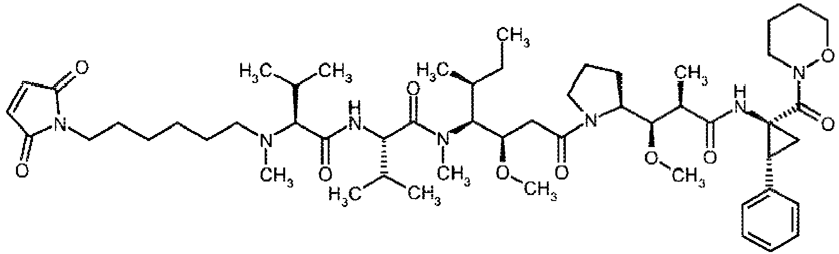

64 mg (70 µmol) *N*-(6-Aminohexyl)-*N*-methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl] amino}-3-oxopropyl]pyrrolidin-1-yl}-5-methyl-1-oxoheptan-4-yl]-*N*-methyl-L-valinamid (Intermediat 97) wurden in 3 ml Dioxan/Wasser 1:1 aufgenommen, dann mit 4 ml gesättigter Natriumhydrogencarbonat-Lösung auf pH 9 eingestellt und anschließend mit 16.3 mg (110 µmol) Methyl-2,5-dioxo-2,5-dihydro-1*H*-pyrrol-1-carboxylat versetzt. Das Reaktionsgemisch wurde 1 h bei RT gerührt und danach im Vakuum eingeengt. Dann wurden nochmals 8 mg (55 µmol) Methyl-2,5-dioxo-2,5-dihydro-1*H*-pyrrol-1-carboxylat nachgesetzt, das Reaktionsgemisch erneut auf pH 9 eingestellt und eine weitere Stunde bei RT gerührt. Anschließend wurde eingeengt und der verbliebene Rückstand mittels präparativer HPLC gereinigt. Isoliert wurden zunächst 31 mg eines noch nicht cyclisierten Intermediats. 27 mg von diesem Intermediat wurden erneut in 2 ml Dioxan/Wasser 1:1 aufgenommen und dann mit 250 µl gesättigter Natriumhydrogencarbonat-Lösung versetzt. Nach 2 Stunden Rühren bei RT wurde das Reaktionsgemisch eingeengt und der Rückstand mittels präparativer HPLC gereinigt. Nach Lyophilisation wurden 20 mg (29% d.Th.) der Titelverbindung erhalten.
HPLC (Methode 5): Rₜ = 1.96 min;
LC-MS (Methode 1): Rₜ = 0.97 min; MS (ESIpos): m/z = 992 (M+H)⁺.

### Intermediat 127

### N-{6-[(2,5-Dioxopyrrolidin-1-yl)oxy]-6-oxohexyl}-N-methyl-1-valyl-N-[(3R,4S,5S)-1-{(2S)-2-[(1R,2R)-3-{[(2S)-1-(benzylamino)-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-y]|-N-methyl-L-valinamid

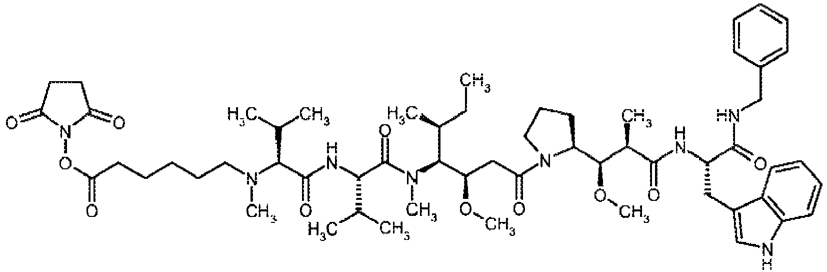

17 mg (18 µmol) *N*-(5-Carboxypentyl)-*N*-methyl-L-valyl-*N*-[(3*R*,4*S*,5*S*)-1-{(2*S*)-2-[(1*R*,2*R*)-3-{[(2*S*)-1-(benzylamino)-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid (Intermediat 98) wurden in 2.8 ml Dichlormethan gelöst und mit 20 mg (174 mmol) 1-Hydroxy-pyrrolidin-2,5-dion und anschließend mit 10 mg (52 µmol) 1-(3-Dimethylaminopropyl)-3-ethyl-carbodiimid-Hydrochlorid und 0.21 mg (0.17 µmol) DMAP versetzt. Nach 4 h Rühren bei RT wurde das Reaktionsgemisch im Vakuum eingeengt. Der verbliebene Rückstand wurde mittels präparativer HPLC gereinigt. Nach Lyophilisation wurden 8.2 mg (43% d.Th.) der Titelverbindung erhalten.
HPLC (Methode 5): Rₜ = 2.0 min;
LC-MS (Methode 1): Rₜ = 0.98 min; MS (ESIpos): m/z = 1071 (M+H)⁺.

### Intermediat 128

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-3-methoxy-1-{(2S)-2-[(1R,2R)-1-methoxy-2-methyl-3-{[(2S)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid

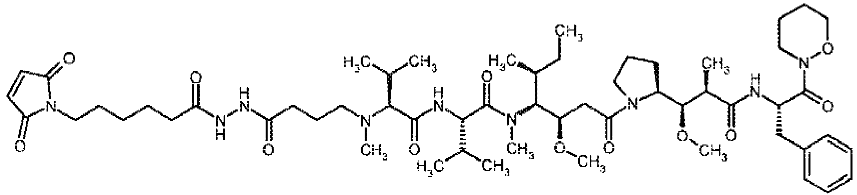

5 mg (5.6 µmol) *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(1,2-oxazinan-2-yl)-1-oxo-3-phenylpropan-2-yl]amino}-3-oxopropyl]pyrrolidin-1-yl}-5-methyl-1-oxoheptan-4-yl]-*N*-methyl-L-valinamid wurden in 845 µl DMF gelöst und anschließend mit 3.2 mg (17 µmol) 1-(3-Dimethylaminopropyl)-3-ethylcarbodiimid-Hydrochlorid, 2.6 mg (17 µmol) 1-Hydroxy-1*H*-benzotriazol-Hydrat, 1.96 µL *N*,*N*-Diisopropylethylamin sowie mit 5.9 mg (22.5 µmol) von kommerziell erhältlichem 6-(2,5-Dioxo-2,5-dihydro-1*H*-pyrrol-1-yl)hexanhydrazid versetzt. Die Mischung wurde über Nacht bei RT gerührt und anschließend im Hochvakuum eingeengt. Der verbliebene Rückstand wurde mittels präparativer HPLC aufgereinigt. So wurden 2.2 mg (36%) der Titelverbindung als farbloser Schaum erhalten.
HPLC (Methode 5): Rₜ = 1.7 min;
LC-MS (Methode 1): Rₜ = 0.88 min; MS (ESIpos): m/z = 1094 (M+H)⁺.

### Intermediat 129

### N-(6-{2-[6-(2,5-Dioxo-2,5-dihydro-1H-pyrrol-1-yl)hexanoyl]hydrazino}-6-oxohexyl)-N-methyl-L-valyl-N-[(3R,4S,5S)-3-methoxy-1-{(2S)-2-[(1R,2R)-1-methoxy-2-methy]-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

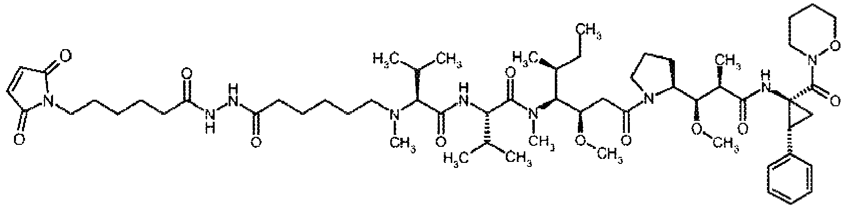

4 mg (4.3 µmol) *N*-(5-Carboxypentyl)-*N*-methyl-L-valyl-*N*-[(3*R*,4*S*,5*S*)-3-methoxy-1-{(2*S*}-2-[(1*R*,2*R*)-1-methoxy-2-methyl-3-{[(1*S*,2*R*)-1-(1,2-oxazinan-2-ylcarbonyl)-2-phenylcyclopropyl] amino}-3-oxopropyl]pyrrolidin-1-yl}-5-methyl-1-oxoheptan-4-yl]-*N*-methyl-L-valinamid wurden in 646 µl DMF gelöst und anschließend mit 2.5 mg (13 µmol) 1-(3-Dimethylaminopropyl)-3-ethylcarbodiimid-Hydrochlorid, 2.0 mg (13 µmol) 1-Hydroxy-1*H*-benzotriazol-Hydrat, 2.25 µL *N*,*N*-Diisopropylethylamin sowie mit 4.5 mg (17 µmol) von kommerziell erhältlichem 6-(2,5-Dioxo-2,5-dihydro-1*H*-pyrrol-1-yl)hexanhydrazid versetzt. Die Mischung wurde 3h bei RT gerührt und anschließend im Hochvakuum eingeengt. Der verbliebene Rückstand wurde mittels präparativer HPLC aufgereinigt. So wurden 1.9 mg (39%) der Titelverbindung als farbloser Schaum erhalten.
HPLC (Methode 5): Rₜ = 1.7 min;
LC-MS (Methode 9): Rₜ = 4.9 min; MS (ESIpos): m/z = 1134 (M+H)⁺.

### Intermediat 130

### N-(4-{[(2R)-1-({5-[(2,5-Dioxopyrrolidin-1-yl)oxy]-5-oxopentanoyl}amino)propan-2-yl]oxy}-4-oxobutyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

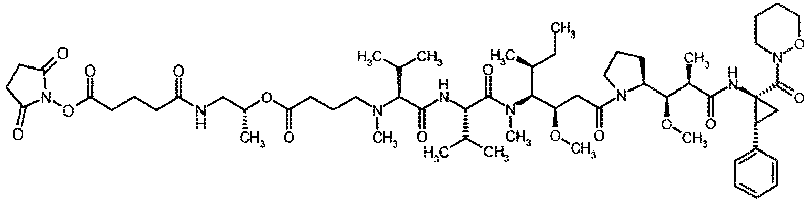

10.5 mg (11.7 µmοl) *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl] amino}-3-oxopropyl]pyrrolidin-1-yl}-5-methyl-1-oxoheptan-4-yl]-*N*-methyl-L-valinamid wurden in 3.7 ml Dichlormethan gelöst und anschließend mit 6.7 mg (35 µmol) 1-(3-Dimethylaminopropyl)-3-ethylcarbodiimid-Hydrochlorid, 0.7 mg (5.8 µmol) 4-Dimethylaminopyridin sowie mit 8.2 mg (47 µmol) von kommerziell erhältlichem *tert*.-Butyl-[(2*R*)-2-hydroxypropyl]carbamat versetzt. Die Mischung wurde über Nacht bei RT gerührt und anschließend im Hochvakuum eingeengt. Der verbliebene Rückstand wurde mittels präparativer HPLC aufgereinigt. So wurden 7.5 mg (61% d. Th.) des Boc-geschützten Intermediats als farbloser Schaum erhalten.
HPLC (Methode 5): Rₜ = 2.0 min;
LC-MS (Methode 1): Rₜ = 1.03 min; MS (ESIpos): m/z = 1056 (M+H)⁺.

Anschließend wurde die Boc-Schutzgruppe mit Trifluoressigsäure abgespalten. 4.9 mg (0.005 mmol) des entschützten Rohprodukts wurden dann ohne weitere Reinigung in 1.8 ml Dichlormethan aufgenommen und mit 3.7 mg (0.011 mmol) 1,1'-[(1,5-Dioxopentan-1,5-diyl)bis(oxy)]dipyrrolidin-2,5-dion, 2.4 µl (0.014 mmol) *N,N*-Diisopropylethylamin und 0.6 mg (5 µmol) 4-Dimethylaminopyridin versetzt. Die Mischung wurde 2h bei RT gerührt und anschließend im Hochvakuum eingeengt. Der verbliebene Rückstand wurde mittels präparativer HPLC aufgereinigt. So wurden 0.77 mg (15% d. Th.) der Titelverbindung als farbloser Schaum erhalten.
HPLC (Methode 5): Rₜ = 1.8 min;
LC-MS (Methode 1): Rₜ = 0.93 min; MS (ESIpos): m/z = 1167 (M+H)⁺.

### Intermediat 131

### N-{4-[(1-{5-[(2,5-Dioxopyrrolidin-1-yl)oxy]-5-oxopentanoyl}piperidin-4-yl)oxy]-4-oxobutyl}-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

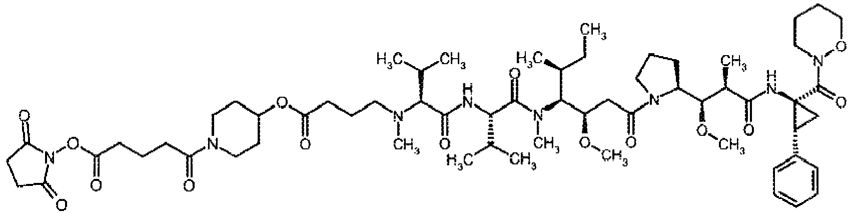

10 mg (11 µmol) *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl] amino}-3-oxopropyl]pyrrolidin-1-yl}-5-methyl-1-oxoheptan-4-yl]-*N*-methyl-L-valinamid wurden in 2 ml Dichlormethan gelöst und anschließend mit 4.3 mg (22 µmοl) 1-(3-Dimethylaminopropyl)-3-ethylcarbodiimid-Hydrochlorid, 0.88 mg (6 µmol) 4-Dimethylaminopyridin sowie mit 5.2 mg (22 µmol) kommerziell erhältlichem Benzyl-4-hydroxypiperidin-1-carboxylat versetzt. Die Mischung wurde über Nacht bei RT gerührt und anschließend im Hochvakuum eingeengt. Der verbliebene Rückstand wurde mittels präparativer HPLC aufgereinigt. So wurden 5 mg (40% d. Th.) des Z-geschützten Intermediats als farbloser Schaum erhalten.
HPLC (Methode 5): Rₜ = 2.1 min;
LC-MS (Methode 1): Rₜ = 1.04 min; MS (ESIpos): m/z = 1116 (M+H)⁺.

Anschließend wurde die Z-Schutzgruppe hydrogenolytisch in Ethanol über Palladium/Aktivkohle abgespalten. 4.6 mg (0.005 mmol) des entschützten Rohprodukts wurden dann ohne weitere Reinigung in 1.8 ml Dichlormethan aufgenommen und mit 3.8 mg (0.012 mmol)1.1'-[(1,5-Dioxopentan-1,5-diyl)bis(oxy)]dipyrrolidin-2,5-dion, 0.8 µl (0.005 mmol) *N,N*-Diisopropylethyl amin und 0.6 mg (5 µmol) 4-Dimethylaminopyridin versetzt. Die Mischung wurde über Nacht bei RT gerührt und anschließend im Hochvakuum eingeengt. Der verbliebene Rückstand wurde mittels präparativer HPLC aufgereinigt. So wurden 0.96 mg (16% d. Th.) der Titelverbindung als farbloser Schaum erhalten.
HPLC (Methode 5): Rₜ = 1.8 min;
LC-MS (Methode 1): Rₜ = 0.94 min; MS (ESIpos): m/z = 1193 (M+H)⁺.

### Intermediat 132

### N-(4-{2-[6-(2,5-dioxo-2,5-dihydro-1H-pyrrol-1-yl)hexanoyl]hydrazinyl}-4-oxobutyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

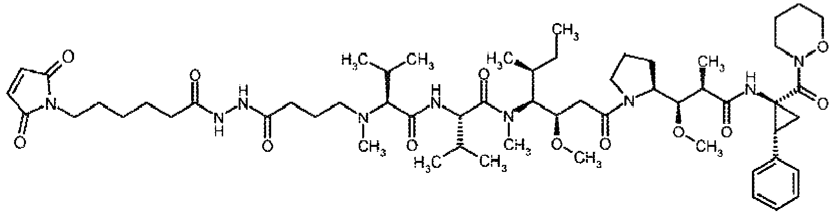

15 mg (16.7 µmol) *N*-(3-Carboxypropyl)-*N*-methyl-L-valyl-*N*-[(3*R*,4*S*,5*S*)-3-methoxy-1-(2*S*)-2-[(1*R*,2*R*)-1-methoxy-2-methyl-3-{[(1*S*,2*R*)-1-(1,2-oxazinan-2-ylcarbonyl)-2-phenylcyclopropyl] amino}-3-oxopropyl]pyrrolidin-1-yl}-5-methyl-1-oxoheptan-4-yl]-*N*-methyl-L-valinamid wurden in 2500 µl DMF gelöst und anschließend mit 9.6 mg (50 µmol) 1-(3-Dimethylaminopropyl)-3-ethylcarbodiimid-Hydrochlorid, 7.6 mg (50 µmol) 1-Hydroxy-1*H-*benzotriazol-Hydrat, 5.8 µL *N*,*N*-Diisopropylethylamin sowie mit 17.4 mg (67 µmol) von kommerziell erhältlichem 6-(2,5-Dioxo-2,5-dihydro-1*H*-pyrrol-1-yl)hexanhydrazid versetzt. Die Mischung wurde über Nacht bei RT gerührt und anschließend im Hochvakuum eingeengt. Der verbliebene Rückstand wurde mittels präparativer IIPLC aufgereinigt. So wurden 11.2 mg (52% d.Th.) der Titelverbindung als farbloser Schaum erhalten.
HPLC (Methode 5): Rₜ = 1.7 min;
LC-MS (Methode 2): Rₜ = 1.09 min; MS (ESIpos): m/z = 1106 (M+H)⁺.

### Intermediat 133

### N-(4-{2-[6-(2,5-dioxo-2,5-dihydro-1H-pyrrol-1-yl)hexanoyl]hydrazinyl}-4-oxobutyl)-N-methyl-L-valyl-N-[(3R,4S,5S)-1-{(2S)-2-[(1R,2R)-3-{[(2S,3S)-1-(benzyloxy)-1-oxo-3-phenylbutan-2-yl]amino}-1-methoxy-2-methyl-3-oxopropyl]pyrrolidin-1-yl}-3-methoxy-5-methyl-1-oxoheptan-4-yl]-N-methyl-L-valinamid

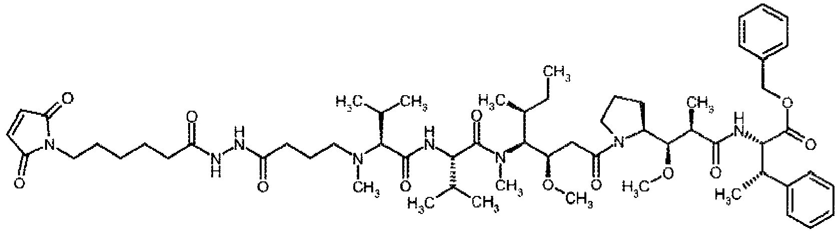

5.8 mg (6.3 µmol) *N*-(3-Carboxypropyl)-*N*-methyl-L-valyl-*N*-[(3*R*,4*S*,5*S*)-1-{(2*S*)*-*2-[(1*R*,2*R*)-3-{[(2*S*,3*S*)-1-(benzyloxy)-1-oxo-3-phenylbutan-2-yl]amino}-1-methoxy-2-methyl-3-oxopropyl] pyrrolidin-1-yl}-3-methoxy-5-methyl-1-oxoheptan-4-yl]-*N*-methyl-L-valinamid wurden in 943 µl DMF gelöst und anschließend mit 3.6 mg (19 µmol) 1-(3-Dimethylaminopropyl)-3-ethylcarbodiimid-Hydrochlorid, 2.9 mg (19 µmol) 1-Hydroxy-1*H*-benzotriazol-Hydrat, 2.2 µL *N*,*N*-Diisopropylethylamin sowie mit 6.6 mg (25 µmol) von kommerziell erhältlichem 6-(2,5-Dioxo-2,5-dihydro-1*H*-pyrrol-1-yl)hexanhydrazid versetzt. Die Mischung wurde über Nacht bei RT gerührt und anschließend im Hochvakuum eingeengt. Der verbliebene Rückstand wurde mittels präparativer HPLC aufgereinigt. So wurden 4.5 mg (64% d.Th.) der Titelverbindung als farbloser Schaum erhalten.
HPLC (Methode 5): Rₜ = 2.0 min;
LC-MS (Methode 1): Rₜ = 1.03 min; MS (ESIpos): m/z = 1129 (M+H)⁺.

### Intermediat 134

### N-[3-({[2-(2,5-Dioxo-2,5-dihydro-1H-pyrrol-1-yl)ethyl]carbamoyl}amino)propyl]-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

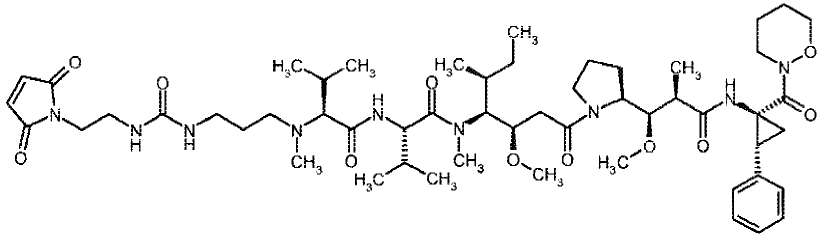

Zunächst wurde 4-Nitrophenyl-[2-(2,5-dioxo-2,5-dihydro-1*H*-pyrrol-1-yl)ethyl]carbamat nach Standardbedingungen ausgehend von kommerziell erhältlichem 1-(2-Aminoethyl)-1*H*-pyrrol-2,5-dion-Trifluoracetat und 4-Nitrophenylchlorocarbonat hergestellt.

5 mg (6 µmol) *N*-(3-Aminopropyl)-*N*-methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl] amino}-3-oxopropyl]pyrrolidin-1-yl}-5-methyl-1-oxoheptan-4-yl]-*N*-methyl-L-valinamid wurden in 1000 µl DMF gelöst und anschließend mit 2 µL *N,N*-Diisopropylethylamin sowie mit 2.2 mg (9 µmol) 4-Nitrophenyl-[2-(2,5-dioxo-2,5-dihydro-1*H*-pyrrol-1-yl)ethyl]carbamat versetzt. Die Mischung wurde 1 h bei RT gerührt und anschließend im Hochvakuum eingeengt. Der verbliebene Rückstand wurde mittels präparativer HPLC aufgereinigt. So wurden 1.6 mg (23% d.Th.) der Titelverbindung als farbloser Schaum erhalten.
HPLC (Methode 5): Rₜ = 1.7 min;
LC-MS (Methode 2): Rₜ = 1.09 min; MS (ESIpos): m/z = 1036 (M+H)⁺.

### Intermediat 135

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(2S)-1-(benzyloxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

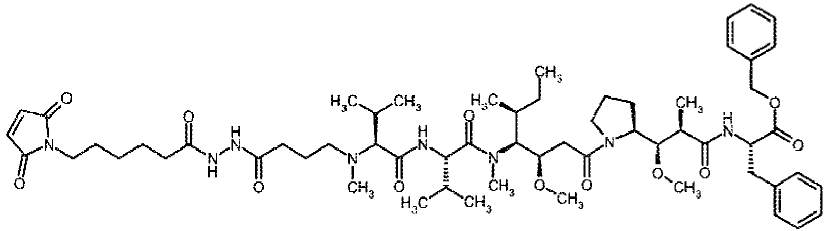

10 mg (11 µmol) *N*-(3-Carboxypropyl)-*N*-methyl-L-valyl-*N*-[(3*R*,4*S*,5*S*)-1-{(2*S*)-2-[(1*R*,2*R*)-3-{[(2*S*)-1-(benzyloxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid wurden in 4000 µl DMF gelöst und anschließend mit 6.3 mg (33 µmol) 1-(3-Dimethylaminopropyl)-3-ethylcarbodiimid-Hydrochlorid, 4.5 mg (33 µmol) 1-Hydroxy-1*H*-benzotriazol-Hydrat, 5.7 µL *N*,*N-*Diisopropylethylamin sowie mit 11.5 mg (44 µmol) von kommerziell erhältlichem 6-(2,5-Dioxo-2,5-dihydro-1*H*-pyrrol-1-yl)hexanhydrazid versetzt. Die Mischung wurde über Nacht bei RT gerührt und anschließend im Hochvakuum eingeengt. Der verbliebene Rückstand wurde mittels präparativer HPLC aufgereinigt. So wurden 2.6 mg (14% d.Th.) der Titelverbindung als farbloser Schaum erhalten.
HPLC (Methode 6): Rₜ = 2.1 min;
LC-MS (Methode 1): Rₜ = 1.01 min; MS (ESIpos): m/z = 1115 (M+H)⁺.

### Intermediat 136

### N-(4-{4-[4-(2,5-Dioxo-2,5-dihydro-1H-pyrrol-1-yl)butanoyl]piperazin-1-yl}-4-oxobutyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

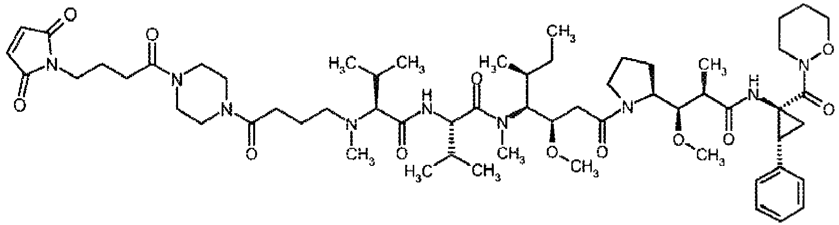

Zunächst wurde 1-[4-Oxo-4-(piperazin-1-yl)butyl]-1*H*-pyrrol-2,5-dion-Trifluoracetat nach Standardbedingungen ausgehend von tert.-Butylpiperazin-1-carboxylat und 4-(2,5-Dioxo-2,5-dihydro-1*H*-pyrrol-1-yl)butansäure über 2 Stufen hergestellt.

5 mg (5.6 µmol) *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl] amino}-3-oxopropyl]pyrrolidin-1-yl}-5-methyl-1-oxoheptan-4-yl]-*N*-methyl-L-valinamid wurden in 1000 µl DMF gelöst und anschließend mit 2.1 mg (11 µmol) 1-(3-Dimethylaminopropyl)-3-ethylcarbodiimid-Hydrochlorid, 1.7 mg (11 µmol) 1-Hydroxy-1*H*-benzotriazol-Hydrat, 2 µL *N,N-*Diisopropylethylamin sowie mit 3.5 mg (5.6 µmol) 1-[4-Oxo-4-(piperazin-1-yl)butyl]-1*H*-pyrrol-2,5-dion-Trifluoracetat versetzt. Die Mischung wurde über Nacht bei RT gerührt. Dann wurden 2.1 mg (5.6 µmol) *O*-(7-Azabenzotriazol-1-yl)-*N*,*N*,*N*',*N*'-tetramethyluronium-hexafluorophosphat hinzugegeben und das Reaktionsgemisch weitere 3h bei RT gerührt. Anschließend wurde das Lösungsmittel im Vakuum entfernt und der verbliebene Rückstand mittels präparativer HPLC aufgereinigt. Die entsprechenden Fraktionen wurden eingeengt und durch Lyophilisation aus Wasser wurden 0.6 mg (10% d.Th.) der Titelverbindung als farbloser Schaum erhalten.
HPLC (Methode 6): Rₜ = 1.9 min;
LC-MS (Methode 1): Rₜ = 0.9 min; MS (ESIpos): m/z = 1132 (M+H)⁺.

### Intermediat 137

### N-(4-{2-[6-(2,5-Dioxo-2,5-dihydro-1H-pyrrol-1-yl)hexanoyl]-1-methylhydrazino}-4-oxobutyl)-N-methyl-L-valyl-N-[(3R,4S,5S)-3-methoxy-1-{(2S)-2-[(1R,2R)-1-methoxy-2-methyl-3-{[(2S)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid

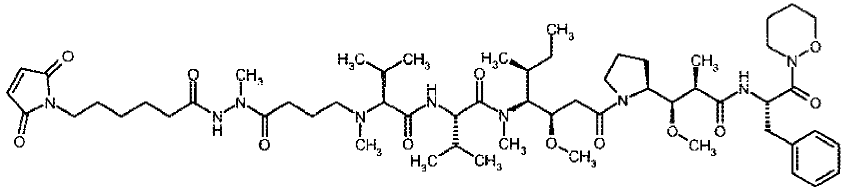

Zunächst wurde 6-(2,5-Dioxo-2,5-dihydro-1*H*-pyrrol-1-yl)-,*N*'-methylhexanhydrazid-Trifluoracetat nach Standardbedingungen ausgehend von kommerziell erhältlicher 6-(2,5-Dioxo-2,5-dihydro-1*H-*pyrrol-1-yl)hexansäure und *tert.*-Butyl-1-methylhydrazincarboxylat über 2 Stufen hergestellt.

6.9 mg (8 µmol) *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(1,2-oxazinan-2-yl)-1-oxo-3-phenylpropan-2-yl]amino}-3-oxopropyl]pyrrolidin-1-yl}-5-methyl-1-oxoheptan-4-yl]-*N*-methyl-L-valinamid wurden in 2540 µl DMF gelöst und anschließend mit 3.6 mg (9 µmol) *O*-(7-Azabenzotriazol-1-yl)-*N,N,N*',*N*'-tetramethyluronium-hexafluorophosphat, 3 µL *N*,*N*-Diisopropylethylamin sowie mit 4.1 mg (12 µmol) 6-(2,5-Dioxo-2,5-dihydro-1*H*-pyrrol-1-yl)-*N'*-methylhexanhydrazid-Trifluoracetat versetzt. Die Mischung wurde über Nacht bei RT gerührt. Anschließend wurde das Lösungsmittel im Vakuum entfernt und der verbliebene Rückstand mittels präparativer HPLC aufgereinigt. So wurden 3.9 mg (45% d.Th.) der Titelverbindung als farbloser Schaum erhalten.
HPLC (Methode 5): Rₜ = 1.8 min;
LC-MS (Methode 1): Rₜ = 0.93 min; MS (ESIpos): m/z = 1108 (M+H)⁺.

### Intermediat 138

### N-{4-[(2-{[4-(2,5-Dioxo-2,5-dihydro-1H-pyrrol-1-yl)butanoyl](methyl)amino}ethyl)(methyl) amino]-4-oxobutyl}-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

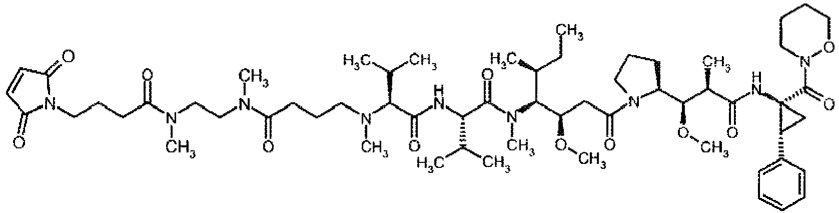

Ausgehend von *tert*.-Butyl-methyl[2-(methylamino)ethyl]carbamat und 4-(2,5-Dioxo-2,5-dihydro-1*H*-pyrrol-1-yl)butansäure wurde zunächst über 2 Stufen 4-(2,5-Dioxo-2,5-dihydro-1*H*-pyrrol-1-yl)-*N*-methyl-*N*-[2-(methylamino)ethyl]butanamid-Trifluoracetat nach hergestellt.

6.6 mg (7.3 µmol) *N*-(3-Carboxypropyl)-*N*-methyl-L-valyl-*N*-[(3*R,*4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclo-propy]amino}-3-oxopropyl]pyrrolidin-1-yl}-5-methyl-1-oxoheptan-4-yl]-*N-*methyl-L-valinamid wurden in 2000 µl DMF gelöst und anschließend mit 5.6 mg (14.7 µmol) *O*-(7-Azabenzotriazol-1-yl)-*N*,*N*,*N'*,*N*'-tetramethyluronium-hexafluorophosphat, 2.6 µL *N,N*-Diisopropylethylamin sowie mit 4.1 mg (9 µmol) 4-(2,5-Dioxo-2,5-dihydro-1*H*-pyrrol-1-yl)-*N*-methyl-*N*-[2-methyl-amino)ethyl]butanamid-Trifluoracetat versetzt. Nach 3 h Rühren bei RT wurden noch mal die gleichen Mengen an HATU und *N*,*N*-Diisopropylethylamin zugesetzt und das Reaktionsgemisch dann über Nacht bei RT gerührt. Anschließend wurde das Lösungsmittel im Vakuum entfernt und der verbliebene Rückstand mittels präparativer HPLC aufgereinigt. So wurden 4 mg (44% d.Th.) der Titelverbindung als farbloser Schaum erhalten.
HPLC (Methode 6): Rₜ = 2.0 min;
LC-MS (Methode 1): Rₜ = 0.91 min; MS (ESIpos): m/z = 1134 (M+H)⁺.

### Intermediat 139

### (2R,3S)-3-Amino-4-{2-[6-(2,5-dioxo-2,5-dihydro-1H-pyrrol-1-yl)hexanoyl]hydrazino}-4-oxobutan-2-yl-(3R,4S,7S,10S)-4-[(2S)-butan-2-yl]-7,10-diisopropyl-3-(2-{(2S)-2-[(1R,2R)-1-methoxy-2-methyl-3-{[(2S)-1-(1,2-oxazinan-2-yl)-1-oxo-3-phenylpropan-2-yl]amino}-3-oxopropyl]pyrrolidin-1-yl}-2-oxoethyl)-5,11-dimethyl-6,9-dioxo-2-oxa-5,8,11-triazapentadecan-15-oat

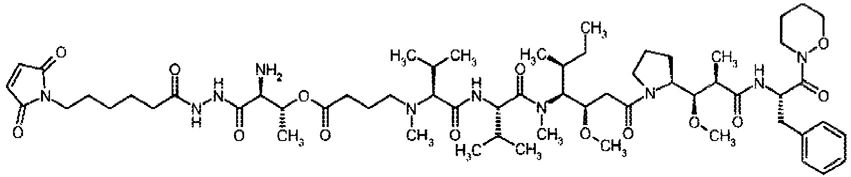

13 mg (14.7 µmol) *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-2-methy]-3-{[(2*S*)-1-(1,2-oxazinan-2-yl)-1-oxo-3-phenylpropan-2-yl]amino}-3-oxopropyl]pyrrolidin-1-yl}-5-methyl-1-oxoheptan-4-yl]-*N*-methyl-L-valinamid wurden in 10 ml Dichlormethan gelöst und anschließend mit 8.4 mg (44 µmol) 1-(3-Dimethylaminopropyl)-3-ethylcarbodiimid-Hydrochlorid, 5.4 mg (44 µmol) 4-Dimethylaminopyridin sowie mit 9 mg (29.3 µmol) von kommerziell erhältlichem Benzyl-*N*-(*tert.*-butoxycarbonyl)-L-threoninat versetzt. Die Mischung wurde 5h bei RT gerührt. Anschließend wurde das Reaktionsgemisch zweimal mit Wasser ausgeschüttelt und die organische Phase über Natriumsulfat getrocknet und im Vakuum eingeengt. Der verbliebene Rückstand wurde mittels präparativer HPLC aufgereinigt. Nach Lyophilisation aus Dioxan/Wasser wurden 14 mg (81% d. Th.) des geschützten Intermediats als farbloser Schaum erhalten.
HPLC (Methode 12): Rₜ = 2.3 min;
LC-MS (Methode 1): Rₜ = 1.13 min; MS (ESIpos): m/z = 1178 (M+H)⁺.

Anschließend wurde die Z-Schutzgruppe hydrogenolytisch in Methanol über 10% Palladium/Aktivkohle abgespalten. 9.5 mg (0.0087 mmol) des entschützten Rohprodukts wurden dann ohne weitere Reinigung in 5 ml DMF aufgenommen und 5 mg (26.2 µmol) 1-(3-Dimethylaminopropyl)-3-ethylcarbodiimid-Hydrochlorid, 4 mg (26.2 µmol) 1-Hydroxy-1*H-*benzotriazol-Hydrat, 54.6 µL *N,N*-Diisopropylethylamin sowie mit 9.1 mg (34.9 µmol) von kommerziell erhältlichem 6-(2,5-Dioxo-2,5-dihydro-1*H*-pyrrol-1-yl)hexanhydrazid versetzt. Die Mischung wurde 1 h bei RT gerührt und anschließend im Hochvakuum eingeengt. Der verbliebene Rückstand wurde mittels präparativer HPLC aufgereinigt. Nach Lyophilisaten aus Dioxan wurden 9.5 mg (84% d.Th.) des Boc-geschützten Intermediats erhalten.
HPLC (Methode 12): Rₜ = 2.1 min;
LC-MS (Methode 1): Rₜ = 0.97 min; MS (ESIpos): m/z = 1295 (M+H)⁺.

Anschließend wurden 9.5 mg (7.3 µmol) mit 0.5 ml Trifluoressigsäure in 2 ml Dichlormethan des Boc-geschützten Intermediats entschützt und nach Lyophilisation aus Dioxan wurden 9 mg (82% d.Th.) der Titelverbindung als farbloser Schaum erhalten.
HPLC (Methode 12): Rₜ = 2.1 min;
LC-MS (Methode 1): Rₜ = 0.84 min; MS (ESIpos): m/z = 1195 (M+H)⁺.

### Intermediat 140

### N-(4-{2-[6-(2,5-Dioxo-2,5-dihydro-1H-pyrrol-1-yl)hexanoyl]-1-methylhydrazino}-4-oxobutyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

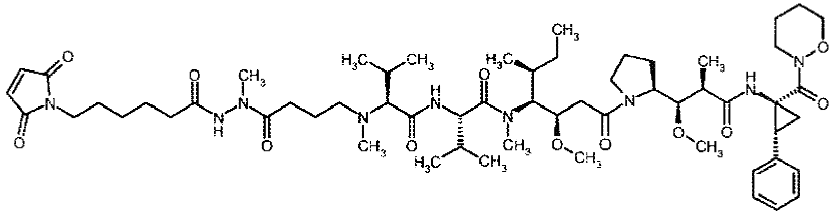

4.1 mg (12µmol) 6-(2,5-Dioxo-2,5-dihydro-1*H*-pyrrol-1-yl)-*N'*-methylhexanhydrazid-Trifluoracetat (Intermediat 22) wurden zusammen mit 6.9 mg (8 µmol) der Verbindung aus Intermediat 61 in 2.5 ml DMF gelöst und anschließend mit 3.5 mg (9 µmol) *O*-(7-Azabenzotriazol-1-yl)-*N,N,N*',*N*'-tetramethyluronium-hexafluorophosphat und 3 µL *N*,*N*-Diisopropylethylamin versetzt. Die Mischung wurde über Nacht bei RT gerührt und anschließend im Hochvakuum eingeengt. Der verbliebene Rückstand wurde mittels präparativer HPLC aufgereinigt. Nach Lyophilisation aus Dioxan wurden 2.6 mg (30% d.Th.) der Titelverbindung erhalten.
HPLC (Methode 5): Rₜ = 1.8 min;
LC-MS (Methode 1): Rₜ = 0.90 und 0.91 min; MS (ESIpos): m/z = 1120 (M+H)⁺.

### Intermediat 141

### N-[4-({1-[4-(2,5-Dioxo-2,5-dihydro-1H-pyrrol-1-yl)butanoyl]piperidin-4-yl}oxy)-4-oxobutyl]-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

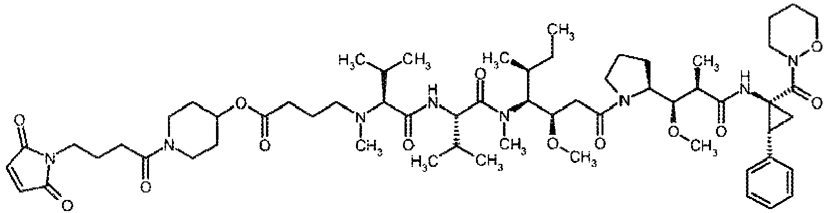

44 mg (49 µmol) N-(3-Carboxypropyl)-*N*-methyl-L-valyl-*N*-[(3*R*,4*S,*5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl] amino}-3-oxopropyl]pyrrolidin-1-yl}-5-methyl-1-oxoheptan-4-yl]-*N*-methyl-L-valinamid wurden in 2 ml Dichlormethan gelöst und anschließend mit 18.8 mg (98 µmol) 1-(3-Dimethylaminopropyl)-3-ethylcarbodiimid-Hydrochlorid, 3.8 mg (24 µmol) 4-Dimethylaminopyridin sowie mit 23 mg (98 µmol) von kommerziell erhältlichem Benzyl-4-hydroxypiperidin-1-carboxylat versetzt. Die Mischung wurde über Nacht bei RT gerührt und anschließend im Hochvakuum eingeengt. Der verbliebene Rückstand wurde mittels präparativer HPLC aufgereinigt. So wurden 22 mg (40% d. Th.) des Z-geschützten Intermediats als farbloser Schaum erhalten.
HPLC (Methode 5): Rₜ = 2.1 min;
LC-MS (Methode 1): Rₜ = 1.04 min; MS (ESIpos): m/z = 1116 (M+H)⁺.

Anschließend wurde die Z-Schutzgruppe hydrogenolytisch in Ethanol über Palladium/Aktivkohle abgespalten.

19 mg (19 µmol) des entschützten Rohprodukts wurden dann ohne weitere Reinigung in 4 ml DMF aufgenommen und mit 7 mg (39 µmol) 4-(2,5-Dioxo-2,5-dihydro-1*H*-pyrrol-1-yl)butansäure, 11 mg (29 µmol) *O*-(7-Azabenzotriazol-1-yl)-*N,N,N',N'*-tetramethyluronium-hexafluorophosphat und 5 µL *N,N*-Diisopropylethylamin versetzt. Die Mischung wurde 1 h bei RT gerührt und anschließend im Hochvakuum eingeengt. Der verbliebene Rückstand wurde mittels präparativer HPLC aufgereinigt. Nach Lyophilisation aus Dioxan wurden 7.5 mg (34% d.Th.) der Titelverbindung erhalten.
HPLC (Methode 5): Rₜ = 1.8 min;
LC-MS (Methode 1): Rₜ = 0.94 min; MS (ESIpos): m/z = 1147 (M+H)⁺.

### Intermediat 142

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(2S)-1-(benzyloxy)-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

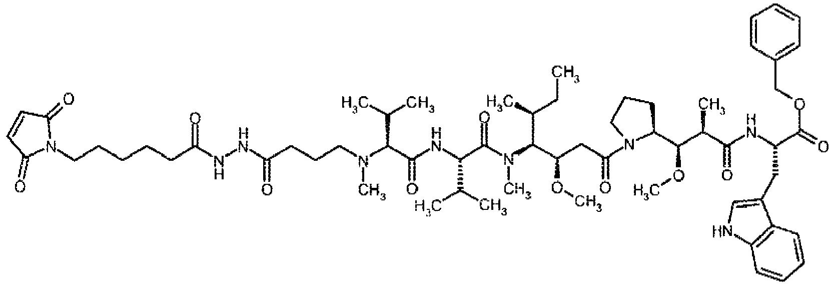

9 mg (9.5 µmol) *N*-(3-Carboxypropyl)-*N*-methyl-L-valyl-*N*-[(3*R*,4*S*,5*S*)-1-{(2*S*)-2-[(1*R*,2*R*)-3-{[(2*S*)-1-(benzyloxy)-3-(1*H*-indol-3-yl)-1-oxopropan-2-yl]amino}-1-methoxy-2-methyl-3-oxopropy]pyrrolidin-1-yl}-3-methoxy-5-methyl-1-oxoheptan-4-yl]-*N-*methyl-L-valinamid (Intermediat 72) wurden in 1000 µl DMF gelöst und anschließend mit 10 mg (38 µmol) von kommerziell erhältlichem 6-(2,5-Dioxo-2,5-dihydro-1*H*-pyrrol-1-yl)hexanhydrazid, 7.2 mg (19µmol) *O*-(7-Azabenzotriazol-1-yl)-*N*,*N*,*N'*,*N*'-tetramethyluronium-hexafluorophosphat und 8 µl *N*,*N-*Diisopropylethylamin versetzt und das Reaktionsgemisch 1 h bei RT gerührt. Anschließend wurde das Lösungsmittel im Vakuum entfernt und der verbliebene Rückstand mittels präparativer HPLC aufgereinigt. Die entsprechenden Fraktionen wurden eingeengt und durch Lyophilisation wurden 6.4 mg (58% d.Th.) der Titelverbindung als farbloser Schaum erhalten.
HPLC (Methode 5): Rₜ = 1.9 min;
LC-MS (Methode 1): Rₜ = 0.99 min; MS (ESIpos): m/z = 1154 (M+H)⁺.

### Intermediat 143

### N-(4-{2-[4-(2,5-Dioxo-2,5-dihydro-1H-pyrrol-1-yl)-2,2-dimethylbutanoyl]hydrazino}-4-oxobutyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

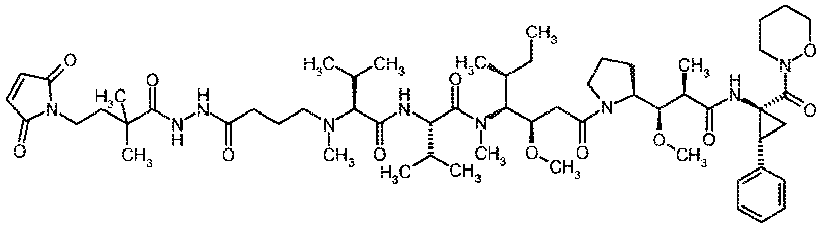

6 mg (6.7 µmol) *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl] amino}-3-oxopropyl]pyrrolidin-1-yl}-5-methyl-1-oxoheptan-4-yl]-*N*-methyl-L-valinamid (Intermediat 61) wurden mit 3 mg (8.7 µmol) 4-(2,5-Dioxo-2,5-dihydro-1*H*-pyrrol-yl)-2,2-dimethylbutanhydrazid-Trifluoracetat in Analogie zu Intermediat 142 zu 2 mg (27% d.Th.) der Titeleverbindung umgesetzt.
HPLC (Methode 12): Rₜ = 2.1 min;
LC-MS (Methode 3): Rₜ = 1.92 min; MS (ESIpos): m/z = 1106 (M+H)⁺.

### Intermediat 144

### N-(4-{2-[4-(2,5-Dioxo-2,5-dihydro-1H-pyrrol-1-yl)-2,2-dimethylbutanoyl]hydrazino}-4-oxobutyl)-N-methyl-L-valyl-N-[(3R,4S,5S)-3-methoxy-1-{(2S)-2-[(1R,2R)-1-methoxy-2-methy]-3-{[(2S)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid

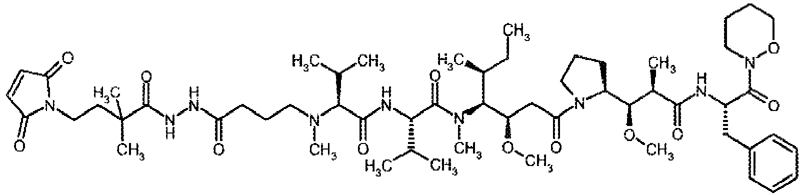

Zu einer Lösung von 5 mg (5.6 µmol) *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(1,2-oxazinan-2-yl)-1-oxo-3-phenylpropan-2-yl]amino}-3-oxopropyl]pyrrolidin-1-yl}-5-methyl-1-oxoheptan-4-yl]-*N*-methyl-L-valinamid in 1 ml DMF wurden 7.65 mg (22.5 µmol) 4-(2,5-Dioxo-2,5-dihydro-1H-pyrrol-1-yl)-2,2-dimethylbutanhydrazid-Trifluoracetat, 3.2 mg (16.9 µmol) EDC, 1.96 µl (11.3 µmol) Diisopropylethylamin sowie 2.6 mg (16.9 µmol) HOBT gegeben. Das Reaktionsgemisch wurde 3 h bei RT gerührt. Anschließend wurden weitere 0.95 mg (2.8 µmol) 4-(2,5-Dioxo-2,5-dihydro-1H-pyrrol-1-yl)-2,2-dimethylbutanhydrazid-Trifluoracetat nachgegeben. Nach Rühren über Nacht wurde das Reaktionsgemisch eingeengt und per präparativer HPLC gereinigt. Es wurden 3.5 mg (85%ig, 48% d.Th.) der Titelverbindung erhalten.
LC-MS (Methode 3): Rₜ = 1.86 min; m/z = 1094 (M+H)⁺.

### Intermediat 145

### N-[3-(2,5-Dioxo-2,5-dihydro-1H-pyrrol-1-yl)propyl]-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenyl cyclopropyl]amino}-3-oxopropyl]pyrrolidin-1-yl}-5-methyl-1-oxoheptan-4-yl]-N-methyl-L-valinamid

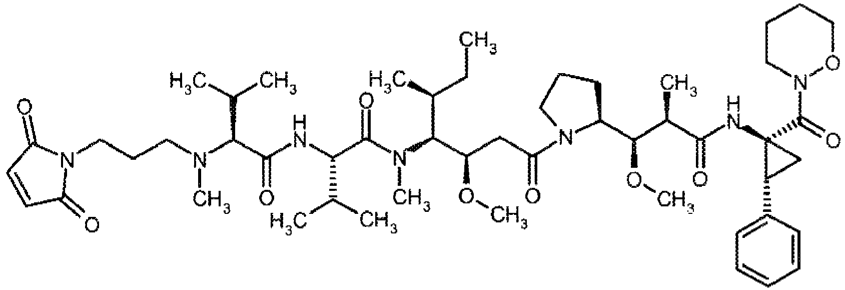

12 mg (14 µmol) *N*-(3-Aminopropyl)-*N*-methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl] amino}-3-oxopropyl]pyrrolidin-1-yl}-5-methyl-1-oxoheptan-4-yl]-*N*-methyl-L-valinamid (Intermediat 66) wurden in 750 µl Dioxan aufgenommen und mit 1.5 ml gesättigter Natriumhydrogencarbonat-Lösung und anschließend mit 3.2 mg (21 µmol) Methyl-2,5-dioxo-2,5-dihydro-1*H*-pyrrol-1-carboxylat versetzt. Das Reaktionsgemisch wurde 1h bei RT gerührt und danach im Vakuum eingeengt. Der verbliebene Rückstand wurde mittels präparativer HPLC gereinigt. Nach Lyophilisation wurden 4.2 mg (32% d.Th.) der Titelverbindung erhalten.
HPLC (Methode 5): Rₜ = 1.7 min;
LC-MS (Methode 1): Rₜ = 0.94 min; MS (ESIpos): m/z = 950 (M+H)⁺.

### Intermediat 146

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-({(2S)-1-[benzyl(methyl)amino]-1-oxo-3-phenylpropan-2-yl}amino)-1-methoxy-2-methyl-3-oxopropyl]pyrrolidin-1-yl}-3-methoxy-5-methyl-1-oxoheptan-4-yl]-N-methyl-L-valinamid

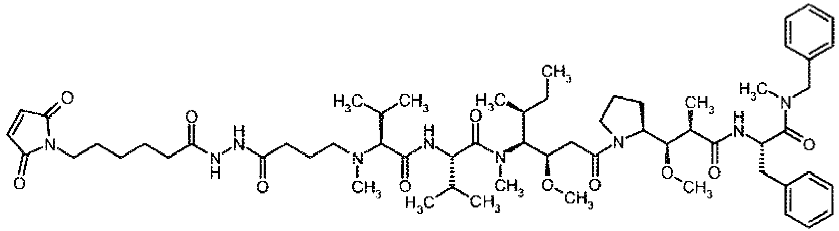

9 mg (9.8 µmol) *N*-(3-Carboxypropyl)-*N*-methyl-L-valyl-*N*-[(3*R*,4*S*,5*S*)-1-{(2*S*)-2-[(1*R*,2*R*)-3-({(2*S*)-1-[henzyl(methyl)amino]-1-oxo-3-phenylpropan-2-yl}amino)-1-methoxy-2-methyl-3-oxopropyl]pyrrolidin-1-yl}-3-methoxy-5-methyl-1-oxoheptan-4-yl]-*N*-methyl-L-valinamid (Intermediat 73) wurden in Analogie zu Intermediat 133 mit 10 mg (39 µmol) 6-(2,5-Dioxo-2,5-dihydro-1*H*-pyrrol-1-yl)hexanhydrazid zu 1.8 mg (15% d.Th.) der Titelverbindung umgesetzt.
HPLC (Methode 12): Rₜ = 2.2 min;
LC-MS (Methode 9): Rₜ = 5.11 min; MS (ESIpos): m/z = 1128 (M+H)⁺.

### Intermediat 147

### N-{4-[(2,5-Dioxopyrrolidin-1-yl)oxy]-4-oxobutyl}-N-methyl-L-valyl-N-[(3R,4S,5S)-1-{(2S)-2-[(1R,2R)-3-{[(2S,3S)-1-(benzyloxy)-1-oxo-3-phenylbutan-2-yl]amino}-1-methoxy-2-methyl-3-oxopropyl]pyrrolidin-1-yl}-3-methoxy-5-methyl-1-oxoheptan-4-yl]-N-methyl-L-valinamid

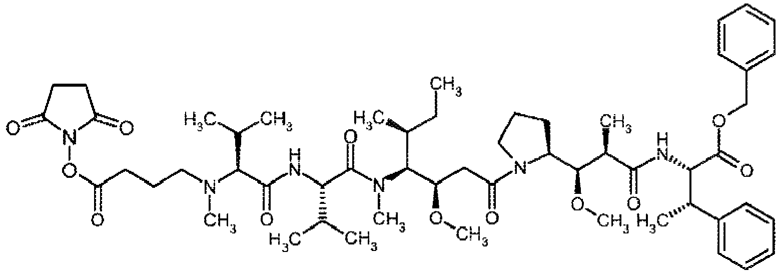

16 mg (17 µmol) *N*-(3-Carboxypropyl)-*N*-methyl-L-valyl-*N*-[(3*R*,4*S*,5*S*)-1-{(2*S*)-2-[(1*R*,2*R*)-3-{[(2*S*,3*S*)-1-(benzyloxy)-1-oxo-3-phenylbutan-2-yl]amino}-1-methoxy-2-methyl-3-oxo-propyl]pyrrolidin-1-yl}-3-methoxy-5-methyl-1-oxoheptan-4-yl]-*N-*methyl-L-valinamid (Intermediat 70) wurden in 2 ml Dichlormethan gelöst und mit 2.6 mg (23 mmol) 1-Hydroxypyrrolidin-2,5-dion und anschließend mit 4 mg (21 µmol) 1-(3-Dimethylaminopropyl)-3-ethylcarbodiimid-Hydrochlorid versetzt. Nach 2 h Rühren bei RT wurden nochmals die gleichen Mengen an 1-Hydroxypyrrolidin-2,5-dion und 1-(3-Dimethylaminopropyl)-3-ethylcarbodiimid-Hydrochlorid nachgesetzt. Dann Rühren über Nacht bei RT wurde das Reaktionsgemisch im Vakuum eingeengt. Der verbliebene Rückstand wurde mittels präparativer HPLC gereinigt. Nach Lyophilisation wurden 10 mg (56% d.Th.) der Titelverbindung erhalten.
HPLC (Methode 5): Rₜ = 2.0 min;

### Intermediat 148

### N-{4-[(2-{[4-(2,5-Dioxo-2,5-dihydro-1H-pyrrol-1-yl)butanoyl](methyl)amino}ethyl)amino]-4-oxobutyl}-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

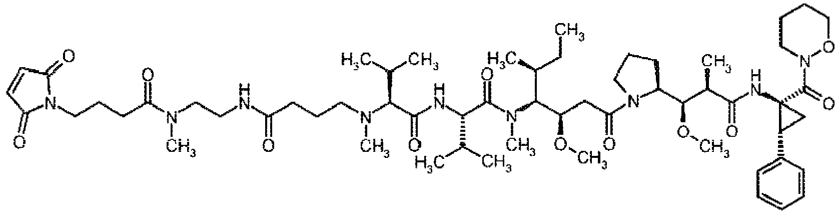

6 mg (7 µmol) *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl] amino}-3-oxopropyl]pyrrolidin-1-yl}-5-methyl-1-oxoheptan-4-yl]-*N*-methyl-L-valinamid (Intermediat 61) wurden mit 2.8 mg (8 µmol) *N*-(2-Aminoethyl)-4-(2,5-dioxo-2,5-dihydro-1*H-*pyrrol-1-yl)-*N*-methylbutanamid-Trifluoracetat, 10.1 mg (27 µmol) *O*-(7-Azabetizotriazol-1-yl)-*N*,*N*,*N'*,*N'*-tetramethyluronium-hexafluorophosphat und 5 µl *N*,*N*-Diisopropylethylamin in 2 ml DMF zusammengegeben und über Nacht bei RT gerührt. Dann wurden nochmals 5 mg (23.5 µmol) *O*-(7-Azabenzotriazol-1-yl)-*N,N*,*N*',*N*'-tetramethyluronium-hexafluorophosphat und 3 µl *N,N-*Diisopropylethylamin nachgesetzt. Nach weiteren 5 h Rühren bei RT wurde das Lösungsmittel im Vakuum entfernt und der verbliebene Rückstand mittels präparativer HPLC aufgereinigt. Die entsprechenden Fraktionen wurden eingeengt und durch Lyophilisation aus Dioxan wurden 1.3 mg (15% d.Th.) der Titelverbindung erhalten.
HPLC (Methode 12): Rₜ = 2.1 min;
LC-MS (Methode 2): Rₜ = 1.21 min; MS (ESIpos): m/z = 1120 (M+H)⁺.

### Intermediat 149

### N-{4-[(2-{[4-(2,5-Dioxo-2,5-dihydro-1H-pyrrol-1-yl)butanoyl]amino}ethyl)(methyl)amino]-4-oxobutyl}-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

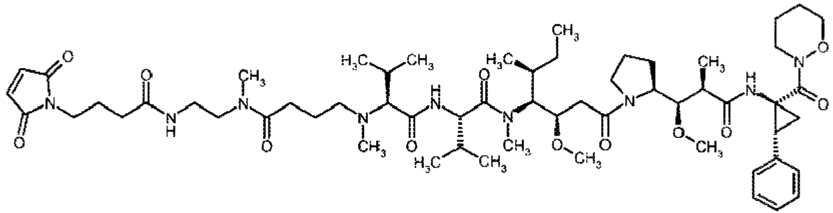

6 mg (7 µmol) *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl] amino}-3-oxopropyl]pyrrolidin-1-yl}-5-methyl-1-oxoheptan-4-yl]-*N*-methyl-L-valinamid (Intermediat 61) wurden mit 3.1 mg (9 µmol) 4-(2,5-Dioxo-2,5-dihydro-1*H*-pyrrol-1-yl)-*N*-[2-(methylamino)ethyl]butanamid-Trifluoracetat, 10.1 mg (27 µmol) O-(7-Azabenzotriazol-1-yl)-*N*,*N*,*N'*,*N'*-tetramethyluronium-hexafluorophosphat und 5 µl *N,N*-Diisopropylethylamin in 2 ml DMF zusammengegeben und 4 h bei RT gerührt. Dann wurde das Lösungsmittel im Vakuum entfernt und der verbliebene Rückstand mittels präparativer HPLC aufgereinigt. Die entsprechenden Fraktionen wurden eingeengt und durch Lyophilisation aus Dioxan wurden 1 mg (13.4% d.Th.) der Titelverbindung erhalten.
HPLC (Methode 12): Rₜ = 2.1 min;
LC-MS (Methode 1): Rₜ = 0.89 min; MS (ESIpos): m/z = 1121 (M+H)⁺.

### Intermediat 150

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-3-methoxy-1-{(2S)-2-[(1R,2R)-1-methoxy-2-methyl-3-oxo-3-{[(1S,2R)-2-phenyl-1-(propylcarbamoyl)cyclopropyl]amino}propyl]pyrrolidin-1-yl}-5-methyl-1-oxoheptan-4-yl]-N-methyl-L-valinamid

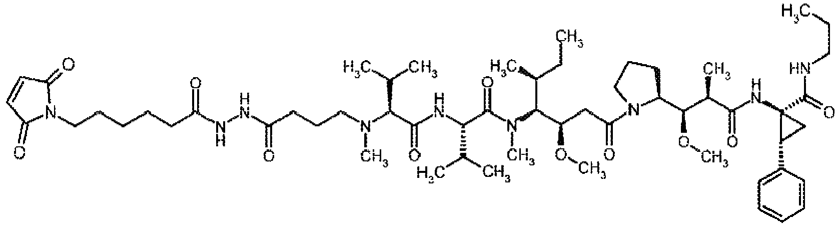

7.9 mg (9 µmol) *N*-(3-Carboxypropyl)-*N*-methyl-L-valyl-*N*-[(3*R*,4*S*,5*S*)-3-methoxy-1-{(2*S*)-1-[(1*R*,2*R*)-1-methoxy-2-methyl-3-oxo-3-{[(1*S*,2*R*)-2-phenyl-1-(propylcarbamoyl)cyclopropyl] amino}propyl]pyrrolidin-1-yl}-5-melhyl-1-oxoheptan-4-yl]-*N*-methyl-1-valinamid wurden in 3 ml DMF gelöst und anschließend mit 10.4 mg (54 µmol) 1-(3-Dimethylaminopropyl)-3-ethylcarbodiimid-Hydrochlorid, 8.3 mg (54 µmol) 1-Hydroxy-1*H*-benzotriazol-Hydrat, 9 µL *N,N-*Diisopropylethylamin sowie mit 9.5 mg (36 µmol) von kommerziell erhältlichem 6-(2,5-Dioxo-2,5-dihydro-1*H*-pyrrol-1-yl)hexanhydrazid versetzt. Die Mischung wurde über Nacht bei RT gerührt und anschließend im Hochvakuum eingeengt. Der verbliebene Rückstand wurde mittels präparativer HPLC aufgereinigt. So wurden 4.3 mg (22% d.Th.) der Titelverbindung als farbloser Schaum erhalten.
HPLC (Methode 6): Rₜ = 1.9 min;
LC-MS (Methode 9): Rₜ = 4.93 min; MS (ESIpos): m/z = 1078 (M+H)⁺.

### Intermediat 151

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(1S,2R)-1-carbamoyl-2-phenylcyclopropyl]amino}-1-methoxy-2-methyl-3-oxopropyl]pyrrolidin-1-yl}-3-methoxy-5-methyl-1-oxoheptan-4-yl]-N-methyl-L-valinamid

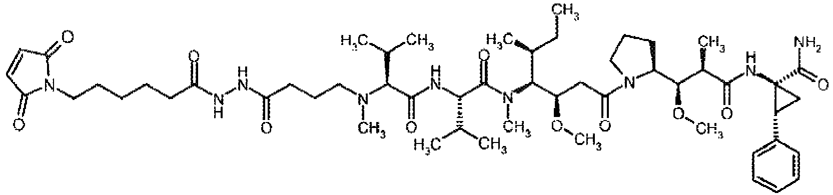

Die Verbindung wurde ausgehend von der Verbindung in Intermediat 81 analog zu Intermediat 150 hergestellt.
HPLC (Methode 5): Rₜ = 1.7 min;
LC-MS (Methode 1): Rₜ = 0.87 min; MS (ESIpos): m/z = 1036 (M+H)⁺.

### Intermediat 152

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(1S,2R)-1-(ethoxycarbonyl)-2-phenylcyclopropyl] amino}-1-methoxy-2-methyl-3-oxopropyl]pyrrolidin-1-yl}-3-methoxy-5-methyl-1-oxoheptan-4-yl]-N-methyl-L-valinamid

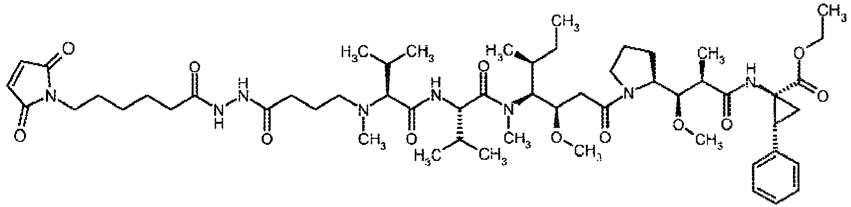

10 mg (12 µmol) *N*-(3-Carboxypropyl)-*N*-melhyl-L-valyl-*N*-[(3*R*,4*S*,5*S*)-1-{(2*S*)-2-[(1*R*,2*R*)-3-{[(1*S*,2*R*)-1-(ethoxycarbonyl)-2-phenylcyclopropyl]amino}-1-methoxy-2-methyl-3-oxopropyl] pyrrolidin-1-yl}-3-methoxy-5-methyl-1-oxoheptan-4-yl]-*N*-methyl-L-valinamid wurden in 3 ml DMF gelöst und anschließend mit 8.9 mg (23 µmol) *O*-(7-Azabenzotriazol-1-yl)-*N,N,N',N'-*tetramethyluronium-hexafluorophosphat, 10 µL *N,N*-Diisopropylethylamin sowie mit 12 mg (47 µmol) von kommerziell erhältlichem 6-(2,5-Dioxo-2,5-dihydro-1*H*-pyrrol-1-yl)hexanhydrazid versetzt. Die Mischung wurde 1 h bei RT gerührt. Anschließend wurde im Hochvakuum eingeengt und der verbliebene Rückstand mittels präparativer HPLC aufgereinigt. So wurden 5.8 mg (37 % d.Th.) der Titelverbindung als farbloser Schaum erhalten.
HPLC (Methode 6): Rₜ = 2.0 min;
LC-MS (Methode 9): Rₜ = 4.99 min; MS (ESIpos): m/z = 1066 (M+H)⁺.

### Intermediat 153

### N-[1-(2,5-Dioxo-2,5-dihydro-1H-pyrrol-1-yl)-12,15-dioxo-3,6,9-trioxa-13,14-diazaoctadecan-18-yl]-N-methyl-L-valyl-N-[(3R,4S,5S)-3-methoxy-1-{(2S)-2-[(1R,2R)-1-methoxy-2-methyl-3-{[(2S)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid

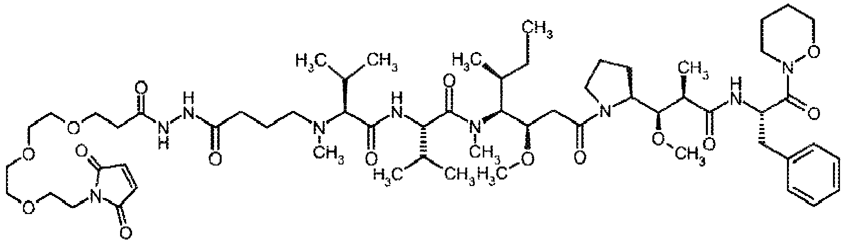

Zu einer Lösung von 5 mg (5.6 µmol) *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(1,2-oxazinan-2-yl)-1-oxo-3-phenylpropan-2-yl]amino}-3-oxopropyl]pyrrolidin-1-yl}-5-methyl-1-oxoheptan-4-yl]-*N*-methyl-L-valinamid in 1 ml DMF wurden 9.7 mg (22.5 µmol) 3-(2-{2-[2-(2,5-dioxo-2,5-dihydro-1*H*-pyrrol-1-yl)ethoxy]ethoxy}ethoxy)propanhydrazid-Trifluoracetat, 3.2 mg (16.9 µmol) EDC, 1.96 µl (11.3 µmol) N,N-Diisopropylethylamin sowie 2.6 mg (16.9 µmol) HOBT gegeben. Das Reaktionsgemisch wurde 3 h bei RT gerührt. Anschließend wurden weitere 1.2 mg (2.8 µmol) 3-(2-{2-[2-(2,3-dioxo-2,5-dihydro-1H-pyrrol-1-yl)ethoxy]ethoxy}ethoxy)propanhydrazid-Trifluoracetat nachgegeben. Das Reaktionsgemisch wurde über Nacht bei RT gerührt und anschließend per präparativer HPLC gereinigt.
Es wurden 3.6 mg (51% d.Th.) der Titelverbindung erhalten.
LC-MS (Methode 1): Rₜ = 0.90 min; m/z = 1185 (M+H)⁺.

### Intermediat 154

### (2R,3S)-3-Amino-4-{2-[6-(2,5-dioxo-2,5-dihydro-1H-pyrrol-1-yl)hexanoyl]hydrazino}-4-oxobutan-2-yl-(3R,4S,7S,10S)-4-[(2S)-butan-2-yl]-7,10-diisopropyl-3-(2-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-2-oxoethyl)-5,11-dimethyl-6,9-dioxo-2-oxa-5,8,11-triazapentadecan-15-oat

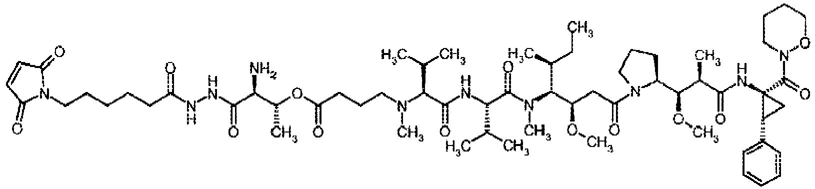

15 mg (17 µmol) *N*-(3-Carboxypropyl)-*N*-methyl-L-valyl-*N*-[(3*R*,4*S*,5*S*)-3-methoxy-1-{(2*S*)-2-[(1*R*,2*R*)~1-methoxy-2-methyl-3-{[(1*S*,2*R*)-1-(1,2-oxazinan-2-ylcarbonyl)-2-phenylcyclopropyl] amino}-3-oxopropyl]pyrrolidin-1-yl}-5-methyl-1-oxoheptan-4-yl]-*N*-methyl-L-valinamid wurden in 10 ml Dichlormethan gelöst und anschließend mit 12.8 mg (67 µmol) 1-(3-Dimethylaminopropyl)-3-ethylcarbodiimid-Hydrochlorid, 10 mg (83 µmol) 4-Dimethylaminopyridin sowie mit 10.3 mg (33 µmol) von kommerziell erhältlichem Benzyl-*N*-(*tert*.-butoxycarbonyl)-L-threoninat versetzt. Die Mischung wurde 4 h zum Rückfluß erhitzt. Dann wurden erneut die gleichen Mengen an Kupplungsreagenz und 4-Dimethylaminopyridin nachgesetzt und das Reaktionsgemisch über Nacht unter Rückfluß erhitzt. Anschließend wurde das Reaktionsgemisch mit Dichlormethan verdünnt, einmal mit Wasser ausgeschüttelt, die organische Phase abgetrennt und im Hochvakuum eingeengt. Der verbliebene Rückstand wurde mittels präparativer HPLC aufgereinigt. So wurden 7.7 mg (37% d. Th.) des geschützten Intermediats als farbloser Schaum erhalten.
HPLC (Methode 12): Rₜ = 2.5 min;
LC-MS (Methode 1): Rₜ = 1.13 min; MS (ESIpos): m/z = 1190 (M+H)⁺.

Anschließend wurde die Benzylester-Schutzgruppe durch Hydrierung unter Wasserstoff-Normaldruck in Methanol über 10% Palladium/Aktivkohle entfernt, und die so erhaltene Säure wie in Intermediat 151 beschrieben mit 6-(2,5-Dioxo-2,5-dihydro-1*H*-pyrrol-1-yl)hexanhydrazid verknüpft. In einem letzten Schritt wurde die Boc-Schutzgruppe mit Trifluoressigsäure abgelöst. Der verbliebene Rückstand wurde mittels präparativer HPLC aufgereinigt. So wurden 0.22 mg (2.5% d. Th. über 3 Stufen) der Titelverbindung als farbloser Schaum erhalten.
HPLC (Methode 12): Rₜ = 2.0 min:
LC-MS (Methode 1): Rₜ = 0.81 min; MS (ESIpos): m/z = 1207 (M+H)⁺.

### Intermediat 155

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(2S)-1-amino-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

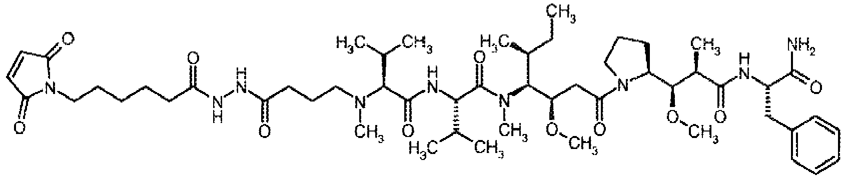

Diese Verbindung wurde in Analogie zur in Intermediat 152 beschriebenen Synthese aus *N*-(3-Carboxypropyl)-*N*-methyl-L-valyl-*N*-[(3*R*,4*S*,5*S*)-1-{(2*S*)-2-[(1*R*,2*R*)-3-{[(2*S*)-1-amino-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid und kommerziell erhältlichem 6-(2,5-Dioxo-2,5-dihydro-1*H*-pyrrol-1-yl)hexanhydrazid hergestellt.
HPLC (Methode 5): Rₜ = 1.6 min;
LC-MS (Methode 1): Rₜ = 0.82 min; MS (ESIpos): m/z = 1024 (M+H)⁺.

### Intermediat 156

### N-(3-{[(1-{[(2,5-Dioxopyrrolidin-1-yl)oxy]carbonyl}cyclopropyl)carbonyl]amino}propyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

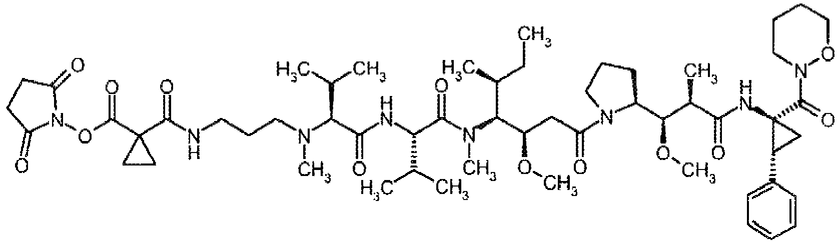

Diese Verbindung wurde in Analogie zur letzten Stufe der in Intermediat 131 beschriebenen Synthese aus *N*-(3-Aminopropyl)-*N-*methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl]amino}-3-oxopropyl]pyrrolidin-1-yl}-5-methyl-1-oxoheptan-4-yl]-*N*-methyl-L-valinamid und 1,1'-[Cyclopropan-1,1-diylbis(carbonyloxy)]dipyrrolidin-2,5-dion, das zuvor aus der entsprechenden Dicarbonsäure gewonnen wurde, hergestellt.
HPLC (Methode 12): Rₜ = 2.0 min;
LC-MS (Methode 1): Rₜ = 0.92 min; MS (ESIpos): m/z = 1080 (M+H)⁺.

### Intermediat 157

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

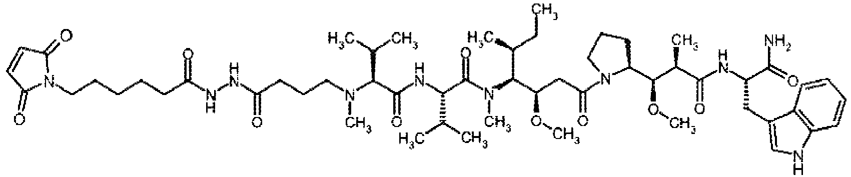

15 mg (18 µmol) (*N*-(3-Carboxypropyl)-*N*-methyl-L-valyl-*N*-[(3*R*,4*S*,5*S*)-1-{(2*S*)-2-[(1*R*,2*R*)-3-{[(2*S*)-1-amino-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid wurden in 3.8 ml DMF gelöst und anschließend mit 27 mg (70 µmol) *O*-(7-Azabenzotriazol-1-yl)-*N,N,N',N'*-tetramethyluronium-hexafluorophosphat, 12 µL *N,N*-Diisopropylethylamin sowie mit 14 mg (53 µmol) von kommerziell erhältlichem 6-(2,5-Dioxo-2,5-dihydro-1*H*-pyrrol-1-yl)hexanhydrazid versetzt. Das Reaktionsgemisch wurde 1 h bei RT gerührt. Anschließend wurde im Hochvakuum eingeengt und der verbliebene Rückstand mittels präparativer HPLC aufgereinigt. So wurden 6.2 mg (33 % d.Th.) der Titelverbindung als farbloser Schaum erhalten.
HPLC (Methode 5): Rₜ = 1.6 min;
LC-MS (Methode 1): Rₜ = 0.85 min; MS (ESIpos): m/z = 1063 (M+H)⁺.

¹H-NMR (500 MHz, DMSO-d₆, charakteristische Signale): δ = 10.8 (d, 1H), 9.8-9.7 (m, 2H), 9.6 und 9.4 (2m, 1H), 8.9, 8.88, 8.78 und 8.75 (4d, 1H), 8.08 und 7.85 (2d, 1H), 7.6-6.9 (m, 9H), 4.7-4.4 (m, 3H), 3.4 (t, 2H), 3.23, 3.2, 3.18, 3.0, und 2.99 (5s, 911), 2.8 (m, 3H), 2.1 (t, 2H), 1.06 und 1.01 (2d, 3H), 0.95-0.8 (m, 15H), 0.8-0.75 (dd, 3H).

### Intermediat 158

### N-[4-({(2R)-1-[(2,5-Dioxopyrrolidin-1-yl)oxy]-4-methyl-1-oxopentan-2-yl}amino)-4-oxobutyl]-N-methyl-L-valyl-N-[(3R,4S,5S)-1-{(2S)-2-[(1R,2R)-3-{[(2S)-1-(benzylamino)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

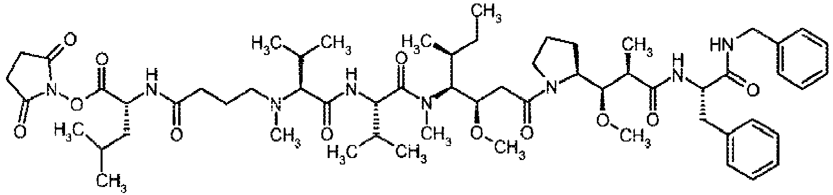

13 mg (14.7 µmol) *N-*(3-Carboxypropyl)-*N*-methyl-L-valyl-*N*-[(3*R*,4*S*,5*S*)-1-{(2*S*)-2-[(1*R*,2*R*)-3-{[(2*S*)-1-(benzylamino)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid wurden in 4 ml Dimethylformamid gelöst und anschließend mit 9.4 mg (25 µmol) *O*-(7-Azabenzotriazol-1-yl)-*N,N,N',N'*-tetramethyluronium-hexafluorophosphat, 6 µL *N,N*-Diisopropylethylamin sowie mit 7 mg (31 µmol) von kommerziell erhältlichem *tert*.-Butyl-D-leucinat-Hydrochlorid versetzt. Die Mischung wurde 5h bei RT gerührt und anschließend im Vakuum eingeengt. Der verbliebene Rückstand wurde mittels präparativer HPLC aufgereinigt. Nach Lyophilisation aus Dioxan/Wasser wurden 6.5 mg (49% d. Th.) des geschützten Intermediats als farbloser Schaum erhalten.
HPLC (Methode 5): Rₜ = 2.2 min;
LC-MS (Methode 1): Rₜ = 1.21 min; MS (ESIpos): m/z = 1076 (M+H)⁺.

Aus diesem geschützten Intermediat wurde zunächst mit Trifluoressigsäure in Dichlormethan die Boc-Schutzgruppe abgspalten, wobei 6.2 mg (99% d.Th.) der entschützten Verbindung erhalten wurden. 5.2 mg (5µmol) dieses Intermediats wurden in 1.5 ml Dichlormethan aufgenommen und mit 0.8 mg (7 µmol) *N*-Hydroxysuccinimid in Gegenwart von 1.2 mg (6 µmol) 1-(3-Dimethylaminopropyl)-3-ethylcarbodiimid-Hydrochlorid und 0.16 mg (1 µmol) 4-Dimethylaminopyridin umgesetzt. Nach 2h Rühren bei RT wurde das Reaktionsgemisch eingeengt und mittels präparativer HPLC gereinigt. Es wurden 1.3 mg der Titelverbindung erhalten, die teilweise zum Edukt hydrolysierte.

### Intermediat 159

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(2S)-1-(benzylamino)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

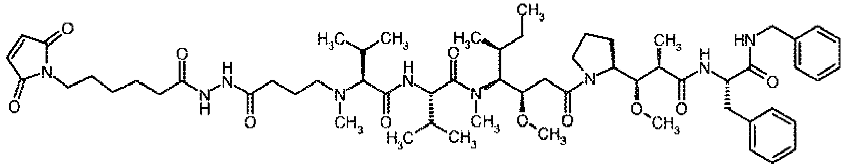

Diese Verbindung wurde in Analogie zur in Intermediat 157 beschriebenen Synthese aus *N*-(3-Carboxypropyl)-*N*-methyl-L-valyl-*N*-[(3*R*,4*S*,5*S*)-1-{(2*S*)-2-[(1*R*,2*R*)-3-{[(2*S*)-1-(benzylamino)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid und kommerziell erhältlichem 6-(2,5-Dioxo-2,5-dihydro-1*H*-pyrrol-1-yl)hexanhydrazid hergestellt.
Ausbeute: 6 mg (53% d.Th.)
HPLC (Methode 5): Rₜ = 1.9 min;
LC-MS (Methode 1): Rₜ = 0.94 min; MS (ESIpos): m/z = 1114 (M+H)⁺.

### Intermediat 160

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(2S)-1-(benzylamino)-3-(1H-indol-3-yl)-1-oxopropan-2-yl]amino}-1-methoxy-1-methyl-3-oxopropyl]pyrrolidin-1-yl}-3-methoxy-5-methyl-1-oxoheptan-4-yl]-N-methyl-L-valinamid

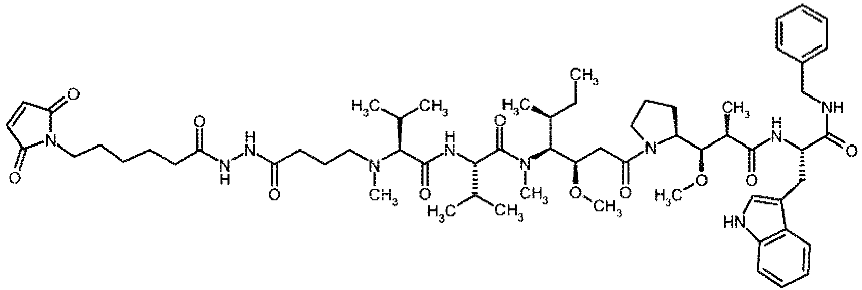

Diese Verbindung wurde in Analogie zur in Intermediat 157 beschriebenen Synthese aus 20 mg (21 µmol) *N*-(3-Carboxypropyl)-*N*-methyl-L-valyl-*N-*[(3*R*,4*S*,5*S*)-1-{(2*S*)-2-[(1*R*,2*R*)-3-{[(2*S*)-1-(benzylamino)-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid und kommerziell erhältlichem 6-(2,5-Dioxo-2,5-dihydro-1*H*-pyrrol-1-yl)hexanhydrazid hergestellt.
Ausbeute: 13 mg (52% d.Th.)
HPLC (Methode 5): Rₜ = 1.9 min;
LC-MS (Methode 1): Rₜ = 0.92 min; MS (ESIpos): m/z = 1153 (M+H)⁺.

### Intermediat 161

### N-(6-{2-[6-(2,5-Dioxo-2,5-dihydro-1H-pyrrol-1-yl)hexanoyl]hydrazino}-6-oxohexyl)-N-methyl-L-valyl-N-[(3R,4S.5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

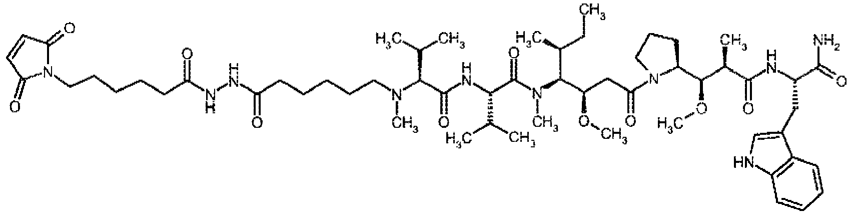

Diese Verbindung wurde in Analogie zur in Intermediat 157 beschriebenen Synthese aus *N*-(5-Carboxypentyl)-*N*-methyl-L-valyl-*N*-[(3*R*,4*S*,5*S*)-1-{(2*S*)-2-[(1*R*,2*R*)-3-{[(2*S*)-1-amino-3-(1*H-*indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid und kommerziell erhältlichem 6-(2,5-Dioxo-2,5-dihydro-1*H*-pyrrol-1-yl)hexanhydrazid hergestellt.
Ausbeute: 0.8 mg (16% d.Th.)
HPLC (Methode 5): Rₜ = 1.6 min;
LC-MS (Methode 1): Rₜ = 0.78 min; MS (ESIpos): m/z = 1092 (M+H)⁺.

### Intermediat 162

### N-{6-[(2,5-Dioxopyrrolidin-1-yl)oxy]-6-oxohexyl}-N-methyl-1-valyl-N-[(3R,4S,5S)-3-methoxy-1-{(2S)-2-[(1R,2R)-1-melhoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

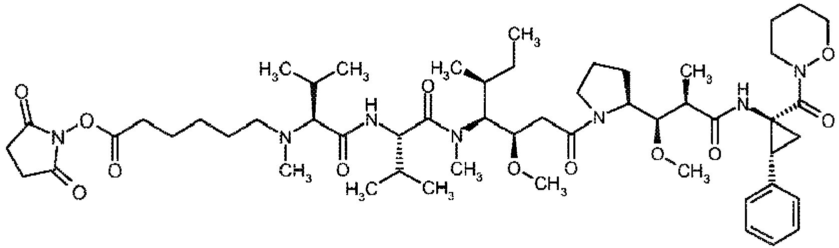

18 mg (20 µmol) *N*-(5-Carboxypentyl)-*N*-methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl] amino}-3-oxopropyl]pyrrolidin-1-yl}-5-methyl-1-oxoheptan-4-yl]-*N*-methyl-L-valinamid (Intermediat 64) wurden in 3.2 ml Dichlormethan gelöst und mit 22 mg (190 mmol) 1-Hydroxypyrrolidin-2,5-dion und anschließend mit 11 mg (60 µmol) 1-(3-Dimethylaminopropyl)-3-ethylcarbodiimid-Hydrochlorid und 0.24 mg (0.17 µmol) DMAP versetzt. Nach 2 h Rühren bei RT wurden erneut 22 mg (190 mmol) 1-Hydroxypyrrolidin-2,5-dion, 11 mg (60 µmol) 1-(3-Dimethylaminopropyl)-3-ethylcarbodiimid-Hydrochlorid und 0.24 mg (0.17 µmol) DMAP zugegeben und das Reaktionsgemisch eine weitere Stunde bei RT gerührt. Anschließend wurde im Vakuum eingeengt. Der verbliebene Rückstand wurde mittels präparativer IIPLC gereinigt. Nach Lyophilisation wurden 8.2 mg (41% d.Th.) der Titelverbindung erhalten.
HPLC (Methode 5): Rₜ = 2.0 min;
LC-MS (Methode 11): Rₜ = 0.9 min; MS (ESIpos): m/z = 1024 (M+H)⁺.

### Intermediat 163

### [(1S,2R)-1-Amino-2-phenylcyclopropyl](1,4-dihydro-3H-2,3-benzoxazin-3-yl)methanon-Trifluoracetat

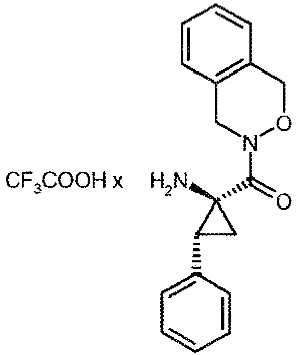

Zunächst wurde ausgehend von 265 mg (0.82 mmol) *tert*.-Butyl-[(1*S*,2*R*)-1-(hydroxycarbanoyl)-2-phenylcyclopropyl]carbamat (Ausgangsverbindung 7) durch Reaktion mit 1,2-Bis(brommethyl)benzol analog einer Literaturvorschrift (siehe H. King, J. Chem. Soc. 1942, 432) das Boc-geschützte Intermediat tert.-Butyl-[(1S,2R)-1-(1,4-dihydro-3H-2,3-benzoxazin-3-ylcarbonyl)-2-phenylcyclopropyl]carbamathergestellt.
Ausbeute: 108 mg (34% d. Th.)
LC-MS (Methode 2): Rₜ = 1.3 min; MS (ESIpos): m/z = 395 (M+H)⁺.

108 mg (0.27 mmol) dieses Intermediats wurden in 3.7 ml Dichlormethan aufgenommen, mit 1.8 ml Trifluoressigsäure versetzt und 15 min bei RT gerührt. Anschließend wurde im Vakuum eingeengt und der verbliebene Rückstand aus Dioxan lyophilisiert. Es wurden 112 mg der Titelverbindung in quantitativer Ausbeute als farbloser Schaum erhalten.
LC-MS (Methode 1): Rₜ = 0.7 min; MS (ESIpos): m/z = 295 (M+H)⁺.

### Intermediat 164

### N-Methyl-L-valyl-N-[(3R,4S,5S)-1-{(2S)-2-[(1R,2R)-3-{[(1S,2R)-1-(1,4-dihydro-3H-2,3-benzoxazin-3-ylcarbonyl)-2-phenylcyclopropyl]amino}-1-methoxy-2-methyl-3-oxopropyl]pyrrolidin-1-yl}-3-methoxy-5-methyl-1-oxoheptan-4-yl]-N-methyl-L-valinamid-Trifluoracetat

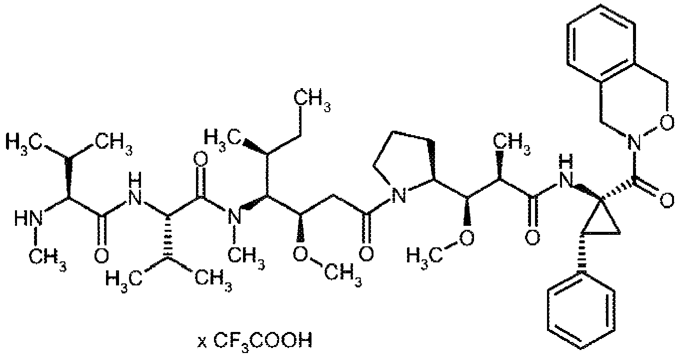

166 mg (0.196 mmol) *N*-[(9*H*-Fluoren-9-ylmethoxy)carbonyl]-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 10) wurden in 40 ml DMF aufgenommen und nacheinander mit 80 mg (0.196 mmol) [(1*S*,2*R*)-1-Amino-2-phenylcyclopropyl](1,4-dihydro-3*H*-2,3-benzoxazin-3-yl)methanon-Trifluoracetat (Intermediat 163), 112 mg (0.294 mmol) *O*-(7-Azabenzotriazol-1-yl)-*N,N,N',N'*-tetramethyluronium-hexafluorophosphat (HATU) sowie 682 µl (3.9 mmol) *N,N*-Diisopropylethylamin versetzt. Die Mischung wurde anschließend über Nacht bei RT gerührt. Das Reaktionsgemisch wurde danach im Vakuum eingeengt, der Rückstand in Ethylacetat aufgenommen und die Lösung mit gesättigter wässriger Natriumchlorid-Lösung gewaschen. Die organische Phase wurde über Magnesiumsulfat getrocknet, filtriert und eingeengt. Der Rückstand wurde schließlich durch präparative HPLC gereinigt. Man erhielt auf diese Weise 19 mg (9% d. Th.) des Fmoc-geschützten Intermediats *N*-[(9*H*-Fluoren-9-ylmethoxy)carbonyl]-*N*-methyl-L-valyl-*N-*[(3*R*,4*S*,5*S*)-1-{(2*S*)-2-[(1*R*,2*R*)-3-{[(1*S*,2*R*)-1-(1,4-dihydro-3*H*-2,3-benzoxazin-3-ylcarbonyl)-2-phenylcyclopropyl]amino}-1-methoxy-2-methyl-3-oxopropyl]pyrrolidin-1-yl}-3-methoxy-5-methyl-1-oxoheptan-4-yl]-*N-*methyl-L-valinamid.
HPLC (Methode 5): Rₜ = 1.68 min;
LC-MS (Methode 1): Rₜ = 1.51 min; MS (ESIpos): m/z = 1083 (M+H)⁺.

19 mg (0.015 mmol) dieses Intermediats wurden in 4 ml DMF gelöst. Nach Zugabe von 817 µl Piperidin wurde das Reaktionsgemisch 5 min bei RT gerührt. Anschließend wurde im Vakuum eingeengt und der Rückstand zunächst mit Diethylether digeriert und anschließend mittels präparativer HPLC gereinigt (Eluent: Acetonitril + 0.1% TFA / 0.1% aq. TFA). Die entsprechenden Fraktionen wurden vereinigt, das Lösungsmittel im Vakuum entfernt und der Rückstand dann aus Dioxan/Wasser lyophilisiert. Es wurden 12 mg (92% d. Th.) der Titelverbindung als farbloser Schaum erhalten.
HPLC (Methode 6): Rₜ = 2.0 min;
LC-MS (Methode 1): Rₜ = 0.94 min; MS (ESIpos): m/z = 861 (M+H)⁺.

### Intermediat 165

### N-(6-Aminohexyl)-N-methyl-L-valyl-N-[(3R,4S,5S)-1-{(2S)-2-[(1R,2R)-3-{[(1S,2R)-1-(1,4-dihydro-3H-2,3-benzoxazin-3-ylcarbonyl)-2-phenylcyclopropyl]amino}-1-methoxy-2-methyl-3-oxopropyl]pyrrolidin-1-yl}-3-methoxy-5-methyl-1-oxoheptan-4-yl]-N-methyl-L-valinamid

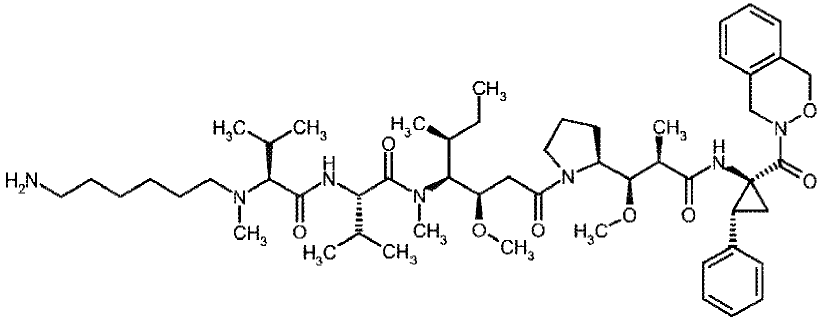

Aus 20 mg (0.021 mmol) von Intermediat 164 wurden in Analogie zur Herstellung von Intermediat 97 mit Benzyl-(6-oxohexyl)carbamat in Gegenwart von Natriumcyanoborhydrid und nachfolgender hydrogenolytischer Abspaltung der Z-Schutzgruppe (mit 5% Palladium auf Kohle als Katalysator, in Methanol als Lösungsmittel) die Titelverbindung hergestellt.
Ausbeute: 4.5 mg (23% d. Th. Über 2 Stufen)
HPLC (Methode 12): Rₜ = 1.9 min:
LC-MS (Methode 1): Rₜ = 0.9 min; MS (ESIpos): m/z = 960 (M+H)⁺.

### Intermediat 166

### N-[6-(2,5-Dioxo-2,5-dihydro-1H-pyrrol-1-yl)hexyl]-N-methyl-L-valyl-N-[(3R,4S,5S)-1-{(2S)-2-[(1R,2R)-3-{[(1S,2R)-1-(1,4-dihydro-3H-2,3-benzoxazin-3-ylcarbonyl)-2-phenylcyclopropyl]amino}-1-methoxy-2-methyl-3-oxopropyl]pyrrolidin-1-yl}-3-methoxy-5-methyl-1-oxoheptan-4-yl]-N-methyl-L-valinamid

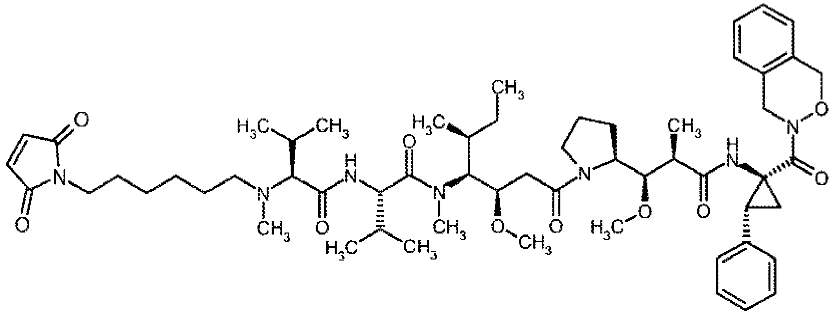

4.4 mg (4.5 µmol) von Intermediat 165 wurden in 1 ml Dioxan/Wasser 1:1 aufgenommen und anschließend mit 1 mg (6.8 µmol) Methyl-2,5-dioxo-2,5-dihydro-1H-pyrrol-1-carboxylat sowie mit 50µl gesättigter wässriger Natriumhydrogencarbonat-Lösung versetzt. Das Reaktionsgemisch wurde 30 min bei RT gerührt. Dann wurden nochmals 50 µl der gesättigten wässrigen Natriumhydrogencarbonatlösung zugegeben und das Reaktionsgemisch weitere 15 min bei RT gerührt und anschließend im Vakuum eingeengt. Der verbliebene Rückstand wurde mittels präparativer HPLC gereinigt. Nach Lyophilisaten wurden 1 mg (21% d.Th.) der Titelverbindung als farbloser Schaum erhalten.
HPLC (Methode 12): Rₜ = 2.1 min;
LC-MS (Methode 1): Rₜ = 1.08 min; MS (ESIpos): m/z = 1040 (M+H)⁺.

### Intermediat 167

### Benzyl-3-{2-[2-(2-oxoethoxy)ethoxy]ethoxy}propanoat

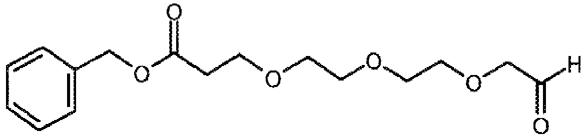

Die Titelverbindung wurde aus 6 g (21.55 mmol) von kommerziell erhältlicher 3-{2-[2-(2-Hydroxyethoxy)ethoxy]ethoxy}propansäure nach Standardbedingungen zunächst durch Veresterung mit Benzylchlorid und Caesiumcarbonat und anschließender Oxidation mit Schwefeltrioxid-Pyridin-Komplex hergestellt.
Ausbeute: 611 mg (10% d.Th. über 2 Stufen)
LC-MS (Methode 2): Rₜ = 1.69 min; MS (ESIpos): m/z = 311 (M+H)⁺.

### Intermediat 168

### N-(2-{2-[2-(2-Carboxyethoxy)ethoxy]ethoxy}ethyl)-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

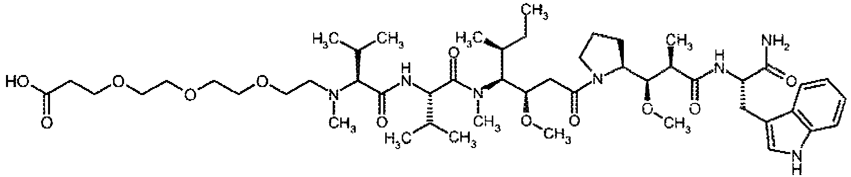

Zunächst wurde in Analogie zu der in den Intermediat 69 beschriebenen Synthese durch Kupplung von *N*-[(9*H*-Fluoren-9-ylmethoxy)carbonyl]-*N*-methyl-L-valyl-*N*-[(2*R*,3*S*,4*S*)*-*1-carboxy-2-methoxy-4-methylhexan-3-yl]-*N*-methyl-L-valinamid (Intermediat 4) und *N^{α}*-{(2*R*,3*R*)-3-Methoxy-2-methyl-3-[(2*S*)-pyrrolidin-2-yl]propanoyl}-L-tryptophanamid-Trifluoracetat (Intermediat 49) in Gegenwart von *O*-(7-Azabenzotriazol-1-yl)-*N,N,N',N'*-tetramethyluronium-hexafluorophosphat und anschließende Abspaltung der Fmoc-Schutzgruppe mittels Piperidin die Amin-Verbindung *N-*Methyl-L-valyl-*N*-[(3*R*,4*S*,5*S*)-1-{(2*S*)-2-[(1*R*,2*R*)-3-{[(2*S*)-1-amino-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-ethyl-L-valinamid als Trifluoracetat hergestellt.

25 mg (0.028 mmol) dieser Verbindung und 17.5 mg (0.06 mmol) von Intermediat 167 wurden in 2 ml Methanol zusammengegeben und mit 12.6 mg (0.14 mmol) Boran-Pyridin-Komplex und 2.5 ml Essigsäure versetzt. Das Reaktionsgemisch wurde über Nacht bei RT gerührt. Dann wurden nochmals die gleichen Mengen Boran-Pyridin-Komplcx und Essisäure zugegeben und das Reaktionsgemisch weitere 24h bei RT gerührt. Anschließend wurde im Vakuum eingeengt und der Rückstand mittels präparativer IIPLC gereinigt. Nach Einengen der entsprechenden Fraktionen und Lyophilisation aus Dioxan/Wasser 1:1 wurden 26.5 mg (88% d.Th.) der Z-geschützten Titelverbindung erhalten.
HPLC (Methode 12): Rₜ = 2.04 min;
LC-MS (Methode 1): Rₜ = 0.97 min; MS (ESIpos): m/z = 1064 (M+H)⁺.

25 mg (0.024 mmol) dieses Intermediats wurden in 10 ml Methanol aufgenommen und über 10%-igem Palladium auf Aktivkohle 45 min lang bei RT unter Wasserstoff-Normaldruck hydriert. Der Katalysator wurde danach abfiltriert und das Lösungsmittel im Vakuum entfernt. Nach Lyophilisation aus Dioxan erhielt man 19.7 mg (85% d.Th.) der Titelverbindung.
HPLC (Methode 12): Rₜ = 1.8 min:
LC-MS (Methode 1): Rₜ = 0.83 min; MS (ESIpos): m/z = 974 (M+H)⁺.

### Intermediat 169

### N-{2-[2-(2-{3-[(2,5-Dioxopyrrolidin-1-yl)oxy]-3-oxopropoxy}ethoxy)ethoxy]ethyl}-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

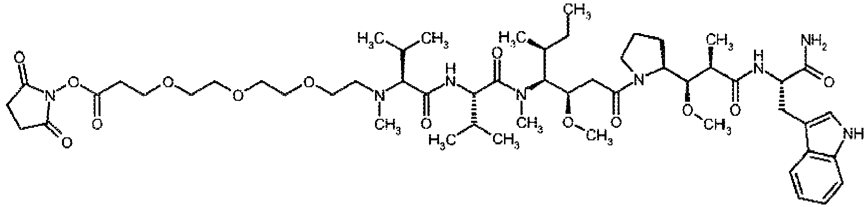

10 mg (10 µmol) von Intermediat 168 wurden in 3 ml DMF gelöst und mit 3.5 mg (30 mmol) 1-Hydroxypyrrolidin-2,5-dion und anschließend mit 2.4 mg (10 µmol) 1-(3-Dimethylaminopropyl)-3-ethylcarbodiimid-Hydrochlorid und 5 µl N,N-Diisopropylethylamin versetzt. Nach 20 h Rühren bei RT wurden 8 mg (0.02 mmol) HATU zugegeben und das Reaktionsgemisch nochmals über Nacht bei RT gerührt und dann im Vakuum eingeengt. Der verbliebene Rückstand wurde mittels präparativer HPLC gereinigt. Nach Lyophilisation aus Dioxan wurden 8.6 mg (64% d.Th.) der Titelverbindung erhalten.
HPLC (Methode 12): Rt = 1.9 min;
LC-MS (Methode 11): Rt = 0.81 min; MS (ESIpos): m/z = 1071 (M+H)+.

### Intermediat 170

### N-(6-Aminohexyl)-N-methyl-L-valyl-N-[(3R,4S,5S)-3-methoxy-1-{(2S)-2-[(1R,2R)-1-methoxy-2-methyl-3-{[(2S,3S)-1-(1,2-oxazinan-2-yl)-1-oxo-3-phenylbutan-2-yl]amino}-3-oxopropyl]pyrrolidin-1-yl}-5-methyl-1-oxoheptan-4-yl]-N-methyl-L-valinamid

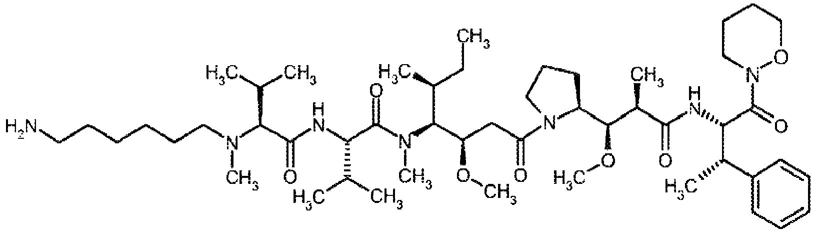

Diese Verbindung wurde in Analogie zu Intermediat 101 über 2 Stufen ausgehend von 26 mg (0.028 mmol) Intermediat 15 hergestellt.
Ausbeute: 16.7 mg (63% d.Th. über 2 Stufen)
IIPLC (Methode 12): Rₜ = 1.9 min;
LC-MS (Methode 1): Rₜ = 0.81 min; MS (ESIpos): m/z = 914 (M+H)⁺.

### Intermediat 171

### N-(6-{[4-(2,5-Dioxo-2,5-dihydro-1H-pyrrol-1-yl)butanoyl]amino}hexyl)-N-methyl-L-valyl-N-[(3R,4S,5S)-3-methoxy-1-{(2S)-2-[(1R,2R)-1-methoxy-2-methyl-3-{[(2S,3S)-1-(1,2-oxazinan-2-yl)-1-oxo-3-phenylbutan-2-yl]amino}-3-oxopropyl]pyrrolidin-1-yl}-5-methyl-1-oxoheptan-4-yl]-N-methyl-L-valinamid

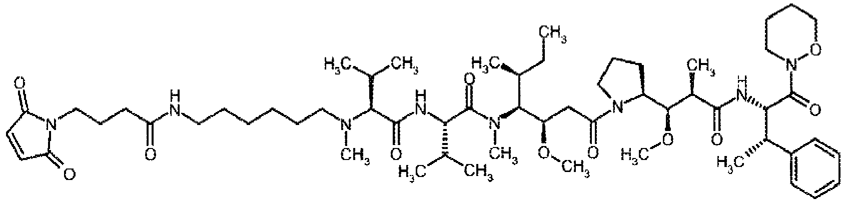

6.7 mg (7.3 µmol) der Verbindung aus Intermediat 170 und 3 mg (14.7 µmol) von kommerziell erhältlicher 4-(2,5-Dioxo-2,5-dihydro-1H-pyrrol-1-yl)butansäure wurden in 2 ml DMF aufgenommen und mit 5.6 mg (14.7 µmol) *O*-(7-Azabenzotriazol-1-yl)-*N,N,N',N'*,-tetramethyluronium-hexafluorophosphat (HATU) sowie 2 µl *N,N*-Diisopropylethylamin versetzt. Die Mischung wurde 30 min bei RT gerührt. Das Reaktionsgemisch wurde eingeengt und der Rückstand mittels präparativer HPLC gereinigt. Die entsprechenden Fraktionen wurden vereinigt, das Lösungsmittel im Vakuum entfernt und der Rückstand anschließend aus Dioxan lyophilisiert. So wurden 4.5 mg (56% d.Th.) der Titelverbindung erhalten.
HPLC (Methode 12): Rₜ = 2.0 min;
LC-MS (Methode 1): Rₜ = 1.12 min; MS (ESIpos): m/z = 1079 (M+H)⁺.

### Intermediat 172

### Benzyl-(2-{2-[2-(2-oxoethoxy)ethoxy]ethoxy}ethyl)carbamat

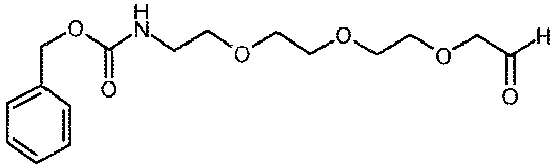

Die Titelverbindung wurde aus kommerziell erhältlichem 2-{2-[2-(2-Amino-ethoxy)ethoxy]ethoxy}ethanol nach Standardbedingungen zunächst durch Einführung der Z-Schutzgruppe und anschließender Oxidation mit Schwefeltrioxid-Pyridin-Komplex hergestellt.
HPLC (Methode 12): Rₜ = 1.4 min;
LC-MS (Methode 11): Rₜ = 0.65 min; MS (ESIpos): m/z = 326 (M+H)⁺.

### Intermediat 173

### Benzyl-{2-[2-(2-oxoethoxy)ethoxy]ethyl}carbamat

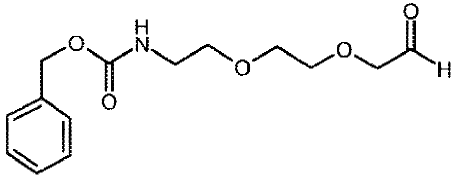

Die Titelverbindung wurde analog zu Intermediat 172 aus kommerziell erhältlichem 2-[2-(2-Aminoethoxy)ethoxy]ethanol nach Standardbedingungen zunächst durch Einführung der Z-Schutzgruppe und anschließender Oxidation mit Schwefeltrioxid-Pyridin-Komplex hergestellt.
HPLC (Methode 12): Rₜ = 1.3 min;
LC-MS (Methode 11): Rₜ = 0.68 min; MS (ESIpos): m/z = 282 (M+H)⁺.

### Intermediat 174

### N-(2-{2-[2-(2-Aminoethoxy)ethoxy]ethoxy}ethyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenyl cyclopropyl]amino}-3-oxopropyl]pyrrolidin-1-yl}-5-methyl-1-oxoheptan-4-yl]-N-methyl-L-valinamid

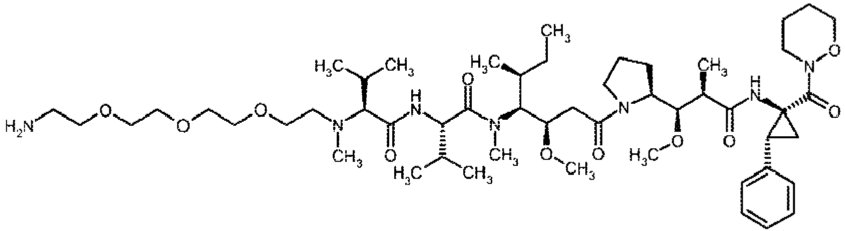

47 mg (0.05 mmol) von Intermediat 16 wurden in Analogie zur Herstellung von Intermediat 167 mit Benzyl-(2-{2-[2-(2-oxoethoxy)ethoxy]ethoxy}ethyl)carbamat in Gegenwart von Boran-Pyridin Komplex reduktiv aminiert. Anschließend wurde die Z-Schutzgruppe hydrogenolytisch mit 5% Palladium auf Kohle als Katalysator und in Methanol als Lösungsmittel entfernt und 38 mg (66% d.Th. über 2 Stufen) der Titelverbindung hergestellt.
HPLC (Methode 5): Rₜ = 1.7 min;
LC-MS (Methode 1): Rₜ = 0.8 min; MS (ESIpos): m/z = 988 (M+H)⁺.

### Intermediat 175

### N-[2-(2-{2-[2-(2,5-Dioxo-2,5-dihydro-1H-pyrrol-1-yl)ethoxy]ethoxy}ethoxy)ethyl]-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

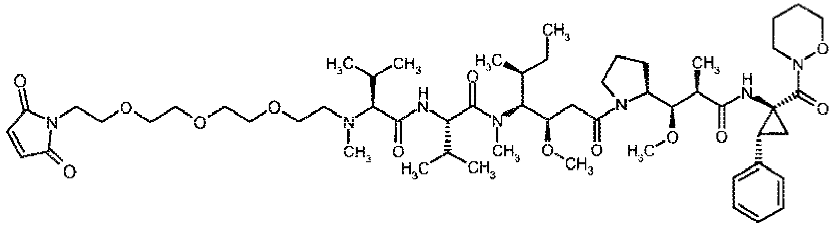

Die Herstellung erfolgte in Analogie zu Intermediat 166 ausgehend von 34 mg (0.03 mmol) von Intermediat 174.
Ausbeute: 8.3 mg (23% d.Th.)
HPLC (Methode 5): Rₜ = 1.9 min;
LC-MS (Methode 1): Rₜ = 0.97 min; MS (ESIpos): m/z = 1068 (M+H)⁺.

### Intermediat 176

### N-(2-{2-[2-(2,5-Dioxo-2,5-dihydro-1H-pyrrol-1-yl)ethoxy]ethoxy}ethyl)-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

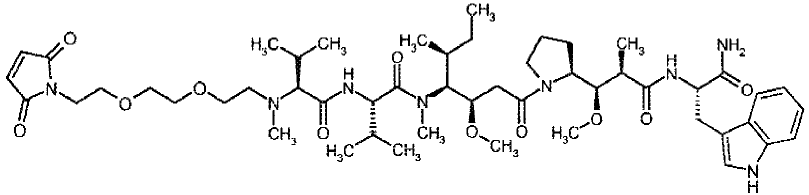

Die Herstellung erfolgte in Analogie zu den Intermediaten 174 und 175 beginnend mit der reduktive Aminierung von Intermediat 16 mit Intermediat 173, anschließender Entschützung und Aufbau des Maleinimids.
HPLC (Methode 12): Rₜ = 1.8 min;
LC-MS (Methode 11): Rₜ = 0.8 min; MS (ESIpos): m/z = 981 (M+H)⁺.

### Intermediat 177

### N-[2-(2-{2-[2-(2,5-Dioxo-2,5-dihydro-1H-pyrrol-1-yl)ethoxy]ethoxy}ethoxy)ethyl]-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

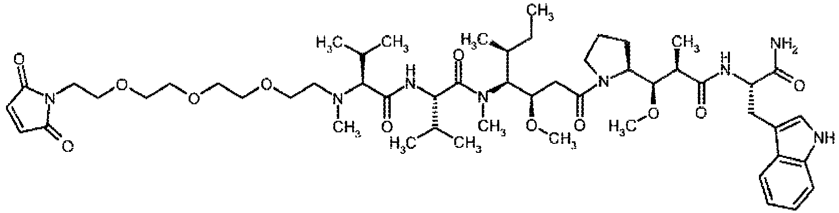

Die Herstellung erfolgte in Analogie zu den Intermediaten 174 und 175 beginnend mit der reduktive Aminierung von Intermediat 16 mit Intermediat 172, anschließender Entschützung und Aufbau des Maleinimids.
HPLC (Methode 12): Rₜ = 1.9 min;
LC-MS (Methode 1): Rₜ = 0.86 min; MS (ESIpos): m/z = 1025 (M+H.)⁺.

### Intermediat 178

### N-{4-[(2,5-Dioxopyrrolidin-1-yl)oxy]-4-oxobutyl}-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

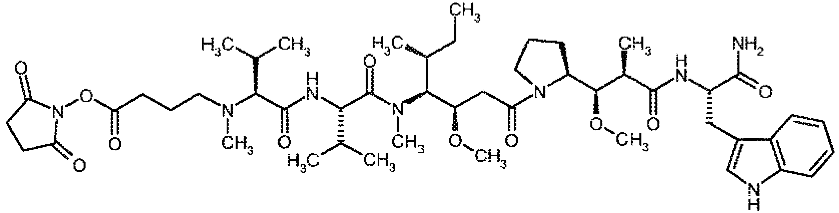

Die Herstellung erfolgte in Analogie zu Intermediaten 162 ausgehend von 6 mg von Intermediat 82.
LC-MS (Methode 1): Rₜ = 0.82 min; MS (ESIpos): m/z = 953 (M+H)⁺.

### Intermediat 179

### 4-[(1E,3S)-3-Amino-4-phenylbut-1-en-1-yl]benzolsulfonsäure-Trifluoracetat

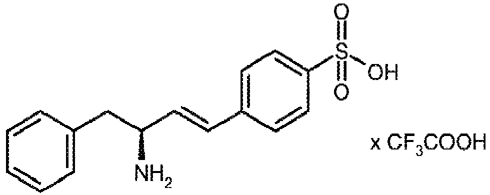

Eine Mischung aus 13.6 mg (0.06 mmol) Palladium(II)-Acetat, 469 mg (1.46 mmol) Kalium-4-iodbenzolsulfonat, 300 mg (1.21 mmol) (S)-tert.-Butyl-1-phenylbut-3-en-2-yl-carbamat, 16.5 mg (0.12 mmol) Phenylharnstoff und 167.6 mg (1.21 mmol) Kaliumcarbonat in 7.5 mL DMF wurde in der Mikrowelle für 15 min. auf 160°C erhitzt. Das Rohprodukt wurde anschließend direkt per präparativer HPLC gereinigt. Es wurden so 312 mg eines Gemisches aus 31% der BOC-geschützten Verbindung und 69% des freien Amins erhalten.

Dieses Gemisch wurde anschließend in 30 mL Dichlormethan aufgenommen, mit 1 mL Trifluoressigsäure versetzt und 20h bei RT gerührt. Nach Einengen im Vakuum wurde der Rückstand mit Diethylether verrührt, der entstandene Niederschlag abgesaugt und mit Diethylether nachgewaschen. Es wurden so 200 mg (62% d.Th.) der Titelverbindung erhalten.
LC-MS (Methode 11): Rₜ = 0.44 min; MS (ESIpos): m/z = 304 (M+H)⁺.

### Intermediat 180

### 4-[(3R)-3-Amino-4-phenylbutyl]benzolsulfonsäure

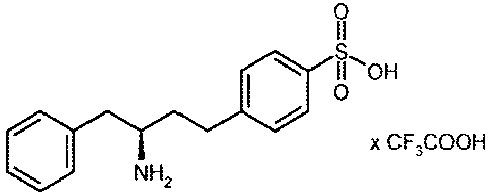

100 mg (0.25 mmol) 4-[(1E,3S)-3-Amino-4-phenylbut-1-en-1-yl]benzolsulfonsäure-Trifluoracetat wurden in 10 mL Essigsäure sowie einigen Tropfen DMF und Wasser suspendiert, mit 70 mg (0.07 mmol) Palladium auf Kohle (10%-ig) versetzt und 24h bei 2.2 bar Wasserstoff-Druck hydriert. Die Lösung wurde filtriert, das Filtrat per präp. HPLC gereinigt.
Es wurden 29 mg (76%ig, 21% d.Th.) Produkt erhalten.
LC-MS (Methode 1): Rₜ = 0.46 min; MS (ESIpos): m/z = 306 (M+H)⁺.

### Intermediat 181

### N-(tert.-Butoxycarbonyl)-N-methyl-L-valyl-N-[(3R,4S,5S)-3-methoxy-1-{(2S)-2-[(1R,2R)-1-methoxy-2-methyl-3-oxo-3-{[(2S,3E)-1-phenyl-4-(4-sulfophenyl)but-3-en-2-yl]amino}propyl]pyrrolidin-1-yl}-5-methyl-1-oxoheptan-4-yl]-N-methyl-L-valinamid

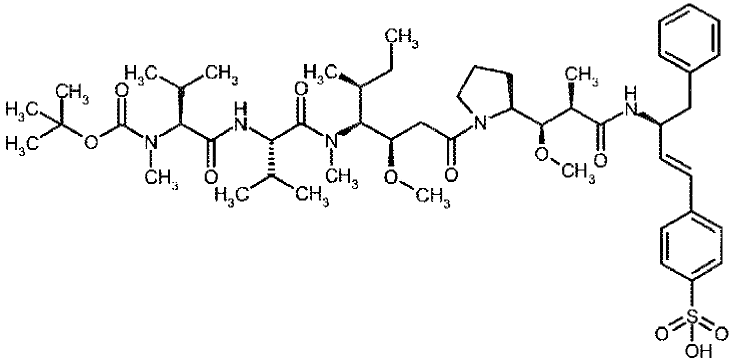

Zu einer Lösung von 90 mg (0.13 mmol) N-(tert.-Butoxycarbonyl)-N-methyl-L-valyl-N-[(3R,4S,5S)-1-{(2S)-2-[(1R,2R)-2-carboxy-1-methoxypropyl]pyrrolidin-1-yl}-3-methoxy-5-methyl-1-oxoheptan-4-yl]-N-methyl-L-valinamid in 4 mL DMF wurden 60 mg (0.16 mmol) HATU und 69 µL (0.39 mmol) Hünig-Base gegeben. Das Reaktionsgemisch wurde 30 min. bei RT gerührt und anschließend mit 60 mg (0.15 mmol) 60.3 mg (0.13 mmol) 4-[(1E,3S)-3-Amino-4-phenylbut-1-en-1-yl]benzolsulfonsäure-Trifluoracetat versetzt. Nach Rühren über Nacht wurde das Reaktionsgemisch per präp. HPLC gereinigt. Es wurden so 127 mg eines 44:56-Gemisches der Titelverbindung und des bereits entschützten Amins erhalten.

LC-MS (Methode 1): Rₜ = 1.21 min; MS (ESIpos): m/z = 971 (M+H)⁺; Rₜ = 0.84 min; MS (ESIpos): m/z = 871 (M+H)⁺ für die entschützte Verbindung.

### Intermediat 182

### N-Methyl-L-valyl-N-[(3R,4S,5S)-3-methoxy-1-{(2S)-2-[(1R,2R)-1-methoxy-2-methyl-3-oxo-3-{[(2S,3E)-1-phenyl-4-(4-sulfophenyl)but-3-en-2-yl]amino}propyl]pyrrolidin-1-yl}-5-methyl-1-oxoheptan-4-yl]-N-methyl-L-valinamid-Trifluoracetat

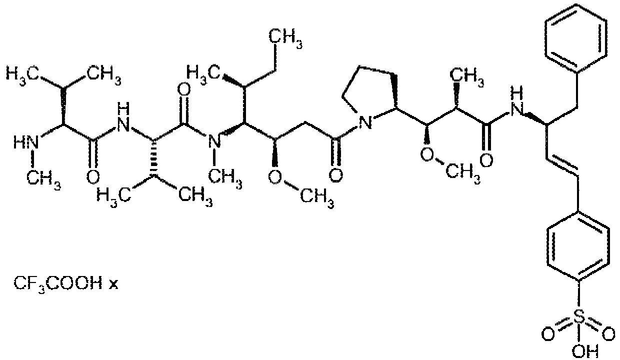

90 mg Intermediat 180 wurden in 4.6 mL Dichlormethan gelöst und mit 0.92 mL Trifluoressigsäure versetzt. Das Reaktionsgemisch wurde 30 min. bei RT gerührt und anschließend eingeengt. Das erhaltene Rohprodukt wurde per präp. HPLC gereinigt.
Es wurden 91 mg (98% d.Th.) der Zielverbindung erhalten.
LC-MS (Methode 1): Rₜ = 0.85 min; MS (ESIpos): m/z = 871 (M+H)⁺

### Intermediat 183

### N-(3-Carboxypropyl)-N-methyl-L-valyl-N-[(3R,4S,5S)-3-methoxy-1-{(2S)-2-[(1R,2R)-1-methoxy-2-methyl-3-oxo-3-{[(2S,3E)-1-phenyl-4-(4-sulfophenyl)but-3-en-2-yl]amino}propyl]pyrrolidin-1-yl}-5-methyl-1-oxoheptan-4-yl]-N-methyl-L-valinamid

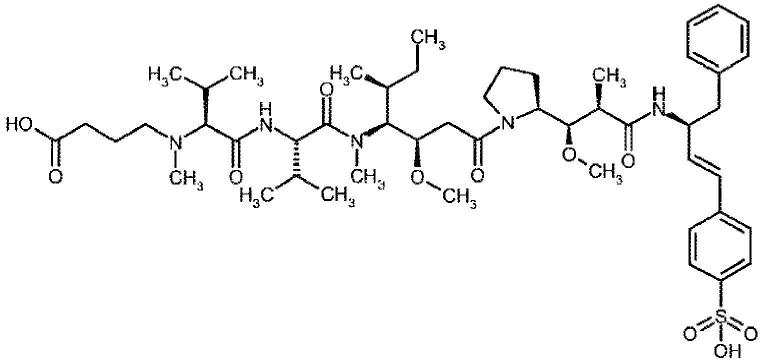

Es wurden 16.7 µL (0.03 mmol) einer 15%igen wässrigen Bernsteinaldehyd-Lösung in 943 µL Methanol vorgelegt und mit 17 mg (0.02 mmol) N-Methyl-L-valyl-N-[(3R,4S,5S)-3-methoxy-1-{(2S)-2-[(1R,2R)-1-methoxy-2-methyl-3-oxo-3-{[(2S,3E)-1-phenyl-4-(4-sulfophenyl)but-3-en-2-yl]amino}propyl]pyrrolidin-1-yl}-5-methyl-1-oxoheptan-4-yl]-N-methyl-L-valinamid-Trifluoracetat (Intermediat 181) sowie 1.1 µL (0.02 mmol) Essigsäure versetzt. Das Reaktionsgemisch wurde 5 min. bei RT gerührt und anschließend mit 2.9 µL (0.02 mmol) Boran-Pyridin-Komplex versetzt. Nach 1h wurden jeweils weitere 2 Äquivalente Bernsteinaldehyd, Essigsäure und Boran-Pyridin-Komplex zugegeben und 20h bei RT gerührt. Das Reaktionsgemisch wurde dann per präp. HPLC gereinigt.
Es wurden so 20 mg (83%ig, 80% d.Th.) der Titelverbindung erhalten.
LC-MS (Methode 1): Rₜ = 0.87 min; MS (ESIpos): m/z = 957 (M+H)⁺

### Intermediat 184

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-3-methoxy-1-{(2S)-2-[(1R,2R)-1-methoxy-2-methyl-3-oxo-3-{[(2S,3E)-1-phenyl-4-(4-sulfophenyl)but-3-en-2-yl]amino}propyl]pyrrolidin-1-yl}-5-methyl-1-oxoheptan-4-yl]-N-methyl-L-valinamid

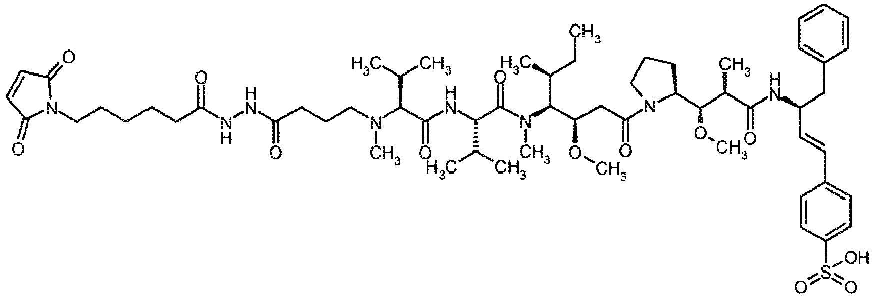

Es wurden 8 mg (7.5 µmol) N-(3-Carboxypropyl)-N-methyl-L-valyl-N-[(3R,4S,5S)-3-methoxy-1-{(2S)-2-[(1R,2R)-1-methoxy-2-methyl-3-oxo-3-{[(2S,3E)-1-phenyl-4-(4-sulfophenyl)but-3-en-2-yl]amino}propyl]pyrrolidin-1-yl}-5-methyl-1-oxoheptan-4-yl]-N-methyl-L-valinamid. 2.8 mg (8.2 µmοl) 6-(2,5-Dioxo-2,5-dihydro-1H-pyrrol-1-yl)hexanhydrazid-Trifluoracetat, 3.4 mg (9 µmol) HATU sowie 3.9 µL Hünig-Base in 0.77 mL DMF 20h bei RT gerührt. Anschließend wurde das Reaktionsgemisch per präp. HPLC gereinigt.

Es wurden 3 mg (31% d.Th.) der Titelverbindung erhalten.

LC-MS (Methode 1): Rₜ = 0.90 min; MS (ESIpos): m/z = 1164 (M+H)⁺

### Intermediat 185

### X-{4-[(2,5-Dioxopyrrolidin-1-yl)oxy]-4-oxobutyl}-N-methyl-L-valyl-N-[(3R,4S,5S)-3-methoxy-1-{(2S)-2-[(1R,2R)-1-methoxy-2-methyl-3-oxo-3-{[(2S,3E)-1-phenyl-4-(4-sulfophenyl)but-3-en-2-yl]amino}propyl]pyrrolidin-1-yl}-5-methyl-1-oxoheptan-4-yl]-N-methyl-L-valinamid

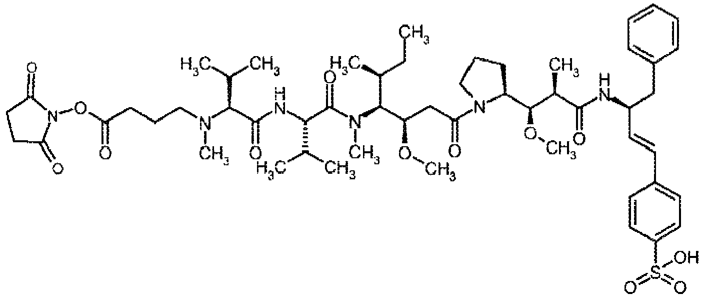

Zu einer Lösung von 8 mg (7.5 µmol) N-(3-Carboxypropyl)-N-methyl-L-valyl-N-[(3R,4S,5S)-3-methoxy-1-{(2S)-2-[(1R,2R)-1-methoxy-2-methyl-3-oxo-3-{[(2S,3E)-1-phenyl-4-(4-sulfophenyl)but-3-en-2-yl]amino}propyl]pyrrolidin-1-yl}-5-methyl-1-oxoheptan-4-yl]-N-methyl-L-valinamid in 2 mL DMF wurden 8.6 mg (74.8 µmol) N-Hydroxysuccinimid, 8.5 mg (22.4 µmol) EDCI und 0.1 mg (0.75 µmol) DMAP gegeben. Das Reaktionsgemisch wurde 20h bei RT gerührt. Anschließend wurden 1.3 µL (7.5 µmol) Hünig-Base zugegeben und 1h nachgerührt. Das Reaktionsgemisch wurde dann per präp. IIPLC gereinigt. Es wurden 2.6 mg (72% Reinheit; 21% d.Th.) der Titelverbindung erhalten.
LC-MS (Methode 1): Rₜ = 0.89 min; MS (ESIpos): m/z = 1054 (M+H)⁺

### Intermediat 186

### N-(tert.-Butoxycarbonyl)-N-methyl-L-valyl-N-[(3R,4S,5S)-3-methoxy-1-{(2S)-2-[(1R,2R)-1-methoxy-2-methyl-3-oxo-3-{[(2R)-1-phenyl-4-(4-sulfophenyl)butan-2-yl]amino}propyl]pyrrolidin-1-yl}-5-methyl-1-oxoheptan-4-yl]-N-methyl-L-valinamid

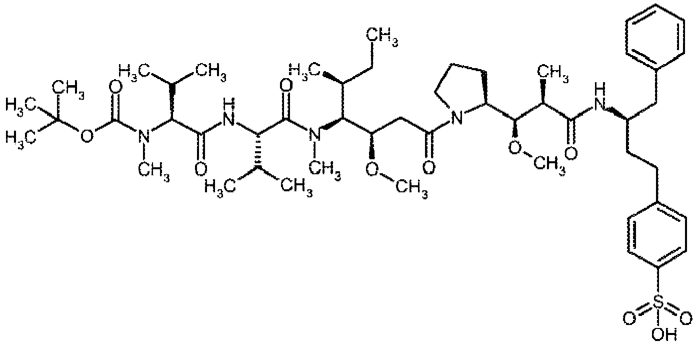

Zu einer Lösung von 43 mg (0.06 mmol) N-(tert.-Butoxycarbonyl)-N-methyl-L-valyl-N-[(3R,4S,5S)-1-{(2S)-2-[(1R,2R)-2-carboxy-1-methoxypropyl]pyrrolidin-1-yl}-3-methoxy-5-methyl-1-oxoheptan-4-yl]-N-methyl-L-valinamid in 1.9 mL DMF wurden 29 mg (0.07 mmol) HATU und 33 µL (0.19 mmol) Hünig-Base gegeben. Das Reaktionsgemisch wurde 30 min. bei RT gerührt und anschließend mit 29 mg (0.07 mmol) 4-[(3R)-3-Amino-4-phenylbutyl]benzolsulfonsäure-Trifluoracetat versetzt. Nach Rühren über Nacht wurde das Reaktionsgemisch per präp. HPLC gereinigt. Es wurden so 58 mg eines 45:55-Gemisches der Titelverbindung und des bereits entschützten Amins erhalten.

LC-MS (Methode 1): Rₜ = 1.09 min; MS (ESIpos): m/z = 973 (M+H)⁺; Rₜ = 0.87 min; MS (ESlpos): m/z = 873 (M+H)⁺ für die entschützte Verbindung.

### Intermediat 187

### N-Methyl-L-valyl-N-[(3R,4S,5S)-3-methoxy-1-{(2S)-2-[(1R,2R)-1-methoxy-2-methyl-3-oxo-3-{[(2R)-1-phenyl-4-(4-sulfophenyl)butan-2-yl]amino}propyl]pyrrolidin-1-yl}-5-methyl-1-oxoheptan-4-yl]-N-methyl-L-valinamid-Trifluoracetat

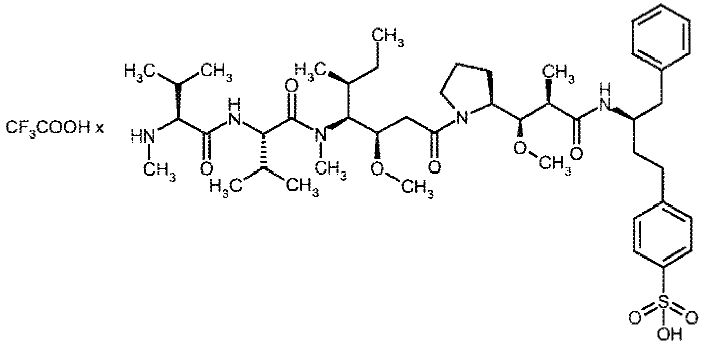

58 mg Intermediat 186 wurden in 4.1 mL Dichlormethan gelöst, mit 0.41 mL Trifluoressigsäure versetzt und 30 min. bei RT gerührt. Nach Einengen im Vakuum wurde das Rohprodukt per präp. HPLC gereinigt.
Es wurden 50 mg (90%-ige Reinheit; 85% d.Th.) Titelverbindung erhalten.
LC-MS (Methode 1): Rₜ = 0.87 min; MS (ESIpos): m/z = 873 (M+H)⁺

### Intermediat 188

### N-(3-Carboxypropyl)-N-methyl-L-valyl-N-[(3R,4S,5S)-3-methoxy-1-{(2S)-2-[(1R,2R)-1-methoxy-2-methyl-3-oxo-3-{[(2R)-1-phenyl-4-(4-sulfophenyl)butan-2-yl]amino}propyl]pyrrolidin-1-yl}-5-methyl-1-oxoheptan-4-yl]-N-methyl-L-valinamid

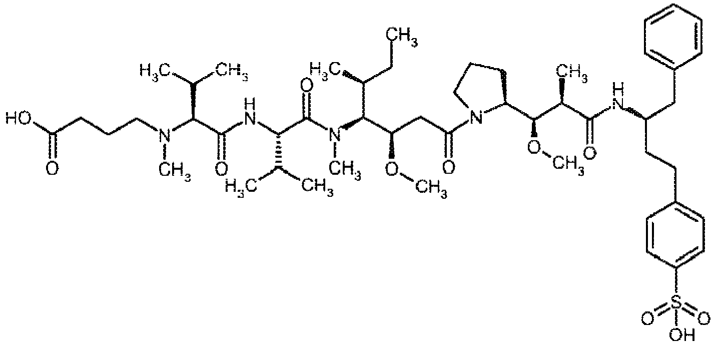

Es wurden 171µL (0.26 mmol) einer 15%igen wässrigen Bernsteinaldehyd-Lösung in 2.5 mL Methanol vorgelegt und mit 50 mg (0.05 mmol) N-Methyl-L-valyl-N-[(3R,4S,5S)-3-methoxy-1-{(2S)-2-[(1R,2R)-1-methoxy-2-methyl-3-oxo-3-{[(2R)-1-phenyl-4-(4-sulfophenyl)butan-2-yl]amino}propyl]pyrrolidin-1-yl}-5-methyl-1-oxoheptan-4-yl]-N-methyl-L-valinamid-Trifluoracetat sowie 11.6 µL (0.2 mmol) Essigsäure versetzt. Das Reaktionsgemisch wurde 5 min. bei RT gerührt und anschließend mit 30 µL (0.24 mmol) Boran-Pyridin-Komplex versetzt. Nach 24stündigem Rühren wurde ein weiteres Äquivalent Boran-Pyridin-Komplex zugegeben und 2h nachgerührt. Das Reaktionsgemisch wurde dann per präp. HPLC gereinigt.
Es wurden 40 mg (90%-ige Reinheit; 66% d.Th.) Titelverbindung erhalten.
LC-MS (Methode 1): Rₜ = 0.91 min; MS (ESIpos): m/z = 959 (M+H)⁺

### Intermediat 189

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-3-methoxy-1-{(2S)-2-[(1R,2R)-1-methoxy-2-methyl-3-{[(2R)-1-phenyl-4-(4-sulfophenyl)butan-2-yl]amino}propyl]pyrrolidin-1-yl}-5-methyl-1-oxoheptan-4-yl]-N-methyl-L-valinamid

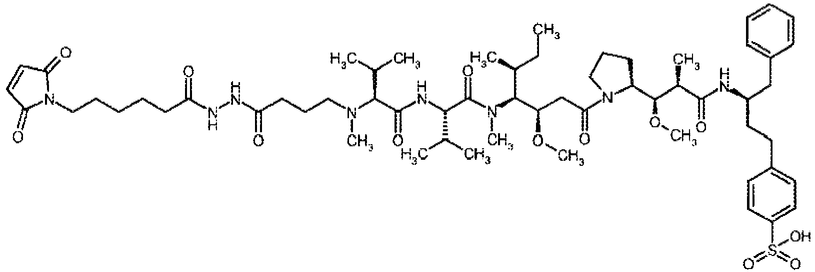

Es wurden 10 mg (9.3 µmol) N-(3-Carboxypropyl)-N-methyl-L-valyl-N-[(3R,4S,5S)-3-methoxy-1-{(2S)-2-[(1R,2R)-1-methoxy-2-methyl-3-oxo-3-{[(2R)-1-phenyl-4-(4-sulfophenyl)butan-2-yl]amino}propyl]pyrrolidin-1-yl}-5-methyl-1-oxoheptan-4-yl]-N-methyl-L-valinamid, 3.5 mg (10.3 µmol) 6-(2,5-Dioxo-2,5-dihydro-1H-pyrrol-1-yl)hexanhydrazid-Trifluoroacetat, 4.3 mg (11.2 µmol) HATU sowie 4.9 µL (28 µmol) Hünig-Base in 1 mL DMF 20h bei RT gerührt. Anschließend wurde das Reaktionsgemisch per präp. IIPLC gereinigt.
Es wurden 4.2 mg (92%-ige Reinheit; 33% d.Th.) der Titelverbindung erhalten.
LC-MS (Methode 1): Rₜ = 0.91 min; MS (ESIpos): m/z = 1166 (M+H)⁺

### Intermediat 190

### N-{4-[(2,5-Dioxopyrrolidin-1-yl)oxy]-4-oxobutyl}-N-methyl-L-valyl-N-[(3R,4S,5S)-3-methoxy-1-{(2S)-2-[(1R,2R)-1-methoxy-2-methyl-3-oxo-3-{[(2R)-1-phenyl-4-(4-sulfophenyl)butan-2-yl]amino}propyl]pyrrolidin-1-yl}-5-methyl-1-oxoheptan-4-yl]-N-methyl-L-valinamid

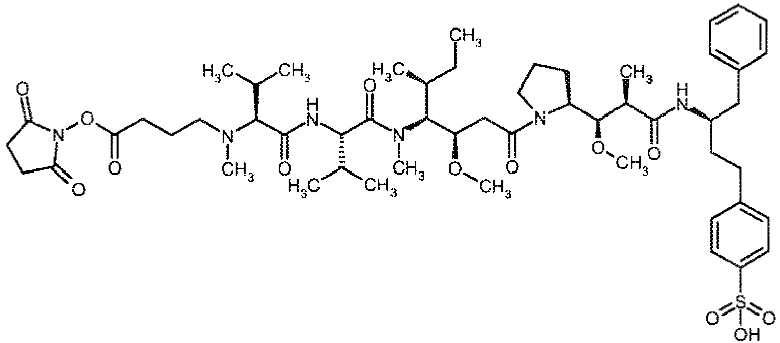

Zu einer Lösung von 10 mg (9.3 µmol) N-(3-Carboxypropyl)-N-methyl-L-valyl-N-[(3R,4S,5S)-3-methoxy-1-{(2S)-2-[(1R,2R)-1-methoxy-2-methyl-3-oxo-3-{[(2R)-1-phenyl-4-(4-sulfophenyl)butan-2-yl]amino}propyl]pyrrolidin-1-yl}-5-methyl-1-oxoheptan-4-yl]-N-methyl-L-valinamid in 2.5 mL DMF wurden 10.7 mg (93 µmol) N-Hydroxysuccinimid, 10.6 mg (28 µmol) EDCI sowie 0.12 mg (0.9 µmol) DMAP gegeben. Das Reaktionsgemisch wurde 20h bei RT gerührt und anschließend per präp. HPLC gereinigt.
Es wurden 3.8 mg (72%-ige Reinheit; 25% d.Th.) Titelverbindung erhalten.
LC-MS (Methode 1): Rₜ = 0.90 min; MS (ESIpos): m/z = 1055 (M+H)⁺

### Intermediat 191

### (2R,3R)-N-[(2S)-3-(1H-Indol-3-yl)-1-(1,2-oxazinan-2-yl)-1-oxopropan-2-yl]-3-methoxy-2-methyl-3-[(2S)-pyrrolidin-2-yl]propanamid-Trifluoracetat

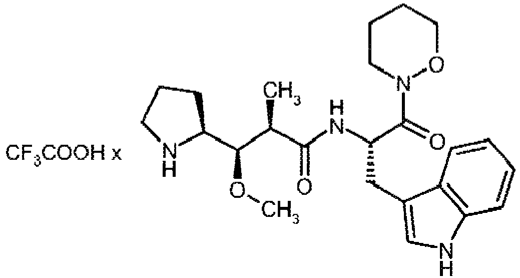

Die Titelverbindung wurde in Analogie zur Synthese von Intermediat 7 über zwei Stufen aus Ausgangsverbindung 1 und (2*S*)-2-Amino-3-(1*H*-indol-3-yl)-1-(1,2-oxazinan-2-yl)propan-1-on Trifluoracetat (Intermediat 99) hergestellt.
Ausbeute über 2 Stufen: 62 mg (67% d. Th.)
HPLC (Methode 6): Rₜ = 1.65 min;
LC-MS (Methode 1): Rₜ = 0.7 min; MS (ESIpos): m/z = 443 (M+H)⁺.

### Intermediat 192

### N-Methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

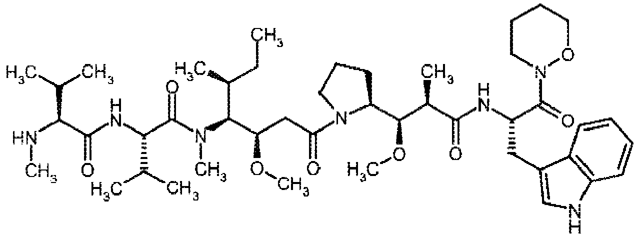

1015 mg (1.59 mmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) wurden in 50 ml DMF aufgenommen, mit 654 mg (2.39 mmol) 2-Brom-1-ethylpyridinium-Tetrafluoroborat (BEP) und 2.8 ml *N,N*-Diisopropylethylamin versetzt und 10 min bei RT gerührt. Dann wurden 1083 mg (1.75 mmol) (2*R*,3*R*)-*N*-[(2*S*)-3-(1*H*-Indol-3-yl)-1-(1,2-oxazinan-2-yl)-1-oxopropan-2-yl]-3-methoxy-2-methyl-3-[(2*S*)-pyrrolidin-2-yl]propanamid-Trifluoracetat (Intermediat 191) zugegeben und die Mischung anschließend 30 min im Ultraschallbad bei RT behandelt. Das Reaktionsgemisch wurde dann im Vakuum eingeengt und der Rückstand in 300 ml Ethylacetat aufgenommen. Die organische Phase wurde nacheinander mit 5%-iger wässriger Zitronensäure-Lösung und 5%-iger wässriger Natriumhydrogenearbonat-Lösung gewaschen, über Magnesiumsulfat getrocknet, filtriert und eingeengt. Das so erhaltene Rohprodukt (1684 mg) wurde ohne weitere Reinigung in 20 ml Acetonitril aufgenommen, mit 2 ml Piperidin versetzt und das Reaktionsgemisch anschließend 10 min bei RT gerührt. Dann wurde im Vakuum eingeengt und der Rückstand mit Diethylether versetzt. Das Lösungsmittel wurde erneut eingedampft und der Rückstand durch Flashchromatographie an Kieselgel (Laufmittel: Dichlormethan/Methano/17%iger wässriger Ammoniak-Lösung 15:1:0.1 -> 15:2:0.2) gereinigt. Die entsprechenden Fraktionen wurden vereinigt, das Lösungsmittel im Vakuum entfernt und der Rückstand aus Acetonitril/Wasser lyophilisiert. So wurden 895 mg (67% über 2 Stufen) der Titelverbindung erhalten.
IIPLC (Methode 12): Rₜ = 1.8 min;
LC-MS (Methode 1): Rₜ = 0.84 min; MS (ESIpos): m/z = 840 (M+H)⁺.

¹H-NMR (500 MHz, DMSO-d₆): δ = 10.8 (d, 1H), 8.3 und 8.05 (2d, 1H), 8.0 (d, 1H), 7.5 (m, 1H), 7.3 (m, 1H), 7.15 und 7.08 (2s, 1H) 7.05-6.9 (m, 2H), 5.12 und 4.95 (2m, 1H), 4.65 (m, 1H), 4.55 (m, 1H), 4.1-3.8 (m, 4H), 3.75 (d, 1H), 3.23, 3.18, 3.17, 3.12, 2.95 und 2.88 (6s, 911), 3.1-3.0 und 2.85 (2m, 2H), 2.65 (d, 1H), 2.4-2.2 (m, 3H), 2.15 (m, 3H), 1.95 (br. m, 2H), 1.85-0.8 (br. m, 11H), 1.08 und 1.04 (2d, 3H), 0.9-0.75 (m, 15H), 0.75-0.65 (dd, 3H) [weitere Signale unter H₂O-Peak verborgen].

### Intermediat 193

### N-(3-Carboxypropyl)-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

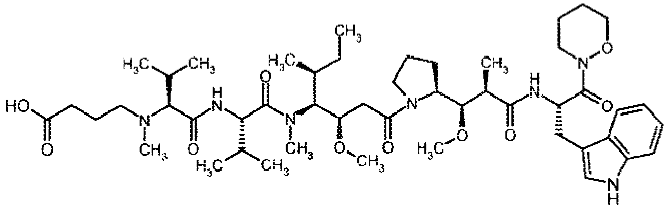

50 mg (0.052 mmol) N-Methyl-L-valyl-N-[(3R,4S,5S)-)-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid (Intermediat 192) und 204µl einer 15%igen wässrigen Lösung von 4-Oxobutansäure wurden in 2 ml Methanol zusammengegeben und mit 23.4 mg (0.252 mmol) Boran-Pyridin-Komplex und 6µl Essigsäure versetzt. Das Reaktionsgemisch wurde über Nacht bei RT gerührt. Anschließend wurde im Vakuum eingeengt und der Rückstand mittels präparativer IIPLC gereinigt. Nach Einengen der entsprechenden Fraktionen wurden 38 mg (78% d.Th.) der Titelverbindung erhalten.
HPLC (Methode 5): Rₜ = 1.7 min;
LC-MS (Methode 9): Rₜ = 4.7 min; MS (ESIpos): m/z = 926 (M+H)⁺.

### Intermediat 194

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

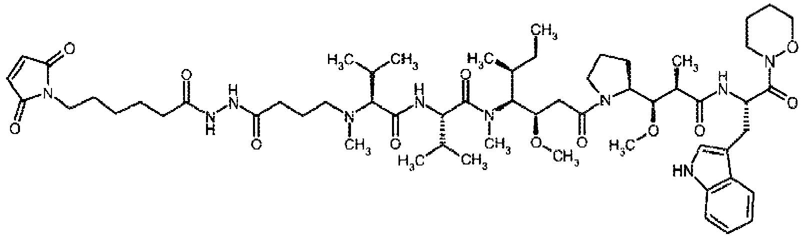

Diese Verbindung wurde in Analogie zur in Intermediat 157 beschriebenen Synthese aus 10 mg (11µmol) N-(3-Carboxypropyl)-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxo-propyl]pyrrolidin-1-yl}-3-methoxy-5-methyl-1-oxoheptan-4-yl]-N-methyl-L-valinamid und kommerziell erhältlichem 6-(2,5-Dioxo-2,5-dihydro-1H-pyrrol-1-yl)hexanhydrazid hergestellt.
Ausbeute: 4.4 mg (35% d.Th.)
HPLC (Methode 5): Rₜ = 1.8 min;
LC-MS (Methode 1): Rₜ = 0.90 min; MS (ESIpos): m/z = 1133 (M+H)⁺.

### Intermediat 195

### N-[6-(2,5-Dioxo-2,5-dihydro-1H-pyrrol-1-yl)hexyl]-N-methyl-L-valyl-N-[(3R,4S,5S)-3-methoxy-1-{(2S)-2-[(1R,2R)-1-methoxy-2-methyl-3-{[(2S,3S)-1-(1,2-oxazinan-2-yl)-1-oxo-3-phenylbutan-2-yl]amino}-3-oxopropyl]pyrrolidin-1-yl}-5-methyl-1-oxoheptan-4-yl]-N-methyl-L-valinamid

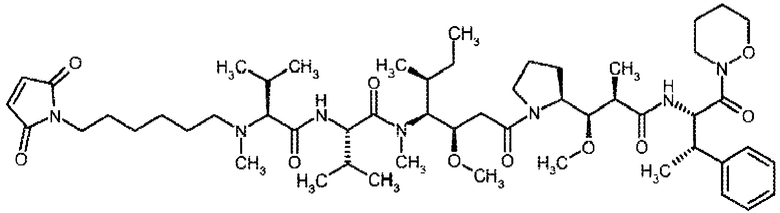

Diese Verbindung wurde in Analogie zu Intermediat 166 ausgehend von 9 mg (0.010 mmol) Intermediat 170 hergestellt.
Ausbeute: 1.1 mg (10% d.Th.)
HPLC (Methode 12): Rₜ = 2.0 min:
LC-MS (Methode 1): Rₜ = 0.99 min; MS (ESIpos): m/z = 994 (M+H)⁺.

### Intermediat 196

### (2S)-2-Amino-1-(2-oxa-3-azabicyclo[2.2.2]oct-5-en-3-yl)-3-phenylpropan-1-on-Trifluoracetat

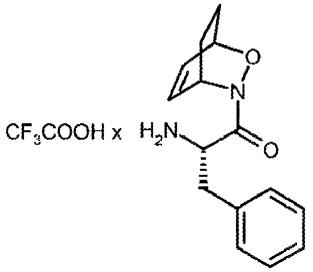

41 mg (0.37 mmol) 2,5-Dioxopyrrolidin-1-yl-*N*-(*tert*.-butoxycarbonyl)-L-phenylalaninat wurden in 10 ml DMF aufgenommen und mit 149 mg (0.41 mmol) 2-Oxa-3-azabicyclo[2.2.2]oct-5-en (Ausgangsverbindung 6) sowie 72 µl (0.41 mmol) *N*,*N*-Diisopropylethylamin versetzt. Die Mischung wurde 1 h bei RT gerührt. Das Lösungsmittel wurde danach im Vakuum entfernt, der Rückstand in Ethylacetat aufgenommen und mit 5%-iger wässriger Zitronensäure-Lösung und anschließend mit 5%-iger wässriger Natriumhydrogencarbonat-Lösung ausgeschüttelt. Die organische Phase wurde eingeengt und der Rückstand durch Flash-Chromatographie an Kieselgel mit Toluol/Ethanol 10:1 als Eluent gereinigt. Die entsprechenden Fraktionen wurden vereinigt und das Lösungsmittel im Vakuum entfernt. Nach Trocknen des Rückstands im Hochvakuum wurden so 69 mg (47% d. Th.) des Boc-geschützten Intermediats *tert*.-Butyl-[(2*S*)-1-(2-oxa-3-azabicyclo[2.2.2]oct-5-en-3-yl)-1-oxo-3-phenylpropan-2-yl]carbamat als Diastereomerengemisch erhalten.
LC-MS (Methode 1): Rₜ = 1.1 min; MS (ESIpos): m/z = 359 (M+H)⁺.

64 mg (0.18 mmol) dieses Intermediats wurden in 10 ml Dichlormethan aufgenommen, mit 1 ml Trifluoressigsäure versetzt und 30 min bei RT gerührt. Anschließend wurde im Vakuum eingeengt und der verbliebene Rückstand aus Wasser/Dioxan lyophilisiert. Auf diese Weise wurden 66 mg (quant.) der Titelverbindung als Schaum erhalten.
HPLC (Methode 6): Rₜ = 1.45 min;
LC-MS (Methode 3): Rₜ = 1.12 min; MS (ESIpos): m/z = 259 (M+H)⁺.

### Intermediat 197

### (2R,3R)-3-Methoxy-2-methyl-N-[(2S)-1-(2-oxa-3-azabicyclo[2.2.2]oct-5-en-3-yl)-1-oxo-3-phenyl-propan-2-yl]-3-[(2S)-pyrrolidin-2-yl]propanamid-Trifluoracetat

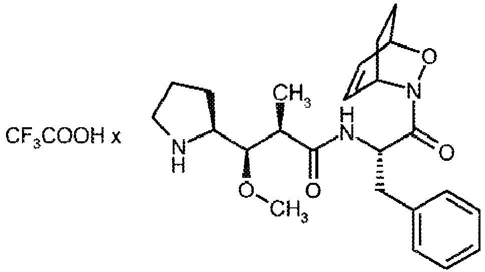

Zunächst wurde (2*R*,3*R*)-3-[(2*S*)-1-(*tert*.-Butoxycarbonyl)pyrrolidin-2-yl]-3-methoxy-2-methylpro-pansäure (Ausgangsverbindung 1) aus 83 mg (0.18 mmol) ihres Dicyclohexylamin-Salzes durch Aufnehmen in Ethylacetat und Ausschütteln mit 5%-iger wässriger Kaliumhydrogensulfat-Lösung freigesetzt. Die organische Phase wurde über Magnesiumsulfat getrocknet, filtriert und eingeengt. Der Rückstand wurde in 10 ml DMF aufgenommen und nacheinander mit 66 mg (0.18 mmol) (2*S*)-2-Amino-1-(2-oxa-3-azabicyclo[2.2.2]oct-5-en-3-yl)-3-phenylpropan-1-on-Trifluoracetat (Intermediat 196), 101 mg (0.266 mmol) *O*-(7-Azabenzotriazol-1-yl)-*N,N,N',N'*-tetramethyluronium-Hexafluorophosphat (HATU) sowie 93 µl (0.53 mmol) *N,N*-Diisopropylethylamin versetzt. Die Mischung wurde 30 min bei RT gerührt. Das Reaktionsgemisch wurde danach eingeengt und der Rückstand durch präparative HPLC gereinigt. Man erhielt so 52 mg (56% d. Th.) des Boc-geschützten Intermediats *tert*.-Butyl-(2*S*)-2-[(1*R*,2*R*)-1-methoxy-2-methyl-3-{[(2*S*)-1-(2-oxa-3-aza-bicyclo[2.2.2]oct-5-en-3-yl)-1-oxo-3-phenylpropan-2-yl]amino}-3-oxopropyl]pyrrolidin-1-carboxylat.
HPLC (Methode 6): Rₜ = 2.13 min;
LC-MS (Methode 1): Rₜ = 1.13 min; MS (ESIpos): m/z = 528 (M+H)⁺.

52 mg (0.1 mmol) dieses Intermediats wurden in 10 ml Dichlormethan aufgenommen, mit 1 ml Trifluoressigsäure versetzt und 20 min bei RT gerührt. Anschließend wurde im Vakuum eingeengt und der verbliebene Rückstand mit 20 ml Diethylether verrührt. Nach 10 min wurde abfiltriert und der Filter-Rückstand im Hochvakuum getrocknet. Auf diese Weise wurden 39 mg (72% d. Th.) der Titelverbindung erhalten.
HPLC (Methode 6): Rₜ = 1.62 min;
LC-MS (Methode 1): Rₜ = 0.68 min; MS (ESIpos): m/z = 428 (M+H)⁺.

### Intermediat 198

### N-Methyl-L-valyl-N-[(3R,4S,5S)-methoxy-1-{(2S)-2-[(1R,2R)-1-methoxy-2-methyl-3-{[(2S)-1-(2-oxa-3-axabicyclo[2.2.2]oct-5-en-3-yl)-1-oxo-3-phenylpropan-2-yl]amino}-3-oxopropyl]pyrrolidin-1-yl}-5-methyl-1-oxoheptan-4-yl]-N-methyl-L-valinamid-Trifluoracetat

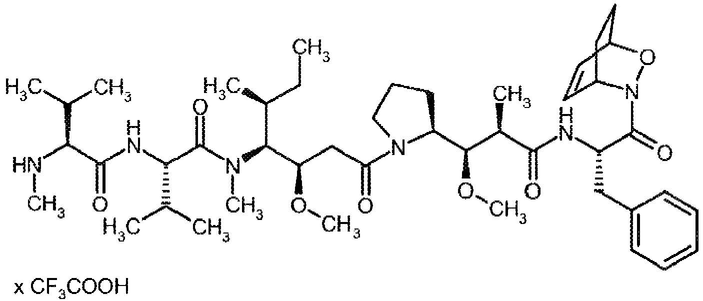

44.5 mg (0.071 mmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) wurden in 10 ml DMF aufgenommen und nacheinander mit 38.6 mg (0.071 mmol) (2*R*,3*R*)-3-Methoxy-2-methyl-*N-*[(2*S*)-1-(2-oxa-3-azabicyclo[2.2.2]oct-5-en-3-yl)-1-oxo-3-phenylpropan-2-yl]-3-[(2*S*)-pyrrolidin-2-yl]propanamid-Trifluoracetat (Intermediat 197), 32.5 mg (0.086 mmol) *O*-(7-Azabenzotriazol-1-yl)-*N,N,N',N'*-tetramethyluronium-Hexafluorophosphat (HATU) sowie 41 µl (0.235 mmol) *N,N-*Diisopropylethylamin versetzt. Die Mischung wurde 1 h bei RT gerührt. Das Reaktionsgemisch wurde dann im Vakuum eingeengt und der Rückstand in Ethylacetat aufgenommen. Die organische Phase wurde nacheinander mit 5%-iger wässriger Zitronensäure-Lösung und 5%-iger wässriger Natriumhydrogencarbonat-Lösung gewaschen, über Magnesiumsulfat getrocknet, filtriert und eingeengt. Es wurden so 73 mg (98% d. Th.) des Fmoc-geschützten Intermediats *N*-[(9*H*-Fluoren-9-ylmethoxy)carbonyl]-*N*-methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(2-oxa-3-azabicyclo[2.2.2]oct-5-en-3-yl)-1-oxo-3-phenylpropan-2-yl]amino}-3-oxopropyl]pyrrolidin-1-yl}-5-methyl-1-oxoheptan-4-yl]-*N*-methyl-L-valinamid erhalten.
HPLC (Methode 6): Rₜ = 2.78 min;
LC-MS (Methode 3): Rₜ = 2.96 min; MS (ESIpos): m/z = 1047 (M+H)⁺.

73 mg (0.071 mmol) dieses Intermediats wurden in 5 ml DMF gelöst. Nach Zugabe von 0.5 ml Piperidin wurde das Reaktionsgemisch 10 min bei RT gerührt. Anschließend wurde im Vakuum eingeengt und der Rückstand mehrfach mit Diethylether digeriert. Nach Abdekantieren des Diethylethers wurde der Rückstand durch präparative HPLC gereinigt (Eluent: Acetonitril / 0.1% aq. TFA). Es wurden 16 mg (26% d. Th.) der Titelverbindung als Schaum erhalten.
HPLC (Methode 6): Rₜ = 1.94 min;
LC-MS (Methode 3): Rₜ = 1.71 min; MS (ESIpos): m/z = 825 (M+H)⁺

¹H-NMR (400 MHz, DMSO-d₆): δ = 8.9-8.6 (m, 3H), 8.4, 8.3, 8.1 und 8.0 (4d, 1H), 7.3-7.1 (m, 5H), 6.7-6.5 (m, 2H), 5.2-4.8 (m, 3H), 4.75-4.55 (m, 3H), 4.05-3.95 (m, 1H), 3.7-3.4 (m, 4H), 3.22, 3.17, 3.15, 3.05, 3.02 und 2.95 (6s, 9H), 3.0 und 2.7 (2 br. m, 2H), 2.46 (m, 3H), 2.4-1.2 (br. m, 13H), 1.1 -0.85 (m, 18H), 0.75 (m. 3H) [weitere Signale unter H₂O-Peak verborgen].

### Intermediat 199

### N-(4-{2-[6-(2,5-Dioxo-2,5-dihydro-1H-pyrrol-1-yl)hexanoyl]hydrazino}-4-oxobutyl)-N-methyl-L-valyl-N-[(3R,4S,5S)-3-methoxy-1-{(2S)-2-[(1R,2R)-1-methoxy-2-methyl-3-{[(2S)-1-(2-oxa-3-azabicyclo[2.2.2]oct-5-en-3-yl)-1-oxo-3-phenylpropan-2-yl]amino}-3-oxopropyl]pyrrolidin-1-yl}-5-methyl-1-oxoheptan-4-yl]-N-methyl-L-valinamid

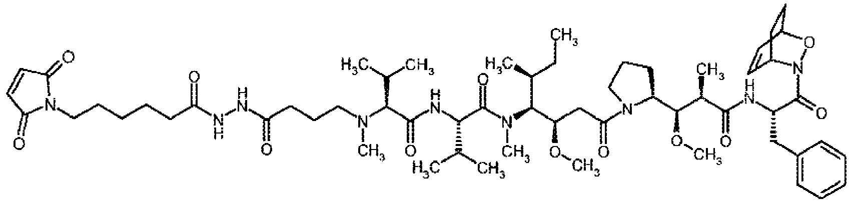

Die Titelverbindung wurde in Analogie zu den Intermediaten 193 und 194 ausgehend von 23 mg (24µmol) N-Methyl-L-valyl-N-[(3R,4S,5S)-3-methoxy-1-{(2S)-2-[(1R,2R)-1-methoxy-2-methyl-3-{[(2S)-1-(2-oxa-3-azabicyclo[2.2.2]oct-5-en-3-yl)-1-oxo-3-phenylpropan-2-yl]amino}-3-oxopropyl]pyrrolidin-1-yl}-5-methyl-1-oxoheptan-4-yl]-N-methyl-L-valinamid-Trifluoracetat (Intermediat 198) hergestellt.
HPLC (Methode 12): Rt = 1.9 min;
LC-MS (Methode 2): Rt = 2.1 min; MS (ESIpos): m/z = 1118 (M+H)+.

### Intermediat 200

### N-[2-(2-{2-[2-(2,5-Dioxo-2,5-dihydro-1H-pyrrol-1-yl)ethoxy]ethoxy}ethoxy)ethyl]-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]ammo}-1-methoxy-2-methyl-3-oxopropyl]pyrrolidin-1-yl}-3-methoxy-5-methyl-1-oxoheptan-4-yl]-N-methyl-L-valinamid

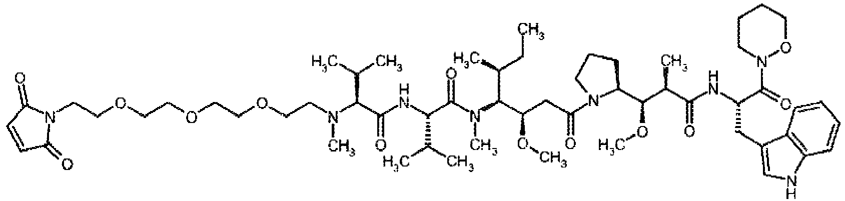

Die Herstellung erfolgte in Analogie zu den Intermediaten 174 und 175 beginnend mit der reduktive Alkylierung von Intermediat 192 mit Intermediat 172, anschließender Entschützung und Aufbau des Maleinimids.
HPLC (Methode 12): Rt = 1.9 min;
LC-MS (Methode 1): Rt = 0.86 min; MS (ESIpos): m/z = 1025 (M+H)+.

### Intermediat 201

### N-{6-[(Bromacetyl)amino]hexyl}-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

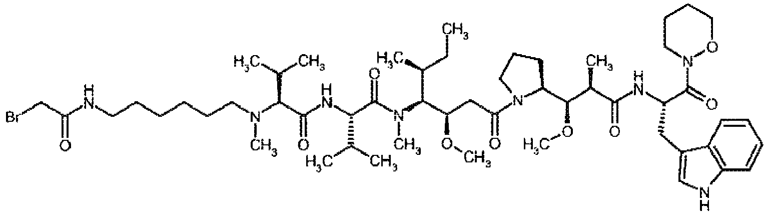

22 mg (0.023 mmol) N-(6-Aminohexyl)-N-methyl-L-valyl-N-(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid (Intermediat 101) wurden in 9.5 ml THF gelöst und bei 0°C wird mit 4.2 µl Triethylamin versetzt. Eine Lösung aus Bromacetylchlorid in THF wurde zutropft und das Reaktionsgemisch 30 min bei 0°C gerührt. Das Reaktionsgemisch wurde eingeengt und der Rückstand durch präparative HPLC gereinigt. So wurden 6.9 mg (26% d.Th.) der Titelverbindung als Schaum erhalten.
HPLC (Methode 5): Rₜ = 1.8 min;
LC-MS (Methode 11): Rₜ = 0.9 min; MS (ESIpos): m/z = 1059 und 1061 (M+H)⁺.

### Intermediat 202

### N-{2-[2-(2-{3-[(2,5-Dioxopyrrolidin-1-yl)oxy]-3-oxopropoxy}ethoxy)ethoxy]ethyl}-N-methyl-L-valyl-N-[(3R,4S,5S)-1-{(2S)-1-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

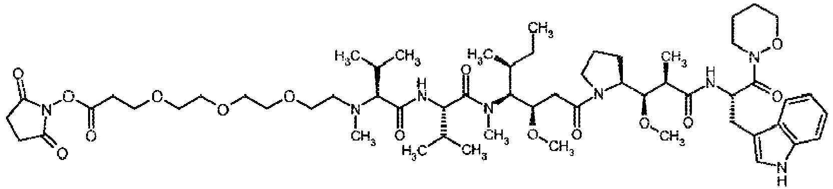

Die Herstellung erfolgte zunächst in Analogie zu Intermediat 168 beginnend mit der reduktiven Alkylierung von Intermediat 192 mit Intermediat 167 und anschließender hydrogenolytischer Spaltung des Benzylesters zu N-(2-{2-[2-(2-Carboxyethoxy)ethoxy]ethoxy}ethyl)-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid.

13 mg (10 µmol) dieses Intermediats wurden in 5 ml DMF gelöst und mit 2.1 mg (20 mmol) 1-Hydroxypyrrolidin-2,5-dion, 6.5 µl *N,N*-Diisopropylethylamin und 7.1 mg (0.02 mmol) HATU versetzt. Das Reaktionsgemisch wurde über Nacht bei RT gerührt und anschließend im Vakuum eingeengt. Der verbliebene Rückstand wurde mittels präparativer IIPLC gereinigt. Nach Lyophilisation aus Acetonitril/Wasser wurden 9.2 mg (62% d.Th.) der Titelverbindung erhalten.
HPLC (Methode 12): Rₜ = 2.0 min;
LC-MS (Methode 2): Rₜ = 2.1 min; MS (ESIpos): m/z = 1141 (M+H)⁺.

### Intermediat 203

### tert.-Butyl-(6-hydrazino-6-oxobexyl)carbamat

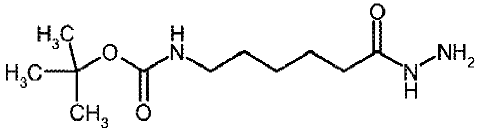

Diese Verbindung wurde nach Standardmethoden der Peptidchemie hergestellt durch Kupplung von 6-[(*tert*.-Butoxycarbonyl)amino]hexansäure mit Benzylhydrazincarboxylat in Gegenwart von EDCI und HOBT und anschlicßender hydrogenolytischer Spaltung der Benzyloxycarbonylschutzgruppe.
LC-MS (Methode 11): Rₜ = 0.59 min; MS (ESIpos): m/z = 246 (M+H)⁺.

### Intermediat 204

### N-{4-[2-(6-Aminohexanoyl)hydrazino]-4-oxobutyl}-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid-Trifluoracetat

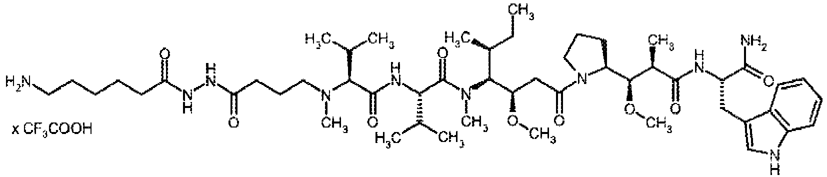

146 mg (50 µmol) (N-(3-Carboxypropyl)-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxo-propyl]pyrrolidin-1-yl}-3-methoxy-5-methyl-1-oxoheptan-4-yl]-N-methyl-L-valinamid wurden in 5 ml DMF gelöst und anschließend mit 30.6 mg (80 µmol) O-(7-Azabenzotriazol-1-yl)-N,N,N',N'-tetramethyluronium-Hexafluorophosphat, 19 µL N,N-Diisopropylethylamin sowie mit 22.4 mg (60 µmol) von *tert*.-Butyl-(6-hydrazino-6-oxohexyl)carbamal versetzt. Das Reaktionsgemisch wurde 1.5 h bei RT gerührt. Anschließend wurde im Hochvakuum eingeengt und der verbliebene Rückstand mittels präparativer IIPLC aufgereinigt. So wurden 43 mg (68 % d.Th.) des geschützten Intermediats erhalten, welche dann in 10 ml Dichlormethan aufgenommen wurden und mit 1 ml Trifluoressigsäure entschützt wurden. Das Reaktionsgemisch wurde eingeengt und der Rückstand mit Dichlormethan verrührt und das Lösungsmittel erneut im Vakuum entfernt. So wurden 45 mg (68% d.Th. über 2 Stufen) der Titelverbindung erhalten.
HPLC (Methode 12): Rₜ = 1.6 min;
LC-MS (Methode 11): Rₜ = 0.66 min; MS (ESIpos): m/z = 983 (M+H)⁺.

### Intermediat 205

### N-(4-{2-[6-({[2-(2,5-Dioxo-2,5-dihydro-1H-pyrrol-1-yl)ethyl]carbamoyl}amino)hexanoyl] hydrazino}-4-oxobutyl)-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

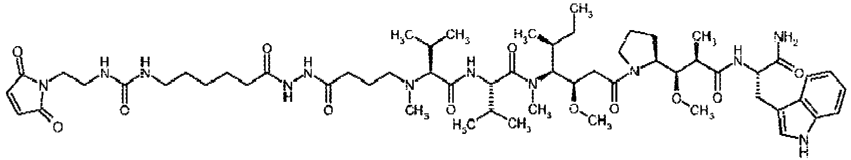

Diese Verbindung wurde in Analogie zu Intermediat 114 ausgehend von den Intermediaten 50 und 204 hergestellt.
Ausbeute: 4 mg (78% d.Th.)
HPLC (Methode 12): Rₜ = 1.7 min;
LC-MS (Methode 11): Rₜ = 0.73 min; MS (ESIpos): m/z = 1149 (M+H)⁺.

### Intermediat 206

### N-(6-{[3-({3-[(2,5-Dioxopyrrolidin-1-yl)oxy]-3-oxopropyl}disulfanyl)propanoyl]amino}hexyl)-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

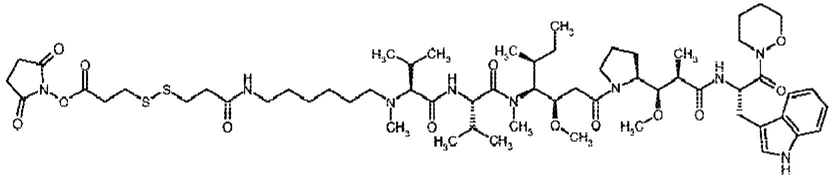

8 mg (10 µmol) von Intermediat 101 wurden in 2 ml DMF gelöst und mit 8.6 mg (20 µmol) 1,1'-{Disulfandiylbis[(1-oxopropan-3,1-diyl)oxy]}dipyrrolidin-2,5-dion und 3.7 µl *N,N*-Diisopropylethylamin versetzt. Das Reaktionsgemisch wurde 2 h bei RT gerührt und anschließend das Lösungsmittel im Vakuum abgedampft und der Rückstand durch präparative HPLC gereinigt. Es wurden 7.2 mg (68% d. Th.) der Titelverbindung erhalten.
HPLC (Methode 5): Rₜ = 1.9 min;
LC-MS (Methode 11): Rₜ = 0.94 min; MS (ESIpos): m/z = 615 [½(M+2H⁺]

### Intermediat 207

### (1S,2R)-1-Amino-2-phenylcyclopropancarbonsäure-Trifluoracetat

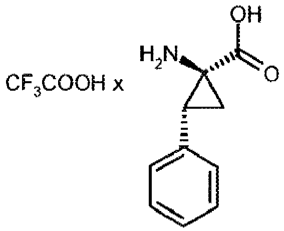

Die Titelverbindung wurde durch Entschützung von 210 mg (0.76 mmol) kommerziell erhältlicher (1*S*,2*R*)-1-[(*tert*.-Butoxycarbonyl)amino]-2-phenylcyclopropancarbonsäure mit Trifluoressigsäure in quantitativer Ausbeute erhalten.
LC-MS (Methode 1): Rₜ = 0.23 min; MS (ESIpos): m/z = 178 (M+H)⁺.

### Intermediat 208

### 9H-Fluoren-9-ylmethyl-(6-oxohexyl)carbamat

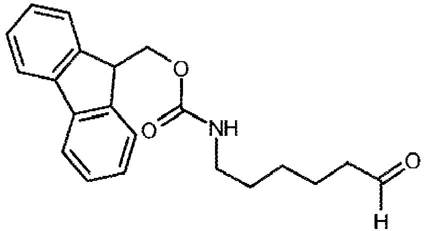

Die Titelverbindung wurde aus 1 g (2.95 mmol) von kommerziell erhältlichem 9H-Fluoren-9-ylmethyl-(6-hydroxyhexyl)carbamat nach Standardbedingungen durch Oxidation mit Schwefeltrioxid-Pyridin-Komplex hergestellt. Es wurden 840 mg (85% d.Th.) der Titelverbindung erhalten.
IIPLC (Methode 12): Rₜ = 2.0 min;
LC-MS (Methode 1): Rₜ = 1.1 min; MS (ESIpos): m/z = 338 (M+H)⁺.

### Intermediat 209

### N-[6-(2,5-Dioxo-2,5-dihydro-1H-pyrrol-1-yl)hexyl]-N-methyl-L-valyl-N-[(3R,4S,5S)-1-{(2S)-2-[(1R,2R)-3-{[(1S,2R)-1-carboxy-2-phenylcyclopropyl]amino}-1-methoxy-2-methyl-3-oxopropyl]pyrrolidin-1-yl}-3-methoxy-5-methyl-1-oxoheptan-4-yl]-N-methyl-L-valinamid

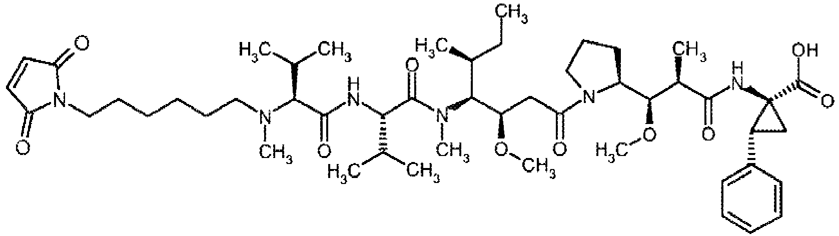

Zunächst wurde in Analogie zu der in Intermediat 75 beschriebenen Synthese durch Kupplung von N-(*tert*.-Butoxycarbonyl)-N-methyl-L-valyl-N-[(3R,4S,5S)-1-{(2S)-2-[(1R,2R)-2-carboxy-1-methoxypropyl]pyrrolidin-1-yl}-3-methoxy-5-methyl-1-oxoheptan-4-yl]-N-methyl-L-valinamid (Intermediat 26) und (1S,2R)-1-Amino-2-phenylcyclopropancarbonsäure-Trifluoracelat (Intermediat 207) in Gegenwart von O-(7-Azabenzotriazol-1-yl)-N,N,N',N'-tetramethyluronium-Hexafluorophosphat und anschließende Abspaltung der Boc-Schutzgruppe mittels Trifluoressigsäure die Amin-Verbindung N-Methyl-L-valyl-N-[(3R,4S,5S)-1-{(2S)-2-[(1R,2R)-3-{[(1S,2R)-1-carboxy-2-phenylcyclopropyl]amino}-1-methoxy-2-methyl-3-oxopropyl]pyrrolidin-1-yl}-3-methoxy-5-methyl-1-oxoheptan-4-yl]-N-methyl-L-valinamid als Trifluoracetat hergestellt.

Zu 22 mg (0.026 mmol) dieser Verbindung in 10 ml Methanol wurden dann 17 mg (0.05 mmol) 9H-Fluoren-9-ylmethyl-(6-oxohexyl)carbamat (Intermediat 208) und 2.3 mg Essigsäure sowie 11.4 mg (0.12 mmol) Boran-Pyridin-Komplex zugegeben. Das Reaktionsgemisch wurde über Nacht bei RT gerührt. Dann wurden nochmals die gleichen Mengen Boran-Pyridin-Kompiex und Essigsäure sowie 8 mg Fluoren-9-ylmethyl-(6-oxohexyl)carbamat zugegeben und das Reaktionsgemisch weitere 24h bei RT gerührt. Anschließend wurde im Vakuum eingeengt und der Rückstand mittels präparativer HPLC gereinigt. Nach Einengen der entsprechenden Fraktionen wurde das Produkt sofort in die nächste Stufe eingesetzt.

33 mg des noch verunreinigten Intermediats wurden in 5 ml DMF aufgenommen und mit 1 ml Piperidin versetzt. Nach 15 min Rühren bei RT wurde das Reaktionsgemisch eingeengt und der erhaltene Rückstand durch präparative HPLC gereinigt. So wurden 11 mg (55% d.Th. über 2 Stufen) des Aminocarbonsäure-Intermediats erhalten.
HPLC (Methode 12): Rₜ = 1.7 min;
LC-MS (Methode 11): Rₜ = 0.7 min; MS (ESIpos): m/z = 843 (M+H)⁺.

6 mg (7.12 µmol) dieses Intermediats wurden in 1 ml Dioxan aufgenommen und anschließend mit 6.6 mg (42.7 µmol) Methyl-2,5-dioxo-2,5-dihydro-1H-pyrrol-1-carboxylat sowie mit 5µl gesättigter wässriger Natriumhydrogencarbonat-Lösung versetzt. Das Reaktionsgemisch wurde 1h bei RT gerührt. Dann wurden nochmals 3 Portionen von jeweils 50 µl der gesättigten wässrigen Natriumhydrogencarbonatlösung zugegeben und das Reaktionsgemisch weitere 30 min bei RT gerührt. Dann wurde das Reaktionsgemisch mit Trifluoressigsäure auf pH 2 angesäuert und anschließend im Vakuum eingeengt. Der verbliebene Rückstand wurde mittels präparativer HPLC gereinigt. Nach Lyophilisation aus Acetonitril/Wasser wurden 4 mg (60% d.Th.) der Titelverbindung als Schaum erhalten.
HPLC (Methode 12): Rₜ = 1.9 min;
LC-MS (Methode 11): Rₜ = 0.88 min; MS (ESIpos): m/z = 923 (M+H)⁺.

### Intermediat 210

### N-{6-[(2,5-Dioxopyrrolidin-1-yl)oxy]-6-oxohexyl}-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

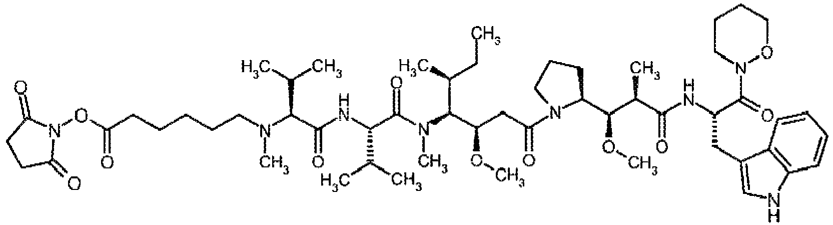

Zunächst wurde 6-Oxohexansäure nach Literaturvorschrift hergestellt (J.Org.Chem. 58, 1993, 2196-2200).

80 mg (0.08 mmol) N-Methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid (Intermediat 192) und 65.4 mg (0.5 mmol) 6-Oxohexansäure wurden in 9 ml Methanol zusammengegeben und mit 10 µl Essigsäure und 37.4 mg (0.4 mmol) Boran-Pyridin-Komplex versetzt. Das Reaktionsgemisch wurde über Nacht bei RT gerührt. Anschließend wurde im Vakuum eingeengt und der Rückstand in Acetonitril/Wasser 1:1 aufgenommen und mit Trifluoressigsäure auf pH 2 gebracht. Das Reaktionsgemisch wurde erneut eingeengt und der Rückstand mittels präparativer HPLC gereinigt. Nach Einengen der entsprechenden Fraktionen wurden 70 mg (86% d. Th.) N-(5-Carboxypentyl)-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid als Trifluoracetat erhalten.
HPLC (Methode 12): Rₜ = 1.9 min;
LC-MS (Methode 1): Rₜ = 0.87 min; MS (ESIpos): m/z = 955 (M+H)⁺.

¹H-NMR (500 MHz, DMSO-d₆, charakteristische Signale): δ = 12.0 (br. M, 1H), 10.8 (s, 1H), 9.4 (m, 1H), 8.9 und 8.8 (2d, 1H), 8.3 und 8.02 (2d, 1H), 7.5 (m, 1H), 7.3 (m, 1H), 7.15 und 7.1 (2s, 1H) 7.05-6.9 (m, 2H), 5.12 und 4.95 (2m, 1H), 4.7-4.5 (m, 2H), 4.1-3.8 (m, 4H), 3.75 (d, 1H), 3.25, 3.2, 3.18, 3.13, 2.98 und 2.88 (6s, 9H), 2.8 (m, 3H), 1.08 und 1.04 (2d, 3H), 0.95-0.8 (m, 15H), 0.8-0.65 (dd, 3H).

22 mg (23 µmol) dieses Intermediats wurden in 1.8 ml Dichlormethan gelöst und mit 13.2 mg (70 µmol) 1-(3-Dimethylaminopropyl)-3-ethylcarbodiimid-Hydrochlorid, 26.5 mg (230 µmol) 1-Hydroxypyrrolidin-2,5-dion sowie 0.28 mg (2 µmol) Dimethylaminopyridin versetzt und das Reaktionsgemisch 2h bei RT gerührt. Anschließend wurde das Reaktionsgemisch im Vakuum eingeengt und der verbliebene Rückstand mittels präparativer HPLC gereinigt. Nach Lyophilisation aus Acetonitril/Wasser wurden 21.3 mg (88% d.Th.) der Titelverbindung erhalten.
HPLC (Methode 12): Rₜ = 1.9 min;
LC-MS (Methode 1): Rₜ = 0.94 min; MS (ESIpos): m/z = 1052 (M+H)⁺.

### Intermediat 211

### N-{6-[(2,5-Dioxopyrrolidin-1-yl)oxy]-6-oxohexyl}-N-methyl-L-valyl-N-[(3R,4S,5S)-3-methoxy-1-{(2S)-2-[(1R,2R)-1-methoxy-2-methyl-3-{[(2S,3S)-1-(1,2-oxazinan-2-yl)-1-oxo-3-phenylbutan-2-yl]amino}-3-oxopropyl]pyrrolidin-1-yl}-5-methyl-1-oxoheptan-4-yl]-N-methyl-L-valinamid

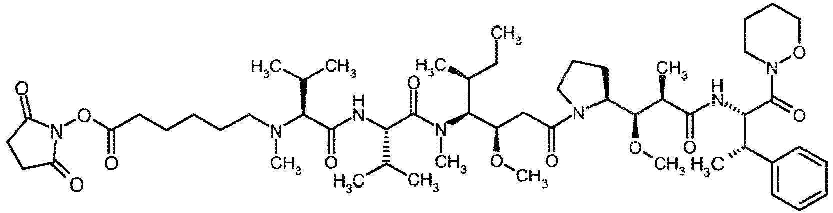

15 mg (20 µmol) *N*-Methyl-L-valyl-*N*-[(3*R*,4*S*,5*S*)-3-methoxy-1-{(2*S*)-2-[(1*R*,2*R*)-1-methoxy-2-methyl-3-{[(2*S*,3*S*)-1-(1,2-oxazinan-2-yl)-1-oxo-3-phenylbutan-2-yl]amino}-3-oxopropyl] pyrrolidin-yl}-5-methyl-1-oxoheptan-4-yl]-*N*-methyl-L-valinamid-Trifluoracetat (Intermediat 15) wurden in Analogie zu Intermediat 210 mit 6-Oxohexansäure reduktiv alkyliert.
Ausb.: 9.2 mg (61% d.Th.)
HPLC (Methode 12): Rₜ = 1.9 min;
LC-MS (Methode 1): Rₜ = 0.87 min; MS (ESIpos): m/z = 929 (M+H)⁺.

9 mg (10 µmol) dieses Intermediats wurden in 3 ml DMF gelöst und mit 5.6 mg (48 µmol) 1-Hydroxypyrrolidin-2,5-dion, 5 µl *N,N*-Diisopropylethylamin und 5.5 mg (0.015 mmol) HATU versetzt und das Reaktionsgemisch 6 h im Ultraschallbad behandelt. Dabei wurden stündlich 5.5 mg HATU nachgesetzt. Anschließend wurde das Reaktionsgemisch im Vakuum eingeengt, der Rückstand in Acetonitril/Wasser aufgenommen und mit Trifluoressigsäure auf pH 2 gebracht. Nach erneutem Einengen im Vakuum wurde der verbliebene Rückstand mittels präparativer HPLC gereinigt. Nach Lyophilisation aus Acetonitril/Wasser wurden 5.8 mg (57 % d. Th.) der Titelverbindung erhalten.
HPLC (Methode 12): Rₜ = 2.0 min;
LC-MS (Methode 1): Rₜ = 0.95 min; MS (ESIpos): m/z = 1027 (M+H)⁺.

### Intermediat 212

### N-{2-[2-(2-{3-[(2,5-Dioxopyrrolidin-1-yl)oxy]-3-oxopropoxy}ethoxy)ethoxy]ethyl}-N-methyl-L-valyl-N-[(3R,4S,5S)-3-methoxy-1-{(2S)-2-[(1R,2R)-1-methoxy-2-methyl-3-{[(2S,3S)-1-(1,2-oxazinan-2-yl)-1-oxo-3-phenylbutan-2-yl]amino}-3-oxopropyl]pyrrolidin-1-yl}-5-methyl-1-oxoheptan-4-yl]-N-methyl-L-valinamid

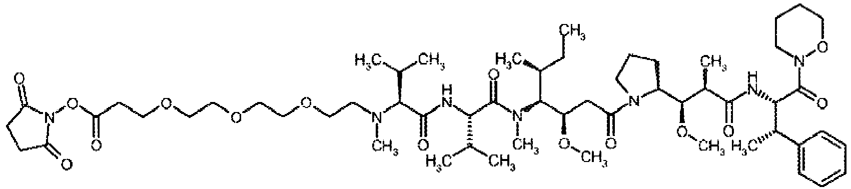

Die Herstellung erfolgte zunächst in Analogie zu Intermediat 168 beginnend mit der reduktiven Alkylierung von Intermediat 15 mit Intermediat 167 und anschließender hydrogenolytischer Spaltung des Benzylesters zu N-(2-{2-[2-(2-Carboxyethoxy)ethoxy]ethoxy}ethyl)-N-methyl-L-valyl-N-[(3R,4S,5S)-3-methoxy-1-{(2S)-2-[(1R,2R)-1-methoxy-2-methyl-3-{[(2S,3S)-1-(1,2-oxazinan-2-yl)-1-oxo-3-phenylbutan-2-yl]amino}-3-oxopropyl]pyrrolidin-1-yl}-5-methyl-1-oxoheptan-4-yl]-N-methyl-L-valinamid.

8.4 mg (8 µmol) dieses Intermediats wurden in 3 ml DMF gelöst und mit 9.5 mg (80 µmol) 1-Hydroxypyrrolidin-2,5-dion, 10 µl *N,N*-Diisopropylethylamin und 9.4 mg (25 µmol) HATU versetzt und das Reaktionsgemisch über Nacht bei RT gerührt und anschließend im Vakuum eingeengt. Anschließend wurde das Reaktionsgemisch im Vakuum eingeengt, der Rückstand in Acetonitril/Wasser aufgenommen und mit Trifluoressigsäure auf pH 2 gebracht. Nach erneutem Einengen im Vakuum wurde der verbliebene Rückstand mittels präparativer HPLC gereinigt. Nach Lyophilisation aus Acetonitril/Wasser wurden 4 mg (32% d.Th.) der Titelverbindung erhalten.
HPLC (Methode 12): Rₜ = 2.0 min;
LC-MS (Methode 1): Rₜ = 0.96 min; MS (ESIpos): m/z = 1117 (M+H)⁺.

### Intermediat 213

### N-{6-[(trans-4-{[(2,5-Dioxopyrrolidin-1-yl)oxy]carbonyl}cyclohexyl)amino]-6-oxohexyl}-N-methyl-L-valyl-N-[(3R4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

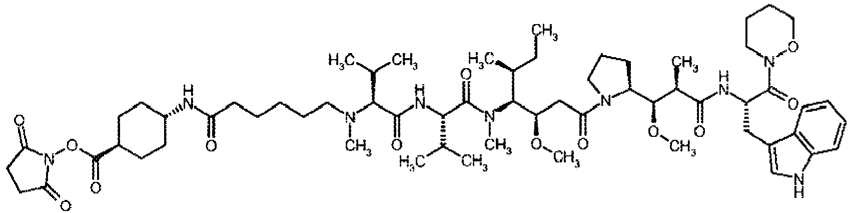

Diese Verbindung wurde in Analogie zu Intermediat 104 ausgehend von N-(5-Carboxypentyl)-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid, dessen Synthese unter Intermediat 210 beschrieben wurde, hergestellt. Es wurden 9.3 mg der Titelverbindung (37% d.Th. über 3 Stufen) erhalten.
HPLC (Methode 12): Rₜ = 1.9 min;
LC-MS (Methode 1): Rₜ = 0.9 min; MS (ESIpos): m/z = 1177 (M+H)⁺.

### Intermediat 214

### N-{4-[(2,5-Dioxopyrrolidin-1-yl)oxy]-4-oxobutyl}-N-methyl-L-valyl-N-[(3R,4S,5S)-1-{(2S)-2-[(1R,2R)-3-{[(1S,2R)-1-hydroxy-1-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

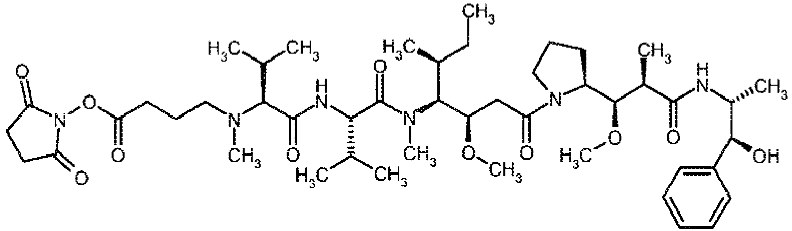

Diese Verbindung wurde in Analogie zu Intermediat 210 durch Überführung von Intermediat 92 in den Aktivester hergestellt.
HPLC (Methode 5): Rₜ = 1.6 min;
LC-MS (Methode 11): Rₜ = 0.82 min; MS (ESIpos): m/z = 901 (M+H)⁺.

### Intermediat 215

### N-{6-[(2,5-Dioxopyrrolidin-1-yl)oxy]-6-oxohexyl}-N-methyl-L-valyl-N-[(3R,4S,5S)-1-{(2S)-2-[(1R,2R)-3-{[(1S,2R)-1-hydroxy-1-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

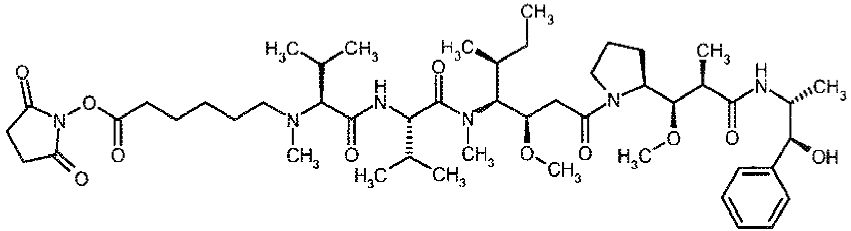

Zunächst wurde aus Intermediat 40 in Analogie zu Intermediat 183 mit Boran-Pyridin-Komplex N-(5-Carboxypentyl)-N-methyl-L-valyl-N-[(3R,4S,5S)-1-{(2S)-2-[(1R,2R)-3-{[(1S,2R)-1-hydroxy-1-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid hergestellt. Aus dieser Verbindung wurde dann in Analogie zu Intermediat 210 der Aktivester generiert. Es wurden 34 mg (36% d.Th. über 2 Stufen) der Titelverbindung erhalten.
HPLC (Methode 5): Rₜ = 1.6 min;
LC-MS (Methode 1): Rₜ = 0.85 min; MS (ESIpos): m/z = 930 (M+H)⁺.

### Intermediat 216

### N-(4-{[(2,5-Dioxopyrrolidin-1-yl)oxy]carbonyl}benzyl)-N-methyl-L-valyl-N-[(3R,4S,5S)-1-{(2S)-2-[(1R,2R)-3-{[(2S)-3-(1H-indol-3-yl)-1-(1,2-oxaxinan-2-yl)-1-oxopropan-2-y]amino}-1-methoxy-2-methyl-3-oxopropyl]pyrrolidin-1-yl}-3-methoxy-5-methyl-1-oxoheptan-4-yl]-N-methyl-L-valinamid

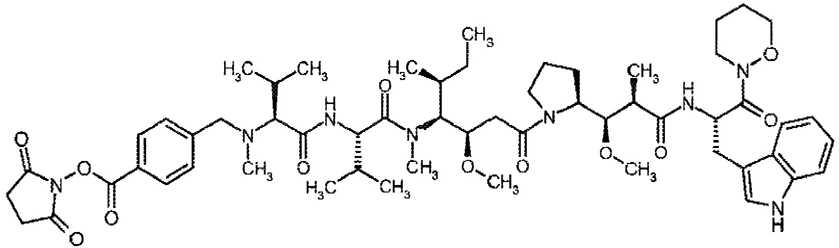

Zunächst wurde in Analogie zur Herstellung von Intermediat 183 Intermediat 192 mit 4-formylbenzoesäure mit Boran-Pyridin-Komplex zu N-(4-Carboxybenzyl)-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid umgesetzt. Aus dieser Verbindung wurden dann in Analogie zu Intermediat 210 11 mg (68% d. Th.) der Titelverbindung generiert.
HPLC (Methode 5): Rₜ = 1.8 min;
LC-MS (Methode 1): Rₜ = 1.13 min; MS (ESIpos): m/z = 1072 (M+H)⁺.

### Intermediat 217

### N-(5-Carboxypentyl)-N-methyl-L-valyl-N-[(3R,4S,5S)-1-{(2S)-2-[(1R,2R)-3-{[(2S)-1-(benzyloxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

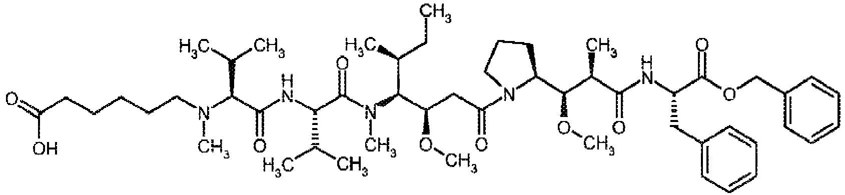

53 mg (84 µmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) und 45 mg (84 µmol) Benzyl-*N*-{(2*R*,3*R*)-3-methoxy-2-methyl-3-[(2*S*)-pyrrolidin-2-yl]propanoyl}-L-phenylalaninat-Trifluoracetat (Intermediat 12) wurden in 2 ml DMF aufgenommen, mit 19 µl *N,N*-Diisopropylethylamin, 14 mg (92 µmol) HOBt sowie 17.6 mg (92 µmol) EDC versetzt und dann über Nacht bei RT gerührt. Anschließend wurde das Reaktionsgemisch eingeengt und der Rückstand mittels präparativer HPLC gereinigt. Es wurden so 59 mg (68% d. Th.) des Fmoc-geschützten Intermediats *N*-[(9*H*-Fluoren-9-ylmethoxy)carbonyl]-*N*-methyl-L-valyl-*N*-[(3*R*,4*S*,5*S*)-1-{(2*S*)-2-(1*R*,2*R*)-3-{[(2*S*)-1-(benzyloxy)-1-oxo-3-phenylpropan-2-yl]amino}-1-methoxy-2-methyl-3-oxopropyl]-pyrrolidin-1-yl}-3-methoxy-5-methyl-1-oxoheptan-4-yl]-*N*-methyl-L-valinamid erhalten.
LC-MS (Methode 1): Rₜ = 1.55 min; m/z = 1044 (M+H)⁺.

57 mg (0.055 mmol) dieses Intermediats wurden zur Abspaltung der Fmoc-Schulzgruppe mit 1.2 ml Piperidin in 5 ml DMF behandelt. Nach Einengen und Reinigung mittels präparativer HPLC wurden 39 mg (76% d. Th.) des freien Amin-Intermediats *N*-Methyl-L-valyl-*N*-[(3*R*,4*S*,5*S*)-1-{(2*S*)-2-[(1*R*,2*R*)-3-{[(2*S*)-1-(benzyloxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid als Trifluoracetat erhalten.
IIPLC (Methode 5): Rₜ = 1.9 min;
LC-MS (Methode 1): Rₜ = 1.01 min; m/z = 822 (M+H)⁺.

60 mg (0.06 mmol) dieses Intermediats wurden in Analogie zu Intermediat 210 mit 6-Oxohexansäure in Gegenwart von Boran-Pyridin-Komplex umgesetzt. Es wurden 45 mg (75% d. Th.) der Titelverbindung als Schaums erhalten.
HPLC (Methode 5): Rₜ = 1.9 min;
LC-MS (Methode 1): Rₜ = 0.97 min; MS (ESIpos): m/z = 9936 (M+H)⁺.

### Intermediat 218

### N-{6-[(2,5-Dioxopyrrolidin-1-yl)oxy]-6-oxohexyl}-N-methyl-L-valyl-N-[(3R,4S,5S)-1-{(2S)-2-[(1R,2R)-3-{[(2S)-1-(benzyloxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

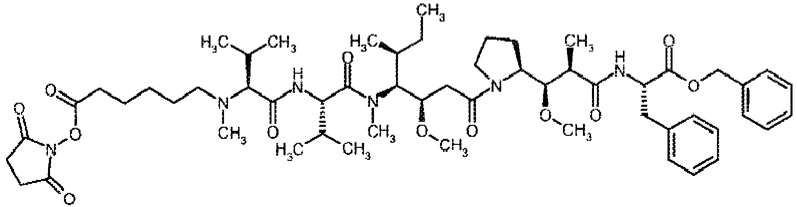

Diese Verbindung wurde durch Überführung von 42 mg (0.05 mmol) von Intermediat 217 in den Aktivester hergestellt.
Ausbeute: 26 mg (54%)
HPLC (Methode 5): Rₜ = 2.1 min;
LC-MS (Methode 1): Rₜ = 1.01 min; MS (ESIpos): m/z = 1034 (M+H)⁺.

### Intermediat 219

### N-{6-[(2,5-Dioxopyrrolidin-1-yl)oxy]-6-oxohexyl}-N-methyl-L-valyl-N-[(3R,4S,5S)-1-{(2S)-2-[(1R,2R)-3-{[(1S)-1-carboxy-2-phenylethyl]amino}-1-methoxy-2-methyl-3-oxopropyl]pyrrolidin-1-yl}-3-methoxy-5-methyl-1-oxoheptan-4-yl]-N-methyl-L-valinamid

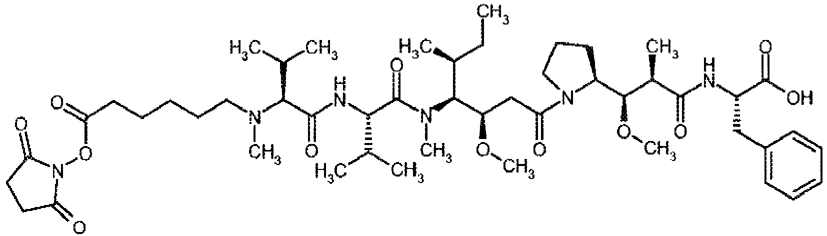

20 mg (0.02 mol) der Verbindung aus Intermediat 218 wurden in 2.4 ml Methanol aufgenommen und über 5%-igem Palladium auf Aktivkohle 30 min lang bei RT unter Wasserstoff-Normaldruck hydriert. Der Katalysator wurde danach abfiltriert und das Lösungsmittel im Vakuum entfernt. Der Rückstand wurde aus Acetonitri/Wasser 1:1 lyophilisiert. Man erhielt 14 mg (92% d.Th.) der Titelverbindung als farblosen Schaum.
HPLC (Methode 5): Rₜ = 1.7 min;
LC-MS (Methode 1): Rₜ = 0.86 min; MS (ESIpos): m/z = 944 (M+H)⁺.

### Intermediat 220

### N-[(3R,4S,5S)-1-{(2S)-2-[(1R,2R)-3-{[(2S)-3-(1H-Indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-oxoheptan-4-yl]-N-methyl-L-valinamid

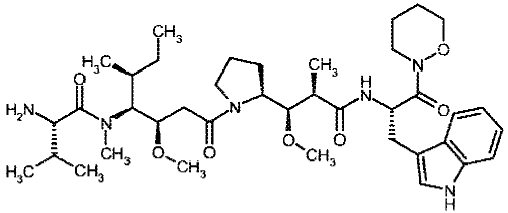

0.5 g (1.01 mmol) von Intermediat 1 wurden in 10 ml Dichlormethan mit 1 ml Trifluoressigsäure versetzt. Nach 30 min Behandlung im Ultraschallbad wurde der Ansatz eingeengt und zunächst mit DCM und dann mit Diethylether nachdestilliert und im Hochvakuum getrocknet. Der ölige Rückstand wurde ohne weitere Reinigung in die nächste Stufe eingesetzt.

500 mg dieses Zwischenproduktes wurden in 20 ml DMF gelöst und mit 466 mg (3.8 mmol) von Intermediat 191, 382 mg (1.01 mmol) O-(7-Azabenzotriazol-1-yl)-N,N,N',N'-tetramethyluronium-hexafluorophosphat (HATU) sowie 440 µL (2.5 mmol) N,N-Diisopropylethylamin versetzt. Die Mischung wurde 1 h bei RT gerührt und danach eingeengt. Der Rückstand wurde in Dichlormethan aufgenommen und zuerst zweimal mit 5%-iger wässriger Zitronensäure-Lösung und dann mit gesättigter wässriger Natriumhydrogencarbonat-Lösung ausgeschüttelt. Die organische Phase wurde eingeengt und der Rückstand durch Flash-Chromatographie an Kieselgel mit Dichlomethan/Methanol 95:5 als Eluent gereinigt. Die entsprechenden Fraktionen wurden vereinigt und das Lösungsmittel im Vakuum entfernt. Nach Trocknen des Rückstands im Hochvakuum wurden 562 mg (65% d. Th. über 2 Stufen) des Z-geschützten intermediats erhalten.

562 mg (0.57 mmol) dieser Zwischenstufe wurden in 50 ml Methanol aufgenommen und mit 155 mg 10%-igem Palladium auf Aktivkohle 20 min lang bei RT unter Wasserstoff-Normaldruck hydriert. Der Katalysator wurde danach abfiltriert und das Lösungsmittel im Vakuum entfernt. Der Rückstand wurde durch präparative HPLC gereinigt. Die entsprechenden Fraktionen wurden vereinigt, das Lösungsmittel im Vakuum abgedampft und der Rückstand aus Dioxan lyophilisiert. Man erhielt 361 mg (87% d.Th.) der Titelverbindung als Schaum.
HPLC (Methode 5): Doppelpeak mit Rt = 1.75 und 1.86 min;
LC-MS (Methode 1): Doppelpeak bei Rt = 0.84 min und 0.91 min mit gleicher Masse; MS (ESIpos): m/z = 944 (M+H)⁺.

### Intermediat 221

### N-{(2S)-2-[(tert.-Butoxycarbonyl)amino]-3-phenylpropyl}-N-methyl-L-valin

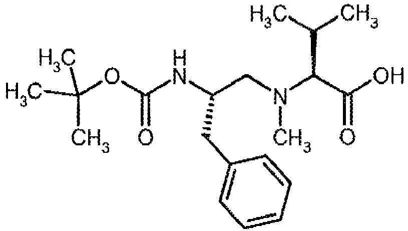

100 mg (0.76 mmol) von kommerziell erhältlichem N-Methyl-L-valin und 285 mg (1.14 mmol) von kommerziell erhältlichem *tert*.-Butyl-[(2S)-1-oxo-3-phenylpropan-2-yl]carbamat wurden in 22 ml Methanol zusammengegeben und mit 340 mg (3.66 mmol) Boran-Pyridin-Komplex und 70 µL Essigsäure versetzt. Das Reaktionsgemisch wurde über Nacht bei RT gerührt. Anschließend wurde im Vakuum eingeengt und der Rückstand durch Flash-Chromatographie an Kieselgel mit Dichlormethan/Methano/17%iger wässriger Ammoniak-Lösung als Eluent gereinigt. Nach Einengen der entsprechenden Fraktionen und Lyophilisation aus Dioxan/Wasser 1:1 wurden 259 mg (93%d.Th.) der Titelverbindung erhalten.
HPLC (Methode 12): Rₜ = 1.6 min;
LC-MS (Methode 11): Rₜ = 0.76 min; MS (ESIpos): m/z = 365 (M+H)⁺.

### Intermediat 222

### N-[(2S)-2-Amino-3-phenylpropyl]-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid-Trifluoracetat

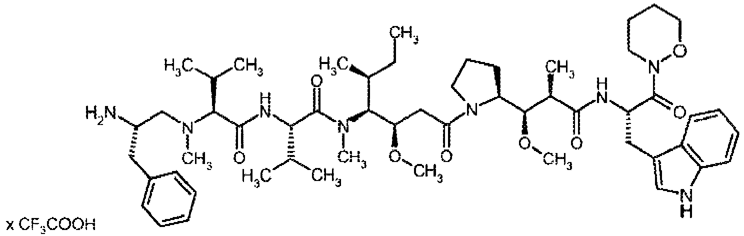

40 mg (0.11 mmol) N-{(2S)-2-[(tert-Butoxycarbonyl)amino]-3-phenylpropyl}-N-methyl-L-valin (Intermediat 221) wurden in 5 ml DMF gelöst und mit 80 mg (0.11 mmol) N-[(3R,4S,5S)-1-{(2S)-2-[(1R,2R)-3-{[(2S)-3-(1H-Indol-3-yl)-1-(1,2-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid (Intermediat 220), 50 mg (0.13 mmol) *O*-(7-Azabenzotriazol-1-yl)-*N,N,N',N'-*tetramethyluronium-hexafluorophosphat (HATU) sowie 57 µL (2.5 mmol) *N,N-*Diisopropylethylamin versetzt. Die Mischung wurde 1 h bei RT gerührt und danach eingeengt. Der Rückstand wurde in Ethylacetat aufgenommen und zuerst mit 5%-iger wässriger Zitronensäure-Lösung und dann mit Wasser gewaschen. Die organische Phase wurde eingeengt und der Rückstand mittels präparativer HPLC gereinigt. Die entsprechenden Fraktionen wurden vereinigt und das Lösungsmittel im Vakuum entfernt. Nach Lyophilisation aus Dioxan wurden 60 mg (50% d. Th.) des geschützten Intermediats erhalten.
HPLC (Methode 12): Rₜ = 2.2 min;
LC-MS (Methode 1): Rₜ = 1.17 min; MS (ESIpos): m/z = 1073 (M+H)⁺.

60 mg (0.05 mmol) dieses Intermediats wurden in 10 ml Dichlormethan aufgenommen, mit 2 ml Trifluoressigsäure versetzt und das Reaktionsgemisch 1.5 h bei RT gerührt. Anschließend wurde das Reaktionsgemisch im Vakuum eingeengt und der verbliebene Rückstand mittels präparativer HPLC gereinigt. Die entsprechenden Farktionen wurden vereinigt, das Lösungsmittel im Vakuum entfernt und der Rückstand aus Dioxan/Wasser lyophilisiert. Auf diese Weise wurden 25 mg (42% d.Th.) der Titelverbindung als Schaum erhalten.
HPLC (Methode 12): Rₜ = 1.9 min;
LC-MS (Methode 1): Rₜ = 0.95 min; MS (ESIpos): m/z = 974 (M+H)⁺.

### Intermediat 223

### N-[(2S)-2-({[2-(2,5-Dioxo-2,5-dihydro-1H-pyrrol-1-yl)ethyl]carbamoyl}amino)-3-phenylpropyl]-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

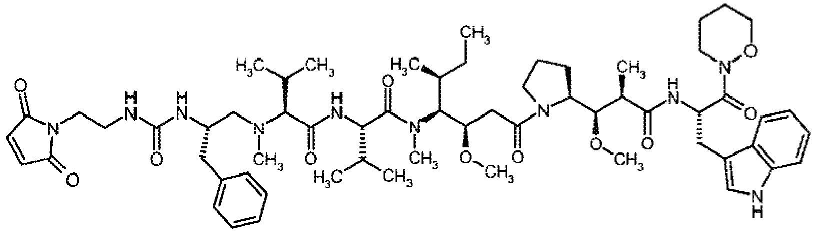

Die Herstellung erfolgte in Analogie zur Synthese von Intermediat 134 ausgehend von 5 mg (4.6 µmol) von Intermediat 222. Es wurden 3.4 mg (65% d. Th.) der Titelverbindung erhalten.
HPLC (Methode 12): Rₜ = 2.0 min:
LC-MS (Methode 1): Rₜ = 0.99 min; MS (ESIpos): m/z = 1140 (M+H)⁺.

### Intermediat 224

### N-[(2S)-2-({[2-(2,5-Dioxo-2,5-dihydro-1H-pyrrol-1-yl)ethyl]carbamoyl}amino)propyl]-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

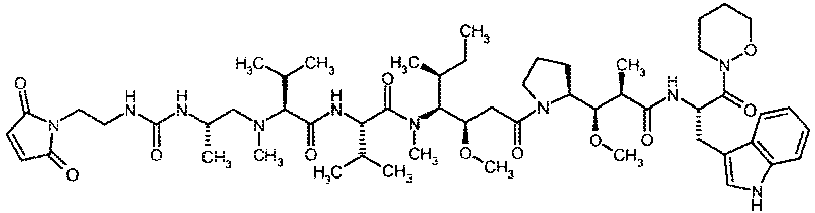

Die Herstellung erfolgte in Analogie zur Synthese von Intermediat 223.
HPLC (Methode 12): Rₜ = 1.9 min;
LC-MS (Methode 1): Rₜ = 0.92 min; MS (ESIpos): m/z = 1064 (M+H)⁺.

### Intermediat 225

### N-(2-Aminoethyl)-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid-Trifluoracetat

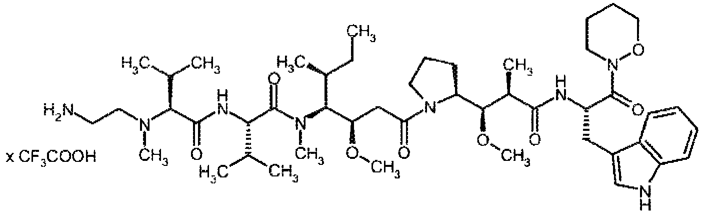

100 mg (0.76 mmol) von kommerziell erhältlichem N-Methyl-L-valin und 182 mg (1.14 mmol) von kommerziell erhältlichem *tert*.-Butyl-(2-oxoethyl)carbamat wurden in 20 ml Methanol zusammengegeben und mit 340 mg (3.66 mmol) Boran-Pyridin-Komplex und 65 µL Essigsäure versetzt. Das Reaktionsgemisch wurde über Nacht bei RT gerührt. Anschließend wurde im Vakuum eingeengt und der Rückstand durch Flash-Chromatographie an Kieselgel mit Dichlormethan/Methano/17%iger wässriger Ammoniak-Lösung (15/4/0.5) als Eluent gereinigt. Nach Einengen der entsprechenden Fraktionen und Lyophilisation aus Dioxan/Wasser 1:1 wurden 190 mg in 39%-iger Reinheit (35% d.Th.) des Intermediats erhalten, das ohne weitere Reinigung weiter umgesetzt wurde.

50 mg (0.07 mmol) dieses Intermediats wurden in 10 ml DMF gelöst und mit 52 mg (0.07 mmol) N-[(3R,4S,5S)-1-{(2S)-2-[(1R,2R)-3-{[(2S)-3-(1H-Indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid (Intermediat 220), 32 mg (0.09 mmol) *O*-(7-Azabenzotriazol-1-yl)-*N,N,N',N'*-tetramethyluronium-Hexafluorophosphat (IIATU) sowie 37 µL (0.2 mmol) *N,N-*Diisopropylethylamin versetzt. Die Mischung wurde über Nacht bei RT gerührt und danach eingeengt. Der Rückstand wurde in Ethylacetat aufgenommen und zuerst mit 5%-iger wässriger Zitronensäure-Lösung und dann mit Wasser ausgeschüttelt. Die organische Phase wurde eingeengt und der Rückstand mittels präparativer HPLC gereinigt. Die entsprechenden Fraktionen wurden vereinigt und das Lösungsmittel im Vakuum entfernt. Nach Lyophilisation aus Dioxan wurden 53 mg (76% d. Th.) des geschützten Intermediats erhalten.

HPLC (Methode 12): Rₜ = 2.0 min;
LC-MS (Methode 1): Rₜ = 1.02 min; MS (ESIpos): m/z = 984 (M+H)⁺.

53 mg (0.05 mmol) dieses Intermediats wurden in 10 ml Dichlormethan aufgenommen, mit 2 ml Trifluoressigsäure versetzt und das Reaktionsgemisch 30 min bei RT gerührt. Anschließend wurde das Reaktionsgemisch im Vakuum eingeengt und der verbliebene Rückstand mittels präparativer HPLC gereinigt. Die entsprechenden Farktionen wurden vereinigt, das Lösungsmittel im Vakuum entfernt und der Rückstand aus Dioxan/Wasser lyophilisiert. Auf diese Weise wurden 21 mg (40% d.Th.) der Titelverbindung in 65%-iger Reinheit erhalten.
HPLC (Methode 12): Rₜ = 1.7 min;
LC-MS (Methode 1): Rₜ = 0.87 min; MS (ESIpos): m/z = 884 (M+H)⁺.

### Intermediat 226

### N-[2-({[2-(2,5-Dioxo-2,5-dihydro-1H-pyrrol-1-yl)ethyl]carbamoyl}amino)ethyl]-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

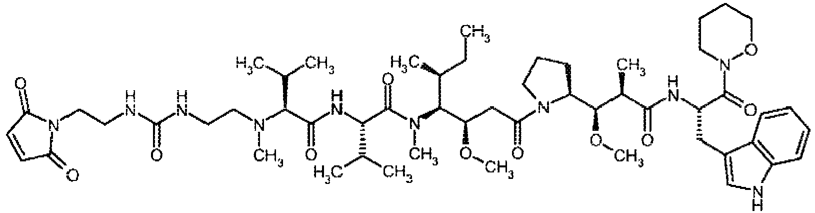

Die Herstellung erfolgie ausgehend von Intermediat 225 in Analogie zur Synthese von Intermediat 134. Es wurden 11.6 mg (59% d.Th.) der Titelverbindung erhalten.
IIPLC (Methode 12): Rₜ = 1.9 min;
LC-MS (Methode 1): Rₜ = 0.90 min; MS (ESIpos): m/z = 1050 (M+H)⁺.

### Intermediat 227

### N-{6-[(2,5-Dioxopyrrolidin-1-yl)oxy]-6-oxohexyl}-N-methyl-L-valyl-N-[(3R,4S,5S)-1-{(2S)-2-[(1R,2R)-3-{[(2S)-1-(benzyloxy)-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

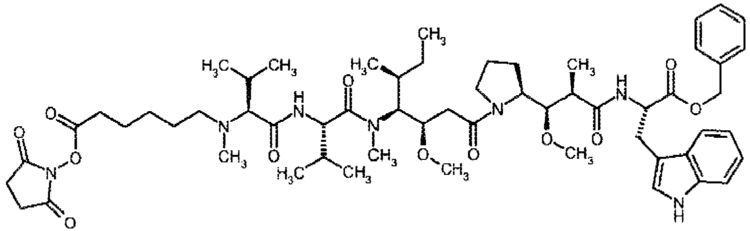

Diese Verbindung wurde analog zu Intermediat 218 durch Überführung in den Aktivester hergestellt.
Ausbeute: 18 mg (51% d. Th.)
HPLC (Methode 5): Rₜ = 2.1 min;
LC-MS (Methode 1): Rₜ = 0.98 min; MS (ESIpos): m/z = 1073 (M+H)⁺.

### Intermediat 228

### (2R,3S)-3-[(tert-Butoxycarbonyl)amino]-4-{2-[6-(2,5-dioxo-2,5-dihydro-1H-pyrrol-1-yl)hexanoyl]hydrazino}-4-oxobutan-2-yl-(3R,4S,7S,10S)-4-[(2S)-butan-2-yl]-7,10-diisopropyl-3-(2-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-2-oxoethyl)-5,11-dimethyl-6,9-dioxo-2-oxa-5,8,11-triazapentadecan-15-oat

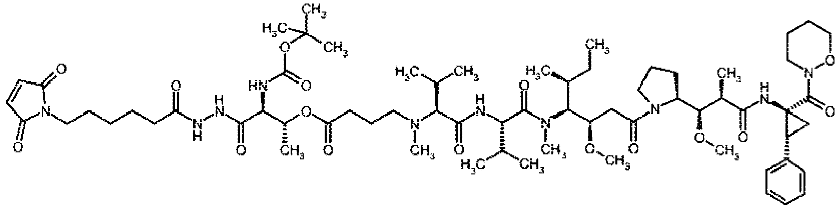

Die Titelverbindung wurde durch Kupplung der bei der Synthese von Intermediat 154 angefallenen Boc-geschützten Zwischenstufe mit kommerziell erhältlichem 6-(2,5-Dioxo-2,5-dihydro-1H-pyrrol-1-yl)hexanhydrazid hergestellt.
HPLC (Methode 12): Rₜ = 2.1 min;
LC-MS (Methode 1): Rₜ = 0.97 min; MS (ESIpos): m/z = 1308 (M+H)⁺.

### Intermediat 229

### (2R,3S)-3-Acetamido-4-{2-[6-(2,5-dioxo-2,5-dihydro-1H-pyrrol-1-yl)hexanoyl]hydrazino}-4-oxobutan-2-yl-(3R,4S,7S,10S)-4-[(2S)-butan-2-yl]-7,10-diisopropyl-3-(2-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-2-oxoethyl)-5,11-dimethyt-6,9-dioxo-2-oxa-5,8,11-triazapentadecan-15-oat

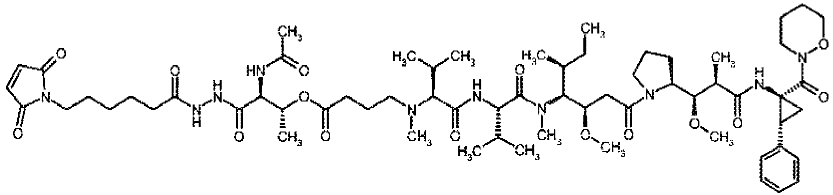

Die Titelverbindung wurde aus 7.5 mg (2.5 µmol) von Intermediat 154 durch Acetylierung mit 2.3 µl Acetanhydrid in 1 ml DMF in Gegenwart von 0.4 µl *N*,*N*-Diisopropylethylamin hergestellt.
Ausbeute: 1.4 mg (40% d. Th.)
HPLC (Methode 12): Rₜ = 1.9 min;
LC-MS (Methode 1): Rₜ = 0.86 min; MS (ESIpos): m/z = 1250 (M+H)⁺.

### Intermediat 230

### (2R,3S)-3-[(tert.-Butoxycarbonyl)amino]-4-{2-[6-(2,5-dioxo-2,5-dihydro-1H-pyrrol-1-yl)hexanoyl]hydrazino}-4-oxobutan-2-yl-(3R,4S,7S,10S)-4-[(2S)-butan-2-yl]-3-(2-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-2-oxoethyl)-7,10-diisopropyl-5,11-dimethyl-6,9-dioxo-2-oxa-5,8,11-triazapentadecan-15-oat

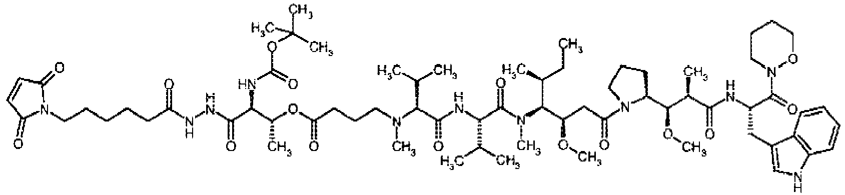

Diese Verbindung wurde in Analogie zu Intermediat 228 ausgehend von Intermediat 193 hergestellt. Es wurden 16 mg (30% d. Th. über 3 Stufen) der Titelverbindung erhalten.
HPLC (Methode 12): Rₜ = 2.0 min;
LC-MS (Methode 1): Rₜ = 1.02 min; MS (ESIpos): m/z = 1335 (M+H)⁺.

### Intermediat 231

### (2R,3S)-3-Acetamido-4-{2-[6-(2,5-dioxo-2,5-dihydro-1H-pyrrol-1-yl)hexanoyl]hydrazino}-4-oxobutan-2-yl-(3R,4S,7S,10S)-4-[(2S)-butan-2-yl]-3-(2-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-2-oxoethyl)-7,10-diisopropyl-5,11-dimethyl-6,9-dioxo-2-oxa-5,8,11-triazapentadecan-15-oat

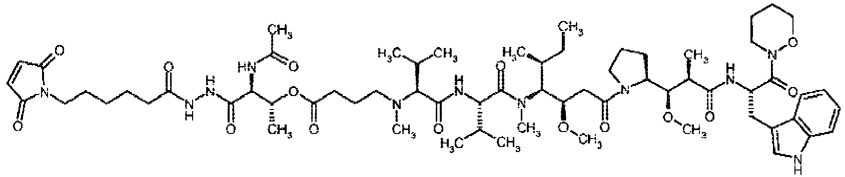

Diese Verbindung wurde aus 8 mg (6 µmol) von Intermediat 230 zunächst durch Entschützung mit Trifluoressigsäure und nachfolgende Acetylierung mit Acetanhydrid in DMF in Gegenwart von *N,N*-Diisopropylethylamin hergestellt. Es wurden 2 mg (37% d. Th. über 2 Stufen) der Titelverbindung erhalten.
IIPLC (Methode 12): Rₜ = 1.9 min;
LC-MS (Methode 1): Rₜ = 0.88 min; MS (ESIpos): m/z = 1277 (M+H)⁺.

### Intermediat 232

### Benzyl-N-[(4-nitrophenoxy)carbonyl]-beta-alaninat

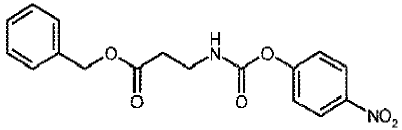

200 mg (0.57 mmol) von kommerziell erhältlichem 4-Methylbenzolsulfonsäure-benzyl-beta-alaninat sowie 229 mg (1.14 mmol) 4-Nitrophenylchlorocarbonat wurden in 15 ml Tetrahydrofuran aufgenommen und das Reaktionsgemisch dann 30 min zum Rückfluss erhitzt. Anschließend wurde das Reaktionsgemisch im Vakuum eingeengt und der Rückstand mittels präparativer HPLC gereinigt. Nach Einengen der entsprechenden Fraktionen und Trocknen des Rückstandes im Hochvakuum wurden 86 mg (44% d.Th.) der Titelverbindung erhalten.
HPLC (Methode 12): Rₜ = 1.8 min;
LC-MS (Methode 1): Rₜ = 1.07 min; MS (ESIpos): m/z = 345 (M+H)⁺.

### Intermediat 233

### N-{2-[({3-[(2,5-Dioxopyrrolidin-1-yl)oxy]-3-oxopropyl}carbamoyl)amino]ethyl}-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

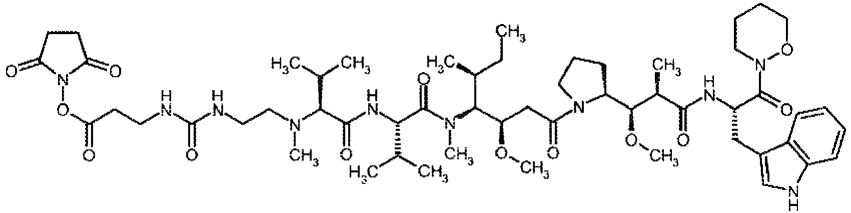

13 mg (10 µmol) von Intermediat 225 und 6.7 mg (20 µmol) von Intermediat 232 wurden in 3 ml DMF gelöst und anschließend mit 7 µL N,N-Diisopropylethylamin versetzt. Die Mischung wurde über Nacht bei RT gerührt und anschließend im Hochvakuum eingeengt. Der verbliebene Rückstand wurde mittels präparativer HPLC aufgereinigt. Nach Einengen der entsprechenden Fraktionen und Trocknen des Rückstandes im Hochvakuum wurden 5.4 mg (38% d.Th.) des geschützten Intermediats erhalten.
HPLC (Methode 5): Rₜ = 2.1 min;
LC-MS (Methode 1): Rₜ = 0. 6in; MS (ESIpos): m/z = 1089 (M+H)⁺.

5.4 mg (5 µmol) dieses Intermediats wurden in 5 ml Methanol gelöst und nach Zugabe von 2 mg 1 Palladium auf Aktivkohle 20 min lang bei RT unter Wasserstoff-Normaldruck hydriert. Der Katalysator wurde danach abfiltriert und das Lösungsmittel im Vakuum entfernt. Nach Trocknen des Rückstands im Hochvakuum wurden 5 mg (quant.) des Säure-Intermediats erhalten.
HPLC (Methode 12): Rₜ = 1.8 min:
LC-MS (Methode 1): Rₜ = 0.84 min; MS (ESIpos): m/z = 999 (M+H)⁺.

5 mg (10 µmol) dieses Intermediats wurden in 1 ml DMF gelöst und mit 5.8 mg (50 mmol) 1-Hydroxypyrrolidin-2,5-dion und anschließend mit 2.6 µl *N,N*-Diisopropylethylamin und 3.8 mg (10 µmol) HATU versetzt. Nach 20 h Rühren bei RT wurde das Reaktionsgemisch im Vakuum eingeengt. Der verbliebene Rückstand wurde mittels präparativer HPLC gereinigt. Nach Lyophilisation aus Dioxan/Wasser 1:1 wurden 1.1 mg (20% d.Th.) der Titelverbindung erhalten.
HPLC (Methode 12): Rₜ = 1.9 min;
LC-MS (Methode 1): Rₜ = 0.87 min; MS (ESIpos): m/z = 1096 (M+H)⁺.

### Intermediat 234

### N-(6-{[(Benzyloxy)carbonyl]amino}hexyl)-N-methyl-L-valyl-N-[(3R,4S,5S)-3-methoxy-1-{(2S)-2-[(1R,2R)-1-methoxy-2-methyl-3-oxo-3-{[(1S)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-L-valinamid

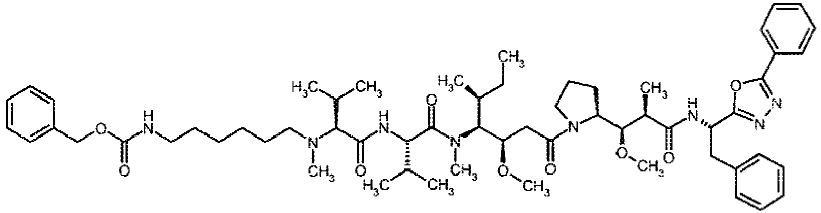

25 mg (30 µmol) N-Methyl-*L*-valyl-N-[(*3R*,4*S,*5*S*)-3-methoxy-1-{(2*S*)-2-[(1*R*,2*R*)-1-methoxy-2-methyl-3-oxo-3-{[(1*S*)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-*L*-valinamid (Intermediat 55) und 45 mg (180 µmol) Benzyl-(6-oxohexyl)carbamat wurden in 3 ml Methanol aufgenommen und mit Essigsäure sauer gestellt. Bei Raumtemperatur wurden anschliessend 15 µl (144 µmol; 9.4M) Boran-Pyridin-Komplex zugegeben. Der Ansatz wurde anschliessend für 24 h bei RT gerührt, wobei nach 8 h erneut Essigsäure sowie 15 µl (144 µmol; 9.4M) Boran-Pyridin-Komplex zugegeben wurden. Das Reaktionsgemisch wurde anschliessend mit TFA auf pH 2 eingestellt und mittels präparativer HPLC aufgereinigt. Die Produktfraktionen wurden vereinigt, eingeengt und der Rückstand im Hochvakuum getrocknet. Es wurden so 15 mg (46% d. Th.) der Titelverbindung als Schaum erhalten.
LC-MS (Methode 1): Rₜ = 1.03 min; m/z = 1066 (M+H)⁺.

### Intermediat 235

### N-(6-Aminohexyl)-N-methyl-L-valyl-N-[(3R,4S,5S)-3-methoxy-1-{(2S)-2-[(1R,2R)-1-methoxy-2-methyl-3-oxo-3-{[(1S)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-L-valinamid

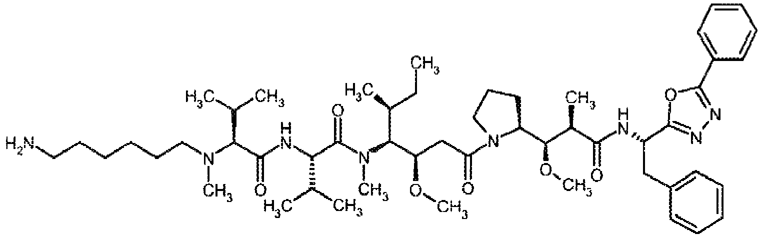

15 mg (14 µmol) N-(6-{[(Benzyloxy)carbonyl]amino}hexyl)-N-methyl-L-valyl-N-[(3R,4S,5S)-3-methoxy-1-{(2S)-2-[(1R,2R)-1-methoxy-2-methyl-3-oxo-3-{[(1S)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-L-valinamid (Intermediat 234) wurden in 3 ml Methanol aufgenommen und 1.8 mg Palladium auf Kohle (5%) zugegeben. Das Reaktionsgemisch wurde anschliessend 2 h bei RT unter Wasserstoff-Normaldruck hydriert. Der Katalysator wurde danach abfiltriert und das Lösungsmittel im Vakuum entfernt. Der Rückstand wurde aus Acetonitril/Wasser 1:1 lyophilisiert. Es wurden 11 mg (86% d. Th.) der Titelverbindung als Schaum erhalten.
LC-MS (Methode 1): Rₜ = 0.81 min; m/z = 932 (M+H)⁺.

### Intermediat 236

### N-[6-(2,5-Dioxo-2,5-dihydro-1H-pyrrol-1-yl)hexyl]-N-methyl-L-valyl-N-[(3R,4S,5S)-3-methoxy-1-{(2S)-2-[(1R,2R)-1-methoxy-2-methyl-3-oxo-3-{[(1S)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-L-valinamid

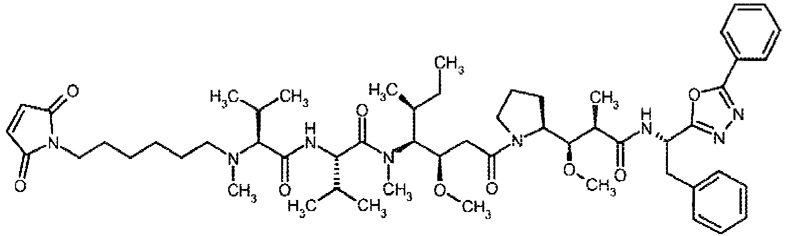

11 mg (12 µmol) N-(6-Aminohexyl)-N-methyl-L-valyl-N-[(3R,4S,5S)-3-methoxy-1-{(2S)-2-[(1R,2R)-1-methoxy-2-methyl-3-oxo-3-{[(1S)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-L-valinamid (Intermediat 235) wurden in 500 µl Dioxan/Wasser 1:1 aufgenommen und mit 253 µl IM wässriger Natriumhydrogencarbonat-Lösung und anschließend mit 2.8 mg (18 µmol) Methyl-2,5-dioxo-2,5-dihydro-1H-pyrrol-1-carboxylat versetzt. Das Reaktionsgemisch wurde 30 min bei RT gerührt und danach mit Trifluoressigsäure sauer gestellt. Das Reaktionsgemisch wurde mittels präparativer HPLC gereinigt. Nach Lyophilisation wurden 0.8 mg (7% d.Th.) der Titelverbindung erhalten.
LC-MS (Methode 1): Rₜ = 1.01 min; m/z = 1012 (M+H)⁺.

### Intermediat 237

### N-(5-Carboxypentyl)-N-methyl-L-valyl-N-[(3R,4S,5S)-3-methoxy-1-{(2S)-2-[(1R,2R)-1-methoxy-2-methyl-3-oxo-3-{[(1S)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-L-valinamid

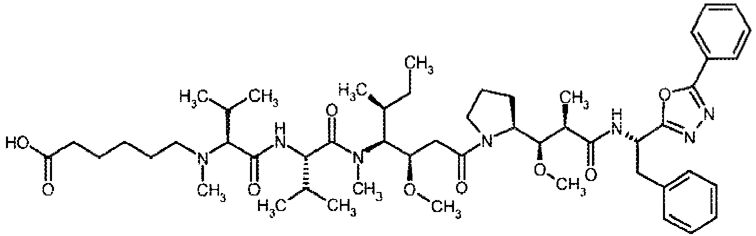

25 mg (30 µmol) N-Methyl-*L*-valyl-N-[(3*R,*4*S*,5*S*)-3-methoxy-1-{(2*S*)-2-[(1*R*,2*R*)-1-methoxy-2-methyl-3-oxo-3-{[(1*S*)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-*L*-valinamid (Intermediat 55)) und 23 mg (180 µmol) 6-Oxohexansäure wurden in 3 ml Methanol aufgenommen und mit Essigsäure sauer gestellt. Bei Raumtemperatur wurden anschliessend 15 µl (144 µmol; 9.4M) Boran-Pyridin-Komplex zugegeben. Das Reaktionsgemisch wurde anschliessend fiir 20 h bei RT gerührt, wobei nach 8 h erneut Essigsäure sowie 15 µl (144 µmol; 9.4M) Boran-Pyridin-Komplex zugegeben wurden. Das Reaktionsgemisch wurde anschliessend mit Trifluoressigsäure auf pH 2 eingestellt und mittels präparativer HPLC aufgereinigt. Die Produktfraktionen wurden vereinigt, eingeengt und der Rückstand lyophilisiert. Es wurden so 21 mg (74% d. Th.) der Titelverbindung als Schaum erhalten.
LC-MS (Methode 1): Rₜ = 0.91 min; m/z = 947 (M+H)⁺.

### Intermediat 238

### N-{6-[(2,5-Dioxopyrrolidin-1-yl)oxy]-6-oxohexyl}-N-methyl-L-valyl-N-[(3R,4S,5S)-3-methoxy-1-{(2S)-2-[(1R,2R)-1-methoxy-2-methyl-3-oxo-3-{[(1S)-2-phenyl-1-(5-phenyl-1,3,4-oxadiazol-2-y])ethyl]amino}propyl]pyrrolidin-1-yl}-5-methyl-1-oxoheptan-4-yl]-N-methyl-L-valinamid

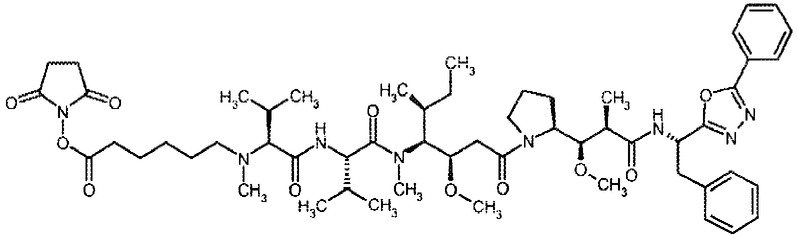

21 mg (22 µmol) von Intermediat 237 wurden in 1 ml DMF gelöst und mit 38 mg (333 µmol) 1-Hydroxypyrrolidin-2,5-dion und anschließend mit 2.4 mg (10 µmol) O-(7-Azabenzotriazol-1-yl)-N,N,N',N'-tetramethyluronium-hexafluorophosphat (IIATU) und 19 µl N,N-Diisopropylethylamin versetzt. Nach 2 h Rühren bei RT wurde das Reaktionsgemisch mittels präparativer HPLC gereinigt. Nach Lyophilisation aus Dioxan wurden 22 mg (96% d.Th.) der Titelverbindung erhalten.
LC-MS (Methode 1): Rₜ = 0.95 min; m/z = 1044 (M+H)⁺.

### Intermediat 239

### N-Methyl-L-threonyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid-Trifluoracetat

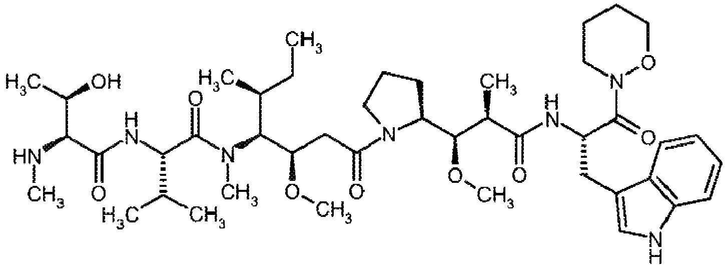

Zunächst wurde *N*-[(Benzyloxy)carbonyl]-*N*-methyl-L-threonin aus 237 mg (0.887 mmol) seines Dicyclohexylamin-Salzes durch Aufnehmen in Ethylacetat und Ausschütteln mit 5%-iger wässriger Schwefelsäure freigesetzt. Die organische Phase wurde über Magnesiumsulfat getrocknet, filtriert und eingeengt. 14.7 mg (0.055 mmol) von *N*-[(Benzyloxy)carbonyl]-*N*-methyl-L-threonin wurden in 3 ml DMF aufgenommen und nacheinander mit 40 mg (0.055 mmol) von Intermediat 220, 12.7 mg (0.066 mmol) 1-(3-Dimethylaminopropyl)-3-ethylcarbodiimid-Hydrochlorid und 10 mg (0.066 mmol) 1-Hydroxy-1*H*-benzotriazol-Hydrat versetzt. Die Mischung wurde anschließend 2 h bei RT gerührt. Das Lösungsmittel wurde danach im Vakuum entfernt und der Rückstand durch präparative HPLC gereinigt. Es wurden so 29 mg (54% d. Th.) des Z-geschützten Intermediats erhalten.
LC-MS (Methode 1): Rₜ = 1.15 min; MS (ESIpos): m/z = 976 (M+H)⁺.

29 mg (0.003 mmol) dieses Intermediats wurden in 5 ml Methanol gelöst und bei RT und Normaldruck 1 h lang über 5 mg 5% Palladium/Kohle hydriert. Der Katalysator wurde anschließend abfiltriert und das Lösungsmittel abgedampft. Der verbliebene Rückstand wurde durch präparative HPLC gereinigt. Es wurden 17 mg (54% d. Th.) der Titelverbindung erhalten.
LC-MS (Methode 1): Rₜ = 0.77 min; MS (ESIpos): m/z = 842 (M+H)⁺.

### Intermediat 240

### N-{6-[(2,5-Dioxopyrrolidin-1-yl)oxy]-6-oxohexyl}-N-methyl-L-threonyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

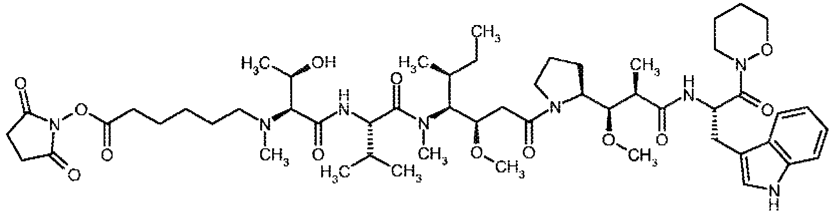

Diese Verbindung wurde in Analogie zu Intermediat 210 aus 15.6 mg (0.016 mmol) Intermediat 239 hergestellt. Es wurden 10.8 mg (67% d. Th. über 2 Stufen) der Titelverbindung erhalten.
HPLC (Methode 5): Rₜ = 1.7 min;
LC-MS (Methode 1): Rₜ = 0. 85 min; MS (ESIpos): m/z = 1053 (M+H)⁺.

### Intermediat 241

### N-methyl-L-valyl-N-[(3R,4S,5S)-1-{(2S)-2-[(1R,2R)-3-{[(2S)-3-(4-hydroxyphenyl)-1-(1,2-oxazinan-2-yl)-1-oxopropan-2-yl]amino}-1-metoxy-2-methyl-3-oxopropyl]pyrrolidin-1-yl}-3-methoxy-5-methyl-1-oxoheptan-4-yl]-N-methyl-L-valinamid-Trifluoracetat

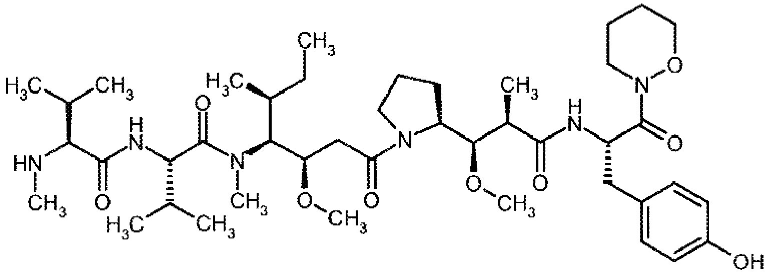

Zunächste wurde in Analogie zu Intermediat 5 Trifluoressigsäure-(2S)-2-amino-3-(4-hydroxyphenyl)-1-(1,2-oxazinan-2-yl)propan-1-on(1:1) hergestellt. Aus diesem Baustein wurde dann in Analogie zu der in Intermediat 75 beschriebenen Synthese durch Kupplung mit *N*-(*tert*.-Butoxycarbonyl)-*N*-methyl-L-valyl-*N*-[(3*R*,4*S*,5*S*)-1-{(2*S*-2-[(1*R*,2*R*)-2-carboxy-1-methoxypropyl]pyrrolidin-1-yl}-3-methoxy-5-methyl-1-oxoheptan-4-yl]-methyl-L-valinamid (Intermediat 26) in Gegenwart von O-(7-Azabenzotriazol-1-yl)-*N*,*N'*,*N*'-tetramethyluronium-hexafluorophosphat und anschließende Abspaltung der Boc-Schutzgruppe mittels Trifluoressigsäure die Titelverbindung hergestellt.
HPLC (Methode 12): Rₜ = 1.7 min;
LC-MS (Methode 1): Rₜ = 0.75 min; MS (ESIpos): m/z = 817 (M+H)⁺.

### Intermediat 242

### N-{6-[(2,5-Dioxopyrrolidin-1-yl)oxy]-6-oxohexyl}-N-methyl-L-valyl-N-[(3R,4S,5S)-1-{(2S)-2-[(1R,2R)-3-{[(2S)-3-(4-hydroxyphenyl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

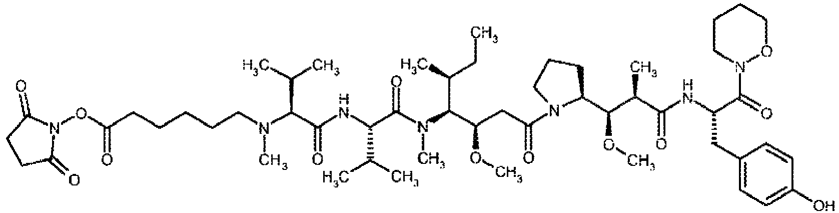

50 mg (0.05 mmol) von Intermediat 241 wurden in Analogie zu Intermediat 210 mit 6-Oxohexansäure in Gegenwart von Boran-Pyridin-Komplex umgesetzt. Anschließend wurden 22.5 mg (0.02 mmol) der erhaltenen Säure in den aktivierten Ester überführt. Es wurden 13.5 mg (36% d. Th. über 2 Stufen) der Titelverbindung erhalten.
HPLC (Methode 12): Rₜ = 1.8 min;
LC-MS (Methode 1): Rₜ = 0.86 min; MS (ESIpos): m/z = 1028 (M+H)⁺.

### Intermediat 243

### N-(6-Aminohexyl)-N-methyl-L-valyl-N-[(3R,4S,5S)-1-{(2S)-2-[(1R,2R)-3-{[(2S)-3-(4-hydroxyphenyl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

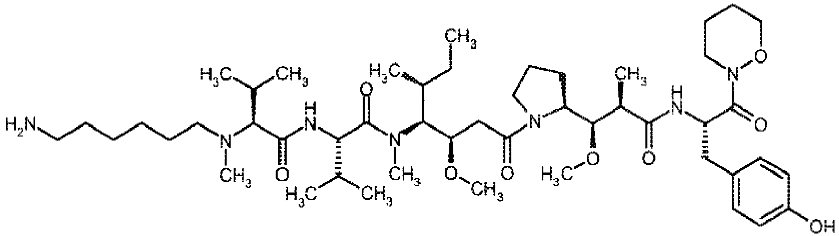

Die Herstellung erfolgte in Analogie zu Intermediat 78 durch reduktive Alkylierung von Intermediat 241 mit Benzyl-(6-oxohexyl)carbamat und Boran-Pyridin-Komplex und anschließender Hydrierung in Methanol als Lösungsmittel.
Ausbeute: 17.5 mg (34% d.Th. über 2 Stufen)
HPLC (Methode 12): Rₜ = 1.7 min;
LC-MS (Methode 1): Rₜ = 0.63 min; MS (ESIpos): m/z = 916 (M+H)⁺.

### Intermediat 244

### N-[6-(2,5-Dioxo-2,5-dihydro-1H-pyrrol-1-yl)hexyl]-N-methyl-L-valyl-N-[(3R,4S,5S)-1-{(2S)-2-[(1R,2R)-3-{[(2S)-3-(4-hydroxyphenyl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

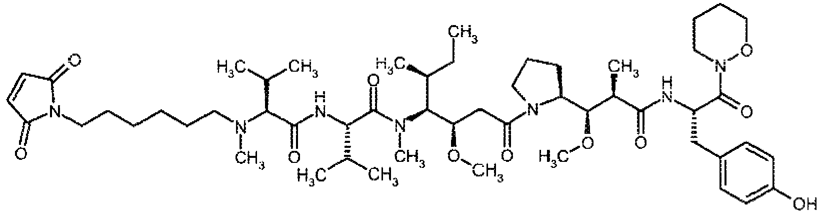

Die Herstellung erfolgte in Analogie zu Intermediat 166 ausgehend von Intermediat 243.
Ausbeute: 1.3 mg (12% d.Th.)
HPLC (Methode 12): Rₜ = 1.9 min;
LC-MS (Methode 1): Rₜ = 0.89 min; MS (ESIpos): m/z = 996 (M+H)⁺.

### Intermediat 245

### 2,5-Dioxopyrrolidin-1-yl-O-[(3R,4S,7S,10S)-4-[(2S)-butan-2-yl]-3-(2-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-2-oxoethyl)-7,10-diisopropyl-5,11-dimethyl-6,9,15-trioxo-2-oxa-5,8,11-triazapentadecan-15-yl]-N-(tert.-butoxycarbonyl)-L-threonyl-beta-alaninat

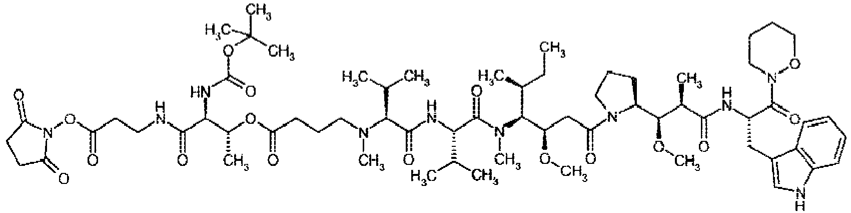

Zunächst wurde Intermediat 193 wie für Intermediat 154 beschrieben mit Benzyl-N-(tert.-butoxycarbonyl)-L-threoninat umgesetzt und anschließend der Benzylester hydrogenolytisch entfernt. 30 mg (0.027 mmol) von dem so erhaltenen N-[4-({(1S,2R)-1-[(tert.-Butoxycarbonyl)amino]-1-carboxypropan-2-yl}oxy)-4-oxobutyl]-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid wurden dann mit 4-Methylbenzolsulfonsäure-benzyl-beta-alaninat in Gegenwart von HATU gekuppelt und der Benzylester erneut durch Hydrogenolyse entfernt (Ausbeute: 24 mg (71% d. Th. über 2 Stufen). Schließlich wurden 10 mg (0.008 mmol) der erhaltenen Säure in den aktivierten Ester überführt. Nach HPLC Reinigung wurden 2.7 mg (23% d. Th.) der Titelverbindung erhalten.
HPLC (Methode 5): Rₜ = 1.9 min;
LC-MS (Methode 1): Rₜ = 1.01 min; MS (ESIpos): m/z = 1295 (M+H)⁺

### Intermediat 246a

### (2S)-2-Amino-1-(4-hydroxy-1,2-oxazolidin-2-yl)-3-(1H-indol-3-yl)propan-1-on-Trifluoracetat (Diastereomer 1)

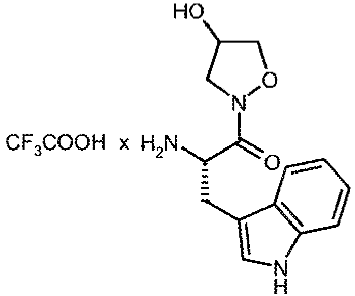

1.6 g (3.982 mmol) 2,5-Dioxopyrrolidin-1-yl-N-(tert.-butoxycarbonyl)-L-tryptophanat wurden in 15 ml DMF gelöst und mit 500 mg (3.982 mmol) 1,2-Oxazolidin-4-ol und 100 µl *N,N-*Diisopropylethylamin versetzt. Das Reaktionsgemisch wurde über Nacht bei RT gerührt. Dann wurden nochmals 100 µl *N,N*-Diisopropylethylamin hinzugegeben, der Ansatz zunächst 5 h im Ultraschallbad behandelt, dann über Nacht bei RT gerührt und anschließend im Vakuum eingeengt. Der verbliebene Rückstand wurde in Ethylacetat aufgenommen und zuerst zweimal mit 5%-iger wässriger Zitronensäure-Lösung, dann mit gesättigter wässriger Natriumhydrogencarbonat-Lösung und schließlich mit Wasser extrahiert. Die organische Phase wurde eingeengt und der Rückstand durch Flash-Chromatographie an Kieselgel mit Dichlormethan/Methanol 95:5 als Eluent in die Diastereomere aufgetrennt. Die entsprechenden Fraktionen von beiden Diastereomeren wurden vereinigt und das Lösungsmittel im Vakuum entfernt. Nach Trocknen der Rückstände im Hochvakuum wurden 272 mg (18% d.Th.) von Diastereomer 1 (R_{f} = 0.18 (Dichlormethan/Methanol 95:5) und 236 mg (16% d.Th.) von Diastereomer 2 (R_{f} = 0.13 (Dichlormethan/Methanol 95:5) sowie 333 mg (22% d.Th.) einer Mischfraktion der Bocgeschützten Intermediate erhalten.

Aus 272 mg (725 µmol) von Diastereomer 1 dieses Intermediats wurde nach Standardbedingungen mit 5 ml Trifluoressigsäure in 20 ml Dichlormethan die Boc-Schutzgruppe abgespalten und nach Lyophilisation aus Dioxan/Wasser 290 mg (quant) der Titelverbindung in 75%iger Reinheit erhalten und ohne weitere Aufreinigung in die nächste Stufe eingesetzt..
HPLC (Methode 12): Rₜ = 1.1 min;
LC-MS (Methode 13): Rₜ = 1.80 min; MS (ESIpos): m/z = 276 (M+H)⁺

### Intermediat 246b

### (2S)-2-Amino-1-(4-hydroxy-1,2-oxazolidin-2-yl)-3-(1H-indol-3-yl)propan-1-on-Trifluoracetat (Diastereomer 2)

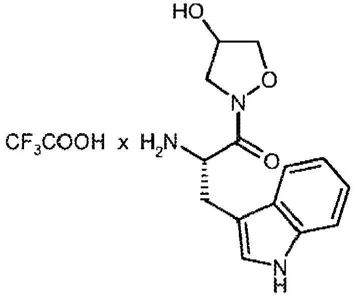

Aus 236 mg (630 µmol) von Diastereomer 2 des unter 246a beschriebenen Intermediats wurden nach Standardbedingungen mit 5 ml Trifluoressigsäure in 20 ml Dichlormethan die Boc-Schutzgruppe abgespalten und nach Einengen, Verrühren mit Diethylether und Trocknen des Rückstandes im Hochvakuum 214 mg (76%) der Titelverbindung erhalten.
LC-MS (Methode 13): Rₜ = 1.84 min; MS (ESIpos): m/z = 276 (M+H)⁺

### Intermediat 247a

### N-{6-[(2,5-Dioxopyrrolidin-1-yl)oxy]-6-oxohexyl)-N-methyl-L-valyl-N-[(3R,4S,5S}-1-{(2S)-2-[(1R,2R)-3-{[(2S)-1-(4-hydroxy-1,2-oxazolidin-2-yl)-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid (Diastereomer 1)

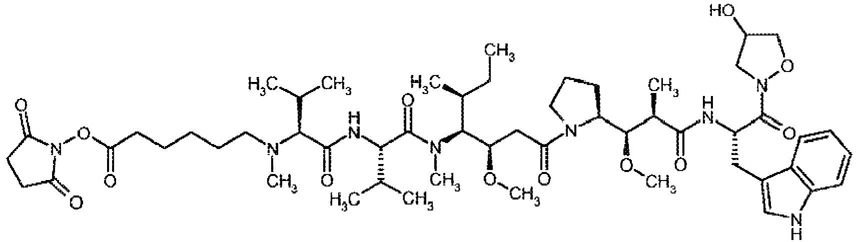

Zur Synthese dieser Verbindung wurde zunächst wie für Intermediat 74 beschrieben die Kupplung der Intermediate 26 und 246a mit anschließender Abspaltung der Boc-Schutzgruppe durchgeführt. Anschließend wurde wie für Intermediat 210 beschrieben die Alkylierung mit 6-Oxohexansäure in Gegenwart von Boran-Pyridin-Komplex und anschließende Überführung der Säure in den Aktivester durchgeführt. Die Titelverbindung wurde durch präparative HPLC gereinigt.
HPLC (Methode 12): Rₜ = 1.8 min;
LC-MS (Methode 1): Rₜ = 0.86 min; MS (ESIpos): m/z = 1053 (M+H)⁺

### Intermediat 247b

### N-{6-[(2,5-Dioxopyrrolidin-1-yl)oxy]-6-oxohexyl}-N-methyl-L-valyl-N-[(3R,4S,5S)-1-{(2S)-2-[(1R,2R)-3-{[(2S)-1-(4-hydroxy-1,2-oxazolidin-2-yl)-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid (Diastereomer 2)

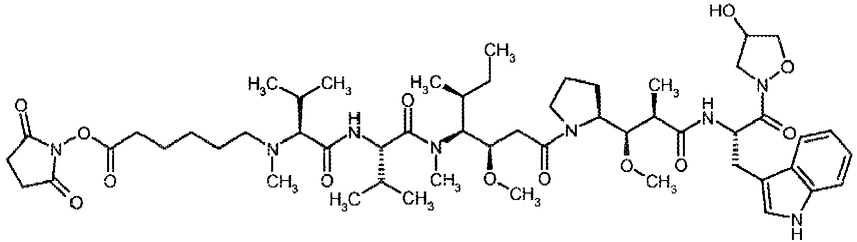

Zur Synthese dieser Verbindung wurde zunächst wie für Intermediat 74 beschrieben die Kupplung der Intermediate 26 und 246b mit anschließender Abspaltung der Boc-Schutzgruppe durchgeführt. Anschließend wurde wie für Intermediat 210 beschrieben die Alkylierung mit 6-Oxohexansäure in Gegenwart von Boran-Pyridin-Komplex und anschließende Überführung der Säure in den Aktivester durchgeführt. Die Titelverbindung wurde durch präparative IIPLC gereinigt.
HPLC (Methode 12): Rₜ = 1.8 min;
LC-MS (Methode 1): Rₜ = 0.86 min; MS (ESIpos): m/z = 1053 (M+H)⁺

### Intermediat 248

### N-(5-Carboxypentyl)-N-methyl-L-valyl-N-[(3R,4S,5S)-1-{(2S)-2-[(1R,2R)-3-{[(2S)-1-tert.-butoxy-3-(4-hydroxyphenyl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

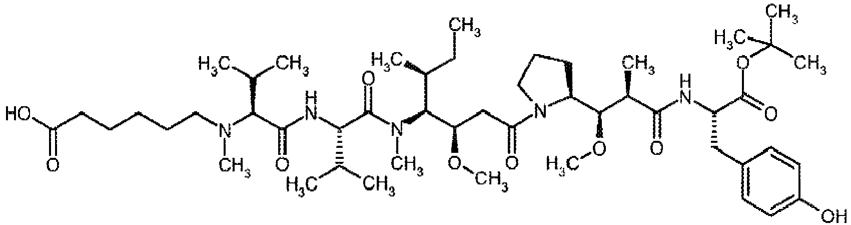

Zunächst wurde in Analogie zu der in Intermediat 86 beschriebenen Synthese durch Kupplung von *N*-(*tert*.-Butoxycarbonyl)-*N*-methyl-L-valyl-*N-*[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 26) und tert.-Butyl-L-tyrosinat in Gegenwart von *O*-(7-Azabenzotriazol-1-yl)-*N*,*N*,*N*',*N*'-tetramethyluronium-hexafluorophosphat und anschließende Abspaltung der Boc-Schutzgruppe mittels Trifluoressigsäure unter Erhalt des tert.-Butylesters (40 min Rühren mit Trifluoressigsäure in Dichlormethan) die Amin-Verbindung tert.-Butyl-N-[(2R,3R)-3-methoxy-3-{(2S)-1-[(3R,4S,5S)-3-methoxy-5-methyl-4-(methyl{(2S)-3-methyl-2-[(N-methyl-L-valyl)amino]butyl}amino)heptanoyl] pyrrolidin-2-yl}-2-methylpropanoyl]-L-tyrosinat als Trifluoracetat hergestellt. Aus 38 mg (0.04 mmol) dieser Verbindung wurden dann in Analogie zur Herstellung von Intermediat 210 durch Umsetzung mit 6-Oxohexansäure in Gegenwart von Boran-Pyridin-Komplex 31 mg (99% d. Th.) der Titelverbindung erhalten.
HPLC (Methode 12): Rₜ = 1.8 min;
LC-MS (Methode 1): Rₜ = 0.88 min; MS (ESIpos): m/z = 918 (M+H)⁺.

### B: Herstellung von Antikörper-Wirkstoff-Konjugaten (ADC)

### B-1. Allgemeines Verfahren zur Generietug von anti-C4.4a Antikörpern

Die anti-C4.4a Antikörper beschrieben durch die Sequenzen gemäß Tabellel und Tabelle 2 wurden durch Screening einer Phagen Display Bibliothek auf rekombinantem humanem C4.4a SEQ ID NO: 1 und murinem C4.4a SEQ ID NO: 2 und auf C4.4a exprimierenden Zellen generiert. Die so gewonnenen Antikörper wurden ins humane IgG1 Format reformatiert und für die hier beschriebenen Ausführungsbeispiele verwendet.

### B-2. Allgemeines Verfahren zur Expression von anti-C4.4a Antikörpern in Säugerzellen

Die Antikörper, zum Beispiel M31-B01 (leichte Kette SEQ ID NO: 346 und schwere Kette SEQ ID NO: 347) oder weitere Antikörper gemäß Tabelle 2 wurden in Säugerzellkultur produziert. Dazu wurden HEK293 6E Zellen transient mit einem geeigneten CMV-Promotor basierenden Expressionplasmid transfiziert. Die schweren und leichten Ketten der Antikörper wurden entweder zusammen in ein Einvektorsystem, oder getrennt in ein Zweivektorsystem kloniert. Der Zellkulturmassstab war entweder bis 1.5 L im Schüttelkolben oder 10 L im "Wave-Bag". Die Expression erfolgte bei 37°C für 5-6 Tage in F17 Medium (Invitrogen) ergänzt mit Tryptone TN1 (Organotechnie) mit 1 % "FCS ultra low IgG" (Invitrogen) und 0.5 mM Valproinsäure. Die Expressionsausbeuten lagen zwischen 100 und 600 mg/l

### B-3. Allgemeines Verfahren zur Aufreinigung von Antikörpern aus Zellüberständen.

Die Antikörper, zum Beispiel M31-B01 (leichte Kette SEQ ID NO: 346 und schwere Kette SEQ ID NO: 347) oder weitere Antikörper gemäß Tabelle 2 wurden aus den Zellkulturüberständen. gewonnen. Die Zellüberstände wurden durch Zentrifugation von Zellen geklärt. Anschließend wurde der Zellüberstand durch Affinitätschromatographie auf einer MabSelect Sure (GE Healthcare) Chromatographiesäule gereinigt. Dazu wurde die Säule in DPBS pH 7.4 (Sigma/Aldrich) equillibriert, der Zellüberstand aufgtretragen und die Säule mit ca 10 Säulenvolumina DPBS pH 7.4 + 500 mM Natriumchlorid gewaschen. Die Antikörper wurden in 50 mM Natriumacetat pH 3.5 + 500 mM Natriumchlorid eluiert und anschliessend durch Gelfiltrationschromatographie auf einer Superdex 200 Säule (GE Helthcare) in DPBS pH 7.4 weiter gereinigt.

### B-4. Allgemeines Verfahren zur Kupplung an Cystein-Seitenketten

In die Kupplungsreaktionen wurden folgende Antikörper eingesetzt:
anti-C4.4a M31-B01
anti-C4.4a B01-3
anti-C4.4a B01-10
anti-C4.4a B01-7
anti-C4.4a D02-4
anti-C4.4a D02-6
anti-C4.4a D02-7

Zu einer Lösung des entsprechenden Antikörpers in PBS-Puffer im Konzentrationsbereich zwischen 1 mg/ml und 15 mg/ml wurden 3 Äquivalente Tris(2-carboxyethyl)phosphin-Hydro-chlorid (TCEP), gelöst in PBS-Puffer, gegeben und 1h bei RT gerührt. Anschließend wurden, je nach angestrebter Beladung, zwischen 2 und 10 Äquivalenten der zu kuppelnden Maleinimid-Vorläufer-Verbindung oder Halogenid-Vorläufer-Verbindung aus (Intermediate 102, 103, 105-109, 111-114, 117-126, 128, 129, 132-146, 148-155, 157, 159-161, 166, 171, 175-177, 184, 189, 194-195, 199-201, 205, 209, 223-224, 226, 228-231, 236 und 244) als Lösung in DMSO hinzugefügt. Dabei sollte die Menge an DMSO 10% des Gesamtvolumens nicht überschreiten. Der Ansatz wurde 60-120 min bei RT gerührt und anschließend über in PBS equillibierte PD 10-Säulen (Sephadex® G-25, GE Healthcare) gegeben und mit PBS-Puffer eluiert. Gegebenenfalls wurde noch eine Aufkonzentration mittels Ultrazentrifugation durchgeführt. Falls notwendig wurde zur besseren Abtrennung niedermlekularer Bestandteile die Aufkonzentration mittels Ultrafiltration nach Rückverdünnung mit PBS Puffer wiederholt.

Üblicherweise wurden, wenn nicht anders angegeben, 5 mg des entsprechenden Antikörpers in PBS Puffer zur Reduktion und der nachfolgenden Kupplung eingesetzt. Nach Aufreinigung über die PD10 Säule wurden so jeweils Lösungen des entsprechenden ADCs in 3.5 ml PBS-Puffer erhalten. Für diese Lösungen wurde dann die jeweils angegebene Proteinkonzentration bestimmt. Weiterhin wurde die Beladung des Antikörpers (Dmg/mAb Ratio) nach den unten beschriebenen Methoden ermittelt.

Nach diesem Verfahren wurden die in den Beispielen 1-3, 5-30, 32-36, 38-59, 61-66, 68-70, 80, 82-85, 87, 88, 92-95, 97, 98, 107, 109-114, 119 und 122 dargestellten Immunokonjugate hergestellt.

In den dargestellten Strukturformeln hat dabei AK_{1A}-AK_{1G} die Bedeutung
AK_{1A} = anti-C4.4a Antikörper M31-B01 (partiell reduziert)- S§¹
AK_{1B} = anti-C4.4a Antikörper B01-3 (partiell reduziert)-S§¹
AK_{1C} = anti-C4.4a Antikörper B01-10 (partiell reduziert)- S§¹
AK_{1D} = anti-C4.4a Antikörper B01-7 (partiell reduziert)-S§¹
AK_{1E} = anti-C4.4a Antikörper D02-4 (partiell reduziert)-S§¹
AK_{1F} = anti-C4.4a Antikörper D02-6 (partiell reduziert)-S§¹
AK_{1G} = anti-C4.4a Antikörper D02-7 (partiell reduziert)-S§¹
wobei
- §¹: die Verknüpfung mit der Succinimid-Gruppe bedeutet,
und
- S: für das Schwefelatom eines Cystein-Restes des partiell reduzierten Antikörpers steht.

### B-5. Allgemeines Verfahren zur Kupplung an Lysin-Seitenketten:

In die Kupplungsreaktionen wurden folgende Antikörper eingesetzt:
anti-C4.4a Antikörper M31-B01
anti-C4.4a Antikörper B01-3

Zu einer Lösung des entsprechenden Antikörpers in PBS-Puffer im Konzentrationsbereich zwischen 1 mg/ml und 15 mg/ml wurden, je nach angestrebter Beladung, zwischen 2 und 5 Äquivalente der zu kuppelnden Vorläufer-Verbindung (Intermediate 104, 110, 115, 116, 127, 130, 131, 147, 156, 158, 162, 169, 178, 185, 190, 202, 206, 210-216, 218, 219, 227, 233, 238, 240, 242, 245, 247a und 247b)) als Lösung in DMSO gegeben. Nach 30 min Rühren bei RT wurde nochmals die gleiche Menge an Vorläufer-Verbindung in DMSO hinzugefügt. Alternativ konnten auch 4 - 10 Äquivalente der zu kuppelnden Vorläufer-Verbindung in einem Schuß zugegeben werden. Dabei sollte die Menge an DMSO 10% des Gesamtvolumens nicht überschreiten. Nach weiteren 30 min Rühren bei RT wurde der Ansatz über in PBS equillibierte PD 10-Säulen (Sephadex® G-25, GE Healthcare) gegeben und mit PBS-Puffer eluiert. Gegebenenfalls wurde noch eine Aufkonzentration mittels Ultrazentrifugation durchgeführt. Falls notwendig wurde zur besseren Abtrennung niedermlekularer Bestandteile die Aufkonzentration mittels Ultrafiltration nach Rückverdünnung mit PBS Puffer wiederholt.

Üblicherweise wurden, wenn nicht anders angegeben, 5 mg des entsprechenden Antikörpers in PBS Puffer zur Kupplung eingesetzt. Nach Aufreinigung über die PD10 Säule wurden so jeweils Lösungen des entsprechenden ADCs in 3.5 ml PBS-Puffer erhalten. Für diese Lösungen wurde dann die jeweils angegebene Proteinkonzentration bestimmt sowie die Beladung des Antikörpers (Drug/mAb Ratio) nach den unten beschriebenen Methoden ermittelt.

Nach diesem Verfahren wurden die in den Beispielen 4, 31, 37, 60, 67, 81, 86, 89-91, 96, 99-106, 108, 118, 120, 121 und 123-125 dargestellten Immunokonjugate hergestellt.

In den dargestellten Strukturformeln hat dabei AK_{2A} und AK_{2B} die Bedeutung
AK_{2A} = anti-C4.4a Antikörper M31-B01-NH§²
AK_{2B} = anti-C4.4a Antikörper B01-3-NH§²
wobei
- §²: die Verknüpfung mit der Carbonylgruppe bedeutet,
und
- NH: für die Seitenketten-Aminogruppe eines Lysin-Restes des Antikörpers steht.

### B-6. Allgemeines Verfahren zur Herstellung von Cystein-Addukten:

10 µmol der oben beschriebenen Maleinimid-Vorläuferverbindungen wurden in 3 ml DMF aufgenommen und mit 2.1 mg (20 µmol) L-Cystein versetzt. Das Reaktionsgemisch wurde 2h bei RT gerührt, anschließend im Vakuum eingeengt und dann mittels präparativer IIPLC gereinigt.

In den dargestellten Strukturformeln hat dabei Cys die Bedeutung 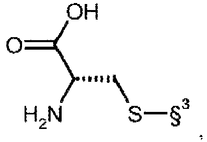 wobei
- §³: die Verknüpfung mit der Linker-Toxophor-Einheit bedeutet.

### Weitere Aufreinigung und Charakterisierung der erfindungsgemäßen Konjugate

Nach erfolgter Umsetzung wurde in einigen Fällen das Reaktionsgemisch beispielsweise durch Ultrafiltration aufkonzentriert und anschließend mittels Chromatographie, beispielsweise mittels einer Sephadex® G-25, entsalzt und gereinigt. Die Elution erfolgte beispielsweise mit Phosphatgepufferter Salzlösung (PBS). Anschließend wurde die Lösung sterilfiltriert und eingefroren. Alternativ kann das Konjugat lyophylisiert werden.

### B-7. Bestimmung der Toxophorbeladung

Von den erhaltenen Lösungen der in den Ausführungsbeispielen beschriebenen Konjugate in PBS Puffer wurde die Toxophorbeladung wie folgt bestimmt:
Die Bestimmung der Toxophor Beladung von Lysin-verknüpften ADCs erfolgte nach massenspektrometrischer Bestimmung der Molekulargewichte der einzelnen Konjugatspezies. Hierbei wurden vorab die Antikörperkonjugate mittels PNGaseF deglycosyliert, die Probe angesäuert und nach HPLC-Trennung massenspektrometrisch mittels ESI-MicroTofQ (Brukcr Daltonik) analysiert. Alle Spektren über das Signal im TIC (Total Ion Chromatogramm) wurden addiert und das Molekulargewicht der verschiedenen Konjugatspezies auf Basis von MaxEnt Deconvolution kalkuliert. Nach Signal Integration der verschiedenen Spezies wurde dann die DAR (= Drug/Antibody Ratio) berechnet.

Zur Protein Identifizierung wurde neben der Molekulargewichtsbestimmung nach Deglykosilierung und/oder Denaturierung ein tryptischer Verdau durchgeführt, der nach Denaturierung, Reduktion und Derivatisierung die Identität des Proteins anhand der nachgewiesenen tryptischen Peptide bestätigte.

Die Bestimmung der Toxophorbeladung von Cystein-verknüpften Konjugaten wurde über Reversed-Phase-Chromatographie des reduzierten und denaturierten ADCs bestimmt. Zur ADC-Lösung (1mg/mL, 50µL) wurde Guanidinium-Hydrochlorid (GuHCl) (28.6 mg) und eine Lösung von DL-Dithiothreitol (DTT) (500mM, 3 µL) gegeben. Die Mischung wurde für eine Stunde bei 55 °C inkubiert und über HPLC analysiert.

Die HPLC-Analyse wurde auf einem Agilent 1260 HPLC-System mit Detektion bei 220 nm durchgeführt. Es wurde eine Polymer Laboratories PLRP-S Polymerie Reversed Phase Säule (Katalognummer PL1912-3802) (2.1x150 mm, 8 µm particle size, 1000 Å) bei einer Flussrate von 1 mL/min mit folgendem Gradienten verwendet: 0 min, 25 %B; 3 min, 25 %B; 28 min, 50 %B. Laufmittel A bestand aus 0.05 % Trifluoressigsäure (TFA) in Wasser, Laufmittel B aus 0.05 % Trifluoressigsäure in Acetonitril.

Die detektierten Peaks wurden durch Retentionszeitvergleich mit der leichten Kette (L0) und der schweren Kette (H0) des nicht konjugierten Antikörpers zugeordnet. Peaks, die ausschließlich in der konjugierten Probe detektiert wurden, wurden der leichten Kette mit einem Toxophor (L1) und den schweren Ketten mit einem, zwei und drei Toxophoren (H1, H2, H3) zugeordnet.

Die durchschnittliche Beladung des Antikörpers mit Toxophoren wurde folgendermaßen berechnet; Zunächst wurde die Leichte-Kette-Beladung aus den durch Integration bestimmten Peakflächen der zu den leichten Ketten gehörenden Peaks L0 und L1 als die Summe der Toxophor-Anzahl gewichteten Integrationsergebnisse von L0 und L1 geteilt durch die Summe der einfach gewichteten Integrationsergebnisse von L0 und L1 berechnet. Genauso wurde die Schwere-Kette-Beladung aus den durch Integration bestimmten Peakflächen der zu den schweren Ketten gehörenden Peaks H0,H1,H2 und H3 als die Summe der Toxophor-Anzahl gewichteten Integrationsergebnisse von H0,H1,H2 und H3 geteilt durch die Summe der einfach gewichteten Integrationsergebnisse von H0,H1,H2 und H3 berechnet. Die DAR ergibt sich aus der Leichte-Kette-Beladung und der Schwere-Kette-Beladung als die zweifache Summe aus Leichte-Kette-Beladung und Schwere-Kette-Beladung. Der Faktor 2 berücksichtigt, dass ein Antikörper aus je zwei leichten und zwei schweren Ketten besteht. In vereinzelten Fällen kann es vorkommen, dass die Toxophorbeladung aufgrund von Ko-Elutionen einiger Peaks nicht exakt möglich ist.

### B-8. Überprüfung der Antigen-Bindung des ADCs

Die Bindefähigkeit des Binders an das Zielmolekül wurde nach erfolgter Kopplung überprüft. Dazu sind dem Fachmann vielfältige Methoden bekannt, beispielsweise kann die Affinität des Konjugats mittels ELISA Technologie oder Oberflächenplasmonresonanzanalyse (BIAcore™ Messungen) überprüft werden. Die Konjugatkonzentration kann der Fachmann mit gängigen Methoden messen, beispielsweise für Antikörper-Konjugate mittels Proteinbestimmung. (siehe auch Doronina et aL; Nature Biotechnol. 2003; 21:778-784 und Polson et al., Blood 2007; 1102:616-623).

### Ansführungsbeispiele Immunkonjugate

### Beispiel 1

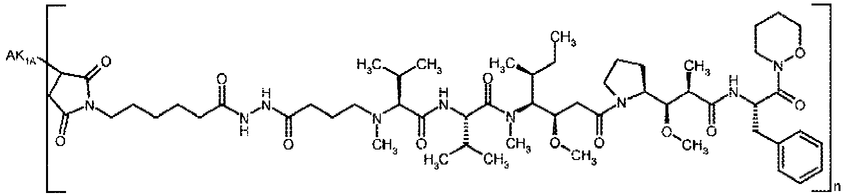

Eingesetzt wurden hier zur Kupplung 70 mg anti-C4.4a M31-B01 in DPBS pH 7.4 und der Ansatz wurde nach der Sephadex-Reinigung durch Ultrazentrifugation aufkonzentriert.
Protein Konzentration: 12.2 mg/ml
Drug/mAb Ratio: 1.5

### Beispiel 2

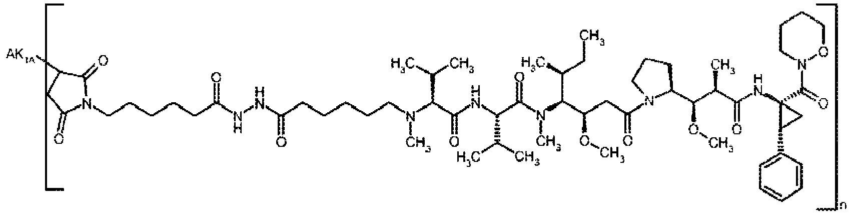

Protein Konzentration: 0.87 mg/ml
Drug/mAb Ratio: 5.8

### Beispiel 3

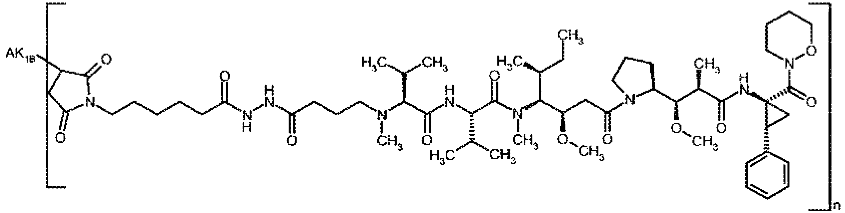

Protein Konzentration: 1.16 mg/ml
Drug/mAb Ratio: 3.1

### Beispiel 4

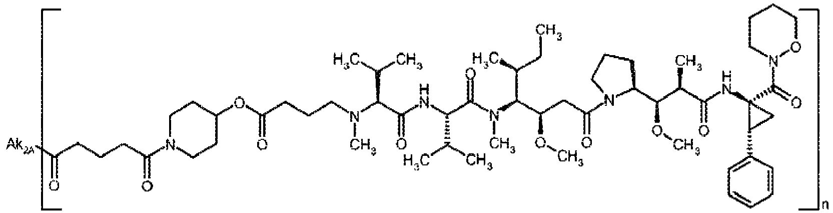

Protein Konzentration: 1.24 mg/ml
Drug/mAb Ratio: 1.6

### Beispiel 5

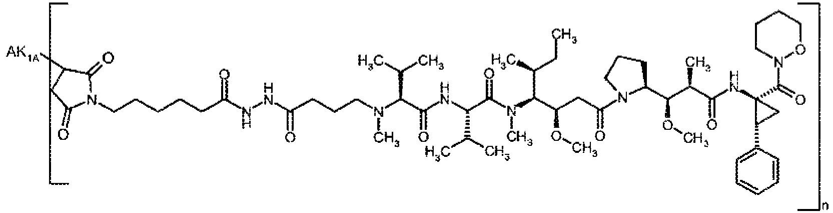

Protein Konzentration: 0.88 mg/ml
Drug/mAb Ratio: 6.9

### Beispiel 6

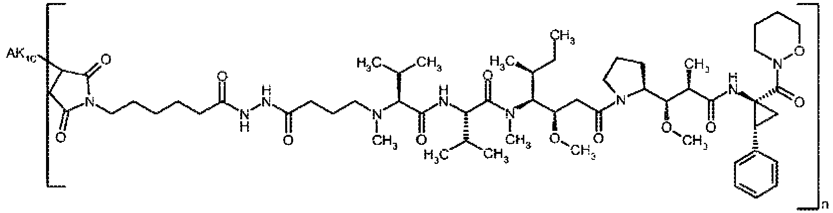

Protein Konzentration: 1.2 mg/ml
Drug/mAb Ratio: 2.8

### Beispiel 7

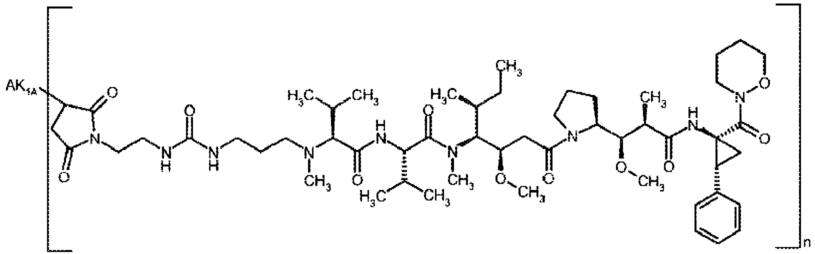

Protein Konzentration: 0.9 mg/ml
Drug/mAb Ratio: 3.9

### Beispiel 8

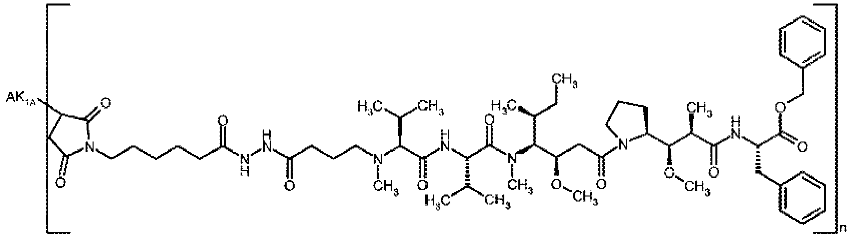

Protein Konzentration: 0.52 mg/ml
Drug/mAb Ratio: 1.6

### Beispiel 9

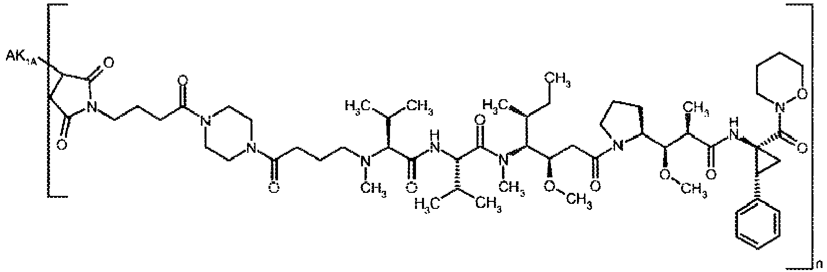

Protein Konzentration: 0.47 mg/ml
Drug/mAb Ratio: 6.6

### Beispiel 10

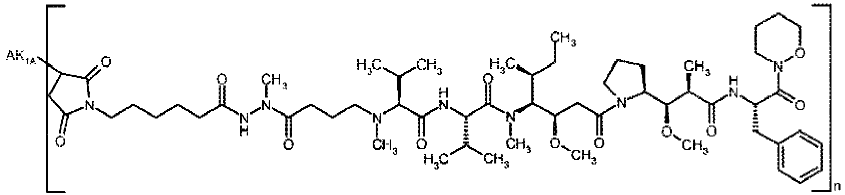

Protein Konzentration: 0.77 mg/ml
Drug/mAb Ratio: 6.9

### Beispiel 11

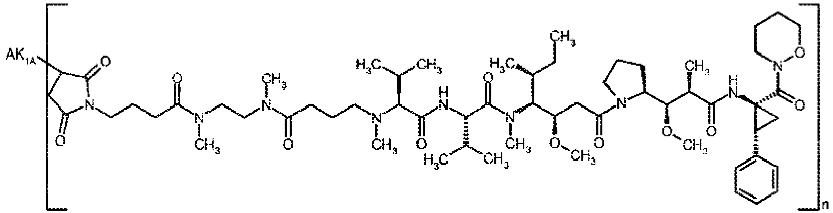

Protein Konzentration: 0.47 mg/ml
Drug/mAb Ratio: 4.0

### Beispiel 12

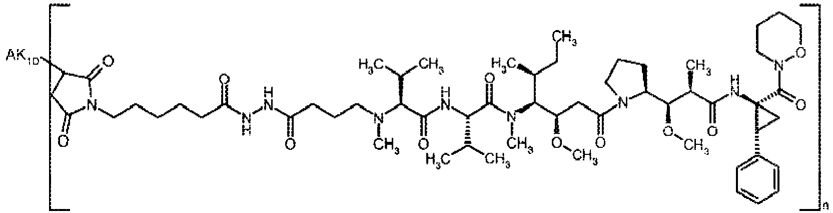

Protein Konzentration: 1.46 mg/ml
Drug/mAb Ratio: 2.5

### Beispiel 13

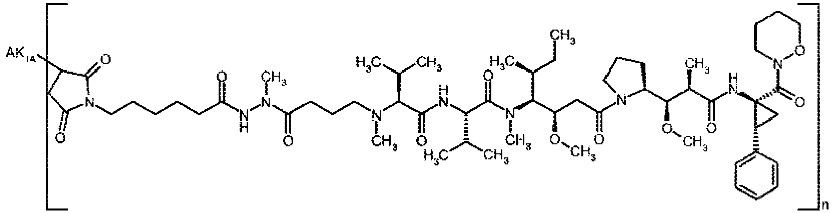

Protein Konzentration: 0.45 mg/ml
Drug/mAb Ratio: 3.3

### Beispiel 14

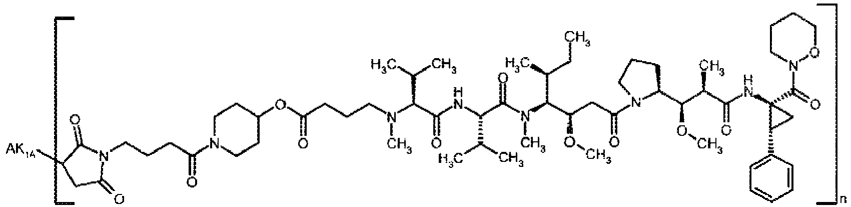

Protein Konzentration: 0.98 mg/ml
Drug/mAb Ratio: 3.6

### Beispiel 15

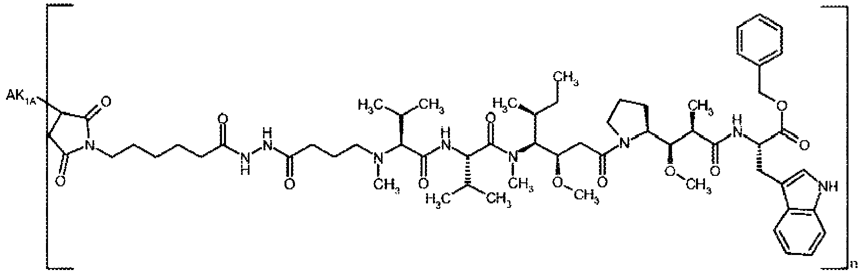

Eingesetzt wurden hier zur Kupplung 70 mg anti-C4.4a M31-B01 in DPBS pH 7,4 und der Ansatz wurde nach der Sephadex-Reinigung durch Ultrazentrifugation aufkonzentriert.
Protein Konzentration: 9.42 mg/ml
Drug/mAb Ratio: 4.1

### Beispiel 16

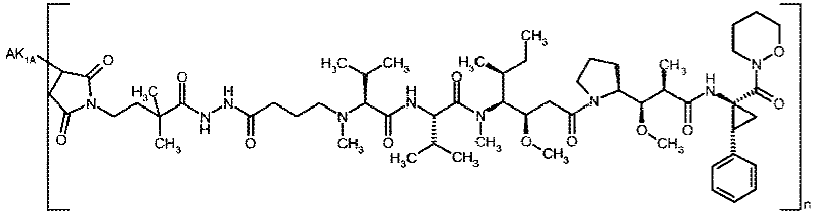

Protein Konzentration: 0.65 mg/ml
Drug/mAb Ratio: 1.8

### Beispiel 17

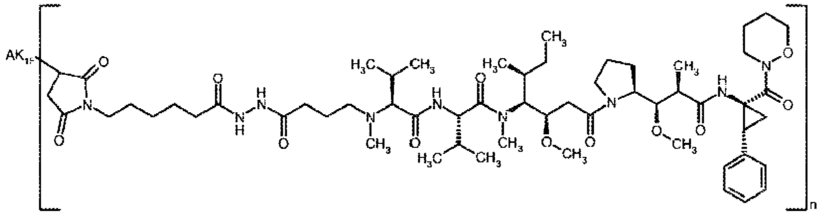

Protein Konzentration: 1.07 mg/ml
Drug/mAb Ratio: nicht bestimmbar

### Beispiel 18

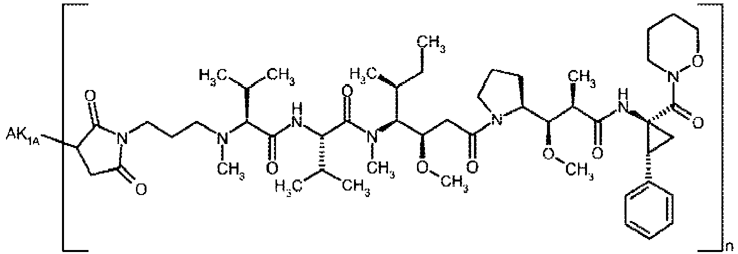

Protein Konzentration: 0.47 mg/ml
Drug/mAb Ratio: 4.4

### Beispiel 19

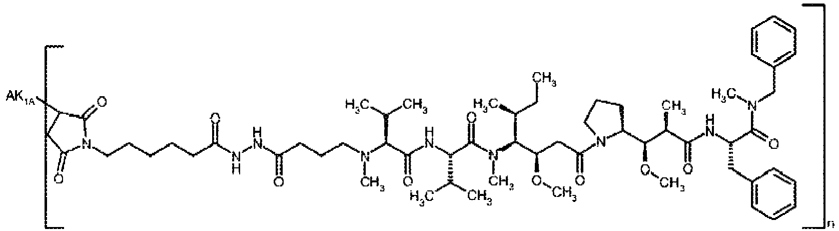

Protein Konzentration: 0.43 mg/ml
Drug/mAb Ratio: 4.8

### Beispiel 20

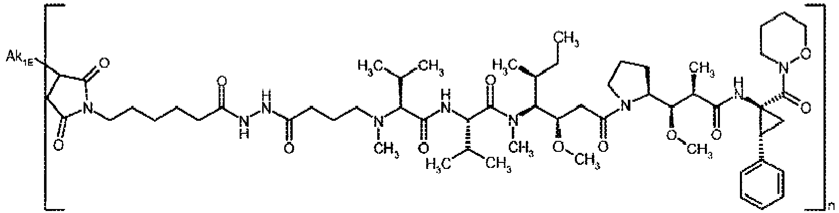

Protein Konzentration: 1.01 mg/ml
Drug/mAb Ratio: 2.6

### Beispiel 21

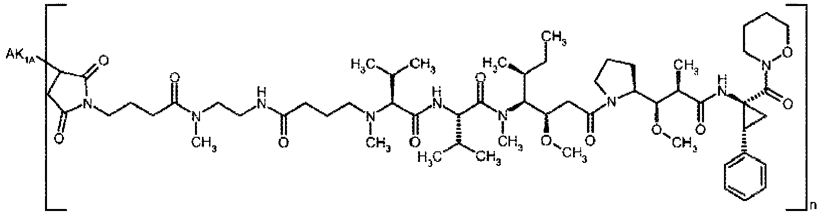

Protein Konzentration: 0.53 mg/ml
Drug/mAb Ratio: 0.6

### Beispiel 22

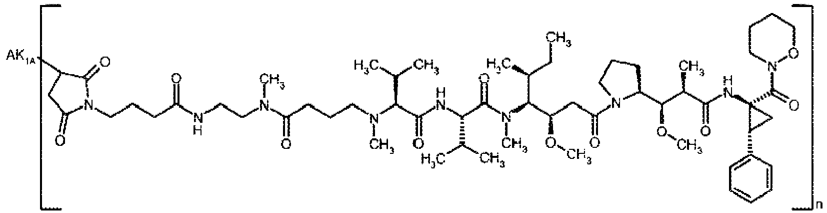

Protein Konzentration: 0.55 mg/ml
Drug/mAb Ratio: 1.3

### Beispiel 23

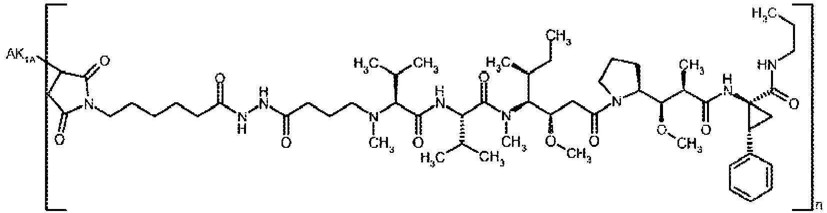

Protein Konzentration: 0.65 mg/ml
Drug/mAb Ratio: 1.1

### Beispiel 24

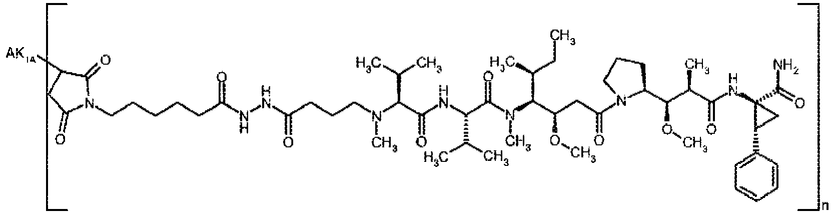

Protein Konzentration: 1.04
Drug/mAb Ratio: 3.5

### Beispiel 25

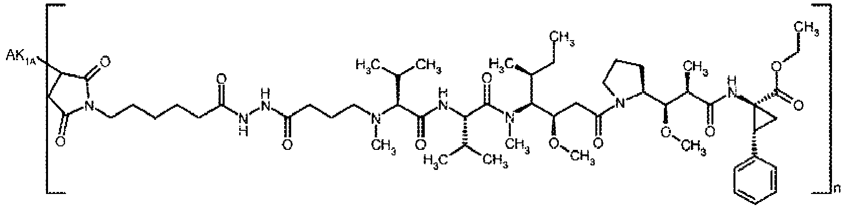

Protein Konzentration: 0.62 mg/ml
Drug/mAb Ratio: 2.4

### Beispiel 26

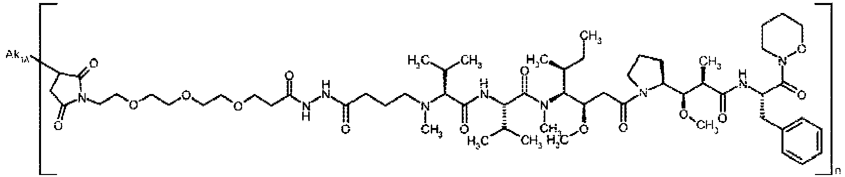

Eingesetzt wurden hier zur Kupplung 90 mg anti-C4.4a M31-B01 in DPBS pH 7,4 und der Ansatz wurde nach der Sephadex-Reinigung durch Ultrazentrifugation aufkonzentriert.
Proteinkonzentration: 11.2 mg/ml
Drug/mAb-Ratio: 2.3

### Beispiel 27

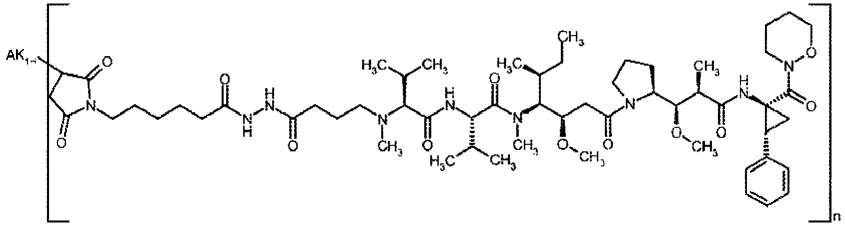

Proteinkonzentration: 1.11 mg/ml
Drug/mAb-Ratio: 2.4

### Beispiel 28

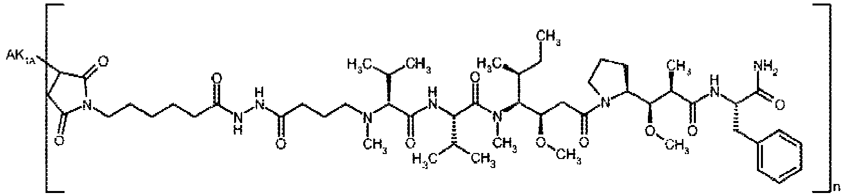

Eingesetzt wurden hier zur Kupplung 70 mg anti-C4.4a M31-B01 in DPBS pH 7,4 und der Ansatz wurde nach der Sephadex-Reinigung durch Ultrazentrifugation aufkonzentriert.
Protein Konzentration: 10.7 mg/ml
Drug/mAb Ratio: 2.2

### Beispiel 29

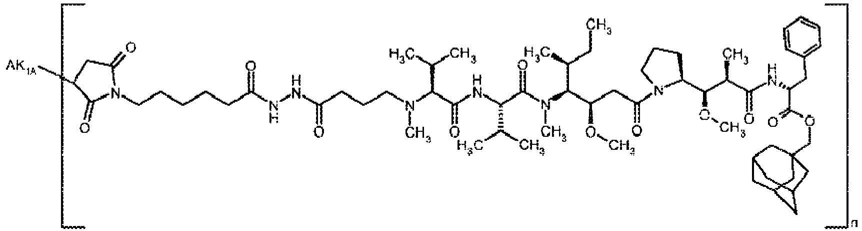

Protein Konzentration: 0.87 mg/ml
Drug/mAb Ratio: 1.8

### Beispiel 30

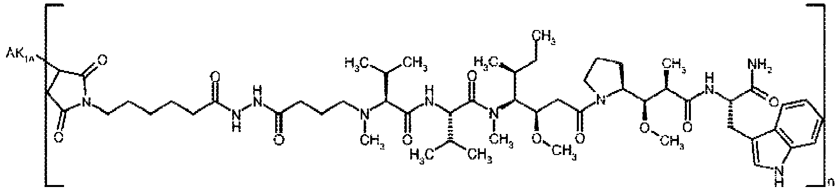

Protein Konzentration: 1.3 mg/ml
Drug/mAb Ratio: 2.1

### Beispiel 31

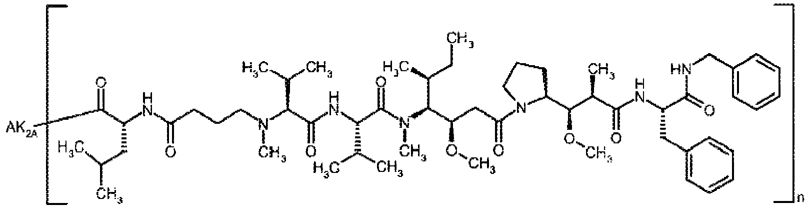

Protein Konzentration: 1.3 mg/ml
Drug/mAb Ratio: 0.3

### Beispiel 32

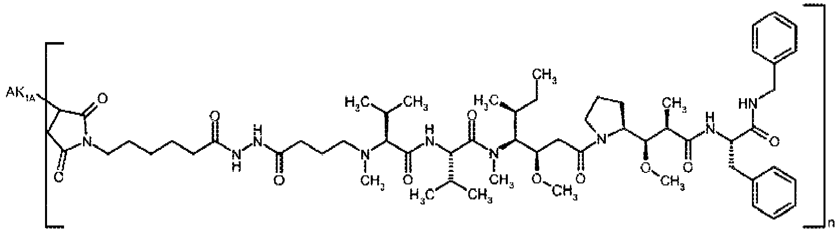

Eingesetzt wurden hier zur Kupplung 70 mg anti-C4.4a M31-B01 in DPBS pH 7,4 und der Ansatz wurde nach der Sephadex-Reinigung durch Ultrazentrifugation aufkonzentriert.
Protein Konzentration: 12.0 mg/ml
Drug/mAb Ratio: 3.2

### Beispiel 33

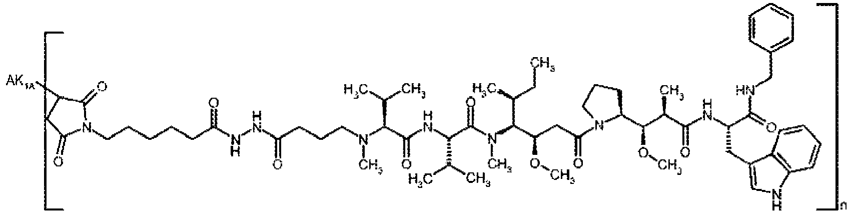

Eingesetzt wurden hier zur Kupplung 90 mg anti-C4.4a M31-B01 in DPBS pH 7,4 und der Ansatz wurde nach der Sephadex-Reinigung durch Ultrazentrifugation aufkonzentriert.
Protein Konzentration: 10.2 mg/ml
Drug/mAb Ratio: 4.3

### Beispiel 34

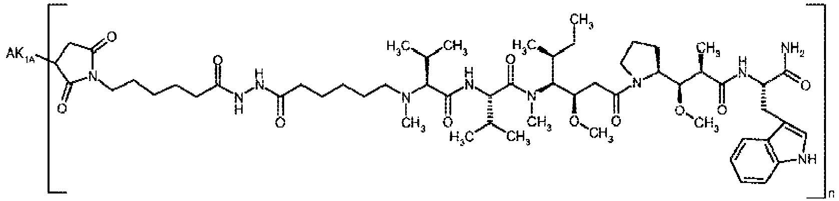

Protein Konzentration: 1.37 mg/ml
Drug/mAb Ratio: 2.6

### Beispiel 35

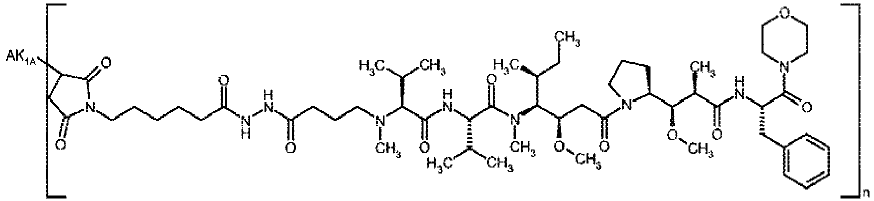

Protein Konzentration: 1.14 mg/ml
Drug/mAb Ratio: 2.0

### Beispiel 36

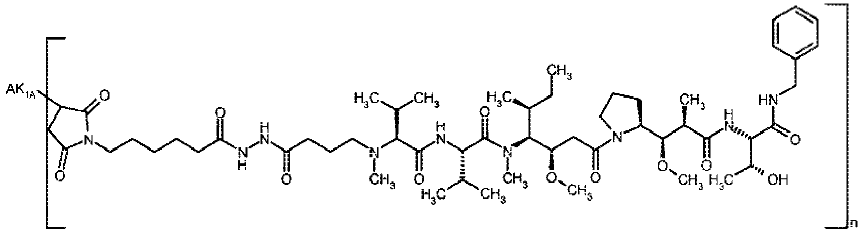

Protein Konzentration: 1.07 mg/ml
Drug/mAb Ratio: 3.5

### Beispiel 37

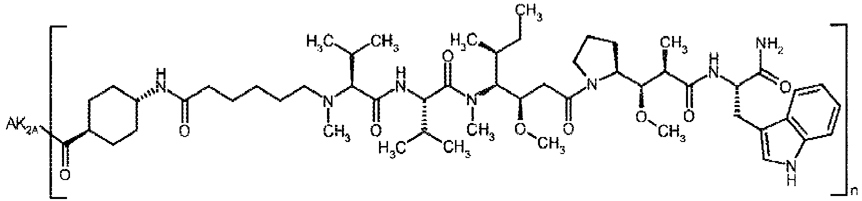

Protein Konzentration: 1.14 mg/ml
Drug/mAb Ratio: 1.9

### Beispiel 38

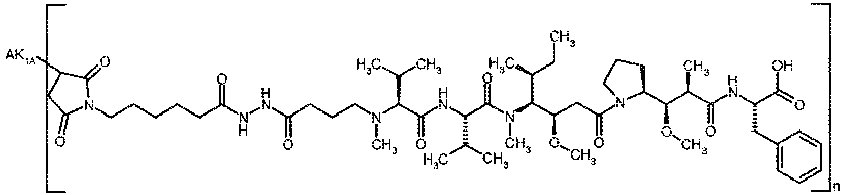

Protein Konzentration: 1.22 mg/ml
Drug/mAb Ratio: 3.3

### Beispiel 39

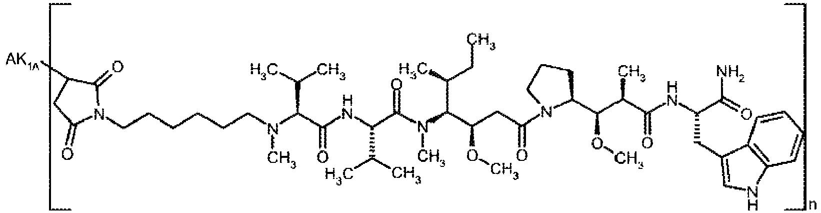

Protein Konzentration: 1.3 mg/ml
Drug/mAb Ratio: 3.2

### Beispiel 40

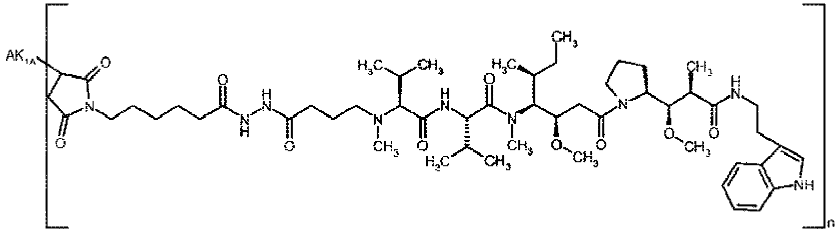

Protein Konzentration: 1.23 mg/ml
Drug/mAb Ratio: 3.3

### Beispiel 41

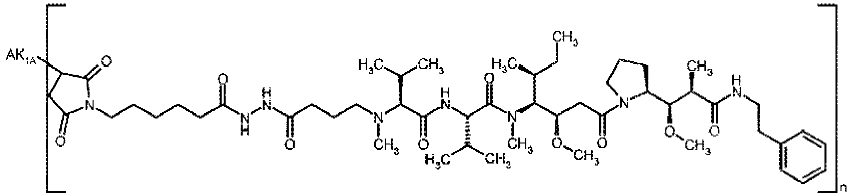

Protein Konzentration: 1.64 mg/ml
Drug/mAb Ratio: 1.8

### Beispiel 42

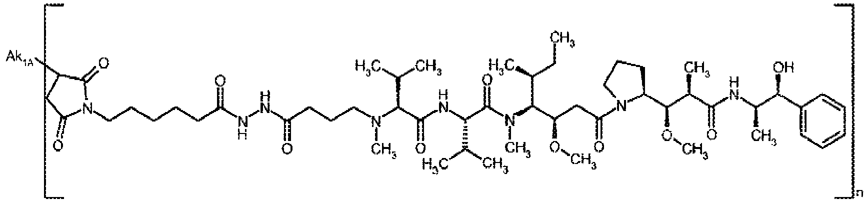

Protein Konzentration: 1.07 mg/ml
Drug/mAb Ratio: 3.1

### Beispiel 43

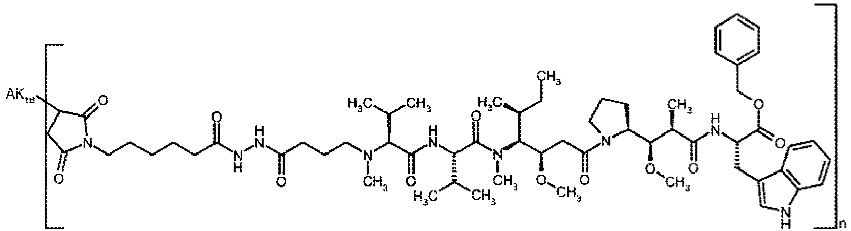

Protein Konzentration: 1.14 mg/ml
Drug/mAb Ratio: 2.3

### Beispiel 44

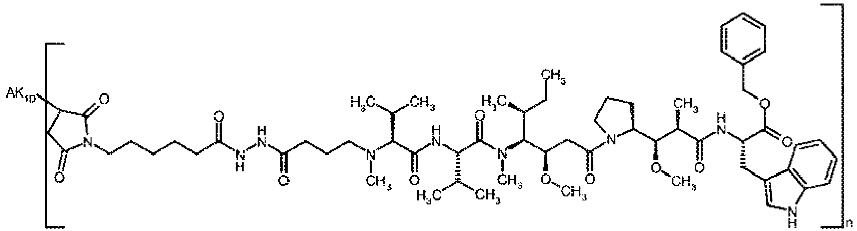

Protein Konzentration: 1.23 mg/ml
Drug/mAb Ratio: 3.4

### Beispiel 45

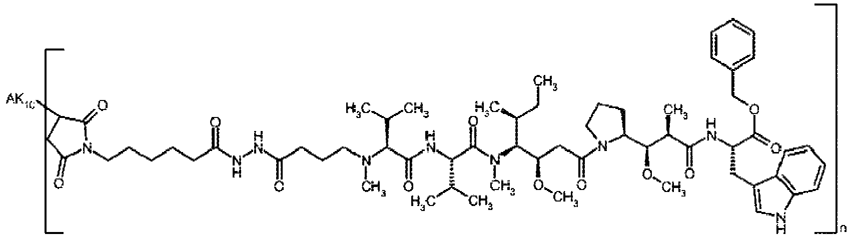

Protein Konzentration: 1.22 mg/ml
Drug/mAb Ratio: 2.5

### Beispiel 46

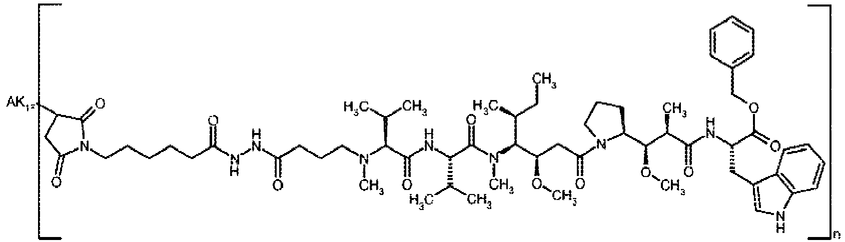

Protein Konzentration: 1.22 mg/ml
Drug/mAb Ratio: 2.4

### Beispiel 47

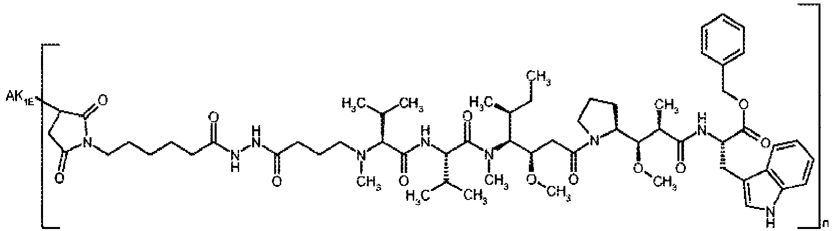

Protein Konzentration: 1.32 mg/ml
Drug/mAb Ratio: nicht bestimmbar

### Beispiel 48

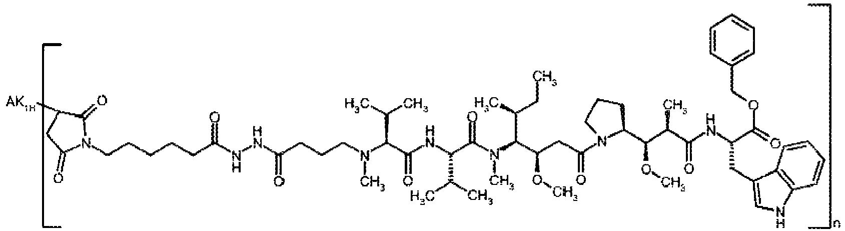

Protein Konzentration: 1.44 mg/ml
Drug/mAb Ratio: 2.3

### Beispiel 49

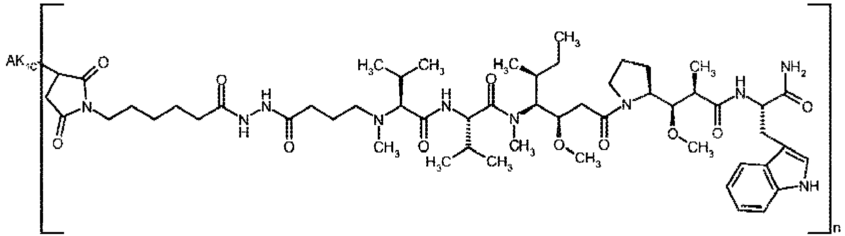

Eingesetzt wurden hier zur Kupplung 250 mg anti-C4.4a B01-10 in DPBS pH 7,4 und der Ansatz wurde nach der Sephadex-Reinigung durch Ultrazentrifugation aufkonzentriert.
Protein Konzentration: 12.8 mg/ml
Drug/mAb Ratio: 5.2

### Beispiel 50

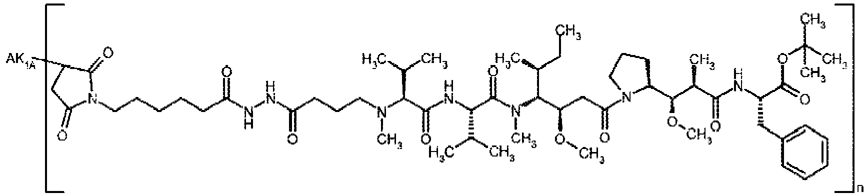

Protein Konzentration: 0.9 mg/ml
Drug/mAb Ratio: 2

### Beispiel 51

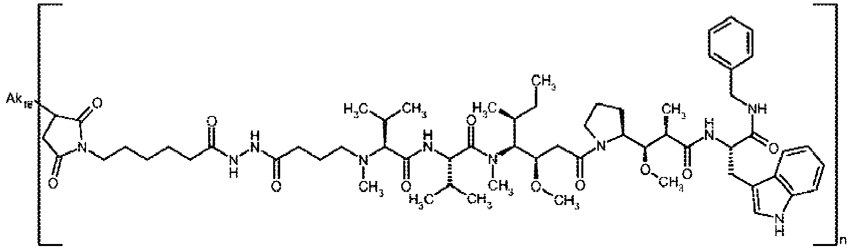

Eingesetzt wurden hier zur Kupplung 250 mg anti-C4.4a B01-3 in DPBS PH 7,4 und der Ansatz wurde nach der Sephadex-Reinigung durch Ultrazentrifugation aufkonzentriert.
Protein Konzentration: 8.0 mg/ml
Drug/mAb Ratio: 4.5

### Beispiel 52

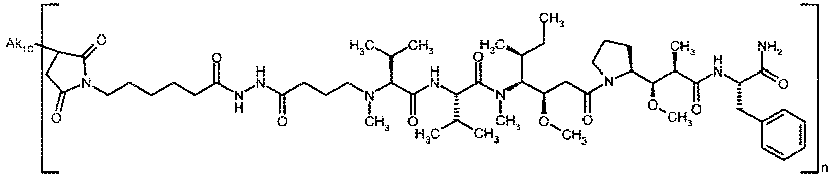

Eingesetzt wurden hier zur Kupplung 250 mg anti-C4.4a B01-10 in DPBS pH 7,4 und der Ansatz wurde nach der Sephadex-Reinigung durch Ultrazentrifugation aufkonzentriert.
Protein Konzentration: 12.3 mg/ml
Drug/mAb Ratio: 5.2

### Beispiel 53

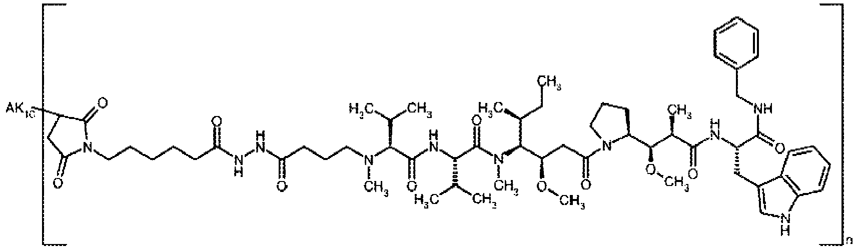

Eingesetzt wurden hier zur Kupplung 250 mg anti-C4.4a B01-10 in DPBS pH 7,4 und der Ansatz wurde nach der Sephadex-Reinigung durch Ultrazentrifugation aufkonzentriert.
Protein Konzentration: 10.2 mg/ml
Drug/mAb Ratio: 4.4

### Beispiel 54

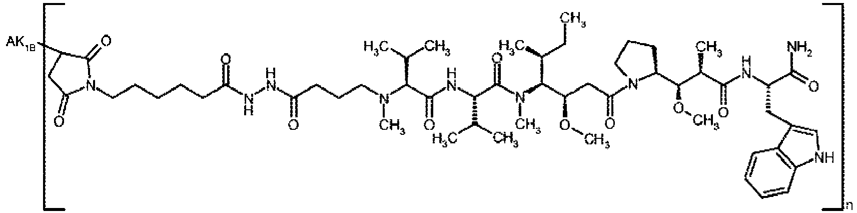

Eingesetzt wurden hier zur Kupplung 50 mg anti-C4.4a B01-3 in DPBS pH 7,4 und der Ansatz wurde nach der Sephadex-Reinigung durch Ultrazentrifugation aufkonzentriert.
Protein Konzentration: 11.5 mg/ml
Drug/mAb Ratio: 5.2

### Beispiel 55

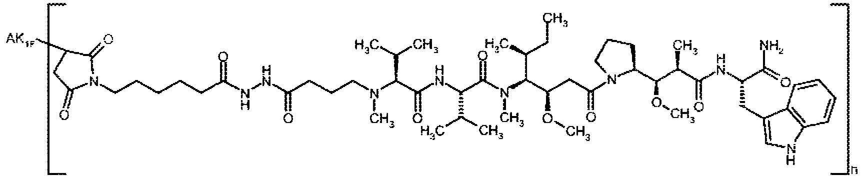

Eingesetzt wurden hier zur Kupplung 250 mg anti-C4.4a D02-6 in DPBS pH 7,4 und der Ansatz wurde nach der Sephadex-Reinigung durch Ultrazentrifugation aufkonzentriert.
Protein Konzentration: 13 mg/ml
Drug/mAb Ratio: 5.2

### Beispiel 56

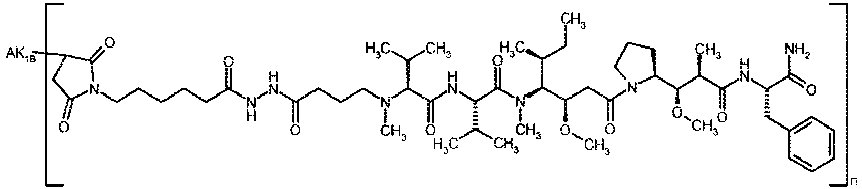

Eingeseitzt wurden hier zur Kupplung 250 mg anti-C4.4a B01-3 in DPBS pH 7,4 und der Ansatz wurde nach der Sephadex-Reinigung durch Ultrazentrifugation aufkonzentriert.
Protein Konzentration: 10.3 mg/ml
Drug/mAb Ratio: 4.9

### Beispiel 57

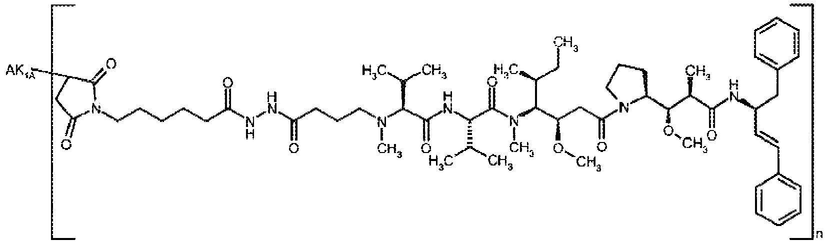

Protein Konzentration: 0.88 mg/ml
Drug/mAb Ratio: 3.2

### Beispiel 58

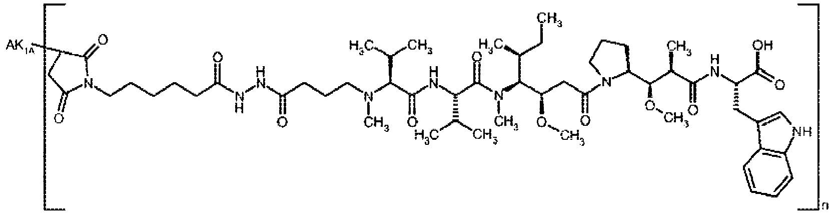

Protein Konzentration: 1.18 mg/ml
Drug/mAb Ratio: 3.4

### Beispiel 59

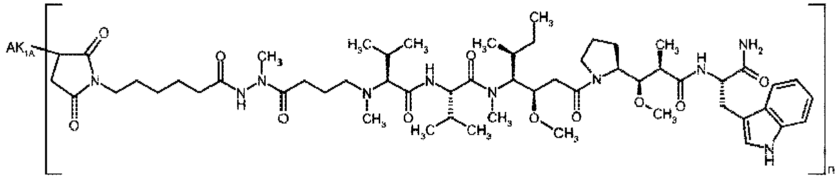

Protein Konzentration: 1.23 mg/ml
Drug/mAb Ratio: 3.0

### Beispiel 60

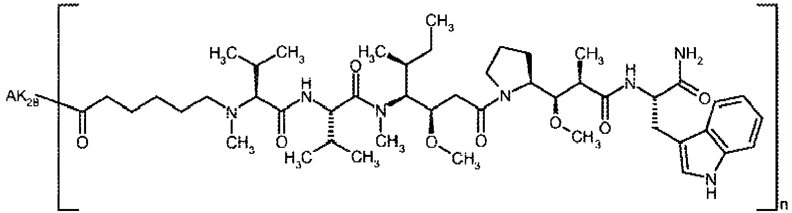

Protein Konzentration: 1.3 mg/ml
Drug/mAb Ratio: 3.3

### Beispiel 61

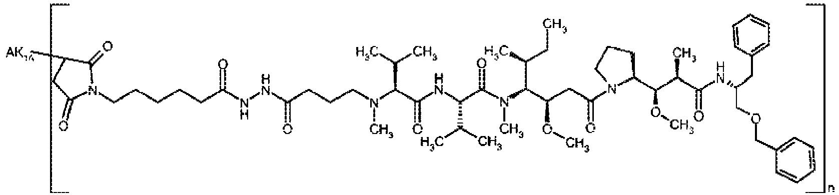

Protein Konzentration: 1.11 mg/ml
Drug/mAb Ratio: nicht bestimmbar

### Beispiel 62

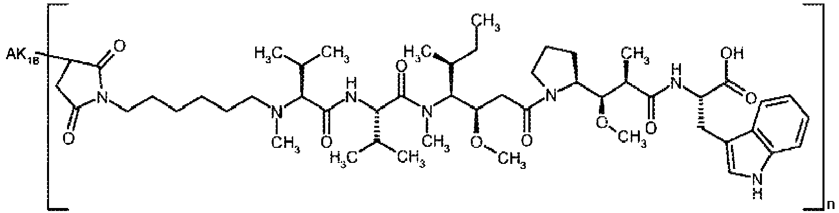

Protein Konzentration: 1.25 mg/ml
Drug/mAb Ratio: 2.4

### Beispiel 63

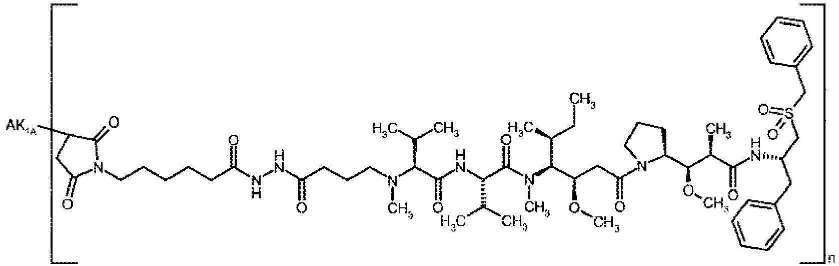

Protein Konzentration: 0.88 mg/ml
Drug/mAb Ratio: 5.0

### Beispiel 64

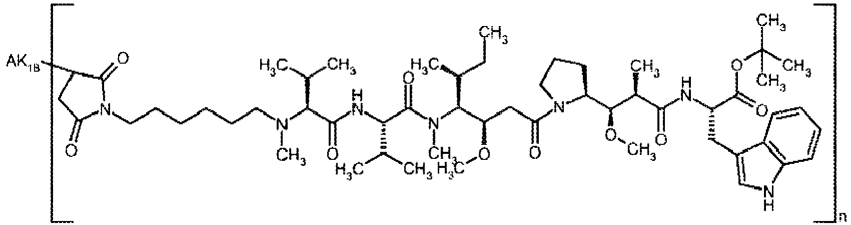

Protein Konzentration: 1.23 mg/ml
Drug/mAb Ratio: 3.3

### Beispiel 65

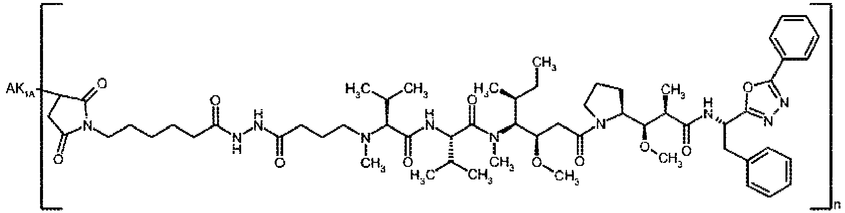

Protein Konzentration: 0.93 mg/ml
Drug/mAb Ratio: 1.8

### Beispiel 66

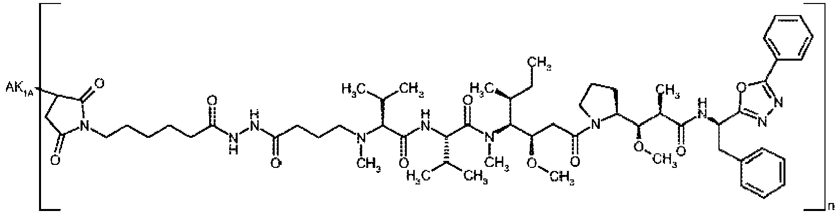

Protein Konzentration: 0.85 mg/ml
Drug/mAb Ratio: 5.3

### Beispiel 67

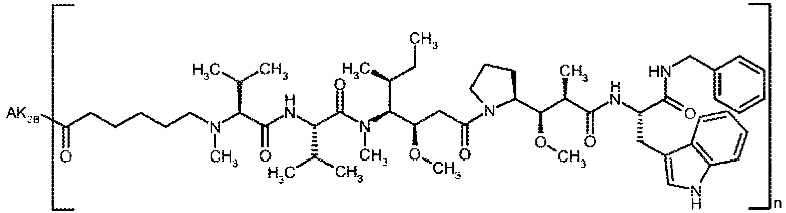

Protein Konzentration: 1.51 mg/ml
Drug/mAb Ratio: 1.4

### Beispiel 68

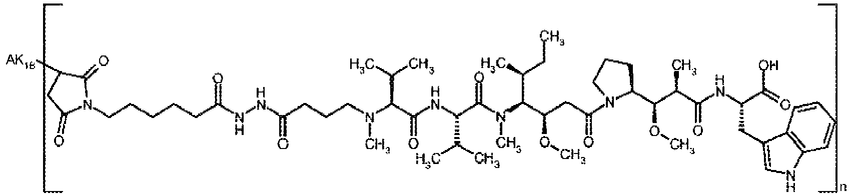

Eingesetzt wurden hier zur Kupplung 150 mg anti-C4.4a B01-3 in DPBS PH 7,4 und der Ansatz wurde nach der Sephadex-Reinigung durch Ultrazentrifugation aufkonzentriert.
Protein Konzentration: 11.0 mg/ml
Drug/mAb Ratio: 4.5

### Beispiel 69

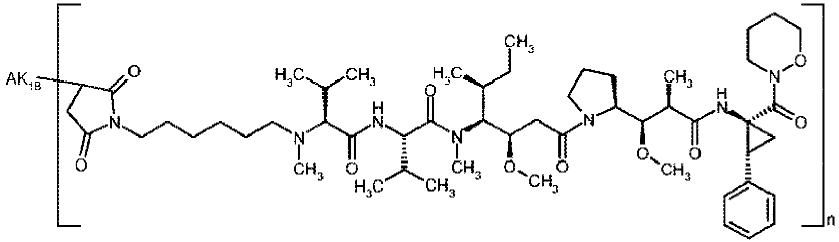

Protein Konzentration: 1.2 mg/ml
Drug/mAb Ratio: 3.3

### Beispiel 70

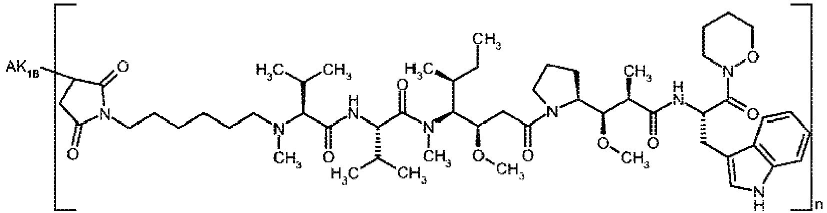

Protein Konzentration: 1.25 mg/ml
Drug/mAb Ratio: 3.1

### Beispiel 71

### N-(4-{2-[6-(3-{[(2R)-2-Amino-2-carboxyethyl]sulfanyl}-2,5-dioxopyrrolidin-1-yl)hexanoyl] hydrazino}-4-oxobutyl)-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

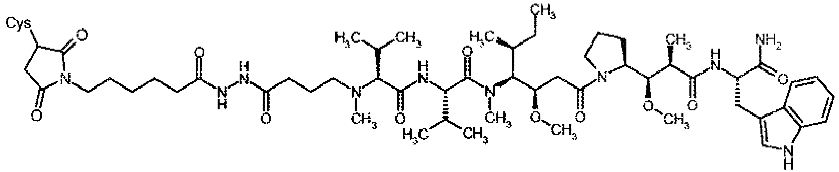

10 mg (10µmol) von Intermediat 157 wurden in 5.2 ml DMF aufgenommen und mit 2.28 mg (20 µmol) L-Cystein versetzt.Das Reaktionsgemisch wurde 2 h bei RT gerührt, anschließend im Vakuum eingeengt und dann mittels präparativer HPLC gereinigt. Es wurden 5.8 mg (48% d.Th. der Titelverbindung erhalten.
HPLC (Methode 5): Rₜ = 1.45 min;
LC-MS (Methode 1): Rₜ = 0.74 min; MS (ESIpos): m/z = 1184 (M+H)⁺.

### Beispiel 72

### N-(4-{2-[6-(3-{[(2R)-2-Amino-2-carboxyethyl]sulfanyl}-2,5-dioxopyrrolidin-1-yl)hexanoyl]hydrazino}-4-oxobutyl)-N-methyl-L-valyl-N-[(3R,4S,5S)-1-{(2S}-2-[(1R,2R)-3-{[(1S)-1-carboxy-2-(1H-indol-3-yl)ethyl]amino}-1-methoxy-2-methyl-3-oxopropyl]pyrrolidin-1-yl}-3-methoxy-5-methyl-1-oxoheptan-4-yl]-N-methyl-L-valinamid

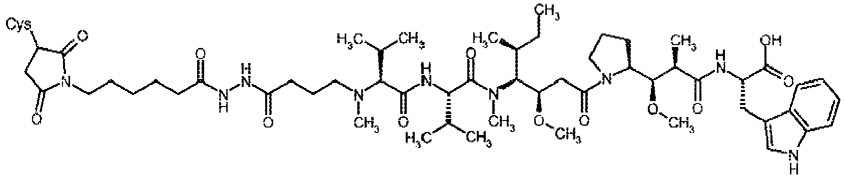

10 mg (10µmol) von Intermediat 113 wurden in 5.2 ml DMF aufgenommen und mit 2.28 mg (20 µmol) L-Cystein versetzt. Das Reaktionsgemisch wurde 2 h bei RT gerührt, anschließend im Vakuum eingeengt und dann mittels präparativer HPLC gereinigt. Es wurden 6 mg (54% d.Th. der Titelverbindung erhalten.
HPLC (Methode 5): Rₜ = 1.5 min;
LC-MS (Methode 1): Rₜ = 0.77 min; MS (ESIpos): m/z = 1185 (M+H)⁺.

### Beispiel 73

### N-(4-{2-[6-(3-{[(2R)-2-Amino-2-carboxyethyl]sulfanyl}-2,5-dioxopyrrolidin-1-yl)hexanoyl] hydrazino}-4-oxobutyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

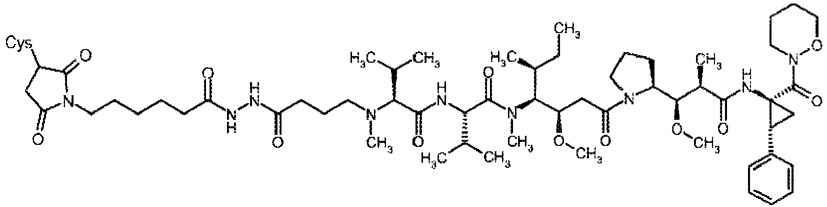

9 mg (8.3 µmol) von Intermediat 132 wurden in 4 ml DMF aufgenommen und mit 3 mg (24.4 µmol) L-Cystein versetzt. Das Reaktionsgemisch wurde über Nacht bei RT gerührt, anschließend im Vakuum eingeengt und dann mittels präparativer HPLC gereinigt. Es wurden 6.8 mg (68% d.Th. der Titelverbindung erhalten.
HPLC (Methode 12): Rₜ = 1.8 min:
LC-MS (Methode 1): Rₜ = 0.78 min; MS (ESIpos): m/z = 1227 (M+H)⁺.

### Beispiel 74

### N-[6-(3-{[(2R)-2-Amino-2-carboxyethyl]sulfanyl}-2,5-dioxopyrrolidin-1-yl)hexyl]-N-methyl-L-valyl-N-[(3R,4S,5S)-1-{(2S)-2-[(1R,2R)-3-{[(2S)-1-amino-3-(1H-indol-3-yl)-1-oxopropan-2-yl] amino}-1-methoxy-2-methyl-3-oxopropyl]pyrrolidin-1-yl}-3-methoxy-5-methyl-1-oxoheptan-4-yl]-N-methyl-L-valinamid

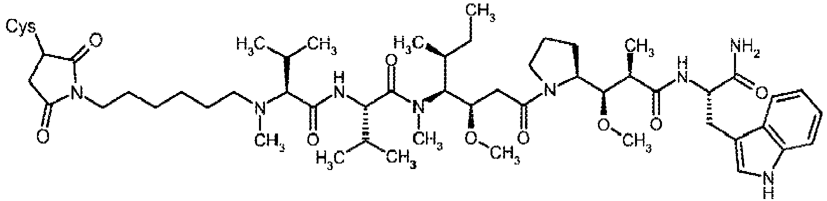

10 mg (10 µmol) von Intermediat 106 wurden in 5.8 ml DMF aufgenommen und mit 2.5 mg (20 µmοl) L-Cystein versetzt. Das Reaktionsgemisch wurde 2h bei RT gerührt, anschließend im Vakuum eingeengt und dann mittels präparativer IIPLC gereinigt. Es wurden 5.2 mg (46% d.Th. der Titelverbindung erhalten.
HPLC (Methode 5): Rₜ = 1.5 min;
LC-MS (Methode 11): Rₜ = 0.71 min; MS (ESIpos): m/z = 1070 (M+H)⁺.

### Beispiel 75

### N-[6-(3-{[(2R)-2-Amino-2-carboxyethyl]sulfanyl}-2,5-dioxopyrrolidin-1-yl)hexyl]-N-methyl-L-valyl-N-[(3R,4S,5S)-1-{(2S)-2-[(1R,2R)-3-{[(1S)-1-carboxy-2-(1H-indol-3-yl)ethyl]amino}-1-methoxy-2-methyl-3-oxopropyl]pyrrolidin-1-yl}-3-methoxy-5-methyl-1-oxoheptan-4-yl]-N-methyl-L-valinamid

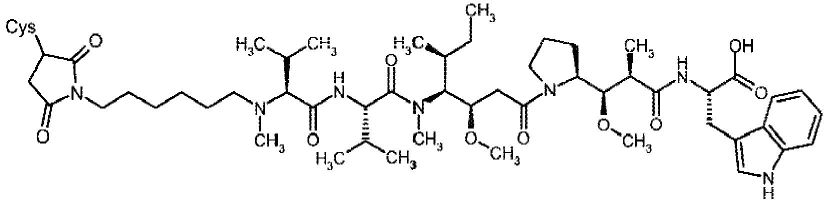

10 mg (10 µmol) von Intermediat 124 wurden in 4 ml DMF aufgenommen und mit 2.5 mg (20 µmol) L-Cystein versetzt. Das Reaktionsgemisch wurde 2h bei RT gerührt, anschließend im Vakuum eingeengt und dann mittels präparativer HPLC gereinigt. Es wurden 7.2 mg (64% d.Th. der Titelverbindung erhalten.
HPLC (Methode 5): Rₜ = 1.6 min;
LC-MS (Methode 1): Rₜ = 0.8 min; MS (ESIpos): m/z = 1071 (M+H)⁺.

### Beispiel 76

### N-[6-(3-{[(2R)-2-Amino-2-carboxyethyl]sulfanyl}-2,5-dioxopyrrolidin-1-yl)hexyl]-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

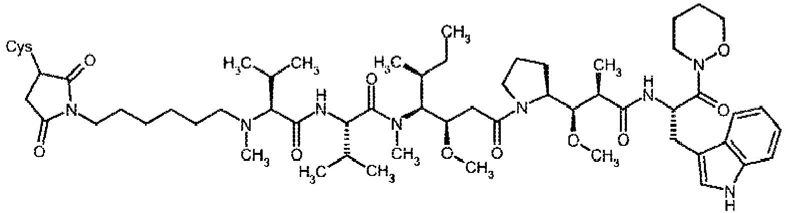

10 mg (10 µmol) von Intermediat 125 wurden in 4 ml DMF aufgenommen und mit 2.4 mg (20 µmol) L-Cystein versetzt. Das Reaktionsgemisch wurde 2h bei RT gerührt, anschließend im Vakuum eingeengt und dann mittels präparativer IIPLC gereinigt. Es wurden 7.7 mg (69% d.Th. der Titelverbindung erhalten.
HPLC (Methode 5): Rₜ = 1.7 min;
LC-MS (Methode 2): Rₜ = 1.91 min; MS (ESIpos): m/z = 1140 (M+H)⁺.

### Beispiel 77

### N-(4-{2-[6-(3-{[(2R)-2-Amino-2-carboxyethyl]sulfanyl}-2,5-dioxopyrrolidin-1-yl)hexanoyl] hydrazino}-4-oxobutyl)-N-methyl-L-valyl-N-[(3R,4S,5S)-1-{(2S)-2-[(1R,2R)-3-{[(2S)-1-(benzylamino)-3-(1H-indol-3-yl)-1-oxopropan-2-yl]amino}-1-methoxy-2-methyl-3-oxopropyl] pyrrolidin-1-yl}-3-methoxy-5-methyl-1-oxoheptan-4-yl]-N-methyl-L-valinamid

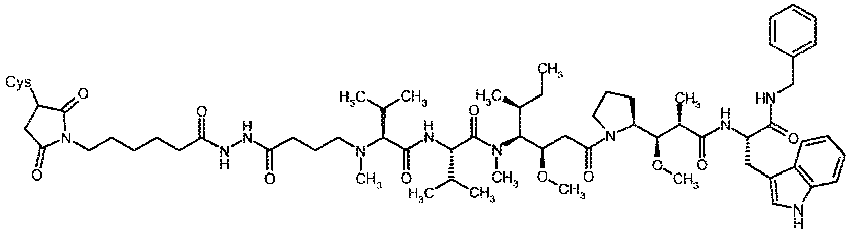

10 mg (10 µmol) von Intermediat 160 wurden in 3 ml DMF aufgenommen und mit 2.1 mg (20 µmol) L-Cystein versetzt. Das Reaktionsgemisch wurde 2h bei RT gerührt, anschließend im Vakuum eingeengt und dann mittels präparativer IIPLC gereinigt. Es wurden 8.1 mg (73% d.Th. der Titelverbindung erhalten.
IIPLC (Methode 5): Rₜ = 1.7 min;
LC-MS (Methode 1): Rₜ = 0.86 min; MS (ESIpos): m/z = 1274 (M+H)⁺.

### Beispiel 78

### N-(4-{2-[6-(3-{[(2R)-2-Amino-2-carboxyethyl]sulfanyl}-2,5-dioxopyrrolidin-1-yl)hexanoyl] hydrazino}-4-oxobutyl)-N-methyl-L-valyl-N-[(3R,4S,5S)-1-{(2S)-2-[(1R,2R)-3-{[(2S)-1-(benzylamino)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

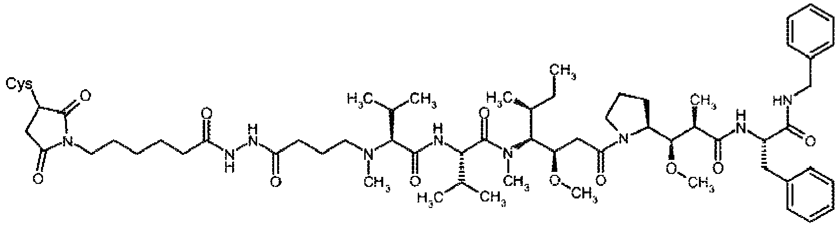

3.5 mg (3 µmol) von Intermediat 159 wurden in 1 ml DMF aufgenommen und mit 0.76 mg (6 µmol) L-Cystein versetzt. Das Reaktionsgemisch wurde 2h bei RT gerührt, anschließend im Vakuum eingeengt und dann mittels präparativer HPLC gereinigt. Es wurden 2.6 mg (65% d.Th. der Titelverbindung erhalten.
HPLC (Methode 5): Rₜ = 1.75 min;
LC-MS (Methode 1): Rₜ = 0.85 min; MS (ESIpos): m/z = 1235 (M+H)⁺.

### Beispiel 79

### N-(6-{2-[6-(3-{[(2R)-2-Amino-2-carboxyethyl]sulfanyl}-2,5-dioxopyrrolidin-1-yl)hexanoyl] hydrazino}-6-oxohexyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid

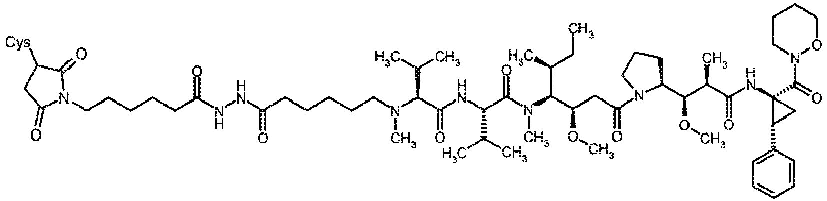

3.6 mg (3 µmol) von Intermediat 129 wurden in 1 ml DMF aufgenommen und mit 0.77 mg (6 µmοl) L-Cystein versetzt. Das Reaktionsgemisch wurde 2h bei RT gerührt, anschließend im Vakuum eingeengt und dann mittels präparativer HPLC gereinigt. Es wurden 1.55 mg (39% d.Th. der Titelverbindung erhalten.
HPLC (Methode 5): Rₜ = 1.6 min;
LC-MS (Methode 1): Rₜ = 0.87 min; MS (ESIpos): m/z = 1255 (M+H)⁺.

### Beispiel 80

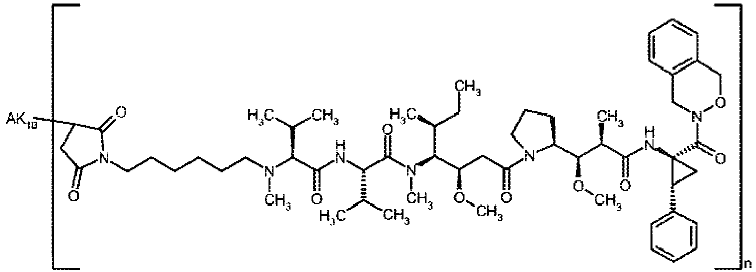

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 0.83 mg/ml
Drug/mAb Ratio: 1.6

### Beispiel 81

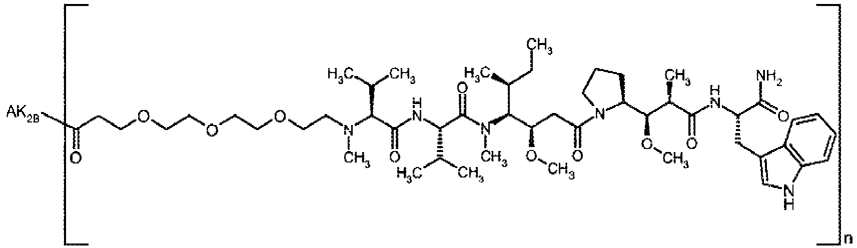

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.59 mg/ml
Drug/mAb Ratio: 3.1
Drug/mAb Ratio: 2.9

### Beispiel 82

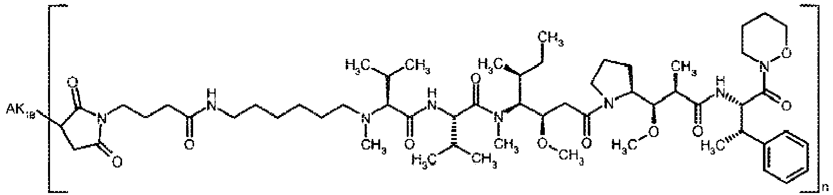

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.25 mg/ml
Drug/mAb Ratio: 4.0

### Beispiel 83

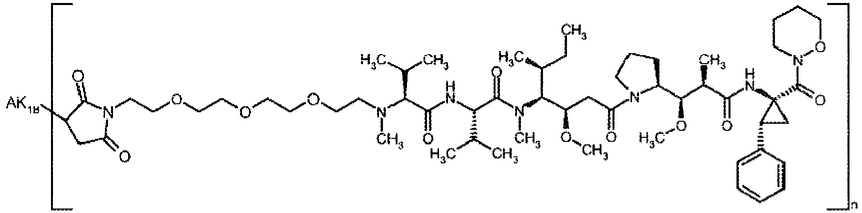

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.27 mg/ml
Drug/mAb Ratio: 3.6

### Beispiel 84

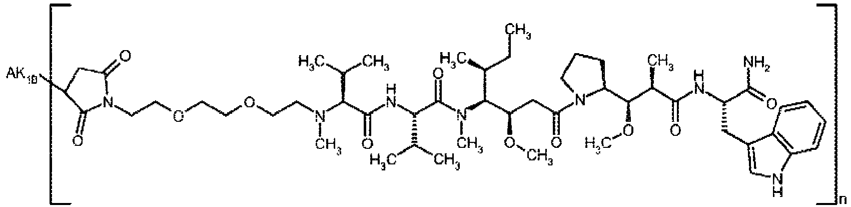

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.54 mg/ml
Drug/mAb Ratio: 4.7

### Beispiel 85

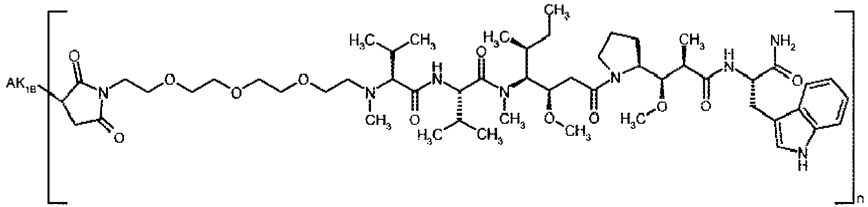

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.73 mg/ml
Drug/mAb Ratio: 4.7

### Beispiel 86

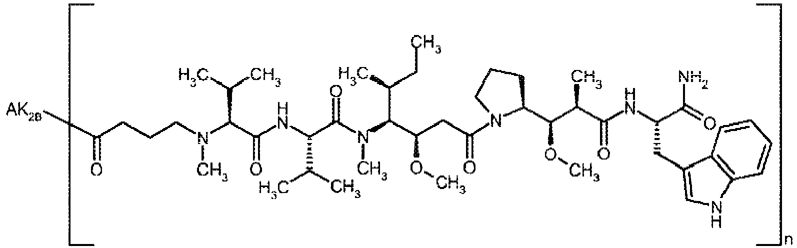

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.66 mg/ml
Drug/mAb Ratio: 1.3

### Beispiel 87

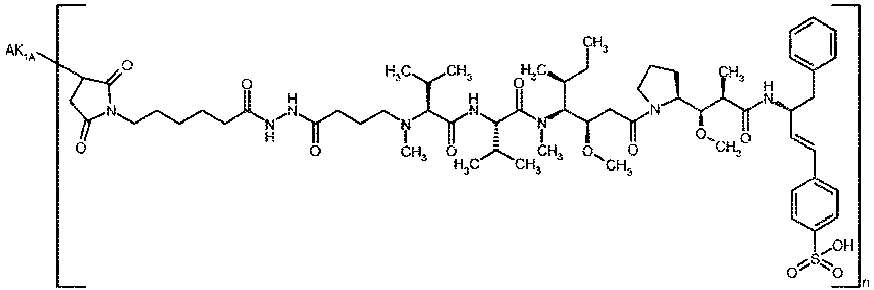

Protein Konzentration: 2.11 mg/ml
Drug/mAb Ratio: 5.5

### Beispiel 88

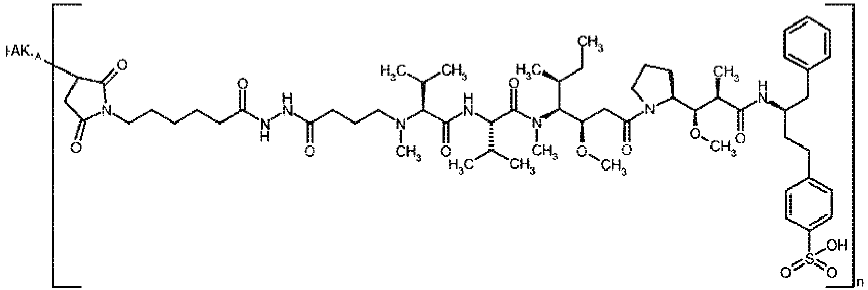

Protein Konzentration: 1.53 mg/ml
Drug/mAb Ratio: 3.4

### Beispiel 89

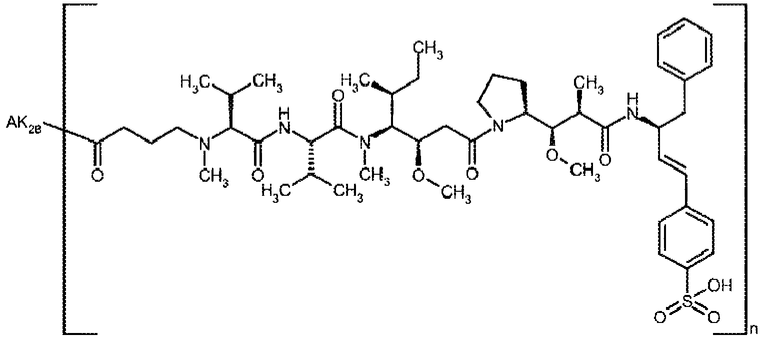

Protein Konzentration: 1.5 mg/ml
Drug/mAb Ratio: 0.2

### Beispiel 90

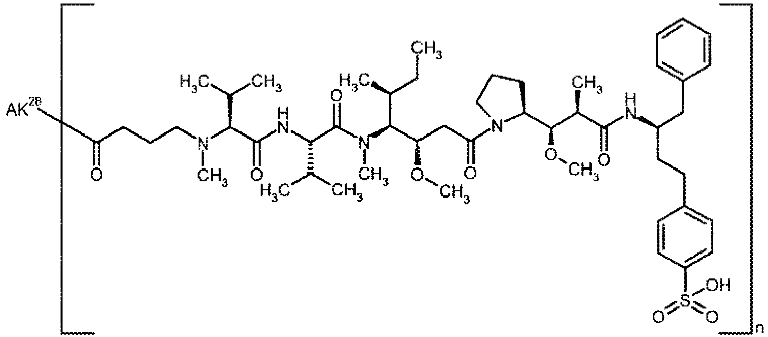

Protein Konzentration: 1.32 mg/ml
Drug/mAb Ratio: 0.1

### Beispiel 91

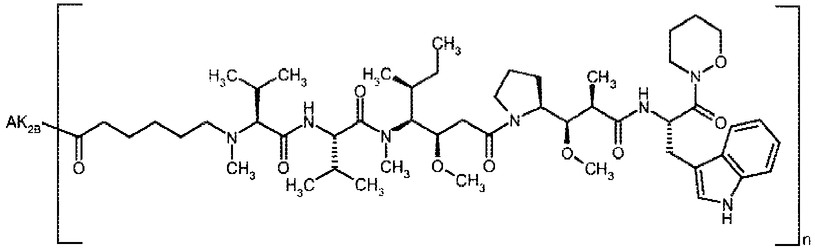

Eingesetzt wurden hier zur Kupplung 80 mg anti-C4.4a B01-3 in PBS und der Ansatz wurde nach der Sephadex-Reinigung durch Ultrazentrifugation aufkonzentriert, rückverdünnt mit PBS und erneut aufkonzentriert.
Protein Konzentration: 10.3 mg/ml
Drug/mAb Ratio: 3.1

### Beispiel 92

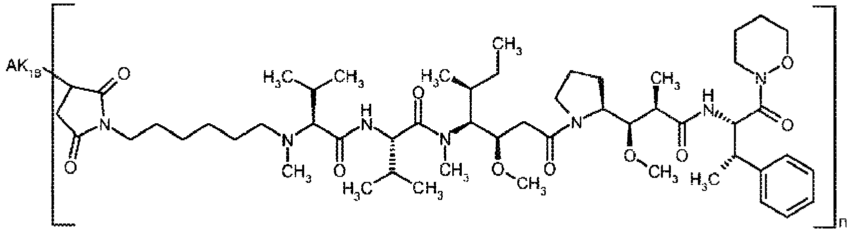

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.09 mg/ml
Drug/mAb Ratio: 1.8

### Beispiel 93

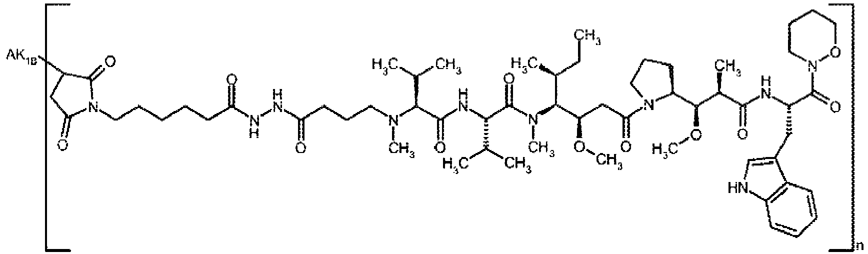

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.52 mg/ml
Drug/mAb Ratio: 4.2

### Beispiel 94

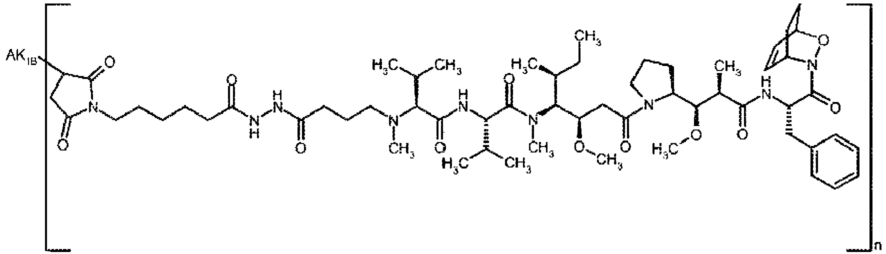

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.1 mg/ml
Drug/mAb Ratio: 3.3

### Beispiel 95

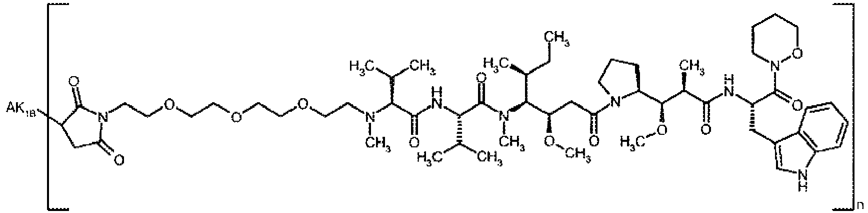

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.43 mg/ml
Drug/mAb Ratio: 4.8

### Beispiel 96

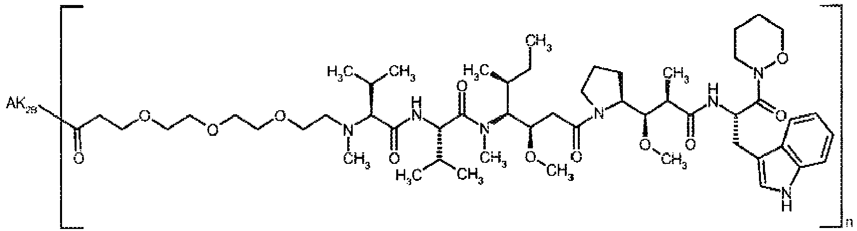

Eingesetzt wurden hier zur Kupplung 5 mg anti-C4.4a B01-3 in PBS und der Ansatz wurde nach der Sephadex-Reinigung durch Ultrazentrifugation aufkonzentriert, rückverdünnt mit PBS und erneut aufkonzentriert.
Protein Konzentration: 1.36 mg/ml
Drug/mAb Ratio: 4.6

### Beispiel 97

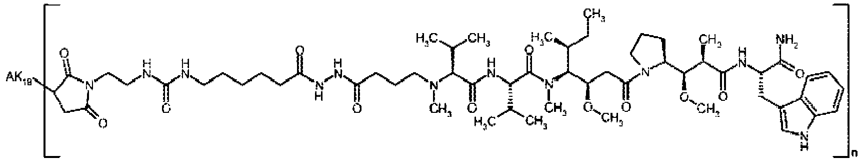

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.33 mg/ml
Drug/mAb Ratio: 4.0

### Beispiel 98

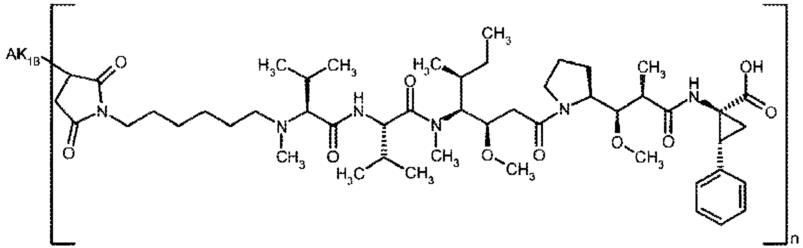

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.33 mg/ml
Drug/mAb Ratio: 4.6

### Beispiel 99

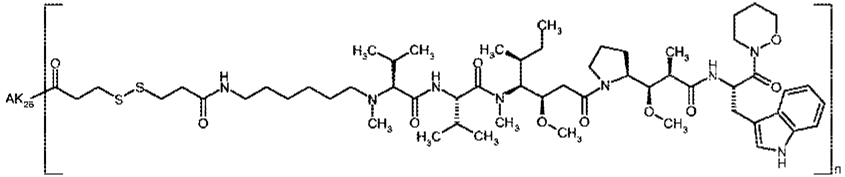

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.47 mg/ml
Drug/mAb Ratio: 1.6

### Beispiel 100

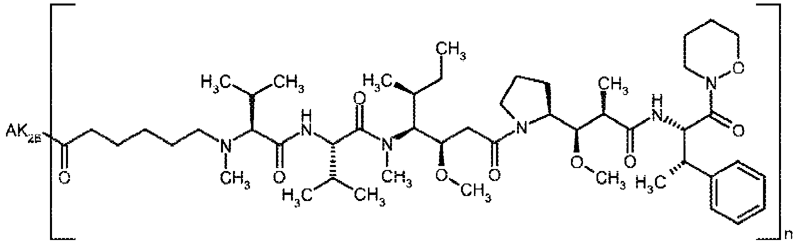

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.49 mg/ml
Drug/mAb Ratio: 4.5

### Beispiel 101

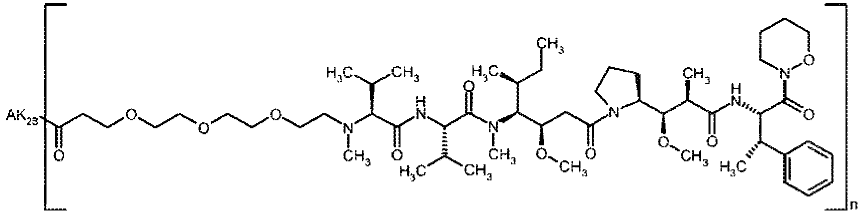

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.29 mg/ml
Drug/mAb Ratio: 3.3

### Beispiel 102

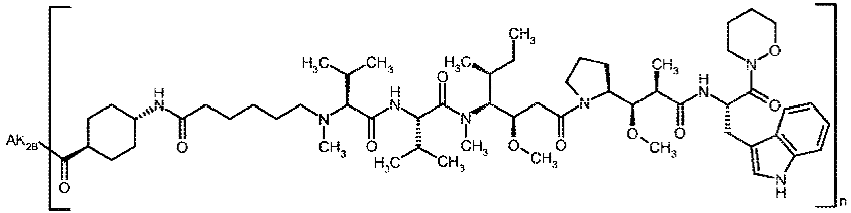

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.74 mg/ml
Drug/mAb Ratio: 3.5

### Beispiel 103

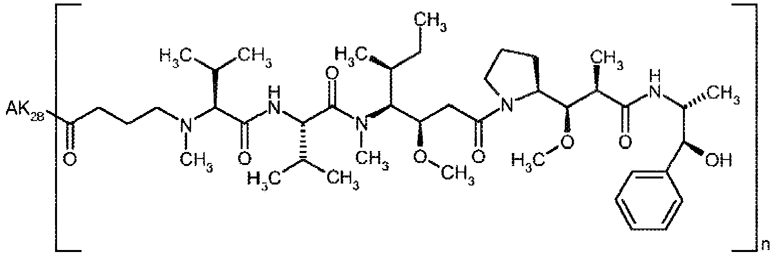

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.09 mg/ml
Drug/mAb Ratio: 3.2

### Beispiel 104

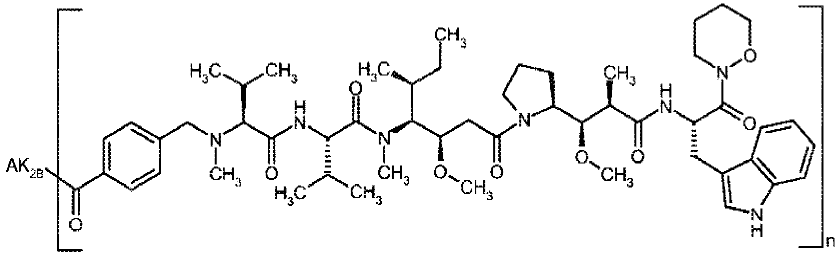

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.63 mg/ml
Drug/mAb Ratio: 0.2

### Beispiel 105

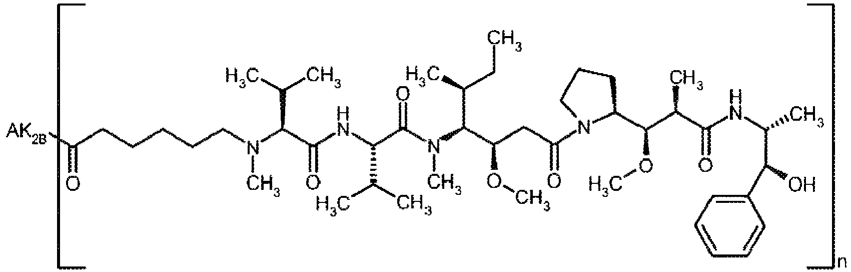

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.41 mg/ml
Drug/mAb Ratio: 7.6

### Beispiel 106

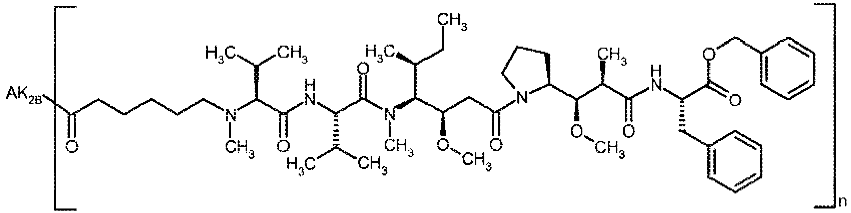

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 2.0 mg/ml
Drug/mAb Ratio: 1.6

### Beispiel 107

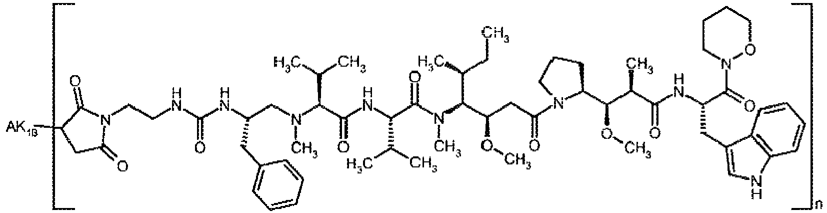

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.67 mg/ml
Drug/mAb Ratio: 2.8

### Beispiel 108

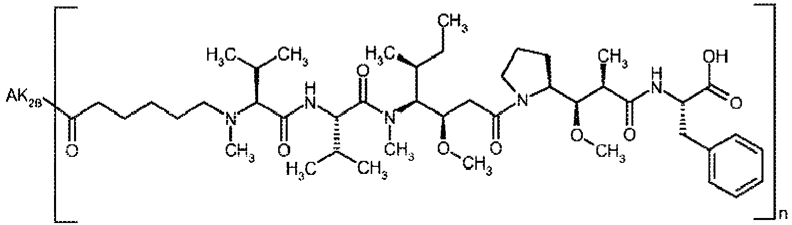

Eingesetzt wurden hier zur Kupplung 5 mg anti-C4.4a B01-3 in PBS und der Ansatz wurde nach der Sephadex-Reinigung durch Ulrazentrifugation aufkonzentriert und rückverdünnt.
Protein Konzentration: 1.91 mg/ml
Drug/mAb Ratio: 5.3

### Beispiel 109

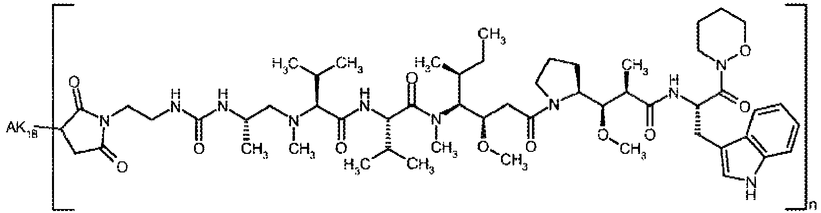

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.82 mg/ml
Drug/mAb Ratio: 4.6

### Beispiel 110

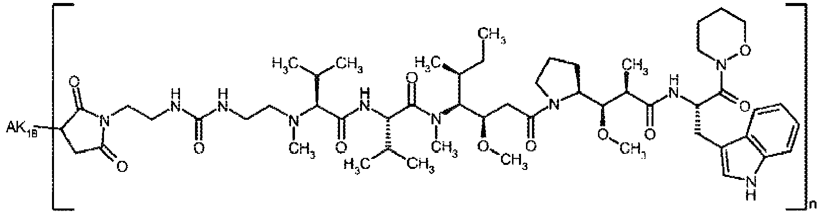

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.9 mg/ml
Drug/mAb Ratio: 4.2

### Beispiel 111

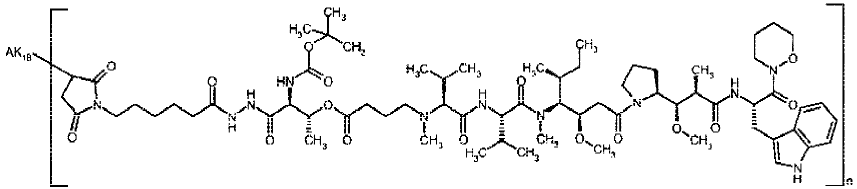

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Proteinkonzentration: 1.89 mg/ml
Drug/mAb-Ratio: 2.7

### Beispiel 112

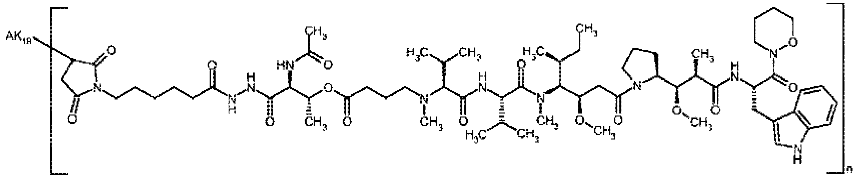

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Proteinkonzentration: 1.73 mg/ml
Drug/mAb-Ratio: 2.3

### Beispiel 113

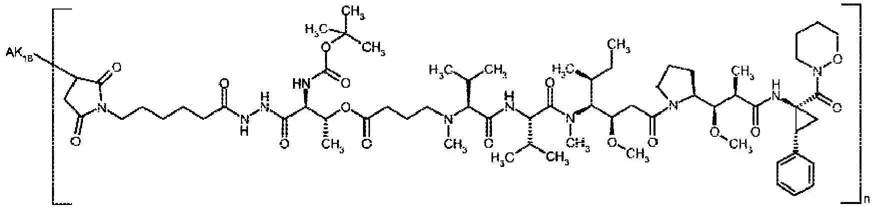

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Proteinkonzentration: 1.71 mg/ml
Drug/mAb-Ratio: 3.3

### Beispiel 114

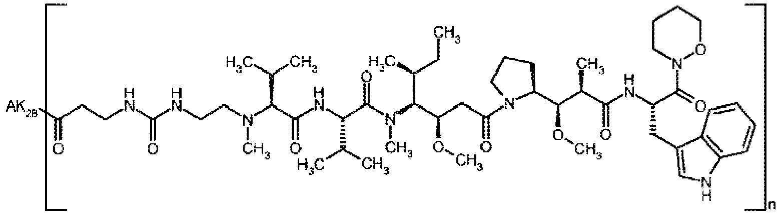

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.47 mg/ml
Drug/mAb Ratio: 3.9

### Beispiel 115

### N-(6-{[(5S)-5-Amino-5-carboxypentyl]amino}-6-oxohexyl)-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid-Trifluoracetat

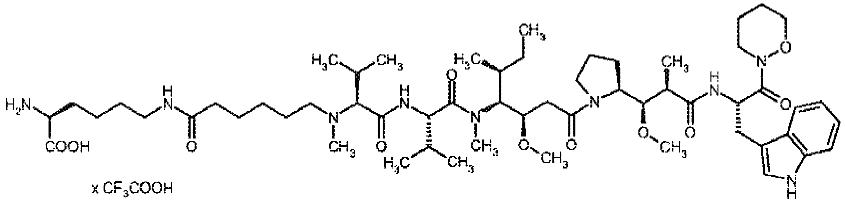

15.5 mg (15 µmοl) von Intermediat 210 wurden in 5 ml DMF aufgenommen und mit 4.4 mg (18 µmol) N²-(tert.-Butoxycarbonyl)-L-lysin sowie 7.7 µL (44 µmol) *N,N*-Diisopropylethylamin versetzt. Das Reaktionsgemisch wurde über Nacht bei RT gerührt und anschließend im Vakuum eingeengt. Der Rückstand wurde dann mittels präparativer HPLC gereinigt. Es wurden 14 mg (81% d.Th.) des geschützten Intermediats der Titelverbindung erhalten, das anschließend in 1 ml Dichlormethan aufgenommen und mit 1 ml Trifluoressigsäure entschützt wurde. Der Ansatz wurde eingeengt und nach Lyophilisation des Rückstands aus Acetonitril/Wasser (1:1) wurden 15 mg (97% d. Th.) der Titelverbindung erhalten.
HPLC (Methode 12): Rₜ = 1.8 min;
LC-MS (Methode 1): Rₜ = 0.79 min; MS (ESIpos): m/z = 1083 (M+H)⁺.

### Beispiel 116

### N-(6-{[(5S)-5-Amino-5-carboxypentyl]amino}-6-oxohexyl)-N-methyl-L-valyl-N-[(3R,4S,5S)-1-{(2S)-2-[(1R,2R)-3-{[(1S)-1-carboxy-2-(1H-indol-3-yl)ethyl]amino}-1-methoxy-2-methyl-3-oxopropyl]pyrrolidin-1-yl}-3-methoxy-5-methyl-1-oxoheptan-4-yl]-N-methyl-L-valinamid

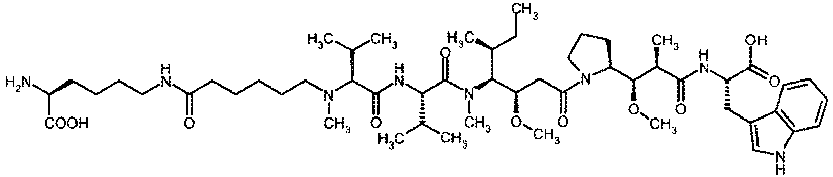

40 mg (40 µmol) von Intermediat 227 wurden in 5 ml DMF aufgenommen und mit 11.5 mg (40 µmοl) N²-[(Benzyloxy)carbonyl]-L-lysin sowie 13 µL (80 µmol) *N,N*-Diisopropylethylamin versetzt. Das Reaktionsgemisch wurde über Nacht bei RT gerührt, anschließend im Vakuum eingeengt und dann mittels präparativer HPLC gereinigt. Es wurden 32.5 mg (70% d.Th.) des geschützten Intermediats der Titelverbindung erhalten.

32.5 mg dieses Intermediats wurden in 10 ml Methanol gelöst und nach Zugabe von 2 mg 10%-igem Palladium auf Aktivkohle 30 min lang bei RT unter Wasserstoff-Normaldruck hydriert. Der Katalysator wurde danach abfiltriert und das Lösungsmittel im Vakuum entfernt. Nach Lyophilisation des Rückstandes aus Dioxan/Wasser 1:1 wurden 26 mg (99% d. Th.) der Titelverbindung erhalten.
HPLC (Methode 12): Rₜ = 1.7 min;
LC-MS (Methode 1): Rₜ = 0.76 min; MS (ESIpos): m/z = 1014 (M+H)⁺.

### Beispiel 117

### N-[(18S)-18-Amino-18-carboxy-12-oxo-3,6,9-trioxa-13-azaoctadec-1-yl]-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid-Trifluoracetat

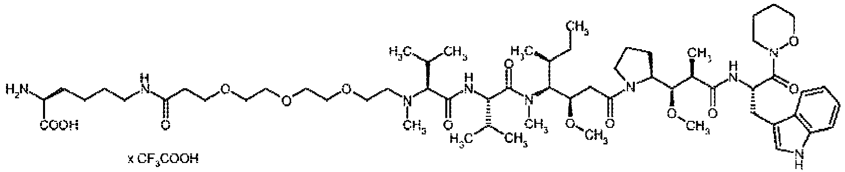

3.5 mg (3 µmol) von Intermediat 202 wurden in 2 ml DMF aufgenommen und mit 0.8 mg (3 µmol) N²-(tert.Butoxycarbonyl)-L-lysin sowie 1.6 µL (10 µmol) *N,N*-Diisopropylethylamin versetzt. Das Reaktionsgemisch wurde über Nacht bei RT gerührt und anschließend im Vakuum eingeengt. Der Rückstand wurde in Acetonitril/Wasser (1:1) aufgenommen, mit Trifluoressigsäure auf pH 2 gebracht und dann mittels präparativer IIPLC gereinigt. Es wurde 1 mg (25% d.Th.) des geschützten Intermediats der Titelverbindung erhalten, das anschließend in 500µl Dichlormethan aufgenommen und mit 500µl Trifluoressigsäure entschützt wurde. Der Ansatz wurde eingeengt und nach Lyophilisation des Rückstands aus Acetonitril/Wasser (1:1) wurde 1 mg (89% d. Th.) der Titelverbindung erhalten.
HPLC (Methode 12): Rₜ = 1.9 min;
LC-MS (Methode 1): Rₜ = 0.82 min; MS (ESIpos): m/z = 1173 (M+H)⁺.

### Beispiel 118

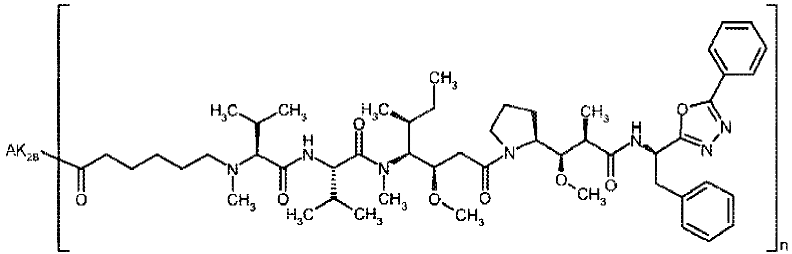

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Proteinkonzentration: 0.89 mg/ml
Drug/mAb Ratio: 1.8

### Beispiel 119

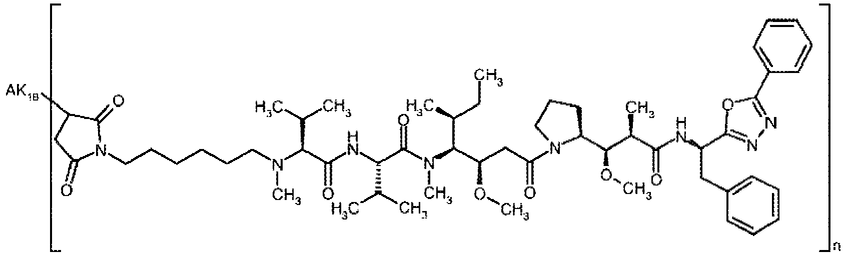

Eingesetzt wurden hier zur Kupplung 5 mg anti-C4.4a B01-3 und der Ansatz wurde nach der Sephadex-Reinigung durch Ultrazentrifugation aufkonzentriert und rückverdünnt.
Proteinkonzentration: 0.57 mg/ml
Drug/mAb Ratio: 1.5

### Beispiel 120

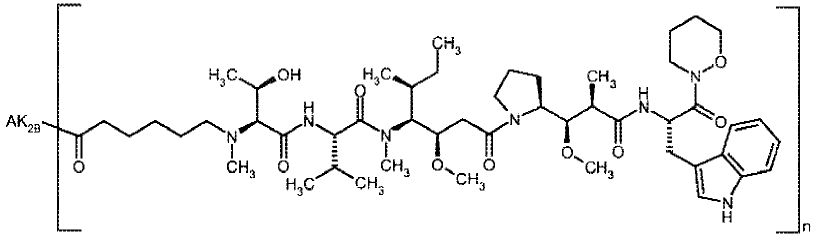

Eingesetzt wurden hier zur Kupplung 5 mg anti-C4.4a B01-3 und das Reaktionsgemisch wurde nach der Sephadex-Reinigung durch Ultrazentrifugation aufkonzentriert und rückverdünnt mit PBS.
Protein Konzentration: 1.39 mg/ml
Drug/mAb Ratio: 7.1

### Beispiel 121

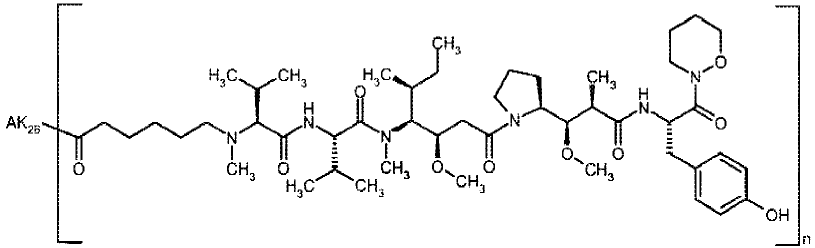

Eingesetzt wurden hier zur Kupplung 5 mg anti-C4.4a B01-3 und das Reaktionsgemisch wurde nach der Sephadex-Reinigung durch Ultrazentrifugation aufkonzentriert und rückverdünnt mit PBS.
Protein Konzentration: 1.54 mg/ml
Drug/mAb Ratio: 2.4

### Beispiel 122

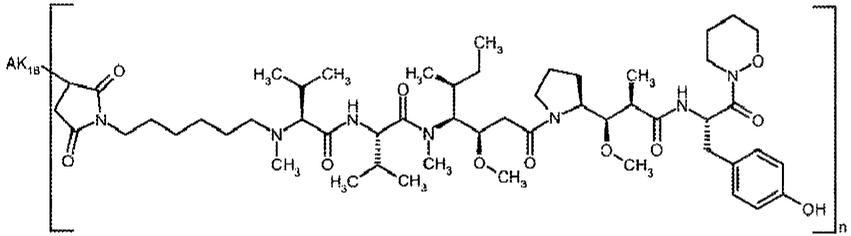

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Protein Konzentration: 1.48 mg/ml
Drug/mAb Ratio: 2.4

### Beispiel 123

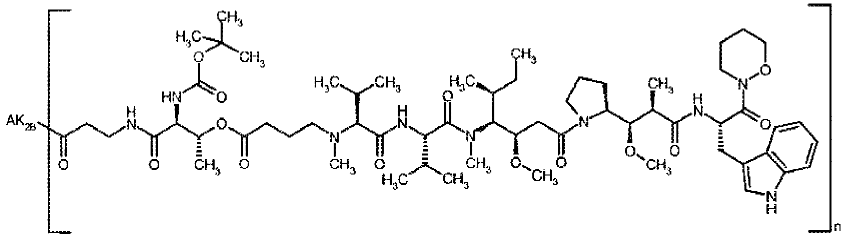

Eingesetzt wurden hier zur Kupplung 5 mg anti-C4.4a B01-3 in PBS und der Ansatz wurde nach der Sephadex-Reinigung durch Ultrazentrifugation aufkonzentriert und rückverdünnt.
Proteinkonzentration: 1.43 mg/ml
Drug/mAb-Ratio: 3.6

### Beispiel 124 Diastereomer 1

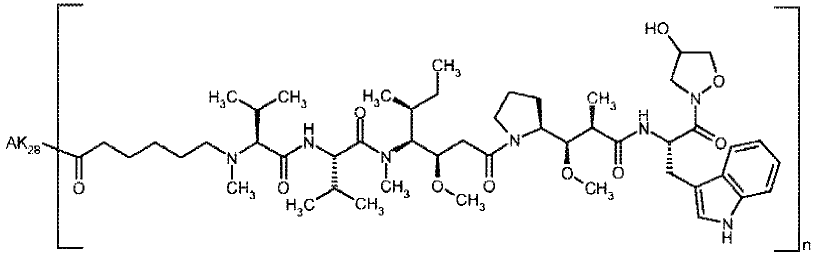

Eingesetzt wurden hier zur Kupplung Intermediat 247a und 5 mg anti-C4.4a B01-3 in PBS und der Ansatz wurde nach der Sephadex-Reinigung durch Ultrazentrifugation aufkonzentriert und rückverdünnt mit PBS.
Protein Konzentration: 1.45 mg/ml
Drug/mAb Ratio: 3.8

### Beispiel 125 Diastereomer 2

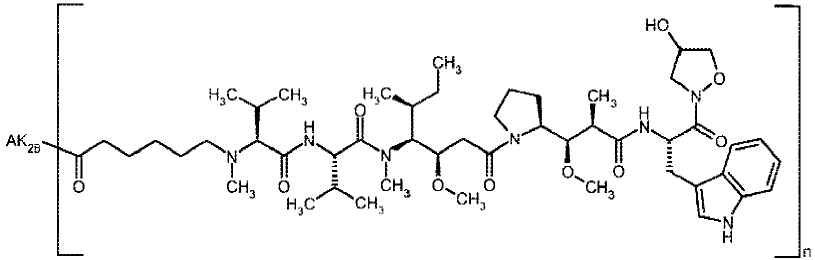

Eingesetzt wurden hier zur Kupplung Intermediat 247a und 5 mg anti-C4.4a B01-3 in PBS und der Ansatz wurde nach der Sephadex-Reinigung durch Ultrazentrifugation aufkonzentriert und rückverdünnt mit PBS.
Protein Konzentration: 1.42 mg/ml
Drug/mAb Ratio: 4.0

### Beispiel 126

### N-(6-{[(5S)-5-Amino-5-carboxypentyl]amino}-6-oxohexyl)-N-methyl-L-threonyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid-Trifluoracetat

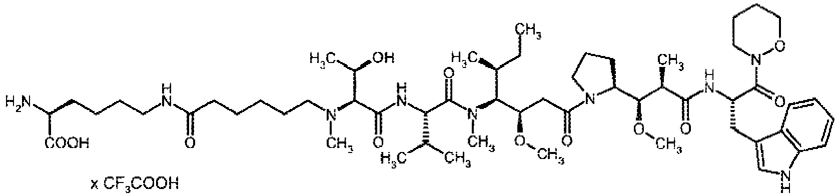

8.6 mg (8 µmol) Intermediat 240 wurden in 5 ml DMF aufgenommen und mit 4.0 mg (16 µmol) N²-(tert.-Butoxycarbonyl)-L-lysin sowie 2 µL (16 µmol) *N,N-*Diisopropylethylamin versetzt. Das Reaktionsgemisch wurde 4h bei RT gerührt, dann nochmals mit den gleichen Mengen N²-(tert.-Butoxycarbonyl)-L-lysin und *N,N*-Diisopropylethylamin versetzt und über Nacht bei RT gerührt. Anschließend wurde das Reaktionsgemisch im Vakuum eingeengt. Der Rückstand wurde dann mittels präparativer IIPLC gereinigt. Es wurden 7 mg (72% d.Th.) des geschützten Intermediats der Titelverbindung erhalten, das anschließend in 1 ml Dichlormethan aufgenommen und mit 0.5 ml Trifluoressigsäure entschützt wurde. Das Reaktionsgemisch wurde eingeengt und der Rückstand durch präparative IIPLC gereinigt. Nach Trocknung im Hochvakuum wurden 3.3 mg (47% d. Th.) der Titelverbindung erhalten.
HPLC (Methode 5): Rₜ = 1.5 min;
LC-MS (Methode 1): Rₜ = 0.8 min; MS (ESIpos): m/z = 1084 (M+H)⁺.

### Beispiel 127

### N-(6-{[(5S)-5-Amino-5-carboxypentyl]amino}-6-oxohexyl)-N-methyl-L-valyl-N-[(3R,4S,5S)-1-{(2S)-2-[(1R,2R)-3-{[(2S)-3-(4-hydroxyphenyl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid-Trifluoracetat

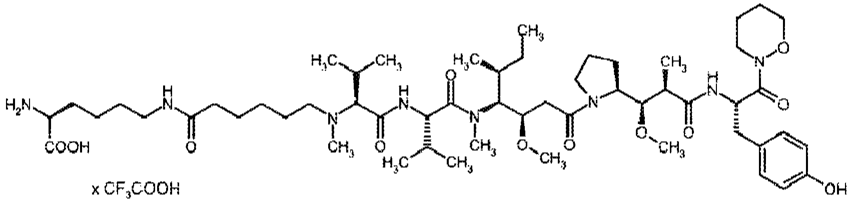

8 mg (8 µmol) Intermediat 242 wurden in 3 ml DMF aufgenommen und mit 2.9 mg (12 µmol) N²-(tert.-Butoxycarbonyl)-L-lysin sowie 2.7 µL (16 µmol) *N,N*-Diisopropylethylamin versetzt. Das Reaktionsgemisch wurde über Nacht bei RT gerührt, dann nochmals mit den gleichen Mengen N²-(tert.-Butoxycarbonyl)-L-lysin und *N,N*-Diisopropylethylamin versetzt und dann weitere 4h bei RT gerührt. Anschließend wurde das Reaktionsgemischz im Vakuum eingeengt. Der Rückstand wurde dann mittels präparativer HPLC gereinigt. Nach Lyophilisation aus Acetonitril/Wasser wurden 6.5 mg (72% d.Th.) des geschützten Intermediats der Titelverbindung erhalten, das anschließend in 5 ml Dichlormethan aufgenommen und mit 0.75 ml Trifluoressigsäure entschützt wurde. Der Ansatz wurde eingeengt und nach Lyophilisation des Rückstands aus Dioxan/Wasser wurden 5 mg (76% d. Th.) der Titelverbindung erhalten.

HPLC (Methode 12): Rₜ = 1.7 min:
LC-MS (Methode 1): Rₜ = 0.69 min; MS (ESIpos): m/z = 1059 (M+H)⁺.

### Beispiel 128

### N-(6-{[(5S)-5-Amino-5-carboxypentyl]amino}-6-oxohexyl)-N-methyl-L-valyl-N-[(3R,4S,5S)-1-{(2S)-2-[(1R,2R)-3-{[(1S)-1-carboxy-2-(4-hydroxyphenyl)ethyl]amino}-1-methoxy-2-methyl-3-oxopropyl]pyrrolidin-1-yl}-3-methoxy-5-methyl-1-oxoheplan-4-yl]-N-methyl-L-valinamid-Trifluoracetat

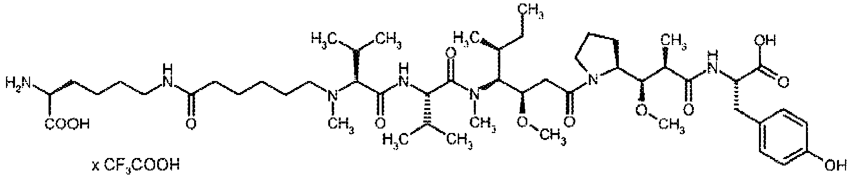

38 mg (41 µmol) Intermediat 248 wurden zunächst in den N-Hydroxysuccinimid-ester überführt. 72 mg des erhaltenen Rohproduktes wurden in 5 ml DMF aufgenommen und mit 24 mg (100 µmοl) N²-(tert.-Butoxycarbonyl)-L-lysin sowie 23 µL *N,N*-Diisopropylethylamin versetzt. Das Reaktionsgemisch wurde über Nacht bei RT gerührt, dann nochmals mit 16 mg N²-(tert.-Butoxycarbonyl)-L-lysin und 12 µL *N,N*-Diisopropylethylamin versetzt und anschließend weitere 2h im Ultraschallbad behandelt. Dann wurde der Ansatz im Vakuum eingeengt und der Rückstand mittels präparativer HPLC gereinigt. Nach Lyophilisation aus Acetonitril/Wasser wurden 20 mg (50% d.Th.) des geschützten Intermediats der Titelverbindung erhalten.

15 mg (12 µmοl) dieses Intermediats wurden anschließend in 3 ml Dichlormethan aufgenommen und mit 1 ml Trifluoressigsäure versetzt. Nach 40 min Rühren bei RT wurden weitere 1.5 ml Trifluoressigsäure zugegeben und der Ansatz 1 h im Ultraschallbad behandelt. Danach wurde die Reaktionsmischung eingeengt und nach Lyophilisation des Rückstands aus Dioxan/Wasser wurden 13 mg (90%, d. Th.) der Titelverbindung erhalten.
HPLC (Methode 12): Rₜ = 1.5 min;
LC-MS (Methode 1): Rₜ = 0.68 min; MS (ESIpos): m/z = 990 (M+H)⁺.

### C: Bewertung der biologischen Wirksamkeit:

Die biologische Wirkung der erfindungsgemäßen Verbindungen wurde in den nachstehend beschriebenen Assays gezeigt

### C-1. Analyse der cytotoxischen Wirkung der gegen C4.4a gerichteten ADCs

Die Analyse der cytototoxischen Wirkung der anti-C4.4a-ADCs erfolgt auf verschiedenen Zelllinien:
- A549 (CCL-185, ATCC), die mit mit der Sequenz für den vollständigen C4.4a Rezeptor transfiziert wurden,
- A549, Mock transfiziert
- A549 Wildtyp (DSMZ, lot 11)
- NCI-H292, endogen C4.4a exprimierende Lungenkrebs-Zelllinie (CRL-1848, ATCC)
- SCC-4 endogen C4.4a exprimierende Plattenepithelkarzinom-Zelllinie (CRL-1624, ATCC)
- SCC-9 endogen C4.4a exprimierende Plattenepithelkarzinom-Zelllinie (CRL-1629, ATCC)
- HCT-116 endogen C4.4a exprimierende Kolonkarzinom-Zelllinie (CCL-247, ATCC)
- HCT-116/VM46, HCT-116 die mit VM46 transfiziert wurden
- A431NS (CRL-2592, ATCC)

Kultivierung der Zellen erfolgt nach Standardmethode, wie bei der American tissue type collection (ATCC) für die jeweiligen Zelllinien angegeben. Zur Durchführung werden die Zellen mit einer Lösung von Trypsin (0.05%) und EDTA (0.02%) in PBS (Biochrom AG #L2143) abgelöst, pelletiert, in Kulturmedium resuspendiert, gezählt und in eine 96-Loch Kulturplatte mit weissem Boden (Costar #3610) ausgesät (2500 Zellen in 100µl/ well) und im Brutschrank bei 37°C und 5% Kohlendioxid inkubiert. Nach 24 h werden die Antikörper-Wirkstoffkonjugate in 100µl Kulturmedium in Konzentrationen von 10⁻⁷M bis 10⁻¹¹M auf die Zellen gegeben (Doppelwerte) und im Brutschrank bei 37°C und 5% Kohlendioxid inkubiert. Nach 72 h wird die Zellviabilität mit dem Cell Titer Glow Luminescent Cell Viability Assay bestimmt.(Promega #G7573 und #G7571). Dazu werden pro Zellansatz 100 µl des Substrats hinzugefügt, die Platten anschließend mit Alufolie abgedeckt, für 2 Minuten mit dem Plattenschüttler bei 180 rpm geschüttelt, für 8 Minuten auf der Laborbank stehen gelassen und dann mit dem Victor X2 (Perkin Elmer) gemessen. Das Substrat detektiert den ATP-Gehalt in den lebenden Zellen, wobei ein Lumineszenz Signal erzeugt wird, dessen Höhe direkt proportional zu Vitalität der Zellen ist. Aus den gemessenen Daten wird die IC₅₀ mit der Laborsoftware Graph Pad Prism berechnet.

In der folgenden Tabelle 3 sind die IC₅₀-Werte¹⁾ repräsentativer Ausführungsbeispiele aus diesem Assay aufgeführt:

**Tabelle 3**

| **Beispiel** | **IC₅₀ [nM] A549 :C4.4a** | **IC₅₀ [nM] A549 Mock** | **Beispiel** | **IC₅₀ [nM] A549 :C4.4a** | **IC₅₀ [nM] A549 Mock** | **Beispiel** | **IC₅₀ [nM] A549 :C4.4a** | **IC₅₀ [nM] A549 Mock** |
|---|---|---|---|---|---|---|---|---|
| 1 | 0.081 | 11.15 | 41 | 0.4 | 2 | 90 | >100 | >100 |
| 2 | 0.7 | 50 | 42 | 0.1 | 14 | 91 | 0.15 | kH |
| 3 | 0.47 | 4.75 | 43 | 0.062 | 6.33 | 92 | 0.29 | >100 |
| 4 | 0.6 | 100 | 44 | 0.044 | 6.93 | 93 | 0.04 | >100 |
| 5 | 0.4 | 20 | 45 | 0.058 | 4.01 | 94 | 0.035 | 100 |
| | 0.2 | 26 | | | | | | |
| | 0.1 | 17 | | | | | | |
| 6 | 0.53 | 4.50 | 46 | 0.062 | 7.74 | 95 | 0.036 | >100 |
| 7 | 0.39 | 32 | 47 | 0.066 | 9.11 | 96 | 0.018 | >100 |
| 8 | 0.01 | 0.15 | 48 | 0.061 | 6.78 | 97 | 0.062 | >100 |
| 9 | 0.43 | 10 | 49 | 0.076 | 100 | 98 | 0.06 | >100 |
| 10 | 0.01 | 25 | 50 | 0.02 | 0.02 | 99 | 0.1 | 80 |
| 11 | 4 | >100 | 51 | 0.044 | 44 | 100 | 0.1 | kH |
| 12 | 0.58 | 6.36 | 52 | 0.04 | 45 | 101 | 0.3 | kH |
| 13 | 0.7 | 14.9 | 53 | 0.046 | 26 | 102 | 0.1 | kH |
| 14 | 0.1 | 65.5 | 54 | 0.074 | >100 | 103 | 0.2 | 30 |
| 15 | 0.030 | 9.53 | 55 | 0.053 | >100 | 104 | 3 | kH |
| 16 | 3.8 | 21 | 56 | 0.037 | 60 | 105 | 0.03 | 50 |
| 17 | 0.62 | 4.19 | 57 | 0.3 | 1 | 106 | 0.05 | 20 |
| 18 | 0.4 | >100 | 58 | 0.04 | >100 | 107 | >100 | kH |
| 19 | 1.2 | 66.1 | 59 | 0.1 | >100 | 108 | 0.03 | >100 |
| 20 | 0.46 | 4.20 | 60 | 0.04 | >100 | 109 | 1 | >100 |
| 21 | 4.5 | 12.7 | 61 | 0.44 | 6.8 | 110 | 0.2 | kH |
| 22 | 5 | 16 | 62 | 0.09 | 50 | 112 | 0.27 | >100 |
| 23 | 0.4 | 0.7 | 63 | 0.1 | 0.4 | 113 | 3 | >100 |
| 24 | 0.3 | 23 | 64 | 0.04 | 0.52 | 114 | 0.05 | >100 |
| 25 | 5.4 | 53 | 65 | 0.03 | 0.04 | 118 | 0.29 | 20 |
| 26 | 0.052 | 11.27 | 66 | 0.03 | 0.04 | 119 | 0.32 | 15 |
| 27 | 0.65 | 6.70 | 67 | 0.08 | 26 | 120 | 0.07 | >100 |
| 28 | 0.062 | >100 | 68 | 0.02 | >100 | 121 | 0.03 | 7 |
| 29 | 0.02 | 2.5 | 69 | 0.17 | 0.27 | 122 | 0.04 | >100 |
| 30 | 0.1 | 71 | 70 | 0.06 | 7 | 123 | 0.02 | >100 |
| 31 | 0.32 | 9 | 80 | 3.0 | >100 | 124 | 0.04 | >100 |
| 32 | 0.035 | 6.19 | 81 | 0.045 | >100 | 125 | 0.04 | >100 |
| 33 | 0.037 | ∼30 | 82 | 0.06 | >100 | | | |
| 34 | | | 83 | 0.27 | >100 | | | |
| 35 | 0.3 | 20 | 84 | 0.13 | >100 | | | |
| 36 | 0.08 | >100 | 85 | 0,14 | >100 | | | |
| 37 | 0.1 | kH | 86 | 0.17 | >100 | | | |
| 38 | 0.03 | 50 | 87 | 0,28 | >100 | | | |
| 39 | 0.04 | 1.5 | 88 | 1.1 | >100 | | | |
| 40 | 0.6 | 50 | 89 | 1.3 | >100 | | | |

| | | | | | | | | |
|---|---|---|---|---|---|---|---|---|
| ¹⁾ Die angegebenen Wirkdaten beziehen sich auf die im vorliegenden experimentellen Teil beschriebenen Ausführungsbeispiele mit den angegebenen Drug/mAB-ratios. Die Werte können bei anderen Drug/mAB-ratios gegebenenfalls abweichen. | | | | | | | | |

### C-2. Bestimmung des Einflusses auf die Tubulinpolymerisation

Krebszellen sind entartete Zellen, die häufig auch durch eine gesteigerte Zellteilung zu einer Tumorbildung führen. Microtubuli bilden die Spindelfasern des Spindelapparates und sind ein essentieller Bestandteil des Zellzyklus. Der geregelte Auf- und Abbau von Microtubuli ermöglicht die genaue Aufteilung der Chromosomen auf die Tochterzellen und stellt einen kontinuierlich dynamischen Prozess dar. Eine Störung dieser Dynamik führt zu einer fehlerhaften Zellteilung und letztlich zum Zelltod. Die gesteigerte Zellteilung von Krebszellen macht diese jedoch auch besonders empfänglich gegenüber Spindelfasergiften, die einen festen Bestandteil der Chemotherapie darstellen- Spindelfasergifte wie Paclitaxel oder Epothilon führen zu einer stark erhöhten Polymerisationsgeschwindigkeit der Microtubuli, während Vinca-Alkaloide oder auch Monomethylauristatin E (MMAE) zu einer stark reduzierten Polymerisationsgeschwindigkeit der Microtubuli führen. In beiden Fällen ist die notwendige Dynamik des Zellzyklus empfindlich gestört. Die im Rahmen der vorliegenden Erfindung untersuchten Verbindungen führen zu einer reduzierten Polymerisationsgeschwindigkeit der Microtubuli.

Zur Untersuchung der Tubulinpolymerisation wurde das "Fluorescence-based Microtubule Polymerisation Assay Kit" der Firma Cytoskeleton (Denver, Colorado, USA; Bestellnummer: BK011) verwendet. Bei diesem Assay wird unpolymerisiertem Tubulin GTP zugesetzt, womit die Polymerisation spontan stattfinden kann. Der Assay beruht auf der Bindung des Fluorophors 4',6-Diamidino-2-phenylindol (DAPI) an Tubulin. Freies und gebundenes DAPI können aufgrund unterschiedlicher Emissionsspektra differenziert werden. Da DAPI eine deutlich höhere Affinität gegenüber polymerisiertem im Vergleich zu nicht-polymerisiertem Tubulin zeigt, läßt sich die Tubulinpolymerisation über die Zunahme der Fluoreszenz gebundener DAPI-Fluorophore verfolgen.

Zur Durchführung dieses Assays wurden die in DMSO gelösten erfindungsgemäßen Verbindungen von ihrer Ausgangskonzentration von 10 mM auf 1 µM in Wasser verdünnt. Neben der Puffer-Kontrolle wurde als Assay-Kontrolle auch polymerisationssteigernd Paclitaxel und andererseits polymensationshemmend Vinblastin mitgeführt. Für die Messung wurden 96-well-Lochplatten mit halber Bodenfläche verwendet. Die Kinetik der Tubulinpolymerisation wurde 1 h lang bei 37°C in einem Fluorimeter verfolgt. Die Anregungswellenlänge betrug 355 nm, die Emission wurde bei 460 nm verfolgt. Für den Bereich des linearen Anstiegs innerhalb der ersten 10 Minuten wurde die Fluoreszenzänderung pro Minute (ΔF/min) berechnet, welche die Polymerisationsgeschwindigkeit der Microtubuli darstellt. Die Potenz der Testsubstanzen wurde anhand der jeweiligen Reduktion der Polymerisationsgeschwindigkeit quantifiziert.

Der Wert der Inhibition von MMAF bei einer Konzentration von 1µM wird als 100% gesetzt.

In Tabelle 4 sind Daten zur Inhibition der Tubulin Polymerisation von repräsentativen Ausführungsbeispielen angegeben.

**Tabelle 4.**

| **Ausführungsbeispiel** | **Konzentration Toxophor [µM]** | **Tubulin-polymerisation in Gegenwart von Toxophor in [%]. Tubulin-polymerisationsgeschwindigkeit bei 1µM MMAF gesetzt auf 100%** |
|---|---|---|
| MMAF | 1 | 100 |
| MMAF | 10 | 34 |
| MMAF | 100 | 0 |
| 115 | 1 | 45 |
| 115 | 10 | 1 |
| 1 16 | 1 | 80 |
| 116 | 10 | 14 |
| 117 | 1 | 60 |
| 117 | 10 | 0 |
| 71 | 1 | 88 |
| 71 | 10 | 25 |
| 72 | 1 | 109 |
| 72 | 10 | 27 |
| 73 | 1 | 120 |
| 74 | 1 | 117 |
| 74 | 10 | 64 |
| 75 | 1 | 107 |
| 75 | 10 | 25 |
| 76 | 1 | 121 |
| 76 | 10 | 35 |
| 77 | 1 | 111 |
| 77 | 10 | 45 |
| 78 | 1 | 110 |
| 117 | 1 | 78 |
| 117 | 10 | 24 |
| 126 | 1 | 102 |
| 126 | 10 | 31 |
| 127 | 1 | 88 |
| 127 | 10 | 21 |
| 128 | 1 | 90 |
| 128 | 10 | 17 |

Das Toxophore MMAF und die Ausführungsbeispiele hemmen konzentrationsabhängig die Tubulinpolymerisation. Bei 100 µM MMAF ist die Tubulinpolymerisation vollständig gehemm,t. Das Ausführungsbeispiel 115 hemmt die Tubulinpolymerisationsgeschwindigkeit bei 1 µM auf 45% des Wertes der bei 1 µM MMAF gemessen wird.

### C-3. In vitro Tests zur Bestimmung der Zell-Permeabilität

Die Zell-Permeabilität einer Substanz kann mittels *in vitro*-Testung in einem Flux-Assay unter Verwendung von Caco-2-Zellen untersucht werden [M.D. Troutman und D.R. Thakker, Pharm. Res. 20 (8), 1210-1224 (2003)]. Hierzu wurden die Zellen auf 24-Loch-Filterplatten für 15-16 Tage kultiviert. Zur Bestimmung der Permeation wurde das jeweilige Ausführungsbeispiel in einem HEPES-Puffer entweder apikal (A) oder basal (B) auf die Zellen gegeben und für 2 h inkubiert. Nach 0 h und nach 2 h wurden Proben aus den Cis- und Transkompartimenten entnommen. Die Proben wurden mittels HPLC (Agilent 1200, Böblingen, Deutschland) unter Verwendung von reverse phase-Säulen aufgetrennt. Das HPLC-System war über ein Turbo Ion Spray-Interface an ein Triple Quadropol-Massenspektrometer API 4000 (Applied Biosystems Applera, Darmstadt, Deutschland) gekoppelt. Die Permeabilität wurde anhand eines Pₐₚₚ-Wertes bewertet, welcher mittels der von Schwab *et al.* publizierten Formel berechnet wurde [D. Schwab et al., J. Med. Chem. 46, 1716-1725 (2003)].

Von entscheidender Bedeutung für Toxophore, die intrazellulär freigesetzt werden, ist die Permeabilität von B nach A [Pₐₚₚ (B-A)]: Je niedriger diese Permeabilität ist, desto länger ist die Verweilzeit des Ausführunsbeispiels in der Zelle nach intrazellulärer Freisetzung und damit auch die Zeit, die für eine Interaktion mit dem biochemischen Target (hier: Tubulin) zur Verfügung steht.

In der folgenden Tabelle 5 sind Permeabilitätsdaten repräsentativer Ausführungsbeispiele aus diesem Assay aufgeführt:

**Tabelle 5**

| **Ausführungsbeispiel** | **Pₐₚₚ (B-A) [nm/s]** |
|---|---|
| 71 | 2 |
| 72 | 1.6 |
| 73 | 2.5 |
| 74 | 5 |
| 75 | 1 |
| 77 | 7 |
| 115 | 2 |
| 116 | 1 |
| 126 | 1.8 |
| 127 | 1.5 |

Die Ausführungsbeispiele weisen eine niedrige Permeabilität von B nach A [Pₐₚₚ (B-A) auf und haben daher eine lange Verweildauer in den CaCo-2-Zellen. Im Vergleich hierzu weisen Monomethylauristatin E (MMAE) und Monomethylauristatin F (MMAF) in diesem Test einen Pₐₚₚ (B-A)-Wert von 73 nm/s auf und haben damit eine deutlich kürzere Verweildauer in den Caco-2 Zellen.

### C-4. In vitro Tests zur Bestimmung der Substrateigenschaften für P-Glycoprotein (P-gp)

Viele Tumorzellen exprimieren Transporterproteine für Wirkstoffe, was häufig mit einer Resistenzentwicklung gegenüber Cytostatika einhergeht. Substanzen, die keine Substrate von solchen Transporterproteinen wie beispielsweise P-Glyeoprotein (P-gp) oder BCRP sind, könnten somit ein verbessertes Wirkprofil aufzeigen.

Die Substrateigenschaften einer Substanz für P-gp (ABCB1) wurden mittels eines Flux-Assays unter Verwendung von LLC-PK1-Zellen, die P-gp überexprimieren (L-MDR1-Zellen), bestimmt [A.II. Sehinkel et al., J. Clin. Invest. 96, 1698-1705 (1995)]. Hierzu wurden die LLC-PK1- oder L-MDR1-Zellen auf 96-Loch-Felterplatten für 3-4 Tage kultiviert. Zur Bestimmung der Permeation wurde die jeweilige Testsubstanz allein oder in Gegenwart eines Inhibitors (wie z.B. Ivermectin oder Verapamil) in einem HEPES-Puffer entweder apikal (A) oder basal (B) auf die Zellen gegeben und für 2 h inkubiert. Nach 0 h und nach 2 h wurden Proben aus den Cis- und Transkompartimenten entnommen. Die Proben wurden mittels HPLC unter Verwendung von reverse phase-Säulen aufgetrennt. Das HPLC-System war über ein Turbo Ion Spray-Interface an ein Triple Quadropol-Massenspektrometer API 3000 (Applied Biosystems Applera, Darmstadt, Deutschland) gekoppelt. Die Permeabilität wurde anhand eines Pₐₚₚ-Wertes bewertet, welcher mittels der von Schwab *et al.* publizierten Formel berechnet wurde [D. Schwab et al., J. Med. Chem. 46, 1716-1725 (2003)].

Von entscheidender Bedeutung für Toxophore, die intrazellulär freigesetzt werden, ist die Permeabilität von B nach A [Pₐₚₚ (B-A)]: Je niedriger diese Permeabilität ist, desto länger ist die Verweilzeit des Ausführunsbeispiels in der Zelle nach intrazellulärer Freisetzung und damit auch die Zeit, die für eine Interaktion mit dem biochemischen Target (hier: Tubulin) zur Verfügung steht.

In der folgenden Tabelle 6 sind Permeabilitätsdaten repräsentativer Ausführungsbeispiele aus diesem Assay, welches in L-MDR1-Zellen durchgeführt wurde, aufgeführt:

**Tabelle 6**

| **Ausführungsbeispiel** | **Pₐₚₚ (B-A) [nm/s]** |
|---|---|
| 71 | 3 |
| 72 | 3.6 |
| 73 | 2.1 |
| 74 | 3.6 |
| 75 | 4 |
| 77 | 2 |
| 115 | 6 |
| 116 | 4 |

Die Ausführungsbeispiele weisen eine niedrige Permeabilität von B nach A [Pₐₚₚ (B-A) auf und haben daher eine lange Verweildauer in den L-MDR1-Zellen.

### C-5. Wirksamkeitstest in vivo

Die Wirksamkeit der erfindungsgemäßen Konjugate wurde *in vivo* beispielsweise mittels Xenograft-Modellen getestet. Der Fachmann kennt Methoden im Stand der Technik, wie die Wirksamkeit eines erfindungsgemäßen Konjugats getestet werden kann (siehe z.B. WO2005/081711; Polson et al., Cancer Res. 2009 Mar 15;69(6):2358-64). Beispielsweise wurden hierzu Nagern (z.B. Mäusen) eine Tumorzelllinie, welche das Zielmolekül des Binders exprimiert, implantiert. Anschließend wurde diesen Tumor-tragenden Nagern entweder ein erfindungsgemäßes Konjugat oder ein Kontrollantikörper-Konjugat oder isotonische Salzlösung appliziert. Die Applikation erfolgte einmalig oder öfters. Das Tumorwachstum wurde mittels einer Schieblehre zwei Mal wöchentlich ermittelt. Nach einem Tumorwachstum von mehreren Wochen wurde die Tumorgröße von Konjugat-behandelten Tieren und der Kontrollgruppe verglichen. Die Konjugat-behandelten Tiere zeigten eine signifikant geringere Tumorgröße.

### C-5a. Testung von ADCs in experimentellen Tumoren in der Maus

Die Vorhersagekraft von Maus-Xenograft Tumormodellen bezogen auf die klinische Situation bei Immunotoxintherapien ist oft begrenzt, zum Einen durch die mangelnde Kreuzreaktivität der therapeutischen Antikörper mit den murinen Spezies und zum Anderen durch das Auftreten von Anti Drug Antikörpern (ADA) im Menschen bei Gabe von murinen oder chimären Antikörpern. Um das volle Potential der spezifischen C4.4a Expression für die Krebstherapie z.B. für einen Immunkonjugatansatz zu nutzen, sind humane, hochaffine, selektive und Spezies-kreuzreaktive Antikörper erforderlich, wie sie erfindungsgemäß bevorzugt zum Einsatz kommen. Mit solchen Antikörpern liefern Maus-Xenograft Tumormodelle bezogen auf die klinische Situation aussagekräftige Erkenntnisse.

Humane Tumorzellen, die C4.4a exprimieren, werden subkutan in die Flanke von immunsupprimierten Mäusen inokuliert, beispielsweise Nude- oder SCID-Mäuse. 1-10 Millionen Zellen werden aus der Zellkultur abgelöst, zentrifugiert und mit 100 µl Medium oder 50% Medium / 50% Matrigel resuspendiert. Die Zellsuspension wird unter die Haut der Maus gespritzt.

Innerhalb von einigen Tagen wächst ein Tumor heran. Die Behandlung beginnt frühestens nach Tumoretablierung bei einer Tumorgröße von 25 mm².

Die Behandlung mit ADCs erfolgt über die intravenöse Route in die Schwanzvene der Maus. Das ADC wird in PBS gelöst und mit einem Volumen von 10 ml /kg appliziert.

Das Behandlungsschema richtet sich nach der Pharmakokinetik des Antikörpers. Als Standard wird drei Mal in Folge jeden vierten Tag behandelt. Die Behandlung kann aber auch weiter fortgesetzt werden oder es kann sich zu einem späteren Zeitpunkt ein zweiter Zyklus mit drei Behandlungstagen anschließen.

Standardmäßig werden 8 Tiere pro Behandlungsgruppe eingesetzt. Diese Zahl kann sich erhöhen, wenn besonders starke Schwankungen beim Tumorwachstum oder nach der Behandlung zu erwarten sind. Neben den Gruppen, die die Wirksubstanzen bekommen wird eine Gruppe als Kontrollgruppe nur mit dem Puffer nach dem gleichen Schema behandelt.

Im Verlauf des Experiments wird die Tumorfläche regelmäßig mit einer Schieblehre in zwei Dimensionen (Länge / Breite) gemessen.

Am Ende des Experiments werden die Tumore entnommen und gewogen. Der Quotient der mittleren Tumorgewichte der Therapiegruppe (T) mit der Kontrollgruppe (C) wird als T/C angegeben. Werden Kontroll- und Behandlungsgruppen zu unterschiedlichen Zeitpunkten beendet, wird der T/C Wert anhand der Tumorflächen der letzten gemeinsamen Messung aller Behandlungs- und Kontrollgruppen errechnet.

1 Mio SCC-4 Zellen werden subkutan in die Flanke von weiblichen NMRI-nude Mäusen inokuliert.

Die intravenöse Behandlung mit den ADCs wird bei einer mittleren Tumorgröße von 30-35 mm² begonnen. Wenn die Kontrollgruppen die maximal zulässige Größe erreicht haben wird der Versuch beendet und die Tumoren entnommen und gewogen. Alle getesteten C4_4a targetierenden ADCs haben Dosis-abhängig das Tumorwachstum gehemmt. In der Dosis von 30 mg/kg erreichten Beispiel 54, Beispiel 49, Beispiel 51 und Beispiel 53 jeweils einen T/C von < 0.1. Signifikante anti-Tumor Wirkung im Vergleich zur Kontrolle konnte für die Beispiele 49, 52, 53, 54 und 56 bis zu einer Dosis von 15 mg/kg erreicht werden, wobei T/C Werte von ≤ 0.29 erreicht wurden.

1 Mio NCI-H292 Zellen werden subkutan in die Flanke von weiblichen NMRI-nude Mäusen inokuliert.

Die intravenöse Behandlung mit den ADCs wird bei einer mittleren Tumorgröße von 30-35 mm² begonnen. Kontroll- und Behandlungsgruppen werden jeweils beendet, wenn die maximal zulässige Tumorgröße erreicht ist. So können Unterschiede im Nachwachsen von Tumoren nach Behandlungsende zur weiteren Charakterisierung der ADCs beitragen. Daher wurde zur Ermittlung der anti-Tumorwirkung im Vergleich zur Kontrolle (T/C) die Tumorflächen am letzten gemeinsamen Messzeitpunkt herangezogen. Im verwendeten NCI-H292 Mausmodell wird gezeigt, dass alle getesteten ADCs das Tumorwachstum Dosis-abhängig im Vergleich zur Kontrolle reduzieren. Eine signifikante anti-Tumor Wirkung konnte für das Beispiel 54 bis zu einer Dosis von 1.9 mg/kg erzielt werden, für das Beispiel 49 bis zu einer Dosis von 3.75 mg/kg. Die minimalen T/C Werte die in diesem Modell erzielt werden, sind für Beispiel 54 ein T/C von 0.16 bei 30 mg/kg, für Beispiel 49 ein T/C von 0.17 bei 30 mg/kg, für Beispiel 53 ein T/C von 0.16 bei 30 mg/kg, für Beispiel 51 ein T/C von 0.17 bei 15 mg/kg. und für Beispiel 70 ein T/C von 0,19 bei 3.75 mg/kg. Bei der vergleichenden Gabe der ADCs mit einer konstanten Dosis von 7.5 mg/kg konnte mit den Beispielen 49 und 54 je ein T/C von 0.20, mit Beispiel 51 ein T/C von 0.27, mit Beispiel 52 ein T/C von 0.22, mit Beispiel 53 ein T/C von 0.23. mit Beispiel 55 ein T/C von 0.24, mit Beispiel 56 ein T/C von 0.21 und Beispiel 70 ein T/C von 0.17 erzielt werden.

### C-6. Pharmakokinetik im A549 Tumormodell mit C4.4a-transfizierten bzw. nichttranfizierten A549 Zellen

Nach i.v. Applikation von 7-30 mg/kg verschiedener ADCs wurden die Plasma- und Tumorkonzentrationen von ADC sowie potentiell auftretenden Metaboliten gemessen und die pharmakokinetischen Parameter wie Clearance (CL), Fläche unter der Kurve (AUC) und Halbwertszeit (t_{1/2}) berechnet.

### Analytik zur Quantifizierung der potentiell auftretenden Metabolite

Die Messung der Verbindungen in Plasma und Tumor erfolgte nach Fällung der Proteine mit Methanol durch eine Hochdruck-Flüsigkeits-Chromatographie (IIPLC) gekoppelt an ein Tandem-Massenspektrometer (MS).

Zur Aufarbeitung von 100 µL Plasma wurden diese mit 400 µL Methanol und 10 µL internem Standard (ISTD, 50 ng/mL in Methanol) versetzt und für 10 Sekunden geschüttelt. Nach dem 5minütigen Zentrifugieren bei 16000 g wurden 250 µL Überstand in ein Autosampler-Vial überführt, mit 250 µL Ammoniumacetat-Puffer (AAC, 10 mM, pH 6.8) aufgefüllt und erneut geschüttelt.

Bei der Aufarbeitung eines Tumors wurde dieser mit der 4fachen Menge an Methanol versetzt. Im Tissuelyser II (Quiagen) wurde die Probe 6 Minuten bei 30 Schlägen pro Minute zerkleinert und anschließend 5 Minuten bei 16000 g abzentrifugiert. 50 µL des Überstandes wurde in ein Autosampler-Vial überfuhrt und mit 50 µL Ammoniumacetat-Puffer (10 mM, pH 6.8), sowie 5 µL ISTD aufgefüllt. Nach erneutem Schütteln war die Tumorprobe messfertig.

Die Messung beider Matrixproben erfolgte schließlich an dem mit einer IIPLC gekoppelten atmospheric pressure ionizatition/tandem Massenspektrometer mittels TurboIonspray interface (TISP) auf einem API4000-Gerät der Firma SCIEX.

Die HPLC/LC-MSMS (TISP)-Analytik lief auf einer HP1100-Pumpe (Agilent) mit der Säule Gemini (5µm CI8 110 A, 50 x 3 mm, Phenomenex).

Zur Kalibrierung wurden Plasmaproben mit Konzentrationen von 0.5 ... 2000 µg/L versetzt. Die Nachweisgrenze (LOQ) lag bei ca. 2 µg/L. Der lineare Bereich erstreckte sich von 2 bis 1000 µg/L

Zur Kalibrierung der Tumorproben wurde der Überstand unbehandelter Tumore mit Konzentrationen von 0.5 - 200 µg/L versetzt. Die Nachweisgrenze lag bei 5 µg/L. Der lineare Bereich erstreckte sich von 5 bis 200 µg/L.

Qualitätskontrollen zur Gültigkeitsprüfung enthielten 5 und 50 µg/L, in Plasma zusätzlich 500 µg/L. Die bestimmten Konzentrationen dieser Proben wichen bis zu 20% vom Sollwert ab (Daten nicht angefügt).

### D. Ausführungsbeipiele für pharmazeutische Zusammensetzungen

Die erfindungsgemäßen Verbindungen können folgendermaßen in pharmazeutische Zubereitungen überführt werden:

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, D-PBS, oder einer Formulierung mit Glycin und Natriumchlorid in Citratpuffer unter Zusatz von Polysorbat 80) gelöst. Die Lösung wird steril filtriert und in sterile und pyrogenfreie Injektionsbehältnisse abgefüllt.

### i.v.-Lösung:

Die erfindungsgemäßen Verbindungen können in die angeführten Applikationsformen überführt werden. Dies kann in an sich bekannter Weise durch "Mischen mit" bzw. "Lösen in" inerten, nichttoxischen, pharmazeutisch geeigneten Hilfsstoffen (z.B. Puffersubstanzen, Stabilisatoren, Lösungsvermittler, Konservierungsmittel) geschehen. Z.B können enthalten sein: Aminosäuren (Glycin, Histidin, Methionin, Arginin, Lysin, Leucin, Isoleucin, Threonin, Glutaminsäure, Phenylalanin und weitere), Zucker und verwandte Stoffe (Glucose, Saccharose, Mannitol, Trehalose, Sucrose, Mannose, Lactose, Sorbitol), Glycerin, Natrium-, Kalium, Ammonium-, und Calciumsalze (z.B. Natriumchlorid, Kaliumchlorid oder Dinatriumhydrogenphosphat und v.a. mehr), Acetat/Essigsäure-Puffersysteme, Phosphatpuffersysteme, Zitronensäure u. Citratpuffersysteme, Trometamol (TRIS und TRIS-Salze), Polysorbate (z.B. Polysorbat 80 und Polysorbat 20), Poloxamere (z.B. Poloxamer 188 und Poloxamer 171), Macrogole (PEG-Derivate, z.B. 3350), Triton X-100, EDTA-Salze, Glutathion, Albumine (z.B. human), Harnstoff, Benzylalkohol, Phenol, Chlorocresol, Metacresol, Benzalkoniumchlorid und viele andere mehr.

### Lyophilisat zur späteren Überführung in eine i.v., s.c. oder i.m.-Lösung:

Alternativ können die erfindungsgemäßen Verbindungen in ein stabiles Lyophilisat (e.v. durch Mithilfe von oben genannten Hilfstoffen) überführt werden und vor Applikation mit einem geeigneten Lösungsmittel (z.B. Wasser für Injektionszwecke, isoton. Kochsalzlösung) rekonstituiert und appliziert werden.

### Folgende Aspekte sind ebenfalls Bestandteil der Erfindung

### Aspekte

1. Binder-Wirkstoff Konjugate der allgemeinen Formel (Ia) 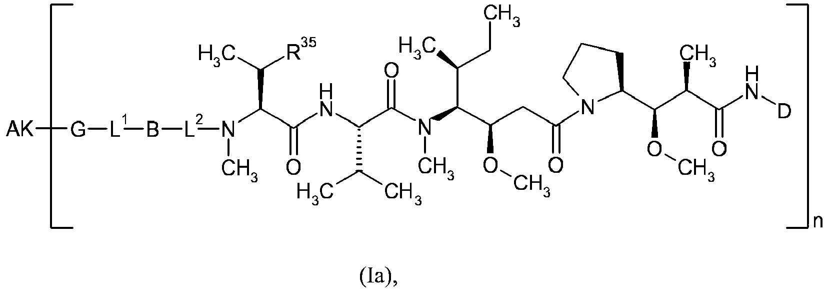 in welcher
   - n: für eine Zahl von 1 bis 50 steht,
   - AK: für einen Binder steht,
   die Gruppe §-G-L¹-B-L²-§§ für einen Linker steht,
   wobei
   § die Verknüpfungsstelle mit der Gruppe AK kennzeichnet und
   §§ die Verknüpfüngsstelle mit dem Stickstoffatom kennzeichnet,
   - D: für eine Gruppe der Formel 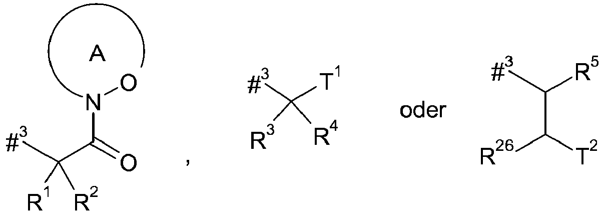 steht, wobei
   #³ die Verknüpfungsstelle mit dem Stickstoffatom kennzeichnet,
   R¹ für Wasserstoff oder Methyl steht,
   R² für Isopropyl, Isobutyl, *sec*.-Butyl, *tert.-*Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenylethyl, Diphenylmethyl, 1*H*-Imidazol-4-ylmethyl oder 1*H*-Indol-3-ylmethyl steht,
   oder
   R¹ und R² zusammen mit dem Kohlenstoffatom, an das sie gebunden sind, eine (1*S*,2*R*)-2-Phenylcyclopropan-1,1-diyl-Gruppe der Formel 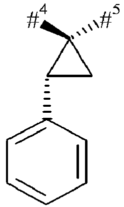 bilden, worin
   #⁴ die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 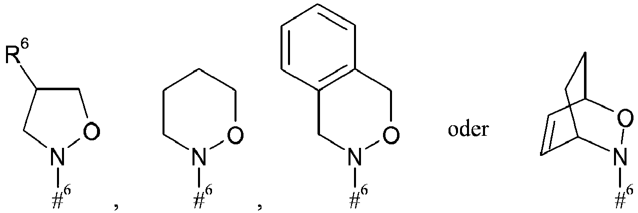 steht, worin
   #⁶ die Verknüpfungsstelle mit der Carbonyl-Gruppe kennzeichnet,
   R⁶ für Wasserstoff, Hydroxy oder Benzyloxy steht,
   R³ für Wasserstoff oder Methyl steht,
   R⁴ für Isopropyl, Isobutyl, *sec*.-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenylethyl, Diphenylmethyl, 1*H-*Imidazol-4-ylmethyl oder 1*H*-Indol-3-ylmethyl steht,
   oder
   R³ und R⁴ zusammen mit dem Kohlenstoffatom, an das sie gebunden sind, eine (1*S*,2*R*)-2-Phenylcyclopropan-1,1-diyl-Gruppe der Formel 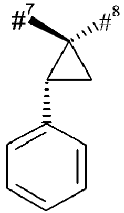 bilden, worin
   #⁷ die Verknüpfungsstelle mit dem angrenzenden Stickstoffatom kennzeichnet,
   #⁸ die Verknüpfungsstelle mit der Gruppe T¹ kennzeichnet,
   T¹ für eine Gruppe der Formel -C(=O)-OR⁷, -C(=O)-NR⁸R⁹, -C(=O)-NH-NH-R¹⁰ oder -CH₂-O-R¹¹ steht,
   worin
   R⁷ für Wasserstoff, Methyl, Ethyl, *n*-Propyl, *tert*.-Butyl, Benzyl oder Adamantylmethyl steht,
   R⁸ für Wasserstoff oder Methyl steht,
   R⁹ für Wasserstoff, Methyl, Ethyl, *n*-Propyl oder Benzyl steht,
   oder
   R⁸ und R⁹ bilden zusammen mit dem Stickstoffatom, an das sie gebunden sind, einen 4- bis 7-gliedrigen Heterocyclus,
   R¹⁰ für Benzoyl steht,
   R¹¹ für Benzyl, das in der Phenylgruppe mit Methoxycarbonyl oder Carboxyl substituiert sein kann, steht,
   R⁵ für Wasserstoff, Methyl oder eine Gruppe der Formel 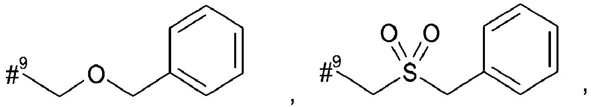 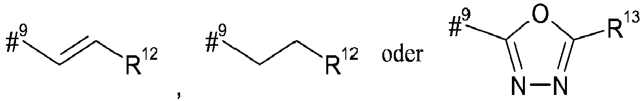 steht, worin
   #⁹ die Verknüpfungsstelle mit -CHC(R²⁶)-T² kennzeichnet,
   R¹² für Phenyl steht, das mit Methoxycarbonyl, Carboxyl oder einer Gruppe der Formel -S(O)₂OH substituiert sein kann,
   R¹³ für Phenyl steht, das mit Methoxycarbonyl oder Carboxyl substituiert sein kann,
   R²⁶ für Wasserstoff oder Hydroxy steht,
   T² für Phenyl, Benzyl, 1*H*-Indol-3-yl oder 1*H*-Indol-3-ylmethyl steht,
   R³⁵ für Methyl oder Hydroxy steht,
   sowie ihre Salze, Solvate und Solvate der Salze.
2. Binder-Wirkstoff Konjugate der allgemeinen Formel (Ia) gemäß Anspruch 1, in welcher
   - n: für eine Zahl von 1 bis 50 steht,
   - AK: für AK₁ oder AK₂ steht
   wobei
   AK₁ für einen Binder, der über ein Schwefelatom des Binders an die Gruppe G gebunden ist,
   AK₂ für einen Binder, der über ein Stickstoffatom des Binders an die Gruppe G gebunden ist,
   - G: für den Fall, dass AK = AK₁ ist, für eine Gruppe der Formel 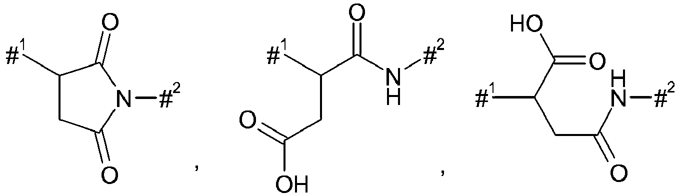 oder 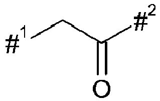 steht, wobei
   #¹ die Verknüpfungsstelle mit dem Schwefelatom des Binders kennzeichnet,
   #² die Verknüpfungsstelle mit der Gruppe L¹ kennzeichnet,
   oder
   für den Fall, dass AK = AK₂ ist, für Carbonyl steht,
   - L¹: für eine Bindung, lineares (C₁-C₁₀)-Alkandiyl, eine Gruppe der Formel 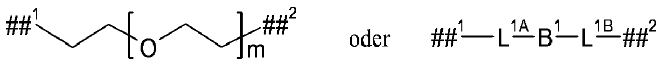 steht, wobei
   m für eine Zahl von 2 bis 6 steht,
   ##¹ die Verknüpfungsstelle mit der Gruppe G kennzeichnet,
   ##² die Verknüpfungsstelle mit der Gruppe B kennzeichnet,
   L^{1A} für lineares (C₂-C₁₀)-Alkandiyl steht,
   B¹ für eine Gruppe der Formel 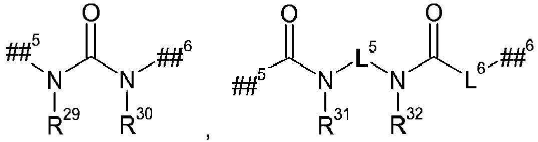 oder 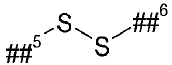 steht, worin
   ##⁵ die Verknüpfungsstelle mit der Gruppe L^{1A} kennzeichnet,
   ##⁶ die Verknüpfungsstelle mit der Gruppe L^{1B} kennzeichnet,
   L⁵ für eine Bindung oder (C₂-C₄)-Alkandiyl steht,
   L⁶ für eine Bindung oder eine Gruppe der Formel 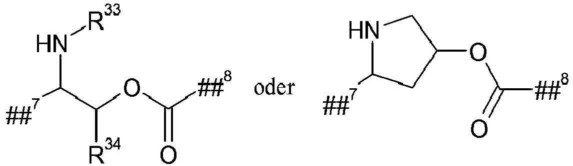 steht, worin
   ##⁷ die Verknüpfungsstelle mit der Carbonylgruppe kennzeichnet,
   ##⁸ die Verknüpfungsstelle mit L^{1B} kennzeichnet,
   R³³ für Wasserstoff, (C₁-C₄)-Alkylcarbonyl, tert.-Butyloxy-carbonyl oder Benzyloxycarbonyl steht,
   R³⁴ für Wasserstoff oder Methyl steht,
   R²⁹ für Wasserstoff oder (C₁-C₄)-Alkyl steht,
   R³⁰ für Wasserstoff oder (C₁-C₄)-Alkyl steht,
   oder
   R²⁹ und R³⁰ zusammen mit den Atomen, an die sie gebunden sind, einen 5- oder 6-gliedrigen Heterocyclus bilden,
   R³¹ für Wasserstoff oder (C₁-C₄)-Alkyl steht,
   R³² für Wasserstoff oder (C₁-C₄)-Alkyl steht,
   oder
   R³¹ und R³² zusammen mit den Atomen, an die sie gebunden sind, einen 5- oder 6-gliedrigen Heterocyclus bilden,
   L^{1B} für lineares (C₂-C₁₀)-Alkandiyl steht,
   und
   wobei (C₁-C₁₀)-Alkandiyl mit 1 bis 4 Substituenten unabhängig voneinander ausgewählt aus der Gruppe Methyl, Hydroxy und Benzyl substituiert sein kann,
   und
   wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
   - B: für eine Bindung oder eine Gruppe der Formel 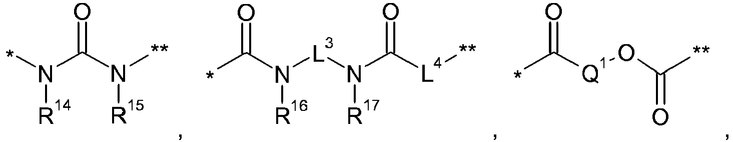 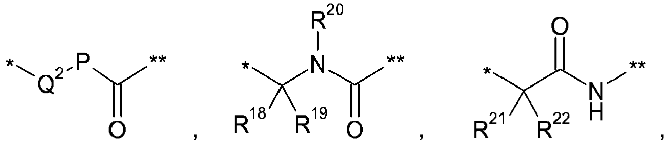 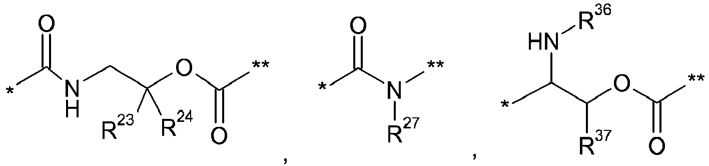 oder 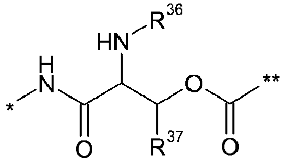 steht, wobei
   * die Verknüpfungsstelle mit L¹ kennzeichnet,
   ** die Verknüpfungsstelle mit L² kennzeichnet,
   P für O oder NH steht,
   L³ für eine Bindung oder (C₂-C₄)-Alkandiyl steht,
   L⁴ für eine Bindung oder eine Gruppe der Formel 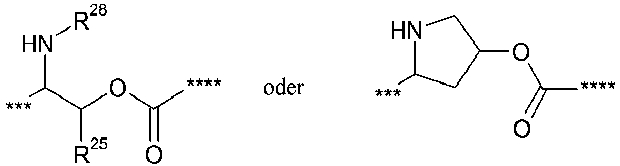 steht, worin
   *** die Verknüpfungsstelle mit der Carbonylgruppe kennzeichnet,
   **** die Verknüpfungsstelle mit L² kennzeichnet,
   R²⁵ für Wasserstoff oder Methyl steht,
   R²⁸ für Wasserstoff, (C₁-C₄)-Alkylcarbonyl, tert.-Butyloxycarbonyl oder Benzyloxycarbonyl steht,
   Q¹ für einen 4- bis 7-gliedrigen Heterocyclus steht,
   Q² für einen 3- bis 7-gliedrigen Carbocyclus oder einen 4- bis 7-gliedrigen Heterocyclus steht,
   R¹⁴ für Wasserstoff oder (C₁-C₄)-Alkyl steht,
   R¹⁵ für Wasserstoff oder (C₁-C₄)-Alkyl steht,
   oder
   R¹⁴ und R¹⁵ zusammen mit den Atomen, an die sie gebunden sind, einen 5- oder 6-gliedrigen Heterocyclus bilden,
   R¹⁶ für Wasserstoff oder (C₁-C₄)-Alkyl steht,
   R¹⁷ für Wasserstoff oder (C₁-C₄)-Alkyl steht,
   oder
   R¹⁶ und R¹⁷ zusammen mit den Atomen, an die sie gebunden sind, einen 5- oder 6-gliedrigen Heterocyclus bilden,
   R¹⁸ für Wasserstoff oder (C₁-C₄)-Alkyl steht,
   R¹⁹ für Wasserstoff oder die Seitengruppe einer natürlichen α-Aminosäure oder ihrer Homologe oder Isomere steht,
   R²⁰ für Wasserstoff oder (C₁-C₄)-Alkyl steht,
   oder
   R¹⁹ und R²⁰ zusammen mit den Atomen, an die sie gebunden sind, einen Pyrrolidinylring bilden,
   R²¹ für Wasserstoff oder (C₁-C₄)-Alkyl steht,
   R²² für Wasserstoff oder (C₁-C₄)-Alkyl steht,
   oder
   R²¹ und R²² zusammen mit den Atomen, an die sie gebunden sind, einen 3- bis 7-gliedrigen Carbocyclus bilden,
   R²³ für (C₁-C₄)-Alkyl steht,
   R²⁴ für Wasserstoff oder (C₁-C₄)-Alkyl steht,
   R²⁷ für Wasserstoff oder (C₁-C₄)-Alkyl steht,
   R³⁶ für Wasserstoff, (C₁-C₄)-Alkylcarbonyl, tert.-Butyloxycarbonyl oder Benzyloxycarbonyl steht,
   R³⁷ für Wasserstoff oder Methyl steht,
   oder
   R³⁶ und R³⁷ bilden zusammen mit den Atomen, an die sie gebunden sind, einen Pyrrolidinring,
   - L²: für lineares (C₂-C₁₀)-Alkandiyl oder für eine Gruppe der Formel 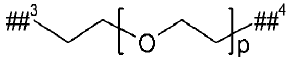 steht, wobei
   p für eine Zahl von 2 bis 6 steht,
   ##³ die Verknüpfungsstelle mit der Gruppe B kennzeichnet,
   ##⁴ die Verknüpfungsstelle mit dem Stickstoffatom kennzeichnet,
   wobei (C₂-C₁₀)-Alkandiyl mit 1 bis 4 Substituenten unabhängig voneinander ausgewählt aus der Gruppe Methyl, Hydroxy und Benzyl substituiert sein kann,
   und
   wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
   D für eine Gruppe der Formel 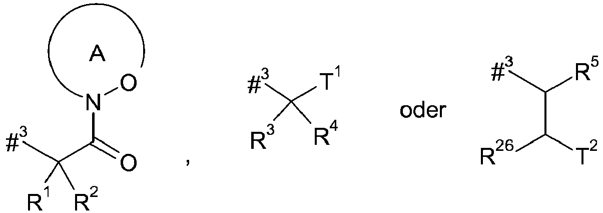 steht, wobei
   #³ die Verknüpfungsstelle mit dem Stickstoffatom kennzeichnet,
   R¹ für Wasserstoff oder Methyl steht,
   R² für Isopropyl, Isobutyl, *sec*.-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenylethyl, Diphenylmethyl, 1*H-*Imidazol-4-ylmethyl oder 1*H*-Indol-3-ylmethyl steht,
   oder
   R¹ und R² zusammen mit dem Kohlenstoffatom, an das sie gebunden sind, eine (1*S*,2*R*)-2-Phenylcyclopropan-1,1-diyl-Gruppe der Formel 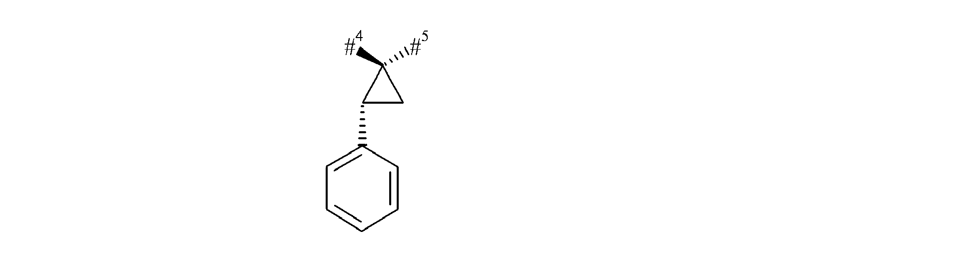 bilden, worin
   #⁴ die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 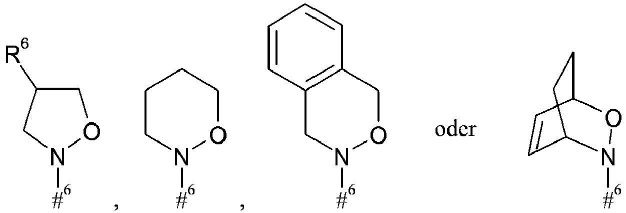 steht, worin
   #⁶ die Verknüpfungsstelle mit der Carbonyl-Gruppe kennzeichnet,
   R⁶ für Wasserstoff, Hydroxy oder Benzyloxy steht,
   R³ für Wasserstoff oder Methyl steht,
   R⁴ für Isopropyl, Isobutyl, *sec*.-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenylethyl, Diphenylmethyl, 1*H-*Imidazol-4-ylmethyl oder 1*H*-Indol-3-ylmethyl steht,
   oder
   R³ und R⁴ zusammen mit dem Kohlenstoffatom, an das sie gebunden sind, eine (1*S*,2*R*)-2-Phenylcyclopropan-1,1-diyl-Gruppe der Formel 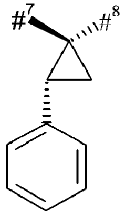 bilden, worin
   #⁷ die Verknüpfungsstelle mit dem angrenzenden Stickstoffatom kennzeichnet,
   #⁸ die Verknüpfungsstelle mit der Gruppe T¹ kennzeichnet,
   T¹ für eine Gruppe der Formel -C(=O)-OR⁷, -C(=O)-NR⁸R⁹, -C(=O)-NH-NH-R¹⁰ oder -CH₂-O-R¹¹ steht, worin
   R⁷ für Wasserstoff, Methyl, Ethyl, *n*-Propyl, *tert*.-Butyl, Benzyl oder Adamantylmethyl steht,
   R⁸ für Wasserstoff oder Methyl steht,
   R⁹ für Wasserstoff, Methyl, Ethyl, *n*-Propyl oder Benzyl steht,
   oder
   R⁸ und R⁹ bilden zusammen mit dem Stickstoffatom, an das sie gebunden sind, einen 4- bis 7-gliedrigen Heterocyclus,
   R¹⁰ für Benzoyl steht,
   R¹¹ für Benzyl, das in der Phenylgruppe mit Methoxycarbonyl oder Carboxyl substituiert sein kann, steht,
   R⁵ für Wasserstoff, Methyl oder eine Gruppe der Formel 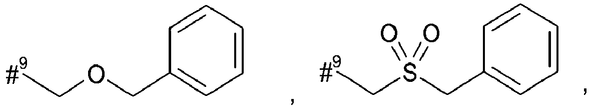 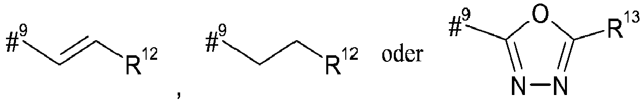 steht, worin
   #⁹ die Verknüpfungsstelle mit -CHC(R²⁶)-T² kennzeichnet,
   R¹² für Phenyl steht, das mit Methoxycarbonyl, Carboxyl oder einer Gruppe der Formel -S(O)₂OH substituiert sein kann,
   R¹³ für Phenyl steht, das mit Methoxycarbonyl oder Carboxyl substituiert sein kann,
   R²⁶ für Wasserstoff oder Hydroxy steht,
   T² für Phenyl, Benzyl, 1*H*-Indol-3-yl oder 1*H*-Indol-3-ylmethyl steht,
   - R³⁵: für Methyl oder Hydroxy steht,
   sowie ihre Salze, Solvate und Solvate der Salze.
3. Binder-Wirkstoff Konjugate der allgemeinen Formel (Ia) gemäß Anspruch 1, in welcher
   - n: für eine Zahl von 1 bis 20 steht,
   - AK: für AK₁ oder AK₂ steht wobei
   AK₁ für einen Antikörper oder ein Antigen-bindendes Antikörperfragment steht, der bzw. das an C4.4a bindet und über das Schwefelatom eines Cystein-Restes des Binders an die Gruppe G gebunden ist,
   AK₂ für einen Antikörper oder ein Antigen-bindendes Antikörperfragment steht, der bzw. das an C4.4a bindet und über die NH-Seitengruppe eines Lysin-Restes des Binders an die Gruppe G gebunden ist,
   - G: für den Fall, dass AK = AK₁ ist, für eine Gruppe der Formel 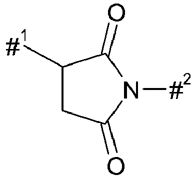 steht, wobei
   #¹ die Verknüpfungsstelle mit dem Cystein-Rest des Binders kennzeichnet,
   #² die Verknüpfungsstelle mit der Gruppe L¹ kennzeichnet,
   oder
   L¹ für den Fall, dass AK = AK₂ ist, für Carbonyl steht, für eine Bindung, lineares (C₂-C₆)-Alkandiyl, eine Gruppe der Formel 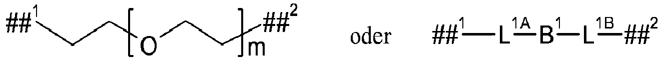 steht, wobei
   m für eine Zahl von 2 bis 6 steht,
   ##¹ die Verknüpfungsstelle mit der Gruppe G kennzeichnet,
   ##² die Verknüpfungsstelle mit der Gruppe B kennzeichnet,
   L^{1A} für lineares (C₂-C₆)-Alkandiyl steht,
   B¹ für eine Gruppe der Formel 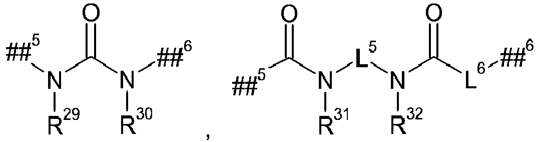 oder 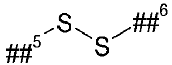 steht, worin
   ##⁵ die Verknüpfungsstelle mit der Gruppe L^{1A} kennzeichnet,
   ##⁶ die Verknüpfungsstelle mit der Gruppe L^{1B} kennzeichnet,
   L⁵ für eine Bindung steht,
   L⁶ für eine Bindung oder eine Gruppe der Formel 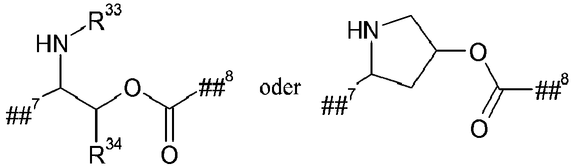 steht, worin
   ##⁷ die Verknüpfungsstelle mit der Carbonylgruppe kennzeichnet,
   ##⁸ die Verknüpfungsstelle mit L^{1B} kennzeichnet,
   R³³ für Wasserstoff, Methylcarbonyl oder tert.-Butyloxy- carbonyl steht,
   R³⁴ für Wasserstoff oder Methyl steht,
   R²⁹ für Wasserstoff steht,
   R³⁰ für Wasserstoff steht,
   R³¹ für Wasserstoff oder Methyl steht,
   R³² für Wasserstoff oder Methyl steht,
   L^{1B} für lineares (C₂-C₆)-Alkandiyl steht,
   und
   wobei (C₂-C₆)-Alkandiyl mit 1 oder 2 Substituenten Methyl substituiert sein kann,
   B für eine Bindung oder eine Gruppe der Formel 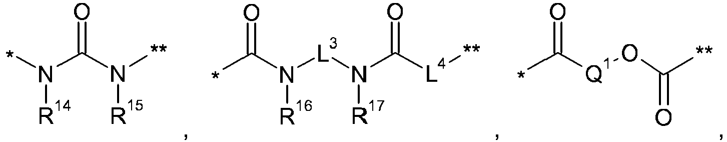 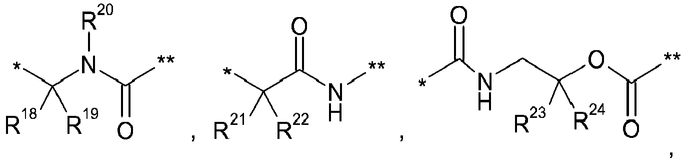 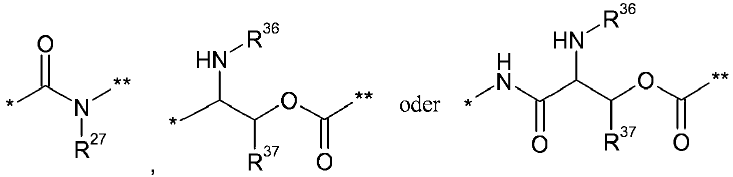 steht, wobei
   * die Verknüpfungsstelle mit L¹ kennzeichnet,
   ** die Verknüpfungsstelle mit L² kennzeichnet,
   L³ für eine Bindung oder Ethan-1,2-diyl steht,
   L⁴ für eine Bindung oder eine Gruppe der Formel 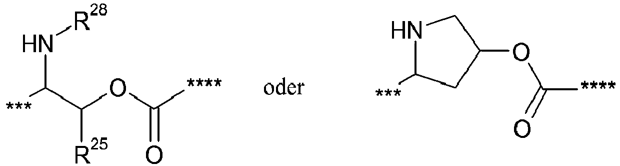 steht, worin
   *** die Verknüpfungsstelle mit der Carbonylgruppe kennzeichnet,
   **** die Verknüpfungsstelle mit L² kennzeichnet,
   R²⁵ für Wasserstoff oder Methyl steht,
   R²⁸ für Wasserstoff, Methylcarbonyl oder tert.-Butyloxycarbonyl steht,
   Q¹ für einen 4- bis 7-gliedrigen Heterocyclus steht,
   R¹⁴ für Wasserstoff steht,
   R¹⁵ für Wasserstoff steht,
   R¹⁶ für Wasserstoff oder Methyl steht,
   R¹⁷ für Wasserstoff oder Methyl steht,
   oder
   R¹⁶ und R¹⁷ zusammen mit den Atomen, an die sie gebunden sind, einen Piperazinylring bilden,
   R¹⁸ für Wasserstoff steht,
   R¹⁹ für Wasserstoff, Methyl, Propan-2-yl, 2-Methylpropan-1-yl oder 1-Methylpropan-1-yl steht,
   R²⁰ für Wasserstoff oder Methyl steht,
   oder
   R¹⁹ und R²⁰ zusammen mit den Atomen, an die sie gebunden sind, einen Pyrrolidinylring bilden,
   R²¹ für Wasserstoff oder Methyl steht,
   R²² für Wasserstoff oder Methyl steht,
   oder
   R²¹ und R²² zusammen mit den Atomen, an die sie gebunden sind, einen Cyclopropylring bilden,
   R²³ für Methyl steht,
   R²⁴ für Wasserstoff oder Methyl steht,
   R²⁷ für Wasserstoff steht,
   R³⁶ für Wasserstoff, Methylcarbonyl oder tert.-Butyloxycarbonyl steht,
   R³⁷ für Wasserstoff oder Methyl steht,
   oder
   R³⁶ und R³⁷ bilden zusammen mit den Atomen, an die sie gebunden sind, einen Pyrrolidinring,
   L² für lineares (C₂-C₆)-Alkandiyl oder für eine Gruppe der Formel 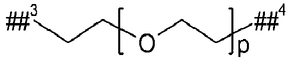 steht, wobei
   p für eine Zahl von 2 bis 6 steht,
   ##³ die Verknüpfungsstelle mit der Gruppe B kennzeichnet,
   ##⁴ die Verknüpfungsstelle mit dem Stickstoffatom kennzeichnet,
   wobei (C₂-C₁₀)-Alkandiyl mit 1 oder 2 Substituenten Methyl substituiert sein kann,
   D für eine Gruppe der Formel 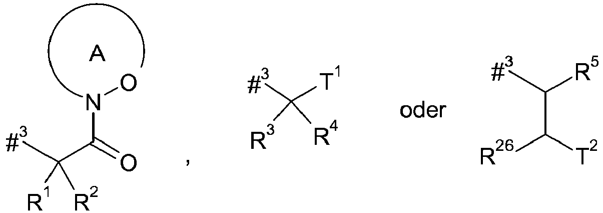 steht, wobei
   #³ die Verknüpfungsstelle mit dem Stickstoffatom kennzeichnet,
   R¹ für Wasserstoff steht,
   R² für 1-Hydroxyethyl, Benzyl, 4-Hydroxybenzyl, 1-Phenylethyl oder 1*H*-Indol-3-ylmethyl steht,
   oder
   R¹ und R² zusammen mit dem Kohlenstoffatom, an das sie gebunden sind, eine (1*S*,2*R*)-2-Phenylcyclopropan-1,1-diyl-Gruppe der Formel 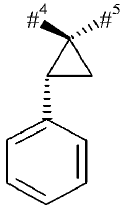 bilden, worin
   #⁴ die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 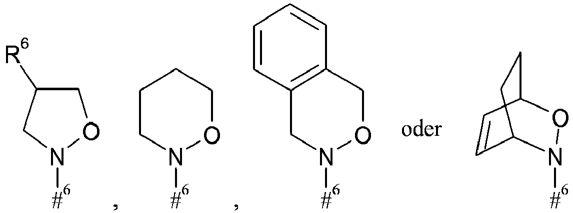 steht, worin
   #⁶ die Verknüpfungsstelle mit der Carbonyl-Gruppe kennzeichnet,
   R⁶ für Wasserstoff, Hydroxy oder Benzyloxy steht,
   R³ für Wasserstoff steht,
   R⁴ für 1-Hydroxyethyl, Benzyl, 4-Hydroxybenzyl, 1-Phenylethyl oder 1*H*-Indol-3-ylmethyl steht,
   oder
   R³ und R⁴ zusammen mit dem Kohlenstoffatom, an das sie gebunden sind, eine (1*S*,2*R*)-2-Phenylcyclopropan-1,1-diyl-Gruppe der Formel 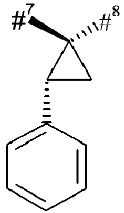 bilden, worin
   #⁷ die Verknüpfungsstelle mit dem angrenzenden Stickstoffatom kennzeichnet,
   #⁸ die Verknüpfungsstelle mit der Gruppe T¹ kennzeichnet,
   T¹ für eine Gruppe der Formel -C(=O)-OR, -C(=O)-NR⁸R⁹, -C(=O)-NH-NH-R¹⁰ oder -CH₂-O-R¹¹ steht,
   worin
   R⁷ für Wasserstoff, Methyl, Ethyl, *n*-Propyl, *tert*.-Butyl, Benzyl oder Adamantylmethyl steht,
   R⁸ für Wasserstoff oder Methyl steht,
   R⁹ für Wasserstoff, Methyl, Ethyl, *n*-Propyl oder Benzyl steht,
   oder
   R⁸ und R⁹ bilden zusammen mit dem Stickstoffatom, an das sie gebunden sind, einen 4- bis 7-gliedrigen Heterocyclus,
   R¹⁰ für Benzoyl steht,
   R¹¹ für Benzyl, das in der Phenylgruppe mit Methoxycarbonyl oder Carboxyl substituiert sein kann, steht,
   R⁵ für Wasserstoff, Methyl oder eine Gruppe der Formel 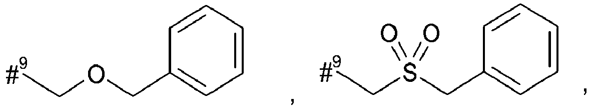 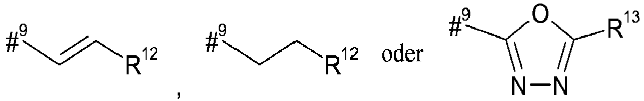 steht, worin
   #⁹ die Verknüpfungsstelle mit -CHC(R²⁶)-T² kennzeichnet,
   R¹² für Phenyl steht, das mit Methoxycarbonyl, Carboxyl oder einer Gruppe der Formel -S(O)₂OH substituiert sein kann,
   R¹³ für Phenyl steht, das mit Methoxycarbonyl oder Carboxyl substituiert sein kann,
   R²⁶ für Wasserstoff oder Hydroxy steht,
   T² für Phenyl, Benzyl, 1*H*-Indol-3-yl oder 1*H*-Indol-3-ylmethyl steht,
   R³⁵ für Methyl oder Hydroxy steht,
   sowie ihre Salze, Solvate und Solvate der Salze.
4. Binder-Wirkstoff Konjugate der allgemeinen Formel (Ia) gemäß einem der Aspekte 1 bis 2, in welcher
   - n: für eine Zahl von 1 bis 10 steht,
   - AK: für AK₁ oder AK₂ steht
   wobei
   AK₁ für einen Antikörper, der die sechs CDR-Sequenzen des Antikörpers B01-3, B01-10, M31-B01 oder D02-6, die variable leichte und variable schwere Kette des Antikörpers B01-3, B01-10, M31-B01 oder D02-6 oder die leichte und schwere Kette des Antiköpers B01-3, B01-10, M31-B01 oder D02-6 umfasst, steht, der über das Schwefelatom eines Cystein-Restes des Binders an die Gruppe G gebunden ist,
   AK₂ für einen Antikörper, der die sechs CDR-Sequenzen des Antikörpers B01-3, B01-10, M31-B01 oder D02-6, die variable leichte und variable schwere Kette des Antikörpers B01-3, B01-10, M31-B01 oder D02-6 oder die leichte und schwere Kette des Antiköpers B01-3, B01-10, M31-B01 oder D02-6 umfasst, steht, der über die NH-Seitengruppe eines Lysin-Restes des Binders an die Gruppe G gebunden ist,
   - G: für den Fall, dass AK = AK₁ ist, für eine Gruppe der Formel 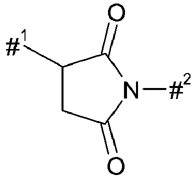 steht, wobei
   #¹ die Verknüpfungsstelle mit dem Cystein-Rest des Binders kennzeichnet,
   #² die Verknüpfungsstelle mit der Gruppe L¹ kennzeichnet,
   oder
   für den Fall, dass AK = AK₂ ist, für Carbonyl steht,
   L¹ für eine Bindung, lineares (C₂-C₆)-Alkandiyl, eine Gruppe der Formel 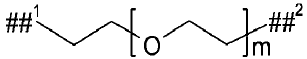 steht, wobei
   m für eine Zahl von 2 oder 3 steht,
   ##¹ die Verknüpfungsstelle mit der Gruppe G kennzeichnet,
   ##² die Verknüpfungsstelle mit der Gruppe B kennzeichnet,
   wobei (C₂-C₆)-Alkandiyl mit 1 oder 2 Substituenten Methyl substituiert sein kann,
   B für eine Bindung oder eine Gruppe der Formel 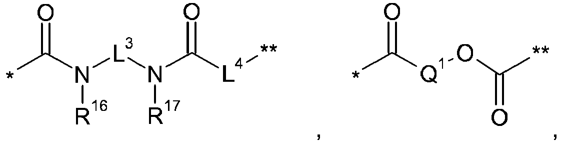 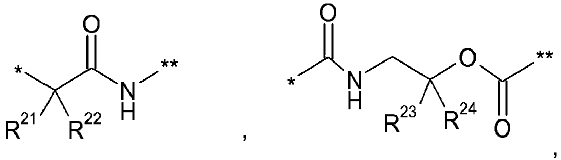 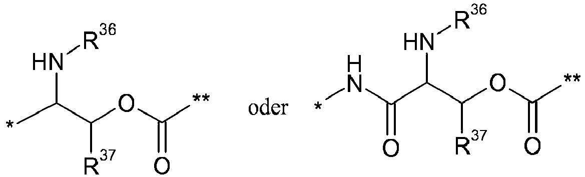 steht, wobei
   * die Verknüpfungsstelle mit L¹ kennzeichnet,
   ** die Verknüpfungsstelle mit L² kennzeichnet,
   L³ für eine Bindung oder Ethan-1,2-diyl steht,
   L⁴ für eine Bindung oder eine Gruppe der Formel 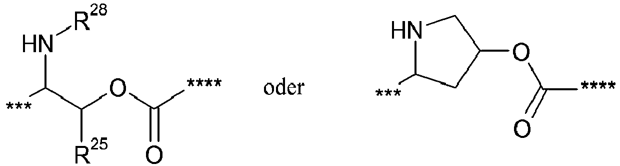 steht, worin
   *** die Verknüpfungsstelle mit der Carbonylgruppe kennzeichnet,
   **** die Verknüpfungsstelle mit L² kennzeichnet,
   R²⁵ für Methyl steht,
   R²⁸ für Wasserstoff, Methylcarbonyl oder tert.-Butyloxycarbonyl steht,
   Q¹ für Piperidin-1,4-diyl steht,
   R¹⁶ für Wasserstoff oder Methyl steht,
   R¹⁷ für Wasserstoff oder Methyl steht,
   oder
   R¹⁶ und R¹⁷ zusammen mit den Atomen, an die sie gebunden sind, einen Piperazinylring bilden,
   R²¹ für Wasserstoff oder Methyl steht,
   R²² für Wasserstoff oder Methyl steht,
   oder
   R²¹ und R²² zusammen mit den Atomen, an die sie gebunden sind, einen Cyclopropylring bilden,
   R²³ für Methyl steht,
   R²⁴ für Wasserstoff steht,
   R³⁶ für Wasserstoff, Methylcarbonyl oder tert.-Butyloxycarbonyl steht,
   R³⁷ für Wasserstoff oder Methyl steht,
   L² für lineares (C₂-C₆)-Alkandiyl oder für eine Gruppe der Formel 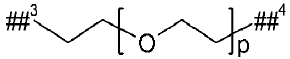 steht, wobei
   p für eine Zahl von 2 bis 6 steht,
   ##³ die Verknüpfungsstelle mit der Gruppe B kennzeichnet,
   ##⁴ die Verknüpfungsstelle mit dem Stickstoffatom kennzeichnet,
   D für eine Gruppe der Formel 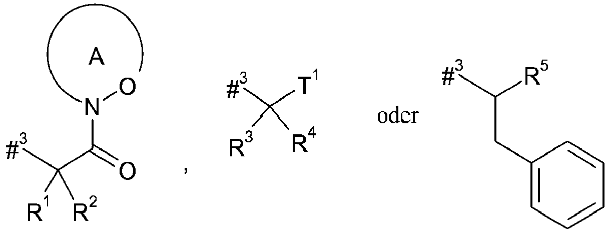 steht, wobei
   #³ die Verknüpfungsstelle mit dem Stickstoffatom kennzeichnet,
   R¹ für Wasserstoff steht,
   R² für 1-Hydroxyethyl, Benzyl, 4-Hydroxybenzyl, 1-Phenylethyl oder 1*H*-Indol-3-ylmethyl steht,
   oder
   R¹ und R² zusammen mit dem Kohlenstoffatom, an das sie gebunden sind, eine (1*S*,2*R*)-2-Phenylcyclopropan-1,1-diyl-Gruppe der Formel 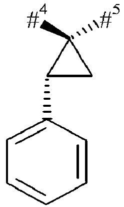 bilden, worin
   #⁴ die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 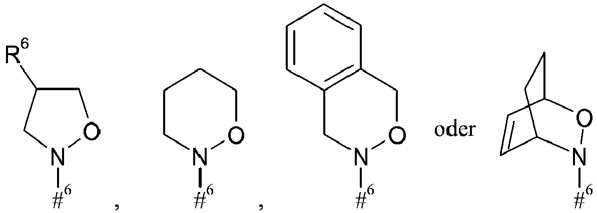 steht, worin
   #⁶ die Verknüpfungsstelle mit der Carbonyl-Gruppe kennzeichnet,
   R⁶ für Wasserstoff, Hydroxy oder Benzyloxy steht,
   R³ für Wasserstoff steht,
   R⁴ für Benzyl, 4-Hydroxybenzyl, 1-Phenylethyl oder 1*H*-Indol-3-ylmethyl steht,
   oder
   R³ und R⁴ zusammen mit dem Kohlenstoffatom, an das sie gebunden sind, eine (1*S*,2*R*)-2-Phenylcyclopropan-1,1-diyl-Gruppe der Formel 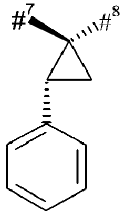 bilden, worin
   #⁷ die Verknüpfungsstelle mit dem angrenzenden Stickstoffatom kennzeichnet,
   #⁸ die Verknüpfungsstelle mit der Gruppe T¹ kennzeichnet,
   T¹ für eine Gruppe der Formel -C(=O)-OR⁷, -C(=O)-NR⁸R⁹ oder -CH₂-O-R¹¹ steht,
   worin
   R⁷ für Wasserstoff, Methyl, Ethyl, *n*-Propyl, *tert*.-Butyl, Benzyl oder Adamantylmethyl steht,
   R⁸ für Wasserstoff oder Methyl steht,
   R⁹ für Wasserstoff, Methyl, Ethyl, *n*-Propyl oder Benzyl steht,
   R¹¹ für Benzyl, das in der Phenylgruppe mit Methoxycarbonyl oder Carboxyl substituiert sein kann, steht,
   R⁵ für Wasserstoff, Methyl oder eine Gruppe der Formel 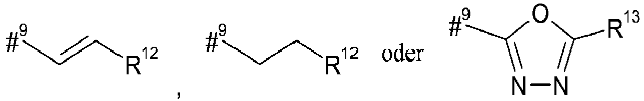 steht, worin
   #⁹ die Verknüpfungsstelle mit -CHCH₂Phenyl kennzeichnet,
   R¹² für Phenyl steht, das mit Methoxycarbonyl, Carboxyl oder einer Gruppe der Formel -S(O)₂OH substituiert sein kann,
   R¹³ für Phenyl steht, das mit Methoxycarbonyl oder Carboxyl substituiert sein kann,
   R³⁵ für Methyl oder Hydroxy steht,
   sowie ihre Salze, Solvate und Solvate der Salze.
5. Binder-Wirkstoff Konjugate der allgemeinen Formel (Ia) gemäß einem der Aspekte Aspekten 1 bis 3, in welcher
   - n: für eine Zahl von 1 bis 10 steht,
   - AK: für AK₂ steht,
   wobei
   AK₂ für einen Antikörper, der die sechs CDR-Sequenzen des Antikörpers B01-3, B01-10, M31-B01 oder D02-6, die variable leichte und variable schwere Kette des Antikörpers B01-3, B01-10, M31-B01 oder D02-6 oder die leichte und schwere Kette des Antiköpers B01-3, B01-10, M31-B01 oder D02-6 umfasst, steht, der über die NH-Seitengruppe eines Lysin-Restes des Binders an die Gruppe G gebunden ist,
   - G: für Carbonyl steht,
   - L¹: für eine Bindung, steht,
   - B: für eine Bindung steht,
   - L²: für lineares (C₃-C₆)-Alkandiyl oder für eine Gruppe der Formel 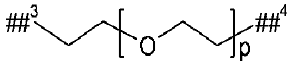 steht, wobei
   p für eine Zahl von 2 oder 3 steht,
   ##³ die Verknüpfungsstelle mit der Gruppe B kennzeichnet,
   ##⁴ die Verknüpfungsstelle mit dem Stickstoffatom kennzeichnet,
   - D: für eine Gruppe der Formel 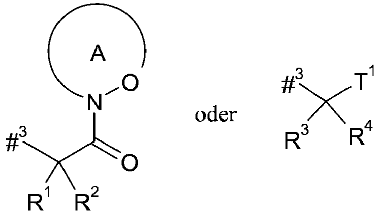 steht, wobei
   #³ die Verknüpfungsstelle mit dem Stickstoffatom kennzeichnet,
   R¹ für Wasserstoff steht,
   R² für Benzyl oder 1*H*-Indol-3-ylmethyl steht,
   oder
   R¹ und R² zusammen mit dem Kohlenstoffatom, an das sie gebunden sind, eine (1*S*,2*R*)-2-Phenylcyclopropan-1,1-diyl-Gruppe der Formel 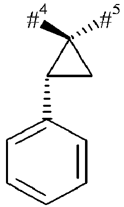 bilden, worin
   #⁴ die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 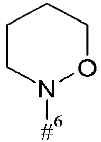 steht, worin
   #⁶ die Verknüpfungsstelle mit der Carbonyl-Gruppe kennzeichnet,
   R³ für Wasserstoff steht,
   R⁴ für Benzyl oder 1*H*-Indol-3-ylmethyl steht,
   oder
   R³ und R⁴ zusammen mit dem Kohlenstoffatom, an das sie gebunden sind, eine (1*S*,2*R*)-2-Phenylcyclopropan-1,1-diyl-Gruppe der Formel 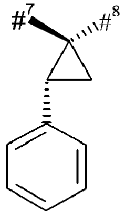 bilden, worin
   #⁷ die Verknüpfungsstelle mit dem angrenzenden Stickstoffatom kennzeichnet,
   #⁸ die Verknüpfungsstelle mit der Gruppe T¹ kennzeichnet,
   T¹ für eine Gruppe der Formel -C(=O)-OR⁷ oder -C(=O)-NR⁸R⁹ steht, worin
   R⁷ für Wasserstoff, Methyl, Ethyl, *n*-Propyl, *tert*.-Butyl, Benzyl oder Adamantylmethyl steht,
   R⁸ für Wasserstoff steht,
   R⁹ für Wasserstoff oder Benzyl steht,
   R³⁵ für Methyl steht,
   sowie ihre Salze, Solvate und Solvate der Salze.
6. Binder-Wirkstoff Konjugate der allgemeinen Formel (Ia) gemäß einem der Aspekte 1 bis 4, in welcher
   - n: für eine Zahl von 1 bis 10 steht,
   - AK: für AK₁ steht,
   wobei
   AK₁ für einen Antikörper, der die sechs CDR-Sequenzen des Antikörpers B01-3, B01-10, M31-B01 oder D02-6, die variable leichte und variable schwere Kette des Antikörpers B01-3, B01-10, M31-B01 oder D02-6 oder die leichte und schwere Kette des Antiköpers B01-3, B01-10, M31-B01 oder D02-6 umfasst, steht, der über das Schwefelatom eines Cystein-Restes des Binders an die Gruppe G gebunden ist,
   - G: für eine Gruppe der Formel 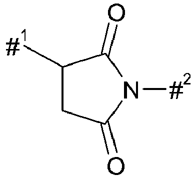 steht, wobei
   #¹ die Verknüpfungsstelle mit dem Cystein-Rest des Binders kennzeichnet,
   #² die Verknüpfungsstelle mit der Gruppe L¹ kennzeichnet,
   - L¹: für eine Bindung, lineares (C₃-C₅)-Alkandiyl oder eine Gruppe der Formel 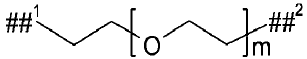 steht, wobei
   m für eine Zahl von 2 oder 3 steht,
   ##¹ die Verknüpfungsstelle mit der Gruppe G kennzeichnet,
   ##² die Verknüpfungsstelle mit der Gruppe B kennzeichnet,
   wobei (C₃-C₅)-Alkandiyl mit 1 oder 2 Substituenten Methyl substituiert sein kann,
   - B: für eine Bindung oder eine Gruppe der Formel 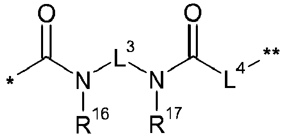 steht, wobei
   * die Verknüpfungsstelle mit L¹ kennzeichnet,
   ** die Verknüpfungsstelle mit L² kennzeichnet,
   L³ für eine Bindung oder Ethan-1,2-diyl steht,
   L⁴ für eine Bindung oder eine Gruppe der Formel 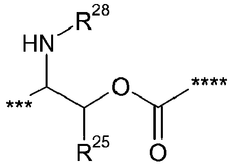 steht, worin
   *** die Verknüpfungsstelle mit der Carbonylgruppe kennzeichnet,
   **** die Verknüpfungsstelle mit L² kennzeichnet,
   R²⁵ für Methyl steht,
   R²⁸ für Wasserstoff, Methylcarbonyl oder tert.-Butyloxycarbonyl steht,
   R¹⁶ für Wasserstoff oder Methyl steht,
   R¹⁷ für Wasserstoff oder Methyl steht,
   oder
   R¹⁶ und R¹⁷ zusammen mit den Atomen, an die sie gebunden sind, einen Piperazinylring bilden,
   - L²: für lineares (C₃-C₅)-Alkandiyl oder für eine Gruppe der Formel 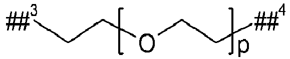 steht, wobei
   p für eine Zahl von 2 oder 3 steht,
   ##³ die Verknüpfungsstelle mit der Gruppe B kennzeichnet,
   ##⁴ die Verknüpfungsstelle mit dem Stickstoffatom kennzeichnet,
   - D: für eine Gruppe der Formel 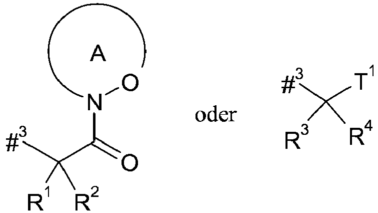 steht, wobei
   #³ die Verknüpfungsstelle mit dem Stickstoffatom kennzeichnet,
   R¹ für Wasserstoff steht,
   R² für Benzyl oder 1*H*-Indol-3-ylmethyl steht,
   oder
   R¹ und R² zusammen mit dem Kohlenstoffatom, an das sie gebunden sind, eine (1*S*,2*R*)-2-Phenylcyclopropan-1,1-diyl-Gruppe der Formel 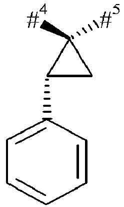 bilden, worin
   #⁴ die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 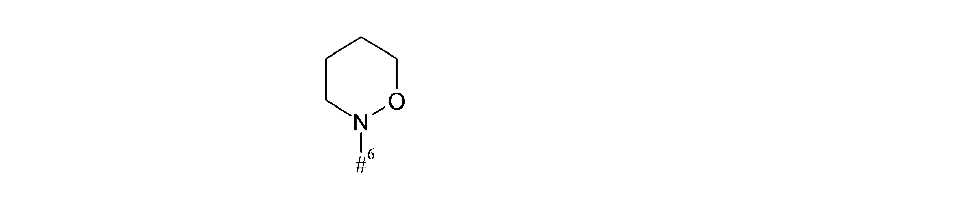 steht, worin
   #⁶ die Verknüpfungsstelle mit der Carbonyl-Gruppe kennzeichnet,
   R³ für Wasserstoff steht,
   R⁴ für Benzyl oder 1*H*-Indol-3-ylmethyl steht,
   oder
   R³ und R⁴ zusammen mit dem Kohlenstoffatom, an das sie gebunden sind, eine (1*S*,2*R*)-2-Phenylcyclopropan-1,1-diyl-Gruppe der Formel 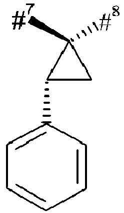 bilden, worin
   #⁷ die Verknüpfungsstelle mit dem angrenzenden Stickstoffatom kennzeichnet,
   #⁸ die Verknüpfungsstelle mit der Gruppe T¹ kennzeichnet,
   T¹ für eine Gruppe der Formel -C(=O)-OR⁷ oder -C(=O)-NR⁸R⁹ steht,
   worin
   R⁷ für Wasserstoff, Methyl, Ethyl, *n*-Propyl, *tert*.-Butyl, Benzyl oder Adamantylmethyl steht,
   R⁸ für Wasserstoff steht,
   R⁹ für Wasserstoff oder Benzyl steht,
   R³⁵ für Methyl steht,
   sowie ihre Salze, Solvate und Solvate der Salze.
7. Verbindungen der Formel (XXXa) 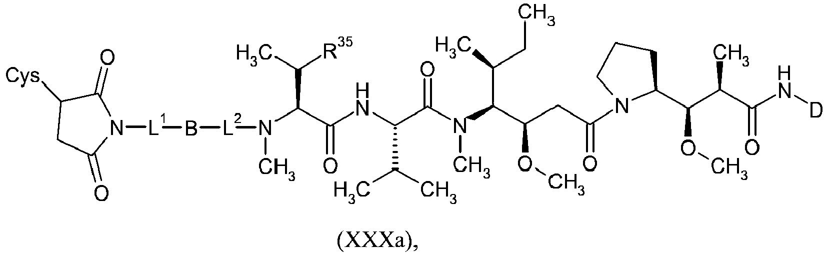 in welcher
   - Cys: für einen Cystein-Rest steht, der über das Schwefelatom der Seitenkette an ein Kohlenstoffatom des Succinimids gebunden ist,
   - L¹: für eine Bindung, lineares (C₁-C₁₀)-Alkandiyl, eine Gruppe der Formel 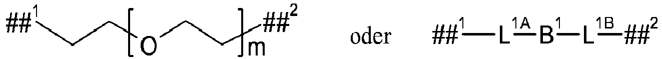 steht, wobei
   - m: für eine Zahl von 2 bis 6 steht,
   - ##¹: die Verknüpfungsstelle mit der Gruppe G kennzeichnet,
   - ##²: die Verknüpfungsstelle mit der Gruppe B kennzeichnet,
   - L^{1A}: für lineares (C₂-C₁₀)-Alkandiyl steht,
   - B¹: für eine Gruppe der Formel 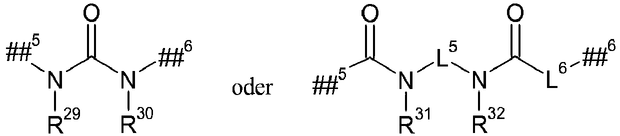 steht, worin
   ##⁵ die Verknüpfungsstelle mit der Gruppe L^{1A} kennzeichnet,
   ##⁶ die Verknüpfungsstelle mit der Gruppe L^{1B} kennzeichnet,
   L⁵ für eine Bindung oder (C₂-C₄)-Alkandiyl steht,
   L⁶ für eine Bindung steht,
   R²⁹ für Wasserstoff oder (C₁-C₄)-Alkyl steht,
   R³⁰ für Wasserstoff oder (C₁-C₄)-Alkyl steht,
   oder
   R²⁹ und R³⁰ zusammen mit den Atomen, an die sie gebunden sind, einen 5- oder 6-gliedrigen Heterocyclus bilden,
   R³¹ für Wasserstoff oder (C₁-C₄)-Alkyl steht,
   R³² für Wasserstoff oder (C₁-C₄)-Alkyl steht,
   oder
   R³¹ und R³² zusammen mit den Atomen, an die sie gebunden sind, einen 5- oder 6-gliedrigen Heterocyclus bilden,
   - L^{1B}: für lineares (C₂-C₁₀)-Alkandiyl steht,
   und
   wobei (C₁-C₁₀)-Alkandiyl mit 1 bis 4 Substituenten unabhängig voneinander ausgewählt aus der Gruppe Methyl, Hydroxy und Benzyl substituiert sein kann,
   und
   wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
   - B: für eine Bindung oder eine Gruppe der Formel 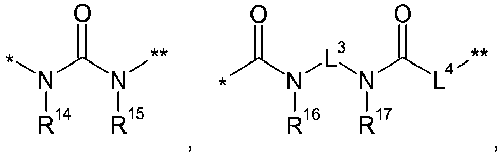 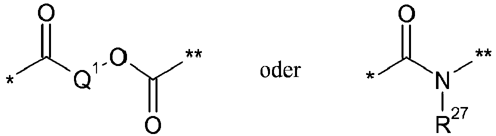 steht, wobei
   * die Verknüpfungsstelle mit L¹ kennzeichnet,
   ** die Verknüpfungsstelle mit L² kennzeichnet,
   P für O oder NH steht,
   L³ für eine Bindung oder (C₂-C₄)-Alkandiyl steht,
   L⁴ für eine Bindung steht,
   Q¹ für einen 4- bis 7-gliedrigen Heterocyclus steht,
   Q² für einen 3- bis 7-gliedrigen Carbocyclus oder einen 4- bis 7-gliedrigen Heterocyclus steht,
   R¹⁴ für Wasserstoff oder (C₁-C₄)-Alkyl steht,
   R¹⁵ für Wasserstoff oder (C₁-C₄)-Alkyl steht,
   oder
   R¹⁴ und R¹⁵ zusammen mit den Atomen, an die sie gebunden sind, einen 5- oder 6-gliedrigen Heterocyclus bilden,
   R¹⁶ für Wasserstoff oder (C₁-C₄)-Alkyl steht,
   R¹⁷ für Wasserstoff oder (C₁-C₄)-Alkyl steht,
   oder
   R¹⁶ und R¹⁷ zusammen mit den Atomen, an die sie gebunden sind, einen 5- oder 6-gliedrigen Heterocyclus bilden,
   R¹⁸ für Wasserstoff oder (C₁-C₄)-Alkyl steht,
   R¹⁹ für Wasserstoff oder die Seitengruppe einer natürlichen α-Aminosäure oder ihrer Homologe oder Isomere steht,
   R²⁰ für Wasserstoff oder (C₁-C₄)-Alkyl steht,
   oder
   R¹⁹ und R²⁰ zusammen mit den Atomen, an die sie gebunden sind, einen Pyrrolidinylring bilden,
   R²¹ für Wasserstoff oder (C₁-C₄)-Alkyl steht,
   R²² für Wasserstoff oder (C₁-C₄)-Alkyl steht,
   oder
   R²¹ und R²² zusammen mit den Atomen, an die sie gebunden sind, einen 3- bis 7-gliedrigen Carbocyclus bilden,
   R²³ für (C₁-C₄)-Alkyl steht,
   R²⁴ für Wasserstoff oder (C₁-C₄)-Alkyl steht,
   R²⁷ für Wasserstoff oder (C₁-C₄)-Alkyl steht,
   - L²: für lineares (C₂-C₁₀)-Alkandiyl oder für eine Gruppe der Formel 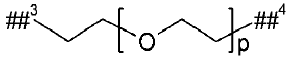 steht, wobei
   p für eine Zahl von 2 bis 6 steht,
   ##³ die Verknüpfungsstelle mit der Gruppe B kennzeichnet,
   ##⁴ die Verknüpfungsstelle mit dem Stickstoffatom kennzeichnet,
   wobei (C₂-C₁₀)-Alkandiyl mit 1 bis 4 Substituenten unabhängig voneinander ausgewählt aus der Gruppe Methyl, Hydroxy und Benzyl substituiert sein kann,
   und
   wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
   - D: für eine Gruppe der Formel 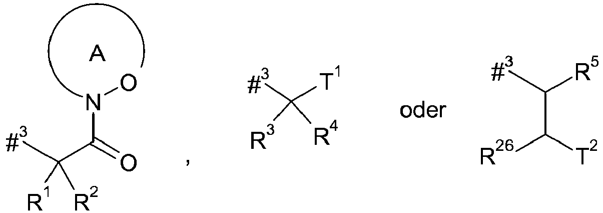 steht, wobei
   #³ die Verknüpfungsstelle mit dem Stickstoffatom kennzeichnet,
   R¹ für Wasserstoff oder Methyl steht,
   R² für Isopropyl, Isobutyl, *sec*.-Butyl, *tert.-Butyl,* Phenyl, Benzyl, 1-Hydroxyethyl, 4-Hydroxybenzyl, 4-Hydroxy-3-nitrobenzyl, 4-Hydroxy-3-aminobenzyl, 1-Phenylethyl, Diphenylmethyl, 1*H*-Imidazol-4-ylmethyl oder 1*H*-Indol-3-ylmethyl steht,
   oder
   R¹ und R² zusammen mit dem Kohlenstoffatom, an das sie gebunden sind, eine (1*S*,2*R*)-2-Phenylcyclopropan-1,1-diyl-Gruppe der Formel 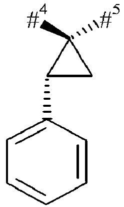 bilden, worin
   #⁴ die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 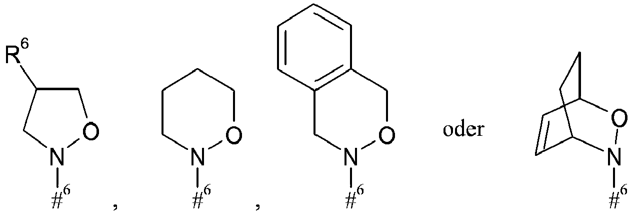 steht, worin
   #⁶ die Verknüpfungsstelle mit der Carbonyl-Gruppe kennzeichnet,
   R⁶ für Wasserstoff, Hydroxy oder Benzyloxy steht,
   R³ für Wasserstoff oder Methyl steht,
   R⁴ für Isopropyl, Isobutyl, *sec*.-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenylethyl, Diphenylmethyl, 1*H*-Imidazol-4-ylmethyl oder 1*H*-Indol-3-ylmethyl steht,
   oder
   R³ und R⁴ zusammen mit dem Kohlenstoffatom, an das sie gebunden sind, eine (1*S*,2*R*)-2-Phenylcyclopropan-1,1-diyl-Gruppe der Formel 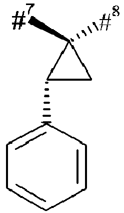 bilden, worin
   #⁷ die Verknüpfungsstelle mit dem angrenzenden Stickstoffatom kennzeichnet,
   #⁸ die Verknüpfungsstelle mit der Gruppe T¹ kennzeichnet,
   T¹ für eine Gruppe der Formel -C(=O)-OR⁷, -C(=O)-NR⁸R⁹, -C(=O)-NH-NH-R¹⁰ oder -CH₂-O-R¹¹ steht,
   worin
   R⁷ für Wasserstoff, Methyl, Ethyl, *n*-Propyl, *tert*.-Butyl, Benzyl oder Adamantylmethyl steht,
   R⁸ für Wasserstoff oder Methyl steht,
   R⁹ für Wasserstoff, Methyl, Ethyl, *n*-Propyl oder Benzyl steht,
   oder
   R⁸ und R⁹ bilden zusammen mit dem Stickstoffatom, an das sie gebunden sind, einen 4- bis 7-gliedrigen Heterocyclus,
   R¹⁰ für Benzoyl steht,
   R¹¹ für Benzyl, das in der Phenylgruppe mit Methoxycarbonyl oder Carboxyl substituiert sein kann, steht,
   R⁵ für Wasserstoff, Methyl oder eine Gruppe der Formel 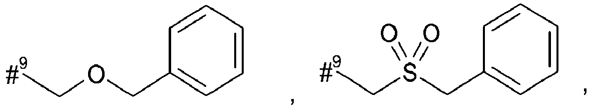 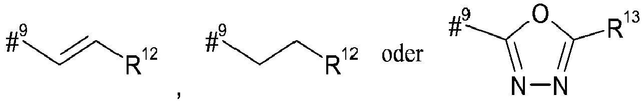 steht, worin
   #⁹ die Verknüpfungsstelle mit -CHC(R²⁶)-T² kennzeichnet,
   R¹² für Phenyl steht, das mit Methoxycarbonyl, Carboxyl oder einer Gruppe der Formel -S(O)₂OH substituiert sein kann,
   R¹³ für Phenyl steht, das mit Methoxycarbonyl oder Carboxyl substituiert sein kann,
   R²⁶ für Wasserstoff oder Hydroxy steht,
   T² für Phenyl, Benzyl, 1*H*-Indol-3-yl oder 1*H*-Indol-3-ylmethyl steht,
   - R³⁵: für Methyl oder Hydroxy steht,
   sowie ihre Salze, Solvate und Solvate der Salze.
8. Verbindungen der Formel (XXXa) 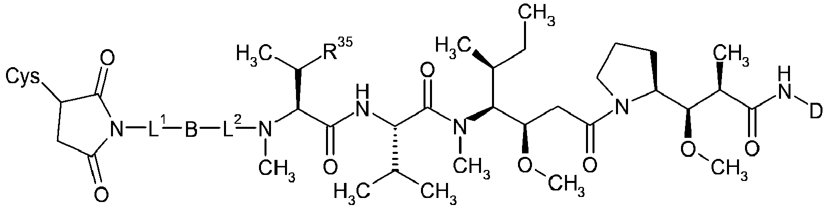 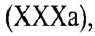 gemäß Anspruch 7, in welcher
   - Cys: für einen Cystein-Rest steht, der über das Schwefelatom der Seitenkette über ein Kohlenstoffatom des Succinimids gebunden ist, steht
   - L¹: für eine Bindung, lineares (C₂-C₆)-Alkandiyl, eine Gruppe der Formel 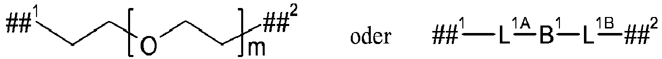 steht, wobei
   m für eine Zahl von 2 oder 3 steht,
   ##¹ die Verknüpfungsstelle mit der Gruppe G kennzeichnet,
   ##² die Verknüpfungsstelle mit der Gruppe B kennzeichnet,
   L^{1A} für lineares (C₂-C₆)-Alkandiyl steht,
   B¹ für eine Gruppe der Formel 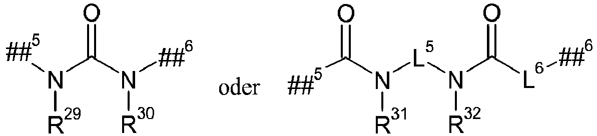 steht, worin
   ##⁵ die Verknüpfungsstelle mit der Gruppe L^{1A} kennzeichnet,
   ##⁶ die Verknüpfungsstelle mit der Gruppe L^{1B} kennzeichnet,
   L⁵ für eine Bindung steht,
   L⁶ für eine Bindung steht,
   R²⁹ für Wasserstoff steht,
   R³⁰ für Wasserstoff steht,
   R³² für Wasserstoff oder Methyl steht, für Wasserstoff oder Methyl steht,
   L^{1B} für lineares (C₂-C₆)-Alkandiyl steht,
   und
   wobei (C₂-C₆)-Alkandiyl mit 1 oder 2 Substituenten Methyl substituiert sein kann,
   B für eine Bindung oder eine Gruppe der Formel 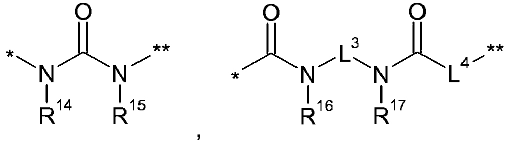 oder 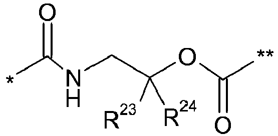 steht, wobei
   * die Verknüpfungsstelle mit L¹ kennzeichnet,
   ** die Verknüpfungsstelle mit L² kennzeichnet,
   L³ für eine Bindung oder Ethan-1,2-diyl steht,
   L⁴ für eine Bindung steht,
   R¹⁴ für Wasserstoff steht,
   R¹⁵ für Wasserstoff steht,
   R¹⁶ für Wasserstoff oder Methyl steht,
   R¹⁷ für Wasserstoff oder Methyl steht,
   oder
   R¹⁶ und R¹⁷ zusammen mit den Atomen, an die sie gebunden sind, Piperazinylring bilden,
   R²³ für Methyl steht,
   R²⁴ für Wasserstoff oder Methyl steht,
   L² für lineares (C₂-C₆)-Alkandiyl oder für eine Gruppe der Formel 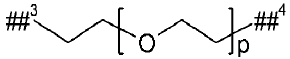 steht, wobei
   p für eine Zahl von 2 oder 3 steht,
   ##³ die Verknüpfungsstelle mit der Gruppe B kennzeichnet,
   ##⁴ die Verknüpfungsstelle mit dem Stickstoffatom kennzeichnet,
   D für eine Gruppe der Formel 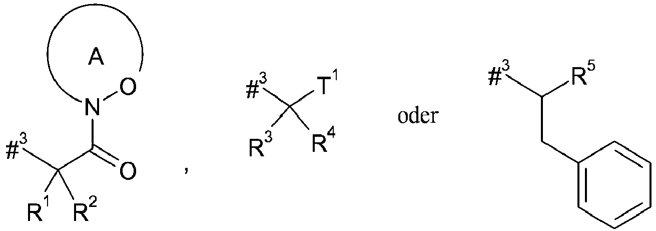 steht, wobei
   #³ die Verknüpfungsstelle mit dem Stickstoffatom kennzeichnet,
   R¹ für Wasserstoff steht,
   R² für 1-Hydroxyethyl, Benzyl, 4-Hydroxybenzyl, 1-Phenylethyl oder 1*H*-Indol-3-ylmethyl steht,
   oder
   R¹ und R² zusammen mit dem Kohlenstoffatom, an das sie gebunden sind, eine (1*S*,2*R*)-2-Phenylcyclopropan-1,1-diyl-Gruppe der Formel 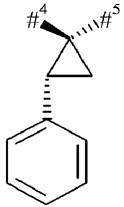 bilden, worin
   #⁴ die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 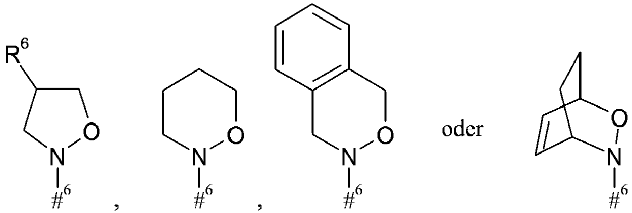 steht, worin
   #⁶ die Verknüpfungsstelle mit der Carbonyl-Gruppe kennzeichnet,
   R⁶ für Wasserstoff, Hydroxy oder Benzyloxy steht,
   R³ für Wasserstoff steht,
   R⁴ für 1-Hydroxyethyl, Benzyl, 4-Hydroxybenzyl, 1-Phenylethyl oder 1*H*-Indol-3-ylmethyl steht,
   oder
   R³ und R⁴ zusammen mit dem Kohlenstoffatom, an das sie gebunden sind, eine (1*S*,2*R*)-2-Phenylcyclopropan-1,1-diyl-Gruppe der Formel 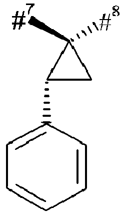 bilden, worin
   #⁷ die Verknüpfungsstelle mit dem angrenzenden Stickstoffatom kennzeichnet,
   #⁸ die Verknüpfungsstelle mit der Gruppe T¹ kennzeichnet,
   T¹ für eine Gruppe der Formel -C(=O)-OR⁷, -C(=O)-NR⁸R⁹, -C(=O)-NH-NH-R¹⁰ oder -CH₂-O-R¹¹ steht,
   worin
   R⁷ für Wasserstoff, Methyl, Ethyl, *n*-Propyl, *tert*.-Butyl, Benzyl oder Adamantylmethyl steht,
   R⁸ für Wasserstoff oder Methyl steht,
   R⁹ für Wasserstoff, Methyl, Ethyl, *n*-Propyl oder Benzyl steht,
   oder
   R⁸ und R⁹ bilden zusammen mit dem Stickstoffatom, an das sie gebunden sind, einen 4- bis 7-gliedrigen Heterocyclus,
   R¹⁰ für Benzoyl steht,
   R¹¹ für Benzyl, das in der Phenylgruppe mit Methoxycarbonyl oder Carboxyl substituiert sein kann, steht,
   R⁵ für Wasserstoff, Methyl oder eine Gruppe der Formel 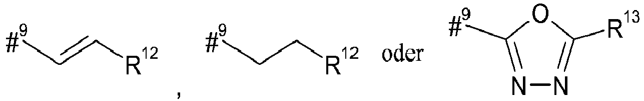 steht, worin
   #⁹ die Verknüpfungsstelle mit -CHCH₂Phenyl kennzeichnet,
   R¹² für Phenyl steht, das mit Methoxycarbonyl, Carboxyl oder einer Gruppe der Formel -S(O)₂OH substituiert sein kann,
   R¹³ für Phenyl steht, das mit Methoxycarbonyl oder Carboxyl substituiert sein kann,
   R³⁵ für Methyl oder Hydroxy steht,
   sowie ihre Salze, Solvate und Solvate der Salze.
9. Verbindungen der Formel (XXXa) gemäß einem der Aspekte 6 oder 7, in welcher
   - Cys: für einen Cystein-Rest steht, der über das Schwefelatom der Seitenkette über ein Kohlenstoffatom des Succinimids gebunden ist, steht
   - L¹: für eine Bindung oder lineares (C₂-C₆)-Alkandiyl steht,
   - B: für eine Bindung oder eine Gruppe der Formel 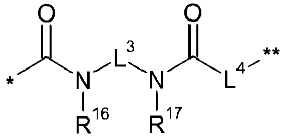 steht, wobei
   * die Verknüpfungsstelle mit L¹ kennzeichnet,
   ** die Verknüpfungsstelle mit L² kennzeichnet,
   L³ für eine Bindung steht,
   L⁴ für eine Bindung steht,
   R¹⁶ für Wasserstoff oder Methyl steht,
   R¹⁷ für Wasserstoff oder Methyl steht,
   - L²: für lineares (C₂-C₆)-Alkandiyl oder für eine Gruppe der Formel 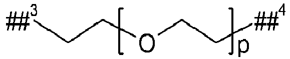 steht, wobei
   p für eine Zahl von 2 oder 3 steht,
   ##³ die Verknüpfungsstelle mit der Gruppe B kennzeichnet,
   ##⁴ die Verknüpfungsstelle mit dem Stickstoffatom kennzeichnet,
   - D: für eine Gruppe der Formel 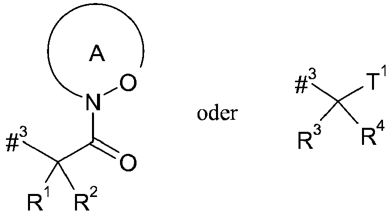 steht, wobei
   #³ die Verknüpfungsstelle mit dem Stickstoffatom kennzeichnet,
   R¹ für Wasserstoff steht,
   R² für Benzyl oder 1*H*-Indol-3-ylmethyl steht,
   oder
   R¹ und R² zusammen mit dem Kohlenstoffatom, an das sie gebunden sind, eine (1*S*,2*R*)-2-Phenylcyclopropan-1,1-diyl-Gruppe der Formel 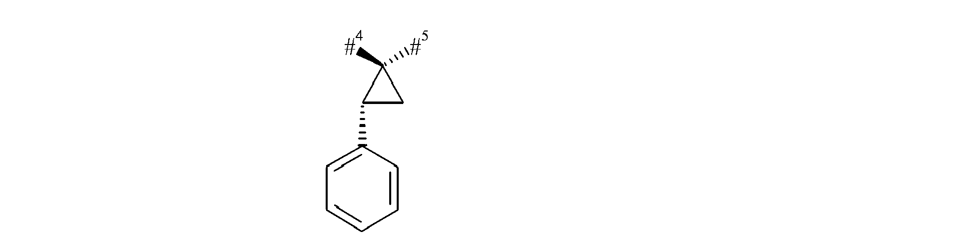 bilden, worin
   #⁴ die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 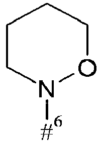 steht, worin
   #⁶ die Verknüpfungsstelle mit der Carbonyl-Gruppe kennzeichnet,
   R³ für Wasserstoff steht,
   R⁴ für Benzyl oder 1*H*-Indol-3-ylmethyl steht,
   oder
   R³ und R⁴ zusammen mit dem Kohlenstoffatom, an das sie gebunden sind, eine (1*S*,2*R*)-2-Phenylcyclopropan-1,1-diyl-Gruppe der Formel 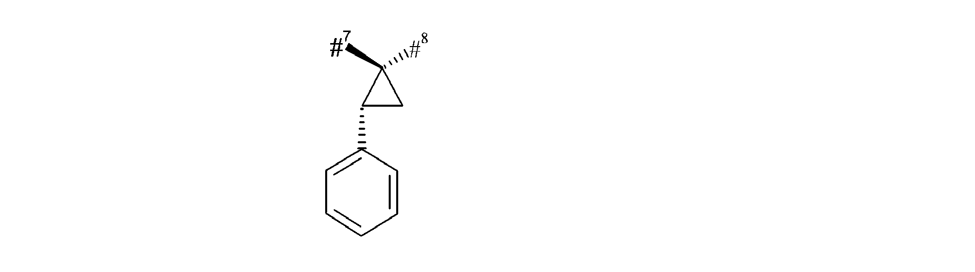 bilden, worin
   #⁷ die Verknüpfungsstelle mit dem angrenzenden Stickstoffatom kennzeichnet,
   #⁸ die Verknüpfungsstelle mit der Gruppe T¹ kennzeichnet,
   T¹ für eine Gruppe der Formel -C(=O)-OR⁷ oder -C(=O)-NR⁸R⁹ steht,
   worin
   R⁷ für Wasserstoff steht,
   R⁸ für Wasserstoff steht,
   R⁹ für Wasserstoff steht,
   R³⁵ für Methyl steht,
   sowie ihre Salze, Solvate und Solvate der Salze.
10. Verbindungen der Formel (XXXI) 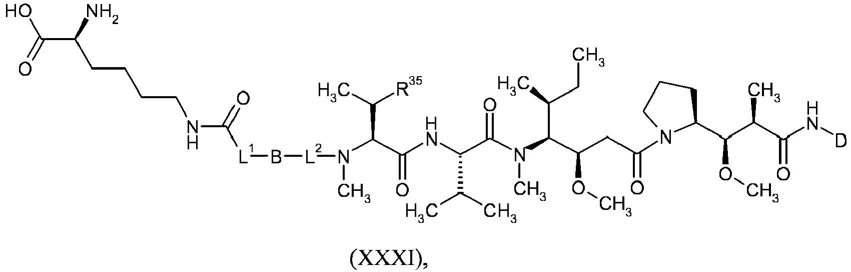 in welcher
   - L¹: für eine Bindung, lineares (C₁-C₁₀)-Alkandiyl, eine Gruppe der Formel 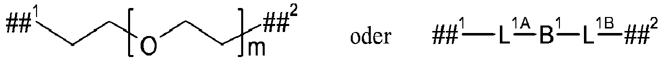 steht, wobei
   m für eine Zahl von 2 bis 6 steht,
   ##¹ die Verknüpfungsstelle mit der Gruppe G kennzeichnet,
   ##² die Verknüpfungsstelle mit der Gruppe B kennzeichnet,
   L^{1A} für lineares (C₂-C₁₀)-Alkandiyl steht,
   B¹ für eine Gruppe der Formel 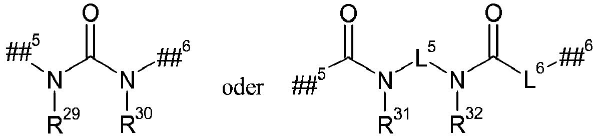 steht, worin
   ##⁵ die Verknüpfungsstelle mit der Gruppe L^{1A} kennzeichnet,
   ##⁶ die Verknüpfungsstelle mit der Gruppe L^{1B} kennzeichnet,
   L⁵ für eine Bindung oder (C₂-C₄)-Alkandiyl steht,
   L⁶ für eine Bindung steht,
   R²⁹ für Wasserstoff oder (C₁-C₄)-Alkyl steht,
   R³⁰ für Wasserstoff oder (C₁-C₄)-Alkyl steht,
   oder
   R²⁹ und R³⁰ zusammen mit den Atomen, an die sie gebunden sind, einen 5- oder 6-gliedrigen Heterocyclus bilden,
   R³¹ für Wasserstoff oder (C₁-C₄)-Alkyl steht,
   R³² für Wasserstoff oder (C₁-C₄)-Alkyl steht,
   oder
   R³ und R³² zusammen mit den Atomen, an die sie gebunden sind, einen 5- oder 6-gliedrigen Heterocyclus bilden,
   L^{1B} für lineares (C₂-C₁₀)-Alkandiyl steht,
   und
   wobei (C₁-C₁₀)-Alkandiyl mit 1 bis 4 Substituenten unabhängig voneinander ausgewählt aus der Gruppe Methyl, Hydroxy und Benzyl substituiert sein kann,
   und
   wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
   - B: für eine Bindung oder eine Gruppe der Formel 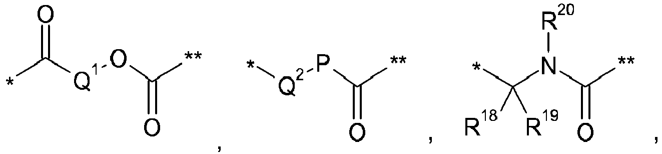 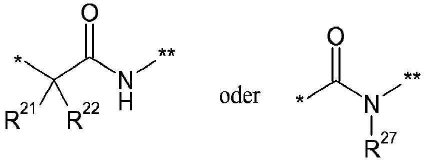 steht, wobei
   * die Verknüpfungsstelle mit L¹ kennzeichnet,
   ** die Verknüpfungsstelle mit L² kennzeichnet,
   P für O oder NH steht,
   Q¹ für einen 4- bis 7-gliedrigen Heterocyclus steht,
   Q² für einen 3- bis 7-gliedrigen Carbocyclus oder einen 4- bis 7-gliedrigen Heterocyclus steht,
   R¹⁸ für Wasserstoff oder (C₁-C₄)-Alkyl steht,
   R¹⁹ für Wasserstoff oder die Seitengruppe einer natürlichen α-Aminosäure oder ihrer Homologe oder Isomere steht,
   R²⁰ für Wasserstoff oder (C₁-C₄)-Alkyl steht,
   oder
   R¹⁹ und R²⁰ zusammen mit den Atomen, an die sie gebunden sind, einen Pyrrolidinylring bilden,
   R²¹ für Wasserstoff oder (C₁-C₄)-Alkyl steht,
   R²² für Wasserstoff oder (C₁-C₄)-Alkyl steht,
   oder
   R²¹ und R²² zusammen mit den Atomen, an die sie gebunden sind, einen 3- bis 7-gliedrigen Carbocyclus bilden,
   R²⁷ für Wasserstoff oder (C₁-C₄)-Alkyl steht,
   - L²: für lineares (C₂-C₁₀)-Alkandiyl oder für eine Gruppe der Formel 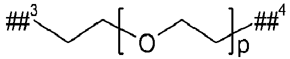 steht, wobei
   p für eine Zahl von 2 bis 6 steht,
   ##³ die Verknüpfungsstelle mit der Gruppe B kennzeichnet,
   ##⁴ die Verknüpfungsstelle mit dem Stickstoffatom kennzeichnet,
   wobei (C₂-C₁₀)-Alkandiyl mit 1 bis 4 Substituenten unabhängig voneinander ausgewählt aus der Gruppe Methyl, Hydroxy und Benzyl substituiert sein kann,
   und
   wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
   - D: für eine Gruppe der Formel 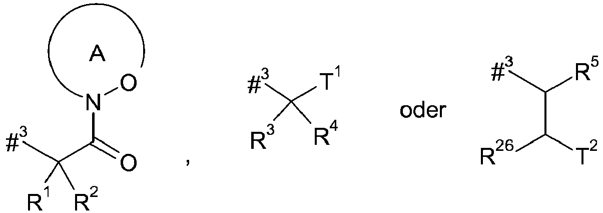 steht, wobei
   #³ die Verknüpfungsstelle mit dem Stickstoffatom kennzeichnet,
   R¹ für Wasserstoff oder Methyl steht,
   R² für Isopropyl, Isobutyl, *sec*.-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenylethyl, Diphenylmethyl, 1*H*-Imidazol-4-ylmethyl oder 1*H*-Indol-3-ylmethyl steht,
   oder
   R¹ und R² zusammen mit dem Kohlenstoffatom, an das sie gebunden sind, eine (1*S*,2*R*)-2-Phenylcyclopropan-1,1-diyl-Gruppe der Formel 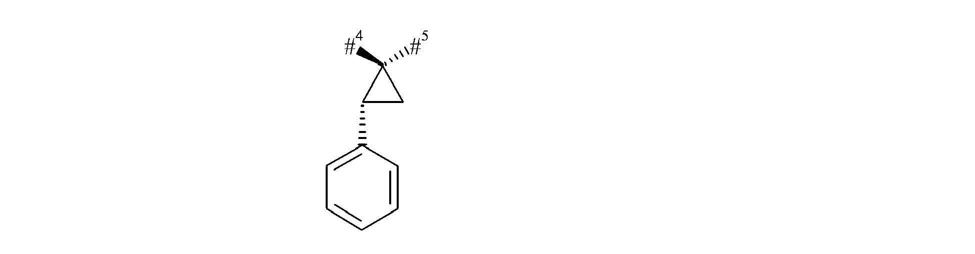 bilden, worin
   #⁴ die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 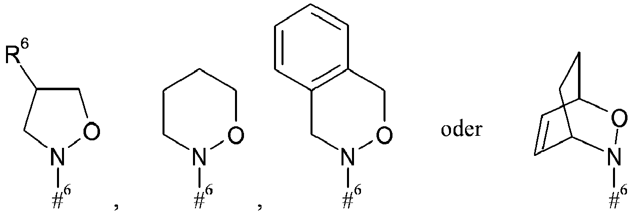 steht, worin
   #⁶ die Verknüpfungsstelle mit der Carbonyl-Gruppe kennzeichnet,
   R⁶ für Wasserstoff, Hydroxy oder Benzyloxy steht,
   R³ für Wasserstoff oder Methyl steht,
   R⁴ für Isopropyl, Isobutyl, *sec*.-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenylethyl, Diphenylmethyl, 1*H*-Imidazol-4-ylmethyl oder 1*H*-Indol-3-ylmethyl steht,
   oder
   R³ und R⁴ zusammen mit dem Kohlenstoffatom, an das sie gebunden sind, eine (1*S*,2*R*)-2-Phenylcyclopropan-1,1-diyl-Gruppe der Formel 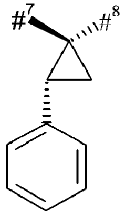 bilden, worin
   #⁷ die Verknüpfungsstelle mit dem angrenzenden Stickstoffatom kennzeichnet,
   #⁸ die Verknüpfungsstelle mit der Gruppe T¹ kennzeichnet,
   T¹ für eine Gruppe der Formel -C(=O)-OR⁷, -C(=O)-NR⁸R⁹, -C(=O)-NH-NH-R¹⁰ oder -CH₂-O-R¹¹ steht,
   worin
   R⁷ für Wasserstoff, Methyl, Ethyl, *n*-Propyl, *tert*.-Butyl, Benzyl oder Adamantylmethyl steht,
   R⁸ für Wasserstoff oder Methyl steht,
   R⁹ für Wasserstoff, Methyl, Ethyl, *n*-Propyl oder Benzyl steht,
   oder
   R⁸ und R⁹ bilden zusammen mit dem Stickstoffatom, an das sie gebunden sind, einen 4- bis 7-gliedrigen Heterocyclus,
   R¹⁰ für Benzoyl steht,
   R¹¹ für Benzyl, das in der Phenylgruppe mit Methoxycarbonyl oder Carboxyl substituiert sein kann, steht,
   R⁵ für Wasserstoff, Methyl oder eine Gruppe der Formel 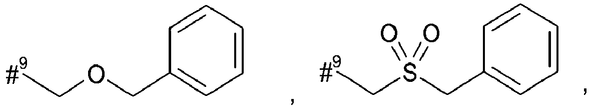 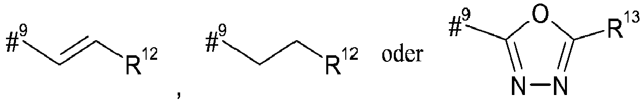 steht, worin
   #⁹ die Verknüpfungsstelle mit -CHC(R²⁶)-T² kennzeichnet,
   R¹² für Phenyl steht, das mit Methoxycarbonyl, Carboxyl oder einer Gruppe der Formel -S(O)₂OH substituiert sein kann,
   R¹³ für Phenyl steht, das mit Methoxycarbonyl oder Carboxyl substituiert sein kann,
   R²⁶ für Wasserstoff oder Hydroxy steht,
   T² für Phenyl, Benzyl, 1*H*-Indol-3-yl oder 1*H*-Indol-3-ylmethyl steht,
   R³⁵ für Methyl oder Hydroxy steht,
   sowie ihre Salze, Solvate und Solvate der Salze.
11. Verbindungen der Formel (XXXI) gemäß Anspruch 10, in welcher
   - L¹: für eine Bindung, lineares (C₂-C₆)-Alkandiyl oder eine Gruppe der Formel 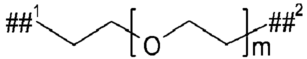 steht, wobei
   m für eine Zahl von 2 oder 3 steht,
   ##¹ die Verknüpfungsstelle mit der Gruppe G kennzeichnet,
   ##² die Verknüpfungsstelle mit der Gruppe B kennzeichnet,
   wobei (C₂-C₆)-Alkandiyl mit 1 oder 2 Substituenten Methyl substituiert sein kann,
   - B: für eine Bindung oder eine Gruppe der Formel 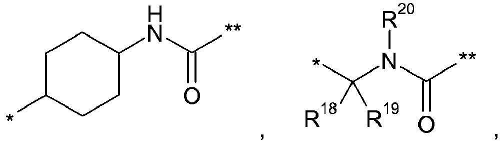 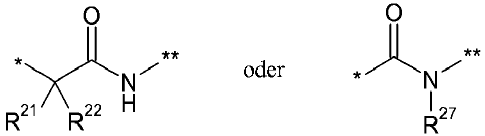 steht, wobei
   * die Verknüpfungsstelle mit L¹ kennzeichnet,
   ** die Verknüpfungsstelle mit L² kennzeichnet,
   R¹⁸ für Wasserstoff steht,
   R¹⁹ für Methyl, Propan-2-yl, 2-Methylpropan-1-yl oder 1-Methylpropan-1-yl steht,
   R²⁰ für Wasserstoff oder (C₁-C₄)-Alkyl steht,
   oder
   R¹⁹ und R²⁰ zusammen mit den Atomen, an die sie gebunden sind, einen Pyrrolidinylring bilden,
   R²¹ für Wasserstoff oder Methyl steht,
   R²² für Wasserstoff oder Methyl steht,
   oder
   R²¹ und R²² zusammen mit den Atomen, an die sie gebunden sind, einen Cyclopropyl-Ring bilden,
   R²⁷ für Wasserstoff oder Methyl steht,
   - L²: für lineares (C₂-C₆)-Alkandiyl oder für eine Gruppe der Formel 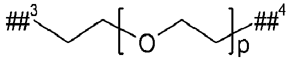 steht, wobei
   p für eine Zahl von 2 oder 3 steht,
   ##³ die Verknüpfungsstelle mit der Gruppe B kennzeichnet,
   ##⁴ die Verknüpfungsstelle mit dem Stickstoffatom kennzeichnet,
   wobei (C₂-C₁₀)-Alkandiyl mit 1 oder 2 Substituenten Methyl substituiert sein kann,
   und
   wobei zwei Kohlenstoffatome der Alkandiyl-Kette in 1,4-Relation zueinander unter Einbezug der gegebenenfalls zwischen ihnen liegenden Kohlenstoffatome zu einem Phenyl-Ring verbrückt sein können,
   - D: für eine Gruppe der Formel 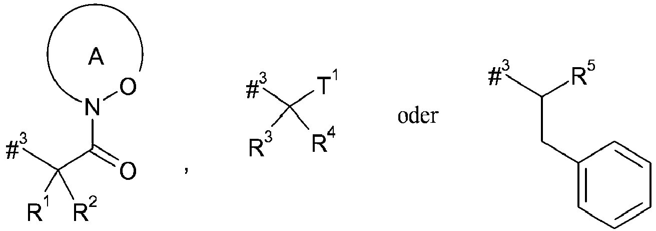 steht, wobei
   #³ die Verknüpfungsstelle mit dem Stickstoffatom kennzeichnet,
   R¹ für Wasserstoff steht,
   R² für 1-Hydroxyethyl, Benzyl, 4-Hydroxybenzyl, 1-Phenylethyl oder 1*H*-Indol-3-ylmethyl steht,
   oder
   R¹ und R² zusammen mit dem Kohlenstoffatom, an das sie gebunden sind, eine (1*S*,2*R*)-2-Phenylcyclopropan-1,1-diyl-Gruppe der Formel 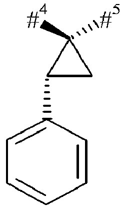 bilden, worin
   #⁴ die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 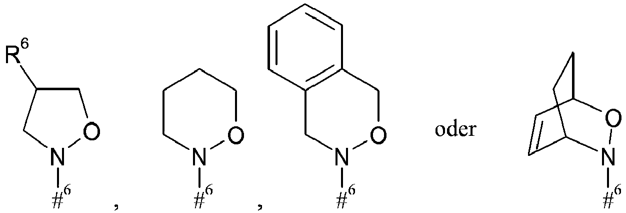 steht, worin
   #⁶ die Verknüpfungsstelle mit der Carbonyl-Gruppe kennzeichnet,
   R⁶ für Wasserstoff, Hydroxy oder Benzyloxy steht,
   R³ für Wasserstoff steht,
   R⁴ für 1-Hydroxyethyl, Benzyl, 4-Hydroxybenzyl, 1-Phenylethyl oder 1*H*-Indol-3-ylmethyl steht,
   oder
   R³ und R⁴ zusammen mit dem Kohlenstoffatom, an das sie gebunden sind, eine (1*S*,2*R*)-2-Phenylcyclopropan-1,1-diyl-Gruppe der Formel 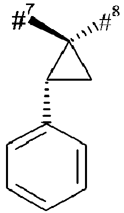 bilden, worin
   #⁷ die Verknüpfungsstelle mit dem angrenzenden Stickstoffatom kennzeichnet,
   #⁸ die Verknüpfungsstelle mit der Gruppe T¹ kennzeichnet,
   T¹ für eine Gruppe der Formel -C(=O)-OR⁷, -C(=O)-NR⁸R⁹, -C(=O)-NH-NH-
   R¹⁰ oder -CH₂-O-R¹¹ steht,
   worin
   R⁷ für Wasserstoff, Methyl, Ethyl, *n*-Propyl, *tert*.-Butyl, Benzyl oder Adamantylmethyl steht,
   R⁸ für Wasserstoff oder Methyl steht,
   R⁹ für Wasserstoff, Methyl, Ethyl, *n*-Propyl oder Benzyl steht,
   oder
   R⁸ und R⁹ bilden zusammen mit dem Stickstoffatom, an das sie gebunden
   sind, einen 4- bis 7-gliedrigen Heterocyclus,
   R¹⁰ für Benzoyl steht,
   R¹¹ für Benzyl, das in der Phenylgruppe mit Methoxycarbonyl oder Carboxyl substituiert sein kann, steht,
   R⁵ für Wasserstoff, Methyl oder eine Gruppe der Formel 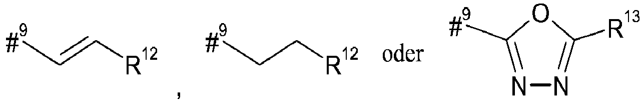 steht, worin
   #⁹ die Verknüpfungsstelle mit -CHCH₂Phenyl kennzeichnet,
   R¹² für Phenyl steht, das mit Methoxycarbonyl, Carboxyl oder einer Gruppe der Formel -S(O)₂OH substituiert sein kann,
   R¹³ für Phenyl steht, das mit Methoxycarbonyl oder Carboxyl substituiert sein kann,
   - R³⁵: für Methyl oder Hydroxy steht,
   sowie ihre Salze, Solvate und Solvate der Salze.
12. Verbindungen der Formel (XXXI) gemäß Anspruch 9, in welcher
   - L¹: für eine Bindung steht,
   - B: für eine Bindung steht,
   - L²: für lineares (C₂-C₆)-Alkandiyl oder für eine Gruppe der Formel 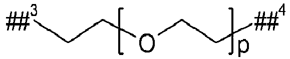 steht, wobei
   p für eine Zahl von 2 oder 3 steht,
   ##³ die Verknüpfungsstelle mit der Gruppe B kennzeichnet,
   ##⁴ die Verknüpfungsstelle mit dem Stickstoffatom kennzeichnet,
   D für eine Gruppe der Formel 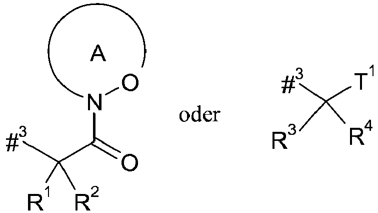 steht, wobei
   #³ die Verknüpfungsstelle mit dem Stickstoffatom kennzeichnet,
   R¹ für Wasserstoff steht,
   R² für Benzyl oder 1*H*-Indol-3-ylmethyl steht,
   oder
   R¹ und R² zusammen mit dem Kohlenstoffatom, an das sie gebunden sind, eine (1*S*,2*R*)-2-Phenylcyclopropan-1,1-diyl-Gruppe der Formel 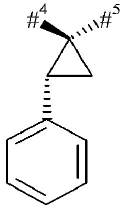 bilden, worin
   #⁴ die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 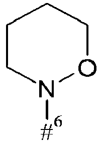 steht, worin
   #⁶ die Verknüpfungsstelle mit der Carbonyl-Gruppe kennzeichnet,
   R⁶ für Wasserstoff, Hydroxy oder Benzyloxy steht,
   R³ für Wasserstoff steht,
   R⁴ für Benzyl oder 1*H*-Indol-3-ylmethyl steht,
   oder
   R³ und R⁴ zusammen mit dem Kohlenstoffatom, an das sie gebunden sind, eine (1*S*,2*R*)-2-Phenylcyclopropan-1,1-diyl-Gruppe der Formel 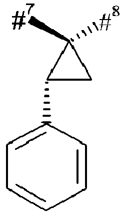 bilden, worin
   #⁷ die Verknüpfungsstelle mit dem angrenzenden Stickstoffatom kennzeichnet,
   #⁸ die Verknüpfungsstelle mit der Gruppe T¹ kennzeichnet,
   T¹ für eine Gruppe der Formel -C(=O)-OR⁷ oder -C(=O)-NR⁸R⁹ steht,
   worin
   R⁷ für Wasserstoff steht,
   R⁸ für Wasserstoff steht,
   R⁹ für Wasserstoff steht,
   R³⁵ für Methyl steht,
   sowie ihre Salze, Solvate und Solvate der Salze.
13. Verbindungen der Formeln (XXXa) und (XXXI) ausgewählt aus der Gruppe:
   *N*-[6-(3-{[(2*R*)-2-Amino-2-carboxyethyl]sulfanyl}-2,5-dioxopyrrolidin-1-yl)hexyl]-*N-*methyl-L-valyl-*N*-[(3*R*,4*S*,5*S*)-1-{(2*S*)-2-[(1*R*,2*R*)-3-{[(1*S*)-1-carboxy-2-(1*H*-indol-3-yl)ethyl]amino}-1-methoxy-2-methyl-3-oxopropyl]pyrrolidin-1-yl}-3-methoxy-5-methyl-1-oxoheptan-4-yl]-*N*-methyl-L-valinamid,
   *N*-[6-(3-{[(2*R*)-2-Amino-2-carboxyethyl]sulfanyl}-2,5-dioxopyrrolidin-1-yl)hexyl]-*N-*methyl-L-valyl-*N*-[(3*R*,4*S*,5*S*)-1-{(2*S*)-2-[(1*R*,2*R*)-3-{[(2*S*)-3-(1*H*-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid,
   N-(6-{[(5S)-5-Amino-5-carboxypentyl]amino}-6-oxohexyl)-N-methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid-Trifluoracetat,
   N-(6-{[(5S)-5-Amino-5-carboxypentyl]amino}-6-oxohexyl)-N-methyl-L-valyl-N-[(3R,4S,5S)-1-{(2S)-2-[(1R,2R)-3-{[(1S)-1-carboxy-2-(1H-indol-3-yl)ethyl]amino}-1-methoxy-2-methyl-3-oxopropyl]pyrrolidin-1-yl}-3-methoxy-5-methyl-1-oxoheptan-4-yl]-N-methyl-L-valinamid,
   sowie ihre Salze, Solvate und Solvate der Salze.
14. Binder-Wirkstoff Konjugate der allgemeinen Formel (I) 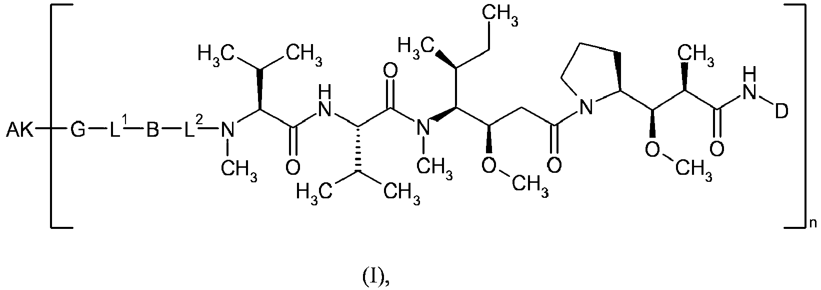 in welcher
   - n: für eine Zahl von 1 bis 50 steht,
   - AK: für einen Binder steht,
   die Gruppe §-G-L¹-B-L²-§§ für einen Linker steht,
   wobei
   § die Verknüpfungsstelle mit der Gruppe AK kennzeichnet und
   §§ die Verknüpfungsstelle mit dem Stickstoffatom kennzeichnet,
   - D: für eine Gruppe der Formel 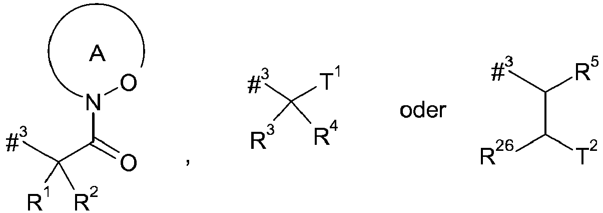 steht, wobei
   #³ die Verknüpfungsstelle mit dem Stickstoffatom kennzeichnet,
   R¹ für Wasserstoff steht,
   R² für 1-Hydroxyethyl, Benzyl, 1-Phenylethyl oder 1*H*-Indol-3-ylmethylsteht,
   oder
   R¹ und R² zusammen mit dem Kohlenstoffatom, an das sie gebunden sind, eine (1*S*,2*R*)-2-Phenylcyclopropan-1,1-diyl-Gruppe der Formel 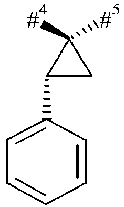 bilden, worin
   #⁴ die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 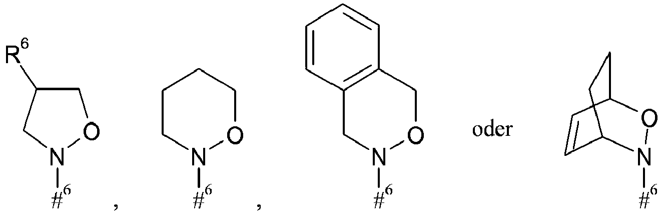 steht, worin
   #⁶ die Verknüpfungsstelle mit der Carbonyl-Gruppe kennzeichnet,
   R⁶ für Wasserstoff, Hydroxy oder Benzyloxy steht,
   R³ für Wasserstoff steht,
   R⁴ für 1-Hydroxyethyl, Benzyl, 1-Phenylethyl oder 1*H*-Indol-3-ylmethyl steht,
   oder
   R³ und R⁴ zusammen mit dem Kohlenstoffatom, an das sie gebunden sind, eine (1*S*,2*R*)-2-Phenylcyclopropan-1,1-diyl-Gruppe der Formel 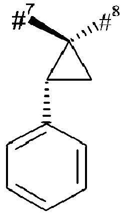 bilden, worin
   #⁷ die Verknüpfungsstelle mit dem angrenzenden Stickstoffatom kennzeichnet,
   #⁸ die Verknüpfungsstelle mit der Gruppe T¹ kennzeichnet,
   T¹ für eine Gruppe der Formel -C(=O)-OR⁷, -C(=O)-NR⁸R⁹, -C(=O)-NH-NH-R¹⁰ oder -CH₂-O-R¹¹ steht,
   worin
   R⁷ für Wasserstoff, Methyl, Ethyl, *n*-Propyl, *tert*.-Butyl, Benzyl oder Adamantylmethyl steht,
   R⁸ für Wasserstoff oder Methyl steht,
   R⁹ für Wasserstoff, Methyl, Ethyl, *n*-Propyl oder Benzyl steht,
   oder
   R⁸ und R⁹ bilden zusammen mit dem Stickstoffatom, an das sie gebunden sind, einen 4- bis 7-gliedrigen Heterocyclus,
   R¹⁰ für Benzoyl steht,
   R¹¹ für Benzyl, das in der Phenylgruppe mit Methoxycarbonyl oder Carboxyl substituiert sein kann, steht,
   R⁵ für Wasserstoff, Methyl oder eine Gruppe der Formel 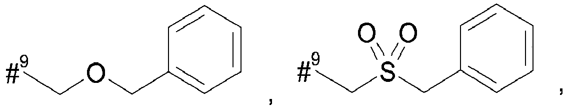 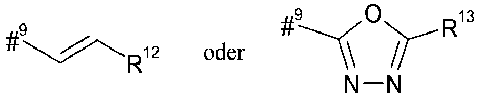 steht, worin
   #⁹ die Verknüpfungsstelle mit -CHC(R²⁶)-T² kennzeichnet,
   R¹² für Phenyl steht, das mit Methoxycarbonyl, Carboxyl oder einer Gruppe der Formel -S(O)₂OH substituiert sein kann,
   R¹³ für Phenyl steht, das mit Methoxycarbonyl oder Carboxyl substituiert sein kann,
   R²⁶ für Wasserstoff oder Hydroxy steht,
   T² für Phenyl, Benzyl, 1*H*-Indol-3-yl oder 1*H*-Indol-3-ylmethyl steht,
   sowie ihre Salze, Solvate und Solvate der Salze.
15. Binder-Wirkstoff Konjugate nach Anspruch 14 mit der allgemeinen Formel (I), in welcher
   - n: für eine Zahl von 1 bis 50 steht,
   - AK: für AK₁ oder AK₂ steht
   wobei
   - AK₁: für einen Binder, der über ein Schwefelatom des Binders an die Gruppe G gebunden ist,
   - AK₂: für einen Binder, der über ein Stickstoffatom des Binders an die Gruppe G gebunden ist,
   - G: für den Fall, dass AK = AK₁ ist, für eine Gruppe der Formel 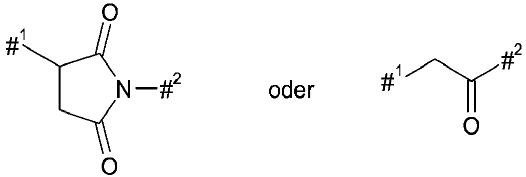 steht, wobei
   #¹ die Verknüpfungsstelle mit dem Schwefelatom des Binders kennzeichnet,
   #² die Verknüpfungsstelle mit der Gruppe L¹ kennzeichnet,
   oder
   für den Fall, dass AK = AK₂ ist, für Carbonyl steht,
   - L¹: für eine Bindung, lineares (C₁-C₁₀)-Alkandiyl oder für eine Gruppe der Formel 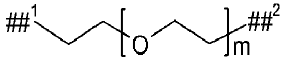 steht, wobei
   m für eine Zahl von 2 bis 6 steht,
   ##¹ die Verknüpfungsstelle mit der Gruppe G kennzeichnet,
   ##² die Verknüpfungsstelle mit der Gruppe B kennzeichnet,
   wobei (C₁-C₁₀)-Alkandiyl mit 1 bis 4 Substituenten Methyl substituiert sein kann,
   und
   wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
   - B: für eine Bindung oder eine Gruppe der Formel 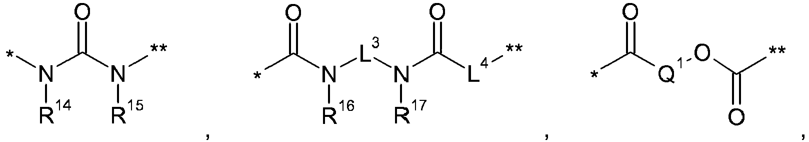 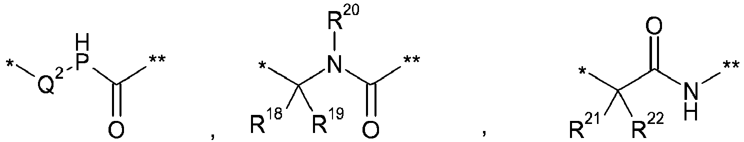 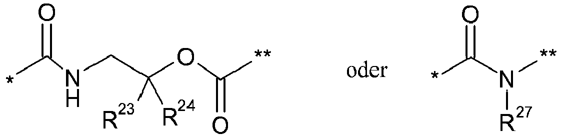 steht, wobei
   * die Verknüpfungsstelle mit L¹ kennzeichnet,
   ** die Verknüpfungsstelle mit L² kennzeichnet,
   P für O oder NH steht,
   L³ für eine Bindung oder (C₂-C₄)-Alkandiyl steht,
   L⁴ für eine Bindung oder eine Gruppe der Formel 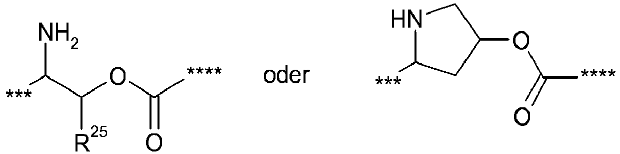 steht, worin
   *** die Verknüpfungsstelle mit der Carbonylgruppe kennzeichnet,
   **** die Verknüpfungsstelle mit L² kennzeichnet,
   R²⁵ für Wasserstoff oder Methyl steht,
   Q¹ für einen 4- bis 7-gliedrigen Heterocyclus steht,
   Q² für einen 3- bis 7-gliedrigen Carbocyclus oder einen 4- bis 7-gliedrigen Heterocyclus steht,
   R¹⁴ für Wasserstoff oder (C₁-C₄)-Alkyl steht,
   R¹⁵ für Wasserstoff oder (C₁-C₄)-Alkyl steht,
   oder
   R¹⁴ und R¹⁵ zusammen mit den Atomen, an die sie gebunden sind, einen 5- oder 6-gliedrigen Heterocyclus bilden,
   R¹⁶ für Wasserstoff oder (C₁-C₄)-Alkyl steht,
   R¹⁷ für Wasserstoff oder (C₁-C₄)-Alkyl steht,
   oder
   R¹⁶ und R¹⁷ zusammen mit den Atomen, an die sie gebunden sind, einen 5- oder 6-gliedrigen Heterocyclus bilden,
   R¹⁸ für Wasserstoff oder (C₁-C₄)-Alkyl steht,
   R¹⁹ für Wasserstoff oder die Seitengruppe einer natürlichen α-Aminosäure oder ihrer Homologe oder Isomere steht,
   R²⁰ für Wasserstoff oder (C₁-C₄)-Alkyl steht,
   oder
   R¹⁹ und R²⁰ zusammen mit den Atomen, an die sie gebunden sind, einen Pyrrolidinylring bilden,
   R²¹ für Wasserstoff oder (C₁-C₄)-Alkyl steht,
   R²² für Wasserstoff oder (C₁-C₄)-Alkyl steht,
   oder
   R²¹ und R²² zusammen mit den Atomen, an die sie gebunden sind, einen 3- bis 7-gliedrigen Carbocyclus bilden,
   R²³ für (C₁-C₄)-Alkyl steht,
   R²⁴ für Wasserstoff oder (C₁-C₄)-Alkyl steht,
   R²⁷ für Wasserstoff oder (C₁-C₄)-Alkyl steht,
   - L²: für lineares (C₂-C₁₀)-Alkandiyl oder für eine Gruppe der Formel 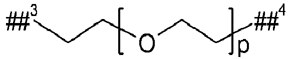 steht, wobei
   p für eine Zahl von 2 bis 6 steht,
   ##³ die Verknüpfungsstelle mit der Gruppe B kennzeichnet,
   ##⁴ die Verknüpfungsstelle mit dem Stickstoffatom kennzeichnet,
   wobei (C₂-C₁₀)-Alkandiyl mit 1 bis 4 Substituenten Methyl substituiert sein kann,
   und
   wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
   - D: die in Anspruch 14 angegebenen Bedeutungen aufweist,
   sowie ihre Salze, Solvate und Solvate der Salze. 18. Binder-
16. Binder-Wirkstoff Konjugate nach Anspruch 14 oder 15 der allgemeinen Formel (I),
   in welcher
   - n: für eine Zahl von 1 bis 50 steht,
   - AK: für AK₁ oder AK₂ steht
   wobei
   AK₁ für einen Antikörper oder ein Antigen-bindendes Antikörperfragment steht und über ein Schwefelatom an die Gruppe G gebunden sind,
   AK₂ für einen Antikörper oder ein Antigen-bindendes Antikörperfragment steht und über ein Stickstoffatom an die Gruppe G gebunden sind,
   - G, L¹, B, L² D: die in den Aspekten 14 oder 15 angegebenen Bedeutungen aufweisen,
   sowie ihre Salze, Solvate und Solvate der Salze.
17. Binder-Wirkstoff Konjugate nach einem der Aspekte 14 bis 16 der allgemeinen Formel (I), in welcher
   - n: für eine Zahl von 1 bis 20 steht,
   - AK: für AK₁ oder AK₂ steht
   wobei
   AK₁ für einen Antikörper oder ein Antigen-bindendes Antikörperfragment steht, der bzw. das an C4.4a bindet und über das Schwefelatom eines Cystein-Restes des Binders an die Gruppe G gebunden ist,
   AK₂ für einen Antikörper oder ein Antigen-bindendes Antikörperfragment steht, der bzw. das an C4.4a bindet und über die NH-Seitengruppe eines Lysin-Restes des Binders an die Gruppe G gebunden ist,
   - G: für den Fall, dass AK = AK₁ ist, für eine Gruppe der Formel 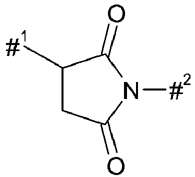 steht, wobei
   #¹ die Verknüpfungsstelle mit dem Cystein-Rest des Binders kennzeichnet,
   #² die Verknüpfungsstelle mit der Gruppe L¹ kennzeichnet,
   oder
   für den Fall, dass AK = AK₂ ist, für Carbonyl steht,
   - L¹: für eine Bindung, lineares (C₂-C₆)-Alkandiyl oder für eine Gruppe der Formel 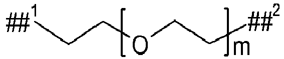 steht, wobei
   m für eine Zahl von 2 bis 6 steht,
   ##¹ die Verknüpfungsstelle mit der Gruppe G kennzeichnet,
   ##² die Verknüpfungsstelle mit der Gruppe B kennzeichnet,
   wobei (C₂-C₆)-Alkandiyl mit 1 oder 2 Substituenten Methyl substituiert sein kann,
   - B: für eine Bindung oder eine Gruppe der Formel 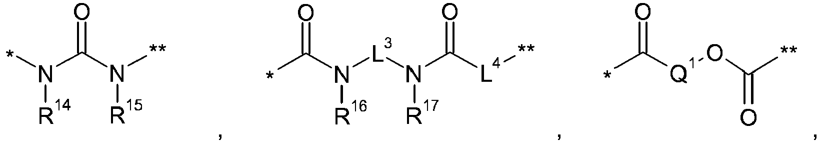 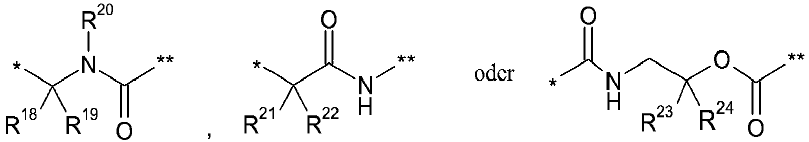 steht, wobei
   * die Verknüpfungsstelle mit L¹ kennzeichnet,
   ** die Verknüpfungsstelle mit L² kennzeichnet,
   L³ für eine Bindung oder Ethan-1,2-diyl steht,
   L⁴ für eine Bindung oder eine Gruppe der Formel 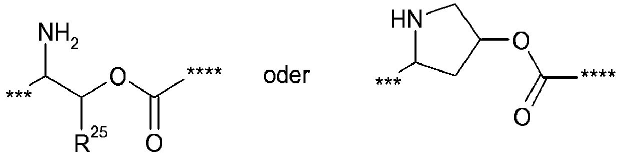 steht, worin
   *** die Verknüpfungsstelle mit der Carbonylgruppe kennzeichnet,
   **** die Verknüpfungsstelle mit L² kennzeichnet,
   R²⁵ für Methyl steht,
   Q¹ für einen 4- bis 6-gliedrigen Carbocyclus oder Piperidin-1,4-diyl steht,
   R¹⁴ für Wasserstoff steht,
   R¹⁵ für Wasserstoff steht,
   R¹⁶ für Wasserstoff oder Methyl steht,
   R¹⁷ für Wasserstoff oder Methyl steht,
   oder
   R¹⁶ und R¹⁷ zusammen mit den Atomen, an die sie gebunden sind, einen Piperazinylring bilden,
   R¹⁸ für Wasserstoff steht,
   R¹⁹ für Wasserstoff, Methyl, Propan-2-yl, 2-Methylpropan-1-yl oder 1-Methylpropan-1-yl steht,
   R²⁰ für Wasserstoff oder Methyl steht,
   oder
   R¹⁹ und R²⁰ zusammen mit den Atomen, an die sie gebunden sind, einen Pyrrolidinylring bilden,
   R²¹ für Wasserstoff oder Methyl steht,
   R²² für Wasserstoff oder Methyl steht,
   oder
   R²¹ und R²² zusammen mit den Atomen, an die sie gebunden sind, einen Cyclopropylring bilden,
   R²³ für Methyl steht,
   R²⁴ für Wasserstoff oder Methyl steht,
   - L²: für lineares (C₂-C₆)-Alkandiyl oder für eine Gruppe der Formel 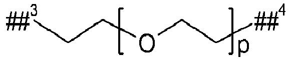 steht, wobei
   p für eine Zahl von 2 bis 6 steht,
   ##³ die Verknüpfungsstelle mit der Gruppe B kennzeichnet,
   ##⁴ die Verknüpfungsstelle mit dem Stickstoffatom kennzeichnet,
   wobei (C₂-C₆)-Alkandiyl mit 1 oder 2 Substituenten Methyl substituiert sein kann,
   - D: für eine Gruppe der Formel 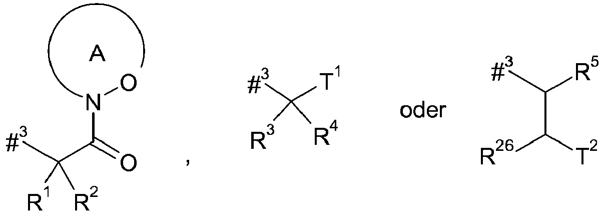 steht, wobei
   #³ die Verknüpfungsstelle mit dem Stickstoffatom kennzeichnet,
   R¹ für Wasserstoff steht,
   R² für 1-Hydroxyethyl, Benzyl, 1-Phenylethyl oder 1*H*-Indol-3-ylmethyl steht,
   oder
   R¹ und R² zusammen mit dem Kohlenstoffatom, an das sie gebunden sind, eine (1*S,*2*R*)-2-Phenylcyclopropan-1,1-diyl-Gruppe der Formel 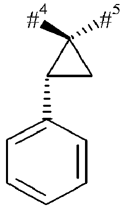 bilden, worin
   #⁴ die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 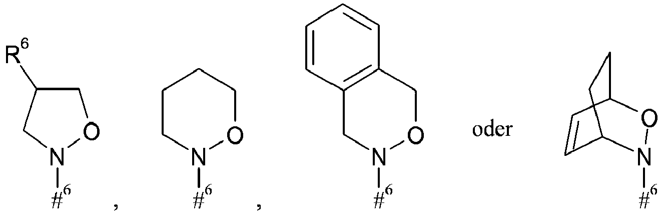 steht, worin
   #⁶ die Verknüpfungsstelle mit der Carbonyl-Gruppe kennzeichnet,
   R⁶ für Wasserstoff, Hydroxy oder Benzyloxy steht,
   R³ für Wasserstoff steht,
   R⁴ für 1-Hydroxyethyl, Benzyl, 1-Phenylethyl oder 1*H*-Indol-3-ylmethyl steht,
   oder
   R³ und R⁴ zusammen mit dem Kohlenstoffatom, an das sie gebunden sind, eine (1*S,*2*R*)-2-Phenylcyclopropan-1,1-diyl-Gruppe der Formel 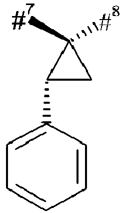 bilden, worin
   #⁷ die Verknüpfungsstelle mit dem angrenzenden Stickstoffatom kennzeichnet,
   #⁸ die Verknüpfungsstelle mit der Gruppe T¹ kennzeichnet,
   T¹ für eine Gruppe der Formel -C(=O)-OR⁷, -C(=O)-NR⁸R⁹, -C(=O)-NH-NH-R¹⁰ oder -CH₂-O-R¹¹ steht,
   worin
   R⁷ für Wasserstoff, Methyl, Ethyl, *n*-Propyl, *tert.*-Butyl, Benzyl oder Adamantylmethyl steht,
   R⁸ für Wasserstoff oder Methyl steht,
   R⁹ für Wasserstoff, Methyl, Ethyl, *n*-Propyl oder Benzyl steht,
   oder
   R⁸ und R⁹ bilden zusammen mit dem Stickstoffatom, an das sie gebunden sind, einen 4- bis 7-gliedrigen Heterocyclus,
   R¹⁰ für Benzoyl steht,
   R¹¹ für Benzyl, das in der Phenylgruppe mit Methoxycarbonyl oder Carboxyl substituiert sein kann, steht,
   R⁵ für Wasserstoff, Methyl oder eine Gruppe der Formel 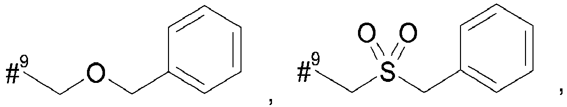 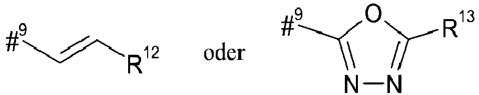 steht, worin
   #⁹ die Verknüpfungsstelle mit -CHC(R²⁶)-T² kennzeichnet,
   R¹² für Phenyl steht, das mit Methoxycarbonyl, Carboxyl oder einer Gruppe der Formel -S(O)₂OH substituiert sein kann,
   R¹³ für Phenyl steht, das mit Methoxycarbonyl oder Carboxyl substituiert sein kann,
   R²⁶ für Wasserstoff oder Hydroxy steht,
   T² für Phenyl, Benzyl, 1*H*-Indol-3-yl oder 1*H*-Indol-3-ylmethyl steht,
   sowie ihre Salze, Solvate und Solvate der Salze.
18. Binder-Wirkstoff Konjugate nach einem der Aspekte 14 bis 17 der allgemeinen Formel (I), in welcher
   - n: für eine Zahl von 1 bis 10 steht,
   - AK: für AK₁ oder AK₂ steht
   wobei
   AK₁ für einen Antikörper, der die sechs CDR-Sequenzen des Antikörpers B01-3, B01-10, M31-B01 oder D02-6, die variable leichte und variable schwere Kette des Antikörpers B01-3, B01-10, M31-B01 oder D02-6 oder die leichte und schwere Kette des Antiköpers B01-3, B01-10, M31-B01 oder D02-6 umfasst, steht, der über das Schwefelatom eines Cystein-Restes des Binders an die Gruppe G gebunden ist,
   AK₂ für einen Antikörper, der die sechs CDR-Sequenzen des Antikörpers B01-3, B01-10, M31-B01 oder D02-6, die variable leichte und variable schwere Kette des Antikörpers B01-3, B01-10, M31-B01 oder D02-6 oder die leichte und schwere Kette des Antiköpers B01-3, B01-10, M31-B01 oder D02-6 umfasst, steht, der über die NH-Seitengruppe eines Lysin-Restes des Binders an die Gruppe G gebunden ist,
   - G: für den Fall, dass AK = AK₁ ist, für eine Gruppe der Formel 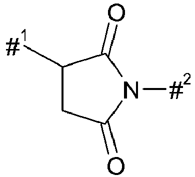 steht, wobei
   #¹ die Verknüpfungsstelle mit dem Cystein-Rest des Binders kennzeichnet,
   #² die Verknüpfungsstelle mit der Gruppe L¹ kennzeichnet,
   oder
   - L¹: für den Fall, dass AK = AK₂ ist, für Carbonyl steht, für eine Bindung, lineares (C₂-C₆)-Alkandiyl oder für eine Gruppe der Formel 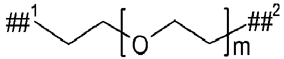 steht, wobei
   m für eine Zahl 2 oder 3 steht,
   ##¹ die Verknüpfungsstelle mit der Gruppe G kennzeichnet,
   ##² die Verknüpfungsstelle mit der Gruppe B kennzeichnet,
   wobei (C₂-C₆)-Alkandiyl mit 1 oder 2 Substituenten Methyl substituiert sein kann,
   - B: für eine Bindung oder eine Gruppe der Formel 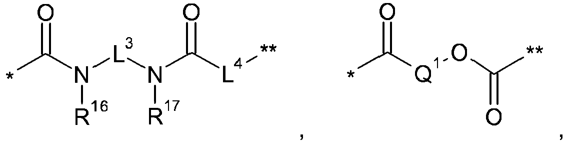 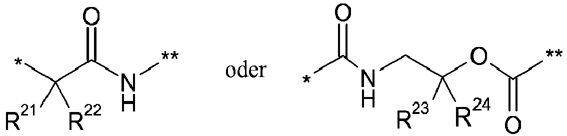 steht, wobei
   * die Verknüpfungsstelle mit L¹ kennzeichnet,
   ** die Verknüpfungsstelle mit L² kennzeichnet,
   L³ für eine Bindung oder Ethan-1,2-diyl steht,
   L⁴ für eine Bindung oder eine Gruppe der Formel 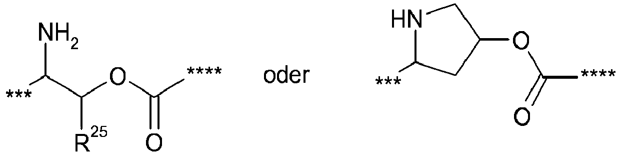 steht, worin
   *** die Verknüpfungsstelle mit der Carbonylgruppe kennzeichnet,
   **** die Verknüpfungsstelle mit L² kennzeichnet,
   R²⁵ für Methyl steht,
   Q¹ für Piperidin-1,4-diyl steht,
   R¹⁶ für Wasserstoff oder Methyl steht,
   R¹⁷ für Wasserstoff oder Methyl steht,
   oder
   R¹⁶ und R¹⁷ zusammen mit den Atomen, an die sie gebunden sind, einen Piperazinylring bilden,
   R²¹ für Wasserstoff oder Methyl steht,
   R²² für Wasserstoff oder Methyl steht,
   oder
   R²¹ und R²² zusammen mit den Atomen, an die sie gebunden sind, einen Cyclopropylring bilden,
   R²³ für Methyl steht,
   R²⁴ für Wasserstoff steht,
   - L²: für lineares (C₂-C₆)-Alkandiyl steht,
   - D: für eine Gruppe der Formel 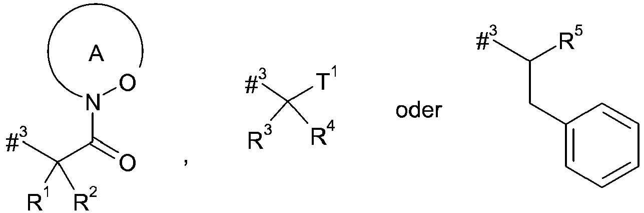 steht, wobei
   #³ die Verknüpfungsstelle mit dem Stickstoffatom kennzeichnet,
   R¹ für Wasserstoff steht,
   R² für 1-Hydroxyethyl, Benzyl, 1-Phenylethyl oder 1*H*-Indol-3-ylmethyl steht,
   oder
   R¹ und R² zusammen mit dem Kohlenstoffatom, an das sie gebunden sind, eine (1*S,*2*R*)-2-Phenylcyclopropan-1,1-diyl-Gruppe der Formel 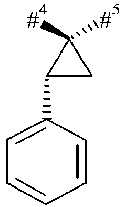 #⁴ bilden, worin die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 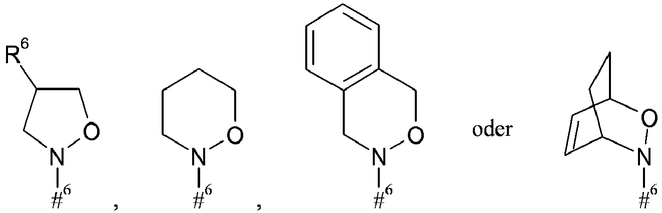 steht, worin
   #⁶ die Verknüpfungsstelle mit der Carbonyl-Gruppe kennzeichnet,
   R⁶ für Wasserstoff, Hydroxy oder Benzyloxy steht,
   R³ für Wasserstoff steht,
   R⁴ für Benzyl, 1-Phenylethyl oder 1*H*-Indol-3-ylmethyl steht,
   oder
   R³ und R⁴ zusammen mit dem Kohlenstoffatom, an das sie gebunden sind, eine (1*S*,2*R*)-2-Phenylcyclopropan-1,1-diyl-Gruppe der Formel 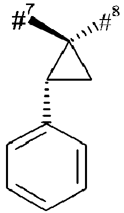 bilden, worin
   #⁷ die Verknüpfungsstelle mit dem angrenzenden Stickstoffatom kennzeichnet,
   #⁸ die Verknüpfungsstelle mit der Gruppe T kennzeichnet,
   T¹ für eine Gruppe der Formel -C(=O)-OR⁷, -C(=O)-NR⁸R⁹ oder -CH₂-O-R¹¹ steht,
   worin
   R⁷ für Wasserstoff, Methyl, Ethyl, *n*-Propyl, Benzyl oder Adamantylmethyl steht,
   R⁸ für Wasserstoff oder Methyl steht,
   R⁹ für Wasserstoff, Methyl, Ethyl, *n*-Propyl oder Benzyl steht,
   R¹¹ für Benzyl, das in der Phenylgruppe mit Methoxycarbonyl oder Carboxyl substituiert sein kann, steht,
   R⁵ für Wasserstoff oder eine Gruppe der Formel 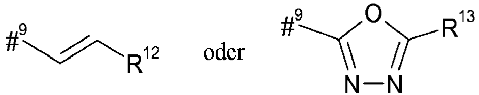 steht, worin
   #⁹ die Verknüpfungsstelle mit -CHC(R²⁶)Phenyl kennzeichnet,
   R¹² für Phenyl steht, das mit Methoxycarbonyl, Carboxyl oder einer Gruppe der Formel -S(O)₂OH substituiert sein kann,
   R¹³ für Phenyl steht, das mit Methoxycarbonyl oder Carboxyl substituiert sein kann,
   sowie ihre Salze, Solvate und Solvate der Salze.
19. Verfahren zur Herstellung der erfindungsgemäßen Verbindungen nach einem der Aspekte 14 bis 18, der allgemeinen Formel (I), dadurch gekennzeichnet, dass man eine Lösung des Binders in einem Puffer
   [A] mit einem geeigneten Reduktionsmittel, wie beispielsweise Dithiothreitol oder Tris(2-carboxyethyl)phosphin-Hydrochlorid, versetzt, und anschließend mit einer Verbindung der Formel (II) 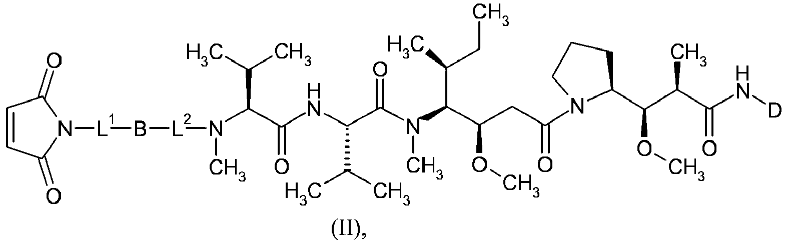 in welcher D, L¹, B und L² jeweils die die in den Aspekten 1 bis 5 angegebenen Bedeutungen haben,
      zu einer Verbindung der Formel (I-A) 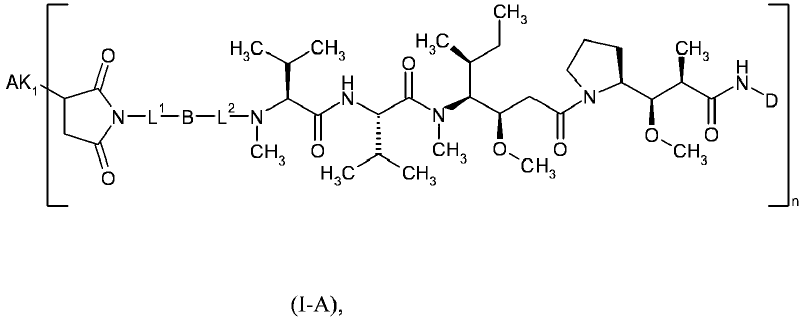 in welcher n, AK₁, D, L¹, B und L² jeweils die die in den Aspekten 1 bis 5 angegebenen Bedeutungen haben, umsetzt,
      oder
   [B] mit einer Verbindung der Formel (III) 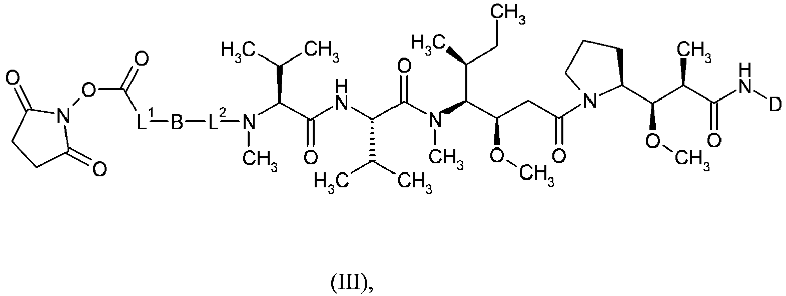 in welcher D, L¹, B und L² jeweils die die in den Aspekten 1 bis 5 angegebenen Bedeutungen haben,
      zu einer Verbindung der Formel (I-B) 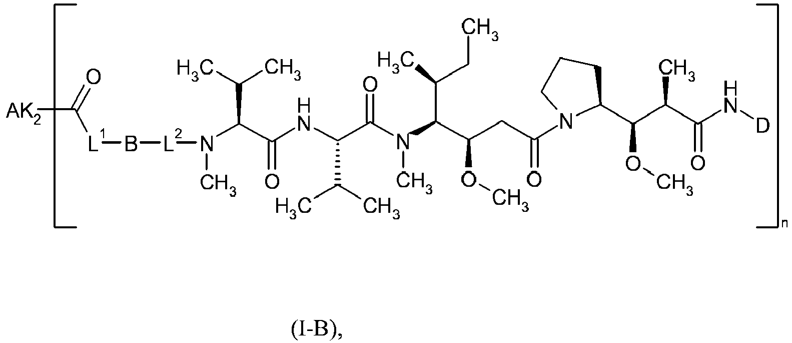 in welcher n, AK₂, D, L¹, B und L² jeweils die die in den Aspekten 1 bis 5 angegebenen Bedeutungen haben, umsetzt.
20. Verbindungen hergestellt nach dem Verfahren gemäß Anspruch 19 oder 50, wobei AK₁ und AK₂ für einen Antikörper, der die sechs CDR-Sequenzen des Antikörpers B01-3, B01-10 oder D02-6, die variable leichte und variable schwere Kette des Antikörpers B01-3, B01-10 oder D02-6 oder die leichte und schwere Kette des Antiköpers B01-3, B01-10 oder D02-6 umfasst, steht sowie ihre Salze, Solvate und Solvate der Salze.
21. Verbindungen der Formel (XXX) 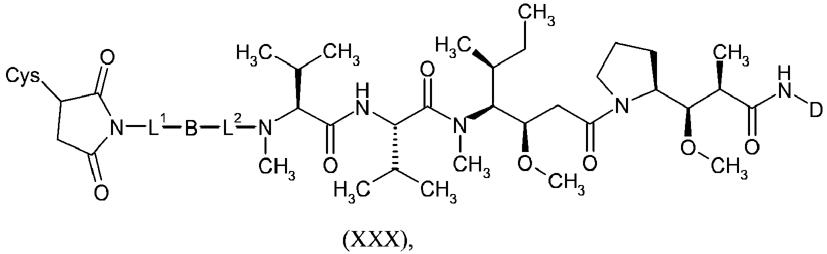 in welcher
   - Cys: für einen Cystein-Rest, der über das Schwefelatom der Seitenkette an ein Kohlenstoffatom des Succinimids gebunden ist, steht
   - L¹: für eine Bindung, lineares (C₁-C₁₀)-Alkandiyl oder für eine Gruppe der Formel 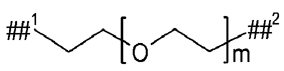 steht, wobei
   m für eine Zahl von 2 bis 6 steht,
   ##¹ die Verknüpfungsstelle mit der Gruppe G kennzeichnet,
   ##² die Verknüpfungsstelle mit der Gruppe B kennzeichnet,
   wobei (C₁-C₁₀)-Alkandiyl mit 1 bis 4 Substituenten Methyl substituiert sein kann,
   und
   wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
   - B: für eine Bindung oder eine Gruppe der Formel 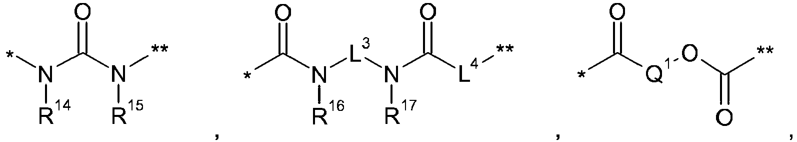 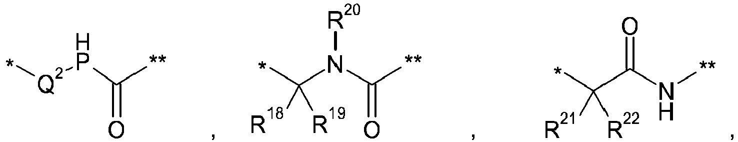 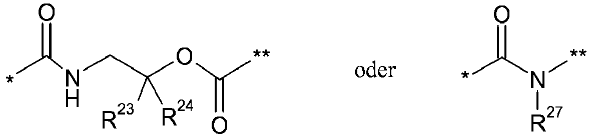 steht, wobei
   * die Verknüpfungsstelle mit L¹ kennzeichnet,
   ** die Verknüpfungsstelle mit L² kennzeichnet,
   P für O oder NH steht,
   L³ für eine Bindung, oder (C₂-C₄)-Alkandiyl steht,
   L⁴ für eine Bindung oder eine Gruppe der Formel 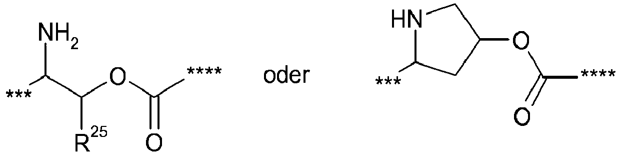 steht, worin
   *** die Verknüpfungsstelle mit der Carbonylgruppe kennzeichnet,
   **** die Verknüpfungsstelle mit L² kennzeichnet,
   R²⁵ für Wasserstoff oder Methyl steht,
   Q¹ für einen 3- bis 7-gliedrigen Carbocyclus oder einen 4- bis 7-gliedrigen Aza- Heterocyclus steht,
   Q² für einen 3- bis 7-gliedrigen Carbocyclus oder einen 4- bis 7-gliedrigen Aza- Heterocyclus steht,
   R¹⁴ für Wasserstoff oder (C₁-C₄)-Alkyl steht,
   R¹⁵ für Wasserstoff oder (C₁-C₄)-Alkyl steht,
   oder
   R¹⁴ und R¹⁵ zusammen mit den Atomen, an die sie gebunden sind, einen 5- oder 6-gliedrigen Heterocyclus bilden,
   R¹⁶ für Wasserstoff oder (C₁-C₄)-Alkyl steht,
   R¹⁷ für Wasserstoff oder (C₁-C₄)-Alkyl steht,
   oder
   R¹⁶ und R¹⁷ zusammen mit den Atomen, an die sie gebunden sind, einen 5- oder 6-gliedrigen Heterocyclus bilden,
   R¹⁸ für Wasserstoff oder (C₁-C₄)-Alkyl steht,
   R¹⁹ für Wasserstoff oder die Seitengruppe einer natürlichen α-Aminosäure oder ihrer Homologe oder Isomere steht,
   R²⁰ für Wasserstoff oder (C₁-C₄)-Alkyl steht,
   oder
   R¹⁹ und R²⁰ zusammen mit den Atomen, an die sie gebunden sind, einen Pyrrolidinylring bilden,
   R²¹ für Wasserstoff oder (C₁-C₄)-Alkyl steht,
   R²² für Wasserstoff oder (C₁-C₄)-Alkyl steht,
   oder
   R²¹ und R²² zusammen mit den Atomen, an die sie gebunden sind, einen 3- bis 7-gliedrigen Carbocyclus bilden,
   R²³ für (C₁-C₄)-Alkyl steht,
   R²⁴ für Wasserstoff oder (C₁-C₄)-Alkyl steht,
   R²⁷ für Wasserstoff oder (C₁-C₄)-Alkyl steht,
   - L²: für lineares (C₂-C₁₀)-Alkandiyl oder für eine Gruppe der Formel 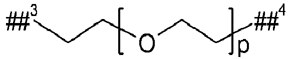 steht, wobei
   p für eine Zahl von 2 bis 6 steht,
   ##³ die Verknüpfungsstelle mit der Gruppe B kennzeichnet,
   ##⁴ die Verknüpfungsstelle mit dem Stickstoffatom kennzeichnet,
   wobei (C₂-C₁₀)-Alkandiyl mit 1 bis 4 Substituenten Methyl substituiert sein kann,
   und
   wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
   - D: für eine Gruppe der Formel 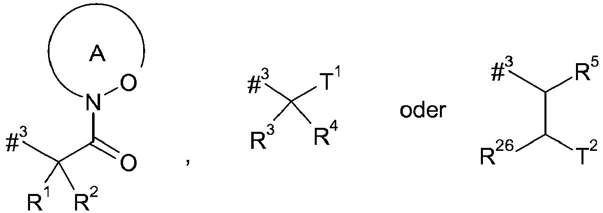 steht, wobei
   #³ die Verknüpfungsstelle mit dem Stickstoffatom kennzeichnet,
   R¹ für Wasserstoff steht,
   R² für 1-Hydroxyethyl, Benzyl, 1-Phenylethyl oder 1*H*-Indol-3-ylmethyl steht,
   oder
   R¹ und R² zusammen mit dem Kohlenstoffatom, an das sie gebunden sind, eine (1*S,*2*R*)-2-Phenylcyclopropan-1,1-diyl-Gruppe der Formel 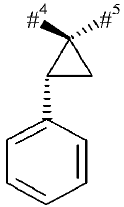 bilden, worin
   #⁴ die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 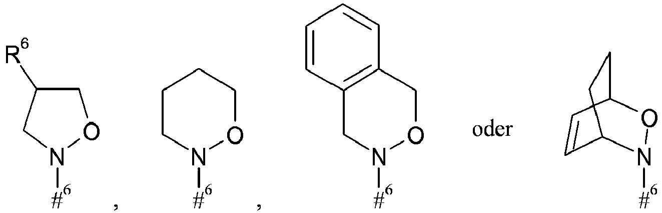 steht, worin
   #⁶ die Verknüpfungsstelle mit der Carbonyl-Gruppe kennzeichnet,
   R⁶ für Wasserstoff, Hydroxy oder Benzyloxy steht,
   R³ für Wasserstoff steht,
   R⁴ für 1-Hydroxyethyl, Benzyl, 1-Phenylethyl oder 1*H*-Indol-3-ylmethyl steht,
   oder
   R³ und R⁴ zusammen mit dem Kohlenstoffatom, an das sie gebunden sind, eine (1*S,*2*R*)-2-Phenylcyclopropan-1,1-diyl-Gruppe der Formel 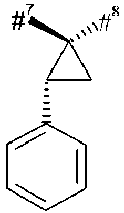 bilden, worin
   #⁷ die Verknüpfungsstelle mit dem angrenzenden Stickstoffatom kennzeichnet,
   #⁸ die Verknüpfungsstelle mit der Gruppe T¹ kennzeichnet,
   T¹ für eine Gruppe der Formel -C(=O)-OR⁷, -C(=O)-NR⁸R⁹, -C(=O)-NH-NH-R¹⁰ oder -CH₂-O-R¹¹ steht,
   worin
   R⁷ für Wasserstoff, Methyl, Ethyl, *n*-Propyl, *tert*.-Butyl, Benzyl oder Adamantylmethyl steht,
   R⁸ für Wasserstoff oder Methyl steht,
   R⁹ für Wasserstoff, Methyl, Ethyl, *n*-Propyl oder Benzyl steht,
   oder
   R⁸ und R⁹ bilden zusammen mit dem Stickstoffatom, an das sie gebunden sind, einen 4- bis 7-gliedrigen Heterocyclus,
   R¹⁰ für Benzoyl steht,
   R¹¹ für Benzyl, das in der Phenylgruppe mit Methoxycarbonyl oder Carboxyl substituiert sein kann, steht,
   R⁵ für Wasserstoff, Methyl oder eine Gruppe der Formel 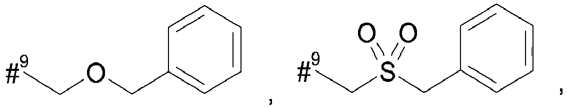 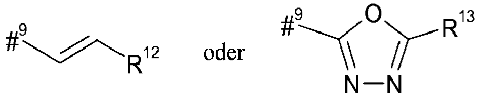 steht, worin
   #⁹ die Verknüpfungsstelle mit -CHC(R²⁶)-T² kennzeichnet,
   R¹² für Phenyl steht, das mit Methoxycarbonyl, Carboxyl oder einer Gruppe der Formel -S(O)₂OH substituiert sein kann,
   R¹³ für Phenyl steht, das mit Methoxycarbonyl oder Carboxyl substituiert sein kann,
   R²⁶ für Wasserstoff oder Hydroxy steht,
   T² für Phenyl, Benzyl, 1*H*-Indol-3-yl oder 1*H*-Indol-3-ylmethyl steht,
   sowie ihre Salze, Solvate und Solvate der Salze.
22. Verbindungen der Formel (XXX) nach Anspruch 19, in welcher
   - Cys: für einen Cystein-Rest steht, der über das Schwefelatom der Seitenkette über ein Kohlenstoffatom des Succinimids gebunden ist, steht
   - L¹: für eine Bindung, lineares (C₂-C₆)-Alkandiyl oder für eine Gruppe der Formel 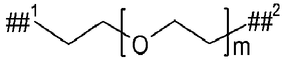 steht, wobei
   m für eine Zahl von 2 bis 6 steht,
   ##¹ die Verknüpfungsstelle mit der Gruppe G kennzeichnet,
   ##² die Verknüpfungsstelle mit der Gruppe B kennzeichnet,
   wobei (C₂-C₆)-Alkandiyl mit 1 oder 2 Substituenten Methyl substituiert sein kann,
   - B: für eine Bindung oder eine Gruppe der Formel 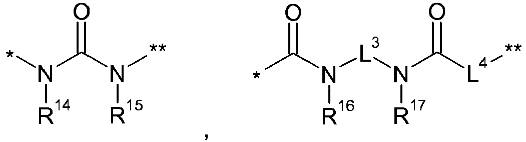 oder 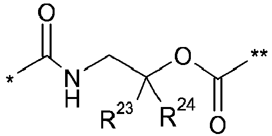 steht, wobei
   * die Verknüpfungsstelle mit L¹ kennzeichnet,
   ** die Verknüpfungsstelle mit L² kennzeichnet,
   L³ für eine Bindung oder Ethan-1,2-diyl steht,
   L⁴ für eine Bindung steht,
   R¹⁴ für Wasserstoff steht,
   R¹⁵ für Wasserstoff steht,
   R¹⁶ für Wasserstoff oder Methyl steht,
   R¹⁷ für Wasserstoff oder Methyl steht,
   oder
   R¹⁶ und R¹⁷ zusammen mit den Atomen, an die sie gebunden sind, einen Piperazinylring bilden,
   R²³ für Methyl steht,
   R²⁴ für Wasserstoff oder Methyl steht,
   - L²: für lineares (C₂-C₆)-Alkandiyl oder für eine Gruppe der Formel 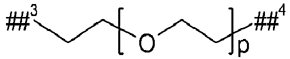 steht, wobei
   p für eine Zahl 2 oder 3 steht,
   ##³ die Verknüpfungsstelle mit der Gruppe B kennzeichnet,
   ##⁴ die Verknüpfungsstelle mit dem Stickstoffatom kennzeichnet,
   - D: für eine Gruppe der Formel 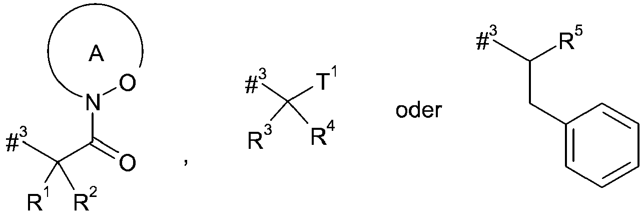 steht, wobei
   #³ die Verknüpfungsstelle mit dem Stickstoffatom kennzeichnet,
   R¹ für Wasserstoff steht,
   R² für 1-Hydroxyethyl, Benzyl, 1-Phenylethyl oder 1*H*-Indol-3-ylmethyl steht,
   oder
   R¹ und R² zusammen mit dem Kohlenstoffatom, an das sie gebunden sind, eine (1*S,*2*R*)-2-Phenylcyclopropan-1,1-diyl-Gruppe der Formel 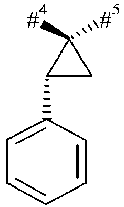 bilden, worin
   #⁴ die Verknüpfungsstelle mit dem angrenzenden Stickstoffatom kennzeichnet,
   #⁵ die Verknüpfungsstelle mit der Carbonyl-Gruppe kennzeichnet,
   der Ring A mit der darin enthaltenen N-O-Gruppierung für einen mono- oder bicyclischen, gegebenenfalls substituierten Heterocyclus der Formel 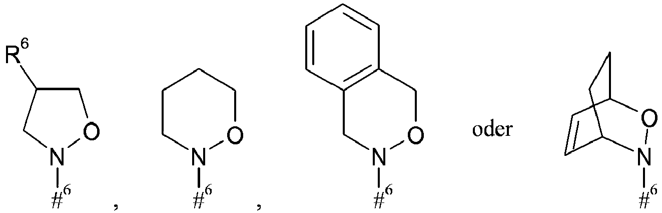 steht, worin
   #⁶ die Verknüpfungsstelle mit der Carbonyl-Gruppe kennzeichnet,
   R⁶ für Wasserstoff, Hydroxy oder Benzyloxy steht,
   R³ für Wasserstoff steht,
   R⁴ für Benzyl, 1-Phenylethyl oder 1*H*-Indol-3-ylmethyl steht,
   oder
   R³ und R⁴ zusammen mit dem Kohlenstoffatom, an das sie gebunden sind, eine (1*S,*2*R*)-2-Phenylcyclopropan-1,1-diyl-Gruppe der Formel 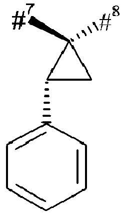 bilden, worin
   #⁷ die Verknüpfungsstelle mit dem angrenzenden Stickstoffatom kennzeichnet,
   #⁸ die Verknüpfungsstelle mit der Gruppe T kennzeichnet,
   T¹ für eine Gruppe der Formel -C(=O)-OR⁷, -C(=O)-NR⁸R⁹, -C(=O)-NH-NH-R¹⁰ oder -CH₂-O-R¹¹ steht,
   worin
   R⁷ für Wasserstoff, Methyl, Ethyl, *n*-Propyl, Benzyl oder Adamantylmethyl steht,
   R⁸ für Wasserstoff oder Methyl steht,
   R⁹ für Wasserstoff, Methyl, Ethyl, *n*-Propyl oder Benzyl steht,
   R¹⁰ für Benzoyl steht,
   R¹¹ für Benzyl, das in der Phenylgruppe mit Methoxycarbonyl oder Carboxyl substituiert sein kann, steht,
   R⁵ für Wasserstoff oder eine Gruppe der Formel 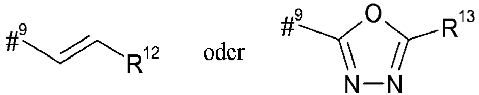 steht, worin
   #⁹ die Verknüpfungsstelle mit -CHC(R²⁶)Phenyl kennzeichnet,
   R¹² für Phenyl steht, das mit Methoxycarbonyl, Carboxyl oder einer Gruppe der Formel -S(O)₂OH substituiert sein kann,
   R¹³ für Phenyl steht, das mit Methoxycarbonyl oder Carboxyl substituiert sein kann,
   sowie ihre Salze, Solvate und Solvate der Salze.
24. Binder-drug conjugates of the general formula (I) or (Ia) according to any of Claims 1 to 5 or 17 or 18 or a conjugate or compound according to any of Claim 21 or 23 wherein n is from 1 to 10.
25. Compound having one of the following structures,
23. Ein Binder-Wirkstoff-Konjugat nach einem der Aspekte 1 - 6, 14 - 18 wobei der Binder an ein Krebs-Zielmolekül bindet.
24. Ein Binder-Wirkstoff-Konjugat nach einem der Aspekte 1 - 6, 14 - 18 oder 23, wobei der Binder an ein extrazelluläres Zielmolekül bindet.
25. Ein Binder-Wirkstoff-Konjugat nach einem der Aspekte 1 - 6, 14 - 18 oder 23 - 24, wobei der Binder an ein extrazelluläres Krebs-Zielmolekül bindet.
26. Ein Binder-Wirkstoff-Konjugat nach einem der Aspekte 1 - 6, 14 - 18 oder 23 - 25, wobei das Zielmolekül, extrazelluläre Zielmolekül, Krebs-Zielmolekül oder extrazelluläre Krebs-Zielmolekül ein Protein ist.
27. Ein Binder-Wirkstoff-Konjugat nach einem der Aspekte 1 - 6, 14 - 18 oder 23 - 26, wobei der Binder nach Bindung an das extrazelluläre Zielmolekül von der das Zielmolekül exprimierenden Zelle internalisiert wird.
28. Ein Binder-Wirkstoff-Konjugat nach einem der Aspekte 1 - 6, 14 - 18 oder 23 - 27, wobei der Binder ein Bindeprotein ist.
29. Ein Binder-Wirkstoff-Konjugat nach einem der Aspekte 1 - 6, 14, 15, 20 oder 23 - 28, wobei der Binder ein Antikörper oder ein antigen-bindendes Antikörperfragment davon oder ein Antikörpermimetikum ist.
30. Ein Binder-Wirkstoff-Konjugat nach einem der Aspekte 1 - 6, 14 - 18 oder 23 - 29, wobei der Antikörper ein monoklonaler Antikörper ist.
31. Ein Binder-Wirkstoff-Konjugat nach einem der Aspekte 1 - 6, 14 - 18 oder 23 - 30, wobei der Antikörper ein humaner, humanisierter oder chimärer Antikörper ist.
32. Ein Binder-Wirkstoff-Konjugat nach einem der Aspekte 1 - 6, 14 - 18 oder 23 - 31, wobei der Antikörper ein intakter oder ein modifizierter intakter Antikörper ist.
33. Ein Binder-Wirkstoff-Konjugat nach einem der Aspekte 1 - 6, 14 - 18 oder 23 - 32, wobei der Antikörper ein Antikörper der IgG Klasse ist.
34. Ein Binder-Wirkstoff-Konjugat nach einem der Aspekte 1 - 6, 14 -16, 20 oder 23 - 33, wobei der Binder an ein extrazelluläres Krebs-Zielmolekül bindet, welches ausgewählt wird aus der Gruppe bestehend aus EGF-Rezeptor (NP _005219.2), Mesothelin (Q13421-3), C4.4a (NP_055215.2) und Carboanhydrase IX (CA IX; NP_001207.2).
35. Ein Binder-Wirkstoff-Konjugat nach einem der Aspekte 1 - 6, 14 -16, 20 oder 23 - 34, wobei der Binder spezifisch an ein extrazelluläres Krebs-Zielmolekül bindet, welches ausgewählt wird aus der Gruppe bestehend aus EGF-Rezeptor (NP_005219.2), Mesothelin (Q13421-3), C4.4a (NP_055215.2) und Carboanhydrase IX (CA IX; Q16790).
36. Ein Binder-Wirkstoff-Konjugat nach einem der Aspekte 1 - 6, 14 -16, 20 oder 23 - 35, wobei der Antikörper an C4.4a bindet.
37. Ein Binder-Wirkstoff-Konjugat nach einem der Aspekte 1 - 6, 14-18 oder 23 - 36, wobei der Antikörper an die S1 Domäne (Aminosäureposition 1-85 von SEQ ID NO: 1) von C4.4a. bindet.
38. Ein Binder-Wirkstoff-Konjugat nach einem der Aspekte 1 - 6, 14-18 oder 23 - 37, wobei der Antikörper spezifisch an C4.4a bindet.
39. Ein Binder-Wirkstoff-Konjugat nach einem der Aspekte 1 - 6, 14-18 oder 23 - 38, wobei der Binder ein anti-C4.4a Antikörper oder antigen-bindendes Antikörperfragment ist, der mit Antikörper M31-B01 und/oder mit dem Antikörper M20-D02-S-A um Bindung an C4.4a kompetiert.
40. Ein Binder-Wirkstoff-Konjugat nach einem der Aspekte 1 - 6, 14 - 16, 20 oder 23 - 39, ausgewählt aus der Gruppe bestehend aus Antikörper, die die CDR Sequenzen der variablen schweren Kette wiedergegeben durch die Sequenzen SEQ ID NO: 75-77 und der die CDR Sequenzen der variablen leichten Kette wiedergegeben durch die Sequenzen SEQ ID NO: 78-80 umfasst (B01-10), Antikörper, der die CDR Sequenzen der variablen schweren Kette wiedergegeben durch die Sequenzen SEQ ID NO: 5, 9 und 13 und der die CDR Sequenzen der variablen leichten Kette wiedergegeben durch die Sequenzen SEQ ID NO: 17, 21 und 25 umfasst (M31-B01), Antikörper, der die CDR Sequenzen der variablen schweren Kette wiedergegeben durch die Sequenzen SEQ ID NO: 6, 10 und 14 und der die CDR Sequenzen der variablen leichten Kette wiedergegeben durch die Sequenzen SEQ IDNO: 18, 22 und 26 umfasst (M20-D02-S-A), Antikörper, der die CDR Sequenzen der variablen schweren Kette wiedergegeben durch die Sequenzen SEQ ID NO: 7, 11 und 15und der die CDR Sequenzen der variablen leichten Kette wiedergegeben durch die Sequenzen SEQ ID NO: 19, 23 und 27 umfasst (M60-G03), Antikörper, der die CDR Sequenzen der variablen schweren Kette wiedergegeben durch die Sequenzen SEQ ID NO: 8, 12 und 16 und der die CDR Sequenzen der variablen leichten Kette wiedergegeben durch die Sequenzen SEQ ID NO: 20, 24 und 28 umfasst (36-H02), Antikörper, der die CDR Sequenzen der variablen schweren Kette wiedergegeben durch die Sequenzen SEQ ID NO: 45-47 und der die CDR Sequenzen der variablen leichten Kette wiedergegeben durch die Sequenzen SEQ ID NO: 48-50 umfasst (B01-3), Antikörper, der die CDR Sequenzen der variablen schweren Kette wiedergegeben durch die Sequenzen SEQ ID NO: 55-57 und der die CDR Sequenzen der variablen leichten Kette wiedergegeben durch die Sequenzen SEQ ID NO: 58-60 umfasst (B01-5), Antikörper, der die CDR Sequenzen der variablen schweren Kette wiedergegeben durch die Sequenzen SEQ ID NO: 65-67 und der die CDR Sequenzen der variablen leichten Kette wiedergegeben durch die Sequenzen SEQ ID NO: 68-70 umfasst (B01-7), Antikörper, der die CDR Sequenzen der variablen schweren Kette wiedergegeben durch die Sequenzen SEQ ID NO: 85-87 und der die CDR Sequenzen der variablen leichten Kette wiedergegeben durch die Sequenzen SEQ ID NO: 88-90 umfasst (B01-12), Antikörper, der die CDR Sequenzen der variablen schweren Kette wiedergegeben durch die Sequenzen SEQ ID NO: 95-97 und der die CDR Sequenzen der variablen leichten Kette wiedergegeben durch die Sequenzen SEQ ID NO: 98-100 umfasst (D02-4), Antikörper, der die CDR Sequenzen der variablen schweren Kette wiedergegeben durch die Sequenzen SEQ ID NO: 105-107 und der die CDR Sequenzen der variablen leichten Kette wiedergegeben durch die Sequenzen SEQ ID NO: 108-110 umfasst (D02-6), Antikörper, der die CDR Sequenzen der variablen schweren Kette wiedergegeben durch die Sequenzen SEQ ID NO: 115-117 und der die CDR Sequenzen der variablen leichten Kette wiedergegeben durch die Sequenzen SEQ ID NO: 118-120 umfasst (D02-7), Antikörper, der die CDR Sequenzen der variablen schweren Kette wiedergegeben durch die Sequenzen SEQ ID NO: 125-127 und der die CDR Sequenzen der variablen leichten Kette wiedergegeben durch die Sequenzen SEQ ID NO: 128-130 umfasst (D02-11), und Antikörper, der die CDR Sequenzen der variablen schweren Kette wiedergegeben durch die Sequenzen SEQ ID NO: 135-137 der die CDR Sequenzen der variablen leichten Kette wiedergegeben durch die Sequenzen SEQ ID NO: 138-140 umfasst (D02-13).
41. Ein Binder-Wirkstoff-Konjugat nach einem der Aspekte 1 - 6, 14 - 18, 20 oder 23 - 40, wobei der C4.4a Antikörper oder antigen-bindenden Antikörperfragmente ausgewählt wird aus der Gruppe bestehend aus Antikörper, die die Aminosäuresequenz der variablen schweren Kette wiedergegeben durch die Sequenz SEQ ID NO: 81 und die die Aminosäuresequenz der variablen leichten Kette wiedergegeben durch die Sequenz SEQ ID NO: 82 umfassen (B01-7),
   Antikörper, die die Aminosäuresequenz der variablen schweren Kette wiedergegeben durch die Sequenz SEQ ID NO: 33 und die die Aminosäuresequenz der variablen leichten Kette wiedergegeben durch die Sequenz SEQ ID NO: 29 umfassen (M31-B01), Antikörper, die die Aminosäuresequenz der variablen schweren Kette wiedergegeben durch die Sequenz SEQ ID NO: 34 und die die Aminosäuresequenz der variablen leichten Kette wiedergegeben durch die Sequenz SEQ ID NO: 30 umfassen (M20-D02 S-A), Antikörper, die die Aminosäuresequenz der variablen schweren Kette wiedergegeben durch die Sequenz SEQ ID NO: 35 und die die Aminosäuresequenz der variablen leichten Kette wiedergegeben durch die Sequenz SEQ ID NO: 31 umfassen (M60-G03), Antikörper, die die Aminosäuresequenz der variablen schweren Kette wiedergegeben durch die Sequenz SEQ ID NO: 36 und die die Aminosäuresequenz der variablen leichten Kette wiedergegeben durch die Sequenz SEQ ID NO: 32 umfassen (M36-H02), Antikörper, die die Aminosäuresequenz der variablen schweren Kette wiedergegeben durch die Sequenz SEQ ID NO: 51 und die die Aminosäuresequenz der variablen leichten Kette wiedergegeben durch die Sequenz SEQ ID NO: 52 umfassen (B01-3), Antikörper, die die Aminosäuresequenz der variablen schweren Kette wiedergegeben durch die Sequenz SEQ ID NO: 61 und die die Aminosäuresequenz der variablen leichten Kette wiedergegeben durch die Sequenz SEQ ID NO: 62 umfassen (B01-5), Antikörper, die die Aminosäuresequenz der variablen schweren Kette wiedergegeben durch die Sequenz SEQ ID NO: 71 und die die Aminosäuresequenz der variablen leichten Kette wiedergegeben durch die Sequenz SEQ ID NO: 72 umfassen (B01-7), Antikörper, die die Aminosäuresequenz der variablen schweren Kette wiedergegeben durch die Sequenz SEQ ID NO: 91 und die die Aminosäuresequenz der variablen leichten Kette wiedergegeben durch die Sequenz SEQ ID NO: 92 umfassen (B01-12), Antikörper, die die Aminosäuresequenz der variablen schweren Kette wiedergegeben durch die Sequenz SEQ ID NO: 101 und die die Aminosäuresequenz der variablen leichten Kette wiedergegeben durch die Sequenz SEQ ID NO: 102 umfassen (D02-4), Antikörper, die die Aminosäuresequenz der variablen schweren Kette wiedergegeben durch die Sequenz SEQ ID NO: 111 und die die Aminosäuresequenz der variablen leichten Kette wiedergegeben durch die Sequenz SEQ ID NO: 112 umfassen (D02-6), Antikörper, die die Aminosäuresequenz der variablen schweren Kette wiedergegeben durch die Sequenz SEQ ID NO: 121 und die die Aminosäuresequenz der variablen leichten Kette wiedergegeben durch die Sequenz SEQ ID NO: 122 umfassen (D02-7), Antikörper, die die Aminosäuresequenz der variablen schweren Kette wiedergegeben durch die Sequenz SEQ ID NO: 131 und die die Aminosäuresequenz der variablen leichten Kette wiedergegeben durch die Sequenz SEQ ID NO: 132 umfassen (D02-11), und Antikörper, die die Aminosäuresequenz der variablen schweren Kette wiedergegeben durch die Sequenz SEQ ID NO: 141 und die die Aminosäuresequenz der variablen leichten Kette wiedergegeben durch die Sequenz SEQ ID NO: 142 umfassen (D02-13).
42. Ein Binder-Wirkstoff-Konjugat nach einem der Aspekte 1 - 6, 14 - 18, 20 oder 23 - 41, wobei der C4.4a Antikörper oder antigen-bindenden Antikörperfragment ausgewählt wird aus der Gruppe bestehend aus
   Antikörper, der die Aminosäuresequenz der leichten Kette repräsentiert durch SEQ ID NO: 346 und der die Aminosäuresequenz der schweren Kette repräsentiert durch SEQ ID NO: 347 umfasst (M31-B01) umfasst,
   Antikörper, der die Aminosäuresequenz der leichten Kette repräsentiert durch SEQ ID NO: 352 und der die Aminosäuresequenz der schweren Kette repräsentiert durch SEQ ID NO: 353 umfasst (B01-3) umfasst,
   Antikörper, der die Aminosäuresequenz der leichten Kette repräsentiert durch SEQ ID NO: 364 und der die Aminosäuresequenz der schweren Kette repräsentiert durch SEQ ID NO: 365 umfasst (B01-10) umfasst, und
   Antikörper, der die Aminosäuresequenz der leichten Kette repräsentiert durch SEQ ID NO: 382 und der die Aminosäuresequenz der schweren Kette repräsentiert durch SEQ ID NO: 383 umfasst (D02-6) umfasst.
43. Ein Binder-Wirkstoff-Konjugat oder eine Verbindung nach einem der Aspekte 1 - 18 oder 20 - 42, zur Behandlung und/oder Prophylaxe von Krankheiten.
44. Ein Binder-Wirkstoff-Konjugat oder eine Verbindung nach einem der Aspekte 1 - 18 oder 20 - 42 zur Verwendung in einem Verfahren zur Behandlung und/ oder Prophylaxe von hyperproliferativen und/oder angiogenen Erkrankungen.
45. Ein Binder-Wirkstoff-Konjugat oder eine Verbindung nach einem der Aspekte 1 - 18 oder 20 - 42 zur Herstellung eines Arzneimittels zur Behandlung und/oder Prophylaxe von hyperproliferativen und/oder angiogenen Erkrankungen.
46. Arzneimittel enthaltend ein Binder-Wirkstoff-Konjugat oder eine Verbindung nach einem der Aspekte 1 - 18 oder 20 - 42, in Kombination mit einem inerten, nicht-toxischen, pharmazeutisch geeigneten Hilfsstoff.
47. Arzneimittel enthaltend ein Binder-Wirkstoff-Konjugat oder eine Verbindung nach einem der Aspekte 1 - 18 oder 20 - 42, in Kombination mit einer oder mehreren anti-hyperproliferativen, zytostatischen oder zytotoxischen Substanzen.
48. Arzneimittel nach Anspruch 46 - 47 zur Behandlung und/oder Prophylaxe von hyperproliferativen und/oder angiogenen Erkrankungen.
49. Verfahren zur Behandlung und/oder Prophylaxe von hyperproliferativen und/oder angiogenen Erkrankungen bei Menschen und Tieren unter Verwendung einer wirksamen Menge mindestens eines Binder-Wirkstoff-Konjugates oder einer Verbindung nach einem der Aspekte 1 - 18 oder 20 - 42, oder eines Arzneimittels, wie in einem der Aspekte 46 bis 48 definiert.
50. Verfahren zur Herstellung der erfindungsgemäßen Verbindungen nach einem der Aspekte 1 bis 6, der allgemeinen Formel (la), dadurch gekennzeichnet, dass man eine Lösung des Binders in einem Puffer
   [A] mit einem geeigneten Reduktionsmittel, wie beispielsweise Dithiothreitol oder Tris(2-carboxyethyl)phosphin-Hydrochlorid, versetzt, und anschließend mit einer Verbindung der Formel (IIa) 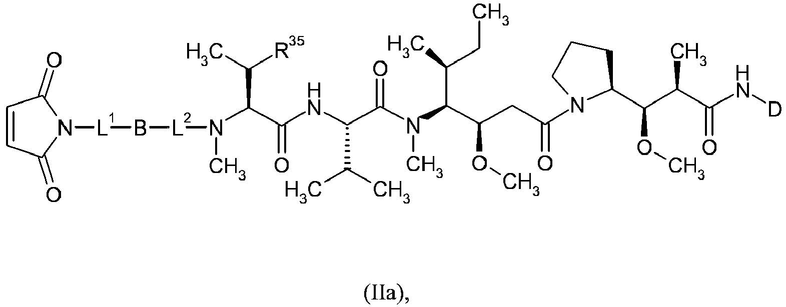 in welcher D, L¹, B, L² und R³⁵ jeweils die in den Aspekten 1 bis 6 angegebenen Bedeutungen haben,
      zu einer Verbindung der Formel (Ia-A) 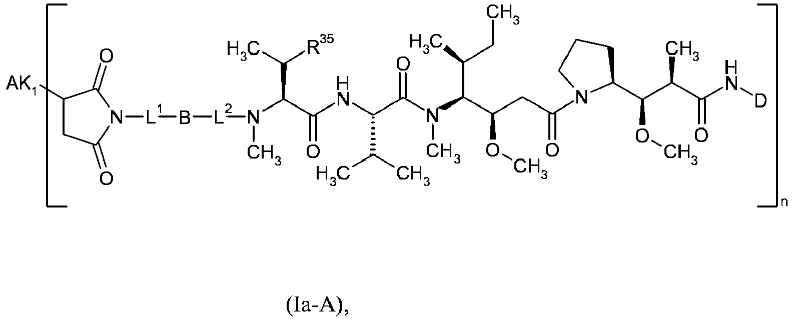 in welcher n, AK₁, D, L¹, B, L² und R³⁵ jeweils die in den Aspekten 1 bis 6 angegebenen Bedeutungen haben,
      umsetzt,
      oder
   [B] mit einer Verbindung der Formel (IIIa) 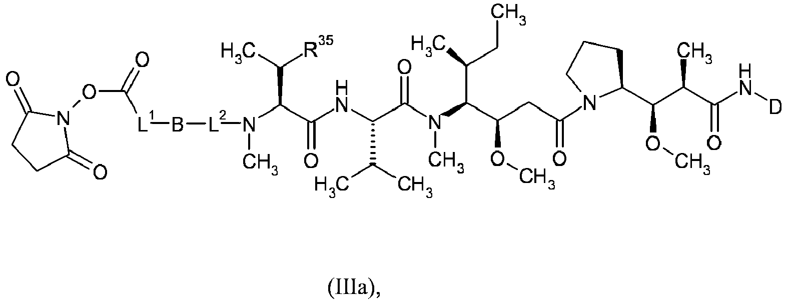 in welcher D, L¹, B, L² und R³⁵ jeweils die in den Aspekten 1 bis 6 angegebenen Bedeutungen haben,
      zu einer Verbindung der Formel (Ia-B) 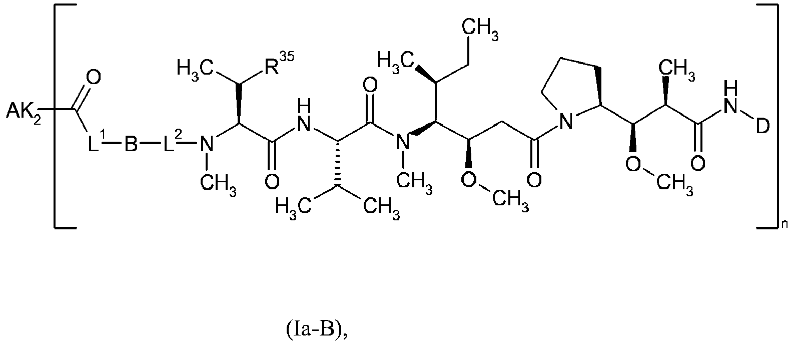 in welcher n, AK₂, D, L¹, B, L² und R³⁵ jeweils die in den Aspekten 1 bis 6 angegebenen Bedeutungen haben,
      umsetzt.
38. Binder-Wirkstoff Konjugate ausgewählt aus den folgenden: 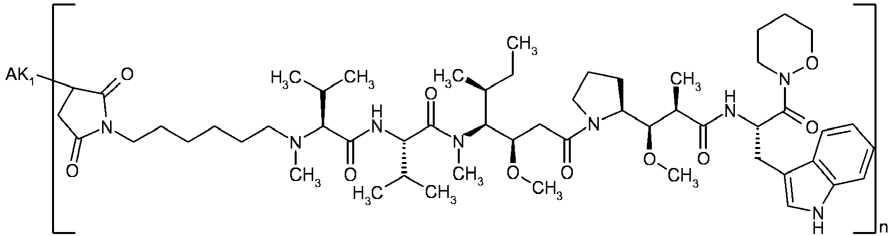 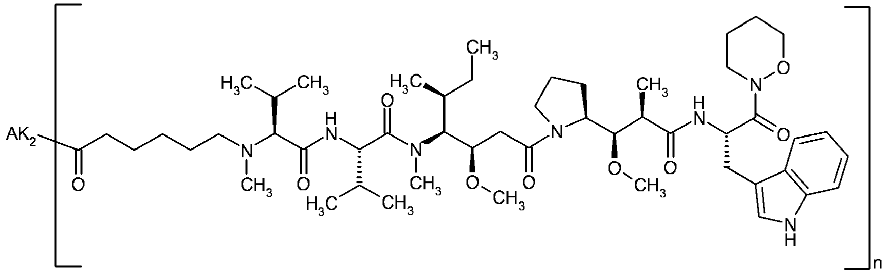 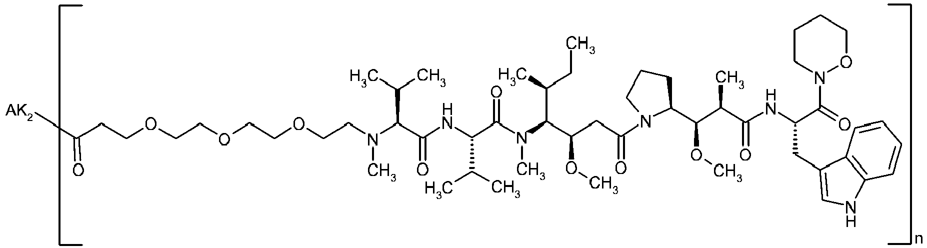 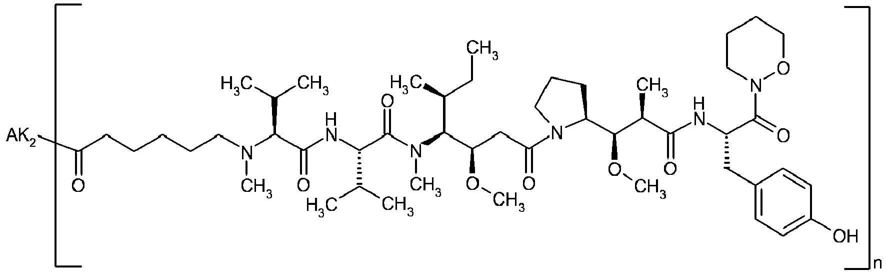 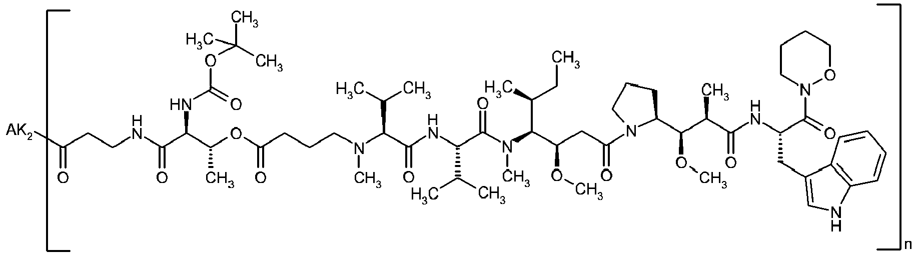 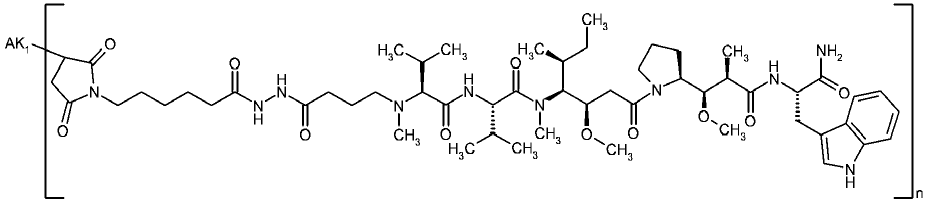 wobei in jeder der Verbindungen
   - n: für eine Zahl von 2 bis 8, vorzugsweise 2 bis 5 steht,
   und
   - AK₁: für einen Antikörper oder ein Antigen-bindendes Antikörperfragment steht, der bzw. das an C4.4a bindet und über das Schwefelatom eines Cystein-Restes des Binders an die Gruppe G gebunden ist,
   - AK₂: für einen Antikörper oder ein Antigen-bindendes Antikörperfragment steht, der bzw. das an C4.4a bindet und über die NH-Seitengruppe eines Lysin-Restes des Binders an die Gruppe G gebunden ist,
   ,
   sowie ihre Salze, Solvate und Solvate der Salze.

## Patentansprüche

1. Verbindungen der Formel (XXXa) 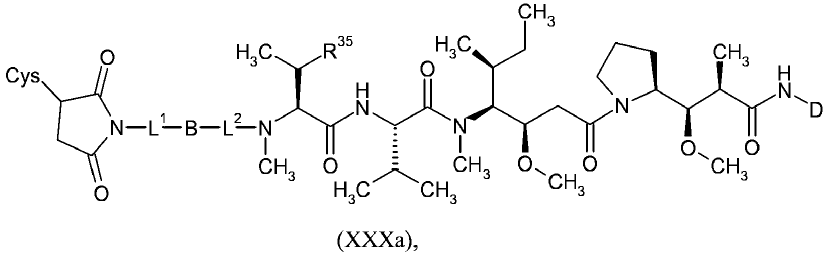 in welcher
Cys für einen Cystein-Rest steht, der über das Schwefelatom der Seitenkette an ein Kohlenstoffatom des Succinimids gebunden ist,
L¹ für eine Bindung, lineares (C₁-C₁₀)-Alkandiyl, oder eine Gruppe der Formel 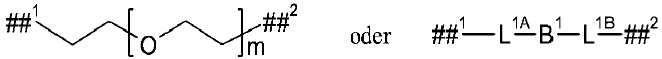 steht, wobei
m für eine Zahl von 2 bis 6 steht,
##¹ die Verknüpfungsstelle mit dem angrenzenden Stickstoffatom kennzeichnet,
##² die Verknüpfungsstelle mit der Gruppe B kennzeichnet,
L^{1A} für lineares (C₂-C₁₀)-Alkandiyl steht,
B¹ für eine Gruppe der Formel 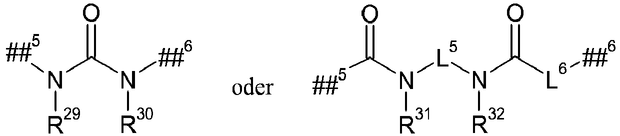 steht, worin
##⁵ die Verknüpfungsstelle mit der Gruppe L^{1A} kennzeichnet,
##⁶ die Verknüpfungsstelle mit der Gruppe L^{1B} kennzeichnet,
L⁵ für eine Bindung oder (C₂-C₄)-Alkandiyl steht,
L⁶ für eine Bindung steht,
R²⁹ für Wasserstoff oder (C₁-C₄)-Alkyl steht,
R³⁰ für Wasserstoff oder (C₁-C₄)-Alkyl steht,
oder
R²⁹ und R³⁰ zusammen mit den Atomen, an die sie gebunden sind, einen 5- oder 6-gliedrigen Heterocyclus bilden,
R³¹ für Wasserstoff oder (C₁-C₄)-Alkyl steht,
R³² für Wasserstoff oder (C₁-C₄)-Alkyl steht,
oder
R³¹ und R³² zusammen mit den Atomen, an die sie gebunden sind, einen 5- oder 6-gliedrigen Heterocyclus bilden,
L^{1B} für lineares (C₂-C₁₀)-Alkandiyl steht,
und
wobei (C₁-C₁₀)-Alkandiyl mit 1 bis 4 Substituenten unabhängig voneinander ausgewählt aus der Gruppe Methyl, Hydroxy und Benzyl substituiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
B für eine Bindung oder eine Gruppe der Formel 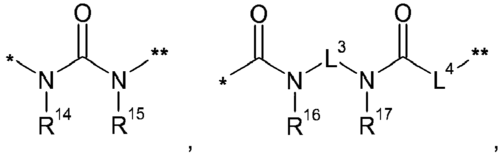 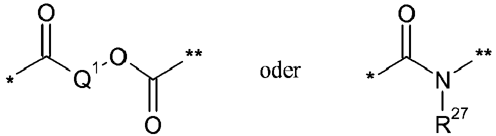 steht, wobei
* die Verknüpfungsstelle mit L¹ kennzeichnet,
** die Verknüpfungsstelle mit L² kennzeichnet,
L³ für eine Bindung oder (C₂-C₄)-Alkandiyl steht,
L⁴ für eine Bindung steht,
Q¹ für einen 4- bis 7-gliedrigen Heterocyclus steht,
R¹⁴ für Wasserstoff oder (C₁-C₄)-Alkyl steht,
R¹⁵ für Wasserstoff oder (C₁-C₄)-Alkyl steht,
oder
R¹⁴ und R¹⁵ zusammen mit den Atomen, an die sie gebunden sind, einen 5- oder 6-gliedrigen Heterocyclus bilden,
R¹⁶ für Wasserstoff oder (C₁-C₄)-Alkyl steht,
R¹⁷ für Wasserstoff oder (C₁-C₄)-Alkyl steht,
oder
R¹⁶ und R¹⁷ zusammen mit den Atomen, an die sie gebunden sind, einen 5- oder 6-gliedrigen Heterocyclus bilden,
R²⁷ für Wasserstoff oder (C₁-C₄)-Alkyl steht,
L² für lineares (C₂-C₁₀)-Alkandiyl oder für eine Gruppe der Formel 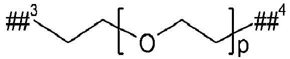 steht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
wobei (C₂-C₁₀)-Alkandiyl mit 1 bis 4 Substituenten unabhängig voneinander ausgewählt aus der Gruppe Methyl, Hydroxy und Benzyl substituiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
D für eine Gruppe der Formel 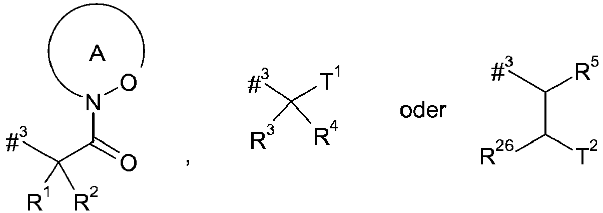 steht, wobei
#³ die Verknüpfungsstelle mit dem Stickstoffatom kennzeichnet,
R¹ für Wasserstoff oder Methyl steht,
R² für Isopropyl, Isobutyl, sec.-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenylethyl, Diphenylmethyl, 1*H*-Imidazol-4-ylmethyl oder 1*H*-Indol-3-ylmethyl steht,
oder
R¹ und R² zusammen mit dem Kohlenstoffatom, an das sie gebunden sind, eine (1*S,*2*R*)-2-Phenylcyclopropan-1,1-diyl-Gruppe der Formel 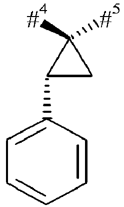 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bicyclischen, gegebenenfalls substituierten Heterocyclus der Formel 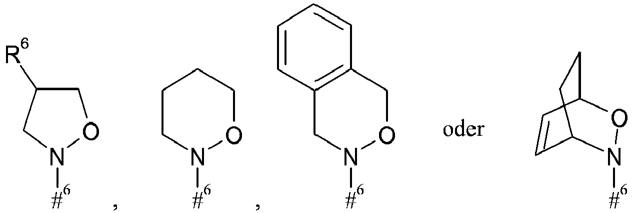 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff oder Methyl steht,
R⁴ für Isopropyl, Isobutyl, *sec*.-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenylethyl, Diphenylmethyl, 1*H*-Imidazol-4-ylmethyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 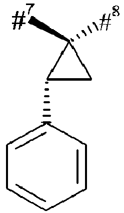 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹, -C(=O)-NH-NH-R¹⁰ oder -CH₂-O-R¹¹ steht,
worin
R⁷ für Wasserstoff, Methyl, Ethyl, *n*-Propyl, *tert*.-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
oder
R⁸ und R⁹ zusammen mit dem Stickstoffatom, an das sie gebunden sind, einen 4- bis 7-gliedrigen Heterocyclus bilden,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der Formel 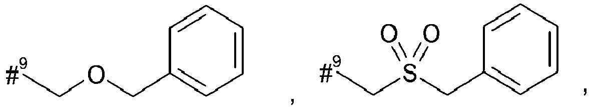 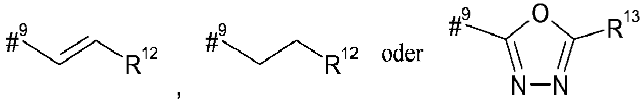 steht, worin
#⁹ die Verknüpfungsstelle mit -CHC(R²⁶)-T² kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
R²⁶ für Wasserstoff oder Hydroxy steht,
T² für Phenyl, Benzyl, 1*H*-Indol-3-yl oder 1*H*-Indol-3-ylmethyl steht,
R³⁵ für Methyl oder Hydroxy steht,
sowie ihre Salze, Solvate und Solvate der Salze.

2. Verbindungen der Formel (XXXa) 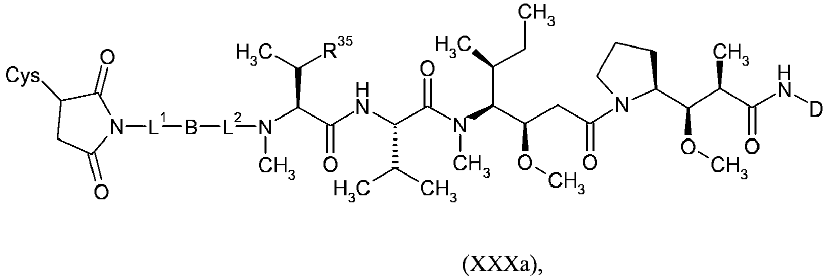 in welcher
Cys für einen Cystein-Rest, der über das Schwefelatom der Seitenkette über ein Kohlenstoffatom des Succinimids gebunden ist, steht
L¹ für eine Bindung, lineares (C₂-C₆)-Alkandiyl, eine Gruppe der Formel 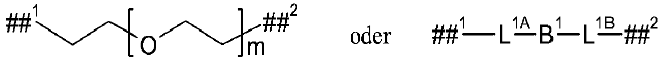 steht, wobei
m für eine Zahl von 2 oder 3 steht,
##¹ die Verknüpfungsstelle mit dem angrenzenden Stickstoffatom kennzeichnet,
##² die Verknüpfungsstelle mit der Gruppe B kennzeichnet,
L^{1A} für lineares (C₂-C₆)-Alkandiyl steht,
B¹ für eine Gruppe der Formel 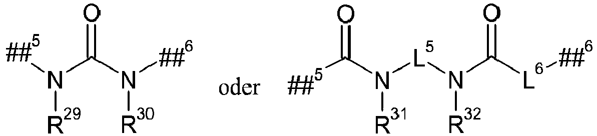 steht, worin
##⁵ die Verknüpfungsstelle mit der Gruppe L^{1A} kennzeichnet,
##⁶ die Verknüpfungsstelle mit der Gruppe L^{1B} kennzeichnet,
L⁵ für eine Bindung steht,
L⁶ für eine Bindung steht,
R²⁹ für Wasserstoff steht,
R³⁰ für Wasserstoff steht,
R³¹ für Wasserstoff oder Methyl steht,
R³² für Wasserstoff oder Methyl steht,
L^{1B} für lineares (C₂-C₆)-Alkandiyl steht,
und
wobei (C₂-C₆)-Alkandiyl mit 1 oder 2 Methylsubstituenten substituiert sein kann,
B für eine Bindung oder eine Gruppe der Formel 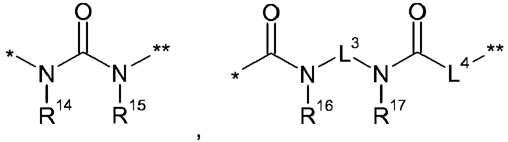 oder 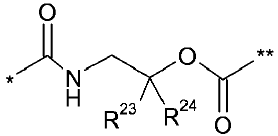 steht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Bindung steht,
R¹⁴ für Wasserstoff steht,
R¹⁵ für Wasserstoff steht,
R¹⁶ für Wasserstoff oder Methyl steht,
R¹⁷ für Wasserstoff oder Methyl steht,
oder
R¹⁶ und R¹⁷ zusammen mit den Atomen, an die sie gebunden sind, einen Piperazinylring bilden,
R²³ für Methyl steht,
R²⁴ für Wasserstoff oder Methyl steht,
L² für lineares (C₂-C₆)-Alkandiyl oder für eine Gruppe der Formel 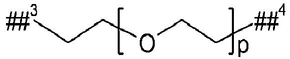 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
D für eine Gruppe der Formel 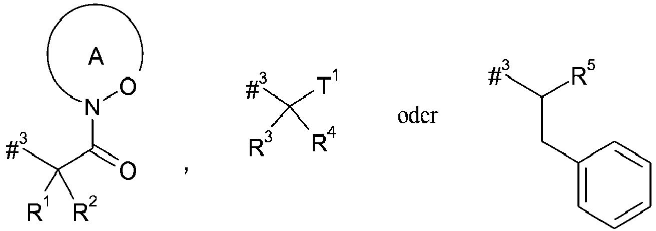 steht, wobei
#³ die Verknüpfungsstelle mit dem Stickstoffatom kennzeichnet,
R¹ für Wasserstoff steht,
R² für 1-Hydroxyethyl, Benzyl, 4-Hydroxybenzyl, 1-Phenylethyl oder 1*H*-Indol-3-ylmethyl steht,
oder
R¹ und R² zusammen mit dem Kohlenstoffatom, an das sie gebunden sind, eine (1*S,*2*R*)-2-Phenylcyclopropan-1,1-diyl-Gruppe der Formel 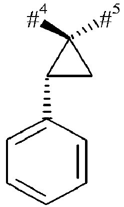 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bicyclischen, gegebenenfalls substituierten Heterocyclus der Formel 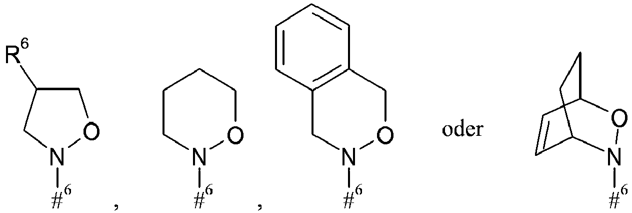 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff steht,
R⁴ für 1-Hydroxyethyl, Benzyl, 4-Hydroxybenzyl, 1-Phenylethyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 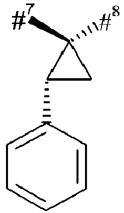 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹, -C(=O)-NH-NH-R¹⁰ oder -CH₂-O-R¹¹ steht,
worin
R⁷ für Wasserstoff, Methyl, Ethyl, *n*-Propyl, *tert*.-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
oder
R⁸ und R⁹ zusammen mit dem Stickstoffatom, an das sie gebunden sind, einen 4- bis 7-gliedrigen Heterocyclus bilden,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der Formel 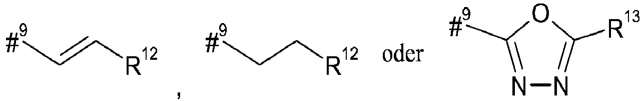 steht, worin
#⁹ die Verknüpfungsstelle mit -CHCH₂Phenyl kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
R³⁵ für Methyl oder Hydroxy steht,
sowie ihre Salze, Solvate und Solvate der Salze.

3. Verbindungen der Formel (XXXa) gemäß Anspruch 1, in welcher
Cys für einen Cystein-Rest, der über das Schwefelatom der Seitenkette über ein Kohlenstoffatom des Succinimids gebunden ist, steht
L¹ für eine Bindung oder lineares (C₂-C₆)-Alkandiyl steht,
B für eine Bindung oder eine Gruppe der Formel 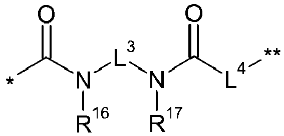 steht, wobei
* die Verknüpfungsstelle mit L¹ kennzeichnet,
** die Verknüpfungsstelle mit L² kennzeichnet,
L³ für eine Bindung steht,
L⁴ für eine Bindung steht,
R¹⁶ für Wasserstoff oder Methyl steht,
R¹⁷ für Wasserstoff oder Methyl steht,
L² für lineares (C₂-C₆)-Alkandiyl oder für eine Gruppe der Formel 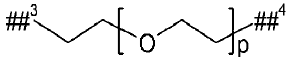 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
D für eine Gruppe der Formel 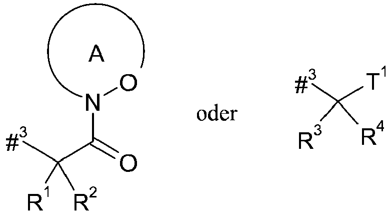 steht, wobei
#³ die Verknüpfungsstelle mit dem Stickstoffatom kennzeichnet,
R¹ für Wasserstoff steht,
R² für Benzyl oder 1*H*-Indol-3-ylmethyl steht,
oder
R¹ und R² zusammen mit dem Kohlenstoffatom, an das sie gebunden sind, eine (1*S,*2*R*)-2-Phenylcyclopropan-1,1-diyl-Gruppe der Formel 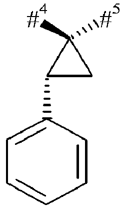 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bicyclischen, gegebenenfalls substituierten Heterocyclus der Formel 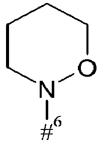 steht, worin
#⁶ die Verknüpfungsstelle mit der Carbonyl-Gruppe kennzeichnet,
R³ für Wasserstoff steht,
R⁴ für Benzyl oder 1*H*-Indol-3-ylmethyl steht,
oder
R³ und R⁴ zusammen mit dem Kohlenstoffatom, an das sie gebunden sind, eine (1*S,*2*R*)-2-Phenylcyclopropan-1,1-diyl-Gruppe der Formel 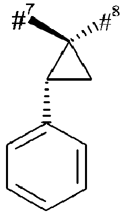 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷ oder -C(=O)-NR⁸R⁹ steht,
worin
R⁷ für Wasserstoff steht,
R⁸ für Wasserstoff steht,
R⁹ für Wasserstoff steht,
R³⁵ für Methyl steht,
sowie ihre Salze, Solvate und Solvate der Salze.

4. Verbindungen der Formel (XXXI) 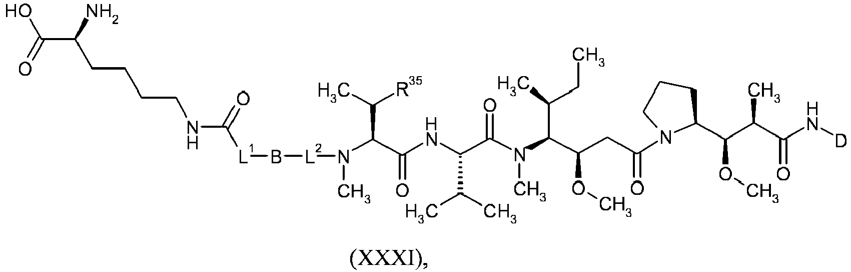 in welcher
L¹ für eine Bindung, lineares (C₁-C₁₀)-Alkandiyl, eine Gruppe der Formel 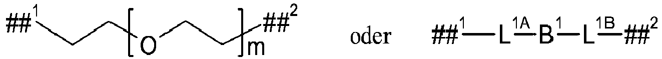 steht, wobei
m für eine Zahl von 2 bis 6 steht,
##¹ die Verknüpfungsstelle mit der angrenzenden Carbonylgruppe kennzeichnet,
##² die Verknüpfungsstelle mit der Gruppe B kennzeichnet,
L^{1A} für lineares (C₂-C₁₀)-Alkandiyl steht,
B¹ für eine Gruppe der Formel 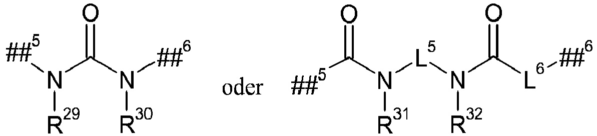 steht, worin
##⁵ die Verknüpfungsstelle mit der Gruppe L^{1A} kennzeichnet,
##⁶ die Verknüpfungsstelle mit der Gruppe L^{1B} kennzeichnet,
L⁵ für eine Bindung oder (C₂-C₄)-Alkandiyl steht,
L⁶ für eine Bindung steht,
R²⁹ für Wasserstoff oder (C₁-C₄)-Alkyl steht,
R³⁰ für Wasserstoff oder (C₁-C₄)-Alkyl steht,
oder
R²⁹ und R³¹ zusammen mit den Atomen, an die sie gebunden sind, einen 5- oder 6-gliedrigen Heterocyclus bilden,
R³¹ für Wasserstoff oder (C₁-C₄)-Alkyl steht,
R³² für Wasserstoff oder (C₁-C₄)-Alkyl steht,
oder
R³¹ und R³² zusammen mit den Atomen, an die sie gebunden sind, einen 5- oder 6-gliedrigen Heterocyclus bilden,
L^{1B} für lineares (C₂-C₁₀)-Alkandiyl steht,
und
wobei (C₁-C₁₀)-Alkandiyl mit 1 bis 4 Substituenten unabhängig voneinander ausgewählt aus der Gruppe Methyl, Hydroxy und Benzyl substituiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
B für eine Bindung oder eine Gruppe der Formel 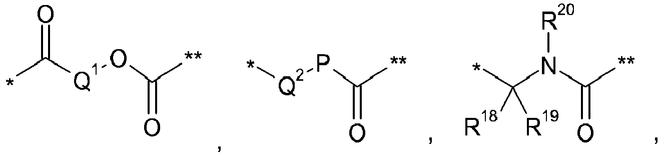 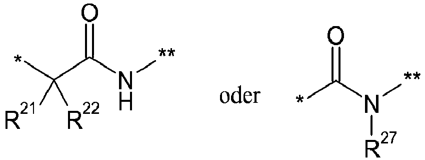 steht, wobei
* die Verknüpfungsstelle mit L¹ kennzeichnet,
** die Verknüpfungsstelle mit L² kennzeichnet,
P für O oder NH steht,
Q¹ für einen 4- bis 7-gliedrigen Heterocyclus steht,
Q² für einen 3- bis 7-gliedrigen Carbocyclus oder einen 4- bis 7-gliedrigen Heterocyclus steht,
R¹⁸ für Wasserstoff oder (C₁-C₄)-Alkyl steht,
R¹⁹ für Wasserstoff oder die Seitengruppe einer natürlichen α-Aminosäure
oder ihrer Homologe oder Isomere steht,
R²⁰ für Wasserstoff oder (C₁-C₄)-Alkyl steht,
oder
R¹⁹ und R²⁰ zusammen mit den Atomen, an die sie gebunden sind, einen Pyrrolidinylring bilden,
R²¹ für Wasserstoff oder (C₁-C₄)-Alkyl steht,
R²² für Wasserstoff oder (C₁-C₄)-Alkyl steht,
oder
R²¹ und R²² zusammen mit den Atomen, an die sie gebunden sind, einen 3- bis 7-gliedrigen Carbocyclus bilden,
R²⁷ für Wasserstoff oder (C₁-C₄)-Alkyl steht,
L² für lineares (C₂-C₁₀)-Alkandiyl oder für eine Gruppe der Formel 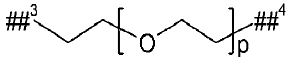 steht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
wobei (C₂-C₁₀)-Alkandiyl mit 1 bis 4 Substituenten unabhängig voneinander ausgewählt aus der Gruppe Methyl, Hydroxy und Benzyl substituiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
D für eine Gruppe der Formel 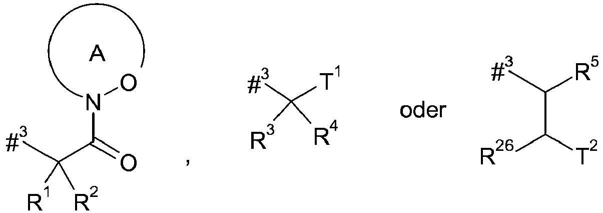 steht, wobei
#³ die Verknüpfungsstelle mit dem Stickstoffatom kennzeichnet,
R¹ für Wasserstoff oder Methyl steht,
R² für Isopropyl, Isobutyl, *sec*.-Butyl, *tert.-*Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenylethyl, Diphenylmethyl, 1*H*-Imidazol-4-ylmethyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 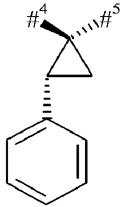 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bicyclischen, gegebenenfalls substituierten Heterocyclus der Formel 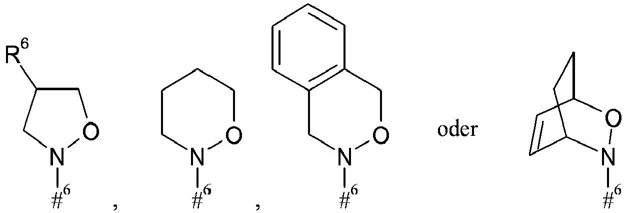 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff oder Methyl steht,
R⁴ für Isopropyl, Isobutyl, *sec.*-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenylethyl, Diphenylmethyl, 1*H*-Imidazol-4-ylmethyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 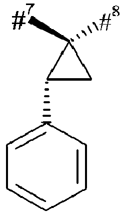 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹, -C(=O)-NH-NH-R¹⁰ oder -CH₂-O-R¹¹ steht,
worin
R⁷ für Wasserstoff, Methyl, Ethyl, *n*-Propyl, *tert*.-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
oder
R⁸ und R⁹ zusammen mit dem Stickstoffatom, an das sie gebunden sind, einen 4- bis 7-gliedrigen Heterocyclus bilden,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der Formel 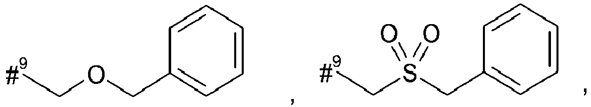 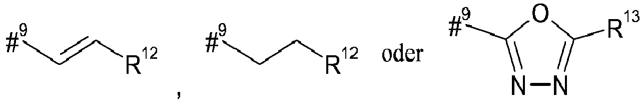 steht, worin
#⁹ die Verknüpfungsstelle mit -CHC(R²⁶)-T² kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
R²⁶ für Wasserstoff oder Hydroxy steht,
T² für Phenyl, Benzyl, 1*H*-Indol-3-yl oder 1*H*-Indol-3-ylmethyl steht,
R³⁵ für Methyl oder Hydroxy steht,
sowie ihre Salze, Solvate und Solvate der Salze.

5. Verbindungen der Formel (XXXI) gemäß Anspruch 4, in welcher
L¹ für eine Bindung steht,
B für eine Bindung steht,
L² für lineares (C₂-C₆)-Alkandiyl oder für eine Gruppe der Formel 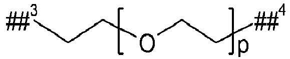 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
D für eine Gruppe der Formel 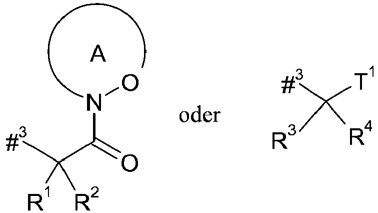 steht, wobei
#³ die Verknüpfungsstelle mit dem Stickstoffatom kennzeichnet,
R¹ für Wasserstoff steht,
R² für Benzyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 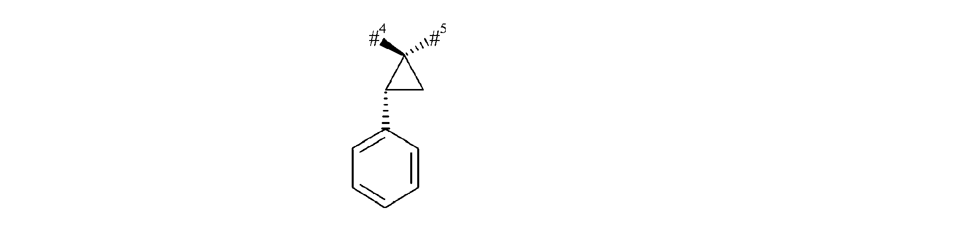 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bicyclischen, gegebenenfalls substituierten Heterocyclus der Formel 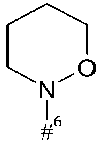 steht, worin
#⁶ die Verknüpfungsstelle mit der Carbonyl-Gruppe kennzeichnet,
R³ für Wasserstoff steht,
R⁴ für Benzyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 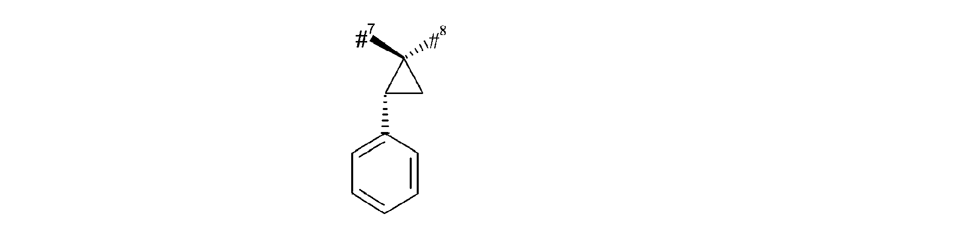 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷ oder -C(=O)-NR⁸R⁹ steht,
worin
R⁷ für Wasserstoff steht,
R⁸ für Wasserstoff steht,
R⁹ für Wasserstoff steht,
R³⁵ für Methyl steht,
sowie ihre Salze, Solvate und Solvate der Salze.

6. Verbindungen der Formel (XXXa) gemäß Anspruch 1, in welcher
L¹ für eine Bindung steht,
B für eine Bindung steht,
L² für lineares (C₂-C₆)-Alkandiyl oder für eine Gruppe der Formel 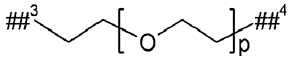 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
sowie ihre Salze, Solvate und Solvate der Salze.

7. Verbindungen der Formel (XXXI) gemäß Anspruch 4, in welcher
L¹ für eine Bindung steht,
B für eine Bindung steht,
L² für lineares (C₂-C₆)-Alkandiyl oder für eine Gruppe der Formel 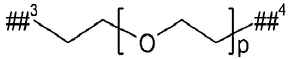 steht, wobei
p für eine Zahl von 2 oder 3 steht,
##³ die Verknüpfungsstelle mit der Gruppe B kennzeichnet,
##⁴ die Verknüpfungsstelle mit dem Stickstoffatom kennzeichnet,
sowie ihre Salze, Solvate und Solvate der Salze.

8. Verbindungen der Formel (XXXa) gemäß Anspruch 1, in welcher
D für eine Gruppe der Formel 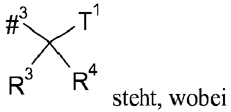
#³ die Verknüpfungsstelle mit dem Stickstoffatom kennzeichnet,
R³ für Wasserstoff steht,
R⁴ für 4-Hydroxybenzyl oder 1*H*-Indol-3-ylmethyl steht,
T¹ für eine Gruppe der Formel -C(=O)-OR⁷ oder -C(=O)-NR⁸R⁹ steht,
worin
R⁷ für Wasserstoff steht,
R⁸ für Wasserstoff steht,
R⁹ für Wasserstoff steht,
sowie ihre Salze, Solvate und Solvate der Salze.

9. Verbindungen der Formel (XXXI) gemäß Anspruch 4, in welcher
D für eine Gruppe der Formel 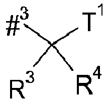 steht, wobei
#³ die Verknüpfungsstelle mit dem Stickstoffatom kennzeichnet,
R³ für Wasserstoff steht,
R⁴ für 4-Hydroxybenzyl oder 1*H*-Indol-3-ylmethyl steht,
T¹ für eine Gruppe der Formel -C(=O)-OR⁷ oder -C(=O)-NR⁸R⁹ steht,
worin
R⁷ für Wasserstoff steht,
R⁸ für Wasserstoff steht,
R⁹ für Wasserstoff steht,
sowie ihre Salze, Solvate und Solvate der Salze.

10. Verbindungen der Formel (XXXa) gemäß Anspruch 1, in welcher
D für eine Gruppe der Formel 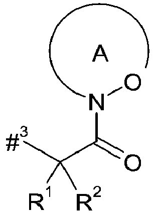 steht, wobei
#³ die Verknüpfungsstelle mit dem Stickstoffatom kennzeichnet,
R¹ für Wasserstoff oder Methyl steht,
R² für Isopropyl, Isobutyl, *sec*.-Butyl, *tert.-*Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenylethyl, Diphenylmethyl, 1*H*-Imidazol-4-ylmethyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 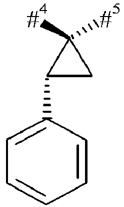 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bicyclischen, gegebenenfalls substituierten Heterocyclus der Formel 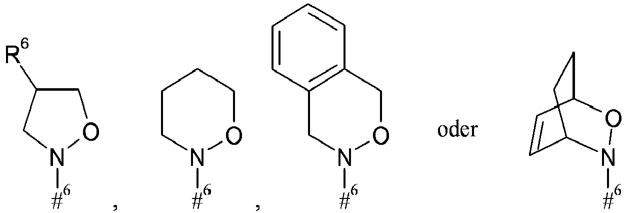 steht, worin
#⁶ die Verknüpfungsstelle mit der Carbonyl-Gruppe kennzeichnet,
R⁶ für Wasserstoff, Hydroxy oder Benzyloxy steht,
sowie ihre Salze, Solvate und Solvate der Salze.

11. Verbindungen der Formel (XXXI) gemäß Anspruch 4, in welcher
D für eine Gruppe der Formel 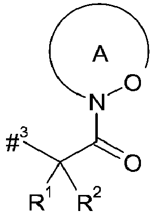 steht, wobei
#³ die Verknüpfungsstelle mit dem Stickstoffatom kennzeichnet,
R¹ für Wasserstoff oder Methyl steht,
R² für Isopropyl, Isobutyl, *sec*.-Butyl, *tert*.-Butyl, Phenyl, Benzyl, 1-Hydroxyethyl, 4-Hydroxybenzyl, 4-Hydroxy-3-nitrobenzyl, 4-Hydroxy-3-aminobenzyl, 1-Phenylethyl, Diphenylmethyl, 1*H*-Imidazol-4-ylmethyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 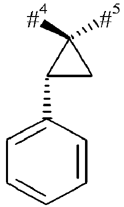 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bicyclischen, gegebenenfalls substituierten Heterocyclus der Formel 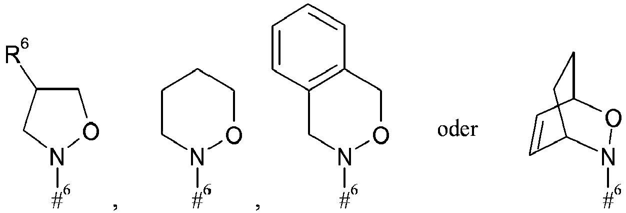 steht, worin
#⁶ die Verknüpfungsstelle mit der Carbonyl-Gruppe kennzeichnet,
R⁶ für Wasserstoff, Hydroxy oder Benzyloxy steht,
sowie ihre Salze, Solvate und Solvate der Salze.

12. Verbindungen der Formel (XXXa) gemäß Anspruch 1, in welcher
D für eine Gruppe der Formel 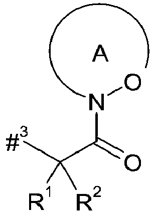 steht, wobei
#³ die Verknüpfungsstelle mit dem Stickstoffatom kennzeichnet,
R¹ für Wasserstoff steht,
R² für Benzyl, 4-Hydroxybenzyl, 1-Phenylethyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 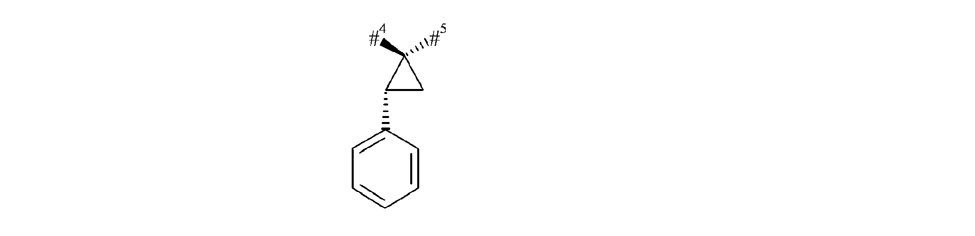 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bicyclischen, gegebenenfalls substituierten Heterocyclus der Formel 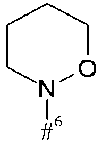 steht, worin
#⁶ die Verknüpfungsstelle mit der Carbonyl-Gruppe kennzeichnet,
sowie ihre Salze, Solvate und Solvate der Salze.

13. Verbindungen der Formel (XXXI) gemäß Anspruch 4, in welcher
D für eine Gruppe der Formel 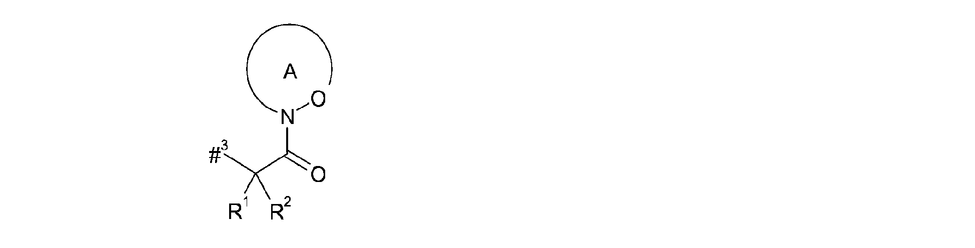 steht, wobei
#³ die Verknüpfungsstelle mit dem Stickstoffatom kennzeichnet,
R¹ für Wasserstoff steht,
R² für Benzyl, 4-Hydroxybenzyl, 1-Phenylethyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 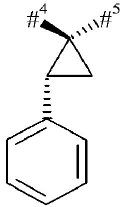 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bicyclischen, gegebenenfalls substituierten Heterocyclus der Formel 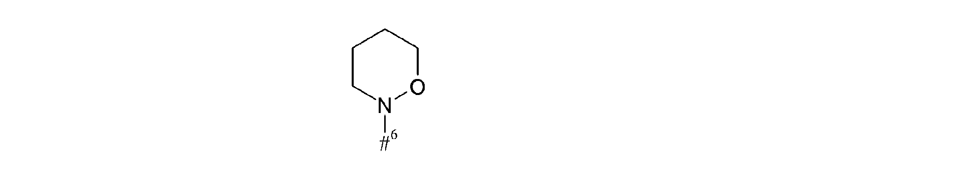 steht, worin
#⁶ die Verknüpfungsstelle mit der Carbonyl-Gruppe kennzeichnet,
sowie ihre Salze, Solvate und Solvate der Salze.

14. Verbindungen der Formel (XXX) 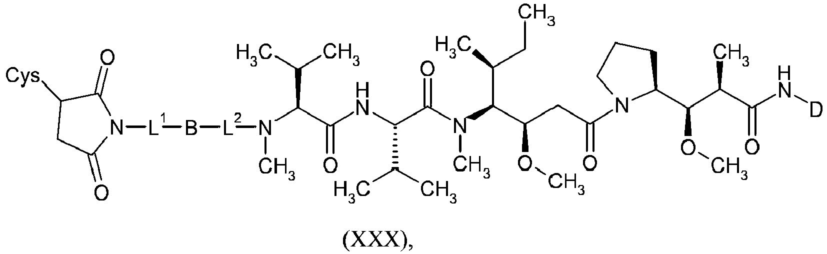 in welcher
Cys für einen Cystein-Rest, der über das Schwefelatom der Seitenkette an ein Kohlenstoffatom des Succinimids gebunden ist, steht
L¹ für eine Bindung, lineares (C₁-C₁₀)-Alkandiyl oder für eine Gruppe der Formel 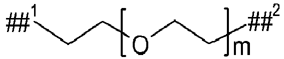 steht, wobei
m für eine Zahl von 2 bis 6 steht,
##¹ die Verknüpfungsstelle mit dem angrenzenden Stickstoffatom kennzeichnet,
##² die Verknüpfungsstelle mit der Gruppe B kennzeichnet,
wobei (C₁-C₁₀)-Alkandiyl mit 1 bis 4 Methylsubstituenten substituiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
B für eine Bindung oder eine Gruppe der Formel 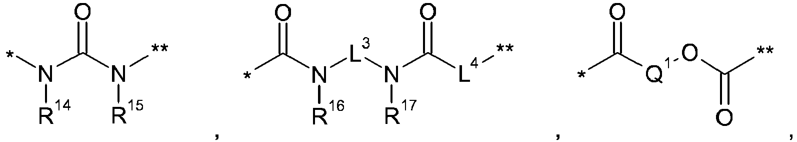 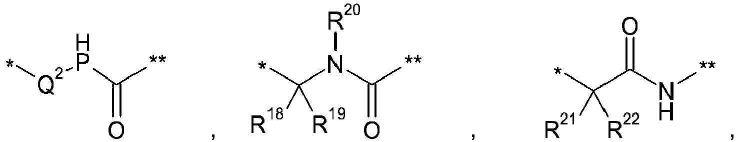 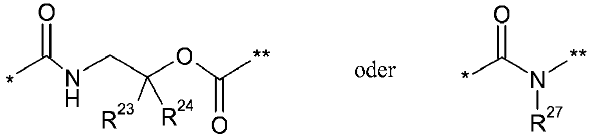 steht, wobei
* die Verknüpfungsstelle mit L¹ kennzeichnet,
** die Verknüpfungsstelle mit L² kennzeichnet,
P für O oder NH steht,
L³ für eine Bindung, oder (C₂-C₄)-Alkandiyl steht,
L⁴ für eine Bindung oder eine Gruppe der Formel 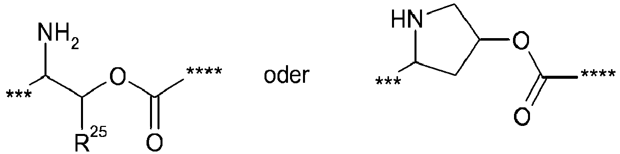 steht, worin
*** die Verknüpfungsstelle mit der Carbonylgruppe kennzeichnet,
**** die Verknüpfungsstelle mit L² kennzeichnet,
R²⁵ für Wasserstoff oder Methyl steht,
Q¹ für einen 3- bis 7-gliedrigen Carbocyclus oder einen 4- bis 7-gliedrigen Aza- Heterocyclus steht,
Q² für einen 3- bis 7-gliedrigen Carbocyclus oder einen 4- bis 7-gliedrigen Aza- Heterocyclus steht,
R¹⁴ für Wasserstoff oder (C₁-C₄)-Alkyl steht,
R¹⁵ für Wasserstoff oder (C₁-C₄)-Alkyl steht,
oder
R¹⁴ und R¹⁵ zusammen mit den Atomen, an die sie gebunden sind, einen 5-oder 6-gliedrigen Heterocyclus bilden,
R¹⁶ für Wasserstoff oder (C₁-C₄)-Alkyl steht,
R¹⁷ für Wasserstoff oder (C₁-C₄)-Alkyl steht,
oder
R¹⁶ und R¹⁷ zusammen mit den Atomen, an die sie gebunden sind, einen 5-oder 6-gliedrigen Heterocyclus bilden,
R¹⁸ für Wasserstoff oder (C₁-C₄)-Alkyl steht,
R¹⁹ für Wasserstoff oder die Seitengruppe einer natürlichen α-Aminosäure oder ihrer Homologe oder Isomere steht,
R²⁰ für Wasserstoff oder (C₁-C₄)-Alkyl steht,
oder
R¹⁹ und R²⁰ zusammen mit den Atomen, an die sie gebunden sind, einen Pyrrolidinylring bilden,
R²¹ für Wasserstoff oder (C₁-C₄)-Alkyl steht,
R²² für Wasserstoff oder (C₁-C₄)-Alkyl steht,
oder
R²¹ und R²² zusammen mit den Atomen, an die sie gebunden sind, einen 3- bis 7-gliedrigen Carbocyclus bilden,
R²³ für (C₁-C₄)-Alkyl steht,
R²⁴ für Wasserstoff oder (C₁-C₄)-Alkyl steht,
R²⁷ für Wasserstoff oder (C₁-C₄)-Alkyl steht,
L² für lineares (C₂-C₁₀)-Alkandiyl oder für eine Gruppe der Formel 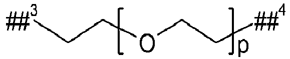 steht, wobei
p für eine Zahl von 2 bis 6 steht,
##³ die Verknüpfungsstelle mit der Gruppe B kennzeichnet,
##⁴ die Verknüpfungsstelle mit dem Stickstoffatom kennzeichnet,
wobei (C₂-C₁₀)-Alkandiyl mit 1 bis 4 Methylsubstituenten substituiert sein kann,
und
wobei zwei Kohlenstoffatome der Alkandiyl-Kette in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können,
D für eine Gruppe der Formel 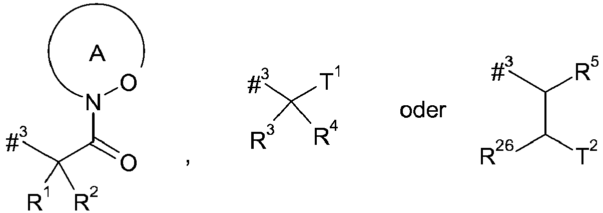 steht, wobei
#³ die Verknüpfungsstelle mit dem Stickstoffatom kennzeichnet,
R¹ für Wasserstoff steht,
R² für 1-Hydroxyethyl, Benzyl, 1-Phenylethyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 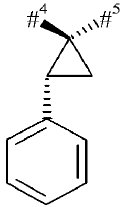 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bicyclischen, gegebenenfalls substituierten Heterocyclus der Formel 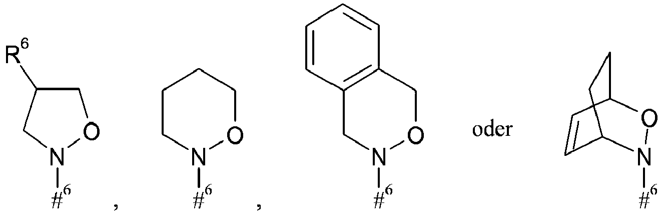 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff steht,
R⁴ für 1-Hydroxyethyl, Benzyl, 1-Phenylethyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 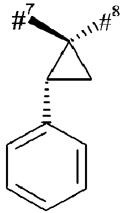 bilden, worin
#⁷ die Verknüpfungsstelle mit dem angrenzenden Stickstoffatom kennzeichnet,
#⁸ die Verknüpfungsstelle mit der Gruppe T¹ kennzeichnet,
T¹ für eine Gruppe der Formel -C(=O)-OR⁷, -C(=O)-NR⁸R⁹, -C(=O)-NH-NH-R¹⁰ oder -CH₂-O-R¹¹ steht,
worin
R⁷ für Wasserstoff, Methyl, Ethyl, *n*-Propyl, *tert*.-Butyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
oder
R⁸ und R⁹ zusammen mit dem Stickstoffatom, an das sie gebunden sind, einen 4- bis 7-gliedrigen Heterocyclus bilden,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff, Methyl oder eine Gruppe der Formel 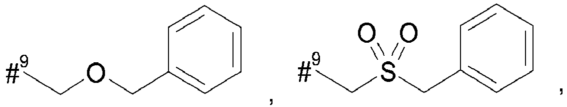 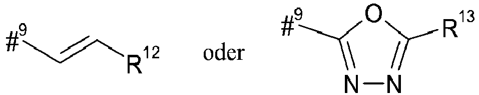 steht, worin
#⁹ die Verknüpfungsstelle mit -CHC(R²⁶)-T² kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
R²⁶ für Wasserstoff oder Hydroxy steht,
T² für Phenyl, Benzyl, 1*H*-Indol-3-yl oder 1*H*-Indol-3-ylmethyl steht,
sowie ihre Salze, Solvate und Solvate der Salze.

15. Verbindungen der Formel (XXX) nach Anspruch 14, in welcher
Cys für einen Cystein-Rest steht, der über das Schwefelatom der Seitenkette an ein Kohlenstoffatom des Succinimids gebunden ist, steht
L¹ für eine Bindung, lineares (C₂-C₆)-Alkandiyl oder für eine Gruppe der Formel 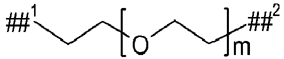 steht, wobei
m für eine Zahl von 2 bis 6 steht,
##¹ die Verknüpfungsstelle mit dem angrenzenden Stickstoffatom kennzeichnet,
##² die Verknüpfungsstelle mit der Gruppe B kennzeichnet,
wobei (C₂-C₆)-Alkandiyl mit 1 oder 2 Methylsubstituenten substituiert sein kann,
B für eine Bindung oder eine Gruppe der Formel 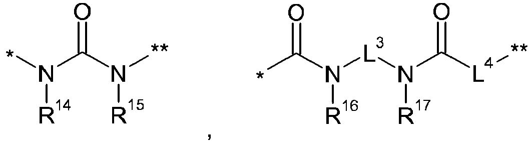 oder 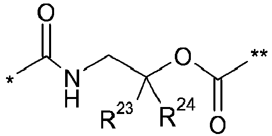 steht, wobei
* die Verknüpfungsstelle mit L¹ kennzeichnet,
** die Verknüpfungsstelle mit L² kennzeichnet,
L³ für eine Bindung oder Ethan-1,2-diyl steht,
L⁴ für eine Bindung steht,
R¹⁴ für Wasserstoff steht,
R¹⁵ für Wasserstoff steht,
R¹⁶ für Wasserstoff oder Methyl steht,
R¹⁷ für Wasserstoff oder Methyl steht,
oder
R¹⁶ und R¹⁷ zusammen mit den Atomen, an die sie gebunden sind, einen Piperazinylring bilden,
R²³ für Methyl steht,
R²⁴ für Wasserstoff oder Methyl steht,
L² für lineares (C₂-C₆)-Alkandiyl oder für eine Gruppe der Formel 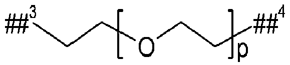 steht, wobei
p für eine Zahl 2 oder 3 steht,
##³ die Verknüpfungsstelle mit der Gruppe B kennzeichnet,
##⁴ die Verknüpfungsstelle mit dem Stickstoffatom kennzeichnet,
D für eine Gruppe der Formel 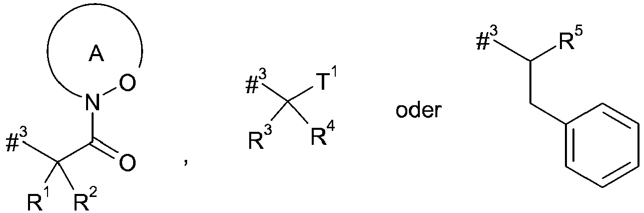 steht, wobei
#³ die Verknüpfungsstelle mit dem Stickstoffatom kennzeichnet,
R¹ für Wasserstoff steht,
R² für 1-Hydroxyethyl, Benzyl, 1-Phenylethyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 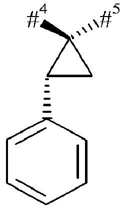 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen mono- oder bicyclischen, gegebenenfalls substituierten Heterocyclus der Formel 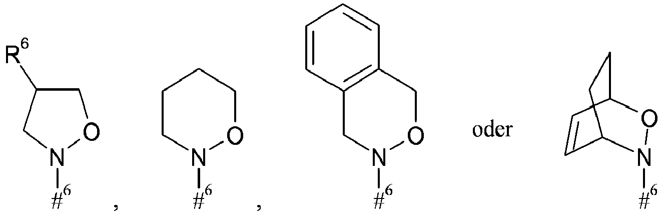 steht, worin
#⁶ die Verknüpfungsstelle mit der Carbonyl-Gruppe kennzeichnet,
R⁶ für Wasserstoff, Hydroxy oder Benzyloxy steht,
R³ für Wasserstoff steht,
R⁴ für Benzyl, 1-Phenylethyl oder 1*H*-Indol-3-ylmethyl steht,
oder
R³ und R⁴ zusammen mit dem Kohlenstoffatom, an das sie gebunden sind, eine (1*S*,2*R*)-2-Phenylcyclopropan-1,1-diyl-Gruppe der Formel 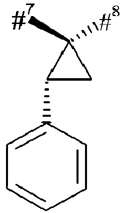 bilden, worin
#⁷ die Verknüpfungsstelle mit dem angrenzenden Stickstoffatom kennzeichnet,
#⁸ die Verknüpfungsstelle mit der Gruppe T kennzeichnet,
T¹ für eine Gruppe der Formel -C(=O)-OR⁷, -C(=O)-NR⁸R⁹, -C(=O)-NH-NH-R¹⁰ oder -CH₂-O-R¹¹ steht,
worin
R⁷ für Wasserstoff, Methyl, Ethyl, *n*-Propyl, Benzyl oder Adamantylmethyl steht,
R⁸ für Wasserstoff oder Methyl steht,
R⁹ für Wasserstoff, Methyl, Ethyl, *n*-Propyl oder Benzyl steht,
R¹⁰ für Benzoyl steht,
R¹¹ für Benzyl, das in der Phenylgruppe mit Methoxycarbonyl oder Carboxyl substituiert sein kann, steht,
R⁵ für Wasserstoff oder eine Gruppe der Formel 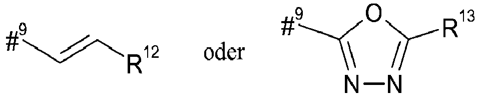 steht, worin
#⁹ die Verknüpfungsstelle mit -CHCH₂Phenyl kennzeichnet,
R¹² für Phenyl steht, das mit Methoxycarbonyl, Carboxyl oder einer Gruppe der Formel -S(O)₂OH substituiert sein kann,
R¹³ für Phenyl steht, das mit Methoxycarbonyl oder Carboxyl substituiert sein kann,
sowie ihre Salze, Solvate und Solvate der Salze.

16. Verbindungen der Formel (XXX) nach Anspruch 14, in welcher
D für eine Gruppe der Formel 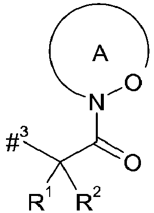 steht, wobei
#³ die Verknüpfungsstelle mit dem Stickstoffatom kennzeichnet,
R¹ für Wasserstoff steht,
R² für Benzyl oder 1*H*-Indol-3-ylmethyl steht,
oder
R¹ und R² zusammen mit dem Kohlenstoffatom, an das sie gebunden sind, eine (1*S*,2*R*)-2-Phenylcyclopropan-1,1-diyl-Gruppe der Formel 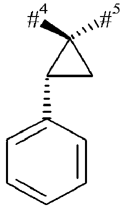 bilden, worin
#⁴ die Verknüpfungsstelle mit dem angrenzenden Stickstoffatom kennzeichnet,
#⁵ die Verknüpfungsstelle mit der Carbonyl-Gruppe kennzeichnet,
der Ring A mit der darin enthaltenen N-O-Gruppierung für einen Heterocyclus der Formel 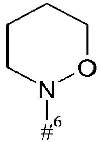 steht, worin
#⁶ die Verknüpfungsstelle mit der Carbonyl-Gruppe kennzeichnet,
sowie ihre Salze, Solvate und Solvate der Salze.

17. Verbindungen der Formel (XXX) nach Anspruch 14, wobei
D für eine Gruppe der Formel 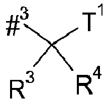 steht, wobei
R³ und R⁴ wie in Anspruch 14 definiert sind,
T¹ für -C(=O)-OH oder -C(=O)-NH₂ steht,
sowie ihre Salze, Solvate und Solvate der Salze.

18. Verbindungen der Formel (XXX) nach Anspruch 14, wobei
B für eine Bindung oder eine Gruppe der Formel 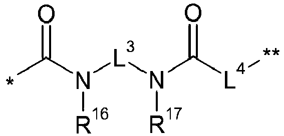 steht, wobei
* die Verknüpfungsstelle mit L¹ kennzeichnet,
** die Verknüpfungsstelle mit L² kennzeichnet,
L³ und L⁴ jeweils für eine Bindung stehen,
sowie ihre Salze, Solvate und Solvate der Salze.

19. Verbindung mit einer der folgenden Strukturen: 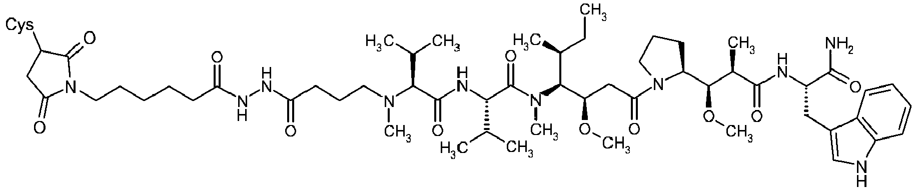 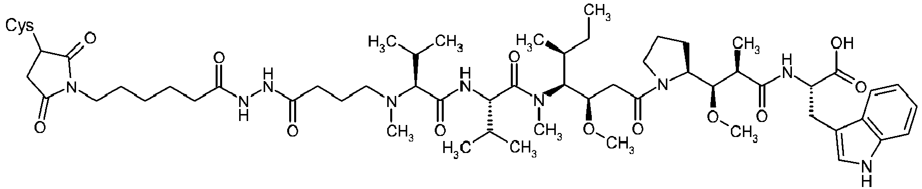 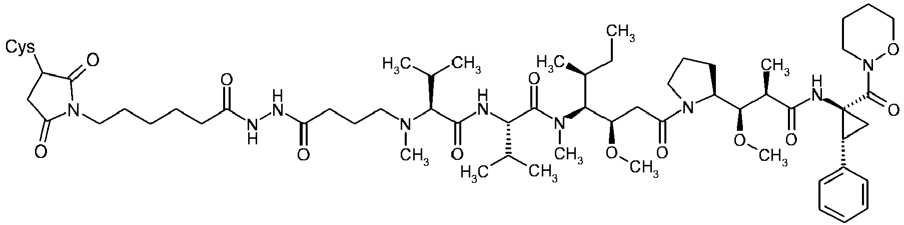 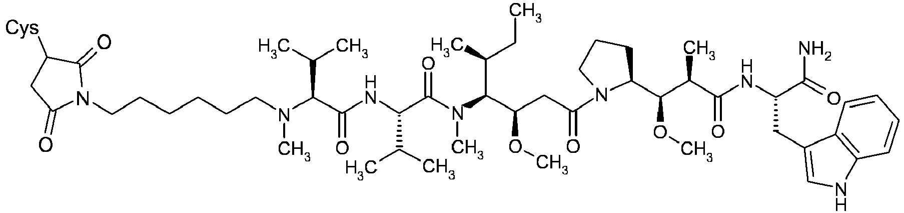 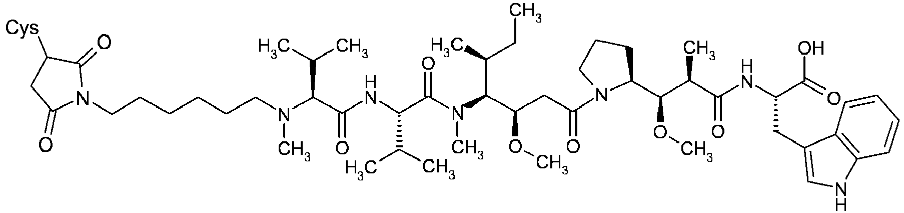 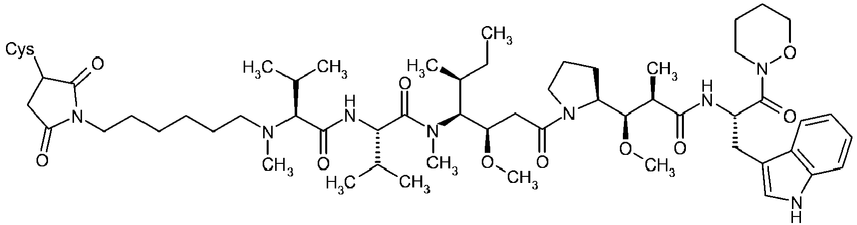 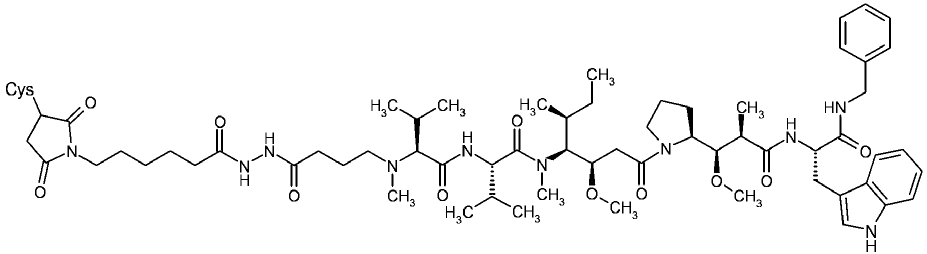 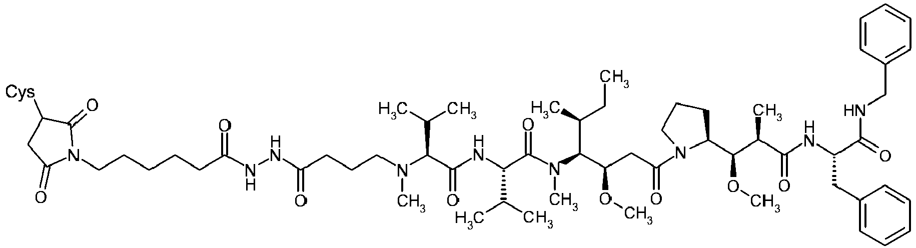 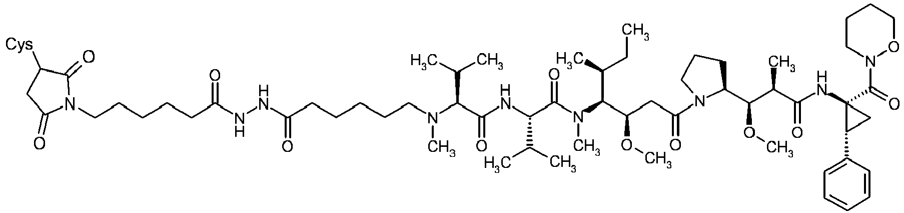 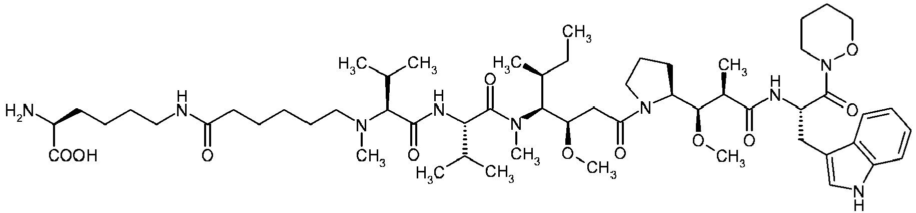 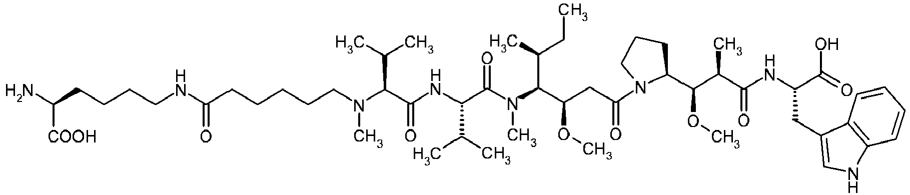 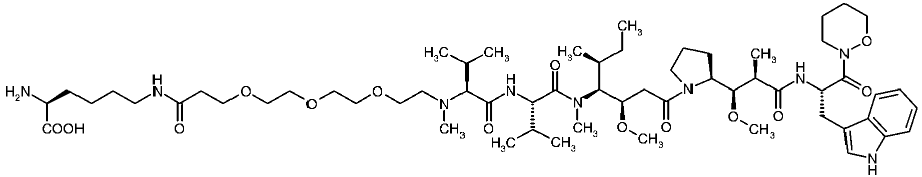 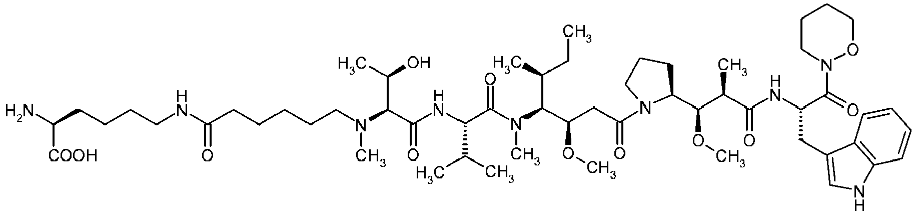 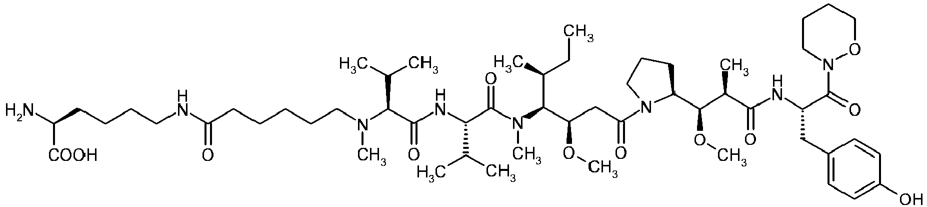 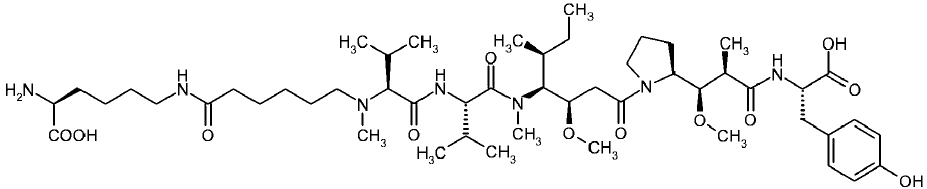 sowie ihre Salze, Solvate und Solvate der Salze.

20. Binder-Wirkstoff Konjugate, beinhaltend eine Verbindung der Formel (XXXa) oder eine Verbindung der Formel (XXXI) nach einem der Ansprüche 1-13, die mit einem Antikörper oder einem antigen-bindenden Antikörperfragment verknüpft ist, wobei ein oder mehrere Cystein-Reste des Antikörpers oder des antigen-bindenden Antikörperfragmentes den Cystein-Rest der Verbindung der Formel (XXXa) oder der Verbindung der Formel (XXXI) beinhalten.

21. Binder-Wirkstoff-Konjugat nach Anspruch 20, wobei der Antikörper oder das antigenbindende Antikörperfragment an ein Krebs-Zielmolekül bindet.

22. Binder-Wirkstoff-Konjugat nach Anspruch 20, wobei das Krebs-Zielmolekül ein extrazelluläres Krebs-Zielmolekül ist.

23. Binder-Wirkstoff-Konjugat nach Anspruch 22, wobei der Antikörper oder das antigenbindende Antikörperfragment nach Bindung an das extrazelluläre Krebs-Zielmolekül von der das Zielmolekül exprimierenden Zelle internalisiert wird.

24. Binder-Wirkstoff-Konjugat nach Anspruch 20, wobei der Antikörper oder das antigenbindende Antikörperfragment ein monoklonaler Antikörper oder ein antigen-bindendes Fragment eines monoklonalen Antikörpers ist.

25. Binder-Wirkstoff-Konjugat nach Anspruch 20, wobei der Antikörper oder das antigenbindende Antikörperfragment ein humaner, humanisierter oder chimärer Antikörper oder ein antigen-bindendes Fragment eines humanen, humanisierten oder chimären Antikörpers ist.

26. Binder-Wirkstoff-Konjugat nach Anspruch 20, wobei der Antikörper oder das antigenbindende Antikörperfragment ein Antikörper der IgG Klasse oder ein antigen-bindendes Fragment eines Antikörpers der IgG Klasse ist.

27. Binder-Wirkstoff-Konjugat nach Anspruch 20, wobei der Antikörper oder das antigenbindende Antikörperfragment an ein extrazelluläres Krebs-Zielmolekül bindet, welches ausgewählt wird aus der Gruppe bestehend aus EGF-Rezeptor (NP_005219.2), Mesothelin (Q13421-3), C4.4a (NP_055215.2) und Carboanhydrase IX (CA IX; NP_001207.2).

28. Binder-Wirkstoff-Konjugat nach Anspruch 20, wobei der Antikörper oder das antigenbindende Antikörperfragment spezifisch an C4.4a bindet.

29. Binder-Wirkstoff-Konjugat nach Anspruch 28, wobei der Antikörper oder das antigenbindende Antikörperfragment an die S1 Domäne (Aminosäureposition 1-85 von SEQ ID NO: 1) von C4.4a. bindet.

30. Binder-Wirkstoff-Konjugat nach Anspruch 28, wobei der Antikörper oder das antigenbindende Antikörperfragment mit Antikörper M31-B01 und/oder mit dem Antikörper M20-D02-S-A um Bindung an C4.4a kompetiert.

31. Binder-Wirkstoff-Konjugat nach Anspruch 28, wobei der Antikörper oder das antigenbindende Antikörperfragment ausgewählt wird aus der Gruppe bestehend aus
Antikörpern und antigen-bindenden Antikörperfragmenten, die die Aminosäuresequenz der variablen schweren Ketten, wiedergegeben durch die Sequenz SEQ ID NO: 81 und die Aminosäuresequenz der variablen leichten Ketten wiedergegeben durch die Sequenz SEQ ID NO: 82 umfassen (B01-7),
Antikörpern, die die Aminosäuresequenz der variablen schweren Kette, wiedergegeben durch die Sequenz SEQ ID NO: 33, und die Aminosäuresequenz der variablen leichten Kette, wiedergegeben durch die Sequenz SEQ ID NO: 29, umfassen (M31-B01),
Antikörper, die die Aminosäuresequenz der variablen schweren Kette, wiedergegeben durch die Sequenz SEQ ID NO: 34, und die Aminosäuresequenz der variablen leichten Kette, wiedergegeben durch die Sequenz SEQ ID NO: 30, umfassen (M20-D02 S-A),
Antikörper, die die Aminosäuresequenz der variablen schweren Kette, wiedergegeben durch die Sequenz SEQ ID NO: 35, und die Aminosäuresequenz der variablen leichten Kette, wiedergegeben durch die Sequenz SEQ ID NO: 31, umfassen (M60-G03),
Antikörper, die die Aminosäuresequenz der variablen schweren Kette, wiedergegeben durch die Sequenz SEQ ID NO: 36, und die Aminosäuresequenz der variablen leichten Kette, wiedergegeben durch die Sequenz SEQ ID NO: 32, umfassen (M36-H02),
Antikörper, die die Aminosäuresequenz der variablen schweren Kette, wiedergegeben durch die Sequenz SEQ ID NO: 51, und die Aminosäuresequenz der variablen leichten Kette, wiedergegeben durch die Sequenz SEQ ID NO: 52, umfassen (B01-3)
Antikörper, die die Aminosäuresequenz der variablen schweren Kette, wiedergegeben durch die Sequenz SEQ ID NO: 61, und die Aminosäuresequenz der variablen leichten Kette, wiedergegeben durch die Sequenz SEQ ID NO: 62, umfassen (B01-5)
Antikörper, die die Aminosäuresequenz der variablen schweren Kette, wiedergegeben durch die Sequenz SEQ ID NO: 71, und die Aminosäuresequenz der variablen leichten Kette, wiedergegeben durch die Sequenz SEQ ID NO: 72, umfassen (B01-7)
Antikörper, die die Aminosäuresequenz der variablen schweren Kette, wiedergegeben durch die Sequenz SEQ ID NO: 91, und die Aminosäuresequenz der variablen leichten Kette, wiedergegeben durch die Sequenz SEQ ID NO: 92, umfassen (B01-12),
Antikörper, die die Aminosäuresequenz der variablen schweren Kette, wiedergegeben durch die Sequenz SEQ ID NO: 101, und die Aminosäuresequenz der variablen leichten Kette, wiedergegeben durch die Sequenz SEQ ID NO: 102, umfassen (D02-4),
Antikörper, die die Aminosäuresequenz der variablen schweren Kette, wiedergegeben durch die Sequenz SEQ ID NO: 111, und die Aminosäuresequenz der variablen leichten Kette, wiedergegeben durch die Sequenz SEQ ID NO: 112, umfassen (D02-6),
Antikörper, die die Aminosäuresequenz der variablen schweren Kette, wiedergegeben durch die Sequenz SEQ ID NO: 121, und die Aminosäuresequenz der variablen leichten Kette, wiedergegeben durch die Sequenz SEQ ID NO: 122, umfassen (D02-7),
Antikörper, die die Aminosäuresequenz der variablen schweren Kette, wiedergegeben durch die Sequenz SEQ ID NO: 131, und die Aminosäuresequenz der variablen leichten Kette, wiedergegeben durch die Sequenz SEQ ID NO: 132, umfassen (D02-11), un
Antikörper, die die Aminosäuresequenz der variablen schweren Kette, wiedergegeben durch die Sequenz SEQ ID NO: 141, und die Aminosäuresequenz der variablen leichten Kette, wiedergegeben durch die Sequenz SEQ ID NO: 142, umfassen (D02-13).

32. Binder-Wirkstoff-Konjugat nach Anspruch 28, wobei der Antikörper oder das antigenbindende Antikörperfragment ausgewählt wird aus der Gruppe bestehend aus
Antikörper, der die Aminosäuresequenz der leichten Kette, repräsentiert durch SEQ ID NO: 346, und die Aminosäuresequenz der schweren Kette, repräsentiert durch SEQ ID NO: 347, umfasst (M31-B01) umfasst,
Antikörper, der die Aminosäuresequenz der leichten Kette, repräsentiert durch SEQ ID NO: 352, und die Aminosäuresequenz der schweren Kette, repräsentiert durch SEQ ID NO: 353, umfasst (B01-3) umfasst,
Antikörper, der die Aminosäuresequenz der leichten Kette, repräsentiert durch SEQ ID NO: 364, und die Aminosäuresequenz der schweren Kette, repräsentiert durch SEQ ID NO: 365, umfasst (B01-10) umfasst, und
Antikörper, der die Aminosäuresequenz der leichten Kette, repräsentiert durch SEQ ID NO: 382, und die Aminosäuresequenz der schweren Kette, repräsentiert durch SEQ ID NO: 383, umfasst (D02-6) umfasst.

33. Binder-Wirkstoff-Konjugat nach einem der Ansprüche 20-32 oder Verbindung der Formel (XXXa) oder der Formel (XXXI) nach einem der Ansprüche 1-13 zur Verwendung in einem Verfahren zur Behandlung und/oder Prophylaxe von hyperproliferativen und/oder angiogenen Erkrankungen.

34. Binder-Wirkstoff-Konjugat nach einem der Ansprüche 20-32 oder Verbindung der Formel (XXXa) oder der Formel (XXXI) nach einem der Ansprüche 1-13 zur Herstellung eines Arzneimittels zur Behandlung und/oder Prophylaxe von hyperproliferativen und/oder angiogenen Erkrankungen.

35. Arzneimittel zur Behandlung und/oder Prophylaxe von hyperproliferativen und/oder angiogenen Erkrankungen, enthaltend eine wirksame Menge eines Binder-Wirkstoff-Konjugat nach einem der Ansprüche 20-32 oder einer Verbindung der Formel (XXXa) oder der Formel (XXXI) nach einem der Ansprüche 1-13, in Kombination mit einem inerten, nicht-toxischen, pharmazeutisch geeigneten Hilfsstoff.

36. Arzneimittel enthaltend ein Binder-Wirkstoff-Konjugat nach einem der Ansprüche 20-32 oder eine Verbindung der Formel (XXXa) oder der Formel (XXXI) nach einem der Ansprüche 1-13, in Kombination mit einer oder mehreren anti-hyperproliferativen, zytostatischen oder zytotoxischen Substanzen.

37. Arzneimittel nach Anspruch 35 oder 36 zur Anwendung in einem Verfahren zur Behandlung und/oder Prophylaxe von hyperproliferativen und/oder angiogenen Erkrankungen.

38. Binder-Wirkstoff-Konjugat nach einem der Ansprüche 20-32 oder eine Verbindung der Formel (XXXa) oder der Formel (XXXI) nach einem der Ansprüche 1-13 zur Anwendung in einem Verfahren zur Behandlung und/oder Prophylaxe von hyperproliferativen und/oder angiogenen Erkrankungen bei Menschen und Tieren.


SEQUENCE LISTING

<110>  Seattle Genetics, Inc.

<120>  NOVEL BINDER-DRUG CONJUGATES (ADCS) AND USE THEREOF

<130>  BHC-00514 / BHC111010

<150>  EP 11163467
<151>  21.04.2011

<150>  EP 11163474
<151>  21.04.2011

<150>  EP 11163472
<151>  21.04.2011

<150>  EP 11163470
<151>  21.04.2011

<150>  EP 11168557
<151>  01.06.2011

<150>  EP 11168556
<151>  01.06.2011

<150>  EP 11168559
<151>  01.06.2011

<150>  EP 11168558
<151>  01.06.2011

<150>  EP 11193621
<151>  14.12.2011

<150>  EP 11193623
<151>  14.12.2011

<150>  EP 11193609
<151>  14.12.2011

<150>  EP 11193618
<151>  14.12.2011


<160>  411

<170>  PatentIn version 3.5

<210>  1
<211>  278
<212>  PRT
<213>  Homo Sapiens

<400>  1

Leu Glu Cys Tyr Ser Cys Val Gln Lys Ala Asp Asp Gly Cys Ser Pro
1               5                   10                  15


Asn Lys Met Lys Thr Val Lys Cys Ala Pro Gly Val Asp Val Cys Thr
            20                  25                  30

Glu Ala Val Gly Ala Val Glu Thr Ile His Gly Gln Phe Ser Leu Ala
        35                  40                  45

Val Arg Gly Cys Gly Ser Gly Leu Pro Gly Lys Asn Asp Arg Gly Leu
    50                  55                  60

Asp Leu His Gly Leu Leu Ala Phe Ile Gln Leu Gln Gln Cys Ala Gln
65                  70                  75                  80

Asp Arg Cys Asn Ala Lys Leu Asn Leu Thr Ser Arg Ala Leu Asp Pro
                85                  90                  95

Ala Gly Asn Glu Ser Ala Tyr Pro Pro Asn Gly Val Glu Cys Tyr Ser
            100                 105                 110

Cys Val Gly Leu Ser Arg Glu Ala Cys Gln Gly Thr Ser Pro Pro Val
        115                 120                 125

Val Ser Cys Tyr Asn Ala Ser Asp His Val Tyr Lys Gly Cys Phe Asp
    130                 135                 140

Gly Asn Val Thr Leu Thr Ala Ala Asn Val Thr Val Ser Leu Pro Val
145                 150                 155                 160

Arg Gly Cys Val Gln Asp Glu Phe Cys Thr Arg Asp Gly Val Thr Gly
                165                 170                 175

Pro Gly Phe Thr Leu Ser Gly Ser Cys Cys Gln Gly Ser Arg Cys Asn
            180                 185                 190

Ser Asp Leu Arg Asn Lys Thr Tyr Phe Ser Pro Arg Ile Pro Pro Leu
        195                 200                 205

Val Arg Leu Pro Pro Pro Glu Pro Thr Thr Val Ala Ser Thr Thr Ser
    210                 215                 220

Val Thr Thr Ser Thr Ser Ala Pro Val Arg Pro Thr Ser Thr Thr Lys
225                 230                 235                 240

Pro Met Pro Ala Pro Thr Ser Gln Thr Pro Arg Gln Gly Val Glu His
                245                 250                 255

Glu Ala Ser Arg Asp Glu Glu Pro Arg Leu Thr Gly Gly Ala Ala Gly
            260                 265                 270

His Gln Asp Arg Ser Asn
        275

<210>  2
<211>  293
<212>  PRT
<213>  Mus musculus

<400>  2

Leu Glu Cys Tyr Ser Cys Val Gln Lys Ala Asp Asp Gly Cys Ser Pro
1               5                   10                  15

His Arg Met Lys Thr Val Lys Cys Gly Pro Gly Val Asp Val Cys Thr
            20                  25                  30

Glu Ala Val Gly Ala Val Glu Thr Ile His Gly Gln Phe Ser Val Ala
        35                  40                  45

Val Arg Gly Cys Gly Ser Gly Ile Pro Gly Lys Asn Asp Arg Gly Leu
    50                  55                  60

Asp Leu His Gly Leu Leu Ala Phe Phe Gln Leu Gln Gln Cys Ser Glu
65                  70                  75                  80

Asp Arg Cys Asn Ala Lys Leu Asn Leu Thr Leu Arg Gly Leu Asn Pro
                85                  90                  95

Ala Gly Asn Glu Ser Ala Tyr Glu Pro Asn Gly Ala Glu Cys Tyr Ser
            100                 105                 110

Cys Val Gly Leu Ser Arg Glu Lys Cys Gln Gly Ser Met Pro Pro Val
        115                 120                 125

Val Asn Cys Tyr Asn Ala Ser Gly Arg Val Tyr Lys Gly Cys Phe Asp
    130                 135                 140

Gly Asn Val Thr Leu Thr Ala Ala Asn Val Thr Val Ser Leu Pro Val
145                 150                 155                 160

Arg Gly Cys Val Gln Asp Glu Thr Cys Thr Arg Asp Gly Val Thr Gly
                165                 170                 175

Pro Gly Phe Thr Leu Ser Gly Ser Cys Cys Gln Gly Pro Arg Cys Asn
            180                 185                 190

Ala Asp Leu Arg Asn Lys Thr Tyr Phe Ser Pro Arg Ile Pro Pro Leu
        195                 200                 205

Val Leu Leu Pro Pro Pro Thr Thr Ala Ala Pro Ser Thr Arg Ala Gln
    210                 215                 220

Asn Ser Ser Ser Thr Thr Ser Thr Ala Ala Pro Thr Thr Thr Thr Ser
225                 230                 235                 240

Ile Ile Lys Pro Thr Thr Ala Gln Ala Ser His Thr Ser Pro His Glu
                245                 250                 255

Met Asp Leu Glu Val Ile Gln Glu Glu Gly Ala Ser Leu Ser Gly Gly
            260                 265                 270

Ala Ala Gly His Gly Gly Thr Ala Gly His Gly Gly Ala Ala Gly His
        275                 280                 285

Gln Asp Arg Ser Asn
    290

<210>  3
<211>  346
<212>  PRT
<213>  Homo Sapiens

<400>  3

<210>  4
<211>  363
<212>  PRT
<213>  Mus musculus

<400>  4

Met Asp Ala Ala Arg Arg Gly Asp Thr Gln Pro Val Met Trp Thr Thr
1               5                   10                  15

Gly Trp Leu Leu Leu Leu Pro Leu Leu Leu Cys Glu Gly Ala Gln Ala
            20                  25                  30

Leu Glu Cys Tyr Ser Cys Val Gln Lys Ala Asp Asp Gly Cys Ser Pro
        35                  40                  45

His Arg Met Lys Thr Val Lys Cys Gly Pro Gly Val Asp Val Cys Thr
    50                  55                  60

Glu Ala Val Gly Ala Val Glu Thr Ile His Gly Gln Phe Ser Val Ala
65                  70                  75                  80

Val Arg Gly Cys Gly Ser Gly Ile Pro Gly Lys Asn Asp Arg Gly Leu
                85                  90                  95

Asp Leu His Gly Leu Leu Ala Phe Phe Gln Leu Gln Gln Cys Ser Glu
            100                 105                 110

Asp Arg Cys Asn Ala Lys Leu Asn Leu Thr Leu Arg Gly Leu Asn Pro
        115                 120                 125

Ala Gly Asn Glu Ser Ala Tyr Glu Pro Asn Gly Ala Glu Cys Tyr Ser
    130                 135                 140

Cys Val Gly Leu Ser Arg Glu Lys Cys Gln Gly Ser Met Pro Pro Val
145                 150                 155                 160

Val Asn Cys Tyr Asn Ala Ser Gly Arg Val Tyr Lys Gly Cys Phe Asp
                165                 170                 175

Gly Asn Val Thr Leu Thr Ala Ala Asn Val Thr Val Ser Leu Pro Val
            180                 185                 190

Arg Gly Cys Val Gln Asp Glu Thr Cys Thr Arg Asp Gly Val Thr Gly
        195                 200                 205

Pro Gly Phe Thr Leu Ser Gly Ser Cys Cys Gln Gly Pro Arg Cys Asn
    210                 215                 220

Ala Asp Leu Arg Asn Lys Thr Tyr Phe Ser Pro Arg Ile Pro Pro Leu
225                 230                 235                 240

Val Leu Leu Pro Pro Pro Thr Thr Ala Ala Pro Ser Thr Arg Ala Gln
                245                 250                 255

Asn Ser Ser Ser Thr Thr Ser Thr Ala Ala Pro Thr Thr Thr Thr Ser

260                 265                 270

Ile Ile Lys Pro Thr Thr Ala Gln Ala Ser His Thr Ser Pro His Glu
        275                 280                 285

Met Asp Leu Glu Val Ile Gln Glu Glu Gly Ala Ser Leu Ser Gly Gly
    290                 295                 300

Ala Ala Gly His Gly Gly Thr Ala Gly His Gly Gly Ala Ala Gly His
305                 310                 315                 320

Gln Asp Arg Ser Asn Met Glu Lys Tyr Pro Gly Lys Gly Gly Ala Gln
                325                 330                 335

Ile Pro Ala Lys Gly Gly Ser Gly Thr Leu Gly Ser Trp Leu Ser Ala
            340                 345                 350

Val Leu Leu Thr Val Val Ala Gly Ala Met Leu
        355                 360

<210>  5
<211>  9
<212>  PRT
<213>  Artificial Sequence

<220>
<223>  C4.4a binder

<400>  5

Phe Ser Asn Ala Trp Met Ser Trp Val
1               5

<210>  6
<211>  9
<212>  PRT
<213>  Artificial Sequence

<220>
<223>  C4.4a binder

<400>  6

Phe Ser Asp Tyr Gln Met Thr Trp Ile
1               5

<210>  7
<211>  9
<212>  PRT
<213>  Artificial Sequence

<220>
<223>  C4.4a binder

<400>  7

Phe Gly His Tyr Tyr Met Phe Trp Ile
1               5


<210>  8
<211>  8
<212>  PRT
<213>  Artificial Sequence

<220>
<223>  C4.4a binder

<400>  8

Phe Ser Ser Asn Tyr Met Ser Trp
1               5


<210>  9
<211>  20
<212>  PRT
<213>  Artificial Sequence

<220>
<223>  C4.4a binder

<400>  9

Val Ser Tyr Ile Ser Ser Ser Gly Ser Thr Ile Tyr Tyr Ala Asp Ser
1               5                   10                  15


Val Lys Gly Arg
            20


<210>  10
<211>  20
<212>  PRT
<213>  Artificial Sequence

<220>
<223>  C4.4a binder

<400>  10

Val Ser Gly Val Ser Trp Asn Gly Ala Arg Thr His Tyr Ala Asp Ser
1               5                   10                  15


Val Lys Gly Arg
            20


<210>  11
<211>  20
<212>  PRT
<213>  Artificial Sequence

<220>
<223>  C4.4a binder

<400>  11

Val Ser Ala Ile Ser Gly Ser Gly Tyr Ser Thr His Tyr Ala Asp Ser
1               5                   10                  15

Val Lys Gly Arg
            20

<210>  12
<211>  20
<212>  PRT
<213>  Artificial Sequence

<220>
<223>  C4.4a binder

<400>  12

Val Ser Ala Ile Ser Ser Ser Gly Ser Ser Thr Tyr Tyr Ala Asp Ser
1               5                   10                  15

Val Lys Gly Arg
            20

<210>  13
<211>  10
<212>  PRT
<213>  Artificial Sequence

<220>
<223>  C4.4a binder

<400>  13

Ala Arg Glu Gly Leu Trp Ala Phe Asp Tyr
1               5                   10

<210>  14
<211>  15
<212>  PRT
<213>  Artificial Sequence

<220>
<223>  C4.4a binder

<400>  14

Ala Lys Gly Asp Tyr Leu Val Tyr Ser Ala Tyr Tyr Phe Asp Ser
1               5                   10                  15

<210>  15
<211>  13
<212>  PRT
<213>  Artificial Sequence

<220>

<223>  C4.4a binder

<400>  15

Ala Arg Leu Pro Tyr Gly Ser Gln Ser Gly Val Asp Tyr
1               5                   10


<210>  16
<211>  17
<212>  PRT
<213>  Artificial Sequence

<220>
<223>  C4.4a binder

<400>  16

Ala Arg Glu Ser Gly Gly Ser Gly Pro Asn Tyr Tyr Tyr Gly Met Asp
1               5                   10                  15


Val




<210>  17
<211>  14
<212>  PRT
<213>  Artificial Sequence

<220>
<223>  C4.4a binder

<400>  17

Thr Gly Ser Ser Ser Asn Ile Gly Ala Gly Tyr Val Val His
1               5                   10


<210>  18
<211>  13
<212>  PRT
<213>  Artificial Sequence

<220>
<223>  C4.4a binder

<400>  18

Ser Gly Ser Ser Ser Asn Val Gly Ser Asn Pro Val Asn
1               5                   10


<210>  19
<211>  14
<212>  PRT
<213>  Artificial Sequence

<220>
<223>  C4.4a binder

<400>  19

Thr Gly Ser Ser Ser Asn Ile Gly Ala Gly Tyr Val Val His
1               5                   10

<210>  20
<211>  14
<212>  PRT
<213>  Artificial Sequence

<220>
<223>  C4.4a binder

<400>  20

Thr Gly Ser Ser Ser Asn Ile Gly Ala Gly Tyr Val Val His
1               5                   10

<210>  21
<211>  7
<212>  PRT
<213>  Artificial Sequence

<220>
<223>  C4.4a binder

<400>  21

Asp Asn Asn Lys Arg Pro Ser
1               5

<210>  22
<211>  7
<212>  PRT
<213>  Artificial Sequence

<220>
<223>  C4.4a binder

<400>  22

Arg Asn Asn Gln Arg Pro Ser
1               5

<210>  23
<211>  7
<212>  PRT
<213>  Artificial Sequence

<220>
<223>  C4.4a binder

<400>  23

Ser Asn Asn Gln Arg Pro Ser
1               5

<210>  24
<211>  7

<212>  PRT
<213>  Artificial Sequence

<220>
<223>  C4.4a binder

<400>  24

Ser Asn Asn Gln Arg Pro Ser
1               5


<210>  25
<211>  12
<212>  PRT
<213>  Artificial Sequence

<220>
<223>  C4.4a binder

<400>  25

Cys Ala Ala Trp Asp Asp Arg Leu Asn Gly Pro Val
1               5                   10


<210>  26
<211>  12
<212>  PRT
<213>  Artificial Sequence

<220>
<223>  C4.4a binder

<400>  26

Cys Ala Ala Trp Asp Asp Arg Leu Asn Gly Trp Val
1               5                   10


<210>  27
<211>  10
<212>  PRT
<213>  Artificial Sequence

<220>
<223>  C4.4a binder

<400>  27

Cys Gln Ser Tyr Asp Ser Ser His Val Leu
1               5                   10


<210>  28
<211>  12
<212>  PRT
<213>  Artificial Sequence

<220>
<223>  C4.4a binder

<400>  28

Cys Gln Ser Tyr Asp Arg Ser Leu Arg Gly Trp Val
1               5                   10

<210>  29
<211>  113
<212>  PRT
<213>  Artificial Sequence

<220>
<223>  C4.4a binder

<400>  29

Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Trp Asp Asp Arg
                85                  90                  95

Leu Asn Gly Pro Val Phe Gly Gly Gly Thr Lys Leu Thr Val Leu Gly
            100                 105                 110

Gln

<210>  30
<211>  112
<212>  PRT
<213>  Artificial Sequence

<220>
<223>  C4.4a binder

<400>  30

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Val Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Asn Gly Trp Val Phe Gly Gly Gly Thr Lys Leu Thr Val Leu Gly Gln
            100                 105                 110

<210>  31
<211>  111
<212>  PRT
<213>  Artificial Sequence

<220>
<223>  C4.4a binder

<400>  31

Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Ser Asn Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Gln Ser Tyr Asp Ser Ser
                85                  90                  95

His Val Leu Phe Gly Gly Gly Thr Lys Leu Thr Val Leu Gly Gln
            100                 105                 110

<210>  32
<211>  113
<212>  PRT

<213>  Artificial Sequence

<220>
<223>  C4.4a binder

<400>  32

Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Ser Asn Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Gln Ser Tyr Asp Arg Ser
                85                  90                  95

Leu Arg Gly Trp Val Phe Gly Gly Gly Thr Lys Leu Thr Val Leu Gly
            100                 105                 110

Gln

<210>  33
<211>  117
<212>  PRT
<213>  Artificial Sequence

<220>
<223>  C4.4a binder

<400>  33

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
            100                 105                 110

Val Thr Val Thr Ser
        115

<210>  34
<211>  122
<212>  PRT
<213>  Artificial Sequence

<220>
<223>  C4.4a binder

<400>  34

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Val Ser Trp Asn Gly Ala Arg Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ala Tyr Tyr Phe Asp Ser Trp
            100                 105                 110

Gly Gln Gly Thr Leu Val Thr Val Thr Ser
        115                 120

<210>  35
<211>  120
<212>  PRT

<213>  Artificial Sequence

<220>
<223>  C4.4a binder

<400>  35

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Gly His Tyr
            20                  25                  30

Tyr Met Phe Trp Ile Arg Gln Ala Pro Gly Lys Gly Leu Glu Trp Val
        35                  40                  45

Ser Ala Ile Ser Gly Ser Gly Tyr Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Leu Pro Tyr Gly Ser Gln Ser Gly Val Asp Tyr Trp Gly Gln
            100                 105                 110

Gly Thr Leu Val Thr Val Thr Ser
        115                 120


<210>  36
<211>  124
<212>  PRT
<213>  Artificial Sequence

<220>
<223>  C4.4a binder

<400>  36

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Asn
            20                  25                  30

Tyr Met Ser Trp Val Arg Gln Ala Pro Gly Lys Gly Leu Glu Trp Val
        35                  40                  45

Ser Ala Ile Ser Ser Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Ser Gly Gly Ser Gly Pro Asn Tyr Tyr Tyr Gly Met Asp
            100                 105                 110

Val Trp Gly Gln Gly Thr Leu Val Thr Val Thr Ser
        115                 120

<210>  37
<211>  339
<212>  DNA
<213>  Artificial Sequence

<220>
<223>  C4.4a binder

<400>  37
cagtctgtgc tgactcagcc accctcagcg tctgggaccc ctgggcagag ggtcaccatc      60

tcctgcactg ggagcagctc caacattggg gcgggttatg ttgtacattg gtatcagcag     120

ctcccaggaa cggcccccaa actcctcatc tatgacaata ataagcgacc ctcaggggtc     180

cctgaccgat tctctggctc caagtctggc acctcagcct ccctggccat cagtgggctc     240

cggtccgagg atgaggctga ttattactgt gcagcatggg atgacaggct gaatggtccg     300

gtgttcggcg gaggaaccaa gttaaccgtc ctaggtcag                            339

<210>  38
<211>  336
<212>  DNA
<213>  Artificial Sequence

<220>
<223>  C4.4a binder

<400>  38
cagtctgtgc tgactcagcc accctcagcg tctgggaccc ccgggcagag ggtcaccatc      60

tcttgttctg gaagcagctc caacgtcggg agtaatcctg taaactggta tcagcagctc     120

ccaggaacgg cccccaaact cctcatctat aggaataatc agcggccctc aggggtccct     180

gaccgattct ctggctccaa gtctggcacc tcagcctccc tggccatcag tgggctccgg     240

tccgaggatg aggctgatta ttactgtgca gcatgggatg acaggctgaa tggttgggtg     300

ttcggcggag gaaccaagct gacggtccta ggtcag                               336

<210>  39

<211>  333
<212>  DNA
<213>  Artificial Sequence

<220>
<223>  C4.4a binder

<400>  39
cagtctgtgc tgactcagcc accctcagcg tctgggaccc ccgggcagag ggtcaccatc      60

tcctgcactg ggagcagctc caacattggg gcgggttatg ttgtacattg gtatcagcag     120

ctcccaggaa cggcccccaa actcctcatc tatagtaata atcagcggcc ctcaggagtc     180

cctgaccgat tctctggctc caagtctggc acctcagcct ccctggccat cagtgggctc     240

cggtccgagg atgaggctga ttattactgc cagtcctatg acagcagcca tgttttattc     300

ggcggaggaa ccaagctgac ggtcctaggt cag                                  333


<210>  40
<211>  339
<212>  DNA
<213>  Artificial Sequence

<220>
<223>  C4.4a binder

<400>  40
cagtctgtgc tgactcagcc accctcagcg tctgggaccc ccgggcagag ggtcaccatc      60

tcctgcactg ggagcagctc caacattggg gcgggttatg ttgtacattg gtatcagcag     120

ctcccaggaa cggcccccaa actcctcatc tatagtaata atcagcggcc ctcaggggtc     180

cctgaccgat tctctggctc caagtctggc acctcagcct ccctggccat cagtgggctc     240

cggtccgagg atgaggctga ttattactgc cagtcctatg acagaagcct gcgtggttgg     300

gtgttcggcg gaggaaccaa gctgacggtc ctaggtcag                            339


<210>  41
<211>  351
<212>  DNA
<213>  Artificial Sequence

<220>
<223>  C4.4a binder

<400>  41
gaggtgcagc tgttggagtc tgggggaggc ttggtacagc ctggggggtc cctgagactc      60

tcctgtgcag cctctggatt caccttcagt aacgcctgga tgagctgggt ccgccaggct     120

ccagggaagg ggctggagtg ggtttcatac attagtagta gtggtagtac catatactac     180

gcagactctg tgaagggccg attcaccatc tccagagaca attccaagaa cacgctgtat     240

ctgcaaatga acagcctgag agccgaggac actgccgtgt attactgtgc gagagaaggg     300

ttatgggcct ttgactactg gggccagggt accctggtca ccgtgactag t              351

<210> 42
<211> 366
<212> DNA
<213> Artificial Sequence

<220>
<223> C4.4a binder

<400> 42
gaggtgcagc tgttggagtc tgggggaggc ttggtacagc ctggggggtc cctgagactc 60

tcctgtgcag cctctggatt caccttcagt gactatcaga tgacctggat ccgccagact 120

ccagggaagg ggctggagtg ggtatcgggt gttagttgga atggcgctag gacgcactat 180

gcagactctg tgaagggccg attcaccatc tccagagaca attccaagaa cacgctgtat 240

ctacaaatga acagcctgag agccgaggac actgccgtgt attactgtgc gaagggcgac 300

tacctggttt actccgcata ctactttgac tcctggggcc agggtaccct ggtcaccgtg 360

actagt 366


<210> 43
<211> 360
<212> DNA
<213> Artificial Sequence

<220>
<223> C4.4a binder

<400> 43
gaggtgcagc tgttggagtc tgggggaggc ttggtacagc ctggggggtc cctgagactc 60

tcctgtgcag cctctggatt caccttcggt cactactata tgttctggat ccgtcaggct 120

ccagggaagg ggctggagtg ggtctcagct attagtggta gtggttatag cacacactac 180

gcagactccg tgaagggccg gttcaccatc tccagagaca attccaagaa cacgctgtat 240

ctgcaaatga acagcctgag agccgaggac actgccgtgt attactgtgc gagactgcca 300

tatggttcgc agagtggcgt tgactactgg ggccagggta ccctggtcac cgtgactagt 360


<210> 44
<211> 372
<212> DNA
<213> Artificial Sequence

<220>
<223> C4.4a binder

<400> 44
gaggtgcagc tgttggagtc tgggggaggc ttggtacagc ctggggggtc cctgagactc 60

tcctgtgcag cctctggatt caccttcagt agcaactaca tgagctgggt ccgccaggct 120

ccagggaagg ggctggagtg ggtctcagct attagtagta gtggtagtag cacatactac 180

gcagactccg tgaagggccg gttcaccatc tccagagaca attccaagaa cacgctgtat 240

ctgcaaatga acagcctgag agccgaggac actgccgtgt attactgtgc gagagaatct 300

ggtgggagcg gaccgaacta ctactacggt atggacgtct ggggccaagg taccctggtc 360

accgtgacta gt 372

<210> 45
<211> 9
<212> PRT
<213> Artificial Sequence

<220>
<223> C4.4a binder

<400> 45

Phe Ser Asn Ala Trp Met Ser Trp Val
1               5

<210> 46
<211> 20
<212> PRT
<213> Artificial Sequence

<220>
<223> C4.4a binder

<400> 46

Val Ser Tyr Ile Ser Ser Ser Gly Ser Thr Ile Tyr Tyr Ala Asp Ser
1               5                   10                  15

Val Lys Gly Arg
            20

<210> 47
<211> 10
<212> PRT
<213> Artificial Sequence

<220>
<223> C4.4a binder

<400> 47

Ala Arg Glu Gly Leu Trp Ala Phe Asp Tyr
1               5                   10

<210> 48
<211> 14
<212> PRT
<213> Artificial Sequence

<220>
<223> C4.4a binder

<400>  48

Thr Gly Ser Ser Ser Asn Ile Gly Ala Gly Tyr Val Val His
1               5                   10


<210>  49
<211>  7
<212>  PRT
<213>  Artificial Sequence

<220>
<223>  C4.4a binder

<400>  49

Asp Asn Asn Lys Arg Pro Ser
1               5


<210>  50
<211>  12
<212>  PRT
<213>  Artificial Sequence

<220>
<223>  C4.4a binder

<400>  50

Cys Ala Ala Trp Asp Asp Arg Leu Asn Gly Pro Val
1               5                   10


<210>  51
<211>  117
<212>  PRT
<213>  Artificial Sequence

<220>
<223>  C4.4a binder

<400>  51

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Asn Ala
            20                  25                  30


Trp Met Ser Trp Val Arg Gln Ala Pro Gly Lys Gly Leu Glu Trp Val
        35                  40                  45


Ser Tyr Ile Ser Ser Ser Gly Ser Thr Ile Tyr Tyr Ala Asp Ser Val
    50                  55                  60


Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
            100                 105                 110

Val Thr Val Ser Ser
        115

<210>  52
<211>  113
<212>  PRT
<213>  Artificial Sequence

<220>
<223>  C4.4a binder

<400>  52

Glu Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Trp Asp Asp Arg
                85                  90                  95

Leu Asn Gly Pro Val Phe Gly Gly Gly Thr Lys Leu Thr Val Leu Gly
            100                 105                 110

Gln

<210>  53
<211>  351
<212>  DNA
<213>  Artificial Sequence

<220>
<223>  C4.4a binder

<400>  53
gaggtgcagc tgctggaaag cggcggagga ctggtgcagc ctggaggcag cctgagactg      60

tcttgcgccg ccagcggctt caccttcagc aacgcctgga tgagctgggt ccgacaggct     120

cctggcaagg gcctggaatg ggtgtcctac atcagcagca gcggcagcac catctactac     180

gccgacagcg tgaagggccg gttcaccatc agccgggaca acagcaagaa caccctgtac     240

ctgcagatga acagcctgcg ggccgaggac accgccgtgt actactgcgc cagagaaggc     300

ctgtgggcct tcgactactg gggccagggc accctggtca ccgtgtctag c              351


<210>  54
<211>  339
<212>  DNA
<213>  Artificial Sequence

<220>
<223>  C4.4a binder

<400>  54
gagagcgtgc tgacccagcc tcctagcgtg tccggcgctc ctggccagag agtgaccatc      60

agctgcaccg gcagcagcag caacatcgga gccggctacg tggtgcactg gtatcagcag     120

ctgcccggca ccgcccccaa gctgctgatc tacgacaaca acaagcggcc tagcggcgtg     180

cccgacagat tcagcggcag caagagcggc accagcgcca gcctggccat cagcggcctg     240

agaagcgagg acgaggccga ctactactgc gccgcctggg acgacagact gaacggccct     300

gtgttcggcg gaggcaccaa gctgaccgtg ctgggacag                            339


<210>  55
<211>  9
<212>  PRT
<213>  Artificial Sequence

<220>
<223>  C4.4a binder

<400>  55

Phe Ser Asn Ala Trp Met Ser Trp Val
1               5


<210>  56
<211>  20
<212>  PRT
<213>  Artificial Sequence

<220>
<223>  C4.4a binder

<400>  56

Val Ser Tyr Ile Ser Ser Ser Gly Ser Thr Ile Tyr Tyr Ala Asp Ser
1               5                   10                  15

Val Lys Gly Arg
            20

<210>  57
<211>  10
<212>  PRT
<213>  Artificial Sequence

<220>
<223>  C4.4a binder

<400>  57

Ala Arg Glu Gly Leu Trp Ala Phe Asp Tyr
1               5                   10

<210>  58
<211>  14
<212>  PRT
<213>  Artificial Sequence

<220>
<223>  C4.4a binder

<400>  58

Thr Gly Ser Ser Ser Asn Ile Gly Ala Gly Tyr Val Val His
1               5                   10

<210>  59
<211>  7
<212>  PRT
<213>  Artificial Sequence

<220>
<223>  C4.4a binder

<400>  59

Asp Asn Asn Lys Arg Pro Ser
1               5

<210>  60
<211>  12
<212>  PRT
<213>  Artificial Sequence

<220>
<223>  C4.4a binder

<400>  60

Cys Ala Ala Tyr Asp Asp Ser Leu Ser Gly Pro Val
1               5                   10

<210>  61
<211>  117

<212>  PRT
<213>  Artificial Sequence

<220>
<223>  C4.4a binder

<400>  61

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
            100                 105                 110

Val Thr Val Ser Ser
        115


<210>  62
<211>  113
<212>  PRT
<213>  Artificial Sequence

<220>
<223>  C4.4a binder

<400>  62

Gln Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe

50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Tyr Asp Asp Ser
                85                  90                  95

Leu Ser Gly Pro Val Phe Gly Gly Gly Thr Lys Leu Thr Val Leu Gly
            100                 105                 110

Gln

<210>  63
<211>  351
<212>  DNA
<213>  Artificial Sequence

<220>
<223>  C4.4a binder

<400>  63
gaggtgcagc tgctggaatc cggcggaggc ctggtgcagc ctggcggatc tctgagactg      60

tcctgcgccg ccagcggctt taccttctcc aacgcctgga tgtcctgggt ccgacaggcc     120

cctggcaagg gactggaatg ggtgtcctac atctcctcct ccggctccac catctactac     180

gccgactccg tgaagggccg gttcaccatc tcccgggaca actccaagaa caccctgtac     240

ctgcagatga actccctgcg ggccgaggac accgccgtgt actactgcgc ccgagagggc     300

ctgtgggcct tcgattattg gggccagggc accctggtca ccgtcagctc a              351

<210>  64
<211>  339
<212>  DNA
<213>  Artificial Sequence

<220>
<223>  C4.4a binder

<400>  64
cagtccgtgc tgacccagcc cccttctgtg tctggcgccc ctggccagag agtgaccatc      60

tcttgcaccg gctcctccag caacatcggc gctggctacg tggtgcactg gtatcagcag     120

ctgcccggca ccgcccccaa gctgctgatc tacgacaaca acaagcggcc ctccggcgtg     180

cccgacagat tctccggctc caagtccggc acctccgcct ccctggccat ctccggcctg     240

agatctgagg acgaggccga ctactactgc gccgcctacg acgactccct gtccggccct     300

gtgttcggcg gaggcacaaa gttaaccgtg ctgggccag                            339

<210>  65
<211>  9
<212>  PRT
<213>  Artificial Sequence

<220>
<223>  C4.4a binder

<400>  65

Phe Ser Asn Ala Trp Met Ser Trp Val
1               5


<210>  66
<211>  20
<212>  PRT
<213>  Artificial Sequence

<220>
<223>  C4.4a binder

<400>  66

Val Ser Tyr Ile Ser Ser Ser Gly Ser Thr Ile Tyr Tyr Ala Asp Ser
1               5                   10                  15


Val Lys Gly Arg
            20


<210>  67
<211>  10
<212>  PRT
<213>  Artificial Sequence

<220>
<223>  C4.4a binder

<400>  67

Ala Arg Glu Gly Leu Trp Ala Phe Asp Tyr
1               5                   10


<210>  68
<211>  14
<212>  PRT
<213>  Artificial Sequence

<220>
<223>  C4.4a binder

<400>  68

Thr Gly Ser Ser Ser Asn Ile Gly Ala Gly Tyr Val Val His
1               5                   10


<210>  69
<211>  7
<212>  PRT

<213>  Artificial Sequence

<220>
<223>  C4.4a binder

<400>  69

Asp Asn Asn Lys Arg Pro Ser
1               5


<210>  70
<211>  12
<212>  PRT
<213>  Artificial Sequence

<220>
<223>  C4.4a binder

<400>  70

Cys Ala Ala Phe Asp Asp Ser Leu Asn Gly Pro Val
1               5                   10


<210>  71
<211>  117
<212>  PRT
<213>  Artificial Sequence

<220>
<223>  C4.4a binder

<400>  71

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Asn Ala
            20                  25                  30


Trp Met Ser Trp Val Arg Gln Ala Pro Gly Lys Gly Leu Glu Trp Val
        35                  40                  45


Ser Tyr Ile Ser Ser Ser Gly Ser Thr Ile Tyr Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Glu Gly Leu Trp Ala Phe Asp Lys Trp Gly Gln Gly Thr Leu
            100                 105                 110

Val Thr Val Ser Ser
        115

<210>  72
<211>  113
<212>  PRT
<213>  Artificial Sequence

<220>
<223>  C4.4a binder

<400>  72

Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Phe Asp Asp Arg
                85                  90                  95

Leu Asn Gly Pro Val Phe Gly Gly Gly Thr Lys Leu Thr Val Leu Gly
            100                 105                 110

Gln

<210>  73
<211>  351
<212>  DNA
<213>  Artificial Sequence

<220>
<223>  C4.4a binder

<400>  73
gaggtgcagc tgctggaatc cggcggaggc ctggtgcagc ctggcggatc tctgagactg      60

tcctgcgccg cctccggctt taccttctcc aacgcctgga tgtcctgggt ccgacaggct     120

cctggcaagg gcctggaatg ggtgtcctac atctcctcct ccggctccac catctactac     180

gccgactccg tgaagggccg gttcaccatc tcccgggaca actccaagaa caccctgtac     240

ctgcagatga actccctgcg ggccgaggac accgccgtgt actactgcgc ccgagagggc 300

ctgtgggcct tcgataagtg gggccagggc accctggtca ccgtcagctc a 351

<210> 74
<211> 339
<212> DNA
<213> Artificial Sequence

<220>
<223> C4.4a binder

<400> 74
cagtccgtgc tgacccagcc tccttccgcc tctggcaccc ctggccagag agtgaccatc 60

tcctgcaccg gctcctccag caacatcggc gctggctacg tggtgcactg gtatcagcag 120

ctgcccggca ccgcccccaa gctgctgatc tacgacaaca acaagcggcc ctccggcgtg 180

cccgacagat tctccggctc caagtccggc acctccgcct ccctggccat ctccggcctg 240

agatctgagg acgaggccga ctactactgc gccgccttcg acgaccggct gaacggccct 300

gtgttcggcg gaggcacaaa gttaaccgtg ctgggccag 339

<210> 75
<211> 9
<212> PRT
<213> Artificial Sequence

<220>
<223> C4.4a binder

<400> 75

Phe Ser Ser Ala Trp Met Ser Trp Val
1               5

<210> 76
<211> 20
<212> PRT
<213> Artificial Sequence

<220>
<223> C4.4a binder

<400> 76

Val Ser Tyr Ile Ser Ser Ser Gly Ser Thr Ile Tyr Tyr Ala Asp Ser
1               5                   10                  15

Val Lys Gly Arg
            20

<210> 77
<211> 10
<212> PRT
<213> Artificial Sequence

<220>
<223>  C4.4a binder

<400>  77

Ala Arg Glu Gly Leu Trp Ala Phe Asp Tyr
1               5                   10


<210>  78
<211>  14
<212>  PRT
<213>  Artificial Sequence

<220>
<223>  C4.4a binder

<400>  78

Thr Gly Ser Ser Ser Asn Ile Gly Ala Gly Tyr Val Val His
1               5                   10


<210>  79
<211>  7
<212>  PRT
<213>  Artificial Sequence

<220>
<223>  C4.4a binder

<400>  79

Asp Asn Asn Lys Arg Pro Ser
1               5


<210>  80
<211>  12
<212>  PRT
<213>  Artificial Sequence

<220>
<223>  C4.4a binder

<400>  80

Cys Ala Ala Tyr Asp Asp Ser Leu Ser Gly Pro Val
1               5                   10


<210>  81
<211>  117
<212>  PRT
<213>  Artificial Sequence

<220>
<223>  C4.4a binder

<400>  81

Glu Val Gln Leu Leu Glu Ser Gly Gly Gly Leu Val Gln Pro Gly Gly

1                   5                   10                  15

Ser Leu Arg Leu Ser Cys Ala Ala Ser Gly Phe Thr Phe Ser Ser Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
            100                 105                 110

Val Thr Val Ser Ser
        115

<210>  82
<211>  113
<212>  PRT
<213>  Artificial Sequence

<220>
<223>  C4.4a binder

<400>  82

Gln Ser Val Leu Thr Gln Pro Pro Ser Val Ser Gly Ala Pro Gly Gln
1                   5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Tyr Asp Asp Ser
                85                  90                  95

Leu Ser Gly Pro Val Phe Gly Gly Gly Thr Lys Leu Thr Val Leu Gly
            100                 105                 110

Gln

<210>  83
<211>  351
<212>  DNA
<213>  Artificial Sequence

<220>
<223>  C4.4a binder

<400>  83
gaggtgcagc tgctggaatc cggcggaggc ctggtgcagc ctggcggatc tctgagactg      60

tcctgcgccg ccagcggctt taccttctcc agcgcctgga tgtcctgggt ccgacaggcc     120

cctggcaagg gactggaatg ggtgtcctac atctcctcct ccggctccac catctactac     180

gccgactccg tgaagggccg gttcaccatc tcccgggaca actccaagaa caccctgtac     240

ctgcagatga actccctgcg ggccgaggac accgccgtgt actactgcgc ccgagagggc     300

ctgtgggcct tcgattattg gggccagggc accctggtca ccgtcagctc a              351

<210>  84
<211>  339
<212>  DNA
<213>  Artificial Sequence

<220>
<223>  C4.4a binder

<400>  84
cagtccgtgc tgacccagcc cccttctgtg tctggcgccc ctggccagag agtgaccatc      60

tcttgcaccg gctcctccag caacatcggc gctggctacg tggtgcactg gtatcagcag     120

ctgcccggca ccgcccccaa gctgctgatc tacgacaaca acaagcggcc ctccggcgtg     180

cccgacagat tctccggctc caagtccggc acctccgcct ccctggccat ctccggcctg     240

agatctgagg acgaggccga ctactactgc gccgcctacg acgactccct gtccggccct     300

gtgttcggcg gaggcacaaa gttaaccgtg ctgggccag                            339

<210>  85
<211>  9
<212>  PRT
<213>  Artificial Sequence

<220>
<223>  C4.4a binder

<400>  85

Phe Ser Ser Ala Trp Met Ser Trp Val
1               5

<210>  86
<211>  20
<212>  PRT
<213>  Artificial Sequence

<220>
<223>  C4.4a binder

<400>  86

Val Ser Tyr Ile Ser Ser Ser Gly Ser Ser Thr Tyr Tyr Ala Asp Ser
1               5                   10                  15

Val Lys Gly Arg
            20

<210>  87
<211>  10
<212>  PRT
<213>  Artificial Sequence

<220>
<223>  C4.4a binder

<400>  87

Ala Arg Glu Gly Leu Trp Ala Phe Asp Lys
1               5                   10

<210>  88
<211>  14
<212>  PRT
<213>  Artificial Sequence

<220>
<223>  C4.4a binder

<400>  88

Ser Gly Ser Ser Ser Asn Ile Gly Ala Gly Tyr Val Val His
1               5                   10

<210>  89
<211>  7
<212>  PRT
<213>  Artificial Sequence

<220>
<223>  C4.4a binder

<400>  89

Asp Asn Asn Gln Arg Pro Ser
1               5

<210>  90
<211>  12
<212>  PRT
<213>  Artificial Sequence

<220>
<223>  C4.4a binder

<400>  90

Cys Ala Ala Phe Asp Asp Arg Leu Ser Gly Pro Val
1               5                   10


<210>  91
<211>  117
<212>  PRT
<213>  Artificial Sequence

<220>
<223>  C4.4a binder

<400>  91

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Ser Ala
            20                  25                  30


Trp Met Ser Trp Val Arg Gln Ala Pro Gly Lys Gly Leu Glu Trp Val
        35                  40                  45


Ser Tyr Ile Ser Ser Ser Gly Ser Ser Thr Tyr Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Glu Gly Leu Trp Ala Phe Asp Lys Trp Gly Gln Gly Thr Leu
            100                 105                 110


Val Thr Val Ser Ser
        115


<210>  92
<211>  113
<212>  PRT
<213>  Artificial Sequence

<220>
<223>  C4.4a binder

<400>  92

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Phe Asp Asp Arg
                85                  90                  95

Leu Ser Gly Pro Val Phe Gly Gly Gly Thr Lys Leu Thr Val Leu Gly
            100                 105                 110

Gln

<210>  93
<211>  351
<212>  DNA
<213>  Artificial Sequence

<220>
<223>  C4.4a binder

<400>  93
gaggtgcagc tgctggaaag cggcggaggc ctggtgcagc ctggcggaag cctgagactg      60

agctgtgccg ccagcggctt caccttcagc agcgcctgga tgagctgggt ccgacaggcc     120

cctggcaagg gcctggaatg ggtgtcctac atcagcagca gcggcagcag cacctactac     180

gccgacagcg tgaagggccg gttcaccatc agccgggaca acagcaagaa caccctgtac     240

ctgcagatga acagcctgcg ggccgaggac accgccgtgt actactgcgc cagagaaggc     300

ctgtgggcct tcgataagtg gggccagggc accctggtca ccgtcagctc a              351

<210>  94
<211>  339
<212>  DNA
<213>  Artificial Sequence

<220>
<223>  C4.4a binder

<400>  94
cagagcgtgc tgacccagcc tcctagcgcc tctggcaccc ctggccagag agtgaccatc      60

agctgcagcg gcagcagcag caacatcgga gccggctacg tggtgcactg gtatcagcag     120

ctgcccggca ccgcccccaa gctgctgatc tacgacaaca accagcggcc cagcggcgtg     180

cccgacagat tttccggcag caagagcggc accagcgcca gcctggccat cagcggcctg     240

agaagcgagg acgaggccga ctactactgc gccgccttcg acgacagact gagcggccct     300

gtgttcggcg gaggcacaaa gttaaccgtg ctgggccag                            339


<210>  95
<211>  9
<212>  PRT
<213>  Artificial Sequence

<220>
<223>  C4.4a binder

<400>  95

Phe Ser Asp Tyr Gln Met Thr Trp Ile
1               5


<210>  96
<211>  20
<212>  PRT
<213>  Artificial Sequence

<220>
<223>  C4.4a binder

<400>  96

Val Ser Gly Val Ser Trp Asn Gly Ala Arg Thr His Tyr Ala Asp Ser
1               5                   10                  15


Val Lys Gly Arg
            20


<210>  97
<211>  15
<212>  PRT
<213>  Artificial Sequence

<220>
<223>  C4.4a binder

<400>  97

Ala Lys Gly Asp Tyr Leu Val Tyr Ser Ala Tyr Tyr Phe Asp Ser
1               5                   10                  15

<210>  98
<211>  13
<212>  PRT
<213>  Artificial Sequence

<220>
<223>  C4.4a binder

<400>  98

Ser Gly Ser Ser Ser Asn Val Gly Ser Asn Pro Val Asn
1               5                   10


<210>  99
<211>  7
<212>  PRT
<213>  Artificial Sequence

<220>
<223>  C4.4a binder

<400>  99

Arg Asn Asn Gln Arg Pro Ser
1               5


<210>  100
<211>  12
<212>  PRT
<213>  Artificial Sequence

<220>
<223>  C4.4a binder

<400>  100

Cys Ala Ala Trp Asp Asp Arg Leu Asn Gly Trp Val
1               5                   10


<210>  101
<211>  122
<212>  PRT
<213>  Artificial Sequence

<220>
<223>  C4.4a binder

<400>  101

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Asp Tyr
            20                  25                  30


Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ala Tyr Tyr Phe Asp Ser Trp
            100                 105                 110

Gly Gln Gly Thr Leu Val Thr Val Ser Ser
        115                 120

<210>  102
<211>  112
<212>  PRT
<213>  Artificial Sequence

<220>
<223>  C4.4a binder

<400>  102

Glu Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Asn Gly Trp Gly Phe Gly Gly Gly Thr Lys Leu Thr Val Leu Gly Gln
            100                 105                 110

<210>  103
<211>  366
<212>  DNA

<213>  Artificial Sequence

<220>
<223>  C4.4a binder

<400>  103
gaggtgcagc tgctggaaag cggcggagga ctggtgcagc ctggaggcag cctgagactg      60

tcttgcgccg ccagcggctt caccttcagc gactaccaga tgacctggat ccgacagacc     120

cctggcaagg gcctggaatg ggtgtccggc atcagctgga acggaggcag cacccactac     180

gccgacagcg tgaagggccg gttcaccatc agccgggaca acagcaagaa caccctgtac     240

ctgcagatga acagcctgcg ggccgaggac accgccgtgt actactgcgc caagggcgac     300

tacctggtgt acagcgccta ctacttcgac agctggggcc agggcaccct ggtcaccgtg     360

tctagc                                                                366


<210>  104
<211>  336
<212>  DNA
<213>  Artificial Sequence

<220>
<223>  C4.4a binder

<400>  104
gagagcgtgc tgacccagcc tcctagcgcc tctggcaccc ctggccagag agtgaccatc      60

agctgctctg gcagcagcag caacatcgga agcaaccccg tgaactggta tcagcagctg     120

cccggcaccg cccccaagct gctgatctac cggaacaacc agcggcctag cggcgtgccc     180

gacagattca gcggcagcaa gagcggcacc agcgccagcc tggccatcag cggcctgaga     240

agcgaggacg aggccgacta ctactgcgcc gcctgggacg acagactgaa cggctggggc     300

ttcggcggag gcaccaagct gaccgtgctg ggacag                               336


<210>  105
<211>  9
<212>  PRT
<213>  Artificial Sequence

<220>
<223>  C4.4a binder

<400>  105

Phe Ser Asp Tyr Gln Met Thr Trp Ile
1               5


<210>  106
<211>  20
<212>  PRT
<213>  Artificial Sequence

<220>

<223>  C4.4a binder

<400>  106

Val Ser Gly Ile Ser Trp Asn Gly Gly Ser Thr His Tyr Ala Asp Ser
1               5                   10                  15

Val Lys Gly Arg
            20

<210>  107
<211>  15
<212>  PRT
<213>  Artificial Sequence

<220>
<223>  C4.4a binder

<400>  107

Ala Lys Gly Asp Tyr Leu Val Tyr Ser Ser Tyr Tyr Phe Lys Ser
1               5                   10                  15

<210>  108
<211>  13
<212>  PRT
<213>  Artificial Sequence

<220>
<223>  C4.4a binder

<400>  108

Ser Gly Ser Ser Ser Asn Ile Gly Ser Asn Pro Val Asn
1               5                   10

<210>  109
<211>  7
<212>  PRT
<213>  Artificial Sequence

<220>
<223>  C4.4a binder

<400>  109

Arg Asn Asn Gln Arg Pro Ser
1               5

<210>  110
<211>  12
<212>  PRT
<213>  Artificial Sequence

<220>
<223>  C4.4a binder

<400>  110

Cys Ala Ala Trp Asp Asp Arg Leu Ser Gly Trp Ala
1               5                   10

<210>  111
<211>  122
<212>  PRT
<213>  Artificial Sequence

<220>
<223>  C4.4a binder

<400>  111

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ser Tyr Tyr Phe Lys Ser Trp
            100                 105                 110

Gly Gln Gly Thr Leu Val Thr Val Ser Ser
        115                 120

<210>  112
<211>  112
<212>  PRT
<213>  Artificial Sequence

<220>
<223>  C4.4a binder

<400>  112

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Ser Gly Trp Ala Phe Gly Gly Gly Thr Lys Leu Thr Val Leu Gly Gln
            100                 105                 110

<210>  113
<211>  366
<212>  DNA
<213>  Artificial Sequence

<220>
<223>  C4.4a binder

<400>  113
gaggtgcagc tgctggaatc cggcggaggc ctggtgcagc ctggcggatc tctgagactg      60

tcctgcgccg cctccggctt caccttctcc gactaccaga tgacctggat cagacagacc     120

cccggcaagg gcctggaatg ggtgtccggc atctcctgga acggcggctc cacccactac     180

gccgactctg tgaagggccg gttcaccatc tcccgggaca actccaagaa caccctgtac     240

ctgcagatga actccctgcg ggccgaggac accgccgtgt actactgcgc caagggcgac     300

tacctggtgt actcctccta ctacttcaag tcctggggcc agggcaccct ggtcaccgtc     360

agctca                                                                366

<210>  114
<211>  336
<212>  DNA
<213>  Artificial Sequence

<220>
<223>  C4.4a binder

<400>  114
cagtccgtgc tgacccagcc tccttccgcc tctggcaccc ctggccagag agtgaccatc      60

tcctgctccg gctcctcctc caacatcggc tccaaccccg tgaactggta tcagcagctg     120

cccggcaccg cccccaagct gctgatctac cggaacaacc agcggccctc cggcgtgccc     180

gacagattct ccggctccaa gtccggcacc tccgcctccc tggccatctc cggcctgaga     240

tctgaggacg aggccgacta ctactgcgcc gcctgggacg accggctgtc tggctgggct 300

tttggcggcg gaacaaagtt aaccgtgctg ggccag 336

<210> 115
<211> 9
<212> PRT
<213> Artificial Sequence

<220>
<223> C4.4a binder

<400> 115

Phe Ser Asp Tyr Gln Met Thr Trp Ile
1               5

<210> 116
<211> 20
<212> PRT
<213> Artificial Sequence

<220>
<223> C4.4a binder

<400> 116

Val Ser Gly Ile Ser Trp Asn Gly Gly Ser Thr His Tyr Ala Asp Ser
1               5                   10                  15

Val Lys Gly Arg
            20

<210> 117
<211> 15
<212> PRT
<213> Artificial Sequence

<220>
<223> C4.4a binder

<400> 117

Ala Lys Gly Asp Tyr Leu Val Tyr Lys Ser Tyr Tyr Phe Lys Ser
1               5                   10                  15

<210> 118
<211> 13
<212> PRT
<213> Artificial Sequence

<220>
<223> C4.4a binder

<400> 118

Ser Gly Ser Ser Ser Asn Ile Gly Ser Asn Pro Val Asn
1               5                   10

<210>  119
<211>  7
<212>  PRT
<213>  Artificial Sequence

<220>
<223>  C4.4a binder

<400>  119

Arg Asn Asn Gln Arg Pro Ser
1               5


<210>  120
<211>  12
<212>  PRT
<213>  Artificial Sequence

<220>
<223>  C4.4a binder

<400>  120

Cys Ala Ala Trp Asp Asp Ser Leu Ser Gly Trp Ala
1               5                   10


<210>  121
<211>  122
<212>  PRT
<213>  Artificial Sequence

<220>
<223>  C4.4a binder

<400>  121

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Asp Tyr
            20                  25                  30


Gln Met Thr Trp Ile Arg Gln Thr Pro Gly Lys Gly Leu Glu Trp Val
        35                  40                  45


Ser Gly Ile Ser Trp Asn Gly Gly Ser Thr His Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95

Ala Lys Gly Asp Tyr Leu Val Tyr Lys Ser Tyr Tyr Phe Lys Ser Trp
            100                 105                 110

Gly Gln Gly Thr Leu Val Thr Val Ser Ser
        115                 120

<210> 122
<211> 112
<212> PRT
<213> Artificial Sequence

<220>
<223> C4.4a binder

<400> 122

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Ser Leu
                85                  90                  95

Ser Gly Trp Ala Phe Gly Gly Gly Thr Lys Leu Thr Val Leu Gly Gln
            100                 105                 110

<210> 123
<211> 366
<212> DNA
<213> Artificial Sequence

<220>
<223> C4.4a binder

<400> 123
gaggtgcagc tgctggaatc cggcggaggc ctggtgcagc ctggcggatc tctgagactg 60

tcctgcgccg cctccggctt caccttctcc gactaccaga tgacctggat cagacagacc 120

cccggcaagg gcctggaatg ggtgtccggc atctcctgga acggcggctc cacccactac 180

gccgactctg tgaagggccg gttcaccatc tcccgggaca actccaagaa caccctgtac 240

ctgcagatga actccctgcg ggccgaggac accgccgtgt actactgcgc caagggcgac 300

tacctggtgt acaagtccta ctacttcaag tcctggggcc agggcaccct ggtcaccgtc 360

agctca 366

<210> 124
<211> 336
<212> DNA
<213> Artificial Sequence

<220>
<223> C4.4a binder

<400> 124
cagtccgtgc tgacccagcc tccttccgcc tctggcaccc ctggccagag agtgaccatc 60

tcctgctccg gctcctcctc caacatcggc tccaaccccg tgaactggta tcagcagctg 120

cccggcaccg cccccaagct gctgatctac cggaacaacc agcggccctc cggcgtgccc 180

gacagattct ccggctccaa gtccggcacc tccgcctccc tggccatctc cggcctgaga 240

tctgaggacg aggccgacta ctactgcgcc gcctgggacg actccctgtc tggctgggct 300

tttggcggcg gaacaaagtt aaccgtgctg ggccag 336

<210> 125
<211> 9
<212> PRT
<213> Artificial Sequence

<220>
<223> C4.4a binder

<400> 125

Phe Ser Asp Tyr Gln Met Thr Trp Ile
1               5

<210> 126
<211> 20
<212> PRT
<213> Artificial Sequence

<220>
<223> C4.4a binder

<400> 126

Val Ser Gly Ile Ser Trp Asn Gly Gly Ser Thr His Tyr Ala Asp Ser
1               5                   10                  15

Val Lys Gly Arg
            20

<210> 127

<211>  15
<212>  PRT
<213>  Artificial Sequence

<220>
<223>  C4.4a binder

<400>  127

Ala Lys Gly Asp Tyr Leu Val Tyr Ser Ser Tyr Tyr Phe Lys Ser
1               5                   10                  15


<210>  128
<211>  13
<212>  PRT
<213>  Artificial Sequence

<220>
<223>  C4.4a binder

<400>  128

Ser Gly Ser Ser Ser Asn Ile Gly Ser Asn Pro Val Asn
1               5                   10


<210>  129
<211>  7
<212>  PRT
<213>  Artificial Sequence

<220>
<223>  C4.4a binder

<400>  129

Arg Asn Asn Gln Arg Pro Ser
1               5


<210>  130
<211>  12
<212>  PRT
<213>  Artificial Sequence

<220>
<223>  C4.4a binder

<400>  130

Cys Ala Ala Trp Asp Asp Arg Leu Ser Gly Trp Gly
1               5                   10


<210>  131
<211>  122
<212>  PRT
<213>  Artificial Sequence

<220>
<223>  C4.4a binder

<400>  131

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ser Tyr Tyr Phe Lys Ser Trp
            100                 105                 110

Gly Gln Gly Thr Leu Val Thr Val Ser Ser
        115                 120


<210>  132
<211>  112
<212>  PRT
<213>  Artificial Sequence

<220>
<223>  C4.4a binder

<400>  132

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Ser Gly Trp Gly Phe Gly Gly Gly Thr Lys Leu Thr Val Leu Gly Gln
            100                 105                 110

<210>  133
<211>  366
<212>  DNA
<213>  Artificial Sequence

<220>
<223>  C4.4a binder

<400>  133
gaggtgcagc tgctggaatc cggcggaggc ctggtgcagc ctggcggatc tctgagactg      60

tcctgcgccg cctccggctt caccttctcc gactaccaga tgacctggat cagacagacc     120

cccggcaagg gcctggaatg ggtgtccggc atctcctgga acggcggctc cacccactac     180

gccgactctg tgaagggccg gttcaccatc tcccgggaca actccaagaa caccctgtac     240

ctgcagatga actccctgcg ggccgaggac accgccgtgt actactgcgc caagggcgac     300

tacctggtgt actcctccta ctacttcaag tcctggggcc agggcaccct ggtcaccgtc     360

agctca                                                                366

<210>  134
<211>  336
<212>  DNA
<213>  Artificial Sequence

<220>
<223>  C4.4a binder

<400>  134
cagtccgtgc tgacccagcc tccttccgcc tctggcaccc ctggccagag agtgaccatc      60

tcctgctccg gctcctcctc caacatcggc tccaaccccg tgaactggta tcagcagctg     120

cccggcaccg cccccaagct gctgatctac cggaacaacc agcggccctc cggcgtgccc     180

gacagattct ccggctccaa gtccggcacc tccgcctccc tggccatctc cggcctgaga     240

tctgaggacg aggccgacta ctactgcgcc gcctgggacg accggctgtc tggctgggga     300

tttggcggcg gaacaaagtt aaccgtgctg ggccag                               336

<210>  135
<211>  9
<212>  PRT
<213>  Artificial Sequence

<220>
<223>  C4.4a binder

<400>  135

Phe Ser Ser Tyr Gln Met Thr Trp Ile
1               5

<210>  136
<211>  20
<212>  PRT
<213>  Artificial Sequence

<220>
<223>  C4.4a binder

<400>  136

Val Ser Gly Ile Ser Trp Asn Gly Gly Ser Thr His Tyr Ala Asp Ser
1               5                   10                  15

Val Lys Gly Arg
            20

<210>  137
<211>  15
<212>  PRT
<213>  Artificial Sequence

<220>
<223>  C4.4a binder

<400>  137

Ala Lys Gly Asp Tyr Leu Val Tyr Lys Ser Tyr Tyr Phe Lys Ser
1               5                   10                  15

<210>  138
<211>  13
<212>  PRT
<213>  Artificial Sequence

<220>
<223>  C4.4a binder

<400>  138

Ser Gly Ser Ser Ser Asn Ile Gly Ser Asn Pro Val Asn
1               5                   10

<210>  139
<211>  7
<212>  PRT
<213>  Artificial Sequence

<220>
<223>  C4.4a binder

<400>  139

Arg Asn Asn Gln Arg Pro Ser

1               5


<210>  140
<211>  12
<212>  PRT
<213>  Artificial Sequence

<220>
<223>  C4.4a binder

<400>  140

Cys Ala Ala Trp Asp Asp Ser Leu Ser Gly Trp Ala
1               5                   10


<210>  141
<211>  122
<212>  PRT
<213>  Artificial Sequence

<220>
<223>  C4.4a binder

<400>  141

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Ser Tyr
            20                  25                  30


Gln Met Thr Trp Ile Arg Gln Ala Pro Gly Lys Gly Leu Glu Trp Val
        35                  40                  45


Ser Gly Ile Ser Trp Asn Gly Gly Ser Thr His Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Lys Gly Asp Tyr Leu Val Tyr Lys Ser Tyr Tyr Phe Lys Ser Trp
            100                 105                 110


Gly Gln Gly Thr Leu Val Thr Val Ser Ser
        115                 120


<210>  142
<211>  112
<212>  PRT
<213>  Artificial Sequence

<220>
<223>  C4.4a binder

<400>  142

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Ser Leu
                85                  90                  95

Ser Gly Trp Ala Phe Gly Gly Gly Thr Lys Leu Thr Val Leu Gly Gln
            100                 105                 110

<210>  143
<211>  366
<212>  DNA
<213>  Artificial Sequence

<220>
<223>  C4.4a binder

<400>  143
gaggtgcagc tgctggaaag cggcggaggc ctggtgcagc ctggcggaag cctgagactg      60

agctgtgccg ccagcggctt caccttcagc agctaccaga tgacctggat cagacaggcc     120

cctggcaagg gcctggaatg ggtgtccggc atcagctgga acggcggcag cacccactac     180

gccgacagcg tgaagggccg gttcaccatc agccgggaca acagcaagaa caccctgtac     240

ctgcagatga acagcctgcg ggccgaggac accgccgtgt actactgcgc caagggcgac     300

tacctggtgt acaagagcta ctacttcaag agctggggcc agggcacact ggtcaccgtc     360

agctca                                                                366

<210>  144
<211>  336
<212>  DNA
<213>  Artificial Sequence

<220>
<223>  C4.4a binder

<400>  144
cagagcgtgc tgacccagcc tcctagcgcc tctggcaccc ctggccagag agtgaccatc        60

agctgcagcg gcagcagcag caacatcggc agcaaccccg tgaactggta tcagcagctg       120

cccggcaccg cccccaagct gctgatctac cggaacaacc agcggcccag cggcgtgccc       180

gacagatttt ccggcagcaa gagcggcacc agcgccagcc tggccatcag cggcctgaga       240

agcgaggacg aggccgacta ctactgcgcc gcctgggacg atagcctgag cggctgggcc       300

tttggcggcg gaacaaagtt aaccgtgctg ggccag                                 336


<210>  145
<211>  9
<212>  PRT
<213>  Artificial Sequence

<220>
<223>  C4.4a binder

<400>  145

Phe Ser Asn Ala Trp Met Ser Trp Val
1               5


<210>  146
<211>  20
<212>  PRT
<213>  Artificial Sequence

<220>
<223>  C4.4a binder

<400>  146

Val Ser Tyr Ile Ser Ser Ser Gly Ser Thr Ile Tyr Tyr Ala Asp Ser
1               5                   10                  15


Val Lys Gly Arg
            20


<210>  147
<211>  10
<212>  PRT
<213>  Artificial Sequence

<220>
<223>  C4.4a binder

<400>  147

Ala Arg Glu Gly Leu Trp Ala Phe Asp Lys
1               5                   10

<210>  148
<211>  14
<212>  PRT
<213>  Artificial Sequence

<220>
<223>  C4.4a binder

<400>  148

Thr Gly Ser Ser Ser Asn Ile Gly Ala Gly Tyr Val Val His
1               5                   10


<210>  149
<211>  7
<212>  PRT
<213>  Artificial Sequence

<220>
<223>  C4.4a binder

<400>  149

Asp Asn Asn Lys Arg Pro Ser
1               5


<210>  150
<211>  12
<212>  PRT
<213>  Artificial Sequence

<220>
<223>  C4.4a binder

<400>  150

Cys Ala Ala Tyr Asp Asp Ser Leu Lys Gly Pro Val
1               5                   10


<210>  151
<211>  117
<212>  PRT
<213>  Artificial Sequence

<220>
<223>  C4.4a binder

<400>  151

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Asn Ala
            20                  25                  30


Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Lys Trp Gly Gln Gly Thr Leu
            100                 105                 110

Val Thr Val Thr Ser
        115

<210>  152
<211>  113
<212>  PRT
<213>  Artificial Sequence

<220>
<223>  C4.4a binder

<400>  152

Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Tyr Asp Asp Ser
                85                  90                  95

Leu Lys Gly Pro Val Phe Gly Gly Gly Thr Lys Leu Thr Val Leu Gly
            100                 105                 110

Gln

<210>  153
<211>  9
<212>  PRT
<213>  Artificial Sequence

<220>
<223>  C4.4a binder

<400>  153

Phe Ser Asn Ala Trp Met Ser Trp Val
1               5


<210>  154
<211>  20
<212>  PRT
<213>  Artificial Sequence

<220>
<223>  C4.4a binder

<400>  154

Val Ser Tyr Ile Ser Ser Ser Gly Ser Thr Ile Tyr Tyr Ala Asp Ser
1               5                   10                  15


Val Lys Gly Arg
            20


<210>  155
<211>  10
<212>  PRT
<213>  Artificial Sequence

<220>
<223>  C4.4a binder

<400>  155

Ala Arg Glu Gly Leu Trp Ala Phe Asp Lys
1               5                   10


<210>  156
<211>  14
<212>  PRT
<213>  Artificial Sequence

<220>
<223>  C4.4a binder

<400>  156

Thr Gly Ser Ser Ser Asn Ile Gly Ala Gly Tyr Val Val His
1               5                   10


<210>  157
<211>  7
<212>  PRT

<213>  Artificial Sequence

<220>
<223>  C4.4a binder

<400>  157

Asp Asn Asn Lys Arg Pro Ser
1               5


<210>  158
<211>  12
<212>  PRT
<213>  Artificial Sequence

<220>
<223>  C4.4a binder

<400>  158

Cys Ala Ala Phe Asp Asp Ser Leu Asn Gly Pro Val
1               5                   10


<210>  159
<211>  117
<212>  PRT
<213>  Artificial Sequence

<220>
<223>  C4.4a binder

<400>  159

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Asn Ala
            20                  25                  30


Trp Met Ser Trp Val Arg Gln Ala Pro Gly Lys Gly Leu Glu Trp Val
        35                  40                  45


Ser Tyr Ile Ser Ser Ser Gly Ser Thr Ile Tyr Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Glu Gly Leu Trp Ala Phe Asp Lys Trp Gly Gln Gly Thr Leu
            100                 105                 110

Val Thr Val Thr Ser
        115

<210>  160
<211>  113
<212>  PRT
<213>  Artificial Sequence

<220>
<223>  C4.4a binder

<400>  160

Gln Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Phe Asp Asp Ser
                85                  90                  95

Leu Asn Gly Pro Val Phe Gly Gly Gly Thr Lys Leu Thr Val Leu Gly
            100                 105                 110

Gln

<210>  161
<211>  9
<212>  PRT
<213>  Artificial Sequence

<220>
<223>  C4.4a binder

<400>  161

Phe Ser Asn Ala Trp Met Ser Trp Val
1               5

<210>  162
<211>  20
<212>  PRT

<213>  Artificial Sequence

<220>
<223>  C4.4a binder

<400>  162

Val Ser Tyr Ile Ser Ser Ser Gly Ser Thr Ile Tyr Tyr Ala Asp Ser
1               5                   10                  15

Val Lys Gly Arg
            20

<210>  163
<211>  10
<212>  PRT
<213>  Artificial Sequence

<220>
<223>  C4.4a binder

<400>  163

Ala Arg Glu Gly Leu Trp Ala Phe Asp Lys
1               5                   10

<210>  164
<211>  14
<212>  PRT
<213>  Artificial Sequence

<220>
<223>  C4.4a binder

<400>  164

Thr Gly Ser Ser Ser Asn Ile Gly Ala Gly Tyr Val Val His
1               5                   10

<210>  165
<211>  7
<212>  PRT
<213>  Artificial Sequence

<220>
<223>  C4.4a binder

<400>  165

Asp Asn Asn Lys Arg Pro Ser
1               5

<210>  166
<211>  12
<212>  PRT
<213>  Artificial Sequence

<220>

<223>  C4.4a binder

<400>  166

Cys Ala Ala Tyr Asp Asp Ser Leu Ser Gly Pro Val
1               5                   10


<210>  167
<211>  117
<212>  PRT
<213>  Artificial Sequence

<220>
<223>  C4.4a binder

<400>  167

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Asn Ala
            20                  25                  30


Trp Met Ser Trp Val Arg Gln Ala Pro Gly Lys Gly Leu Glu Trp Val
        35                  40                  45


Ser Tyr Ile Ser Ser Ser Gly Ser Thr Ile Tyr Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Glu Gly Leu Trp Ala Phe Asp Lys Trp Gly Gln Gly Thr Leu
            100                 105                 110


Val Thr Val Thr Ser
        115


<210>  168
<211>  113
<212>  PRT
<213>  Artificial Sequence

<220>
<223>  C4.4a binder

<400>  168

Gln Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Phe Asp Asp Ser
                85                  90                  95

Leu Asn Gly Pro Val Phe Gly Gly Gly Thr Lys Leu Thr Val Leu Gly
            100                 105                 110

Gln

<210>  169
<211>  9
<212>  PRT
<213>  Artificial Sequence

<220>
<223>  C4.4a binder

<400>  169

Phe Ser Ser Ala Trp Met Ser Trp Val
1               5

<210>  170
<211>  20
<212>  PRT
<213>  Artificial Sequence

<220>
<223>  C4.4a binder

<400>  170

Val Ser Tyr Ile Ser Ser Ser Gly Ser Ser Thr Tyr Tyr Ala Asp Ser
1               5                   10                  15

Val Lys Gly Arg
            20

<210>  171
<211>  10

<212>  PRT
<213>  Artificial Sequence

<220>
<223>  C4.4a binder

<400>  171

Ala Arg Glu Gly Leu Trp Ala Phe Asp Trp
1               5                   10


<210>  172
<211>  14
<212>  PRT
<213>  Artificial Sequence

<220>
<223>  C4.4a binder

<400>  172

Ser Gly Ser Ser Ser Asn Ile Gly Ala Gly Tyr Val Val His
1               5                   10


<210>  173
<211>  7
<212>  PRT
<213>  Artificial Sequence

<220>
<223>  C4.4a binder

<400>  173

Asp Asn Asn Gln Arg Pro Ser
1               5


<210>  174
<211>  12
<212>  PRT
<213>  Artificial Sequence

<220>
<223>  C4.4a binder

<400>  174

Cys Ala Ala Tyr Asp Asp Ser Leu Ser Gly Pro Val
1               5                   10


<210>  175
<211>  117
<212>  PRT
<213>  Artificial Sequence

<220>
<223>  C4.4a binder

<400>  175

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Trp Trp Gly Gln Gly Thr Leu
            100                 105                 110

Val Thr Val Thr Ser
        115

<210>  176
<211>  113
<212>  PRT
<213>  Artificial Sequence

<220>
<223>  C4.4a binder

<400>  176

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Tyr Asp Asp Ser
                85                  90                  95

Leu Ser Gly Pro Val Phe Gly Gly Gly Thr Lys Leu Thr Val Leu Gly
            100                 105                 110

Gln

<210>  177
<211>  9
<212>  PRT
<213>  Artificial Sequence

<220>
<223>  C4.4a binder

<400>  177

Phe Ser Ser Ala Trp Met Ser Trp Val
1               5

<210>  178
<211>  20
<212>  PRT
<213>  Artificial Sequence

<220>
<223>  C4.4a binder

<400>  178

Val Ser Tyr Ile Ser Ser Ser Gly Ser Ser Thr Tyr Tyr Ala Asp Ser
1               5                   10                  15

Val Lys Gly Arg
            20

<210>  179
<211>  10
<212>  PRT
<213>  Artificial Sequence

<220>
<223>  C4.4a binder

<400>  179

Ala Arg Glu Gly Leu Trp Ala Phe Asp Asn
1               5                   10

<210>  180
<211>  14
<212>  PRT
<213>  Artificial Sequence

<220>
<223>  C4.4a binder

<400>  180

Ser Gly Ser Ser Ser Asn Ile Gly Ala Gly Tyr Val Val His
1               5                   10


<210>  181
<211>  7
<212>  PRT
<213>  Artificial Sequence

<220>
<223>  C4.4a binder

<400>  181

Asp Asn Asn Gln Arg Pro Ser
1               5


<210>  182
<211>  12
<212>  PRT
<213>  Artificial Sequence

<220>
<223>  C4.4a binder

<400>  182

Cys Ala Ala Tyr Asp Asp Ser Leu Asn Gly Pro Val
1               5                   10


<210>  183
<211>  117
<212>  PRT
<213>  Artificial Sequence

<220>
<223>  C4.4a binder

<400>  183

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Asn Ala
            20                  25                  30


Trp Met Ser Trp Val Arg Gln Ala Pro Gly Lys Gly Leu Glu Trp Val
        35                  40                  45


Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
            100                 105                 110

Val Thr Val Thr Ser
        115

<210>  184
<211>  113
<212>  PRT
<213>  Artificial Sequence

<220>
<223>  C4.4a binder

<400>  184

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Tyr Asp Asp Ser
                85                  90                  95

Leu Asn Gly Pro Val Phe Gly Gly Gly Thr Lys Leu Thr Val Leu Gly
            100                 105                 110

Gln

<210>  185
<211>  9
<212>  PRT
<213>  Artificial Sequence

<220>
<223>  C4.4a binder

<400>  185

Phe Ser Ser Ala Trp Met Ser Trp Val
1               5


<210>  186
<211>  20
<212>  PRT
<213>  Artificial Sequence

<220>
<223>  C4.4a binder

<400>  186

Val Ser Tyr Ile Ser Ser Ser Gly Ser Ser Thr Tyr Tyr Ala Asp Ser
1               5                   10                  15


Val Lys Gly Arg
            20


<210>  187
<211>  10
<212>  PRT
<213>  Artificial Sequence

<220>
<223>  C4.4a binder

<400>  187

Ala Arg Glu Gly Leu Trp Ala Phe Asp Tyr
1               5                   10


<210>  188
<211>  14
<212>  PRT
<213>  Artificial Sequence

<220>
<223>  C4.4a binder

<400>  188

Ser Gly Ser Ser Ser Asn Ile Gly Ala Gly Tyr Val Val His
1               5                   10


<210>  189
<211>  7
<212>  PRT
<213>  Artificial Sequence

<220>
<223>  C4.4a binder

<400>  189

Asp Asn Asn Gln Arg Pro Ser
1               5


<210>  190
<211>  12
<212>  PRT
<213>  Artificial Sequence

<220>
<223>  C4.4a binder

<400>  190

Cys Ala Ala Trp Asp Asp Arg Leu Asn Gly Pro Val
1               5                   10


<210>  191
<211>  117
<212>  PRT
<213>  Artificial Sequence

<220>
<223>  C4.4a binder

<400>  191

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Ser Ala
            20                  25                  30


Trp Met Ser Trp Val Arg Gln Ala Pro Gly Lys Gly Leu Glu Trp Val
        35                  40                  45


Ser Tyr Ile Ser Ser Ser Gly Ser Ser Thr Tyr Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Glu Gly Leu Trp Ala Phe Asp Tyr Trp Gly Gln Gly Thr Leu
            100                 105                 110


Val Thr Val Ser Ser
        115


<210>  192

<211>  113
<212>  PRT
<213>  Artificial Sequence

<220>
<223>  C4.4a binder

<400>  192

Glu Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Trp Asp Asp Arg
                85                  90                  95

Leu Asn Gly Pro Val Phe Gly Gly Gly Thr Lys Leu Thr Val Leu Gly
            100                 105                 110

Gln

<210>  193
<211>  11
<212>  PRT
<213>  Artificial Sequence

<220>
<223>  C4.4a binder

<400>  193

Phe Thr Phe Ser Asn Ala Trp Met Ser Trp Val
1               5                   10

<210>  194
<211>  20
<212>  PRT
<213>  Artificial Sequence

<220>
<223>  C4.4a binder

<400>  194

Val Ser Tyr Ile Ser Ser Ser Gly Ser Thr Ile Tyr Tyr Ala Asp Ser
1               5                   10                  15

Val Lys Gly Arg
            20

<210>  195
<211>  10
<212>  PRT
<213>  Artificial Sequence

<220>
<223>  C4.4a binder

<400>  195

Ala Arg Glu Gly Leu Trp Ala Phe Asp Tyr
1               5                   10

<210>  196
<211>  14
<212>  PRT
<213>  Artificial Sequence

<220>
<223>  C4.4a binder

<400>  196

Thr Gly Ser Ser Ser Asn Ile Gly Ala Gly Tyr Val Val His
1               5                   10

<210>  197
<211>  7
<212>  PRT
<213>  Artificial Sequence

<220>
<223>  C4.4a binder

<400>  197

Asp Asn Asn Lys Arg Pro Ser
1               5

<210>  198
<211>  12
<212>  PRT
<213>  Artificial Sequence

<220>
<223>  C4.4a binder

<400>  198

Cys Ala Ala Tyr Asp Asp Arg Leu Asn Gly Pro Val

1               5                   10

<210>  199
<211>  117
<212>  PRT
<213>  Artificial Sequence

<220>
<223>  C4.4a binder

<400>  199

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
            100                 105                 110

Val Thr Val Ser Ser
        115

<210>  200
<211>  113
<212>  PRT
<213>  Artificial Sequence

<220>
<223>  C4.4a binder

<400>  200

Glu Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Tyr Asp Asp Arg
                85                  90                  95

Leu Asn Gly Pro Val Phe Gly Gly Gly Thr Lys Leu Thr Val Leu Gly
            100                 105                 110

Gln

<210>  201
<211>  11
<212>  PRT
<213>  Artificial Sequence

<220>
<223>  C4.4a binder

<400>  201

Phe Thr Phe Ser Ser Ala Trp Met Ser Trp Val
1               5                   10

<210>  202
<211>  20
<212>  PRT
<213>  Artificial Sequence

<220>
<223>  C4.4a binder

<400>  202

Val Ser Tyr Ile Ser Ser Ser Gly Ser Ser Thr Tyr Tyr Ala Asp Ser
1               5                   10                  15

Val Lys Gly Arg
            20

<210>  203
<211>  10
<212>  PRT
<213>  Artificial Sequence

<220>
<223>  C4.4a binder

<400>  203

Ala Arg Glu Gly Leu Trp Ala Phe Asp Gly
1               5                   10


<210>  204
<211>  14
<212>  PRT
<213>  Artificial Sequence

<220>
<223>  C4.4a binder

<400>  204

Ser Gly Ser Ser Ser Asn Ile Gly Ala Gly Tyr Val Val His
1               5                   10


<210>  205
<211>  7
<212>  PRT
<213>  Artificial Sequence

<220>
<223>  C4.4a binder

<400>  205

Asp Asn Asn Gln Arg Pro Ser
1               5


<210>  206
<211>  12
<212>  PRT
<213>  Artificial Sequence

<220>
<223>  C4.4a binder

<400>  206

Cys Ala Ala Tyr Asp Asp Ser Leu Asn Arg Pro Val
1               5                   10


<210>  207
<211>  117
<212>  PRT
<213>  Artificial Sequence

<220>
<223>  C4.4a binder

<400>  207

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Gly Trp Gly Gln Gly Thr Leu
            100                 105                 110

Val Thr Val Ser Ser
        115

<210>  208
<211>  113
<212>  PRT
<213>  Artificial Sequence

<220>
<223>  C4.4a binder

<400>  208

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Tyr Asp Asp Ser
                85                  90                  95

Leu Asn Arg Pro Val Phe Gly Gly Gly Thr Lys Leu Thr Val Leu Gly

            100                 105                 110

Gln

<210> 209
<211> 11
<212> PRT
<213> Artificial Sequence

<220>
<223> C4.4a binder

<400> 209

Phe Thr Phe Ser Asn Ala Trp Met Ser Trp Val
1               5                   10

<210> 210
<211> 20
<212> PRT
<213> Artificial Sequence

<220>
<223> C4.4a binder

<400> 210

Val Ser Tyr Ile Ser Ser Ser Gly Ser Thr Ile Tyr Tyr Ala Asp Ser
1               5                   10                  15

Val Lys Gly Arg
            20

<210> 211
<211> 10
<212> PRT
<213> Artificial Sequence

<220>
<223> C4.4a binder

<400> 211

Ala Arg Glu Gly Leu Trp Ala Phe Asp Tyr
1               5                   10

<210> 212
<211> 14
<212> PRT
<213> Artificial Sequence

<220>
<223> C4.4a binder

<400> 212

Thr Gly Ser Ser Ser Asn Ile Gly Ala Gly Tyr Val Val His
1               5                   10

<210>  213
<211>  7
<212>  PRT
<213>  Artificial Sequence

<220>
<223>  C4.4a binder

<400>  213

Asp Asn Asn Lys Arg Pro Ser
1               5

<210>  214
<211>  12
<212>  PRT
<213>  Artificial Sequence

<220>
<223>  C4.4a binder

<400>  214

Cys Ala Ala Phe Asp Asp Ser Leu Asn Gly Pro Val
1               5                   10

<210>  215
<211>  117
<212>  PRT
<213>  Artificial Sequence

<220>
<223>  C4.4a binder

<400>  215

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys

85                  90                  95

Ala Arg Glu Gly Leu Trp Ala Phe Asp Tyr Trp Gly Gln Gly Thr Leu
            100                 105                 110

Val Thr Val Ser Ser
        115

<210>  216
<211>  113
<212>  PRT
<213>  Artificial Sequence

<220>
<223>  C4.4a binder

<400>  216

Gln Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Phe Asp Asp Ser
                85                  90                  95

Leu Asn Gly Pro Val Phe Gly Gly Gly Thr Lys Leu Thr Val Leu Gly
            100                 105                 110

Gln

<210>  217
<211>  9
<212>  PRT
<213>  Artificial Sequence

<220>
<223>  C4.4a binder

<400>  217

Phe Ser Ser Ala Trp Met Ser Trp Val
1               5

<210>  218
<211>  20
<212>  PRT
<213>  Artificial Sequence

<220>
<223>  C4.4a binder

<400>  218

Val Ser Tyr Ile Ser Ser Ser Gly Ser Ser Thr Tyr Tyr Ala Asp Ser
1               5                   10                  15

Val Lys Gly Arg
            20

<210>  219
<211>  10
<212>  PRT
<213>  Artificial Sequence

<220>
<223>  C4.4a binder

<400>  219

Ala Arg Glu Gly Leu Trp Ala Phe Asp Lys
1               5                   10

<210>  220
<211>  14
<212>  PRT
<213>  Artificial Sequence

<220>
<223>  C4.4a binder

<400>  220

Thr Gly Ser Ser Ser Asn Ile Gly Ala Gly Tyr Val Val His
1               5                   10

<210>  221
<211>  7
<212>  PRT
<213>  Artificial Sequence

<220>
<223>  C4.4a binder

<400>  221

Asp Asn Asn Lys Arg Pro Ser
1               5

<210>  222
<211>  12
<212>  PRT
<213>  Artificial Sequence

<220>
<223>  C4.4a binder

<400>  222

Cys Ala Ala Phe Asp Asp Ser Leu Asn Gly Pro Val
1               5                   10


<210>  223
<211>  117
<212>  PRT
<213>  Artificial Sequence

<220>
<223>  C4.4a binder

<400>  223

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Ser Ala
            20                  25                  30


Trp Met Ser Trp Val Arg Gln Ala Pro Gly Lys Gly Leu Glu Trp Val
        35                  40                  45


Ser Tyr Ile Ser Ser Ser Gly Ser Ser Thr Tyr Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Glu Gly Leu Trp Ala Phe Asp Lys Trp Gly Gln Gly Thr Leu
            100                 105                 110


Val Thr Val Thr Ser
        115


<210>  224
<211>  113
<212>  PRT
<213>  Artificial Sequence

<220>

<223>  C4.4a binder

<400>  224

Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Phe Asp Asp Ser
                85                  90                  95

Leu Asn Gly Pro Val Phe Gly Gly Gly Thr Lys Leu Thr Val Leu Gly
            100                 105                 110

Gln

<210>  225
<211>  11
<212>  PRT
<213>  Artificial Sequence

<220>
<223>  C4.4a binder

<400>  225

Phe Thr Phe Ser Ser Ala Trp Met Ser Trp Val
1               5                   10

<210>  226
<211>  20
<212>  PRT
<213>  Artificial Sequence

<220>
<223>  C4.4a binder

<400>  226

Val Ser Tyr Ile Ser Ser Ser Gly Ser Ser Thr Tyr Tyr Ala Asp Ser
1               5                   10                  15

Val Lys Gly Arg
            20

<210>  227
<211>  10
<212>  PRT
<213>  Artificial Sequence

<220>
<223>  C4.4a binder

<400>  227

Ala Arg Glu Gly Leu Trp Ala Phe Asp Lys
1               5                   10

<210>  228
<211>  14
<212>  PRT
<213>  Artificial Sequence

<220>
<223>  C4.4a binder

<400>  228

Ser Gly Ser Ser Ser Asn Ile Gly Ala Gly Tyr Val Val His
1               5                   10

<210>  229
<211>  7
<212>  PRT
<213>  Artificial Sequence

<220>
<223>  C4.4a binder

<400>  229

Asp Asn Asn Gln Arg Pro Ser
1               5

<210>  230
<211>  12
<212>  PRT
<213>  Artificial Sequence

<220>
<223>  C4.4a binder

<400>  230

Cys Ala Ala Phe Asp Asp Ser Leu Asn Gly Pro Val
1               5                   10

<210>  231
<211>  117

<212>  PRT
<213>  Artificial Sequence

<220>
<223>  C4.4a binder

<400>  231

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Lys Trp Gly Gln Gly Thr Leu
            100                 105                 110

Val Thr Val Ser Ser
        115


<210>  232
<211>  113
<212>  PRT
<213>  Artificial Sequence

<220>
<223>  C4.4a binder

<400>  232

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Gln Arg Pro Ser Gly Val Pro Asp Arg Phe

50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Phe Asp Asp Ser
                85                  90                  95

Leu Asn Gly Pro Val Phe Gly Gly Gly Thr Lys Leu Thr Val Leu Gly
            100                 105                 110

Gln

<210>  233
<211>  11
<212>  PRT
<213>  Artificial Sequence

<220>
<223>  C4.4a binder

<400>  233

Phe Thr Phe Ser Ser Ala Trp Met Ser Trp Val
1               5                   10

<210>  234
<211>  20
<212>  PRT
<213>  Artificial Sequence

<220>
<223>  C4.4a binder

<400>  234

Val Ser Tyr Ile Ser Ser Ser Gly Ser Ser Thr Tyr Tyr Ala Asp Ser
1               5                   10                  15

Val Lys Gly Arg
            20

<210>  235
<211>  10
<212>  PRT
<213>  Artificial Sequence

<220>
<223>  C4.4a binder

<400>  235

Ala Arg Glu Gly Leu Trp Ala Phe Asp Lys
1               5                   10

<210>  236
<211>  14
<212>  PRT
<213>  Artificial Sequence

<220>
<223>  C4.4a binder

<400>  236

Ser Gly Ser Ser Ser Asn Ile Gly Ala Gly Tyr Val Val His
1               5                   10


<210>  237
<211>  7
<212>  PRT
<213>  Artificial Sequence

<220>
<223>  C4.4a binder

<400>  237

Asp Asn Asn Gln Arg Pro Ser
1               5


<210>  238
<211>  12
<212>  PRT
<213>  Artificial Sequence

<220>
<223>  C4.4a binder

<400>  238

Cys Ala Ala Phe Asp Asp Arg Leu Ser Gly Pro Val
1               5                   10


<210>  239
<211>  117
<212>  PRT
<213>  Artificial Sequence

<220>
<223>  C4.4a binder

<400>  239

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Ser Ala
            20                  25                  30


Trp Met Ser Trp Val Arg Gln Ala Pro Gly Lys Gly Leu Glu Trp Val

35                  40                  45

Ser Tyr Ile Ser Ser Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Lys Trp Gly Gln Gly Thr Leu
            100                 105                 110

Val Thr Val Ser Ser
        115

<210>  240
<211>  113
<212>  PRT
<213>  Artificial Sequence

<220>
<223>  C4.4a binder

<400>  240

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Phe Asp Asp Arg
                85                  90                  95

Leu Ser Gly Pro Val Phe Gly Gly Gly Thr Lys Leu Thr Val Leu Gly
            100                 105                 110

Gln

<210>  241
<211>  9
<212>  PRT
<213>  Artificial Sequence

<220>
<223>  C4.4a binder

<400>  241

Phe Ser Asp Tyr Gln Met Thr Trp Ile
1               5


<210>  242
<211>  20
<212>  PRT
<213>  Artificial Sequence

<220>
<223>  C4.4a binder

<400>  242

Val Ser Gly Val Ser Trp Asn Gly Ala Arg Thr His Tyr Ala Asp Ser
1               5                   10                  15


Val Lys Gly Arg
            20


<210>  243
<211>  15
<212>  PRT
<213>  Artificial Sequence

<220>
<223>  C4.4a binder

<400>  243

Ala Lys Gly Asp Tyr Leu Val Tyr Ser Ser Tyr Tyr Phe Lys Ser
1               5                   10                  15


<210>  244
<211>  13
<212>  PRT
<213>  Artificial Sequence

<220>
<223>  C4.4a binder

<400>  244

Ser Gly Ser Ser Ser Asn Val Gly Ser Asn Pro Val Asn
1               5                   10


<210>  245

<211>  7
<212>  PRT
<213>  Artificial Sequence

<220>
<223>  C4.4a binder

<400>  245

Arg Asn Asn Gln Arg Pro Ser
1               5


<210>  246
<211>  13
<212>  PRT
<213>  Artificial Sequence

<220>
<223>  C4.4a binder

<400>  246

Cys Ala Ala Trp Asp Asp Arg Leu Asn Gly Trp Thr Gly
1               5                   10


<210>  247
<211>  122
<212>  PRT
<213>  Artificial Sequence

<220>
<223>  C4.4a binder

<400>  247

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Asp Tyr
            20                  25                  30


Gln Met Thr Trp Ile Arg Gln Thr Pro Gly Lys Gly Leu Glu Trp Val
        35                  40                  45


Ser Gly Val Ser Trp Asn Gly Ala Arg Thr His Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Lys Gly Asp Tyr Leu Val Tyr Ser Ser Tyr Tyr Phe Lys Ser Trp
            100                 105                 110

Gly Gln Gly Thr Leu Val Thr Val Thr Ser
        115                 120

<210>  248
<211>  112
<212>  PRT
<213>  Artificial Sequence

<220>
<223>  C4.4a binder

<400>  248

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Val Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Asn Gly Trp Gly Phe Gly Gly Gly Thr Lys Leu Thr Val Leu Gly Gln
            100                 105                 110

<210>  249
<211>  11
<212>  PRT
<213>  Artificial Sequence

<220>
<223>  C4.4a binder

<400>  249

Phe Thr Phe Ser Ser Tyr Gln Met Thr Trp Ile
1               5                   10

<210>  250
<211>  20
<212>  PRT
<213>  Artificial Sequence

<220>
<223>  C4.4a binder

<400>  250

Val Ser Gly Ile Ser Trp Asn Gly Gly Ser Thr His Tyr Ala Asp Ser
1               5                   10                  15

Val Lys Gly Arg
            20

<210>  251
<211>  15
<212>  PRT
<213>  Artificial Sequence

<220>
<223>  C4.4a binder

<400>  251

Ala Lys Gly Asp Tyr Leu Val Tyr Ser Ala Tyr Tyr Phe Asp Ser
1               5                   10                  15

<210>  252
<211>  13
<212>  PRT
<213>  Artificial Sequence

<220>
<223>  C4.4a binder

<400>  252

Ser Gly Ser Ser Ser Asn Ile Gly Ser Asn Pro Val Asn
1               5                   10

<210>  253
<211>  7
<212>  PRT
<213>  Artificial Sequence

<220>
<223>  C4.4a binder

<400>  253

Arg Asn Asn Gln Arg Pro Ser
1               5

<210>  254
<211>  12
<212>  PRT
<213>  Artificial Sequence

<220>
<223>  C4.4a binder

<400>  254

Cys Ala Ala Trp Asp Asp Arg Leu Asn Gly Trp Gly
1               5                   10

<210>  255
<211>  122
<212>  PRT
<213>  Artificial Sequence

<220>
<223>  C4.4a binder

<400>  255

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ala Tyr Tyr Phe Asp Ser Trp
            100                 105                 110

Gly Gln Gly Thr Leu Val Thr Val Ser Ser
        115                 120

<210>  256
<211>  112
<212>  PRT
<213>  Artificial Sequence

<220>
<223>  C4.4a binder

<400>  256

Glu Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn

20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Asn Gly Trp Gly Phe Gly Gly Gly Thr Lys Leu Thr Val Leu Gly Gln
            100                 105                 110

<210>  257
<211>  11
<212>  PRT
<213>  Artificial Sequence

<220>
<223>  C4.4a binder

<400>  257

Phe Thr Phe Ser Asp Tyr Gln Met Thr Trp Ile
1               5                   10

<210>  258
<211>  20
<212>  PRT
<213>  Artificial Sequence

<220>
<223>  C4.4a binder

<400>  258

Val Ser Gly Ile Ser Trp Asn Gly Gly Ser Thr His Tyr Ala Asp Ser
1               5                   10                  15

Val Lys Gly Arg
            20

<210>  259
<211>  15
<212>  PRT
<213>  Artificial Sequence

<220>
<223>  C4.4a binder

<400>  259

Ala Lys Gly Asp Tyr Leu Val Tyr Ser Ser Tyr Tyr Phe Lys Tyr
1               5                   10                  15


<210>  260
<211>  13
<212>  PRT
<213>  Artificial Sequence

<220>
<223>  C4.4a binder

<400>  260

Ser Gly Ser Ser Ser Asn Ile Gly Ser Asn Pro Val Asn
1               5                   10


<210>  261
<211>  7
<212>  PRT
<213>  Artificial Sequence

<220>
<223>  C4.4a binder

<400>  261

Arg Asn Asn Gln Arg Pro Ser
1               5


<210>  262
<211>  12
<212>  PRT
<213>  Artificial Sequence

<220>
<223>  C4.4a binder

<400>  262

Cys Ala Ala Trp Asp Asp Arg Leu Asn Gly Trp Ala
1               5                   10


<210>  263
<211>  122
<212>  PRT
<213>  Artificial Sequence

<220>
<223>  C4.4a binder

<400>  263

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Asp Tyr

20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ser Tyr Tyr Phe Lys Tyr Trp
            100                 105                 110

Gly Gln Gly Thr Leu Val Thr Val Ser Ser
        115                 120

<210>  264
<211>  112
<212>  PRT
<213>  Artificial Sequence

<220>
<223>  C4.4a binder

<400>  264

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Asn Gly Trp Ala Phe Gly Gly Gly Thr Lys Leu Thr Val Leu Gly Gln
            100                 105                 110

<210>  265
<211>  11
<212>  PRT
<213>  Artificial Sequence

<220>
<223>  C4.4a binder

<400>  265

Phe Thr Phe Ser Asp Tyr Gln Met Thr Trp Ile
1               5                   10


<210>  266
<211>  20
<212>  PRT
<213>  Artificial Sequence

<220>
<223>  C4.4a binder

<400>  266

Val Ser Gly Ile Ser Trp Asn Gly Gly Ser Thr His Tyr Ala Asp Ser
1               5                   10                  15


Val Lys Gly Arg
            20


<210>  267
<211>  15
<212>  PRT
<213>  Artificial Sequence

<220>
<223>  C4.4a binder

<400>  267

Ala Lys Gly Asp Tyr Leu Val Tyr Ser Ser Tyr Tyr Phe Lys Ser
1               5                   10                  15


<210>  268
<211>  13
<212>  PRT
<213>  Artificial Sequence

<220>
<223>  C4.4a binder

<400>  268

Ser Gly Ser Ser Ser Asn Ile Gly Ser Asn Pro Val Asn
1               5                   10


<210>  269

<211>  7
<212>  PRT
<213>  Artificial Sequence

<220>
<223>  C4.4a binder

<400>  269

Arg Asn Asn Gln Arg Pro Ser
1               5


<210>  270
<211>  12
<212>  PRT
<213>  Artificial Sequence

<220>
<223>  C4.4a binder

<400>  270

Cys Ala Ala Trp Asp Asp Ser Leu Asn Gly Trp Gly
1               5                   10


<210>  271
<211>  122
<212>  PRT
<213>  Artificial Sequence

<220>
<223>  C4.4a binder

<400>  271

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Asp Tyr
            20                  25                  30


Gln Met Thr Trp Ile Arg Gln Thr Pro Gly Lys Gly Leu Glu Trp Val
        35                  40                  45


Ser Gly Ile Ser Trp Asn Gly Gly Ser Thr His Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Lys Gly Asp Tyr Leu Val Tyr Ser Ser Tyr Tyr Phe Lys Ser Trp
            100                 105                 110

Gly Gln Gly Thr Leu Val Thr Val Ser Ser
        115                 120

<210>  272
<211>  112
<212>  PRT
<213>  Artificial Sequence

<220>
<223>  C4.4a binder

<400>  272

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Ser Leu
                85                  90                  95

Asn Gly Trp Gly Phe Gly Gly Gly Thr Lys Leu Thr Val Leu Gly Gln
            100                 105                 110

<210>  273
<211>  11
<212>  PRT
<213>  Artificial Sequence

<220>
<223>  C4.4a binder

<400>  273

Phe Thr Phe Ser Asp Tyr Gln Met Thr Trp Ile
1               5                   10

<210>  274
<211>  20
<212>  PRT
<213>  Artificial Sequence

<220>
<223>  C4.4a binder

<400>  274

Val Ser Gly Ile Ser Trp Asn Gly Gly Ser Thr His Tyr Ala Asp Ser
1               5                   10                  15

Val Lys Gly Arg
            20

<210>  275
<211>  15
<212>  PRT
<213>  Artificial Sequence

<220>
<223>  C4.4a binder

<400>  275

Ala Lys Gly Asp Tyr Leu Val Tyr Lys Ser Tyr Tyr Phe Lys Ser
1               5                   10                  15

<210>  276
<211>  13
<212>  PRT
<213>  Artificial Sequence

<220>
<223>  C4.4a binder

<400>  276

Ser Gly Ser Ser Ser Asn Ile Gly Ser Asn Pro Val Asn
1               5                   10

<210>  277
<211>  7
<212>  PRT
<213>  Artificial Sequence

<220>
<223>  C4.4a binder

<400>  277

Arg Asn Asn Gln Arg Pro Ser
1               5

<210>  278
<211>  12
<212>  PRT
<213>  Artificial Sequence

<220>
<223>  C4.4a binder

<400>  278

Cys Ala Ala Trp Asp Asp Arg Leu Ser Gly Trp Gly
1               5                   10

<210>  279
<211>  122
<212>  PRT
<213>  Artificial Sequence

<220>
<223>  C4.4a binder

<400>  279

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Lys Ser Tyr Tyr Phe Lys Ser Trp
            100                 105                 110

Gly Gln Gly Thr Leu Val Thr Val Ser Ser
        115                 120

<210>  280
<211>  112
<212>  PRT
<213>  Artificial Sequence

<220>
<223>  C4.4a binder

<400>  280

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn

20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Ser Gly Trp Gly Phe Gly Gly Gly Thr Lys Leu Thr Val Leu Gly Gln
            100                 105                 110

<210>  281
<211>  11
<212>  PRT
<213>  Artificial Sequence

<220>
<223>  C4.4a binder

<400>  281

Phe Thr Phe Ser Asp Tyr Gln Met Thr Trp Ile
1               5                   10

<210>  282
<211>  20
<212>  PRT
<213>  Artificial Sequence

<220>
<223>  C4.4a binder

<400>  282

Val Ser Gly Ile Ser Trp Asn Gly Gly Ser Thr His Tyr Ala Asp Ser
1               5                   10                  15

Val Lys Gly Arg
            20

<210>  283
<211>  15
<212>  PRT
<213>  Artificial Sequence

<220>
<223>  C4.4a binder

<400>  283

Ala Lys Gly Asp Tyr Leu Val Tyr Ser Ser Tyr Tyr Phe Lys Ser
1               5                   10                  15


<210>  284
<211>  13
<212>  PRT
<213>  Artificial Sequence

<220>
<223>  C4.4a binder

<400>  284

Ser Gly Ser Ser Ser Asn Ile Gly Ser Asn Pro Val Asn
1               5                   10


<210>  285
<211>  7
<212>  PRT
<213>  Artificial Sequence

<220>
<223>  C4.4a binder

<400>  285

Arg Asn Asn Gln Arg Pro Ser
1               5


<210>  286
<211>  12
<212>  PRT
<213>  Artificial Sequence

<220>
<223>  C4.4a binder

<400>  286

Cys Ala Ala Trp Asp Asp Arg Leu Ser Gly Trp Gly
1               5                   10


<210>  287
<211>  122
<212>  PRT
<213>  Artificial Sequence

<220>
<223>  C4.4a binder

<400>  287

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Asp Tyr

20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ser Tyr Tyr Phe Lys Ser Trp
            100                 105                 110

Gly Gln Gly Thr Leu Val Thr Val Ser Ser
        115                 120

<210>  288
<211>  112
<212>  PRT
<213>  Artificial Sequence

<220>
<223>  C4.4a binder

<400>  288

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Ser Gly Trp Gly Phe Gly Gly Gly Thr Lys Leu Thr Val Leu Gly Gln
            100                 105                 110

<210>  289
<211>  11
<212>  PRT
<213>  Artificial Sequence

<220>
<223>  C4.4a binder

<400>  289

Phe Thr Phe Ser Asp Tyr Gln Met Thr Trp Ile
1               5                   10


<210>  290
<211>  20
<212>  PRT
<213>  Artificial Sequence

<220>
<223>  C4.4a binder

<400>  290

Val Ser Gly Ile Ser Trp Asn Gly Gly Ser Thr His Tyr Ala Asp Ser
1               5                   10                  15


Val Lys Gly Arg
            20


<210>  291
<211>  15
<212>  PRT
<213>  Artificial Sequence

<220>
<223>  C4.4a binder

<400>  291

Ala Lys Gly Asp Tyr Leu Val Tyr Lys Ser Tyr Tyr Phe Lys Ser
1               5                   10                  15


<210>  292
<211>  13
<212>  PRT
<213>  Artificial Sequence

<220>
<223>  C4.4a binder

<400>  292

Ser Gly Ser Ser Ser Asn Ile Gly Ser Asn Pro Val Asn
1               5                   10


<210>  293

<211>  7
<212>  PRT
<213>  Artificial Sequence

<220>
<223>  C4.4a binder

<400>  293

Arg Asn Asn Gln Arg Pro Ser
1               5


<210>  294
<211>  12
<212>  PRT
<213>  Artificial Sequence

<220>
<223>  C4.4a binder

<400>  294

Cys Ala Ala Trp Asp Asp Ser Leu Asn Gly Trp Gly
1               5                   10


<210>  295
<211>  122
<212>  PRT
<213>  Artificial Sequence

<220>
<223>  C4.4a binder

<400>  295

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Asp Tyr
            20                  25                  30


Gln Met Thr Trp Ile Arg Gln Thr Pro Gly Lys Gly Leu Glu Trp Val
        35                  40                  45


Ser Gly Ile Ser Trp Asn Gly Gly Ser Thr His Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Lys Gly Asp Tyr Leu Val Tyr Lys Ser Tyr Tyr Phe Lys Ser Trp
            100                 105                 110

Gly Gln Gly Thr Leu Val Thr Val Ser Ser
        115                 120

<210>  296
<211>  112
<212>  PRT
<213>  Artificial Sequence

<220>
<223>  C4.4a binder

<400>  296

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Ser Leu
                85                  90                  95

Asn Gly Trp Gly Phe Gly Gly Gly Thr Lys Leu Thr Val Leu Gly Gln
            100                 105                 110

<210>  297
<211>  9
<212>  PRT
<213>  Artificial Sequence

<220>
<223>  consensus sequence for M20 D02 S-A derived CDR H1

<220>
<221>  VARIANT
<222>  (3)..(3)
<223>  X = D or S

<400>  297

Phe Ser Xaa Tyr Gln Met Thr Trp Ile
1               5

<210>  298
<211>  20
<212>  PRT
<213>  Artificial Sequence

<220>
<223>  consensus sequence for M20 D02 S-A derived CDRH2


<220>
<221>  VARIANT
<222>  (4)..(4)
<223>  X = V or I

<220>
<221>  VARIANT
<222>  (9)..(9)
<223>  X = A or G

<220>
<221>  VARIANT
<222>  (10)..(10)
<223>  X = R or S

<400>  298

Val Ser Gly Xaa Ser Trp Asn Gly Xaa Xaa Thr His Tyr Ala Asp Ser
1               5                   10                  15


Val Lys Gly Arg
            20


<210>  299
<211>  15
<212>  PRT
<213>  Artificial Sequence

<220>
<223>  consensus for M20 D02 S-A derived CDRH3


<220>
<221>  VARIANT
<222>  (9)..(9)
<223>  X = S, K, or R

<220>
<221>  VARIANT
<222>  (10)..(10)
<223>  X =  A, S or R

<220>
<221>  VARIANT
<222>  (14)..(14)
<223>  X =  D, K, E or R

<220>
<221>  VARIANT
<222>  (15)..(15)
<223>  X =  S or Y

<400>  299

Ala Lys Gly Asp Tyr Leu Val Tyr Xaa Xaa Tyr Tyr Phe Xaa Xaa
1               5                   10                  15


<210>  300
<211>  13
<212>  PRT
<213>  Artificial Sequence

<220>
<223>  consensus seqeunce derived from M20 D02 S-A CDRL1


<220>
<221>  VARIANT
<222>  (7)..(7)
<223>  X = V or I

<400>  300

Ser Gly Ser Ser Ser Asn Xaa Gly Ser Asn Pro Val Asn
1               5                   10


<210>  301
<211>  12
<212>  PRT
<213>  Artificial Sequence

<220>
<223>  consensus sequence derived from M20 D02 S-A derived CDR L3


<220>
<221>  VARIANT
<222>  (3)..(3)
<223>  X = A, Q or R

<220>
<221>  VARIANT
<222>  (5)..(5)
<223>  X = D or G

<220>
<221>  VARIANT
<222>  (7)..(7)
<223>  X = R or S

<220>
<221>  VARIANT
<222>  (9)..(9)
<223>  X = N, W or S

<220>
<221>  VARIANT
<222>  (12)..(12)
<223>  X = V, A or G

<400>  301

Cys Ala Xaa Trp Xaa Asp Xaa Leu Xaa Gly Trp Xaa
1               5                   10

<210>  302
<211>  9
<212>  PRT
<213>  Artificial Sequence

<220>
<223>  consensus sequence derived from M31 B01 CDR H1

<220>
<221>  VARIANT
<222>  (3)..(3)
<223>  X = N or S

<220>
<221>  VARIANT
<222>  (4)..(4)
<223>  X = A or Y

<400>  302

Phe Ser Xaa Xaa Trp Met Ser Trp Val
1               5

<210>  303
<211>  20
<212>  PRT
<213>  Artificial Sequence

<220>
<223>  consensus sequence derived from M31 B01 CDR H2

<220>
<221>  VARIANT
<222>  (10)..(10)
<223>  X = T or S

<220>
<221>  VARIANT
<222>  (11)..(11)
<223>  X = I or T

<400>  303

Val Ser Tyr Ile Ser Ser Ser Gly Ser Xaa Xaa Tyr Tyr Ala Asp Ser
1               5                   10                  15

Val Lys Gly Arg
            20

<210>  304
<211>  10
<212>  PRT
<213>  Artificial Sequence

<220>
<223>  consensus sequence derived from M31 B01 CDR H3


<220>
<221>  VARIANT
<222>  (10)..(10)
<223>  X = Y, K, G, W or N

<400>  304

Ala Arg Glu Gly Leu Trp Ala Phe Asp Xaa
1               5                   10


<210>  305
<211>  14
<212>  PRT
<213>  Artificial Sequence

<220>
<223>  consensus sequence derived from M31 B01 CDR L1


<220>
<221>  VARIANT
<222>  (1)..(1)
<223>  X = T or S

<400>  305

Xaa Gly Ser Ser Ser Asn Ile Gly Ala Gly Tyr Val Val His
1               5                   10


<210>  306
<211>  7
<212>  PRT
<213>  Artificial Sequence

<220>
<223>  consensus sequence derived from M31 B01 CDR L2


<220>
<221>  VARIANT
<222>  (4)..(4)
<223>  X = K or Q

<400>  306

Asp Asn Asn Xaa Arg Pro Ser
1               5


<210>  307
<211>  12
<212>  PRT
<213>  Artificial Sequence

<220>
<223>  consensus sequence derived from M31 B01 CDR L3

<220>
<221> VARIANT
<222> (4)..(4)
<223> X = W, E, F or Y

<220>
<221> VARIANT
<222> (7)..(7)
<223> X = R, S or M

<220>
<221> VARIANT
<222> (9)..(9)
<223> X = N, K or S

<220>
<221> VARIANT
<222> (10)..(10)
<223> X = G or R

<220>
<221> VARIANT
<222> (11)..(11)
<223> X = P or A

<400> 307

Cys Ala Ala Xaa Asp Asp Xaa Leu Xaa Xaa Xaa Val
1               5                   10


<210> 308
<211> 351
<212> DNA
<213> Artificial Sequence

<220>
<223> C4.4a binder

<400> 308
gaggtgcagc tgctggagag cggggggggg ctggtgcagc cgggggggag cctgcgcctg      60

agctgcgcgg cgagcgggtt tacctttagc aacgcgtgga tgagctgggt gcgccaggcg     120

ccggggaaag ggctggagtg ggtgagctat attagcagca gcgggagcac catttattat     180

gcggatagcg tgaaagggcg ctttaccatt agccgcgata acagcaaaaa caccctgtat     240

ctgcagatga acagcctgcg cgcggaggat accgcggtgt attattgcgc gcgcgagggg     300

ctgtgggcgt ttgataaatg gggcaggggg accctggtga ccgtgaccag c              351


<210> 309
<211> 339
<212> DNA
<213> Artificial Sequence

<220>
<223> C4.4a binder

<400> 309

cagagcgtgc tgacccagcc gccgagcgcg agcgggaccc cggggcagcg cgtgaccatt 60

agctgcaccg ggagcagcag caacattggg gcggggtatg tggtgcattg gtatcagcag 120

ctgccgggga ccgcgccgaa actgctgatt tatgataaca acaaacgccc gagcggggtg 180

ccggatcgct ttagcgggag caaaagcggg accagcgcga gcctggcgat tagcgggctg 240

cgcagcgagg atgaggcgga ttattattgc gcggcgtatg atgatagcct gaaagggccg 300

gtgtttgggg gggggaccaa actgaccgtg ctggggcag 339

<210> 310
<211> 351
<212> DNA
<213> Artificial Sequence

<220>
<223> C4.4a binder

<400> 310
gaggtgcagc tgctggagag cggggggggg ctggtgcagc cgggggggag cctgcgcctg 60

agctgcgcgg cgagcgggtt tacctttagc aacgcgtgga tgagctgggt gcgccaggcg 120

ccggggaaag ggctggagtg ggtgagctat attagcagca gcgggagcac catttattat 180

gcggatagcg tgaaagggcg ctttaccatt agccgcgata acagcaaaaa caccctgtat 240

ctgcagatga acagcctgcg cgcggaggat accgcggtgt attattgcgc gcgcgagggg 300

ctgtgggcgt ttgataaatg gggcaggggg accctggtga ccgtgaccag c 351

<210> 311
<211> 339
<212> DNA
<213> Artificial Sequence

<220>
<223> C4.4a binder

<400> 311
cagagcgtgc tgacccagcc gccgagcgtg agcggggcgc cggggcagcg cgtgaccatt 60

agctgcaccg ggagcagcag caacattggg gcggggtatg tggtgcattg gtatcagcag 120

ctgccgggga ccgcgccgaa actgctgatt tatgataaca acaaacgccc gagcggggtg 180

ccggatcgct ttagcgggag caaaagcggg accagcgcga gcctggcgat tagcgggctg 240

cgcagcgagg atgaggcgga ttattattgc gcggcgtttg atgatagcct gaacgggccg 300

gtgtttgggg gggggaccaa actgaccgtg ctggggcag 339

<210> 312
<211> 351
<212> DNA
<213> Artificial Sequence

<220>

<223>  C4.4a binder

<400>  312
gaggtgcagc tgctggagag cggggggggg ctggtgcagc cgggggggag cctgcgcctg      60

agctgcgcgg cgagcgggtt tacctttagc aacgcgtgga tgagctgggt gcgccaggcg     120

ccggggaaag ggctggagtg ggtgagctat attagcagca gcgggagcac catttattat     180

gcggatagcg tgaaagggcg ctttaccatt agccgcgata acagcaaaaa caccctgtat     240

ctgcagatga acagcctgcg cgcggaggat accgcggtgt attattgcgc gcgcgagggg     300

ctgtgggcgt ttgataaatg gggcaggggg accctggtga ccgtgaccag c               351


<210>  313
<211>  339
<212>  DNA
<213>  Artificial Sequence

<220>
<223>  C4.4a binder

<400>  313
cagagcgtgc tgacccagcc gccgagcgtg agcggggcgc cggggcagcg cgtgaccatt      60

agctgcaccg ggagcagcag caacattggg gcggggtatg tggtgcattg gtatcagcag     120

ctgccgggga ccgcgccgaa actgctgatt tatgataaca acaaacgccc gagcggggtg     180

ccggatcgct ttagcgggag caaaagcggg accagcgcga gcctggcgat tagcgggctg     240

cgcagcgagg atgaggcgga ttattattgc gcggcgtttg atgatagcct gaacgggccg      300

gtgtttgggg gggggaccaa actgaccgtg ctggggcag                            339


<210>  314
<211>  351
<212>  DNA
<213>  Artificial Sequence

<220>
<223>  C4.4a binder

<400>  314
gaggtgcagc tgctggagag cggggggggg ctggtgcagc cgggggggag cctgcgcctg      60

agctgcgcgg cgagcgggtt tacctttagc agcgcgtgga tgagctgggt gcgccaggcg     120

ccggggaaag ggctggagtg ggtgagctat attagcagca gcgggagcag cacctattat     180

gcggatagcg tgaaagggcg ctttaccatt agccgcgata acagcaaaaa caccctgtat     240

ctgcagatga acagcctgcg cgcggaggat accgcggtgt attattgcgc gcgcgagggg     300

ctgtgggcgt ttgattggtg gggcaggggg accctggtga ccgtgaccag c               351


<210>  315
<211>  339
<212>  DNA

<213>  Artificial Sequence

<220>
<223>  C4.4a binder

<400>  315
cagagcgtgc tgacccagcc gccgagcgcg agcgggaccc cggggcagcg cgtgaccatt      60

agctgcagcg ggagcagcag caacattggg gcggggtatg tggtgcattg gtatcagcag     120

ctgccgggga ccgcgccgaa actgctgatt tatgataaca accagcgccc gagcggggtg     180

ccggatcgct ttagcgggag caaaagcggg accagcgcga gcctggcgat tagcgggctg     240

cgcagcgagg atgaggcgga ttattattgc gcggcgtatg atgatagcct gagcgggccg     300

gtgtttgggg gggggaccaa actgaccgtg ctggggcag                            339


<210>  316
<211>  351
<212>  DNA
<213>  Artificial Sequence

<220>
<223>  C4.4a binder

<400>  316
gaggtgcagc tgctggagag cggggggggg ctggtgcagc cgggggggag cctgcgcctg      60

agctgcgcgg cgagcgggtt tacctttagc aacgcgtgga tgagctgggt gcgccaggcg     120

ccggggaaag ggctggagtg ggtgagctat attagcagca gcgggagcac catttattat     180

gcggatagcg tgaaagggcg ctttaccatt agccgcgata acagcaaaaa caccctgtat     240

ctgcagatga acagcctgcg cgcggaggat accgcggtgt attattgcgc gcgcgagggg     300

ctgtgggcgt ttgattattg gggcaggggg accctggtga ccgtgaccag c              351


<210>  317
<211>  339
<212>  DNA
<213>  Artificial Sequence

<220>
<223>  C4.4a binder

<400>  317
cagagcgtgc tgacccagcc gccgagcgcg agcgggaccc cggggcagcg cgtgaccatt      60

agctgcagcg ggagcagcag caacattggg gcggggtatg tggtgcattg gtatcagcag     120

ctgccgggga ccgcgccgaa actgctgatt tatgataaca accagcgccc gagcggggtg     180

ccggatcgct ttagcgggag caaaagcggg accagcgcga gcctggcgat tagcgggctg     240

cgcagcgagg atgaggcgga ttattattgc gcggcgtatg atgatagcct gaacgggccg     300

gtgtttgggg gggggaccaa actgaccgtg ctggggcag                            339

<210>  318
<211>  351
<212>  DNA
<213>  Artificial Sequence

<220>
<223>  C4.4a binder

<400>  318
gaggtgcagc tgctggagag cggggggggg ctggtgcagc cgggggggag cctgcgcctg      60

agctgcgcgg cgagcgggtt tacctttagc agcgcgtgga tgagctgggt gcgccaggcg      120

ccggggaaag ggctggagtg ggtgagctat attagcagca gcgggagcag cacctattat      180

gcggatagcg tgaaagggcg ctttaccatt agccgcgata acagcaaaaa caccctgtat      240

ctgcagatga acagcctgcg cgcggaggat accgcggtgt attattgcgc gcgcgagggg      300

ctgtgggcgt ttgattattg gggcaggggg accctggtga ccgtgagcag c      351


<210>  319
<211>  339
<212>  DNA
<213>  Artificial Sequence

<220>
<223>  C4.4a binder

<400>  319
gagagcgtgc tgacccagcc gccgagcgcg agcgggaccc cggggcagcg cgtgaccatt      60

agctgcagcg ggagcagcag caacattggg gcggggtatg tggtgcattg gtatcagcag      120

ctgccgggga ccgcgccgaa actgctgatt tatgataaca accagcgccc gagcggggtg      180

ccggatcgct ttagcgggag caaaagcggg accagcgcga gcctggcgat tagcgggctg      240

cgcagcgagg atgaggcgga ttattattgc gcggcgtggg atgatcgcct gaacggcccg      300

gtgtttgggg gggggaccaa actgaccgtg ctggggcag      339


<210>  320
<211>  351
<212>  DNA
<213>  Artificial Sequence

<220>
<223>  C4.4a binder

<400>  320
gaggtgcagc tgctggagag cggggggggg ctggtgcagc cgggggggag cctgcgcctg      60

agctgcgcgg cgagcgggtt tacctttagc aacgcgtgga tgagctgggt gcgccaggcg      120

ccggggaaag ggctggagtg ggtgagctat attagcagca gcgggagcac catttattat      180

gcggatagcg tgaaagggcg ctttaccatt agccgcgata acagcaaaaa caccctgtat      240

ctgcagatga acagcctgcg cgcggaggat accgcggtgt attattgcgc gcgcgagggg      300

ctgtgggcgt ttgattattg ggggcagggg accctggtga ccgtgagcag c 351

<210> 321
<211> 339
<212> DNA
<213> Artificial Sequence

<220>
<223> C4.4a binder

<400> 321
gagagcgtgc tgacccagcc gccgagcgtg agcggggcgc cggggcagcg cgtgaccatt 60

agctgcaccg ggagcagcag caacattggg gcggggtatg tggtgcattg gtatcagcag 120

ctgccgggga ccgcgccgaa actgctgatt tatgataaca acaaacgccc gagcggggtg 180

ccggatcgct ttagcgggag caaaagcggg accagcgcga gcctggcgat tagcgggctg 240

cgcagcgagg atgaggcgga ttattattgc gcggcgtatg atgatcgcct gaacgggccg 300

gtgtttgggg gggggaccaa actgaccgtg ctggggcag 339

<210> 322
<211> 351
<212> DNA
<213> Artificial Sequence

<220>
<223> C4.4a binder

<400> 322
gaggtgcagc tgctggagag cggggggggg ctggtgcagc cgggggggag cctgcgcctg 60

agctgcgcgg cgagcgggtt tacctttagc agcgcgtgga tgagctgggt gcgccaggcg 120

ccggggaaag ggctggagtg ggtgagctat attagcagca gcgggagcag cacctattat 180

gcggatagcg tgaaagggcg ctttaccatt agccgcgata acagcaaaaa caccctgtat 240

ctgcagatga acagcctgcg cgcggaggat accgcggtgt attattgcgc gcgcgagggg 300

ctgtgggcgt ttgatgggtg ggggcagggg accctggtga ccgtgagcag c 351

<210> 323
<211> 339
<212> DNA
<213> Artificial Sequence

<220>
<223> C4.4a binder

<400> 323
cagagcgtgc tgacccagcc gccgagcgcg agcgggaccc cggggcagcg cgtgaccatt 60

agctgcagcg ggagcagcag caacattggg gcggggtatg tggtgcattg gtatcagcag 120

ctgccgggga ccgcgccgaa actgctgatt tatgataaca accagcgccc gagcggggtg 180

ccggatcgct ttagcgggag caaaagcggg accagcgcga gcctggcgat tagcgggctg 240

cgcagcgagg atgaggcgga ttattattgc gcggcgtatg atgatagcct gaaccgcccg 300

gtgtttgggg gggggaccaa actgaccgtg ctggggcag 339

<210> 324
<211> 351
<212> DNA
<213> Artificial Sequence

<220>
<223> C4.4a binder

<400> 324
gaggtgcagc tgctggagag cggggggggg ctggtgcagc cgggggggag cctgcgcctg 60

agctgcgcgg cgagcgggtt tacctttagc aacgcgtgga tgagctgggt gcgccaggcg 120

ccggggaaag ggctggagtg ggtgagctat attagcagca gcgggagcac catttattat 180

gcggatagcg tgaaagggcg ctttaccatt agccgcgata acagcaaaaa caccctgtat 240

ctgcagatga acagcctgcg cgcggaggat accgcggtgt attattgcgc gcgcgagggg 300

ctgtgggcgt ttgattattg gggcaggggg accctggtga ccgtgagcag c 351

<210> 325
<211> 339
<212> DNA
<213> Artificial Sequence

<220>
<223> C4.4a binder

<400> 325
cagagcgtgc tgacccagcc gccgagcgtg agcggggcgc cggggcagcg cgtgaccatt 60

agctgcaccg ggagcagcag caacattggg gcggggtatg tggtgcattg gtatcagcag 120

ctgccgggga ccgcgccgaa actgctgatt tatgataaca acaaacgccc gagcggggtg 180

ccggatcgct ttagcgggag caaaagcggg accagcgcga gcctggcgat tagcgggctg 240

cgcagcgagg atgaggcgga ttattattgc gcggcgtttg atgatagcct gaacgggccg 300

gtgtttgggg gggggaccaa actgaccgtg ctggggcag 339

<210> 326
<211> 351
<212> DNA
<213> Artificial Sequence

<220>
<223> C4.4a binder

<400> 326
gaggtgcagc tgctggagag cggggggggg ctggtgcagc cgggggggag cctgcgcctg 60

agctgcgcgg cgagcgggtt tacctttagc agcgcgtgga tgagctgggt gcgccaggcg 120

ccggggaaag ggctggagtg ggtgagctat attagcagca gcgggagcag cacctattat 180

gcggatagcg tgaaagggcg ctttaccatt agccgcgata acagcaaaaa caccctgtat 240

ctgcagatga acagcctgcg cgcggaggat accgcggtgt attattgcgc gcgcgagggg 300

ctgtgggcgt ttgataaatg gggcaggggg accctggtga ccgtgaccag c 351

<210> 327
<211> 339
<212> DNA
<213> Artificial Sequence

<220>
<223> C4.4a binder

<400> 327
cagagcgtgc tgacccagcc gccgagcgcg agcgggaccc cggggcagcg cgtgaccatt 60

agctgcaccg ggagcagcag caacattggg gcggggtatg tggtgcattg gtatcagcag 120

ctgccgggga ccgcgccgaa actgctgatt tatgataaca acaaacgccc gagcggggtg 180

ccggatcgct ttagcgggag caaaagcggg accagcgcga gcctggcgat tagcgggctg 240

cgcagcgagg atgaggcgga ttattattgc gcggcgtttg atgatagcct gaacgggccg 300

gtgtttgggg gggggaccaa actgaccgtg ctggggcag 339

<210> 328
<211> 351
<212> DNA
<213> Artificial Sequence

<220>
<223> C4.4a binder

<400> 328
   gaggtgcagc tgctggagag cggggggggg ctggtgcagc cgggggggag cctgcgcctg 60
   agctgcgcgg cgagcgggtt tacctttagc agcgcgtgga tgagctgggt gcgccaggcg 120
   ccggggaaag ggctggagtg ggtgagctat attagcagca gcgggagcag cacctattat 180
   gcggatagcg tgaaagggcg ctttaccatt agccgcgata acagcaaaaa caccctgtat 240
   ctgcagatga acagcctgcg cgcggaggat accgcggtgt attattgcgc gcgcgagggg 300
   ctgtgggcgt ttgataaatg gggcaggggg accctggtga ccgtgagcag c 351

<210> 329
<211> 339
<212> DNA
<213> Artificial Sequence

<220>
<223> C4.4a

<400> 329
cagagcgtgc tgacccagcc gccgagcgcg agcgggaccc cggggcagcg cgtgaccatt 60

agctgcagcg ggagcagcag caacattggg gcggggtatg tggtgcattg gtatcagcag 120

ctgccgggga ccgcgccgaa actgctgatt tatgataaca accagcgccc gagcggggtg 180

ccggatcgct ttagcgggag caaaagcggg accagcgcga gcctggcgat tagcgggctg 240

cgcagcgagg atgaggcgga ttattattgc gcggcgtttg atgatagcct gaacgggccg 300

gtgtttgggg gggggaccaa actgaccgtg ctggggcag 339

<210> 330
<211> 351
<212> DNA
<213> Artificial Sequence

<220>
<223> C4.4a binder

<400> 330
   gaggtgcagc tgctggagag cggggggggc ctggtgcagc cgggggggag cctgcgcctg 60
   agctgcgcgg cgagcgggtt tacctttagc agcgcgtgga tgagctgggt gcgccaggcg 120
   ccggggaaag ggctggagtg ggtgagctat attagcagca gcgggagcag cacctattat 180
   gcggatagcg tgaaagggcg ctttaccatt agccgcgata acagcaaaaa caccctgtat 240
   ctgcagatga acagcctgcg cgcggaggat accgcggtgt attattgcgc gcgcgagggg 300
   ctgtgggcgt ttgataaatg ggggcagggg accctggtga ccgtgagcag c 351

<210> 331
<211> 339
<212> DNA
<213> Artificial Sequence

<220>
<223> C4.4a binder

<400> 331
cagagcgtgc tgacccagcc gccgagcgcg agcgggaccc cggggcagcg cgtgaccatt 60

agctgcagcg ggagcagcag caacattggg gcggggtatg tggtgcattg gtatcagcag 120

ctgccgggga ccgcgccgaa actgctgatt tatgataaca accagcgccc gagcggggtg 180

ccggatcgct ttagcgggag caaaagcggg accagcgcga gcctggcgat tagcgggctg 240

cgcagcgagg atgaggcgga ttattattgc gcggcgtttg atgatcgcct gagcgggccg 300

gtgtttgggg gggggaccaa actgaccgtg ctggggcag 339

<210> 332
<211> 366
<212> DNA
<213> Artificial Sequence

<220>
<223> C4.4a binder

<400>  332
   gaggtgcagc tgctggagag cggggggggg ctggtgcagc cgggggggag cctgcgcctg      60
   agctgcgcgg cgagcgggtt tacctttagc gattatcaga tgacctggat tcgccagacc     120
   ccggggaaag ggctggagtg ggtgagcggg gtgagctgga acggggcgcg cacccattat     180
   gcggatagcg tgaaagggcg ctttaccatt agccgcgata acagcaaaaa caccctgtat     240
   ctgcagatga acagcctgcg cgcggaggat accgcggtgt attattgcgc gaaaggggat     300
   tatctggtgt atagcagcta ttattttaaa agctgggggc aggggaccct ggtgaccgtg     360
   accagc                                                                366


<210>  333
<211>  336
<212>  DNA
<213>  Artificial Sequence

<220>
<223>  C4.4a binder

<400>  333
cagagcgtgc tgacccagcc gccgagcgcg agcgggaccc cggggcagcg cgtgaccatt      60

agctgcagcg ggagcagcag caacgtgggg agcaacccgg tgaactggta tcagcagctg     120

ccggggaccg cgccgaaact gctgatttat cgcaacaacc agcgcccgag cggggtgccg     180

gatcgcttta gcgggagcaa aagcgggacc agcgcgagcc tggcgattag cgggctgcgc     240

agcgaggatg aggcggatta ttattgcgcg gcgtgggatg atcgcctgaa cggtgggggg     300

tttggggggg ggaccaaact gaccgtgctg gggcag                               336


<210>  334
<211>  363
<212>  DNA
<213>  Artificial Sequence

<220>
<223>  C4.4a binder

<400>  334
gagcagctgc tggagagcgg gggggggctg gtgcagccgg gggggagcct gcgcctgagc      60

tgcgcggcga gcgggtttac ctttagcagc tatcagatga cctggattcg ccagaccccg     120

gggaaagggc tggagtgggt gagcgggatt agctggaacg gggggagcac ccattatgcg     180

gatagcgtga aagggcgctt taccattagc cgcgataaca gcaaaaacac cctgtatctg     240

cagatgaaca gcctgcgcgc ggaggatacc gcggtgtatt attgcgcgaa aggggattat     300

ctggtgtata gcgcgtatta ttttgatagc tgggggcagg ggaccctggt gaccgtgagc     360

agc                                                                   363

<210>  335
<211>  333
<212>  DNA
<213>  Artificial Sequence

<220>
<223>  C4.4a binder

<400>  335
caggtgctga cccagccgcc gagcgcgagc gggaccccgg ggcagcgcgt gaccattagc      60

tgcagcggga gcagcagcaa cattgggagc aacccggtga actggtatca gcagctgccg     120

gggaccgcgc cgaaactgct gatttatcgc aacaaccagc gcccgagcgg ggtgccggat     180

cgctttagcg ggagcaaaag cgggaccagc gcgagcctgg cgattagcgg gctgcgcagc     240

gaggatgagg cggattatta ttgcgcggcg tgggatgatc gcctgaacgg gtgggggttt     300

ggggggggga ccaaactgac cgtgctgggg cag                                   333


<210>  336
<211>  366
<212>  DNA
<213>  Artificial Sequence

<220>
<223>  C4.4a binder

<400>  336
gaggtgcagc tgctggagag cggggggggg ctggtgcagc cgggggggag cctgcgcctg      60

agctgcgcgg cgagcgggtt tacctttagc gattatcaga tgacctggat tcgccagacc     120

ccggggaaag ggctggagtg ggtgagcggg attagctgga acggggggag cacccattat     180

gcggatagcg tgaaagggcg ctttaccatt agccgcgata acagcaaaaa caccctgtat     240

ctgcagatga acagcctgcg cgcggaggat accgcggtgt attattgcgc gaaaggggat     300

tatctggtgt atagcagcta ttattttaaa tattgggggc aggggaccct ggtgaccgtg     360

agcagc                                                                366


<210>  337
<211>  336
<212>  DNA
<213>  Artificial Sequence

<220>
<223>  C4.4a binder

<400>  337
cagagcgtgc tgacccagcc gccgagcgcg agcgggaccc cggggcagcg cgtgaccatt      60

agctgcagcg ggagcagcag caacattggg agcaacccgg tgaactggta tcagcagctg     120

ccggggaccg cgccgaaact gctgatttat cgcaacaacc agcgcccgag cggggtgccg     180

gatcgcttta gcgggagcaa aagcgggacc agcgcgagcc tggcgattag cgggctgcgc     240

agcgaggatg aggcggatta ttattgcgcg gcgtgggatg atcgcctgaa cgggtgggcg 300

tttggggggg ggaccaaact gaccgtgctg gggcag 336

<210> 338
<211> 366
<212> DNA
<213> Artificial Sequence

<220>
<223> C4.4a binder

<400> 338
   gaggtgcagc tgctggagag cggggggggc ctggtgcagc cgggggggag cctgcgcctg 60
   agctgcgcgg cgagcgggtt tacctttagc gattatcaga tgacctggat tcgccagacc 120
   ccggggaaag ggctggagtg ggtgagcggg attagctgga acggggggag cacccattat 180
   gcggatagcg tgaaagggcg ctttaccatt agccgcgata acagcaaaaa caccctgtat 240
   ctgcagatga acagcctgcg cgcggaggat accgcggtgt attattgcgc gaaaggggat 300
   tatctggtgt atagcagcta ttattttaaa agctgggggc aggggaccct ggtgaccgtg 360
   agcagc 366

<210> 339
<211> 336
<212> DNA
<213> Artificial Sequence

<220>
<223> C4.4a binder

<400> 339
cagagcgtgc tgacccagcc gccgagcgcg agcgggaccc cggggcagcg cgtgaccatt 60

agctgcagcg ggagcagcag caacattggg agcaacccgg tgaactggta tcagcagctg 120

ccggggaccg cgccgaaact gctgatttat cgcaacaacc agcgcccgag cggggtgccg 180

gatcgcttta gcgggagcaa aagcgggacc agcgcgagcc tggcgattag cgggctgcgc 240

agcgaggatg aggcggatta ttattgcgcg gcgtgggatg atagcctgaa cgggtggggg 300

tttggggggg ggaccaaact gaccgtgctg gggcag 336

<210> 340
<211> 366
<212> DNA
<213> Artificial Sequence

<220>
<223> C4.4a binder

<400> 340
gaggtgcagc tgctggagag cggggggggc ctggtgcagc cgggggggag cctgcgcctg 60

agctgcgcgg cgagcgggtt tacctttagc gattatcaga tgacctggat tcgccagacc 120

ccggggaaag ggctggagtg ggtgagcggg attagctgga acggggggag cacccattat 180

gcggatagcg tgaaagggcg ctttaccatt agccgcgata acagcaaaaa caccctgtat 240

ctgcagatga acagcctgcg cgcggaggat accgcggtgt attattgcgc gaaaggggat 300

tatctggtgt ataaaagcta ttattttaaa agctgggggc aggggaccct ggtgaccgtg 360

agcagc 366

<210> 341
<211> 336
<212> DNA
<213> Artificial Sequence

<220>
<223> C4.4a binder

<400> 341
cagagcgtgc tgacccagcc gccgagcgcg agcgggaccc cggggcagcg cgtgaccatt 60

agctgcagcg ggagcagcag caacattggg agcaacccgg tgaactggta tcagcagctg 120

ccggggaccg cgccgaaact gctgatttat cgcaacaacc agcgcccgag cggggtgccg 180

gatcgcttta gcgggagcaa aagcgggacc agcgcgagcc tggcgattag cgggctgcgc 240

agcgaggatg aggcggatta ttattgcgcg gcgtgggatg atcgcctgag cgggtggggg 300

tttggggggg ggaccaaact gaccgtgctg gggcag 336

<210> 342
<211> 366
<212> DNA
<213> Artificial Sequence

<220>
<223> C4.4a binder

<400> 342
gaggtgcagc tgctggagag cggggggggg ctggtgcagc cgggggggag cctgcgcctg 60

agctgcgcgg cgagcgggtt tacctttagc gattatcaga tgacctggat tcgccagacc 120

ccggggaaag ggctggagtg ggtgagcggg attagctgga acggggggag cacccattat 180

gcggatagcg tgaaagggcg ctttaccatt agccgcgata acagcaaaaa caccctgtat 240

ctgcagatga acagcctgcg cgcggaggat accgcggtgt attattgcgc gaaaggggat 300

tatctggtgt atagcagcta ttattttaaa agctgggggc aggggaccct ggtgaccgtg 360

agcagc 366

<210> 343
<211> 336
<212> DNA
<213> Artificial Sequence

<220>
<223>  C4.4a binder

<400>  343
cagagcgtgc tgacccagcc gccgagcgcg agcgggaccc cggggcagcg cgtgaccatt      60

agctgcagcg ggagcagcag caacattggg agcaacccgg tgaactggta tcagcagctg     120

ccggggaccg cgccgaaact gctgatttat cgcaacaacc agcgcccgag cggggtgccg     180

gatcgcttta gcgggagcaa aagcgggacc agcgcgagcc tggcgattag cgggctgcgc     240

agcgaggatg aggcggatta ttattgcgcg gcgtgggatg atcgcctgag cgggtggggg     300

tttggggggg ggaccaaact gaccgtgctg gggcag                               336


<210>  344
<211>  366
<212>  DNA
<213>  Artificial Sequence

<220>
<223>  C4.4a binder

<400>  344
gaggtgcagc tgctggagag cggggggggg ctggtgcagc cgggggggag cctgcgcctg      60

agctgcgcgg cgagcgggtt tacctttagc gattatcaga tgacctggat tcgccagacc     120

ccggggaaag ggctggagtg ggtgagcggg attagctgga acggggggag cacccattat     180

gcggatagcg tgaaagggcg ctttaccatt agccgcgata acagcaaaaa caccctgtat     240

ctgcagatga acagcctgcg cgcggaggat accgcggtgt attattgcgc gaaaggggat     300

tatctggtgt ataaaagcta ttattttaaa agctgggggc aggggaccct ggtgaccgtg     360

agcagc                                                                366


<210>  345
<211>  336
<212>  DNA
<213>  Artificial Sequence

<220>
<223>  C4.4a binder

<400>  345
cagagcgtgc tgacccagcc gccgagcgcg agcgggaccc cggggcagcg cgtgaccatt      60

agctgcagcg ggagcagcag caacattggg agcaacccgg tgaactggta tcagcagctg     120

ccggggaccg cgccgaaact gctgatttat cgcaacaacc agcgcccgag cggggtgccg     180

gatcgcttta gcgggagcaa aagcgggacc agcgcgagcc tggcgattag cgggctgcgc     240

agcgaggatg aggcggatta ttattgcgcg gcgtgggatg atagcctgaa cgggtggggg     300

tttggggggg ggaccaaact gaccgtgctg gggcag                               336

<210>  346
<211>  217
<212>  PRT
<213>  Homo Sapiens

<400>  346

Asp Ile Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Trp Asp Asp Arg
                85                  90                  95

Leu Asn Gly Pro Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Gly Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  347
<211>  447
<212>  PRT
<213>  Homo Sapiens

<400>  347

Gln Val Glu Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys
        435                 440                 445

<210>  348
<211>  217
<212>  PRT
<213>  Homo Sapiens

<400>  348

Glu Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Trp Asp Asp Arg
                85                  90                  95

Leu Asn Gly Pro Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  349
<211>  446
<212>  PRT
<213>  Homo Sapiens

<400>  349

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly
        435                 440                 445

<210>  350
<211>  217
<212>  PRT
<213>  Homo Sapiens

<400>  350

Glu Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Trp Asp Asp Arg
                85                  90                  95

Leu Asn Gly Pro Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  351
<211>  446
<212>  PRT
<213>  Homo Sapiens

<400>  351

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly
        435                 440                 445

<210>  352
<211>  217
<212>  PRT
<213>  Homo Sapiens

<400>  352

Glu Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Trp Asp Asp Arg
                85                  90                  95

Leu Asn Gly Pro Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  353
<211>  446
<212>  PRT
<213>  Homo Sapiens

<400>  353

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys

305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly
        435                 440                 445

<210>  354
<211>  217
<212>  PRT
<213>  Homo Sapiens

<400>  354

Glu Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Tyr Asp Asp Arg

85                  90                  95

Leu Asn Gly Pro Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  355
<211>  446
<212>  PRT
<213>  Homo Sapiens

<400>  355

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys

85                  90                  95

Ala Arg Glu Gly Leu Trp Ala Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
        340                 345                 350

Pro Ser Arg Asp Glu Leu Thr Lys Asn Gln Val Ser Leu Thr Cys Leu
    355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
        420                 425                 430

His Asn His Tyr Thr Gln Lys Ser Leu Ser Leu Ser Pro Gly
    435                 440                 445

<210>  356
<211>  217
<212>  PRT
<213>  Homo Sapiens

<400>  356

Gln Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
        20                  25                  30

Tyr Val Val His Trp Tyr Gln Gln Leu Pro Gly Thr Ala Pro Lys Leu
    35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Tyr Asp Asp Ser
                85                  90                  95

Leu Ser Gly Pro Val Phe Gly Gly Gly Thr Lys Leu Thr Val Leu Gly
        100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  357
<211>  446
<212>  PRT
<213>  Homo Sapiens

<400>  357

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly
        435                 440                 445

<210>  358
<211>  217
<212>  PRT
<213>  Homo Sapiens

<400>  358

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Tyr Asp Asp Ser
                85                  90                  95

Leu Asn Arg Pro Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  359
<211>  446
<212>  PRT
<213>  Homo Sapiens

<400>  359

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Gly Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly
        435                 440                 445

<210> 360
<211> 217
<212> PRT
<213> Homo Sapiens

<400> 360

Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Phe Asp Asp Arg
                85                  90                  95

Leu Asn Gly Pro Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  361
<211>  446
<212>  PRT
<213>  Homo Sapiens

<400>  361

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Lys Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
        180                 185                 190

Leu Gly Thr Gln Thr Tyr Ile Cys Asn Val Asn His Lys Pro Ser Asn
    195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
        260                 265                 270

Val Lys Phe Asn Trp Tyr Val Asp Gly Val Glu Val His Asn Ala Lys
    275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
        340                 345                 350

Pro Ser Arg Asp Glu Leu Thr Lys Asn Gln Val Ser Leu Thr Cys Leu
    355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu

420                 425                 430

His Asn His Tyr Thr Gln Lys Ser Leu Ser Leu Ser Pro Gly
        435                 440                 445

<210>  362
<211>  217
<212>  PRT
<213>  Homo Sapiens

<400>  362

Gln Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Phe Asp Asp Ser
                85                  90                  95

Leu Asn Gly Pro Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu

195                 200                 205

Lys Thr Val Ala Pro Thr Glu Cys Ser
    210                 215

<210>  363
<211>  446
<212>  PRT
<213>  Homo Sapiens

<400>  363

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn

195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly
        435                 440                 445

<210>  364
<211>  217
<212>  PRT
<213>  Homo Sapiens

<400>  364

Gln Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Lys Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Tyr Asp Asp Ser
                85                  90                  95

Leu Ser Gly Pro Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  365
<211>  446
<212>  PRT
<213>  Homo Sapiens

<400>  365

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly
        435                 440                 445

<210>  366
<211>  217
<212>  PRT
<213>  Homo Sapiens

<400>  366

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Phe Asp Asp Ser
                85                  90                  95

Leu Asn Gly Pro Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  367
<211>  446
<212>  PRT
<213>  Homo Sapiens

<400>  367

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Lys Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly
        435                 440                 445

<210> 368
<211> 217
<212> PRT
<213> Homo Sapiens

<400> 368

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ala Gly
            20                  25                  30

Tyr Val Val His Trp Tyr Gln Gln Leu Pro Gly Thr Ala Pro Lys Leu
            35                  40                  45

Leu Ile Tyr Asp Asn Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Phe Asp Asp Arg
                85                  90                  95

Leu Ser Gly Pro Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  369
<211>  446
<212>  PRT
<213>  Homo Sapiens

<400>  369

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Lys Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly
        435                 440                 445


<210>  370
<211>  216
<212>  PRT
<213>  Homo Sapiens

<400>  370

Asp Ile Val Leu Thr Gln Pro Pro Ser Ala Ser Gly Thr Pro Gly Gln
1               5                   10                  15

Arg Val Thr Ile Ser Cys Ser Gly Ser Ser Ser Asn Val Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Asn Gly Trp Val Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Gly Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  371
<211>  452
<212>  PRT
<213>  Homo Sapiens

<400>  371

Gln Val Glu Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Val Ser Trp Asn Gly Ala Arg Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ala Tyr Tyr Phe Asp Ser Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn

305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Lys
    450

<210>  372
<211>  216
<212>  PRT
<213>  Homo Sapiens

<400>  372

Glu Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Val Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg

65                  70                  75                  80

Ser Glu Asp Glu Ala Asp Tyr Tyr Cys Ala Ala Trp Asp Asp Arg Leu
                85                  90                  95

Asn Gly Trp Val Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  373
<211>  451
<212>  PRT
<213>  Homo Sapiens

<400>  373

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Val Ser Trp Asn Gly Ala Arg Thr His Tyr Ala Asp Ser Val
    50                  55                  60

Lys Gly Arg Phe Thr Ile Ser Arg Asp Asn Ser Lys Asn Thr Leu Tyr

65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ala Tyr Tyr Phe Asp Ser Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly
    450

<210> 374
<211> 216
<212> PRT
<213> Homo Sapiens

<400> 374

Glu Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Asn Gly Trp Val Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  375
<211>  451
<212>  PRT
<213>  Homo Sapiens

<400>  375

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ala Tyr Tyr Phe Asp Ser Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly
    450

<210>  376
<211>  216
<212>  PRT
<213>  Homo Sapiens

<400>  376

Glu Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Asn Gly Trp Val Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210> 377
<211> 451
<212> PRT
<213> Homo Sapiens

<400> 377

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ala Tyr Tyr Phe Asp Ser Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly
    450

<210>  378
<211>  216
<212>  PRT
<213>  Homo Sapiens

<400>  378

Glu Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Asn Gly Trp Gly Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  379
<211>  451
<212>  PRT
<213>  Homo Sapiens

<400>  379

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ala Tyr Tyr Phe Asp Ser Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val

355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly
    450

<210>  380
<211>  216
<212>  PRT
<213>  Homo Sapiens

<400>  380

Glu Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Asn Gly Trp Gly Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu

115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  381
<211>  451
<212>  PRT
<213>  Homo Sapiens

<400>  381

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ala Tyr Tyr Phe Asp Ser Trp
            100                 105                 110

Gly Gln Gly Thr Leu Val Thr Val Ser Ser Ala Ser Thr Lys Gly Pro

115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly
    450

<210>  382
<211>  216
<212>  PRT
<213>  Homo Sapiens

<400>  382

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Ser Gly Trp Ala Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  383
<211>  451
<212>  PRT
<213>  Homo Sapiens

<400>  383

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ser Tyr Tyr Phe Lys Ser Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly
    450

<210>  384
<211>  216
<212>  PRT
<213>  Homo Sapiens

<400>  384

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Ser Leu
                85                  90                  95

Ser Gly Trp Ala Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  385
<211>  451
<212>  PRT
<213>  Homo Sapiens

<400>  385

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Lys Ser Tyr Tyr Phe Lys Ser Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly
    450

<210> 386
<211> 216
<212> PRT
<213> Homo Sapiens

<400> 386

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Asn Gly Trp Ala Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  387
<211>  451
<212>  PRT
<213>  Homo Sapiens

<400>  387

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ser Tyr Tyr Phe Lys Tyr Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr

405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly
    450

<210>  388
<211>  216
<212>  PRT
<213>  Homo Sapiens

<400>  388

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Ser Leu
                85                  90                  95

Asn Gly Trp Gly Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr

                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210> 389
<211> 451
<212> PRT
<213> Homo Sapiens

<400> 389

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ser Tyr Tyr Phe Lys Ser Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro

                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly
    450

<210> 390
<211> 216
<212> PRT
<213> Homo Sapiens

<400> 390

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Ser Gly Trp Gly Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
        180                 185                 190

Arg Ser Tyr Ser Cys Gln Val Thr His Glu Gly Ser Thr Val Glu Lys
    195                 200                 205

Thr Val Ala Pro Thr Glu Cys Ser
    210                 215

<210>  391
<211>  451
<212>  PRT
<213>  Homo Sapiens

<400>  391

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Lys Ser Tyr Tyr Phe Lys Ser Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly
    450

<210>  392
<211>  216
<212>  PRT
<213>  Homo Sapiens

<400>  392

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Ser Gly Trp Gly Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  393
<211>  451
<212>  PRT
<213>  Homo Sapiens

<400>  393

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ser Tyr Tyr Phe Lys Ser Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly
    450

<210>  394
<211>  216
<212>  PRT
<213>  Homo Sapiens

<400>  394

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Ser Leu
                85                  90                  95

Asn Gly Trp Gly Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  395
<211>  451
<212>  PRT
<213>  Homo Sapiens

<400>  395

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Lys Ser Tyr Tyr Phe Lys Ser Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly

    450

<210> 396
<211> 216
<212> PRT
<213> Homo Sapiens

<400> 396

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Ser Leu
                85                  90                  95

Ser Gly Trp Ala Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser

210                 215

<210>  397
<211>  451
<212>  PRT
<213>  Homo Sapiens

<400>  397

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gln Met Thr Trp Ile Arg Gln Ala Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Lys Ser Tyr Tyr Phe Lys Ser Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser

210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly
    450

<210>  398
<211>  10
<212>  PRT
<213>  Homo sapiens

<400>  398

Gly Tyr Ser Phe Thr Ser Tyr Trp Ile Gly
1               5                   10


<210>  399
<211>  20
<212>  PRT
<213>  Homo sapiens

<400>  399

Trp Met Gly Ile Ile Asp Pro Gly Asp Ser Arg Thr Arg Tyr Ser Pro
1               5                   10                  15


Ser Phe Gln Gly
            20


<210>  400
<211>  11
<212>  PRT
<213>  Homo sapiens

<400>  400

Gly Gln Leu Tyr Gly Gly Thr Tyr Met Asp Gly
1               5                   10


<210>  401
<211>  14
<212>  PRT
<213>  Homo sapiens

<400>  401

Thr Gly Thr Ser Ser Asp Ile Gly Gly Tyr Asn Ser Val Ser
1               5                   10


<210>  402
<211>  11
<212>  PRT
<213>  Homo sapiens

<400>  402

Leu Met Ile Tyr Gly Val Asn Asn Arg Pro Ser
1               5                   10


<210>  403
<211>  10
<212>  PRT
<213>  Homo sapiens

<400>  403

Ser Ser Tyr Asp Ile Glu Ser Ala Thr Pro
1               5                   10

<210>  404
<211>  120
<212>  PRT
<213>  Homo sapiens

<400>  404

Gln Val Glu Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ser Tyr
            20                  25                  30

Trp Ile Gly Trp Val Arg Gln Ala Pro Gly Lys Gly Leu Glu Trp Met
        35                  40                  45

Gly Ile Ile Asp Pro Gly Asp Ser Arg Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Gly Gln Leu Tyr Gly Gly Thr Tyr Met Asp Gly Trp Gly Gln
            100                 105                 110

Gly Thr Leu Val Thr Val Ser Ser
        115                 120

<210>  405
<211>  113
<212>  PRT
<213>  Homo sapiens

<400>  405

Asp Ile Ala Leu Thr Gln Pro Ala Ser Val Ser Gly Ser Pro Gly Gln
1               5                   10                  15

Ser Ile Thr Ile Ser Cys Thr Gly Thr Ser Ser Asp Ile Gly Gly Tyr
            20                  25                  30

Asn Ser Val Ser Trp Tyr Gln Gln His Pro Gly Lys Ala Pro Lys Leu
        35                  40                  45

Met Ile Tyr Gly Val Asn Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Asp Ile Glu
                85                  90                  95

Ser Ala Thr Pro Val Phe Gly Gly Gly Thr Lys Leu Thr Val Leu Gly
            100                 105                 110

Gln

<210>  406
<211>  360
<212>  DNA
<213>  Homo sapiens

<400>  406
caggtggaat tggttcagag cggcgcggaa gtgaaaaaac cgggcgaaag cctgaaaatt      60

agctgcaaag gttccggata ttcctttact tcttattgga ttggttgggt gcgccaggcc     120

cctgggaagg gtctcgagtg gatgggcatt atcgatccgg gtgatagccg tacccgttat     180

tctccgagct ttcagggcca ggtgaccatt agcgcggata aaagcattag caccgcgtat     240

cttcaatgga gcagcctgaa agcgagcgat acggccatgt attattgcgc gcgtggtcag     300

ctttatggtg gtacttatat ggatggttgg ggccaaggca ccctggtgac ggttagctca     360

<210>  407
<211>  339
<212>  DNA
<213>  Homo sapiens

<400>  407
gatatcgcac tgacccagcc agcttcagtg agcggctcac caggtcagag cattaccatc      60

tcgtgtacgg gtactagcag cgatattggt ggttataatt ctgtgtcttg gtaccagcag     120

catcccggga aggcgccgaa acttatgatt tatggtgtta ataatcgtcc ctcaggcgtg     180

agcaaccgtt ttagcggatc caaaagcggc aacaccgcga gcctgaccat tagcggcctg     240

caagcggaag acgaagcgga ttattattgc tcttcttatg atattgagtc tgctactcct     300

gtgtttggcg gcggcacgaa gttaaccgtc ctaggtcag                            339

<210>  408
<211>  450
<212>  PRT

<213>  Homo sapiens

<400>  408

Gln Val Glu Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ser Tyr
            20                  25                  30

Trp Ile Gly Trp Val Arg Gln Ala Pro Gly Lys Gly Leu Glu Trp Met
        35                  40                  45

Gly Ile Ile Asp Pro Gly Asp Ser Arg Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Gly Gln Leu Tyr Gly Gly Thr Tyr Met Asp Gly Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  409
<211>  217
<212>  PRT
<213>  Homo sapiens

<400>  409

Asp Ile Ala Leu Thr Gln Pro Ala Ser Val Ser Gly Ser Pro Gly Gln
1               5                   10                  15

Ser Ile Thr Ile Ser Cys Thr Gly Thr Ser Ser Asp Ile Gly Gly Tyr
            20                  25                  30

Asn Ser Val Ser Trp Tyr Gln Gln His Pro Gly Lys Ala Pro Lys Leu
        35                  40                  45

Met Ile Tyr Gly Val Asn Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Asp Ile Glu
                85                  90                  95

Ser Ala Thr Pro Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Gly Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  410
<211>  1353
<212>  DNA
<213>  Homo sapiens

<400>  410

caggtggaat tggttcagag cggcgcggaa gtgaaaaaac cgggcgaaag cctgaaaatt 60

agctgcaaag gttccggata ttcctttact tcttattgga ttggttgggt gcgccaggcc 120

cctgggaagg gtctcgagtg gatgggcatt atcgatccgg gtgatagccg tacccgttat 180

tctccgagct ttcagggcca ggtgaccatt agcgcggata aaagcattag caccgcgtat 240

cttcaatgga gcagcctgaa agcgagcgat acggccatgt attattgcgc gcgtggtcag 300

ctttatggtg gtacttatat ggatggttgg ggccaaggca ccctggtgac ggttagctca 360

gcctccacca agggtccatc ggtcttcccc ctggcaccct cctccaagag cacctctggg 420

ggcacagcgg ccctgggctg cctggtcaag gactacttcc ccgaaccggt gacggtgtcg 480

tggaactcag gcgccctgac cagcggcgtg cacaccttcc cggctgtcct acagtcctca 540

ggactctact ccctcagcag cgtggtgacc gtgccctcca gcagcttggg cacccagacc 600

tacatctgca acgtgaatca caagcccagc aacaccaagg tggacaagaa agttgagccc 660

aaatcttgtg acaaaactca cacatgccca ccgtgcccag cacctgaact cctgggggga 720

ccgtcagtct tcctcttccc cccaaaaccc aaggacaccc tcatgatctc ccggacccct 780

gaggtcacat gcgtggtggt ggacgtgagc cacgaagacc ctgaggtcaa gttcaactgg 840

tacgtggacg gcgtggaggt gcataatgcc aagacaaagc cgcgggagga gcagtacaac 900

agcacgtacc gggtggtcag cgtcctcacc gtcctgcacc aggactggct gaatggcaag 960

gagtacaagt gcaaggtctc caacaaagcc ctcccagccc ccatcgagaa aaccatctcc 1020

aaagccaaag ggcagccccg agaaccacag gtgtacaccc tgcccccatc ccgggatgag 1080

ctgaccaaga accaggtcag cctgacctgc ctggtcaaag gcttctatcc cagcgacatc 1140

gccgtggagt gggagagcaa tgggcagccg gagaacaact acaagaccac gcctcccgtg 1200

ctggactccg acggctcctt cttcctctac agcaagctca ccgtggacaa gagcaggtgg 1260

cagcagggga acgtcttctc atgctccgtg atgcatgagg ctctgcacaa ccactacacg 1320

cagaagagcc tctccctgtc tccgggtaaa tga 1353

<210> 411
<211> 654
<212> DNA
<213> Homo sapiens

<400> 411
gatatcgcac tgacccagcc agcttcagtg agcggctcac caggtcagag cattaccatc 60

tcgtgtacgg gtactagcag cgatattggt ggttataatt ctgtgtcttg gtaccagcag 120

catcccggga aggcgccgaa acttatgatt tatggtgtta ataatcgtcc ctcaggcgtg 180

agcaaccgtt ttagcggatc caaaagcggc aacaccgcga gcctgaccat tagcggcctg 240

caagcggaag acgaagcgga ttattattgc tcttcttatg atattgagtc tgctactcct 300

gtgtttggcg gcggcacgaa gttaaccgtc ctaggtcagc ccaaggctgc cccctcggtc 360

actctgttcc cgccctcctc tgaggagctt caagccaaca aggccacact ggtgtgtctc 420

ataagtgact tctacccggg agccgtgaca gtggcctgga aggcagatag cagccccgtc 480

aaggcgggag tggagaccac cacaccctcc aaacaaagca acaacaagta cgcggccagc 540

agctatctga gcctgacgcc tgagcagtgg aagtcccaca gaagctacag ctgccaggtc 600

acgcatgaag ggagcaccgt ggagaagaca gtggccccta cagaatgttc atag 654